(19) Europäisches Patentamt / European Patent Office / Office européen des brevets

(11) **EP 3 303 636 B1**

(12) **EUROPEAN PATENT SPECIFICATION**

(45) Date of publication and mention
of the grant of the patent:
**10.11.2021 Bulletin 2021/45**

(21) Application number: **16804415.4**

(22) Date of filing: **02.06.2016**

(51) Int Cl.:
*A61K 38/20* (2006.01)  *A61K 35/28* (2015.01)
*A61P 35/00* (2006.01)  *C12N 5/0775* (2010.01)
*A61K 38/43* (2006.01)

(86) International application number:
**PCT/US2016/035472**

(87) International publication number:
**WO 2016/196765 (08.12.2016 Gazette 2016/49)**

(54) **COMPANION METHODS FOR IL-2-BASED THERAPIES AND MESENCHYMAL STEM CELL-BASED THERAPIES**

BEGLEITVERFAHREN FÜR IL-2-BASIERTE THERAPIEN UND THERAPIEN AUF DER BASIS VON MESENCHYMALEN STAMMZELLEN

MÉTHODES D'ACCOMPAGNEMENT POUR THÉRAPIES À BASE D'IL-2 ET THÉRAPIES À BASE DE CELLULES SOUCHES MÉSENCHYMATEUSES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.06.2015 US 201562170619 P**
**03.06.2015 US 201562170604 P**
**12.06.2015 US 201562175203 P**

(43) Date of publication of application:
**11.04.2018 Bulletin 2018/15**

(73) Proprietor: **Aelan Cell Technologies, Inc.**
**San Francisco, CA 94107 (US)**

(72) Inventors:
 • **LUNYAK, Victoria**
 **San Francisco, CA 94107 (US)**
 • **GAUR, Meenakshi**
 **San Francisco, CA 94116 (US)**

(74) Representative: **Cooley (UK) LLP**
**22 Bishopsgate**
**London EC2N 4BQ (GB)**

(56) References cited:
**WO-A1-92/16235     US-A1- 2006 160 187**
**US-A1- 2013 095 065     US-A1- 2013 252 821**

• **PANELLI MONICA C ET AL: "Forecasting the cytokine storm following systemic interleukin (IL)-2 administration", JOURNAL OF TRANSLATIONAL MEDICINE, BIOMED CENTRAL, vol. 2, no. 1, 2 June 2004 (2004-06-02), page 17, XP021009819, ISSN: 1479-5876, DOI: 10.1186/1479-5876-2-17**
• **Anonymous: "Agilent Whole Human Genome Oligo Microarray Kit with SurePrint Technology Whole Human Genome Oligo Microarray Kit", , 1 January 2004 (2004-01-01), pages 1-4, XP055514859, Retrieved from the Internet: URL:http://www.swisslabs.eu/uploads/files/ Whole_Human_Genome_Oligo_Microarray_Kit. pd f [retrieved on 2018-10-12]**
• **SEOK-GU KANG ET AL: "Cytotoxicity of human umbilical cord blood-derived mesenchymal stem cells against human malignant glioma cells", CHILD'S NERVOUS SYSTEM, SPRINGER, BERLIN, DE, vol. 24, no. 3, 30 October 2007 (2007-10-30), pages 293-302, XP019588767, ISSN: 1433-0350**
• **PANELLI ET AL.: 'Forecasting the cytokine storm following systemic interleukin (IL) -2 administration' J. TRANSLATIONAL MED, [Online] vol. 2, no. 17, 02 June 2004, pages 1 - 14, XP021009819 Retrieved from the Internet: <URL:doi:10.1186/1479-5876-2-17>**

**(Cont. next page)**

• PING NIU ET AL: "Transcriptional profiling of interleukin-2-primed human adipose derived mesenchymal stem cells revealed dramatic changes in stem cells response imposed by replicative senescence", ONCOTARGET, vol. 6, no. 20, 14 July 2015 (2015-07-14), XP055514228, DOI: 10.18632/oncotarget.4852

**Description**

**BACKGROUND OF THE INVENTION**

Interleukin 2 (IL-2) is a type of cytokine signaling molecule in the immune system and is used therapeutically. IL-2 is manufactured using recombinant DNA technology and is marketed as a protein therapeutic called aldesleukin (branded as Proleukin®). IL-2 is approved in several countries for the treatment of cancers (metastatic melanoma and renal cell carcinoma) and HIV.

IL-2 has been approved as a chemotherapeutic agent for cancer treatment with a high-dose regimen, but it may also be administered in a low-dose form. The high-dose regimen involves giving the drug intravenously, every eight hours, as tolerated, for up to 15 doses. High-dose IL-2 therapy produces overall response rates of only about 15% to 20%; moreover, it is associated with significant toxicities that affect essentially every organ system. Because of the severity of these side effects, patients are hospitalized and sometimes need intensive care unit support while the drug is being given; in severe cases, IL-2 treatment is discontinued.

Human mesenchymal stem cells (MSCs) are currently one of the primary sources of stem cells for transplantation to treat a variety of conditions (Kucerova, Cancer Res July 1, 2007 67; 6304). Such transplanted stem cells in the presence of a pro-inflammatory or otherwise inhospitable environment *in vivo* may produce unwanted adverse events. Little is known regarding the extent to which the beneficial properties of MSCs are affected by their local environment. PANELLI MONICA C ET AL: "Forecasting the cytokine storm following systemic interleukin (IL)-2 administration", JOURNAL OF TRANSLATIONAL MEDICINE, BIOMED CENTRAL, vol. 2, no. 1, 2 June 2004, page 17, and US 2013/252821A1 relate to adverse events occurring during or after IL-2-based therapy.

Taken together, to date, little is known in how to determine potential adverse events associated with the administration of an IL-2 therapy, and the potential adverse effects of the environment on transplanted MSCs. Thus there exists a need for companion methods and kits for improving the treatment of patients who are treated with IL-2, and for patients receiving MSC-based therapies. Described herein are methods and kits for these purposes.

**SUMMARY OF THE INVENTION**

The present invention is described in the appended claims. Described herein are companion methods and kits for determining whether an individual eligible to receive an IL-2-based therapy or whether an individual already receiving an IL-2-based therapy will potentially experience adverse events associated with that IL-2-based therapy. If it is determined that the individual may experience adverse events, such as an increased risk of tumorigenesis or metastasis, rather than the eradication of the underlying disease such as cancer, a treatment decision can be taken to not undergo any further IL-2-based therapy. Likewise, if it is determined that it is likely that the individual may not experience adverse events associated with the IL-2 based therapy, a decision can be made to commence or continue the administration of the IL-2 based therapy.

Also described herein are companion methods and kits for determining whether an individual eligible to receive a MSC-based therapy will potentially experience adverse events associated with the therapy.

Thus, in one aspect of the disclosure, provided herein are methods for determining whether an individual eligible to receive an IL-2-based therapy may experience an adverse event associated with the IL-2-based therapy, the method comprising: (a) measuring the expression levels of at least two biomarkers selected from a panel of biomarkers in a sample from the individual, wherein either (1) the individual has received at least one dose of an IL-2-based therapy or (2) the sample is combined with IL-2 *in vitro,* and wherein the panel of biomarkers comprises TIE-1, TIE-2, TIMP-4, FGF1, LIF, TGFBR2, CSF1, TGFα, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1; and (b) comparing the levels of the biomarkers to reference levels, wherein an increase in the levels above the reference levels indicates that the individual may experience an adverse event associated with the IL-2-based therapy, and wherein a decrease or no change in the levels compared to the references levels indicates that the individual may not experience an adverse event associated with the IL-2-based therapy. In one variation of the disclosure, the individual has received (e.g. has been administered) at least one dose of an IL-2-based therapy; in some variations, the individual has received the IL-2-based therapy for the treatment of a cancer. In some variations, the sample has been obtained from the individual 24, 48, or 72 hours after having received the IL-2-based therapy. In another variation, the sample from the individual is combined with IL-2 *in vitro* for further analysis; in some variations, the sample can be combined for a period of about 24 hours, after which the biomarkers are measured 24, 48, or 72 hours following removal of the IL-2. As contemplated herein, the sample can be any biological sample; in one variation, the sample is a blood, plasma, or serum sample. In particular variation, the method comprises measuring the expression levels of at least three biomarkers from the panel of biomarkers, at least four biomarkers from the panel of biomarkers, or at least five biomarkers from the panel of biomarkers. In some variations, the method comprises measuring the expression levels of TIE-1, TIE-2, TIMP-4, VEGFA, PLEKHA1, VEGFB, CRMP1, FERMT1, CTSB, PLEKHA6, GNB2L1, and TGFβ1. In some variations, the method may further comprise measuring additional biomarkers. In one particular variation, the method further comprises measuring the expression level of SIVA1, and querying for decreased expression of this biomarker. As contemplated herein, either the RNA or protein expression levels can be measured. Accordingly, in one variation, the method comprises measuring protein levels of the biomarkers, for example with an ELISA assay, an antibody proteomic array, immunohistochemistry, or mass spectrometry. In another variation, the method comprises measuring RNA levels of the biomarkers, for example with a Q-PCR assay or RNA-seq. In some variations, the method comprises obtaining the sample from the individual as a part of the method. Upon determination of the results of the expression, the method may comprise further comprising administering an effective amount of the IL-2-based therapy to the individual if it is determined in step (b) that the individual may not experience an adverse event associated with the IL-2-based therapy. The method may even further comprise administering a rejuvenation therapy to the individual if it is determined in step (b) that the individual may experience an adverse event associated with the IL-2-based therapy.

In another aspect, the disclosure provides a method of treating an individual for cancer with an IL-2-based therapy. In one variation, the method of treating an individual for cancer with an IL-2-based therapy comprises (a) measuring the expression levels of at least two biomarkers selected from a panel of biomarkers in a sample from the individual, wherein either (1) the individual has received at least one dose of an IL-2-based therapy or (2) the sample is combined with IL-2 *in vitro,* and wherein the panel of biomarkers comprises TIE-1, TIE-2, TIMP-4, FGF1, LIF, TGFBR2, CSF1, TGFα, TGFβ1, IL17D, SDF2, TGFBRAP1, FGF11, TNFSF13B, FGF14, IL1B, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1; (b) comparing the levels of the biomarkers to reference levels, wherein no change or a decrease in the levels below the reference levels indicates that the individual may not experience an adverse event associated with the IL-2-based therapy; and (c) administering an effective amount of the IL-2-based therapy to the individual if it determined in step (b) that the individual may not experience an adverse event associated with the IL-2-based therapy. In another variation, the method of treating an individual for cancer with an IL-2-based therapy comprises administering an effective amount of the IL-2-based therapy to the individual when the expression levels of at least two biomarkers is decreased or exhibits no change, in comparison to reference levels in a sample from the individual wherein either (1) the individual has received at least one dose of an IL-2-based therapy or (2) the sample is combined with IL-2 *in vitro,* and wherein the panel of biomarkers comprises TIE-1, TIE-2, TIMP-4, FGF1, LIF, TGFBR2, CSF1, TGFα, TGFβ1, IL17D, SDF2, TGFBRAP1, FGF11, TNFSF13B, FGF14, IL1B, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1. In one variation of the disclosure, the individual has received (e.g. has been administered) at least one dose of an IL-2-based therapy. In some variations, the sample has been obtained from the individual 24, 48, or 72 hours after having received the IL-2-based therapy. In another variation, the sample from the individual is combined with IL-2 *in vitro* for further analysis; in some variations, the sample can be combined for a period of about 24 hours, after which the biomarkers are measured 24, 48, or 72 hours following removal of the IL-2. As contemplated herein, the sample can be any biological sample; in one variation, the sample is a blood, plasma, or serum sample. In particular variation, the method comprises measuring the expression levels of at least three biomarkers from the panel of biomarkers, at least four biomarkers from the panel of biomarkers, or at least five biomarkers from the panel of biomarkers. In some variations, the method comprises measuring the expression levels of TIE-1, TIE-2, TIMP-4, VEGFA, PLEKHA1, VEGFB, CRMP1, FERMT1, CTSB, PLEKHA6, GNB2L1, and TGFβ1. In some variations, the method may further comprise measuring additional biomarkers. In one particular variation, the method further comprises measuring the expression level of SIVA1, and querying for decreased expression of this biomarker. As contemplated herein, either the RNA or protein expression levels can be measured. Accordingly, in one variation, the method comprises measuring protein levels of the biomarkers, for example with an ELISA assay, an antibody proteomic array, immunohistochemistry, or mass spectrometry. In another variation, the method comprises measuring RNA levels of the biomarkers, for example with a Q-PCR assay or RNA-seq. In some variations, the method comprises obtaining the sample from the individual as a part of the method. The method may even further comprise administering a rejuvenation therapy to the individual if it is determined that the individual may experience an adverse event associated with the IL-2-based therapy.

In another aspect, the disclosure provides a method of determining whether a population of mesenchymal stem cells (MSCs) is suitable for administration into an individual for a MSC-based therapy, comprising: (a) incubating IL-2 with the population of MSCs; (b) measuring the expression levels in the MSCs of at least two biomarkers selected from a panel of biomarkers, wherein the panel of biomarkers comprises TIE-1, TIE-2, TIMP-4, FGF1, LIF, TGFBR2, CSF1, TGFα, TGFβ1, IL17D, SDF2, TGFBRAP1, FGF11, TNFSF13B, FGF14, IL1B, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1; and (c) comparing the levels of the biomarkers to reference levels, wherein an increase in the levels above the reference levels indicates that the MSCs are not suitable for administration into an individual and no change or a decrease in the levels below the reference levels indicates that the MSCs are suitable for administration into an individual. In some variations, the method comprises measuring the expression levels of at least three biomarkers from the panel of biomarkers, measuring the expression levels of at least four biomarkers from the panel of biomarkers, or measuring the expression levels of at least five biomarkers from the panel of biomarkers. In some variations, the method comprises measuring the expression levels of TIE-1, TIE-2, TIMP-4, VEGFA, PLEKHA1, VEGFB, CRMP1, FERMT1, CTSB, PLEKHA6, GNB2L1, and TGFβ1. In some variations, the method may further comprise measuring additional biomarkers. In one particular variation, the method further comprises measuring the expression level of SIVA1, and querying for decreased expression of this biomarker. As contemplated herein, either the RNA or protein expression levels can be measured. Accordingly, in one variation, the method comprises measuring protein levels of the biomarkers, for example with an ELISA assay, an antibody proteomic array, immunohistochemistry, or mass spectrometry. In another variation, the method comprises measuring RNA levels of the biomarkers, for example with a Q-PCR assay or RNA-seq. In some variations, the incubation period is for about 24 hours. In some variations, the measuring is carried out 24, 48, or 72 hours following the incubation period with IL-2. In some variations, the method further comprises administering the population of cells to the individual. The method may further comprise rejuvenating the cells prior to administering the cells to the individual.

another aspect of the disclosure provided herein are kits for assessing the suitability of a population of MSCs for transplant or for determining whether an IL-2-based therapy should be administered comprising reagents for measuring the expression level of at least two biomarkcrs selected from a panel of biomarkcrs in a sample, wherein the panel of biomarkcrs comprises TIE-1, TIE-2, TIMP-4, FGF1, LIF, TGFBR2, CSF1, TGFα, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1. In some variations, the kit comprises reagents for measuring the expression level of at least three biomarkers in the sample, for measuring the expression level of at least four biomarkers in the sample, or for measuring the expression level of at least five biomarkers in the sample. In some variations, the kit comprises reagents for measuring TIE-1, TIE-2, TIMP-4, VEGFA, PLEKHA1, VEGFB, CRMP1, FERMT1, CTSB, PLEKHA6, GNB2L1, and TGFβ1. In some variations, the kit comprises IL-2.

It is to be understood that one, some, or all of the properties of the various variations described herein may be combined to form other variations of the present disclosure. These and other aspects will become apparent to one of skill in the art.

**BRIEF DESCRIPTION OF THE DRAWINGS**

FIG. 1A is schematic illustrating the positioning of MSCs *in vivo* on blood vessels, rendering them capable of sensing and responding to an inflammatory environment, and treatment with cytokines.

FIG. 1B is a chart illustrating one exemplary method of testing an individual, or a stem cell to determine if an IL-2-based therapy should be initiated or continued.

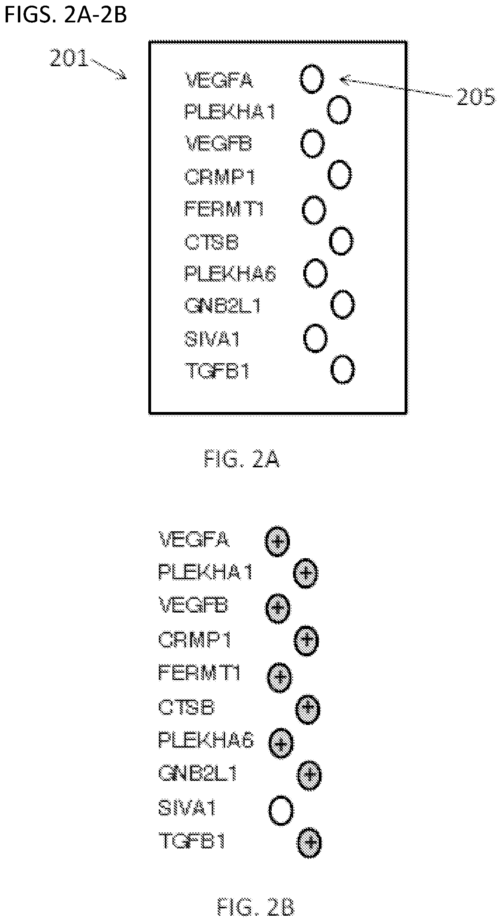

FIG. 2A is a schematic of an exemplary assay for determining if an IL-2-based therapy should be administered. FIG. 2B illustrates example screening results using the assay shown in FIG. 2A. In FIG. 2B the result is positive, with the expression of several markers increased, indicating that potential adverse events could be associated with the administration of IL-2.

FIGS. 3A-3D illustrate how replicative senescence (SEN) impairs the migratory properties of the human adipose derived mesenchymal stem cells (hADSCs, also referred to herein interchangeably hADSCs). FIG. 3A shows a growth curve of hADSCs and is represented as cumulative population doubling over day in culture. FIG. 3B shows detection of senescence-associated β-galactosidase. FIG. 3C shows *ex vivo* migration assays for SR (left) and SEN (right) hADSCs. FIG. 3D shows the migration of SR-hADSCs (left) and SEN-hADSCs (right).

FIGS. 4A-4C illustrate the gene expression of IL-2 receptor isoforms and their association with membrane in SR-hADSCs and SEN-hADSCs induced with IL-2. FIG. 4A shows a schematic representation of IL-2 receptor composition. FIG. 4B shows IL-2 receptors α, β, and γ assessed by quantitative PCR (Q-PCR) SR and SEN hADSC, either, in the presence or absence of IL-2. FIG. 4C shows the cellular membrane-associated protein levels of IL-2Ra and IL-2Rβ.

FIG. 5 illustrates the effect of stimulation of the SR and SEN hADSCs with IL-2. STAT5A, STAT5B and VEGFA mRNA expression was assessed by quantitative RT-PCR.

FIGS. 6A-6D show a comparison of gene expression levels between SR and SEN cells upon IL-2 treatment.

FIGS. 7A-7D illustrate gene expression levels for SR and SEN cells upon IL-2 treatment among functionally coherent sets of genes.

FIGS. 8A-8D illustrate the analysis for RNA-seq profiling of SR and SEN hADSCs subjected to IL-2 treatment. FIG. 8A provides a schematic representation of the RNA-seq analysis design. FIG. 8B shows the distributions of the gene-specific RNA-seq read counts for each condition prior to ACTB normalization. FIG. 8C shows the condition-specific RNA-seq read counts for ACTB that were used for normalization. FIG. 8D shows distributions of the gene specific RNA-seq read counts for each condition after ACTB normalization.

FIG. 9 shows External RNA Controls Consortium (ERCC, a common set of external RNA controls) dose response used for quality control of RNA-seq experiments.

FIGS. 10A-10B represent tables of the genes differentially expressed upon IL-2 treatment in SEN and SR hAMCS.

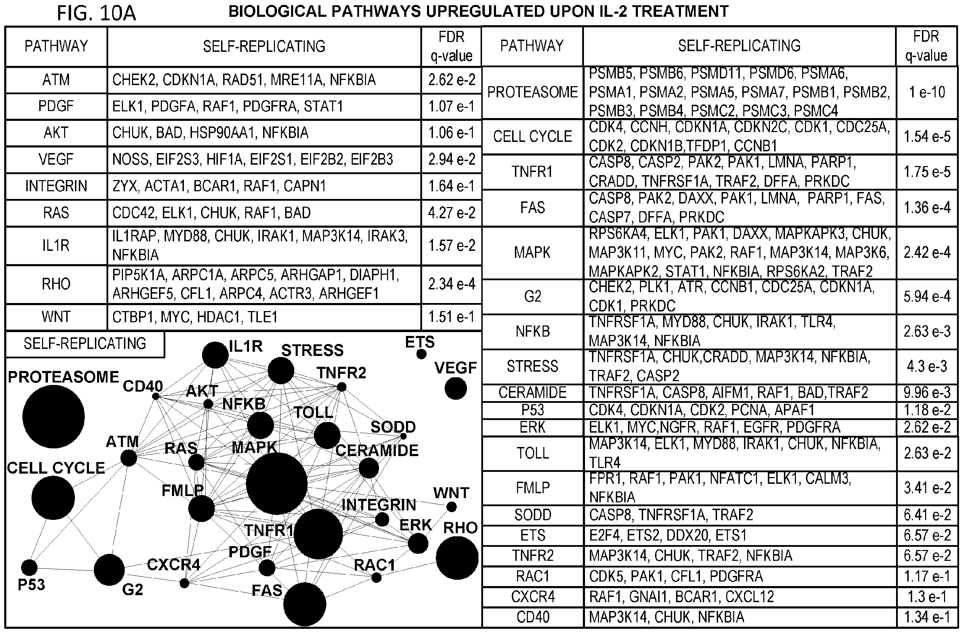

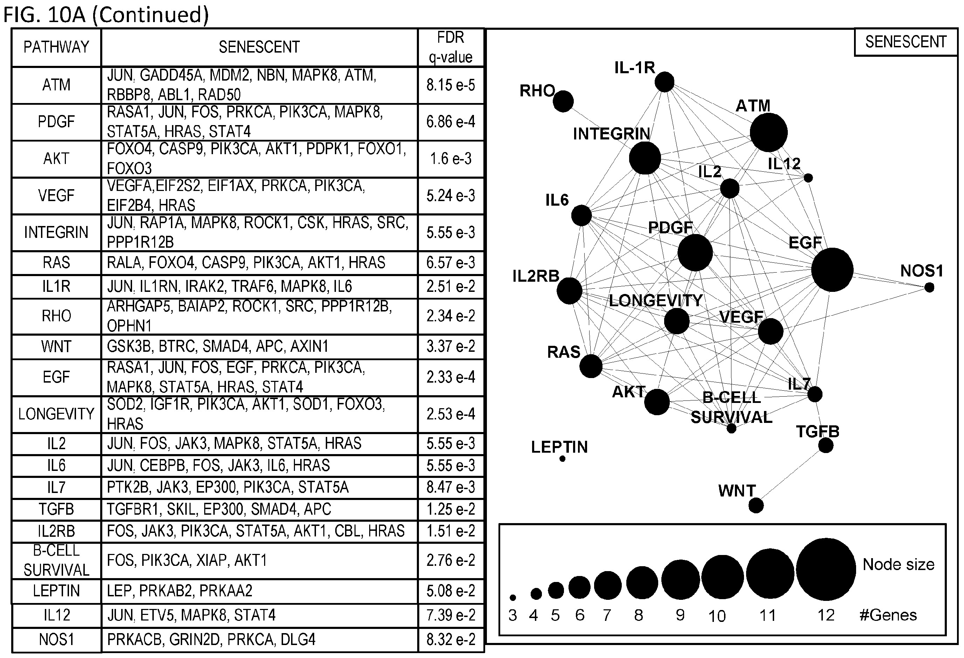

FIG. 10A shows biological pathways enriched for genes up-regulated upon IL-2 treatment in SR and SEN hADSCs.

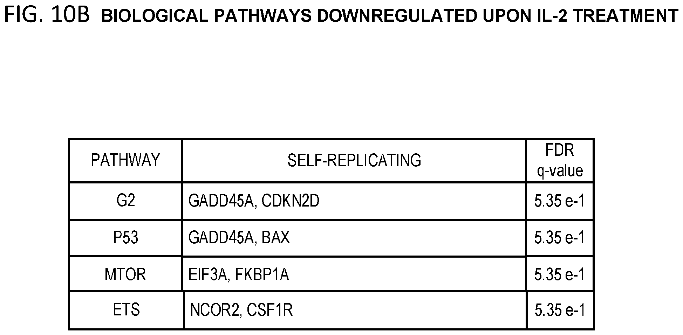

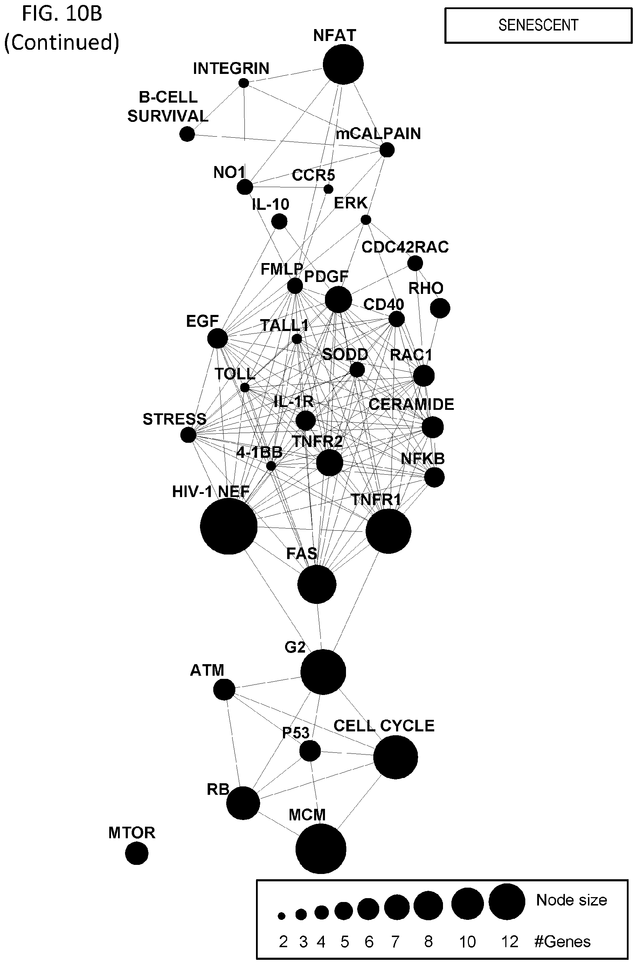

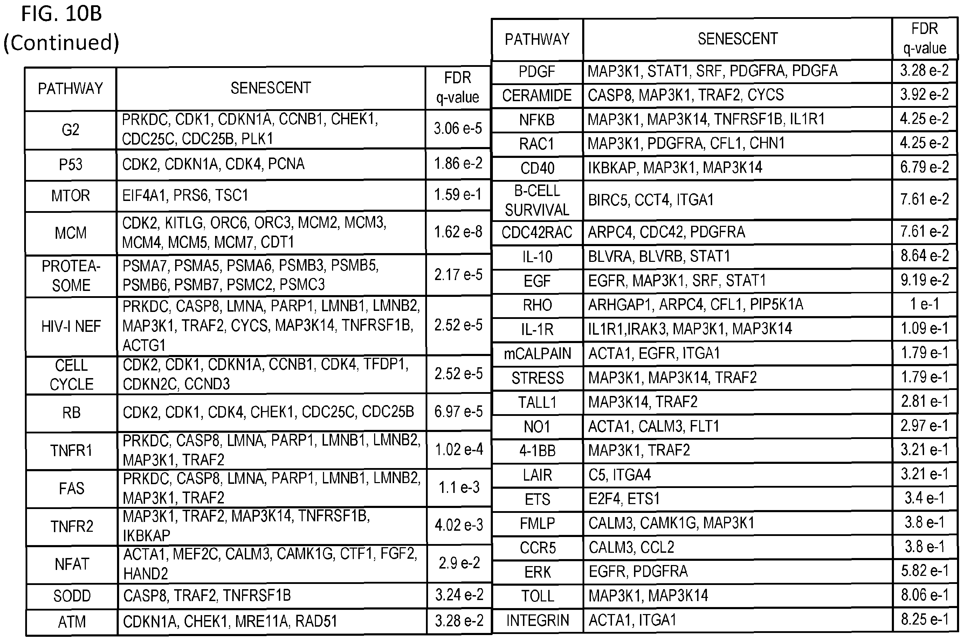

FIG. 10B shows biological pathways enriched for genes down-regulated upon IL-2 treatment in SR and SEN hADSCs.

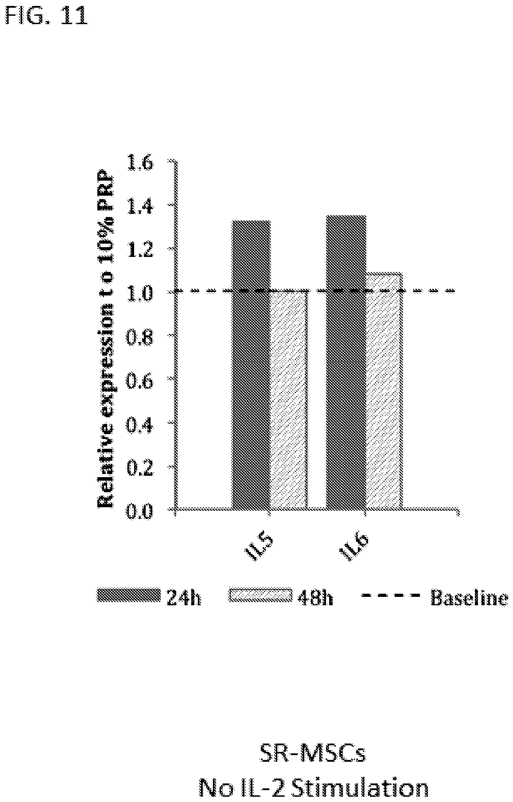

FIGS. 11-90 illustrate the increase in secretion of the named proteins (factors) from SR-hADSCs or SEN-hADSCs, following incubation with media alone (no IL-2 stimulation) or following stimulation with IL-2.

## DETAILED DESCRIPTION

### I. Methods

#### 1. Introduction

Provided herein are companion methods and kits useful for IL-2-based therapies and for mesenchymal stem cell-based therapies.

Specifically, in one variation of the disclosure, provided herein are methods for determining whether an individual eligible to receive an IL-2-based therapy, for treatment of a condition such as cancer or HIV, may experience an adverse event associated with that IL-2-based therapy. The method involves measuring an increase in the expression levels of certain biomarkers in a sample from the individual upon exposure to IL-2, such biomarkers indicative of the cellular senescence of MSCs in the sample. The expression of such biomarkers would indicate that the individual could experience an adverse event (such as tumorigenesis or metastasis) if the IL-2-based therapy were to be administered or continued. Treatment decisions can be made based on the practice of this method and use of the kits described herein.

In another variation of the disclosure, provided here are methods for determining the suitability of a population of MSCs for administration (e.g. transplant) into an individual for an MSC-based therapy. The method involves measuring a change in the expression levels of certain biomarkers in the MSCs upon exposure to IL-2, such biomarkers associated with cellular senescence of the MSCs. If an increase in the biomarkers is observed, it would indicate that if the cells were administered to an individual, the individual may experience adverse events associated with the transplantation, such as metastatic transformation and invasive growth. Conversely if no change or a decrease in the expression levels of biomarkers is observed, it could indicate the suitability of the population of cells for administration. Treatment decisions regarding MSC transplantation can be made based on the practice of this method and use of the kits described herein.

These are discussed in further detail herein.

#### 2. SEN-MSCs and SR-MSCs

Provided herein are methods for measuring the expression levels of biomarkers upon exposing a sample comprising MSCs to IL-2, wherein the biomarkers are indicative of cellular senescence. As used herein SEN MSCs are those cells that are replicatively senescent. Replicative senescence is characterized by growth arrest, apoptosis resistance, high levels of metabolic activities, morphological and cell-size changes, high levels of expression of the tumor suppressors P16, P21, P53 and/or RB, increased activity of senescence associated beta galactosidase (SA-$\beta$-gal) and the loss of the ability to synthesize and repair DNA. The replicative aging of MSCs can influence their biological properties.

SR-MSCs, on the other hand, are associated with being productive: express a set of coding or non-coding RNAs indicative of quality; are self-renewing; are not senescent; are not nearing senescence; have been passaged 6 times or less; exhibit high growth potential; produce proteins of interest; allow for long-term tissue regeneration; induce long-term correction of a disease; exhibit no or only a low chance of immortalization; exhibit no or low tumorigenic potential; and contain few or no pro-viral integrations. In an exemplary variation, productive stem cells are self-renewing. In some variations, productive stem cells exhibit at least two, three, four, five, or more of the features associated with being productive.

#### 3. Detection of the Biomarkers of Interest

The companion methods described herein are dependent on measuring expression levels of at least two biomarkers from a panel of biomarkers associated with cellular senescence, for example the panel may comprise biomarkers that are anti-apoptotic, angiogenic, tumorigenic, lead to vascular development, responsible for invasive growth, metastasis, cell motility, migration and the like. Generally, the methods provide measurement of the markers to make a determination if use of an IL-2 therapy, or transplantation of MSCs, could lead to adverse events. As used herein, adverse events following an IL-2 therapy or MSC transplantation include, but are not limited to an increase in tumorigenesis, anti-apoptotic activity, angiogenesis, vascular development, invasive growth, metastasis, cell motility, and migration.

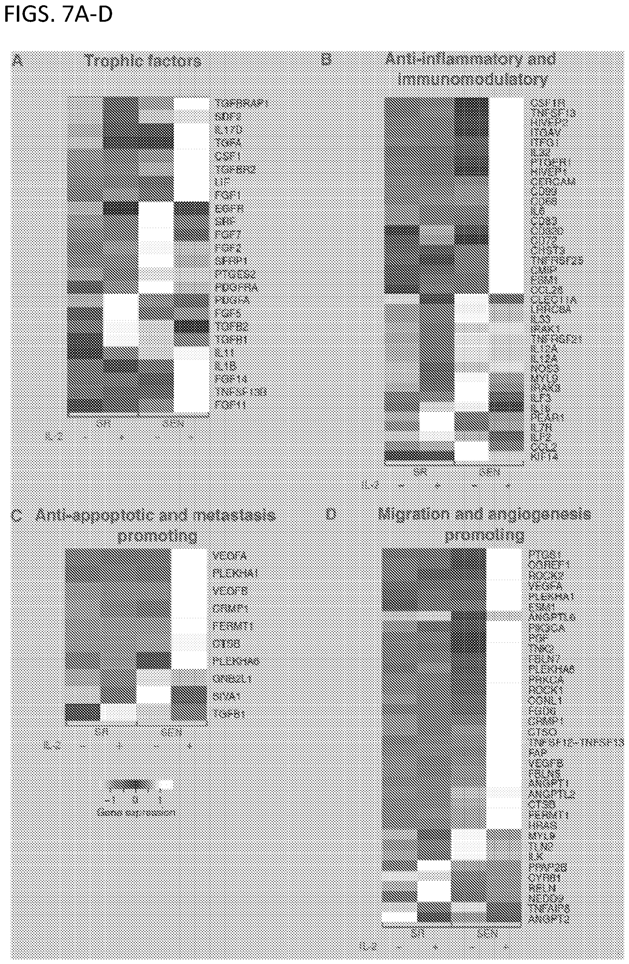

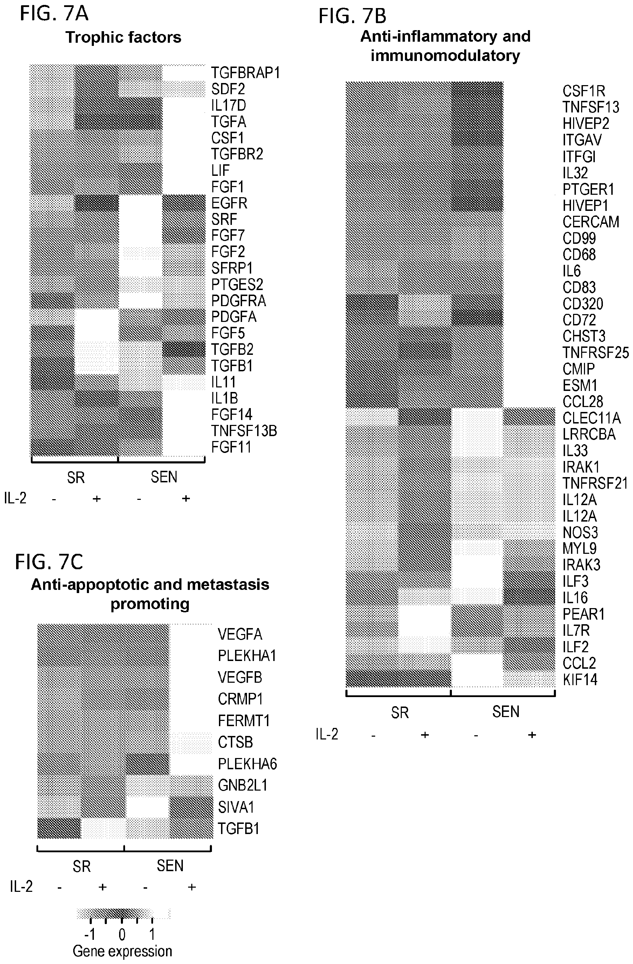

As provided herein, biomarkers associated with cellular senescence that are upregulated in response to IL-2 can include those listed in Tables 1-4, Table 5B, and FIGS. 7A-7D.

In some variations the method comprises measuring RNA levels of the biomarkers. Such methods are known to those in the art, and include, but are not limited to the use of an Q-PCR, array-based technologies, RNA-seq, transcriptome analysis, single-cell transcriptomic analysis, and in situ-hybridization.

In some variations the method comprises measuring protein levels of the biomarkers. Such methods are known to those in the art, and include, but are not limited to the use of an ELISA assay, proteomic array, immunohistochemistry, Western Blot, mass spectrometry (MS), an antibody array, or a chemiluminescence assay.

In some variations the method comprises measuring both the RNA and protein levels of the biomarkers.

The biomarkers are measured, and compared to a reference level. As contemplated herein, a reference level can comprise a sample from the same individual before IL-2 treatment; can comprise a sample from a healthy individual who has not received any IL-2; or can comprise a collection of samples representing a heterogeneous group of individuals who have not received IL-2 treatment.

In particular variations, it is determined that the RNA expression levels of a biomarker is increased if it displays at least 1.1, at least 1.2, at least 1.3, at least 1.4, at least 1.5, at least 2, at least 3, at least 4, at least 5, at least 6, at least 7, at least 8, at least 9, at least 10, at least 15, at least 20, at least 25, at least 50, at least 75, or even at least 100-fold higher expression than the reference level of the biomarker. Fold can generally refer to raw fold values, GFOLD values, or fold values calculated using algorithms known to a skilled artisan.

RNA-dependent differences in the gene expression can be measured using a GFOLD calculation method (Feng et al, Bioinformatics. 2012), for estimating fold changes that takes into account the uncertainty of gene expression measurement by RNA-seq. In these variations, the use of GFOLD allows for the relative difference of gene expression and facilitates the comparison of genes with different expression levels or of different lengths.

In particular variations, it is determined that the protein expression levels of a biomarker is increased if it displays at least 1.1, at least 1.2, at least 1.3, at least 1.4, at least 1.5, at least 2, at least 3, at least 4, at least 5, at least 6, at least 7, at least 8, at least 9, at least 10, at least 15, at least 20, at least 25, at least 50, at least 75, or even at least 100 fold higher expression fold higher expression than the reference level of the biomarker.

### 4. Identity of the Biomarkers of Interest

As referred to herein, by "biomarker" it is meant any biological molecule (or fragment thereof) of interest, e.g. a biomarker which is present on the cell cytoplasm, surface, or secreted out. Such biomarkers include, but are not limited to, biomolecules comprising polypeptides, proteins, carbohydrates, lipids, glycoproteins, ribonucleoproteins, lipoproteins, glycolipids and fragments thereof. Where the biomarker comprises a protein, the protein can be a secreted protein, an intracellular protein, or a membrane protein. Biomarker proteins include, but are not limited to, peptides, polypeptides, glycoproteins, lipoproteins, cytokines, growth factors, antibodies, and other immunogenic molecules. The biomarker also may be a transmembrane protein or may be bound to a transmembrane protein or membrane lipid, for example.

As provided herein, biomarkers associated with cellular senescence that are upregulated in response to IL-2 are those listed in Tables 1-4, Table 5B, and FIGS. 7A-7D.

In some variations, the methods comprise measuring the expression levels of at least 3, at least 4, at least 5, at least 6, at least 7, at least 8, at least 9, at least 10, at least 11, at least 12, at least 13, at least 14, at least 15, at least 16, at least 17, at least 18, at least 19, or even at least 20 biomarkers from the panel of biomarkers for determination of potential adverse events associated with IL-2-based therapeutics and/or for the determination of the suitability of a population of MSCs for transplantation.

In some variations, the expression levels of at least two biomarkers selected from any of the biomarkers presented in Tables 1-4 are measured. In some variations, the expression levels of at least two biomarkers presented in FIGS. 7A-7D are measured. In some variations, the expression levels of at least two biomarkers presented in Table 5B are measured

In one variation, the expression levels of at least two biomarkers are measured, wherein the first biomarker is TIE-1 and the second biomarker is selected from the group consisting of TIE-2, TIMP-4, FGF1, LIF, TGFBR2, CSF1, TGFα, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is TIE-2 and the second biomarker is selected from the group consisting of TIE-1, TIMP-4, FGF1, LIF, TGFBR2, CSF1, TGFα, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1. In one variation, the expression levels of at least two biomarkers are measured, wherein the first biomarker is TIE-1 and the second biomarker is selected from the group consisting of TIE-1, TIE-2, TIMP-4, FGF1, LIF, TGFBR2, CSF1, TGFα, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is TIMP-4 and the second biomarker is selected from the group consisting of TIE-1, TIE-2, FGF1, LIF, TGFBR2, CSF1, TGFα, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is FGF1 and the second biomarker is selected from the group consisting of TIE-1, TIE-2, TIMP-4, LIF, TGFBR2, CSF1, TGFα, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is LIF and the second biomarker is selected from the group consisting of TIE-1, TIE-2, TIMP-4, FGF1, TGFBR2, CSF1, TGFα, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is TGFBR2 and the second biomarker is selected from the group consisting of TIE-1, TIE-2, TIMP-4, FGF1, LIF, CSF1, TGFα, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is CSF1 and the second biomarker is selected from the group consisting of TIE-1, TIE-2, TIMP-4, FGF1, LIF, TGFBR2, TGFα, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is TGFα and the second biomarker is selected from the group consisting of TIE-1, TIE-2, TIMP-4, FGF1, LIF, TGFBR2, CSF1, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is TGFβ1 and the second biomarker is selected from the group consisting of TIE-2, TIMP-4, FGF1, LIF, TGFBR2, CSF1, TGFα, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is IL17D and the second biomarker is selected from the group consisting of TIE-1, TIMP-4, FGF1, LIF, TGFBR2, CSF1, TGFα, TGFβ1, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is SDF2 and the second biomarker is selected from the group consisting of TIE-1, TIE-2, TIMP-4, FGF1, LIF, TGFBR2, CSF1, TGFα, TGFβ1, IL17D, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is TGFBRAP1 and the second biomarker is selected from the group consisting of TIE-1, TIE-2, FGF1, LIF, TGFBR2, CSF1, TGFα, TGFβ1, IL17D, SDF2, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is FGF11 and the second biomarker is selected from the group consisting of TIE-1, TIE-2, TIMP-4, LIF, TGFBR2, CSF1, TGFα, TGFβ1, IL17D, SDF2, TGFBRAP1, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is TNFSF13B and the second biomarker is selected from the group consisting of TIE-1, TIE-2, TIMP-4, FGF1, TGFBR2, CSF1, TGFα, TGFβ1, IL17D, SDF2, TGFBRAP1, FGF11, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is FGF14 and the second biomarker is selected from the group consisting of TIE-1, TIE-2, TIMP-4, FGF1, LIF, CSF1, TGFα, TGFβ1, IL17D, SDF2, TGFBRAP1, FGF11, TNFSF13B, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is IL1β and the second biomarker is selected from the group consisting of TIE-1, TIE-2, TIMP-4, FGF1, LIF, TGFBR2, TGFα, TGFβ1 IL17D, SDF2, TGFBRAP1, FGF11, TNFSF13B, FGF14, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is IL-11, and the second biomarker is selected from the group consisting of TIE-1, TIE-2, TIMP-4, FGF1, LIF, TGFBR2, CSF1, TGFβ1, IL17D, SDF2, TGFBRAP1, FGF11, TNFSF13B, FGF14, IL1β, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is IL-32 the second biomarker is selected from the group consisting of TIE-2, TIMP-4, FGF1, LIF, TGFBR2, CSF1, TGFα, IL17D, SDF2, TGFBRAP1, FGF11, TNFSF13B, FGF14, IL1β, IL-11, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is IL-6 and the second biomarker is selected from the group consisting of TIE-1, TIMP-4, FGF1, LIF, TGFBR2, CSF1, TGFα, TGFβ1, SDF2, TGFBRAP1, FGF11, TNFSF13B, FGF14, IL1β, IL-11, IL-32, IL1RN, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is IL1RN and the second biomarker is selected from the group consisting of TIE-1, TIE-2, TIMP-4, FGF1, LIF, TGFBR2, CSF1, TGFα, TGFβ1, IL17D, TGFBRAP1, FGF11, TNFSF13B, FGF14, IL1β, IL-11, IL-32, IL-6, IL-20RB, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is IL-20RB and the second biomarker is selected from the group consisting of TIE-1, TIE-2, FGF1, LIF, TGFBR2, CSF1, TGFα, TGFβ1, IL17D, SDF2, FGF11, TNFSF13B, FGF14, IL1β, IL-11, IL-32, IL-6, IL1RN, IL-21R , PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is IL-21R and the second biomarker is selected from the group consisting of TIE-1, TIE-2, TIMP-4, LIF, TGFBR2, CSF1, TGFα, TGFβ1, IL17D, SDF2, TGFBRAP1, TNFSF13B, FGF14, IL1β, IL-11, IL-32, IL-6, IL1RN, IL-20RB, PLAU, GNB2L1, PLEKHA6, CTSB, FERMT1, CRMP1, VEGFB, VEGFA, and PLEKHA1.

In one variation, the expression levels of at least two biomarkers are measured, wherein the first biomarker is PLAU and the second biomarker is selected from the group consisting of TIE-1, TIE-2, TIMP-4, FGF1, TGFBR2, CSF1, TGFα, TGFβ1, IL17D, SDF2, TGFBRAP1, FGF11, FGF14, IL1β, IL-11, IL-32, IL-6, IL1RN, IL-20RB, IL-21R , GNB2L1, PLEKHA6, CTSB, FERMT1, CRMP1, VEGFB, VEGFA, and PLEKHA1.

In one variation, the expression levels of at least two biomarkers are measured, wherein the first biomarker is GNB2L1 and the second biomarker is selected from the group consisting of TIE-1, TIE-2, TIMP-4, FGF1, LIF, CSF1, TGFα, TGFβ1, IL17D, SDF2, TGFBRAP1, FGF11, TNFSF13B, IL1β, IL-11, IL-32, IL-6, IL1RN, IL-20RB, IL-21R , PLAU, PLEKHA6, CTSB, FERMT1, CRMP1, VEGFB, VEGFA, and PLEKHA1.

In one variation, the expression levels of at least two biomarkers are measured, wherein the first biomarker is PLEKHA6and the second biomarker is selected from the group consisting of TIE-1, TIE-2, TIMP-4, FGF1, LIF, TGFBR2, TGFα, TGFβ1, IL17D, SDF2, TGFBRAP1, FGF11, TNFSF13B, FGF14, IL-11, IL-32, IL-6, IL1RN, IL-20RB, IL-21R , PLAU, GNB2L1, CTSB, FERMT1, CRMP1, VEGFB, VEGFA, and PLEKHA1.

In one variation, the expression levels of at least two biomarkers are measured, wherein the first biomarker is CTSB and the second biomarker is selected from the group consisting of TIE-1, TIMP-4, FGF1, LIF, TGFBR2, CSF1, TGFα, TGFβ1, SDF2, TGFBRAP1, FGF11, TNFSF13B, FGF14, IL1β, IL-11, IL-32, IL1RN, IL-20RB, IL-21R , GNB2L1, PLEKHA6, FERMT1, CRMP1, VEGFB, VEGFA, and PLEKHA1.

In one variation, the expression levels of at least two biomarkers are measured, wherein the first biomarker is FERMT1 and the second biomarker is selected from the group consisting of TIE-1, TIE-2, TIMP-4, FGF1, LIF, TGFBR2, CSF1, TGFα, TGFβ1, IL17D, TGFBRAP1, FGF11, TNFSF13B, FGF14, IL1β, IL-11, IL-32, IL-6, IL-20RB, IL-21R , PLAU, GNB2L1, PLEKHA6, CTSB, CRMP1, VEGFB, VEGFA, and PLEKHA1.

In one variation, the expression levels of at least two biomarkers are measured, wherein the first biomarker is CRMP1 and the second biomarker is selected from the group consisting of TIE-1, TIE-2, FGF1, LIF, TGFBR2, CSF1, TGFα, TGFβ1, IL17D, SDF2, FGF11, TNFSF13B, FGF14, IL1β, IL-11, IL-32, IL-6, IL1RN, IL-21R , PLAU, GNB2L1, PLEKHA6, CTSB, FERMT1, VEGFB, VEGFA, and PLEKHA1.

In one variation, the expression levels of at least two biomarkers are measured, wherein the first biomarker is VEGFB and the second biomarker is selected from the group consisting of TIE-1, TIE-2, TIMP-4, LIF, TGFBR2, CSF1, TGFα, TGFβ1, IL17D, SDF2, TGFBRAP1, TNFSF13B, FGF14, IL1β, IL-11, IL-32, IL-6, IL1RN, IL-20RB, PLAU, GNB2L1, PLEKHA6, CTSB, FERMT1, CRMP1, VEGFA, and PLEKHA1.

In one variation, the expression levels of at least two biomarkers are measured, wherein the first biomarker is VEGFA and the second biomarker is selected from the group consisting of TIE-1, TIE-2, TIMP-4, FGF1, TGFBR2, CSF1, TGFα, TGFβ1, IL17D, SDF2, TGFBRAP1, FGF11, FGF14, IL1β, IL-11, IL-32, IL-6, IL1RN, IL-20RB, IL-21R , GNB2L1, PLEKHA6, CTSB, FERMT1, CRMP1, VEGFB and PLEKHA1.

In one variation, the expression levels of at least two biomarkers are measured, wherein the first biomarker is PLEKHA1the second biomarker is selected from the group consisting of TIE-1, TIE-2, TIMP-4, FGF1, LIF, CSF1, TGFα, TGFβ1, IL17D, SDF2, TGFBRAP1, FGF11, TNFSF13B, IL1β, IL-11, IL-32, IL-6, IL1RN, IL-20RB, IL-21R , PLAU, PLEKHA6, CTSB, FERMT1, CRMP1, VEGFB, and VEGFA.

In other variations provided herein, of the at least two biomarkers measured, at least one biomarker is a factor leading to vascular development, including factors involved in vascular development and remodeling related to angiogenesis such as TIE-1, TIE-2, TIMP-4, VEGFA, VEGFB, FBLN5, FBLN7, PGF, ANGPT1, ANGPT2, ANGPTL2, ANGPTL6, TNFSF12, PRKCA, PIK3CA, and ESM1.

In some variations provided herein, of the at least two biomarkers measured, at least one biomarker is an anti-inflammatory or immunomodulatory factor such as CD99,CERCAM,HIVEP1,PTGER1,IL-32,ITFG1,ITGA V, HIVEP2, CSF1R, TNFSF13, IRAK3, MYL9, NOS3, IL12A, TNFRSF21, IRAK1, IL33, LRRC8A, CLEC11A, CCL28, ESM1, CMIP, TNFRSF25, CHST3, CD72, CD320, CD83, IL6, CD68, CD99, IL-16, or ILF3. The method may further comprise determining if KIF 14, CCL2, ILF2, IL7R, PEAR 1, or IL 16 expression levels decrease.

In some variations provided herein, of the at least two biomarkers measured, at least one biomarker is a transforming growth factors (TGFα, TGFβ1 or TGFβ2), transforming growth factor beta receptor TGFBR2, or transforming growth factor beta receptor-associated protein TGFBRAP1.

In some variations provided herein, of the at least two biomarkers measured, at least one biomarker is a cell motility, migration and invasive growth promoting factor such as TIE-1, TIE-2, TIMP-4, CGNL1, CGREF1, CRMP1, FGD6, TNK2, PTGS1, TNFAIP8, CTSB, CTSO, FAP, FERMT1, PLEKHA1, PLEKHA6, ROCK1, or ROCK2.

In some variations provided herein, of the at least two biomarkers measured, at least one biomarker is an anti-apoptotic factor, for example VEGFA, VEGFB, PLEKHA1, PLEKHA6, CRMP1, FERMY1, CTSB, TGFB1, or GNB2L1.

In some variations provided herein, the decrease in expression levels of CHD24, CYR61, ILK, NEDD9, MYL9, PPAP2B, RELN , ICAM 2, ICAM3 and TLN2 are additionally monitored as these are factors that promote cell adhesion.

In some variations, the decrease in expression levels of SIVA1 is additionally measured.

In some variations provided herein, of the at least two biomarkers measured, at least one biomarker is an interleukin, for example IL1b, IL3, IL5, IL6, IL9, IL10, IL12b, IL18-binding protein-α, IL9, IL-11, IL12a, IL12b, IL-4, or IL-16.

In some variations provided herein, of the at least two biomarkers measured, at least one biomarker is an interleukin receptor, for example IL1Rα, IL1R4, IL10Rβ, IL18Rβ, IL1R2, IL-21R, IL-2Rβ, IL-2Ry, IL5Rα, IL1R1, IL1R2, or IL1R4.

In some variations provided herein, of the at least two biomarkers measured, at least one biomarker is a chemokine, for example CCL8, CCL13, CCL15, CCL17, CCL18, CCL20, CCL22, CCL24, CCL26, CXCL9, CXCL11, CCL2, CCL4, CCL5, CCL23, CCL25, CCL27, CXCL10, CCL23, CXCL16, or CCL27.

In some variations provided herein, of the at least two biomarkers measured, at least one biomarker is a growth factor, hormone or growth factor receptor for example FGF6, IGF1, IGF2, LAP, NT3, PDGFAA, PDGFAB, SCF, TGF2, TGFα, TGFβ1, TGFb3, TNFβ, PDGFRα, PDGFRβ, VEGF, VEGFD, VEGFR, FGF4, FGF9, HGF like, IGFBP 6, PDGFBβ, IFNγ, SDF1A, DR6, ENDOGLIN , ERBB3, FAS LG, GDNF, GITR LG, LEPR, SCFR, SIGLEC 5, TIE-1&2, BDNF, BMP4, FGF7, IGFBP2, DR6, ANG, CNTF, EGF, EOTAXIN 1, NGFR, ACRP30, AGRP, ANGPT2, LEP, NT4, HGF, PRL, SCFR, FAS LG, IGFBP 1, OR IGFBP 2.

In one variation, the at least two biomarkers are selected from a panel of biomarkers comprising TIE-1, TIE-2, TIMP-4, FGF1, LIF, TGFBR2, CSF1, TGFα, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the method comprises measuring the expression levels of at least 2, least 3, at least 4, at least 5, at least 6, at least 7, at least 8, at least 9, at least 10, at least 11, at least 12, at least 13, at least 14, at least 15, at least 16, at least 17, at least 18, at least 19, or even at least 20 biomarkers selected from TIE-1, TIE-2, TIMP-4, FGF1, LIF, TGFBR2, CSF1, TGFα, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1.

In one variation, the levels of TIE-1, TIE-2, TIMP-4, FGF1, LIF, TGFBR2, CSF1, TGFα, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1 are measured.

In one variation, the method comprises measuring the expression levels of at least 2, at least 3, at least 4, at least 5, at least 6, at least 7, or even at least 8 biomarkers selected from VEGFA, PLEKHA1, VEGFB, CRMP1, FERMT1, CTSB, PLEKHA6, GNB2L1, and TGFβ1.

In one variation, the method comprises measuring the expression levels VEGFA, PLEKHA1, VEGFB, CRMP1, FERMT1, CTSB, PLEKHA6, GNB2L1, and TGFβ1.

In some variations, the method further comprises measuring the level of SIVA1. SIVA1 is one of the few biomarkers observed to decrease in SEN-MSCs, when exposed to IL-2.

In some variations, the expression of at least two biomarkers selected from a panel of biomarkers comprising TIE-1, TIE-2, TIMP-4, FGF1, LIF, TGFBR2, CSF1, TGFα, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1 is measured, and the expression level of SIVA1 is measured. If it is determined that the expression level of the at least two biomarkers is increased, relative to reference levels, and the expression level of SIVA1 is decreased, relative to reference levels, then it is determined that either (1) the individual has an increased likelihood of experiencing adverse events associated with the IL-2-based therapy; or (2) the population of MSCs is not suitable for use in transplantation.

In some variations, the expression of at least two biomarkers selected from a panel of biomarkers comprising TIE-1, TIE-2, TIMP-4, FGF1, LIF, TGFBR2, CSF1, TGFα, TGFβ1, IL17D, SDF2, TGFBRAP1, FGF11, TNFSF13B, FGF14, IL1β, IL-11, IL-32, IL-6, IL1RN, IL-20RB, IL-21R , PLAU, GNB2L1, PLEKHA6, CTSB, FERMT1, CRMP1, VEGFB, VEGFA, and PLEKHA1 is measured, and the expression level of SIVA1 is measured. If it is determined that the expression level of the at least two biomarkers is decreased, or exhibits no change, relative to reference levels, and the expression level of SIVA1 is increased, relative to reference levels, then it is determined that either (1) the individual has an increased likelihood of experiencing adverse events associated with the IL-2-based therapy; or (2) the population of MSCs is not suitable for use in transplantation.

In some variations, the expression of at least two biomarkers selected from a panel of biomarkers comprising VEGFA, PLEKHA1, VEGFB, CRMP1, FERMT1, CTSB, PLEKHA6, GNB2L1, and TGFβ1 is measured, and the expression level of SIVA1 is measured. If it is determined that the expression level of the at least two biomarkers is increased, relative to reference levels, and the expression level of SIVA1 is decreased, relative to reference levels, then it is determined that either (1) the individual has an increased likelihood of experiencing adverse events associated with the IL-2-based therapy; or (2) the population of MSCs is not suitable for use in transplantation.

In some variations, the expression of at least two biomarkers selected from a panel of biomarkers comprising TIE-1, TIE-2, TIMP-4, VEGFA, PLEKHA1, VEGFB, CRMP1, FERMT1, CTSB, PLEKHA6, GNB2L1, and TGFβ1 is measured, and the expression level of SIVA1 is measured. If it is determined that the expression level of the at least two biomarkers is decreased, or exhibits no change, relative to reference levels, and the expression level of SIVA1 is increased, relative to reference levels, then it is determined that either (1) the individual has an increased likelihood of experiencing adverse events associated with the IL-2-based therapy; or (2) the population of MSCs is not suitable for use in transplantation.

In a particular variation of the disclosure, the RNA expression level of the TGFβ1 biomarker is decreased by about at 0.25-fold in the sample upon IL-2 treatment, as compared to a reference level (sample not treated with IL-2). The fold can be raw fold or a GFOLD value.

In a particular variation of the disclosure, the RNA expression level of the SIVA1 biomarker is decreased by about at 0.2-fold in the sample upon IL-2 treatment, as compared to a reference level (sample not treated with IL-2). The fold can be raw fold or a GFOLD value.

In a particular variation of the disclosure, the RNA expression level of the CRMP1 biomarker is increased by about at least 0.22-fold in the sample upon IL-2 treatment, as compared to a reference level (sample not treated with IL-2). The fold can be raw fold or a GFOLD value.

In a particular variation of the disclosure, the RNA expression level of the VEGFB biomarker is increased by about at least 0.19-fold in the sample upon IL-2 treatment, as compared to a reference level (sample not treated with IL-2). The fold can be raw fold or a GFOLD value.

In a particular variation of the disclosure, the RNA expression level of the VEGFA biomarker is increased by about at least 1.5-fold or about 1.78-fold in the sample upon IL-2 treatment, as compared to a reference level (sample not treated with IL-2). The fold can be raw fold or a GFOLD value.

In a particular variation of the disclosure, the RNA expression level of the PLEKHA1 biomarker is increased by about at least 0.46-fold in the sample upon IL-2 treatment, as compared to a reference level (sample not treated with IL-2). The fold can be raw fold or a GFOLD value.

In a particular variation of the disclosure, the RNA expression level of the CTSB biomarker is increased by about at least .25-fold in the sample upon IL-2 treatment, as compared to a reference level (sample not treated with IL-2). The fold can be raw fold or a GFOLD value.

In a particular variation of the disclosure, the RNA expression level of TGFβ1 is decreased by about -0.25 GFOLD and SIVA1 is decreased by about -0.2 GFOLD upon IL-2 treatment, and the levels of CRMP1 is increased by about 0.22 GFOLD, VEGFB is increased by about 0.19 GFOLD, VEGFA is increased by about 1.78 GFOLD, PLEKHA1 is increased by about 0.459 GFOLD, CTSB is increased by about 0.25 GFOLD under the same conditions.

In a particular variation of the disclosure, the protein expression level of the TIE-1 biomarker is increased by about at least 2-fold, at least 3-fold, or about 3.73-fold in the sample upon IL-2 treatment, as compared to a reference level (sample not treated with IL-2).

In a particular variation of the disclosure, the protein expression level of the TIE-2 biomarker is increased by about at least 2-fold, at least 3-fold, at least 4-fold, at least 5-fold or about 5.24-fold in the sample upon IL-2 treatment, as compared to a reference level (sample not treated with IL-2).

In a particular variation of the disclosure, the protein expression level of the TIMP-4 biomarker is increased by about at least 2-fold, at least 3-fold, at least 4-fold, or about 4.31-fold in the sample upon IL-2 treatment, as compared to a reference level (sample not treated with IL-2).

In a particular variation of the disclosure, the protein expression level of the IL-11 biomarker is increased by about at least 1.1-fold, at least 1.2-fold, at least 1.3-fold, at least 1.4, or about 1.42-fold in the sample upon IL-2 treatment, as compared to a reference level (sample not treated with IL-2).

In a particular variation of the disclosure, the protein expression level of the IL1β biomarker is increased by about at least 1.1-fold, or about 1.14-fold in the sample upon IL-2 treatment, as compared to a reference level (sample not treated with IL-2).

In a particular variation of the disclosure, the protein expression level of the TGFα biomarker is increased by about at least 1.5-fold, at least 2-fold, or about 2.4-fold in the sample upon IL-2 treatment, as compared to a reference level (sample not treated with IL-2).

In a particular variation of the disclosure, the protein expression level of the TGFβ1 biomarker is increased by about at least 1.1-fold, at least 1.2-fold, a least 1.3 fold, at least 1.4 fold, or about 1.44-fold in the sample upon IL-2 treatment, as compared to a reference level (sample not treated with IL-2).

In a particular variation of the disclosure, in a sample, the protein expression level of the TIE-1 biomarker is increased by about 3.73-fold , the protein expression level of TIE-2 is increased by about 5.24 times , the protein expression level of TIMP-4 is increased by about 4.31 times, the protein expression level of IL-11 is increased by about 1.42 times, the protein expression level of IL1β is increased by about 1.14 times, the protein expression level of TGFα is increased by about 2.4 times, TGFβ1 is increased by about 1.44 times , as compared to reference levels.

### 5. Companion Methods for IL-2 Therapeutics

As contemplated herein, administration of IL-2 influences the secretory properties MSCs and IL-2 treatment may lead to potential adverse outcomes in certain individuals. Described herein are diagnostic kits, assays and methods that may be used to determine whether an individual eligible to receive an IL-2-based therapy may experience an adverse event associated with the IL-2-based therapy, based on the characterization of production of certain biomarkers (as described above). Also described herein are methods of treating a patient with IL-2.

As used herein, potential adverse events associated with an IL-2-based therapy are associated with cellular senescence, and include events such as angiogenesis, tumorigenesis, vascular development, invasive growth, metastasis, cell motility, migration, and the like. The invention allows the determination of whether an individual may experience, will experience, or is likely to experience an adverse event associated with an IL-2-based therapy. In one variation, the disclosure allows determination of an increased likelihood of experiencing an adverse event by about 10%, 20%, 30%, 40%, 50%, 60%, 70%, 80%, 90%, or even about 100%.

In one variation, the individual has received at least one dose of an IL-2-based therapy before the method is carried out on a sample from the individual. In this variation, the method for determining whether an individual eligible to receive an IL-2-based therapy may experience an adverse event associated with the IL-2-based therapy comprises: measuring the expression levels of at least two biomarkers selected from a panel of biomarkers indicative of MSC senescence described herein (the panel of biomarkers are anti-apoptotic, angiogenic, tumorigenic, lead to vascular development, responsible for invasive growth, metastasis, cell motility, migration and the like) in a sample from the individual, wherein the individual has received at least one dose of an IL-2-based therapy; and comparing the levels of the biomarkers to reference levels, wherein an increase in the levels above the reference levels indicates that the individual may experience an adverse event associated with the IL-2-based therapy, and wherein a decrease or no change in the levels compared to the references levels indicates that the individual may not experience an adverse event associated with the IL-2-based therapy.

In another variation, a sample from the individual is obtained, and exposed to (incubated with, combined with, etc) IL-2 *in vitro.* In this variation, the method for determining whether an individual eligible to receive an IL-2-based therapy may experience an adverse event associated with the IL-2-based therapy comprises: measuring the expression levels of at least two biomarkers selected from a panel of biomarkers indicative of MSC senescence in a sample from the individual, wherein the individual has received at least one dose of an IL-2-based therapy; and comparing the levels of the biomarkers to reference levels, wherein an increase in the levels above the reference levels indicates that the individual may experience an adverse event associated with the IL-2-based therapy, and wherein a decrease or no change in the levels compared to the references levels indicates that the individual may not experience an adverse event associated with the IL-2-based therapy.

In another variation, the method is directed at treating an individual for cancer with an IL-2-based therapy, the method comprising administering an effective amount of the IL-2-based therapy to the individual when the expression levels of at least two biomarkers is increased in comparison to reference levels in a sample from the individual wherein either (1) the individual has received at least one dose of an IL-2-based therapy or (2) the sample is combined with IL-2 *in vitro,* and wherein the panel of biomarkers comprises biomarkers for cellular senescence.

The biomarkers, if present in elevated levels, are indicative of the presence of SEN MSCs, and indicate a likelihood that further treatment with IL-2 could lead to adverse effects such as the promotion of tumorigenesis, invasion, or metastasis in the individual. Based on this, it can be determined that the individual should not receive an IL-2-based therapy. Alternatively, if upon practice of these methods, it is determined that use of an IL-2-based therapy would likely not lead to adverse effects (e.g. likely to not promote tumorigenesis, invasion, or metastasis), based on the expression of a particular set of biomarkers, then it could be determined that it is acceptable to administer an IL-2-based therapy, and optionally would be administered the IL-2-based therapy.

As used herein, an "IL-2-based therapy" is one that involves administration of IL-2 alone, or IL-2 in combination with another agent. Administration of IL-2 contemplates administration of an active portion of IL-2, either alone or fused to other motifs. An IL-2-based therapy can be administered by any form of injection, including intravenous (IV), intramuscular (IM), or transdermal or subcutaneous (SC) injection; by an oral or nasal route; or by topical administration (cream, droplets, etc.). In a particular variation of the disclosure, IL-2 is used as a sustained-release formulation, or a formulation that is administered using a sustained release device. Such devices are well known in the art, and include, for example, transdermal patches, and miniature implantable pumps that can provide for drug delivery over time in a continuous, steady-state fashion at a variety of doses to achieve a sustained-release effect. Sublingual or eye drop formulations may also be contemplated.

As provided herein, the sample used for the expression analysis can be any sample, including, but limited to a tissue, biopsy, blood, plasma, serum, urine, saliva, CSF, stool, lymph, semen, and sweat. In particular variations, the sample is a blood, plasma, or serum sample; in an exemplary variation the sample is blood. Without being bound to any theory, because MSCs are positioned in perivascular compartments, the factors produced by MSCs in response to the IL-2 administration may be systemic/circulatory, and could be readily measurable in a blood sample.

In the variations provided herein, the expression levels of the sample can be measured at any time following the administration of the IL-2-based therapy to the individual or at any time following *in vitro* mixing of the IL-2 (mixing of an active portion of IL-2, either alone or fused to other motifs with the sample), for example, 15 minutes, 30 minutes, 1 hour, 2, 3, 4, 6, 12, 15, 24, 36, 48, 72, or even 96 hours afterwards.

In some variations, an individual is administered multiple doses of the IL-2-based therapy prior to measurement. In some variations the individual is administered a single dose of the IL-2-based therapy prior to measurement. Typically the dose of IL-2 depends on the specific IL-2 product selection. Approximately 0.001 to 0.1 mg/kg of patient body weight can be administered; in some variations, about 0.005, 0.01, 0.05 mg/kg may be administered. In some variations, 600,000 IU/kg is administered (IU can be determined by a lymphocyte proliferation bioassay and is expressed in International Units (IU) as established by the World Health Organization 1st International Standard for human IL-2. In some variations, IL-2 doses ranges from 0.01 MIU/day to 3.0 MIU/day/patient (MIU are millions international unit). IL-2-based therapies can be administered one from one or more times per day to one or more times per week; including once every other day. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of IL-2 can include a single treatment or, can include a series of treatments. In one variation, the compositions are administered every 8 hours for five days, followed by a rest period of 2 to 14 days, e.g., 9 days, followed by an additional five days of administration every 8 hours.

The companion methods for IL-2 treatment provided herein may be particularly suited as a companion method for cancer, given the widespread use of IL-2-based therapeutics for cancer treatment. However, the methods described are not limited for cancer therapeutics, and are applicable to any disease for which an IL-2 is an approved or candidate treatment. In some variations, however, the individual has received the IL-2-based therapy for the treatment of a cancer. In some variations the cancer is selected from among renal cell carcinoma, melanoma, breast cancer, pancreatic cancer, ovarian cancer, colon cancer, lung cancer, non-small cell lung cancer, in situ carcinoma (ISC), squamous cell carcinoma (SCC), thyroid cancer, cervical cancer, uterine cancer, prostate cancer, testicular cancer, brain cancer, bladder cancer, stomach cancer, hepatoma, melanoma, glioma, retinoblastoma, mesothelioma, myeloma, lymphoma, and leukemia. In some examples, the cancer is a late-stage cancer, a metastatic cancer, or a relapsed cancer. In other variations, the IL-2 treatment is for any condition associated to or caused by an undesirable immune response, for example for inflammatory, immune-related or autoimmune diseases, including without limitation HCV-related vasculitis, uveitis, myositis, type I diabetes, systemic lupus erythematous, systemic vasculitis, psoriasis, allergy, asthma, Crohn's disease, Multiple Sclerosis, Rheumatoid Arthritis, atherosclerosis, autoimmune thyroid disease, neuro-degenerative diseases, Alzheimer's disease, graft-versus- host disease, spontaneous abortion and allograft rejection.

Where it is determined that the individual is suitable to receive an IL-2-based therapy, the treatment may be altered based on the determined levels of detected biomarkers in the sample, relative to the reference sample. The methods can be used to modify the treatment regimen (e.g. escalating the dosage or dosing schedule), based on the determined levels of biomarkers in the sample relative to the reference sample.

In a particular variation, if it is determined that the individual will likely not (e.g. may not) experience adverse effects from an IL-2-based therapy, and is then administered the IL-2-based therapy, the individual may subsequently be tested again to monitor for adverse effects. Thus, in this variation, an individual is tested for the likelihood of potential adverse effects both before treatment and once treatment has commenced. The dose and schedule of administration can be adjusted accordingly. For example, low-dosage regimens for IL-2 treatments may be clinically implemented. In addition to the companion methods described herein, the IL-2 treatment effect can be monitored by additional measurements, for example effects of IL-2-based therapies on the differentiation of T-cells *in vivo.*

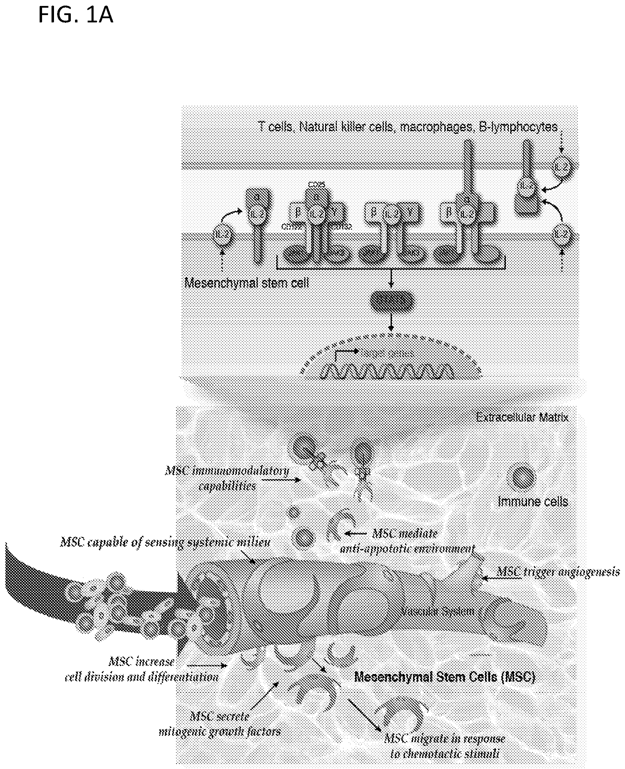

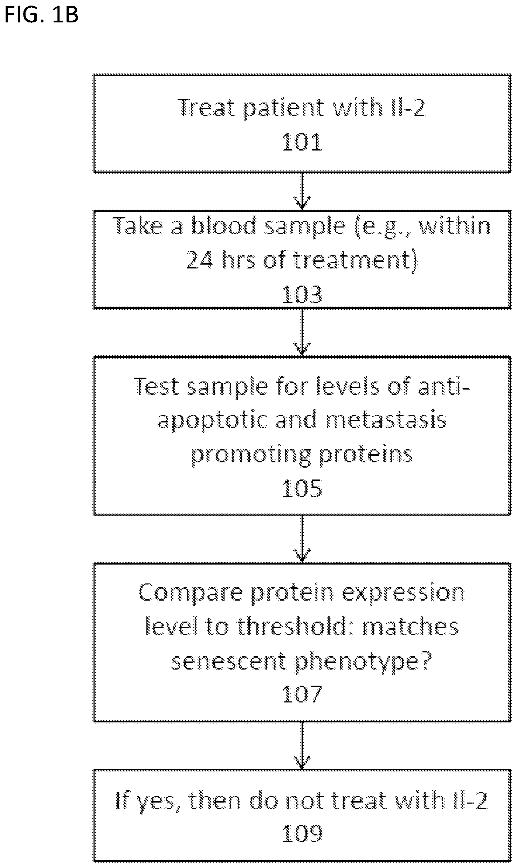

FIG. 1B illustrates an exemplary method of treating a patient with IL-2 by querying the replicative senescence status of MSC. In this example, the patient is treated with one or more doses of IL-2 101, and within a predetermined period of time (e.g., between a start time of 1 hour, 2 hours, 3 hours, 4 hours, 5 hours, 6 hours, 7 hours, 8 hours, 9 hours, 10 hours, 11 hours, 12 hours, etc., and a stop time of 12 hours, 13 hours, 14 hours, 15 hours, 16 hours, 17 hours, 18 hours, 19 hours, 20 hours, 21 hours, 22 hours, 23 hours, 24 hours, 30 hours, 36 hours, 42 hours, 48 hours, 72 hours, or 96 hours etc.), taking a sample from the patient 103. The sample may be any appropriate fluid or tissue as provided herein (e.g., blood, blood plasma, blood serum, urine, etc.). This sample may then be examined to detect the presence/absence and/or level of a panel of biomarkers 105.

**6. Quality Control of MSCs Prior to Use in Cell-Based Therapies**

MSCs are administered (transplanted) for a variety of indications. An important aspect of the disclosure is the determination of which MSCs would be suitable for administration, prior to the administration. Specifically, the methods provided allow for the selection of populations of MSCs suitable for administration, upon testing, in order to avoid or reduce the likelihood of anti-apoptotic, angiogenic, and tumorigenic activities associated with the transplanted MSCs in the *in vivo* environment that may be proinflammatory or otherwise not conducive.

Thus in another aspect provided herein are methods for assessing the quality and potential of stem cells in a sample. Such methods and kits are useful for helping to ensure the safety and quality of a population of MSCs before it is used in an individual.

In one variation, a method of determining whether a population of MSCs is suitable for administration into an individual for a stem cell-based therapy, comprises (a) incubating the population of MSCs with IL-2 (incubating with an active portion of IL-2, either alone or fused to other motifs); (b) measuring the expression levels in the cells of a panel of biomarkers associated with cellular senescence, wherein the panel of biomarkers are anti-apoptotic, angiogenic, tumorigenic, lead to vascular development, responsible for invasive growth, metastasis, cell motility, migration and the like; (c) comparing the levels of the biomarkers to reference levels, wherein an increase in the levels above the reference levels indicates that the stem cells are not optimal for administration into an individual and could lead to adverse effects if administered. However, no change or a decrease in the levels below the reference levels could indicate that the stem cells are suitable for administration into an individual. Generally by suitable, it is intended to convey that the cells would cause little or no adverse events, such as be tumorigenic, metastasis promoting, anti-apoptotic and the like.

In some variations, the population of cells is intended for autologous use. In other variations, the population of cells is intended for allogeneic use. In some variations, the population of cells comprise cells of a homogenous origin, e.g. from a single individual. In some variations, the populations of cells comprise cells of heterogeneous origin, e.g. composed of cells from a variety of sources.

In the variations provided herein, the expression levels of the sample can be measured at any time following the incubation of the IL-2, for example, 15 minutes, 30 minutes, 1 hour, 2, 3, 4, 6, 12, 15, 24, 36, 48, 72, or even 96 hours after incubation with IL-2.

**II. Kits and Articles of Manufacture**

The present application also provides kits for measuring the levels of biomarkers in a sample.

In one variation, the kits are for assessing the suitability of a population of MSCs for transplant.

In another variation, the kits are for determining whether an IL-2-based therapy should be administered to an individual in need thereof.

In one variation, a kit comprises reagents for measuring the RNA expression level of at least two biomarkers in a sample. For example, the kit may comprise probes or primers, e.g. Q-PCR primers, specific to at least two biomarkers selected from any of the biomarkers described herein.

In a particular variation, the kit comprises at least two reagents specific for measuring a biomarker selected from TIE-1, TIE-2, TIMP-4, FGF1, FGF11,FGF14,LIF, TGFBR2, CSF1, TGFα, TGFβ1,IL17D, SDF2, TGFBRAP1,TNFSF13B,IL1B,IL-11,IL-32, IL-6, IL1RN, IL-20RB, IL-21R, PLAU,GNB2L1,PLEKHA6, PLEKHA1,CTSB,FERMT1,CRMP1,VEGFB, and SIVA1, and VEGFA. In some variations the primer comprises a label, for example a fluorescent label. In some variations, the kit comprises at least 3, at least 4, at least 5, at least 6, at least 7, at least 8, at least 9, at least 10, at least 11, at least 12, at least 13, at least 14, at least 15, at least 16, at least 17, at least 18, at least 19, or even at least 20 reagents, e.g. probes or primers, for measuring the RNA expression level of biomarkers in a sample.

In other variations, a kit comprises reagents for measuring the protein expression level of at least two biomarkers in sample. For example, the kit may comprise antibodies specific to at least two biomarkers selected from TIE-1, TIE-2, TIMP-4, FGF1,FGF11, FGF14,LIF,TGFBR2, CSF1, TGFα, TGFβ1,IL17D, SDF2, TGFBRAP1,TNFSF13B,IL1B,IL-11,IL-32, IL-6, IL1RN, IL-20RB, IL-21R, PLAU,GNB2L1,PLEKHA6, PLEKHA1,CTSB,FERMT1,CRMP1,VEGFB, and SIVA1, and VEGFA. In some variations the antibody comprises a label, for example a fluorescent label. In some variations, the kit comprises an array or ELISA plate comprising at least 3, at least 4, at least 5, at least 6, at least 7, at least 8, at least 9, at least 10, at least 11, at least 12, at least 13, at least 14, at least 15, at least 16, at least 17, at least 18, at least 19, or even at least 20 reagents, e.g. antibodies, for measuring the protein expression level of biomarkers in a sample.

In some variations, the kits further comprise an IL-2 composition (a composition comprising full length-IL-2, or a portion of IL-2, either alone or fused to other motifs with the sample). This IL-2 composition can be used for testing a sample *in vitro* or for admiration into an individual, to practice the methods of the disclosure.

In some variations, the kit further comprises a reference standard for use in the assay (a sample to be tested along side to determine the reference levels are generated under the same conditions). In some variations, the kit further comprises a written list of values that serve as reference levels, for example reference levels from a population of healthy individuals, or individuals not treated with IL-2, that provide a reference against which the results of the assay can be checked.

In some variations as contemplated herein, the kits provide multiple assays for the measurement of expression levels of particular biomarkers that can be used in parallel, or in serial. For example a kit may comprise three assay plates, with Q-PCR primers or antibodies, one directed to the detection of growth factor biomarkers, one directed to the detection of anti-apoptotic biomarkers, and one directed to the detection of factors involved in cell motility and migration. In another variation, the kit may comprise three assay plates, useful for assaying the tested sample at three different time points. It is understood that the kit may comprise four, five, or more such plates for the purpose of serial or parallel detection of biomarkers.

The present application also provides articles of manufacture comprising any one of the kits described herein.

It is to be understood that the terminology employed herein is used for the purpose of describing particular variations only and is not intended to be limiting since the scope of the present invention will be limited only by the appended claims. The following examples are for illustrative purposes. These are intended to show certain aspects and variations of the present invention but are not intended to limit the invention in any manner.

## EXAMPLES

### Example 1: Materials and Methods

This example provides exemplary methods of the invention and provides the materials and methods subsequently used in Examples 2-10.

*Isolation, culture and characterization of MSCs*

MSCs used in this research were isolated from human adipose tissues obtained from healthy adult female donors age 32 and 49 undergoing routine liposuction procedures at the UCSD medical center, San Diego, CA. The MSC isolation protocol was approved by the local ethics committee and performed as previously described. Isolated adipose-derived stem cell lines were grown in DMEM/F12 medium (Life Technologies). In accordance with the MSC minimal definition criteria set by the International Society for Cellular Therapy, flow cytometric analysis showed that hADSCs express CD29, CD73, CD90 and CD105 but do not express CD11b, CD14, CD19, CD34, CD45, CD80, CD86 (antibodies from eBiosciense, USA). Morphological analysis showed that the cells present a fibroblast-like morphology, were plastic adherent and capable of adipogenic, chondrogenic and osteogenic differentiation under *in vitro* conditions using commercially available differentiation mediums (Invitrogen, USA). Cumulative population doublings (PD) were calculated as PD = log(N/NO) x 3.33 across the multiple passages as a function of the number of days of growth in culture, where NO is the number of cells plated in the flask and N is the number of cells harvested at this passage. hADSCs PD 4 or PD 6 SR populations and PD 41 and 45 for SEN populations were used in all experiments. Treatment with recombinant IL-2 (Peprotech, USA) was performed as described (Deenick EK, Gett AV, Hodgkin PD (2003). J Immunol 170: 4963-4972). 20U/ml of IL-2 was added to the culturing media for 24 hours at 37°C.

*Senescence—associated SA-β galactosidase assay*

The assay for monitoring the expression of pH-dependent senescence-associated (3-galactosidase activity (SA-(βGal) was performed as described in manufacturer's kit (BioVision) and previously published in Wang J, Geesman GJ, Hostikka SL, Atallah M, Blackwell B, et al. (2011) Inhibition of activated pericentromeric SINE/Alu repeat transcription in senescent human adult stem cells reinstates self-renewal. Cell cycle 10: 3016-3030. The cultured hADSCs were washed with PBS for 15 minutes at room temperature, washed with twice with PBS and stained with X-Gal containing supplement overnight at 37°C. The cells were washed twice with PBS, and the images were captured using a microscope (Nikons, TE300, DXM1200 Digital Camera, Japan).

*Migration and invasion assay*

Transwell filters were from Corning Incorporated (Acton, MA, USA) and all the cytokines in use were obtained from Peprotech Inc. (Rocky Hill, NJ, USA). The migration assay was performed as described in Perez LM, Bernal A, San Martin N, Galvez BG (2013), Arch Physiol Biochem 119: 195-201 using 8mm thick Transwell chambers. For the Transwell migration assay, $1.0 \times 10^4$ cells were suspended in 80ul of serum-free alpha-MEM and seeded in the upper chamber of 24-well Transwell plates containing 8 mm pore size filters (Corning, Costar, USA). In the lower chamber, 600 ul of DMEM or medium containing cytokines: IL-2, IL-6, IL-8, TNF-α, HMGβ1 was added. The concentrations in used were: 50ng/ml IL-2, IL-6, IL-8 and HMGβ1; 30ng/ml TNF-α as described in (Perez et al., 2013, Arch Physiol Biochem 119: 195- 201). hADSCs were incubated at 37°C for 16h. The cells retained in the upper chamber were removed by swab and those that had migrated through the filter were fixed with 4% paraformaldehyde for 20 minutes at room temperature and stained overnight with 5% toluidine blue. The cells were counted at the lower side; in five different randomly selected 10x fields using a bright-field microscope (Nikons, TE300, DXM1200 Digital Camera, Japan). These experiments were done with hADSCs of two donors age 32 and 41, ether SR or SEN populations, with each donor sampled more than three times.

*Enzyme-linked immunosorbent assays (ELISA)*

hADSCs (SR or SEN) were plated at a density of $10^5$ cells per 10 cm$^2$ dish and treated with 20 U/mL of IL-2 for 24 hour, with untreated controls as previously described in Deenick EK, Gett AV, Hodgkin PD (2003) Stochastic model of T cell proliferation: a calculus revealing IL-2 regulation of precursor frequencies, cell cycle time, and survival. J Immunol 170: 4963-4972. Then, cell membrane-associated protein fractions were prepared using Mem-PER Plus #89842 (ThermoFisher Scientific) following the manufacturer's protocol. Measurements of the concentrations of IL-2 receptors *alpha* and *beta* were obtained using human IL-2R *alpha* and human IL-2R *beta* ELISA kits #ELH-IL-2Ra and #ELH-IL-2Rb (RayBiotech, Inc) respectively. The optical densities for the standards (recombinant IL-2 receptors *alpha* and *beta*) as well as the experimental samples were measured at 450 nm by SPECTRA Max Plus (Molecular Devices) and concentrations were calculated as described in the manufacturer's protocol.

*Real-time quantitative polymerase chain reaction*

Total RNA was isolated from hADSCs using the RNeasy Mini Kit (Qiagen, Germany). cDNA was then synthesized using the RevertAid First Strand cDNA Synthesis Kit (Fermentas, USA). Real-time quantitative polymerase chain reaction (Q-PCR) was performed using TaqMan instrument. The expression levels were calculated as 2-$^{\Delta\Delta Ct}$, where relative expression was determined by normalization to beta-actin gene expression. All assays were conducted in triplicates and negative control samples without cDNA were used. Primers for the Q-PCR were as follows:

IL-2 Receptor Alpha chain (IL-2Rα) For: 5'- CTGCCACTCGGAACACAAC-3' and Rev: 5'- TGGTCCACTGGCTGCATT-3'.

IL-2 Receptor Beta chain (IL-2Rβ) For: 5'- ACTCGAGAGCCAACATCTCC-3' and Rev: 5'- TCCGAGGATCAGGTTGCAG-3'.

IL-2 Receptor Gamma 1 chain (IL-2Rγ1) For: 5'- TGGATGGGCAGAAACGCTA-3' and Rev: 5'- GGCTTCCAATGCAAACAGGA-3'.

STAT 5A For: 5'- ACGCAGGACACAGAGAATGA -3' and Rev: 5'-CTGGGCAAACTGAGCTTGG-3' .

STAT 5B For: 5'- ACACAGCTCCAGAACACGT -3' and Rev: 5'-TGTTGGCTTCTCGGACCAA-3'.

VEGF A For: 5'- GGAGGAGGGCAGAATCATCA -3' and Rev: 5'-ATCAGGGGCACACAGGATG-3'.

*Transcriptomic analysis*

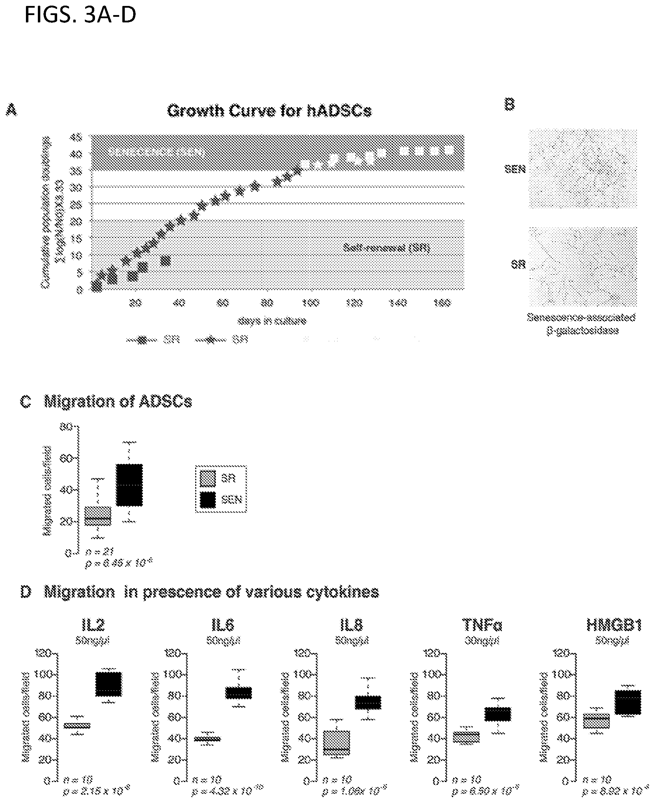

Transcriptomic analysis was performed with IL-2 treated and untreated (control group) SR and SEN hADSCs as previously described in Deenick EK, Gett AV, Hodgkin PD (2003) Stochastic model of T cell proliferation: a calculus revealing IL-2 regulation of precursor frequencies, cell cycle time, and survival. J Immunol 170: 4963-4972. The two genotypes shown in FIG. 3A were used for the analysis of four different conditions: SR or SEN cells, with or without IL-2 stimulation, respectively. The same amount ($10^6$) cells was seeded in DMEM F12 media for each experimental condition, and IL-2 treatment was performed by adding 20U/ml of recombinant IL-2 (Peprotech, USA) directly into the media for 24 hours as previously described in Deernick et al. 2003. Total RNA was isolated from samples using TRIzol reagent (Invitrogen, USA) according to the manufacturer's instructions. Samples from two different patients were combined together for the relevant conditions and RNA concentrations were measured with the Qubit 2.0 fluorimeter using the RNA HS Assay kit (Invitrogen, Life technologies, USA).

100ng of total RNA of each sample was used to construct the libraries for sequencing on the Ion Proton™ System (Life technologies, USA) following the manufacturer's instructions. Prior to rRNA depletion and RNA-seq library construction, the ERCC RNA Spike-In Control mix (Ambion, Life Technologies) was added to total RNA for quality control analysis. The ERCC RNA Spike-In control mix contains 92 transcripts 250-2000 nt in length that mimic natural eukaryotic mRNAs. According to the protocol provided by manufacturer for 100 ng of total RNA was added to 2u1 of Mixl in dilution 1:1000 of spike-in. Afterwards, rRNA depletion was performed with the Low Input Ribominus Eukaryote System v2 (Ambion, Life technologies, USA). cDNA libraries were constructed with Ion total RNA-seq kit v2 (Ambion, Life technologies, USA), and barcoded with Ion Xpress RNA-seq barcode (Ambion, Life technologies). The size distribution and quantification of the libraries were performed on a Bioanalyzer 2100 (Agilent technologies, USA). Library sequencing was performed on the Ion Proton™ System with PI chip, and each library was sequenced 3 times.

*RNA-seq data analysis*

RNA-seq reads from individual Ion Proton™ System sequencing runs were combined for each of the four conditions. Sequence reads were mapped to the reference human genome assembly hgl9 (GRCh37) using the Torrent Mapping Alignment Program (TMAP, Life technologies). The quality of the four condition-specific combined RNA-seq runs was evaluated by comparing the expected counts of ERCC spike-in RNA sequences, obtained from the manufacturer's website, against the observed counts of RNA-seq tags that map to the same sequences. Initial gene expression levels were taken as the sum of exon-mapped reads for individual NCBI RefSeq gene models (c), and lowly expressed genes (read counts per million<1) were removed from subsequent analyses. For each library, individual gene expression levels were normalized using the beta-actin *(ACTB)* expression levels *(cACTB)* and the total exon length l of each gene. For library *j*, the beta-acting normalization factor $s_j$ was calculated as

$$\frac{\frac{1}{n}\sum_{k=1}^{n} c_{ACTB,k}}{c_{ACTB,j}}$$

and the final normalized expression value for gene i in library j was calculated as

$$e_{i,j} = \frac{c_{i,j} \times s_j}{l_i}.$$

Differential gene expression analysis between pairs of libraries was performed using the program GFOLD v1.1.3, Feng J, Meyer CA, Wang Q, Liu JS, Shirley Liu X, et al. (2012) GFOLD: a generalized fold change for ranking differentially expressed genes from RNA-seq data. Bioinformatics 28: 2782-2788. GFOLD was chosen based on its demonstrated superior performance in characterizing differentially expressed genes in the absence of replicate data sets. GFOLD analysis yields a score that measures the extent of differential gene expression between conditions; the recommended GFOLD score cut-off of $\pm 0.01$ was used to define differentially expressed genes here. Functional enrichment analysis for differentially expressed genes between pairs of libraries was performed using the program GSEA v2.1.0. Specifically, individual pathways containing multiple genes that are up-regulated or down-regulated upon IL-2 treatment in SR, SEN or both were identified in this way. Individual pathways for specific sets of differentially regulated genes (IL-2+ up-regulated in SR and/or SEN and IL-2+ down-regulated in SR and/or SEN) were related using networks where the nodes correspond to pathways and the edges correspond to the presence of shared genes between pathways.

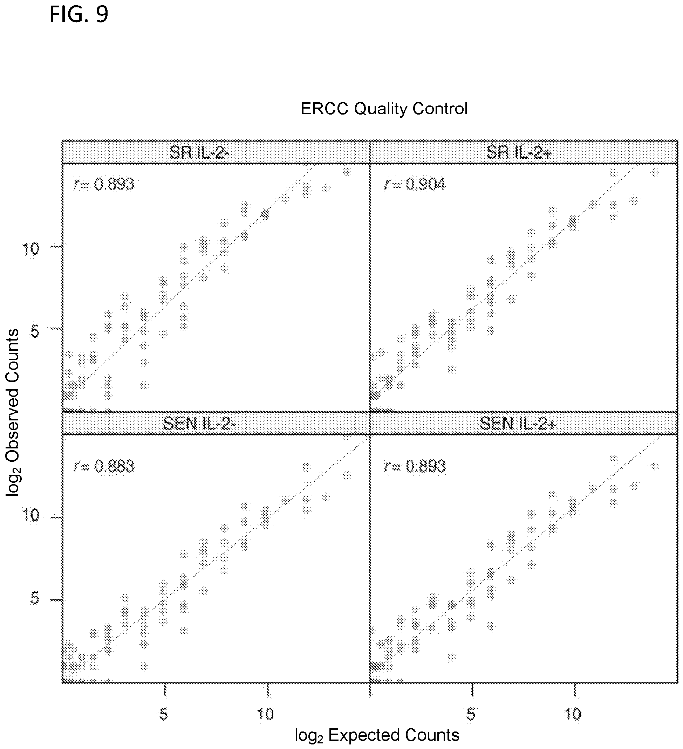

FIG. 9 shows External RNA Controls Consortium (ERCC , a common set of external RNA controls) dose response used for quality control of RNA-seq experiments. For each of the four condition-specific RNA-seq pools, the expected counts of ERCC spike-in RNA sequences were regressed against the observed counts of RNA-seq tags that map to the same sequences. Observed versus expected counts were highly correlated, as indicated by the shape of the regression and the Pearson correlation r-values, consistent with high quality RNA-seq results.

## Example 2: Characterization of the MSC senescent phenotype

Described in this example is a study that was conducted to evaluate the impact of replicative senescence on the transcriptional activity of human adipose-derived MSCs (hADSCs) in response to IL-2 signaling.

IL-2 signals via specific receptors, with three classes of cell surface receptors formed by various combinations of three IL-2R subunits: IL-2R$\alpha$ (CD25), IL-2R$\beta$ (CD 122) and IL-2Rg (CD 132). The experimental results indicate that hADSCs transcriptionally express all three receptors, however protein expression of the IL-2R$\alpha$ (IL-2Ra) in hADSCs is lower than seen for IL-2R$\beta$. These observations indicate that an IL-2 receptor composition consisting of IL-2R$\beta$ and IL-2R$\gamma$ isoforms might mediate the predominant form of IL-2 cytokine recognition by hADSCs. The receptor composition changes only slightly upon replicative aging of the hADSCs, indicating that responsiveness of hADSCs to IL-2 does not change upon their senescence.

hADSCs were isolated and cultured as described above. *Ex vivo* replicative senescence led to decreased proliferation, accumulation of DNA damage and morphological changes: hADSCs became much larger with an irregular and flat shape, and nuclei became more circumscribed in phase contrast microscopy with the granular cytoplasm appearance of many inclusions and aggradations. The growth curve of hADSCs obtained from two different patients are shown in FIG. 3A. Typical staining for senescence-associated SA-$\beta$ galactosidase activity for either hADSCs in linear growth rate, SR, or when cell lines cease their proliferation, SEN, is shown in FIG. 3B.

## Example 3: SEN-MSCs demonstrate a higher propensity for migration

This examples shows that replicative senescence affects the migratory potential of hADSCs. Migration assays were performed, using a set of cytokines and growth factors using the Transwell system as described in the Materials and Methods section, below. It was observed that adipose-derived MSCs undergoing replicative senescence demonstrated a higher propensity for migration. It was observed that SEN hADSCs showed significantly higher basal migration capacity then their SR counterparts (FIG. 3C). FIG. 3C shows *ex vivo* migration assays for self-renewing (SR, on left) and senescent (SEN, on right) hADSCs. The black lines indicate the median values, and the whiskers indicate the range of values. Statistical differences were evaluated by a T-test with the p-value (p) as depicted.

In addition, the response of SEN hADSCs to different cytokine chemo-attractants was measured. It was observed that hADSCs have an increased ability to migrate for late passages in comparison to early passages (FIG. 3D), indicating that replicative senescence increases the migratory properties of hADSCs in response to the tested chemo-attractants. IL-2 was the most potent chemo-taxis stimulant on SEN-MSCs, whereas the TNF-$\alpha$ was less potent among the tested chemo-attractants in these experiments (FIG. 3D). FIG. 3D shows the migration of self-renewing SR (on left) and senescence SEN (right) hADSCs. hADSCs were induced to migrate in the presence of different cytokines (50 ng/ml IL-2, IL-6, IL-8, HMGBI; 30 ng/ml TNF). The graphic represents the mean of ten independent experiments (n=10). P-values (p) related to experimental measurements are listed under the graphs.

These data indicated that replicative senescence modifies the migratory properties of hADSCs and may influence the response of MSCs to the inflammatory environment and influence their immunomodulation output.

## Example 4: Differential response to Il-2 stimulation in human adipose-derived MSCs upon replicative senescence

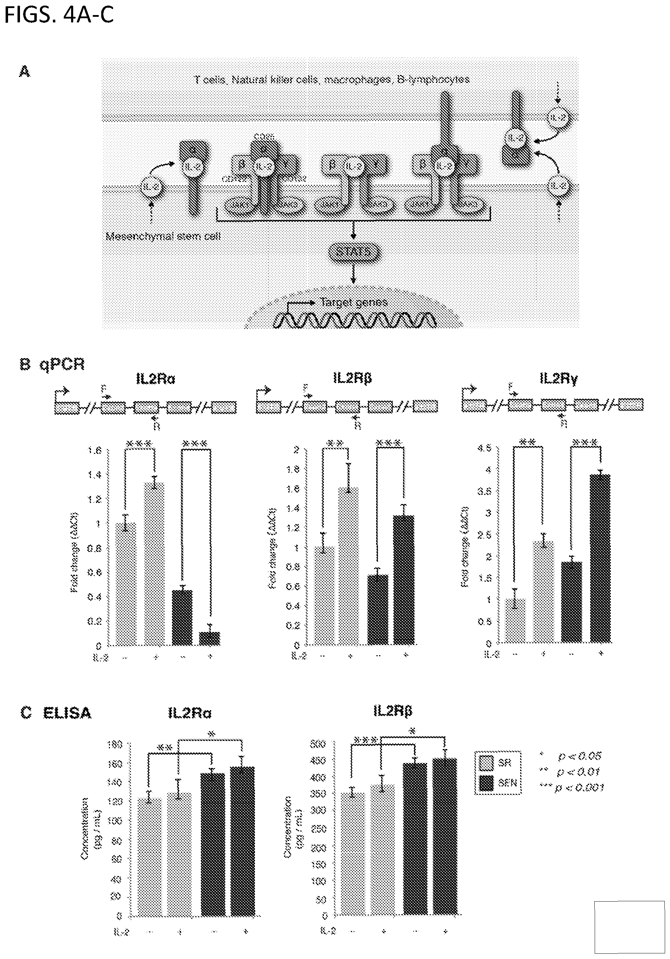

Assessment of the IL-2 receptor isoforms expression, by Q-PCR, demonstrated significant changes in expression of the IL-2R$\alpha$ isoform in comparison to IL-2R$\gamma$ and IL-2R$\beta$ upon replicative senescence *ex vivo* (FIG. 4B). FIG. 4B shows IL-2 receptors $\alpha$, $\beta$, and $\gamma$ assessed by quantitative PCR (Q-PCR) in un-stimulated (IL-2-)SR cells (first bar) and SEN cells (third bar) and upon stimulation with 20ug/ml of recombinant IL-2 (IL-2+ ) (SR cells, second bar; SEN cells, fourth bar). Data shown as fold change ($\Delta\Delta CT$). Mean$\pm$ SD from three independent experiments is shown. Notably, the increased accumulation of the IL-2R$\beta$ and IL-2Ra transcripts was recorded after IL-2 treatment in both SR and SEN hADSCs, whereas IL-2Ra expression was abrogated when senescent cells were subjected to similar treatments (FIG. 4B).

However, the data indicated that protein level expression of the cellular membrane associated IL-2R$\alpha$ receptor showed the opposite pattern (FIG. 4C). FIG. 4C shows the cellular membrane-associated levels of IL-2R$\alpha$ and IL-2R$\beta$. The levels were quantified by ELISA in un-stimulated (IL-2-) SEN (third bar) and SR (first bar) hADSCs and upon stimulation with 20ug/ml of recombinant IL-2 (IL-2+ ) SEN (fourth bar) and SR (second bar). Data are expressed as pictogram per milliliter. Results are the mean of three independent experiments (mean± SD). Statistical significance was estimated by a t-test, where ***p < 0.001, **p <0.01, *p<0.05.

Although the transcriptional status of IL-2 receptor isoforms does vary between the two different cell states (SR and SEN), it does not seem to be dependent upon IL-2 exposure (induction) as measured by the ELISA assay (described in the Materials and Methods, above). The data also demonstrated that protein encoding IL-2α receptor chain is less abundant than the IL-2Rβ isoform (compare 120 pg/ml of IL-2Rα to 350 pg/ml IL-2Rβ to 150 pg/ml of IL-2Rα and 440 pg/ml IL-2Rβ upon replicative senescence *ex vivo*) as shown in FIG. 4C. These data indicate that hADSCs response to IL-2 stimulation occurs through the intermediate-affinity receptor dimer composed of IL-2Rβ (CD 122) and the common IL-2Rγ (CD 132).

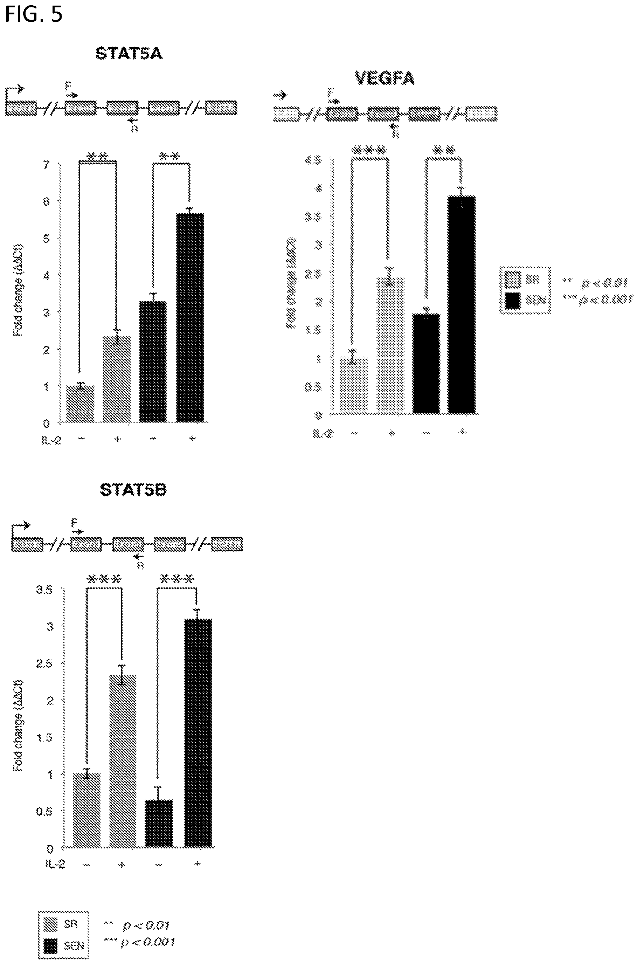

IL-2 signals via JAK1 and JAK3 to activate STAT5A and STAT5B, and additionally uses Ras-MAP kinase and phosphoinositol 3-kinase dependent signaling pathways. The expression of downstream target of IL-2, STAT5, is shown in FIGS. 5, 7A and 7B and the table of FIGS. 10A and 10B. In hADSCs, both STAT5A and STAT5B gene transcription follows the IL-2/STAT5 signaling axis.

FIG. 5 illustrates the effect of stimulation of the SR and SEN hADSCs with IL-2. IL-2 upregulates mRNA of a mediator of IL-2 signaling STAT5 gene. STAT5A and STAT5B mRNA expression was assessed by quantitative RT-PCR in un-stimulated (IL-2-) SR (first bar) and SEN cells (third bar) and upon stimulation with 20ug/ml of recombinant IL-2 (IL-2+) (SR +IL-2, second bar; SEN + IL-2, third bar). Data are shown as fold change (ΔΔCT). Mean± SD from three independent experiments is shown. The position of the Q-PCR primers is depicted graphically. Statistical significance was estimated by the t-test, where ***p < 0.001, **p<0.01.

It was next investigated how IL-2 and its downstream target STAT5 affects transcriptional outcomes in hADSCs upon their replicative senescence *ex vivo.*

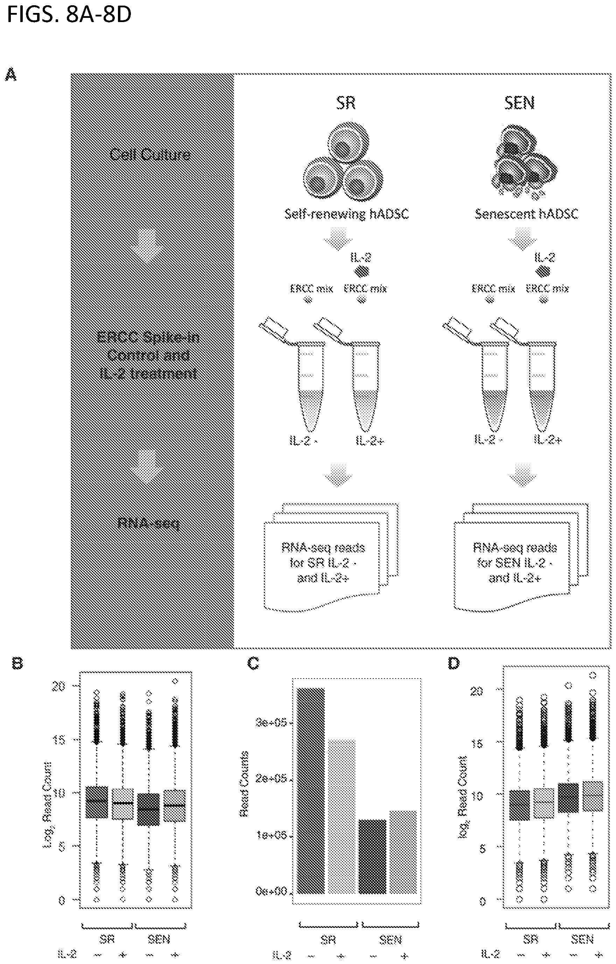

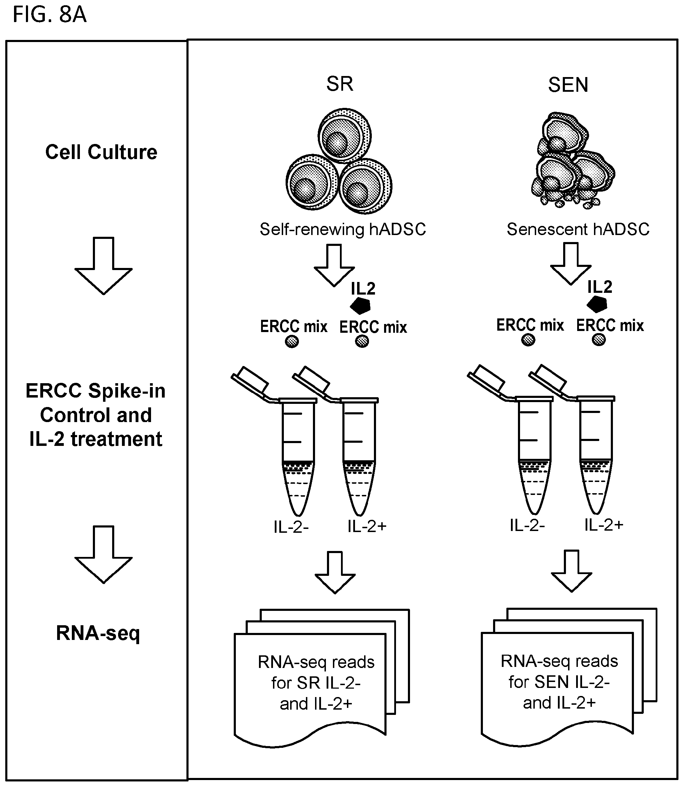

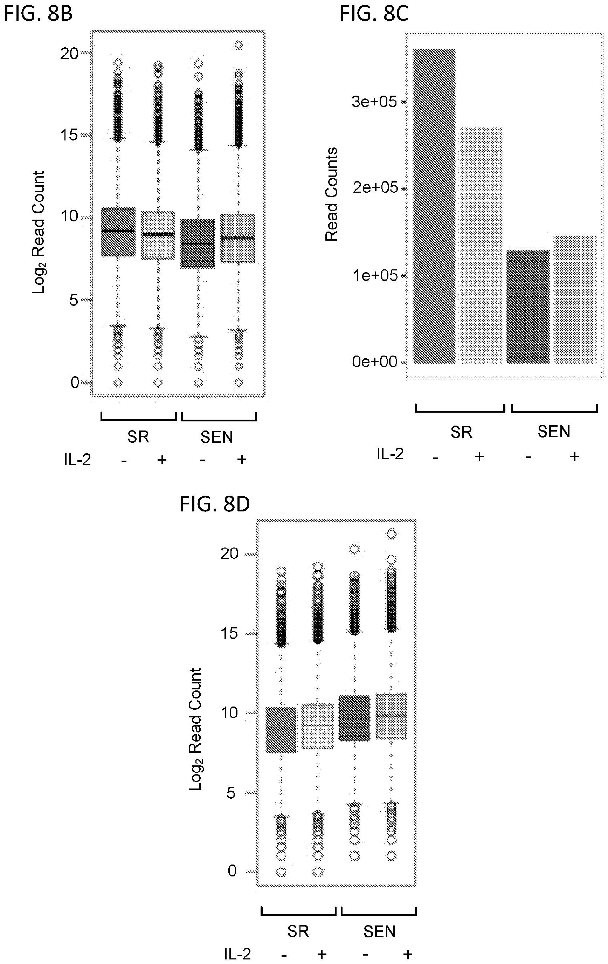

Exposure to IL-2 resulted in altered gene expression in human MCSs upon replicative senescence. To address how the transcriptional response to the IL-2/STAT5 axis changes upon replicative aging of hADSCs *ex vivo,* a RNA-seq transcriptome analysis was performed, using the Ion Proton™ System as described in Example 1 and shown in FIG. 8A. The gene expression levels in hADSCs across four conditions (libraries) was compared: self-renewal upon normal *ex vivo* culture (SR IL-2-), self-renewal upon 24 hrs recombinant IL-2 stimulation (SR IL-2+), replicative senescence upon normal *ex vivo* culture (SEN IL-2-), and replicative senescence upon 24 hrs recombinant IL-2 stimulation (SEN IL-2+). Distributions of the total read counts for the four conditions representing each condition are shown in FIG. 8B.

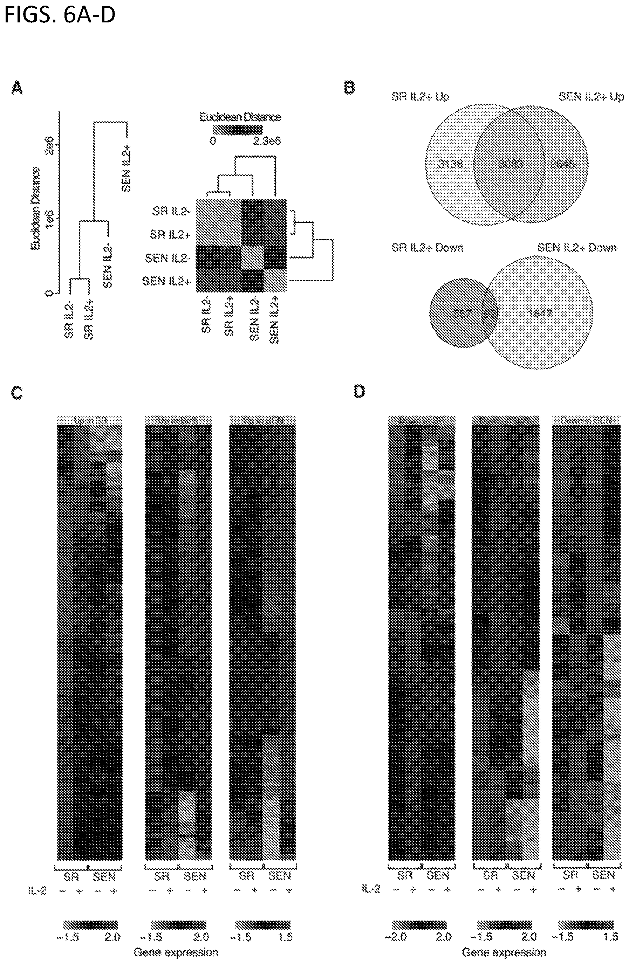

Beta-actin expression levels were used to normalize gene expression levels between conditions (as described in Example 1). This approach was taken to allow for the fact that overall gene expression levels may change upon IL-2 treatment. Beta-actin normalized gene expression distributions reveal overall up-regulation of gene expression upon IL-2 treatment in both SR and SEN states (FIG. 8D). However, comparison of individual gene expression levels among the four conditions indicates that IL-2 treatment more significantly affects SEN-compared to SR-hADSCs (FIG. 6A). FIG. 6A shows a hierarchical clustering showing the pairwise distance between conditions based on comparison of condition-specific gene expression profiles. The SR IL-2- and SR IL-2+ conditions group closely together when individual gene expression levels are compared followed by the SEN IL-2- condition. The SEN IL-2+ condition is an outlier amongst the four conditions showing a substantially divergent pattern of individual gene expression levels. This indicates that that the biological response to IL-2 treatment in hADSCs upon senescence may significantly impede MSC function via global transcriptional de-regulation in response to IL-2.

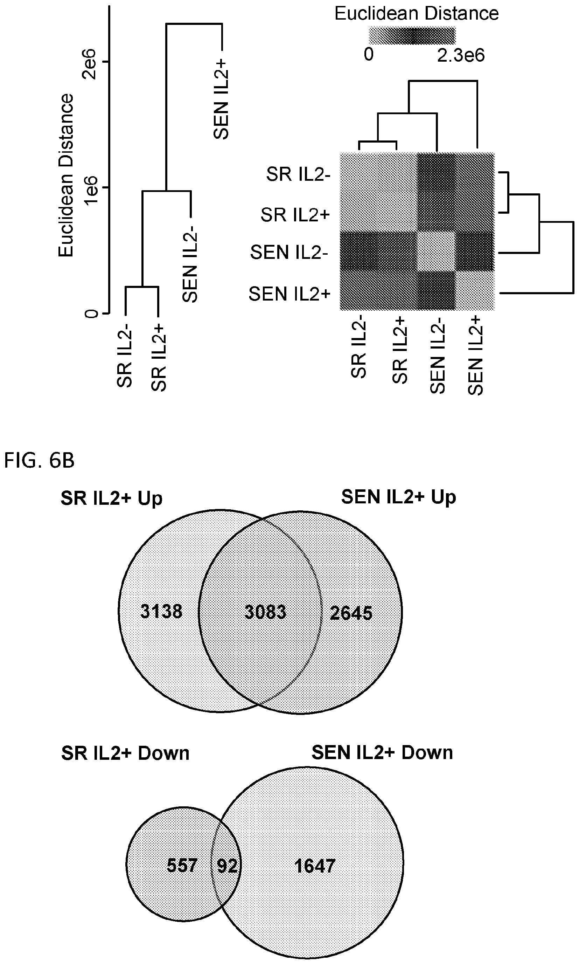

Expression levels were further compared between conditions in order to identify individual genes that are differentially expressed, up- and down-regulated, in response to IL-2 treatment in both SR and SEN states (FIG. 6B). FIG 6B is a Venn diagram showing the numbers of genes, which are up-regulated and downregulated upon IL-2 treatment. There are several more genes that are up-regulated (8,866) compared to down-regulated (2,296) upon IL-2 treatment in both SR and SEN hADSCs. There is also a substantially higher proportion of genes that are up-regulated in both SR and SEN hADSCs (35%) compared to genes that are down-regulated in both states (4%). The greatest asymmetry is seen for genes that are down regulated in SEN hADSCs upon IL-2 treatment (1,739); there are many more such genes than seen for the SR IL-2+ condition (649). This difference indicates that the overall divergence of the SEN IL-2+ condition is largely attributed to genes that are down-regulated upon IL-2 treatment, which is an unexpected result given the overall up-regulation across both SR and SEN upon IL-2 treatment (FIG. 6B and FIG. 8D).

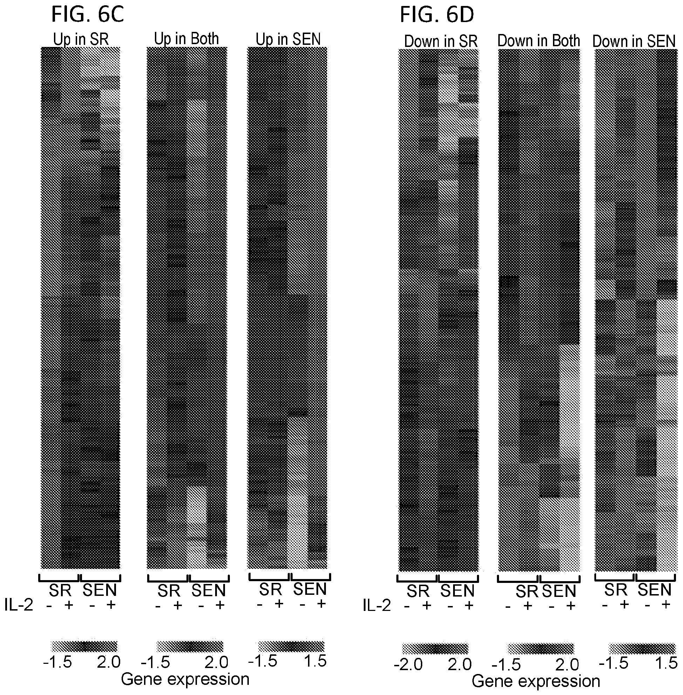

FIGS. 6C-6D shows heat maps showing the expression levels of genes that are up-regulated (FIG. 6C) and down-regulated (FIG. 6D) upon IL-2 treatment. Normalized gene expression levels are shown as heat maps in grayscale. Groups correspond to genes that are up- or down-regulated in SR-only, SEN-only or both conditions.

Taken together, these data indicate that SEN hADSCs have lost the ability to generate coordinated regulatory changes in response to IL-2 treatment to the same extent that exists for actively proliferating SR cells. The greater number of up-regulated genes seen for SR IL-2+, compared to SEN IL-2-, is consistent with this interpretation.

Provided herein are pathways enriched upon IL-treatment. FIG. 10A shows a table (FIG. 10A) indicating biological pathways enriched for genes up-regulated upon IL-2 treatment in SR and SEN hADSCs. In FIG. 10A, enriched pathways are shown along with the individual IL-2+ up-regulated genes belonging to the pathway and the pathway enrichment significance levels. Pathways with gene members up-regulated in SR are shown in the left column, and pathways with gene members up-regulated in SEN are shown in the right column. Pathways with gene members up-regulated in both SR and SEN are shown in the top row followed by pathways with gene members up-regulated only in SEN, and finally pathways with gene members up-regulated only in SR. Networks are shown relating pathways that are up-regulated in SR (left column) and pathways that are up-regulated in SEN (right column). The network nodes represent pathways, and the sizes of the nodes correspond to the number of up-regulated genes in that pathway. Pathway nodes are connected by edges if the pathways share up-regulated genes, and edge-weights correspond to the number of up-regulated genes shared between the pathways.

FIG. 10B is a table (FIG. 10B) illustrating biological pathways enriched for genes down- regulated upon IL-2 treatment in SR and SEN hADSCs. Enriched pathways are shown along with the individual IL-2+ down-regulated genes belonging to the pathway and the pathway enrichment significance levels. Pathways with gene members down-regulated in SR are shown in the left column, and pathways with gene members down-regulated in SEN are shown in the right column. Pathways with gene members down-regulated in both SR and SEN are shown in the top row followed by pathways with gene members down-regulated only in SEN, and finally pathways with gene members down-regulated only in SR. A network is shown relating pathways that are down-regulated in SEN (left column). The network nodes represent pathways, and the sizes of the nodes correspond to the number of SEN down-regulated genes in that pathway. Pathway nodes are connected by edges if the pathways share SEN down-regulated genes, and edge-weights correspond to the number of down-regulated genes shared between the pathways.

**Example 5: Trophic properties of the hADSCs after IL-2 stimulation are susceptible to replicative aging *ex-vivo***

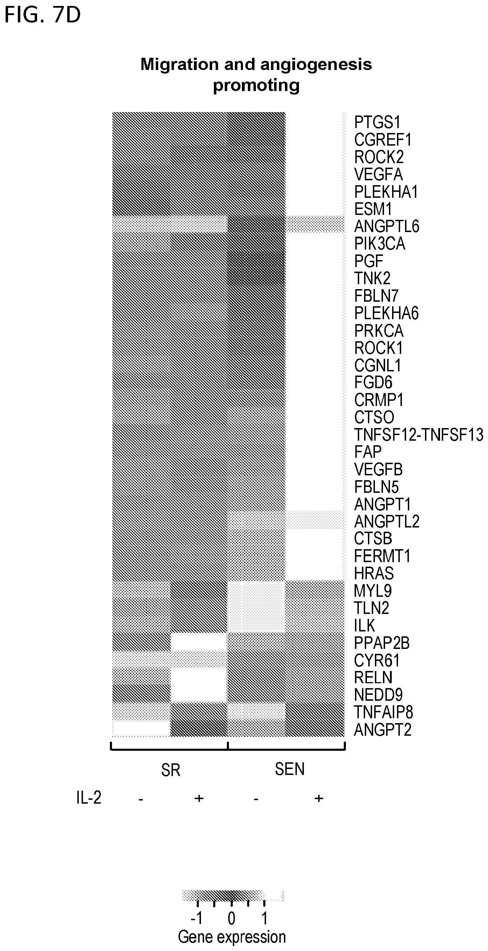

FIGS. 7A-7D illustrate gene expression levels for SR and SEN cells upon IL-2 treatment among functionally coherent sets of genes. Expression levels are shown for sets of genes characterized as (in FIG. 7 A) trophic factors, (in FIG. 7B) anti-inflammatory and immunomodulatory, (as shown in FIG. 7C) anti-apoptotic and metastasis promoting, and (as shown in FIG. 7D) migration and angiogenesis promoting. Normalized gene expression levels are shown as heat maps in grayscale.

The trophic properties of the MSCs after IL-2 exposure are susceptible to replicative aging *ex vivo* (e.g., FIG. 7A, Table 1). The secretion of a broad range of bioactive molecules is believed to be the main mechanism by which MSCs achieve their therapeutic effects. MSCs secrete an array of growth factors and anti-inflammatory proteins with complex feedback mechanisms among the many types of immune cells.

Table 1 shows the differential expression of trophic factors upon IL-2 treatment in SEN and SR cells. The SR GFOLD values represent the fold difference in SR cells treated with IL-2, relative to SR cells not treated with IL-2; the SEN GFOLD values represent the fold difference in SEN cells treated with IL-2, relative to SEN cells not treated with IL-2.

**Table 1: Differential Expression of Trophic Factors Upon IL-2 Treatment**

| Gene Symbol | Gene ID | SR IL-2- | SR IL-2+ | SEN IL-2- | SEN IL-2+ | SR GFOLD | SEN GFOLD |
|---|---|---|---|---|---|---|---|
| TGFBRAP1 | NM_001142621 | 941 | 1475 | 2242 | 2601 | 0.51 | 0.12 |
| SDF2 | NM_006923 | 685 | 992 | 1466 | 1516 | 0.37 | 0.00 |
| IL17D | NM_138284 | 9 | 49 | 69 | 124 | 0.97 | 0.34 |
| TGFα | NM_001099691 | 147 | 295 | 316 | 484 | 0.67 | 0.37 |
| CSF1 | NM_000757 | 1466 | 1691 | 3953 | 4906 | 0.09 | 0.24 |
| TGFBR2 | NM_001024847 | 3416 | 4028 | 20586 | 25244 | 0.16 | 0.26 |
| LIF | NM_002309 | 163 | 227 | 942 | 1652 | 0.13 | 0.67 |
| FGF1 | NM_001144892 | 287 | 237 | 524 | 741 | 0.00 | 0.31 |
| EGFR | NM_005228 | 4980 | 5431 | 5971 | 5368 | 0.06 | -0.09 |
| SRF | NM_003131 | 1689 | 1742 | 2132 | 1957 | 0.00 | -0.02 |
| FGF7 | NM_002009 | 756 | 766 | 1131 | 946 | 0.00 | -0.11 |

(continued)

| Gene Symbol | Gene ID | SR IL-2- | SR IL-2+ | SEN IL-2- | SEN IL-2+ | SR GFOLD | SEN GFOLD |
|---|---|---|---|---|---|---|---|
| FGF2 | NM_002006 | 5573 | 5301 | 8776 | 8185 | -0.01 | -0.05 |
| SFRP1 | NM_003012 | 1351 | 1346 | 4190 | 3575 | 0.00 | -0.15 |
| PTGES2 | NM_025072 | 521 | 746 | 1778 | 1699 | 0.33 | 0.00 |
| PDGFRA | NM_006206 | 3877 | 4533 | 5085 | 3109 | 0.15 | -0.63 |
| PDGFA | NM_002607 | 612 | 866 | 790 | 682 | 0.32 | -0.04 |
| FGF5 | NM_004464 | 4322 | 5738 | 3329 | 2895 | 0.34 | -0.12 |
| TGFB2 | NM_001135599 | 352 | 422 | 166 | 314 | 0.02 | 0.60 |
| TGFβ1 | NM_000660 | 4834 | 6297 | 3413 | 5582 | 0.32 | 0.64 |
| IL-11 | NM_000641 | 583 | 758 | 152 | 950 | 0.19 | 2.35 |
| IL1B | NM_000576 | 73 | 108 | 75 | 229 | 0.06 | 1.17 |
| FGF14 | NM_004115 | 61 | 69 | 89 | 255 | 0.00 | 1.11 |
| TNFSF13B | NM_001145645 | 0 | 12 | 10 | 104 | 0.65 | 2.12 |
| FGF11 | NM_004112 | 67 | 48 | 10 | 198 | 0.00 | 3.09 |

The data indicated that the expression of growth factors in hADSCs upon stimulation with IL-2 is subjected to significant changes upon replicative senescence *ex vivo.* While the exposure of actively proliferating (SR) hADSCs to IL-2 resulted in increased expression of mitogenic proteins such as stromal cell-derived factor 2 (SDF2) and SDFL2, and prostaglandin E synthetase 2 (PTGES2), both SR and SEN hADSCs are marked by significant increases of transforming growth factors alpha and beta (TGFα, TGFβ1 and TGFβ2), transforming growth factor beta receptor TGFBR2 and transforming growth factor beta receptor-associated protein TGFBRAP1, as well as transforming growth factor beta-induced (TGFBI), which are known to increase fibroblast, epithelial and endothelial cell division when secreted in systemic milieu (FIG. 7A and Tables 5A-5D).

In addition, both SR and SEN IL-2 stimulated hADSCs were marked by up-regulation of colony stimulating factor 1 (CSF-1), LIF, IL-11, IL-17D, IL-1β and tumor necrosis factor (ligand) superfamily TNFSF13B, a cytokine encoding gene that stimulates B- and T-cell function (FIGS. 7A, 7B and FIGS. 10A-10B).

Taking into account that paracrine IL-17D induces expression of IL-6, IL-8, and GM-CSF genes in endothelial cells, and IL-1β stimulates fibroblast growth factor activity (TGFα, TGFβ1 and TGFβ2 genes are notably up-regulated in IL-2-exposed hADSCs) in autocrine and paracrine fashion, along with thymocyte and B-cell proliferation and maturation by inducing release of IL-2 from these cells, the data indicate that the transcriptional status of both SR and SEN hADSCs may point to enhanced immunomodulatory properties of these cells after IL-2 exposure via a complex regulatory feedback loop.

Both SR and SEN hADSCs exposed to IL-2 are marked by significant increases in expression of transforming growth factors alpha and beta (TGFα, TGFβ1 and TGFβ2), transforming growth factor beta receptor TGFBR2 and transforming growth factor beta receptor-associated protein TGFBRAP1, as well as transforming growth factor beta-induced (TGFBI) genes (FIG. 7A).

Provided herein is a TGFβ biomarker. TGFβ is believed to be important in regulation of the immune system by promoting differentiation of CD+4 T-cells and inhibiting immune-surveillance, thereby imposing immunosuppression. However, the higher level of TGFβ expression in adipose-derived human MSCs after exposure to IL-2 might promote carcinogenesis. In addition, differences in the IL-2 dependent expression of growth factors upon senescence of hADSCs that have not been observed in SR cells were also noted. This includes up-regulation of a subset of fibroblast growth factor family members (FGF 1, FGF 11, FGF 14) accompanied by down-regulation of other members, such as FGF2, FGF 5, FGF7, (FIG. 7A, Table 1, and Tables 5A-5D).

IL-2 exposed SEN hADSCs are marked by EGF mRNA up-regulation, but down-regulation of mRNA to its receptor EGFR, together a decrease in expression of the serum response factor SRF and the secreted modulator of WNT signaling SFRP1. Interestingly, the expression of both a potent mitogen for cells of mesenchymal origin that promotes wound healing, PDGFA, and its receptor, PDGFRA, is significantly suppressed in SEN hADSCs in comparison to SR cells subjected to IL-2 exposure (FIG. 7A, FIG. 10A, Table 1, and Tables 5A-5D).

These data indicate senescence-related differences in the nature of IL-2 mediated transcriptional response in hADSCs that might impede these cells immunomodulatory properties *ex vivo* and, ultimately, *in vivo*.

A panel of anti-inflammatory and immunomodulatory markers for IL-2 treatment, IL-2 in combination with other drugs and IL-2 exposed human MSCs is shown (Table 2).

The disclosure also contemplates the determination of which pathways are regulated in response to IL-2 treatment. Genes designated as up- or down-regulated in IL-2 treated SR and SEN hADSCs were analyzed, using an integrated gene-set enrichment and pathway network approach to capture the biological reality of coordinated cellular responses to IL-2 stimulation. To do this, pathways that were statistically enriched for up- or down-regulated genes were identified, and then chosen based on the differentially expressed genes that they have in common (FIGS. 10A- 10B). The pathway network representation was weighted based on the numbers of differentially expressed genes in each pathway and the extent to which different pathways share sets of differentially expressed genes. This approach allowed identification of a highly connected network structure with numerous functionally related pathways as well as functionally relevant network substructures.

Upon senescence of hADSCs, IL-2 is less stimulatory for the gene pathways promoting proliferation (cell cycle pathway, q-value=1.54 e-5), imposing G2 checkpoint (G2 pathway, q-value=5.94e-4), p53 pathway (q-value=1.18e-2), major signal transduction MAPK pathway (MAPK, q-value=2.42e-4) and its major subgroup ERK pathway (ERK, q-value =2.62e-2), which regulate important cellular function such as survival, migration and proliferation.

The data also provide information regarding the functionality of MSCs in carcinogenic settings. Both SR and SEN hADSCs exposed to IL-2 are marked by significant increases in expression of transforming growth factors alpha and beta (TGFα, TGFβ1 and TGFβ2), transforming growth factor beta receptor TGFBR2 and transforming growth factor beta receptor-associated protein TGFBRAP1, as well as transforming growth factor beta-induced (TGFBI) genes (FIG. 7A).

IL-2 treated SEN hADSCs show that prominent up-regulated genes are enriched for pathways associated with inflammation (IL-6 pathway, q-value=5.55e-3) and EGF signaling (q-value=2.33e-4) that have been proven to provide a survival advantages to MSCs. The SEN hADSCs exposed to IL-2 are also marked by increased expression of IL-1 R, IL-6 and IL-12 (FIG. 7B).

The observed connection to the angiogenic VEGF pathway (q-value=5.24e-3) (FIG. 10A, right side and FIG. 7D) and the enhanced capacity of SEN hADSC to migration (FIGS. 3A, 3B) may indicate that IL-2 exposed SEN-MSCs could acquire properties necessary to support a tumorigenic environment and metastasis. In addition, up-regulation of the genes included in nitric oxide synthase pathway (iNOS) NOS1 pathway (q-value=8.32e-2) in hADSC upon replicative senescence once again support that MSCs undergoing senescence can acquire metastasis-promoting properties via immunosuppression.

Pathways important for support of proliferation and DNA repair are down-regulated in hADCSs upon senescence: Cell Cycle pathway (q-value=2.52e-5), MCM pathway (q-value=1.62e-8), RB pathway (q-value=6.97e-5) ATM pathway (q-value=3.28e-2), p53 pathway (q-value=1.86 e-2) shown in FIG. 10B. Overall, the data indicated that there are more biological pathways subjected to IL-2 triggered down-regulation in senescence then in self-renewal and these biological pathways are interconnected (FIG. 10B), further linking together a physiological impairment of IL-2 response upon replicative aging of hADSCs, thus indicateing that such impairment might be an integral to adipose-derived stem cell deviated function in vivo and upon clinical applications.

The data provide a list of molecular marker targets critical for assessment of immunomodulatory and anti-inflammatory events and making informative decision for prioritizing autologous or allogeneic MSCs usage for clinical applications, and/ or used as a companionship diagnostics of monitoring cancer treatment with an IL-2 agent and/or IL-2 with cell therapies in clinical settings (Table 2).

Table 2 shows the differential expression of anti-inflammatory and immunomodulatory factors upon IL-2 treatment in SEN and SR cells. The SR GFOLD values represent the fold difference in SR cells treated with IL-2, relative to SR cells not treated with IL-2; the SEN GFOLD values represent the fold difference in SEN cells treated with IL-2, relative to SEN cells not treated with IL-2.

**Table 2: Differential Expression of Anti-Inflammatory and Immunomodulatory Factors Upon IL-2 Treatment**

| Gene Symbol | Gene ID | SR IL-2+ | SR IL-2+ | SEN IL-2- | SEN IL-2+ | SR GFOL D | SEN GFOLD |
|---|---|---|---|---|---|---|---|
| CSF1R | NM_005211 | 80 | 43 | 189 | 455 | -0.27 | 0.98 |
| TNFSF13 | NM_003808 | 69 | 55 | 104 | 172 | 0.00 | 0.31 |
| HIVEP2 | NM_006734 | 2739 | 2790 | 3876 | 5495 | 0.00 | 0.43 |
| ITGAV | NM_002210 | 6797 | 7110 | 10656 | 17227 | 0.01 | 0.65 |
| ITFG1 | NM_030790 | 1067 | 1164 | 2398 | 3793 | 0.00 | 0.57 |

(continued)

| Gene Symbol | Gene ID | SR IL-2+ | SR IL-2+ | SEN IL-2- | SEN IL-2+ | SR GFOL D | SEN GFOLD |
|---|---|---|---|---|---|---|---|
| IL-32 | NM_001012631 | 404 | 422 | 526 | 1397 | 0.00 | 1.24 |
| PTGER1 | NM_000955 | 9 | 19 | 37 | 104 | 0.00 | 0.86 |
| HIVEP1 | NM_002114 | 1142 | 1217 | 1447 | 2046 | 0.00 | 0.38 |
| CERCAM | NM_016174 | 6943 | 7119 | 12879 | 16369 | 0.00 | 0.31 |
| CD99 | NM_001277710 | 6948 | 7069 | 13830 | 16928 | 0.00 | 0.25 |
| CD68 | NM_001251 | 3830 | 3590 | 9144 | 11331 | -0.02 | 0.26 |
| IL6 | NM_000600 | 434 | 502 | 782 | 996 | 0.00 | 0.19 |
| CD83 | NM_001040280 | 19 | 27 | 69 | 107 | 0.00 | 0.12 |
| CD320 | NM_016579 | 1068 | 782 | 990 | 1360 | -0.29 | 0.32 |
| CD72 | NM_001782 | 61 | 36 | 73 | 122 | -0.06 | 0.25 |
| CHST3 | NM_004273 | 2867 | 2935 | 2766 | 3706 | 0.00 | 0.34 |
| TNFRSF25 | NM_003790 | 61 | 68 | 56 | 98 | 0.00 | 0.25 |
| CMIP | NM_198390 | 1503 | 1435 | 1289 | 2460 | 0.00 | 0.82 |
| ESM1 | NM_007036 | 2562 | 1887 | 1863 | 7612 | -0.34 | 1.94 |
| CCL28 | NM_148672 | 68 | 37 | 44 | 170 | -0.20 | 1.39 |
| CLEC11A | NM_002975 | 1071 | 1403 | 1601 | 1449 | 0.25 | -0.02 |
| LRRC8A | NM_001127244 | 3710 | 4256 | 6709 | 6370 | 0.12 | -0.02 |
| IL33 | NM_033439 | 55 | 84 | 220 | 198 | 0.03 | 0.00 |
| IRAK1 | NM_001569 | 5587 | 6539 | 9009 | 9094 | 0.17 | 0.00 |
| TNFRSF21 | NM_014452 | 244 | 331 | 636 | 616 | 0.16 | 0.00 |
| IL12A | NM_000882 | 14 | 36 | 94 | 91 | 0.29 | 0.00 |
| NOS3 | NM_000603 | 48 | 83 | 137 | 142 | 0.19 | 0.00 |
| MYL9 | NM_006097 | 10584 | 19391 | 38407 | 32665 | 0.83 | -0.21 |
| IRAK3 | NM_007199 | 181 | 304 | 613 | 488 | 0.43 | -0.13 |
| ILF3 | NM_012218 | 5977 | 5913 | 6830 | 6368 | 0.00 | -0.04 |
| IL-16 | NM_001172128 | 129 | 72 | 150 | 120 | -0.35 | 0.00 |
| PEAR1 | NM_001080471 | 1926 | 2387 | 510 | 187 | 0.21 | -1.16 |
| Gene Symbol | Gene ID | SR IL-2- | SR IL-2+ | SEN IL-2- | SEN IL-2+ | SR GFOL D | SEN GFOL D |
| IL7R | NM_002185 | 831 | 954 | 634 | 545 | 0.04 | -0.02 |
| ILF2 | NM_001267809 | 1344 | 1961 | 1878 | 1580 | 0.43 | -0.13 |
| CCL2 | NM_002982 | 98 | 147 | 160 | 102 | 0.15 | -0.23 |
| KIF14 | NM_014875 | 329 | 331 | 381 | 251 | 0.00 | -0.33 |

**Example 6: Anti-Inflammatory and Immunomodulatory Properties of IL-2 Exposed Human MSCs**

Next, it was investigated how exposure to the IL-2 pro-inflammatory environment, when imposed on replicative aging, affects the expression of the genes assigned to provide immunomodulatory properties of hADSCs (e.g., the anti-inflammatory and immunomodulatory properties of IL-2 exposed human MSC). The data demonstrated that the capacity for immunomodulation is affected by replicative aging of the human adipose-derived MCS during *ex vivo* passaging (FIG. 7B and Table 2).

IL-2 exposure in SR hADSCs activates distinct set of genes attributed to T cell regulation. IL-2 exposure of self-renewing hADSCs results in up-regulation of genes, such as TNFRSF21 (involved in T cels differentiation), IL12A (T-cell activator), ILF2 (potent regulator of transcription of the IL-2 gene during T-cell activation), IL33 (paracrine inducer of T-helper type 2 associated cytokines) and down-regulation of CCL28 (chemotactic factor for CD+4, CD+8 T-cells), CD320 (receptor molecule with autocrine and paracrine function to augment the proliferation of plasma cells) shown in FIG. 7B, Table 2, and Tables 5A-5D.

Contrary to that, IL-2 exposed SEN hADSCs were characterized by significant transcriptional up-regulation of CD320, a number of integrins which could be involved in modulation of T-cell function (ITG 11, ITGA V, ITFG 1 ), and genes encoding important regulatory molecules such as: the T-cell adhesion receptor (CD99), a factor attributed to the maintenance of naïve T-cells (CHST3), T-cell activators (HIVEP 1 and HIVEP2), a gene involved in T-cell signaling pathway (CMIP) and an autocrine/paracrine factor, PTGER1, involved in inhibition of CD+ cell proliferation (FIG. 7B, Table 2, and Tables 5A-5D).

The data also demonstrated that SR hADSCs exposed to IL-2 trigger down-regulation of transcriptional activities of the genes encoding surface receptors that play a role in B-cell proliferation and differentiation (CD72) and horning macrophages (CD68). Both of these genes are significantly transcriptionally up-regulated in senescent cells upon similar treatment (FIG. 7B, Table 2, Tables 5A-5D). In addition, IL-2 treated SEN hADSCs are set apart from similarly treated SR cells by transcriptional down-regulation of the genes required for pro-B to pre-B transitioning, the LRRC8A and PEARI genes, that regulate a number of non-adherent myeloid progenitors. In contrast, the genes involved in lymphocyte activation and homeostasis (CD83 and TNFRSF25) as well as leukocyte transmigration (CERCAM), and the genes responsible for endothelial cell-leukocyte interaction (ESM1), and a gene important for control monocytes/macrophage mediated immunological process (TNFSF 13), are up-regulated in SEN hADSCs (FIG. 7B, Table 2, and Tables 5A-5D).

IL-2 exposure results in the differential expression of a number of cytokines and factors critical for chemotaxis (shown in FIG. 7B and Table 2).

SR hADSCs are marked by up-regulation of IL-33, IL-12A, IL10RB, IL1RAP, IL7R, ILF2 and NOS3 genes, while IL-16 and CSF1R genes are down-regulated in these cells.

In SEN hADSCs treated under similar conditions with IL-2, the genes encoding cytokines IL-32, IL-6, IL1RN, IL-20RB, IL-21R and inducers of inflammation TNFSF13 and TNFSF12, as well as the gene encoding extracellular matrix remodeler PLAU are up-regulated.

At the same time, several factors essential for cytokinesis such as MYL9, KIF14, IRAC3, as well as the genes encoding chemotactic factor that attracts monocytes and basophils (CCL2) and the CLEC11A gene regulating proliferation and differentiation of hematopoietic precursor cells, are down-regulated (FIG. 7B).

Similar down-regulation is also found for several interleukin receptor encoding genes IL7R, IL1R1, IL15RA, and interleukin enhancer binding factors ILF2 and ILF3.

These observations, together with IL-2 dependent differential transcriptional expression of cytokines in SEN hADSCs (up-regulation of IL-32, IL-6, PLAU genes; down-regulation of CCL2, CLEC11A, ILF3, IRAK3, KIF14, MYL9 genes) and in SR hADSCs (up-regulation of IL12A, IL7R, IRAK1, NOS3 genes; down-regulation of IL-16, CSF1R genes), indicate that the immunomodulatory properties of hADSCs are susceptible to senescence imposed changes.

The observed connection to the angiogenic VEGF pathway (q-value=5.24e-3) (FIG. 10A, right side and FIG. 7D) and the enhanced capacity of SEN hADSC to migration (FIGS. 4A,B) indicates that IL-2 exposed SEN-MSCs could acquire properties necessary to support a tumorigenic environment and metastasis. In addition, up-regulation of the genes included in nitric oxide synthase pathway (iNOS) NOS1 pathway (q-value=8.32e-2) in hADSC upon replicative senescence also indicate that MSCs undergoing senescence can acquire metastasis-promoting properties via immunosuppression.

**Example 7: Anti-apoptotic and metastasis promoting properties of IL-2-stimulated hADSCs upon replicative senescence**

These experiments were also used to examine the anti-apoptotic and metastasis promoting properties of IL-2 exposed MSC upon replicative senescence.

The panel of anti-apoptotic and metastasis promoting markers for IL-2 treatment, IL-2 in combination with other drugs, or IL-2 in combination with MSC is shown in Table 3. The data provide a list of molecular marker targets important for assessment of anti- apoptotic and metastasis promoting events and making informative decision for prioritizing autologous or allogeneic MSCs usage for clinical applications, and/ or used as a companion diagnostics of monitoring cancer treatment with an IL-2 agent and/or IL-2 with cell therapies in clinical settings (Table 3).

Table 3 shows the differential expression of anti-apoptotic and metastasis factors upon IL-2 treatment in SEN and SR cells. The SR GFOLD values represent the fold difference in SR cells treated with IL-2, relative to SR cells not treated with IL-2; the SEN GFOLD values represent the fold difference in SEN cells treated with IL-2, relative to SEN cells not treated with IL-2.

**Table 3: Differential Expression of Anti-Apoptotic and Metastasis Factors Upon IL-2 Treatment**

| Gene Symbol | Gene ID | SR IL-2- | SR IL-2+ | SEN IL-2- | SEN IL-2+ | SR GFOLD | SEN GFOLD |
|---|---|---|---|---|---|---|---|
| VEGFA | NM_001025366 | 5690 | 5914 | 5781 | 20606 | 0.00 | 1.78 |
| PLEKHA1 | NM_001195608 | 716 | 676 | 713 | 1096 | 0.00 | 0.46 |
| VEGFB | NM_003377 | 989 | 1070 | 1832 | 2253 | 0.00 | 0.19 |
| CRMP1 | NM_001288661 | 168 | 209 | 401 | 547 | 0.00 | 0.23 |
| FERMT1 | NM_017671 | 76 | 70 | 264 | 325 | 0.00 | 0.02 |
| CTSB | NM_147780 | 28365 | 28109 | 115877 | 138711 | 0.00 | 0.25 |
| PLEKHA6 | NM_014935 | 78 | 57 | 150 | 251 | 0.00 | 0.40 |
| GNB2L1 | NM_006098 | 16273 | 18409 | 33137 | 32212 | 0.14 | -0.01 |
| SIVA1 | NM_006427 | 410 | 591 | 674 | 512 | 0.31 | -0.20 |
| TGF$\beta$1 | NM_000660 | 4834 | 6297 | 3413 | 5582 | 0.32 | 0.64 |

For example, MSCs have been proven to assist reversal of apoptosis in cardiomyoblasts after ischemia, as well as damaged neurons and lung fibroblasts. Stanniocalcin 1 (STC 1) has been identified as an essential factor capable of potent apoptotic reversal in fibroblasts damaged by UV and acidity.

The data indicate that IL-2 exposure transcriptionally upregulates both STC1 and STC2 genes, and such activation is not dependent on the replicative aging of hADSCs, at least *ex vivo* (Tables 5A-5D). In addition, paracrine effectors such as VEGF and TGFB1 have been implicated in the reversal of apoptosis in endothelial cells. The expression of genes encoding both of these factors is up-regulated in SR and SEN hADSCs upon IL-2 treatment (FIG. 7C, FIG. 5, Table 3, and Tables 5A-5D).

The third graph of FIG. 5 shows that IL-2 upregulates transcription of the VEGFA gene upon replicative senescence of hADSCs. *VEGFA* gene expression was assessed by quantitative Q-PCR in unstimulated (IL-2-) senescent (dark) and self-renewing (light) hADSCs and upon stimulation with 20ug/ml of recombinant IL-2 ( IL-2+). Data shown as fold change $\Delta\Delta CT$ Mean $\pm$ SD from three independent experiments is shown. The position of the q-PCR primers are depicted graphically. Statistical significance was estimated by *t* - test, where \*\*\**p* < 0.001, \*\**p* < 0.01.

However, transcriptional activity of VEGFA is notably higher in senescence than in actively proliferating cells as further verified by Q-PCR analysis shown in FIG. 7C. Notably, the SIVA1 gene encoding a pro-apoptotic factor and a potent inducer of T lymphocytes apoptosis is significantly down-regulated in senescent cells upon IL-2 treatment in comparison to proliferating hADSCs (FIG. 7C, Table 3, and Tables 5A-5D). SIVA1 is not a strictly pro-apoptotic factor, but also a potent suppressor of tumor metastasis. Importantly, a number of the factors responsible for invasive growth and metastasis are significantly up-regulated in SEN hADSCs exposed to IL-2 in comparison with similarly treated SR cells (FIG. 7C and Table 3). This includes RACKI, PLEKHAI, PLEKHA6, CTSB, CRMPI, FERMTI genes. These data indicated that pretreatment/exposure of hADSCs with IL-2 may enhance the antiapoptotic properties of these cells in general, and that such enhancement is effected by replicative senescence, at least in culture.

It was demonstrated that in IL-2 treated SEN hADSCs, prominent up-regulated genes are enriched for pathways associated with inflammation (IL-6 pathway, q-value=5.55e-3) and EGF signaling (q-value=2.3 3 e-4) that have been proven to provide a survival advantages to MSCs. The SEN hADSCs exposed to IL-2 are also marked by increased expression of IL-1$\beta$, IL-6 and IL-12 (FIG. 7B), cytokines known to stimulate IL-17 from lymphocytes.

The data also indicated that lymphocytes are the only source of IL-17 production, and those MSCs, particularly upon their senescence, display high transcriptional activity of IL-17 when subjected to a pro-inflammatory environment (FIG. 7A). The MCS-derived IL-17 together with MCS-derived CSF-1 may induce systemic neutrophil expansion and macrophages infiltration similar to studies indicating a critical role for these factors in promoting cancer progression and metastasis as well as in a number of inflammatory diseases including psoriasis.

**Example 8: Transcriptional profiling indicates gene targets regulating enhanced migration and angiogenesis in IL-2 stimulated hADSCs upon replicative senescence**

The panel of the markers indicating enhanced migration and angiogenesis in IL-2 treatments upon aging is shown (Table 4)

The list of molecular marker targets critical for assessment of migration and angiogenesis promoting events can assist in making informative decisions for prioritizing autologous or allogeneic MSCs usage for clinical applications, and/ or used as a companion diagnostics of monitoring cancer treatment with an IL-2 agent and/or IL-2 with cell therapies in clinical settings (Table 4).Transcriptional profiling indicates gene targets regulating enhanced migration and angiogenesis in IL-2 stimulated ADS Cs upon replicative senescence. Further analysis of the transcriptional response indicates that IL-2 stimulation of SEN hADSCs s enhances the expression of genes involved in vascular development and remodeling related to angiogenesis. It was observed significant up-regulation of the VEGFA, VEGFB, FBLNS, FBLN7, PGF, ANGPTI, ANGPT2, ANGPTL2, ANGPTL6, TNFSF12, PRKCA, PIK3CA, HRAS genes as well as a gene encoding a potent modulator of endothelial cell-leukocyte adhesion, ESM1 (FIG. 7D, FIGS. 10A- 10B, Table 4, and Tables 5A-5D).

Table 4 shows the differential expression of migration and angiogenesis factors upon IL-2 treatment in SEN and SR cells. The SR GFOLD values represent the fold difference in SR cells treated with IL-2, relative to SR cells not treated with IL-2; the SEN GFOLD values represent the fold difference in SEN cells treated with IL-2, relative to SEN cells not treated with IL-2.

**Table 4: Differential Expression of Migration and Angiogenesis Promoting Factors Upon IL-2 Treatment**

| Gene Symbol | Gene ID | SR IL-2- | SR IL-2+ | SEN IL-2- | SEN IL-2+ | SR GFOLD | SEN GFOLD |
|---|---|---|---|---|---|---|---|
| PTGS1 | NM_000962 | 1202 | 1233 | 2595 | 8456 | 0.00 | 1.63 |
| CGREF1 | NM_0011662 39 | 60 | 77 | 137 | 384 | 0.00 | 1.15 |
| ROCK2 | NM_004850 | 2014 | 2149 | 2171 | 2737 | 0.00 | 0.24 |
| VEGFA | NM_0010253 66 | 5690 | 5914 | 5781 | 20606 | 0.00 | 1.78 |
| PLEKHA1 | NM_0011956 08 | 716 | 676 | 713 | 1096 | 0.00 | 0.46 |
| ESM1 | NM_007036 | 2562 | 1887 | 1863 | 7612 | -0.34 | 1.94 |
| ANGPTL6 | NM_031917 | 339 | 195 | 279 | 342 | -0.50 | 0.02 |
| PIK3CA | NM_006218 | 519 | 597 | 720 | 939 | 0.00 | 0.22 |
| PGF | NM_002632 | 471 | 471 | 842 | 1503 | 0.00 | 0.69 |
| TNK2 | NM_005781 | 582 | 624 | 813 | 1176 | 0.00 | 0.38 |
| FBLN7 | NM_153214 | 201 | 195 | 268 | 353 | 0.00 | 0.13 |
| PLEKHA6 | NM_014935 | 78 | 57 | 150 | 251 | 0.00 | 0.40 |
| PRKCA | NM_002737 | 3139 | 3258 | 4215 | 5298 | 0.00 | 0.26 |
| ROCK1 | NM_005406 | 2280 | 2326 | 2662 | 3068 | 0.00 | 0.12 |
| CGNL1 | NM_0012523 35 | 11 | 22 | 67 | 115 | 0.00 | 0.27 |
| FGD6 | NM_018351 | 419 | 411 | 749 | 1055 | 0.00 | 0.33 |
| CRMP1 | NM_0012886 61 | 168 | 209 | 401 | 547 | 0.00 | 0.23 |
| CTSO | NM_001334 | 222 | 275 | 499 | 617 | 0.01 | 0.10 |

(continued)

| Gene Symbol | Gene ID | SR IL-2- | SR IL-2+ | SEN IL-2- | SEN IL-2+ | SR GFOLD | SEN GFOLD |
|---|---|---|---|---|---|---|---|
| TNFSF12-TNFSF13 | NM_172089 | 268 | 259 | 464 | 577 | 0.00 | 0.11 |
| FAP | NM_004460 | 1810 | 1767 | 4594 | 6279 | 0.00 | 0.39 |
| VEGFB | NM_003377 | 989 | 1070 | 1832 | 2253 | 0.00 | 0.19 |
| FBLN5 | NM_006329 | 5879 | 6117 | 8801 | 10137 | 0.00 | 0.15 |
| ANGPT1 | NM_001146 | 386 | 406 | 1069 | 1346 | 0.00 | 0.20 |
| ANGPTL2 | NM_012098 | 1241 | 1362 | 4927 | 5560 | 0.00 | 0.11 |
| CTSB | NM_147780 | 28365 | 28109 | 115877 | 138711 | 0.00 | 0.25 |
| FERMT1 | NM_017671 | 76 | 70 | 264 | 325 | 0.00 | 0.02 |
| HRAS | NM_176795 | 604 | 604 | 996 | 1124 | 0.00 | 0.03 |
| MYL9 | NM_006097 | 10584 | 19391 | 38407 | 32665 | 0.83 | -0.21 |
| TLN2 | NM_015059 | 2058 | 2352 | 4839 | 4309 | 0.09 | -0.10 |
| ILK | NM_001014795 | 5437 | 6108 | 8647 | 8164 | 0.11 | -0.03 |
| PPAP2B | NM_003713 | 1703 | 2546 | 2327 | 1579 | 0.48 | -0.45 |
| CYR61 | NM_001554 | 31391 | 30861 | 12856 | 12074 | 0.00 | -0.05 |
| RELN | NM_005045 | 2490 | 2989 | 925 | 671 | 0.17 | -0.29 |
| NEDD9 | NM_006403 | 2617 | 3093 | 2123 | 1956 | 0.15 | -0.01 |
| TNFAIP8 | NM_001077654 | 109 | 101 | 67 | 100 | 0.00 | 0.05 |
| ANGPT2 | NM_001118887 | 430 | 259 | 89 | 179 | -0.47 | 0.58 |

The vascular endothelial growth factor, VEGF, released by MCSs, enables recruitment of endothelial lineage cells and initiation of vascularization as was previously reported. It is further demonstrated that up-regulation of VEGFA gene expression in SEN hADSCs can be detected by quantitative RT-PCR analysis and IL-2 exposure results in a statistically significant increase of VEGFA gene transcription in SR and SEN hADSCs (FIG. 7D and Table 4).

In response to IL-2, a group of genes responsible for cell motility, migration and invasive growth are significantly up-regulated only in the hADSCs undergoing replicative senescence: CGNL1, CGREF 1, CRMP1, FGD6, TNK2, PTGS1, TNFAIP8, CTSB, CTSO, FAP, FERMT1, PLEKHA1, PLEKHA6, ROCK1, ROCK2. A set of genes promoting cell adhesion, such as CHD24, CYR61, ILK, NEDD9, MYL9, PPAP2B, RELN and TLN2 were down-regulated (FIG. 7D, Table 4, and Tables 5A-5D). These data further the support experimental evidence for the enhanced migration capacity of SEN hADSCs shown in FIG. 3B.

### Example 9: Proteomic antibody array data

Table 6 provides the raw values for all proteomic array data.

MSCs from a 38year old patient in 10% PRP containing StemPro MSC SFM Xeno-free medium plated on a substrate. SR or SEN hADSC determined as described in Example 1 were plated at a density of 2500 cells /cm2 in 700ul/cm2 of 10% PRP containing StemPro MSC SFM Xeno-free medium. SEN and SR hADSCs were stimulated with IL-2 for 24 hrs or remained without stimulation with IL-2 (IL-2-), after which media was exchanged to the 10% PRP containing StemPro MSC SFM Xeno-free medium. Cells were kept at 37°C and 5% CO2 for 24, 48, and 72 hrs after which media was collected and analyzed. Equal volumes of medium were analyzed on RayBio C-Series Human Cytokine Antibody Array AAH-CYT-2000 (RayBiotech, Inc). C-Series Human Cytokine Antibody Array AAH-CYT-2000 is based on chemiluminescence assay principle and contains antibodies to 174 proteins of interest. Data were extracted from the membranes using LI-COR Biosciences densitometry software (Li-COR). The raw data were normalized by taking the ratio between Average Intensity of the given protein signal/ to Average Intensity Negative Control, to account for differences in exposure and array to array variation. Data are presented in FIGS. 11-90 and Table 6 demonstrate protein factors secreted upon IL-2 exposure. These proteins include, hut not limited to factors indicated in the tables in FIGS.7A-7D and secretory profiles recapitulate transcriptional data.

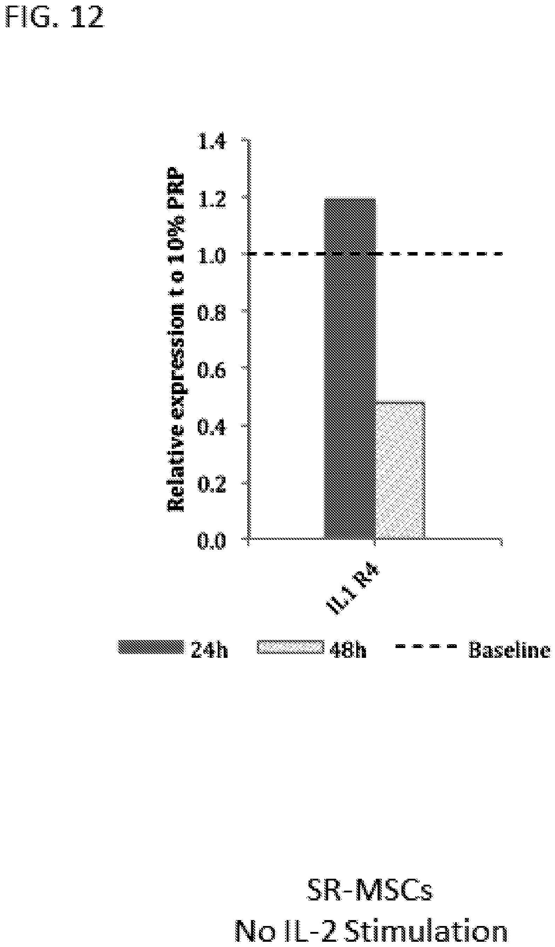

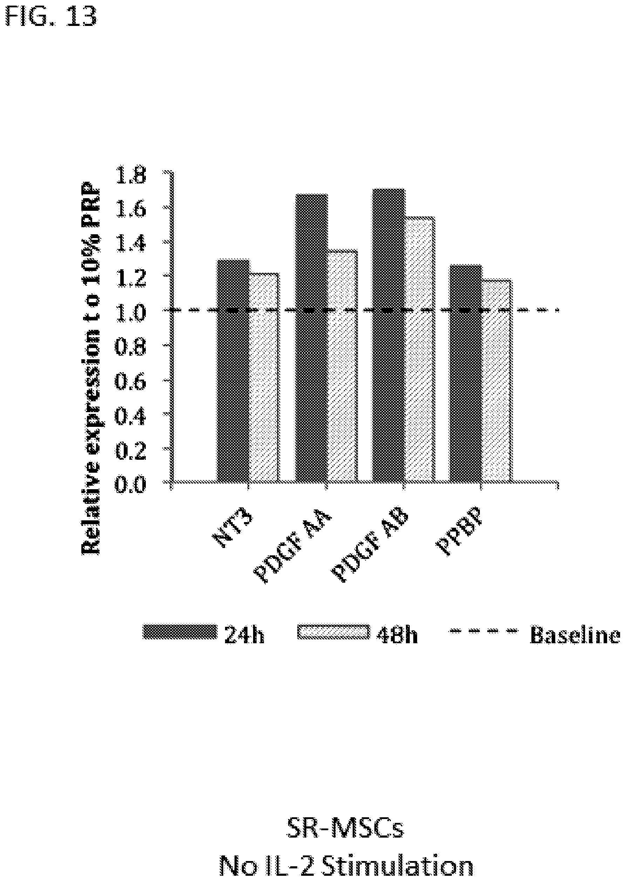

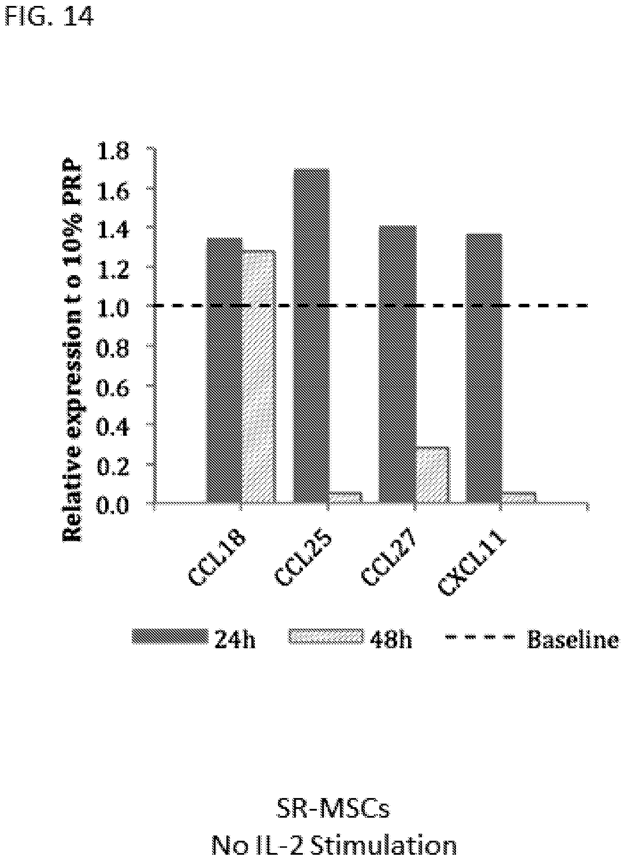

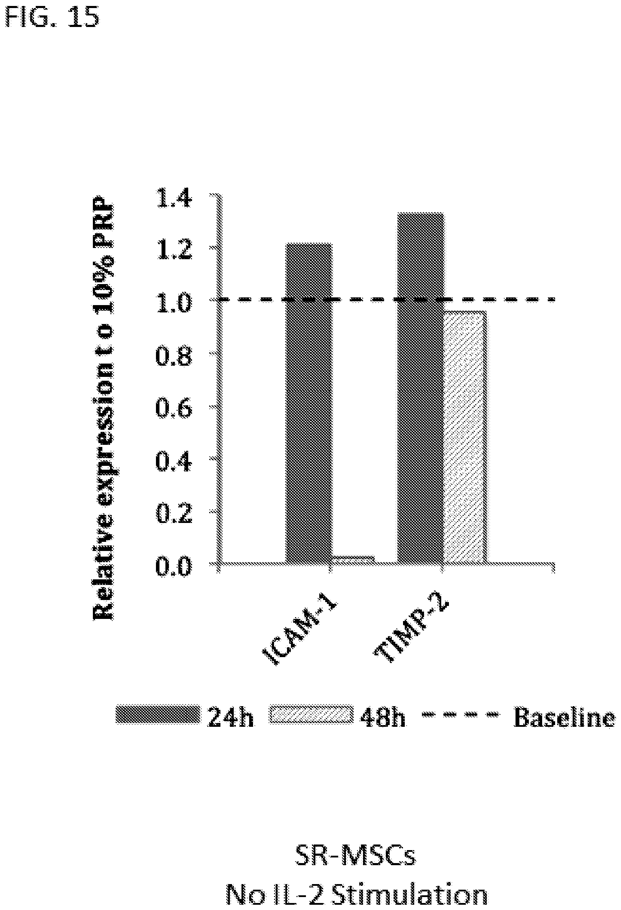

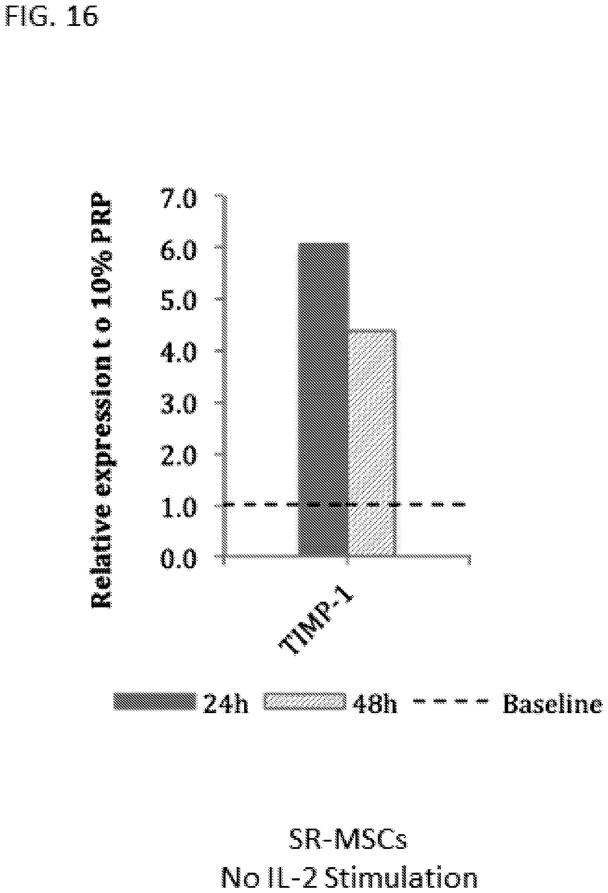

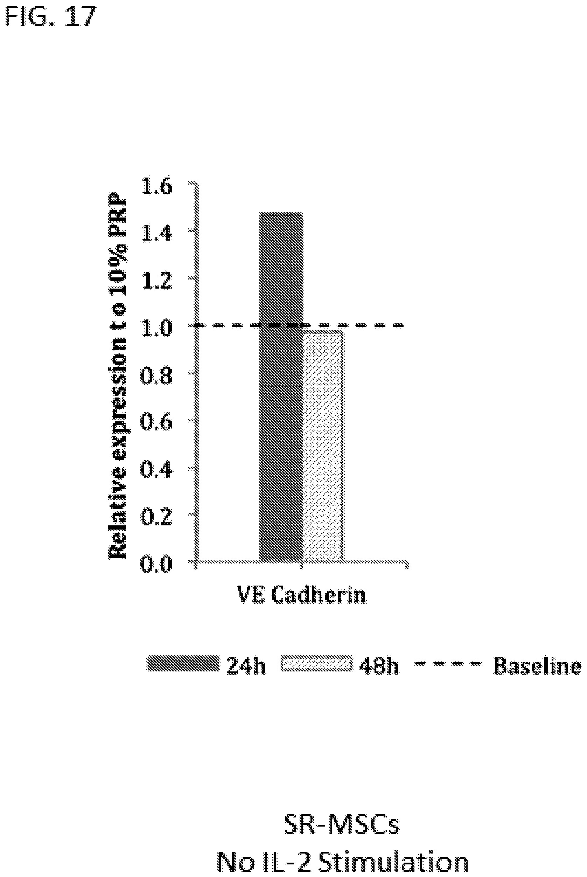

FIGS. 11-17 show the increase in secretion of the below named proteins (factors) from SR-hADSCs, 24 hours post incubation with media containing 10% PRP (platelet-rich plasma) alone (no IL-2 stimulation). Secretion levels are shown relative to the amount of the corresponding protein present at basal levels in the 10% PRP containing media, used for hADSC support. FIG. 11 shows the increase in secretion of Interleukin 5 (IL5) and Interleukin 6 (IL6). FIG. 12 shows the increase in secretion of Interleukin 1 receptor 4 (IL1R4). FIG. 13 shows the increase in secretion of Neurotrophin 3 (NT3), platelet derived growth factor A alpha (PDGF AA), platelet derived growth factor A beta (PDGF AB), and pro-platelet basic protein (PPBP). FIG. 14 shows the increase in secretion of Chemokine (C-C motif) ligand 18 (CCL18), Chemokine (C-Cmotif) ligand 25 (CCL25), Chemokine (C-C motif) ligand 27 (CCL27), and CXC chemokine ligand 11 (CXCL11). FIG. 15 shows the increase in secretion of Intercellular Adhesion Molecule 1 (ICAM-1) and Metalloproteinase inhibitor 2 (TIMP-2). FIG. 16 shows the increase in secretion of Metalloproteinase inhibitor 1 (TIMP-1). FIG. 17 shows the increase in secretion of vascular epithelium (VE) Cadherin (calcium dependent cell adhesion protein).

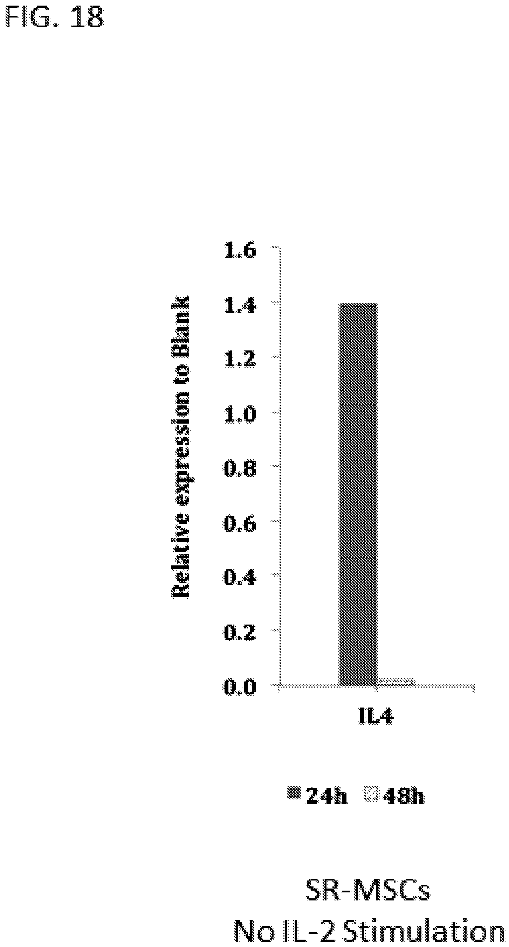

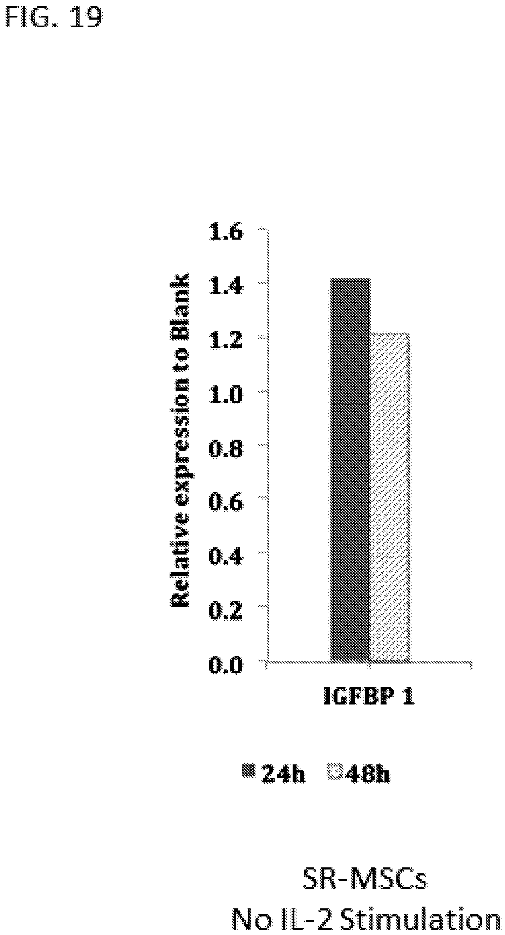

FIGS. 18-19 show the increase in secretion of the below named proteins (factors) from SR-hADSCs, 24 hours post incubation with 10% PRP alone (no IL-2 stimulation). These proteins were found to not be present in PRP. FIG. 18 shows the increase in secretion of Interleukin 4 (IL4). FIG. 19 shows the increase in secretion of insulin-like growth factor-binding protein-1 (IGFBP1).

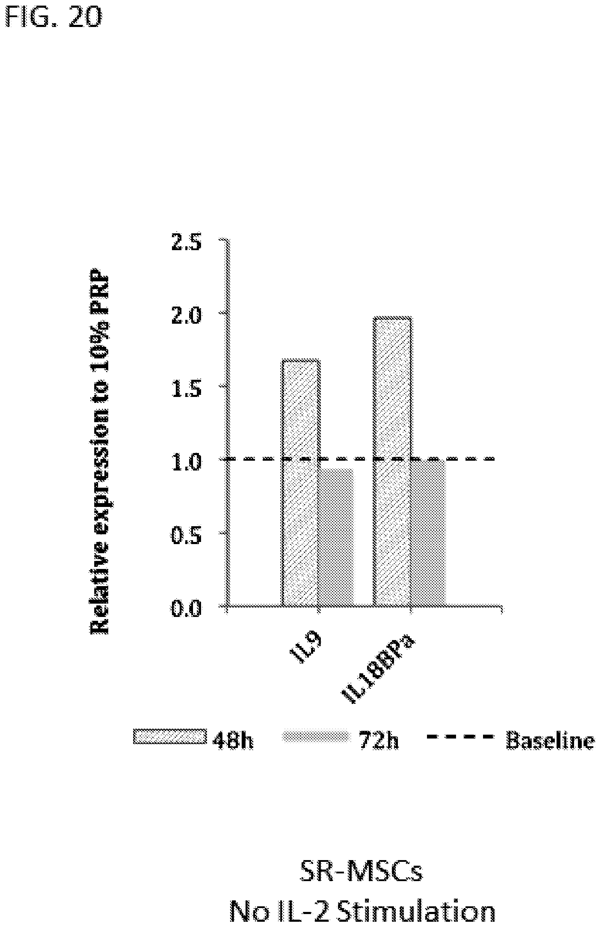

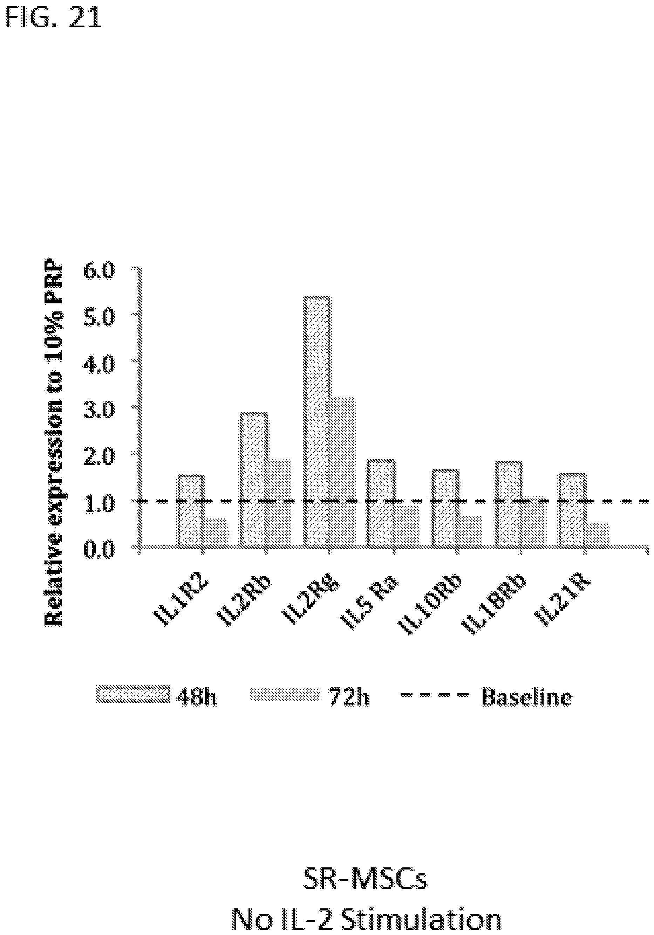

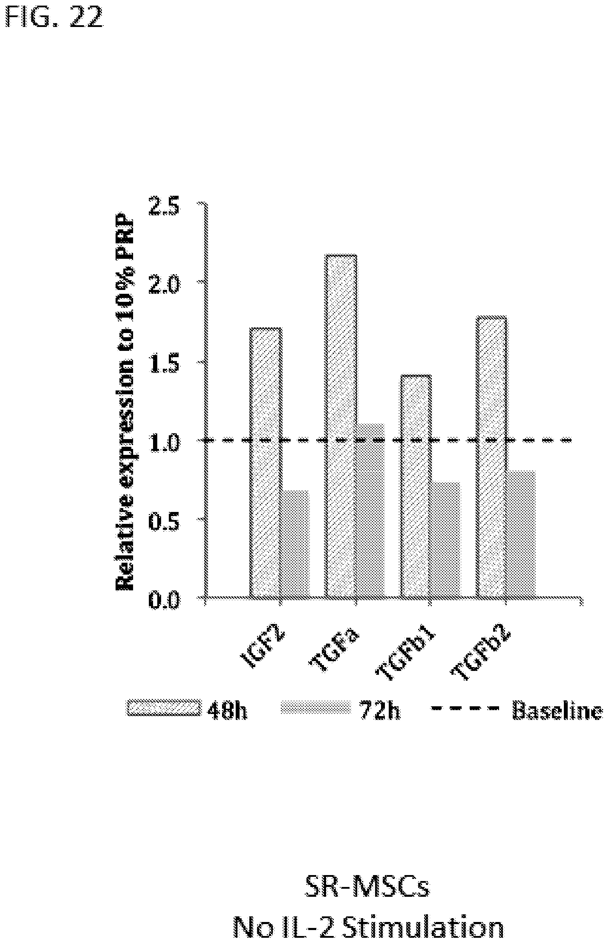

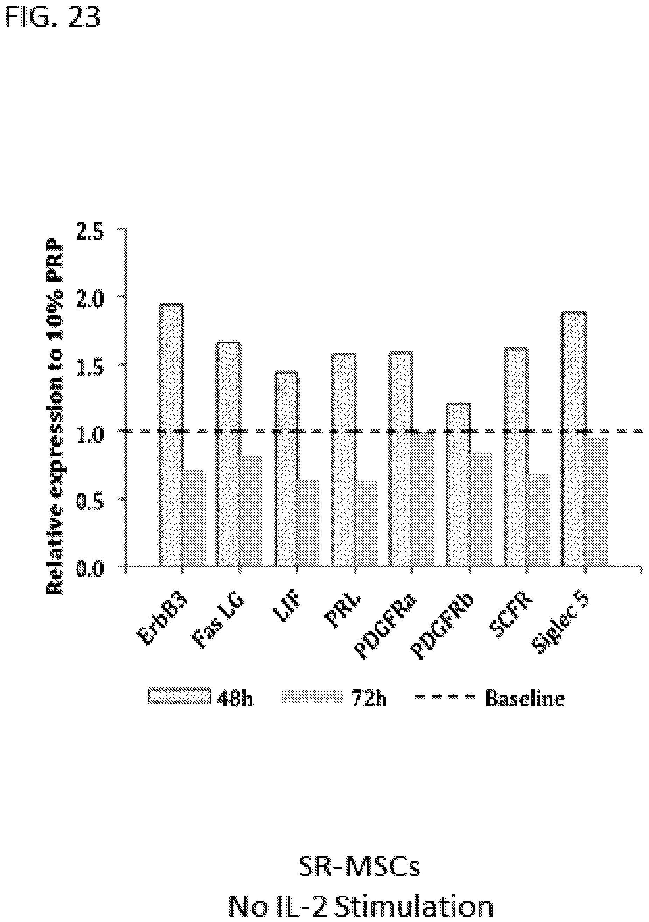

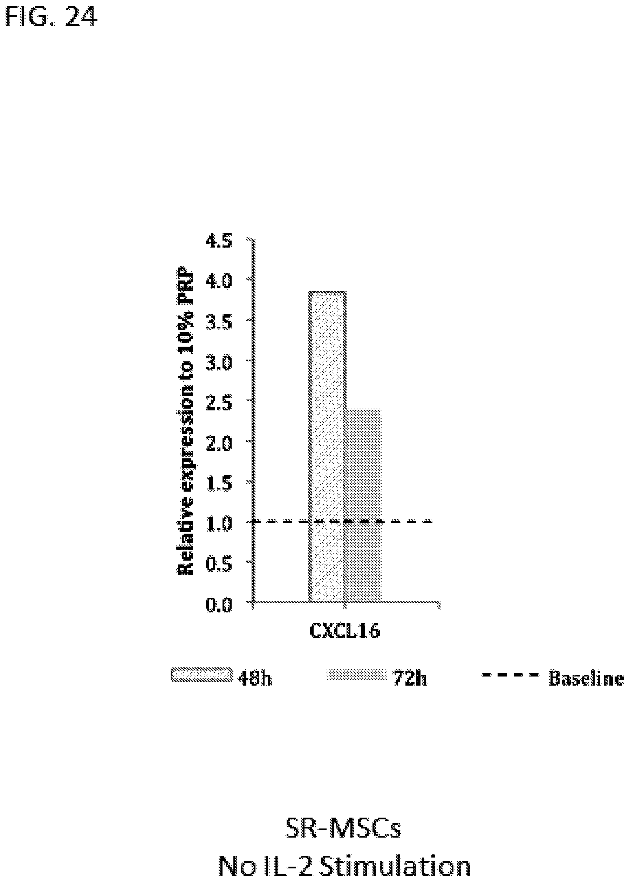

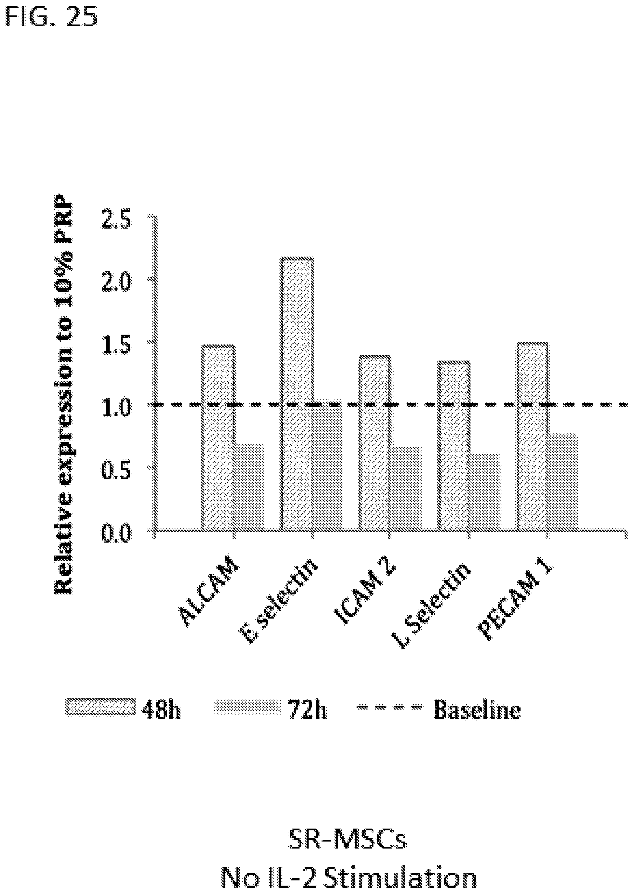

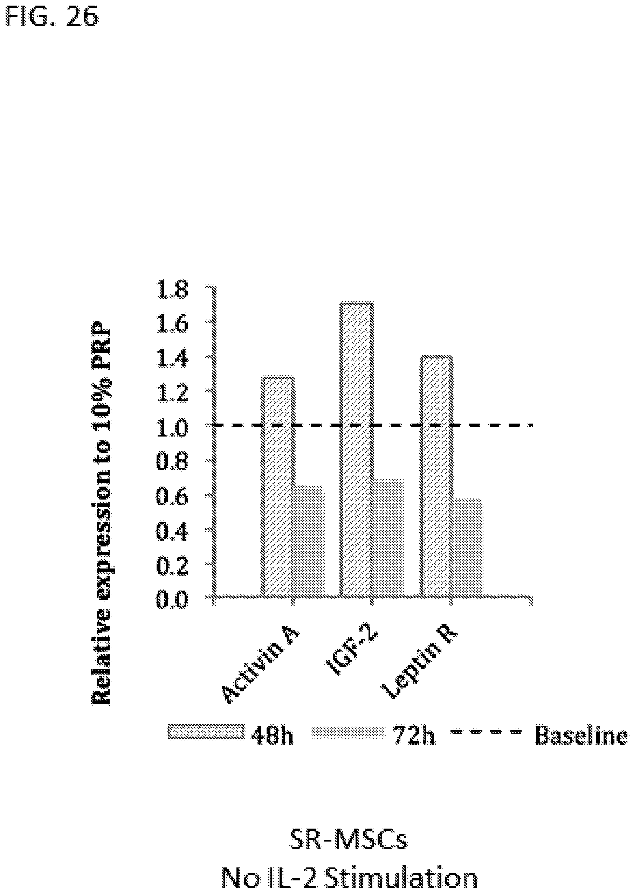

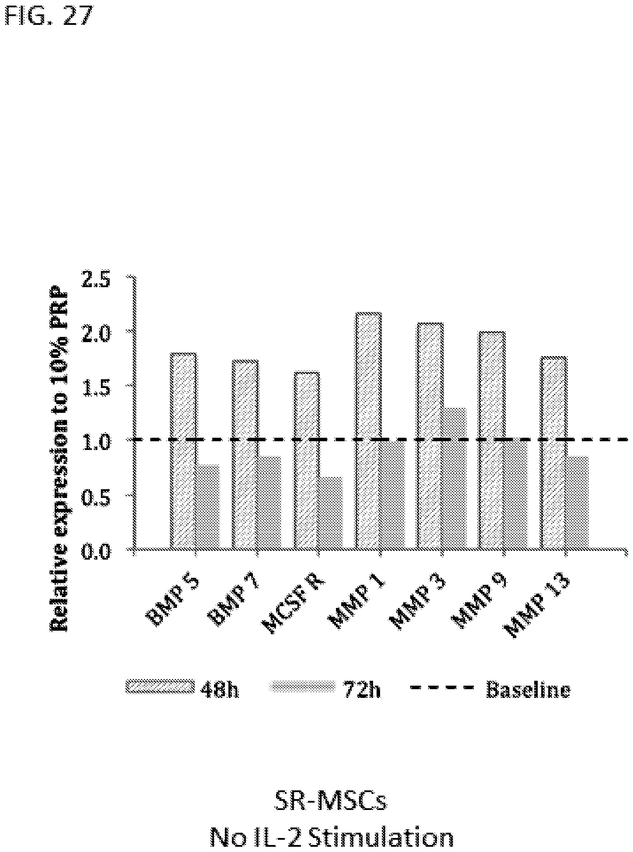

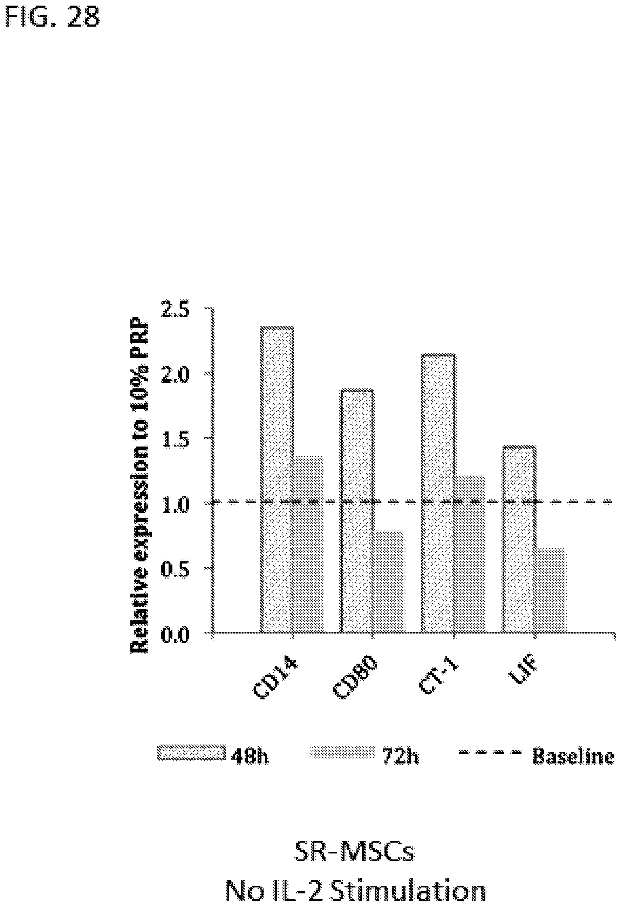

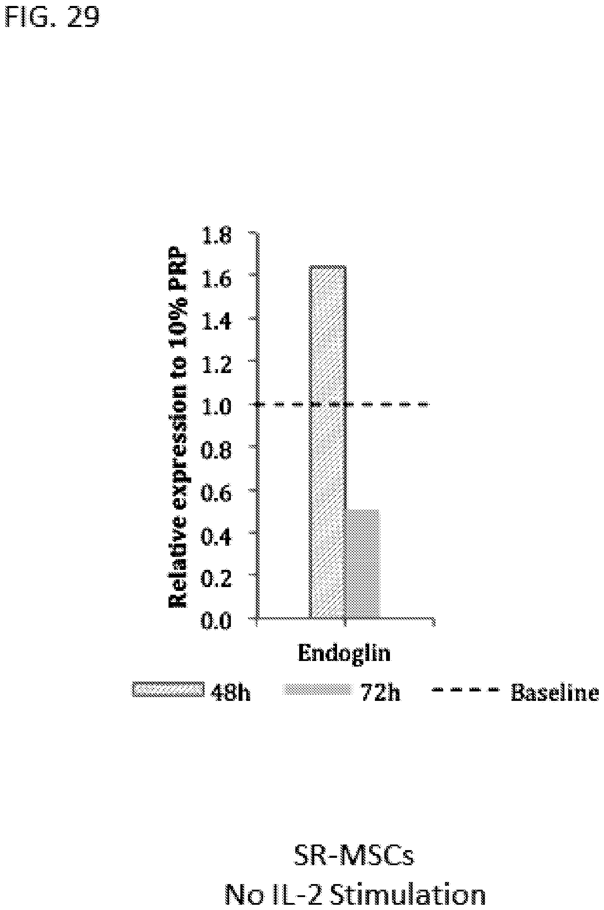

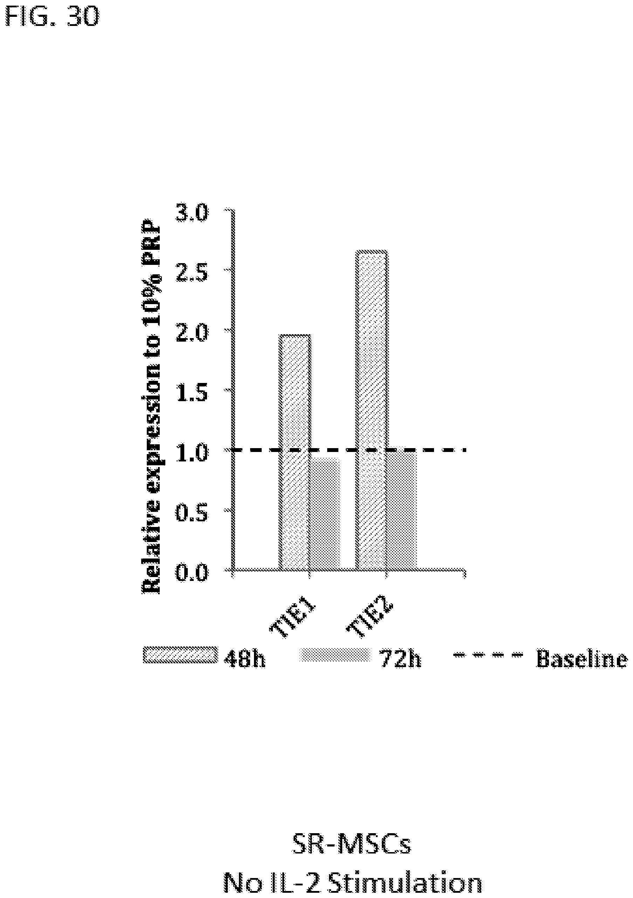

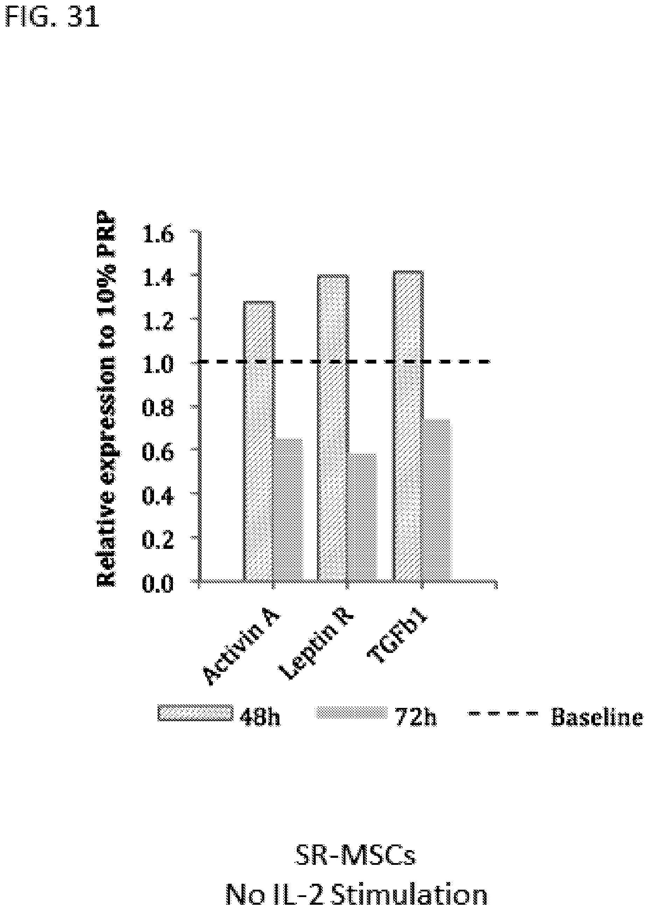

FIGS. 20-31 show an increase in the below named proteins (factors) from SR-hADSCs, 48 hours post incubation with 10% PRP alone (no IL-2 stimulation). Secretion levels are shown relative to the amount of the corresponding protein present at basal levels in the 10% PRP containing media, used for hADSC support. FIG. 20 shows the increase in secretion of Interleukin 9 (IL9) and Interleukin 18 binding protein alpha (IL18BPa). FIG. 21 shows the increase in secretion of Interleukin 1 receptor type II (IL1R2), Interleukin 2 receptor beta (IL-2Rb), Interleukin 2 receptor gamma (IL-2Rg), Interleukin 5 receptor alpha (IL5Ra), Interleukin 10 receptor beta (IL10Rb), Interleukin 18 receptor accessory protein (IL18Rb), and Interleukin 21 receptor (IL-21R). FIG. 22 shows the increase in secretion of Insulin-like growth factor 2 (IGF2), Transforming growth factor alpha (TGFα), Transforming growth factor beta 1/latency-associated peptide (LAP) (TGFβ1), and Transforming growth factor beta 2 (TGFb2). FIG. 23 shows the increase in secretion of Receptor tyrosine-protein kinase ErbB-3 (ErbB3), Fas ligand (Fas LG), Leukemia inhibitory factor (LIF), Prolactin (PRL) factor, platelet-derived growth factor receptor alpha (PDGFRα), platelet-derived growth factor receptor beta (PDGFRβ), Stem cell factor kit receptor (SCFR), and Sialic acid-binding Ig-like Lectin 5 (Siglec 5). FIG. 24 shows the increase in secretion of CXC chemokine ligand 16 (CXCL16). FIG. 25 shows the increase in secretion of activated leukocyte cell adhesion molecule (ALCAM), E selectin (cell surface glycoprotein in immune-adhesion), Intercellular adhesion molecule 2 (ICAM2), L selectin (Leukocyte adhesion molecule), and Platelet endothelial cell adhesion molecule (PECAM 1). FIG. 26 shows the increase in secretion of Activin A (INHBA), Insulin-like growth factor 2 (IGF-2), and Leptin Receptor (LEPR). FIG. 27 shows the increase in secretion of Bone morphogenetic protein 5 (BMP5), Bone morphogenetic protein 7 (BMP7), Macrophage colony-stimulating factor 1 receptor (MCSFR), matrix metalloproteinase 1 (MMP1), matrix metalloproteinase 3 (MMP3), matrix metalloproteinase 9 (MMP9), and matrix metalloproteinase 13 (MMP13). FIG. 28 shows the increase in secretion of monocyte differentiation antigen (CD14), cell differentiation antigen (CD80), Cardiotrophin-1 (CT-1), and Leukemia inhibitory factor (LIF). FIG. 29 shows the increase in secretion of Endoglin (ENG). FIG. 30 shows the increase in secretion of Tyrosine kinase with immunoglobulin-like and EGF-like domains 1 (TIE-1) and Tyrosine kinase with immunoglobulin-like and EGF-like domains 2 (TIE-2). FIG. 31 shows the increase in secretion of Activin A (Inhibin beta A, INHBA), Leptin Receptor (Leptin R), and Transforming growth factor beta 1 (TGFβ1).

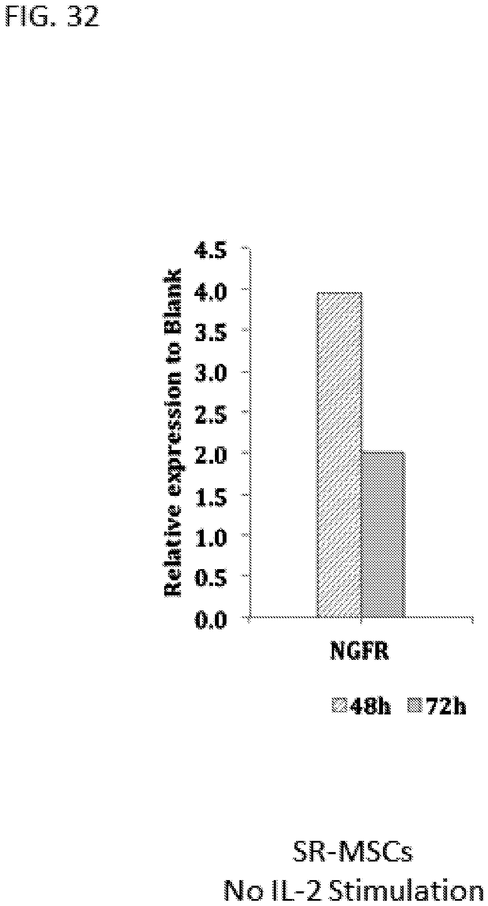

FIG. 32 shows the increase in secretion of Nerve growth factor receptor (NGFR) from SR-MSCs, 48 hours post incubation with 10% PRP alone (no IL-2 stimulation). NGFR was found to not be present in PRP.

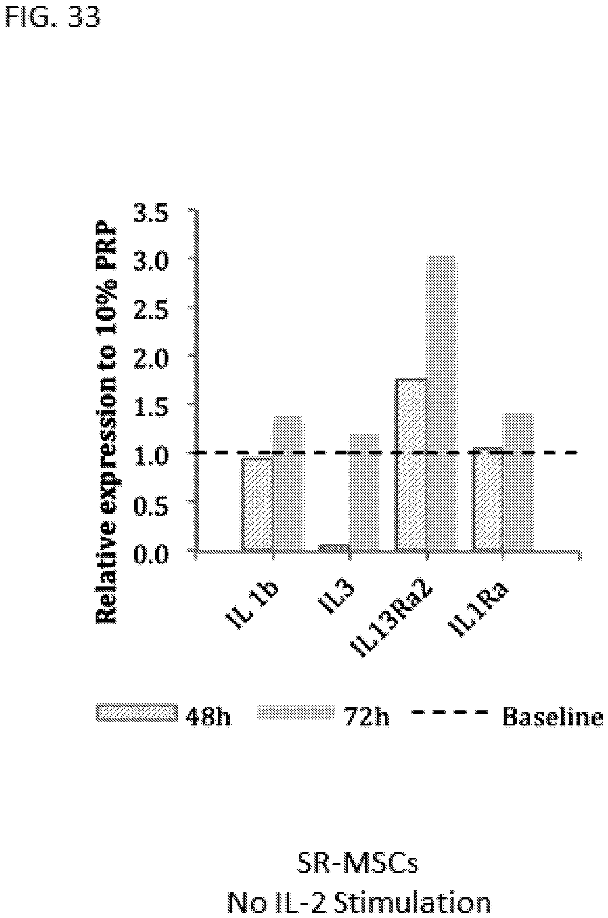

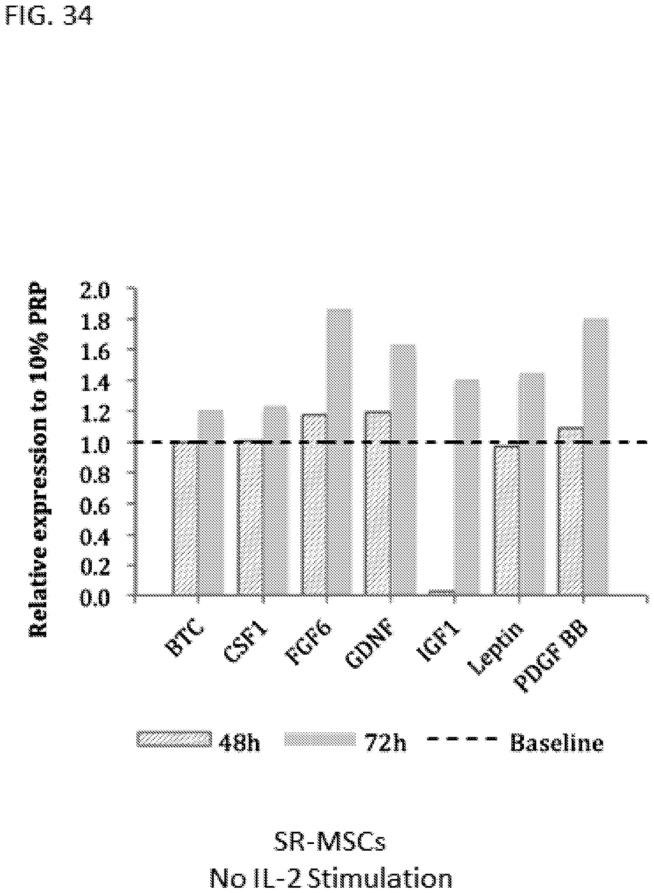

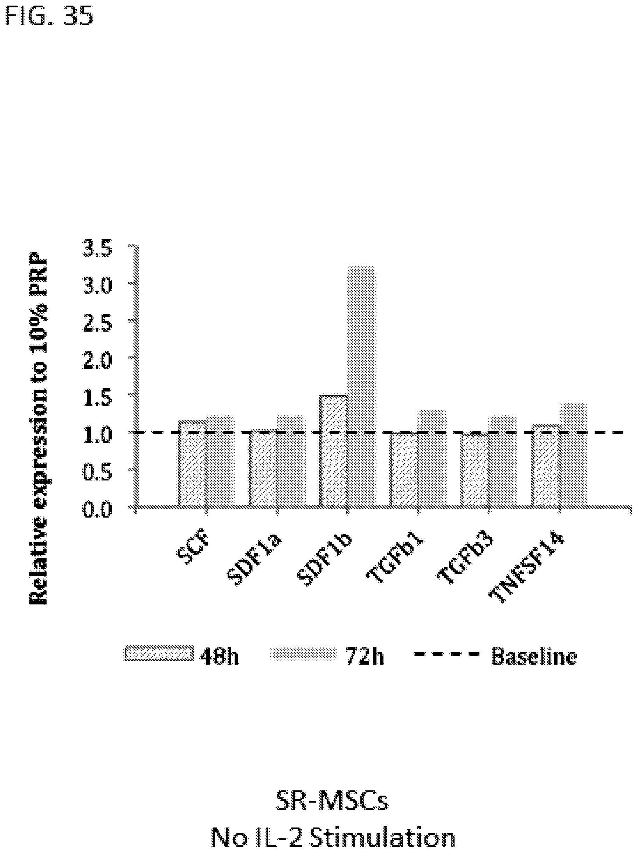

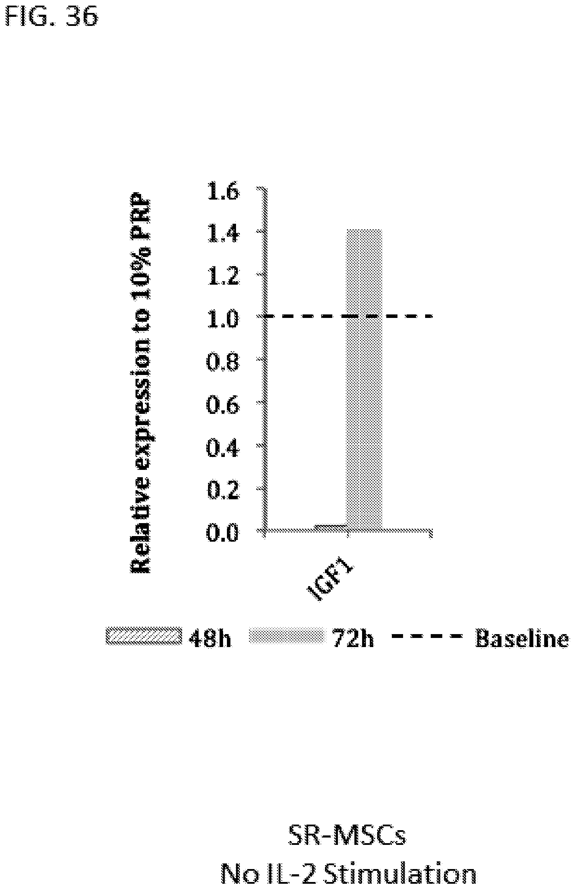

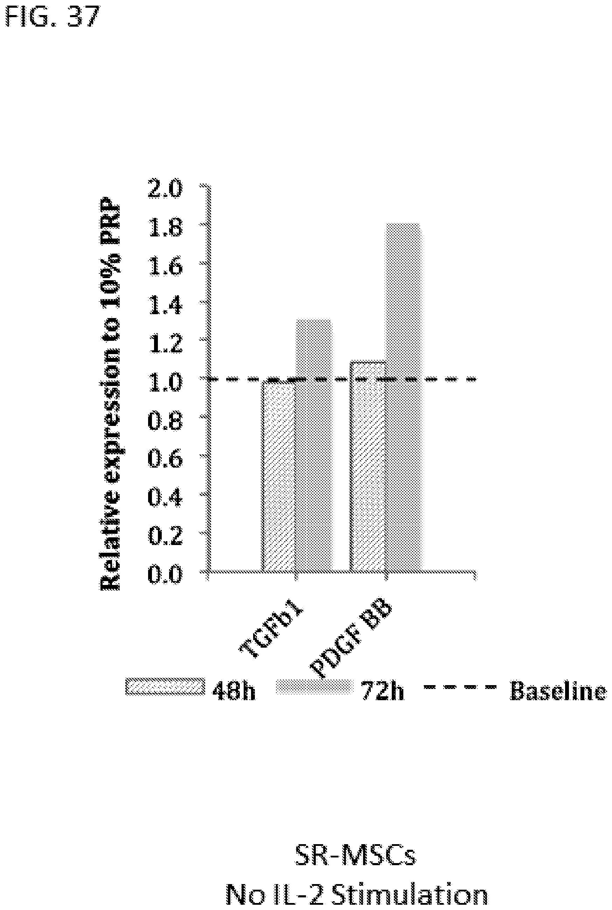

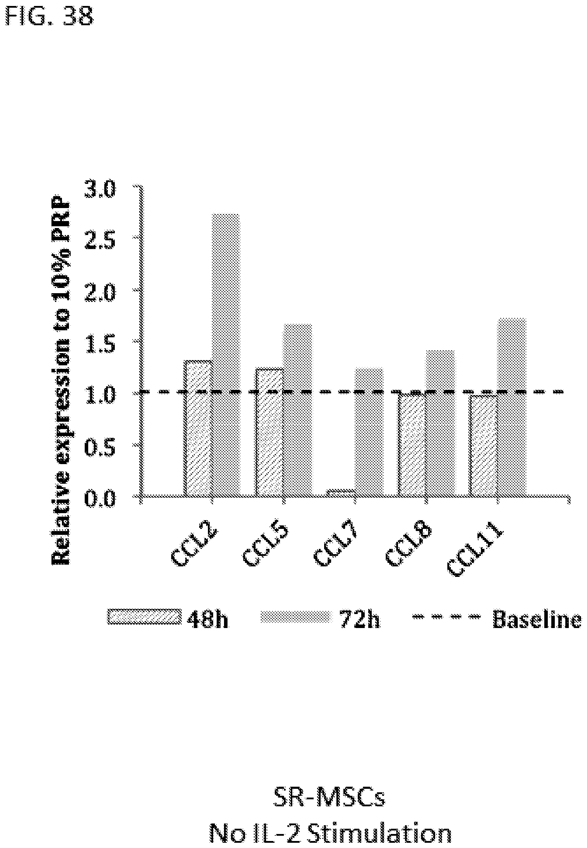

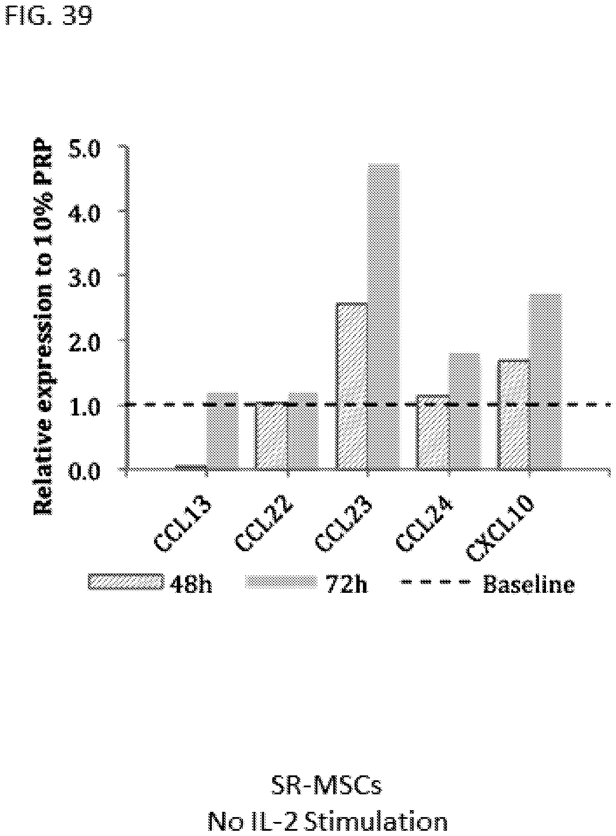

FIGS. 33-39 show the increase in secretion of the below named proteins (factors) from SR-hADSCs, 72 hours post incubation with 10% PRP alone (no IL-2 stimulation). Secretion levels are shown relative to the amount of the corresponding protein present at basal levels in the 10% PRP containing media, used for hADSC support. FIG. 33 shows the increase in secretion of Interleukin 1 beta (IL1β), Interleukin 3 (IL3), Interleukin-13 Receptor subunit alpha-2 (IL13Rα2), and Interleukin 1 receptor alpha (IL1Rα). FIG. 34 shows the increase in secretion of Probetacellulin (BTC), Colony stimulating factor (CSF1), Fibroblast growth factor 6 (FGF6), Glial cell line-derived neurotrophic factor (GDNF), insulin-like growth factor 1 (IGF-1), Leptin, and platelet-derived growth factor B beta (PDGF BB). FIG. 35 shows the increase in secretion of stem cell factor/c-kit ligand (SCF), Stromal Cell-Derived Factor-1 alpha (SDF1a), Stromal Cell-Derived Factor-1 beta (SDF1b), Transforming growth factor beta 1 (TGFβ1), Transforming growth factor beta 3 (TGFb3), and tumor necrosis factor superfamily member 14 (TNFSF14). FIG. 36 shows the increase in secretion of Insulin-like growth factor 1 (IGF1). FIG. 37 shows the increase in secretion of Transforming growth factor beta 1 (TGFβ1) and platelet-derived growth factor B beta (PDGF BB). FIG. 38 shows the increase in secretion of Chemokine (C-C motif) ligand 2 (CCL2), Chemokine (C-C motif) ligand 5 (CCL5), Chemokine (C-C motif) ligand 7 (CCL7), Chemokine (C-C motif) ligand 8 (CCL8), and Chemokine (C-C motif) ligand 11 (CCL11). FIG. 39 shows the increase in secretion of Chemokine (C-C motif) ligand 13 (CCL13), Chemokine (C-C motif) ligand 22 (CCL22), Chemokine (C-C motif) ligand 23 (CCL23), Chemokine (C-C motif) ligand 24 (CCL24), and CXC Chemokine ligand 10 (CXCL10).

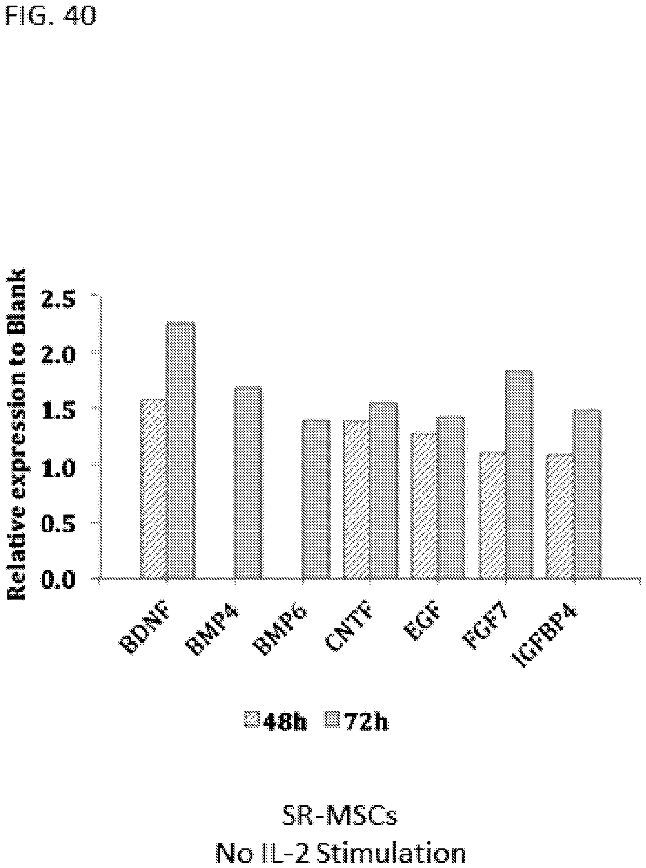

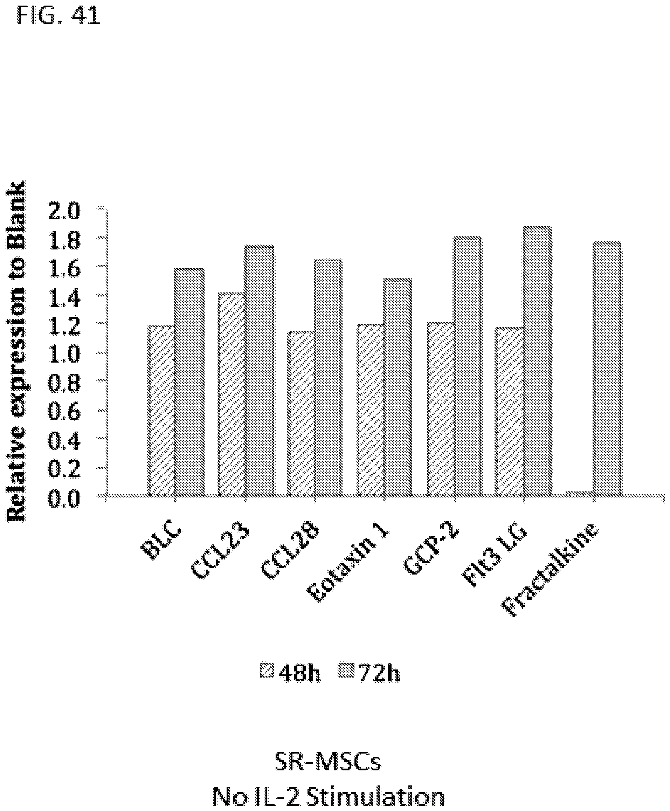

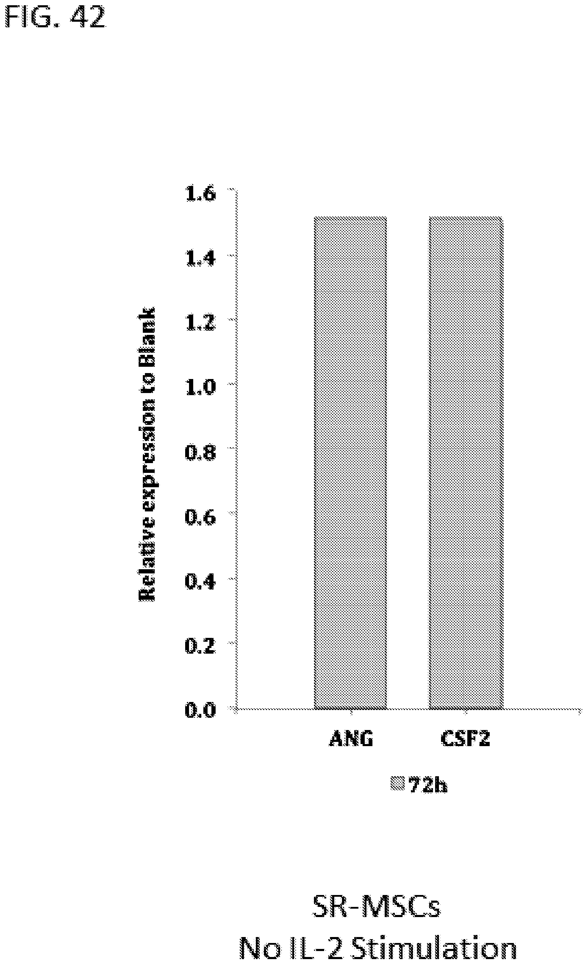

FIGS. 40-42 show the increase in secretion of the below named proteins (factors) from SR-hADSCs, 72 hours post incubation with 10% PRP alone (no IL-2 stimulation). These factors were found to not be present in PRP. FIG. 40 shows the increase in secretion of Brain-derived neurotrophic factor (BDNF), Bone morphogenetic protein 4 (BMP4), Bone morphogenetic protein 6 (BMP6), Ciliary neurotrophic factor (CNTF), Epidermal growth factor (EGF), Fibroblast growth factor 7 (FGF7), and insulin-like growth factor-binding protein-4 (IGFBP4). FIG. 41 shows the increase in secretion of chemokine (C-X-C motif) ligand 13 (BLC), Chemokine (C-C motif) ligand 23 (CCL23), Chemokine (C-C motif) ligand 28 (CCL28), chemokine (C-C motif) ligand 11 (Eotaxin 1), Chemokine (C-X-C motif) ligand 6 (GCP-2), FLT3LG (Fms-Related Tyrosine Kinase 3 Ligand), and Fractalkine (CX3CL1). FIG. 42 shows the increase in secretion of Angiotensin (ANG) and colony stimulating factor 2 (CSF2).

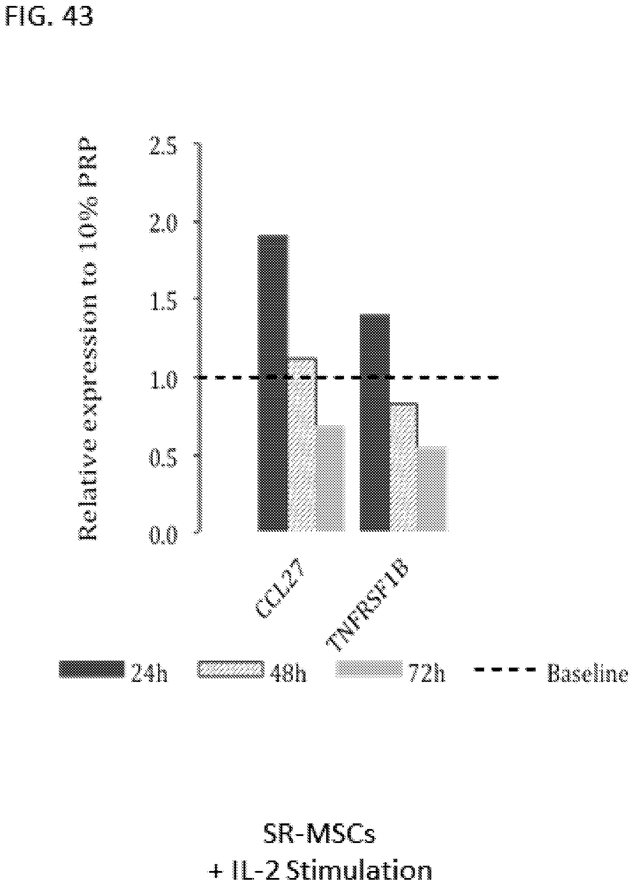

FIG. 43 shows the increase in secretion of Chemokine (C-C motif) ligand 27 (CCL27) and TNFRSF1B (Tumor Necrosis Factor Receptor Superfamily, Member 1B) from SR-hADSCs, 24 hours post stimulation with IL-2. Secretion levels are shown relative to the amount of the corresponding protein present at basal levels in the 10% PRP containing media, used for hADSC support.

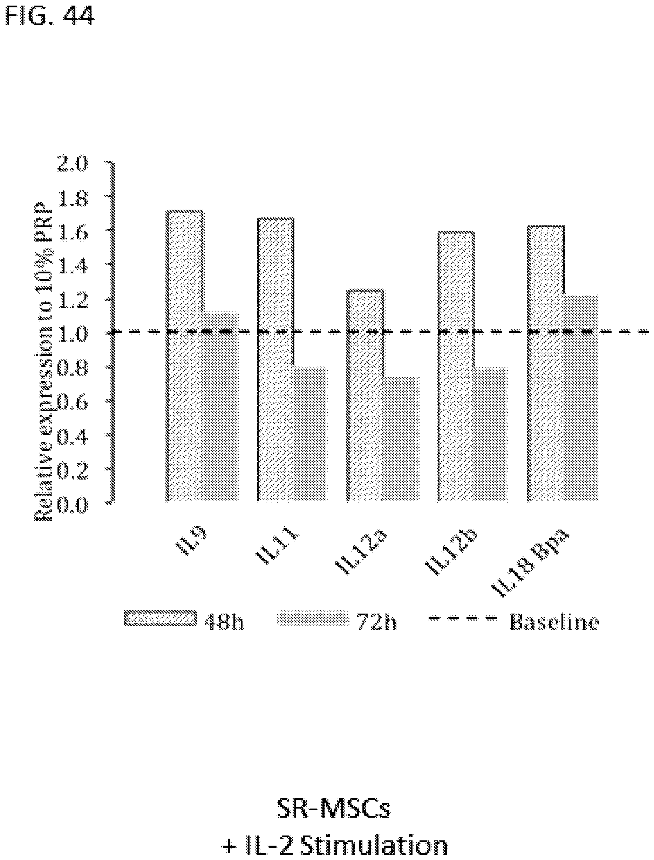

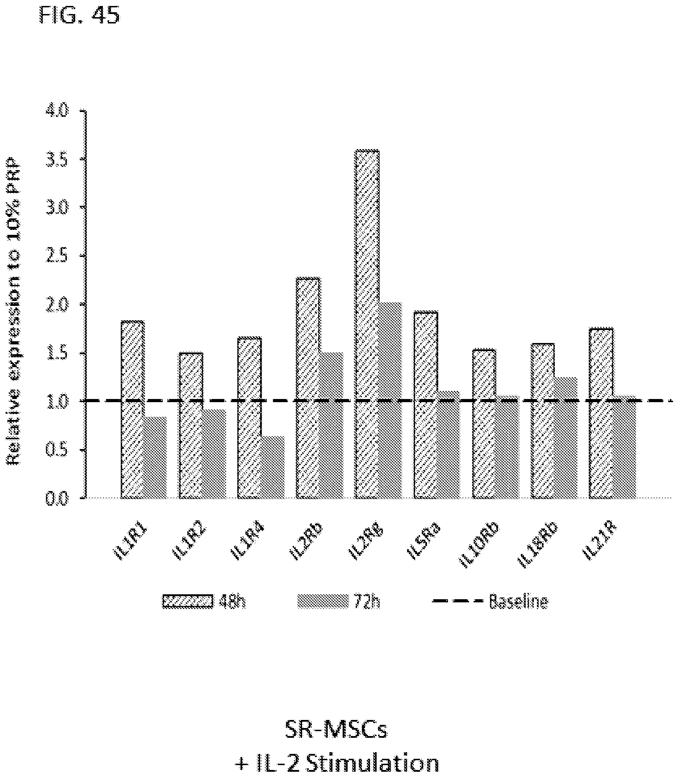

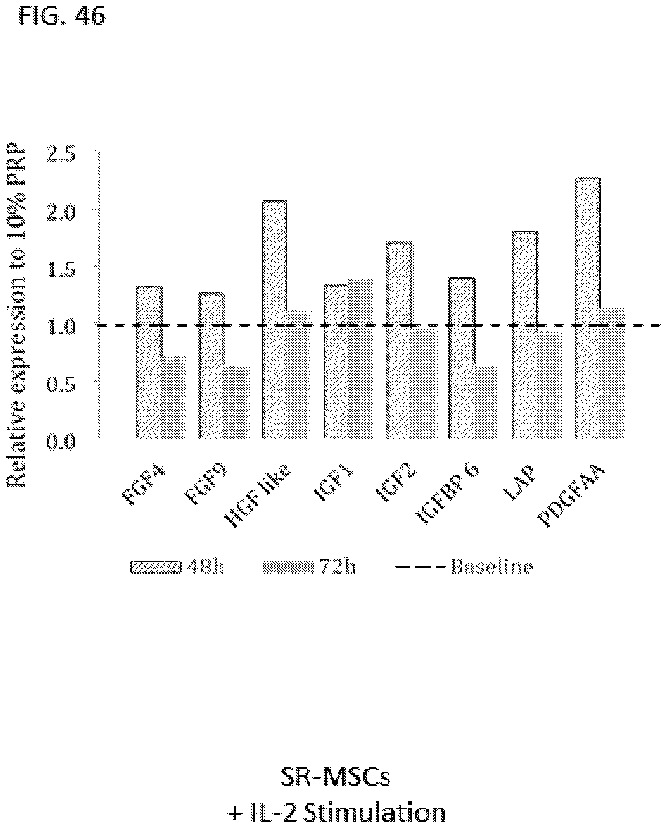

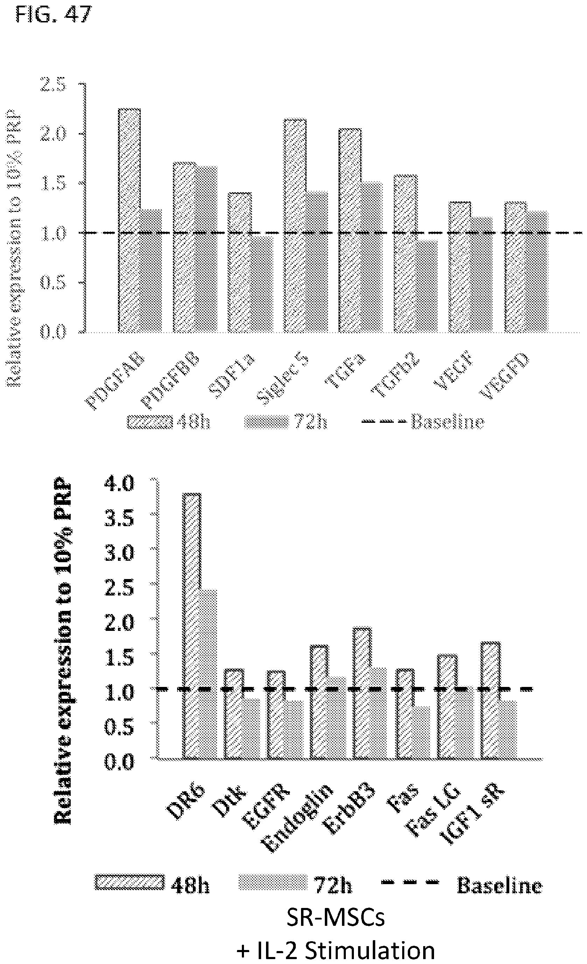

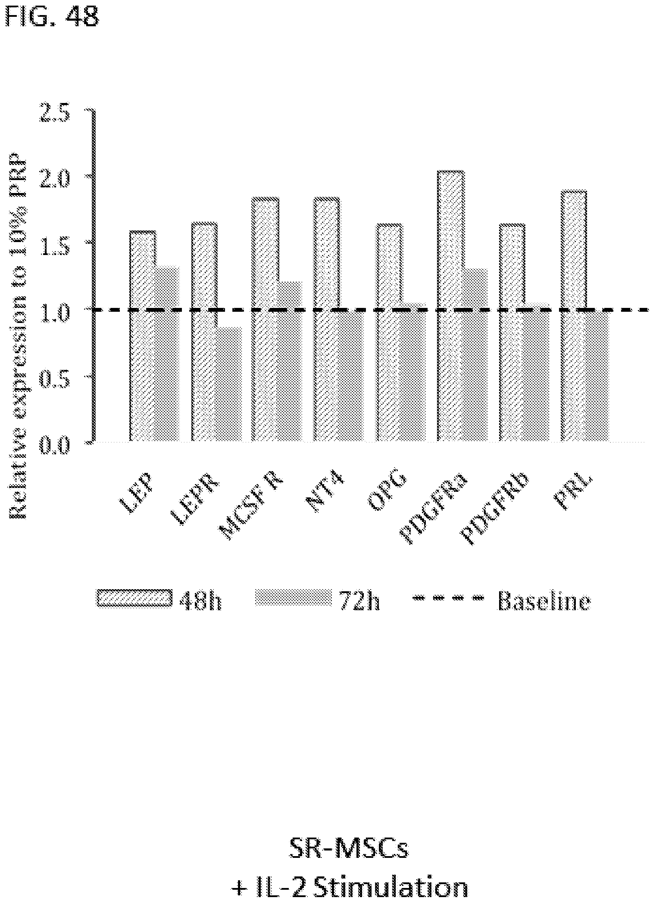

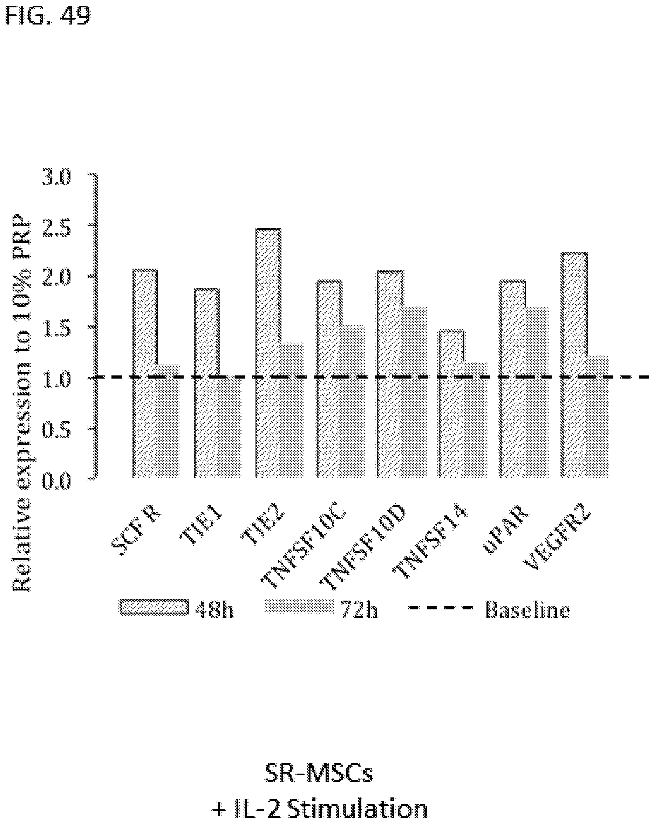

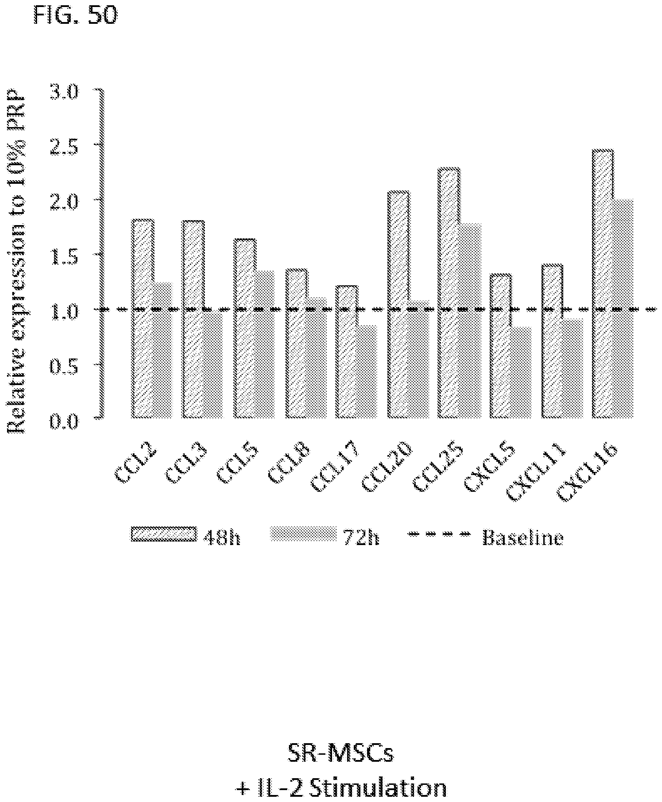

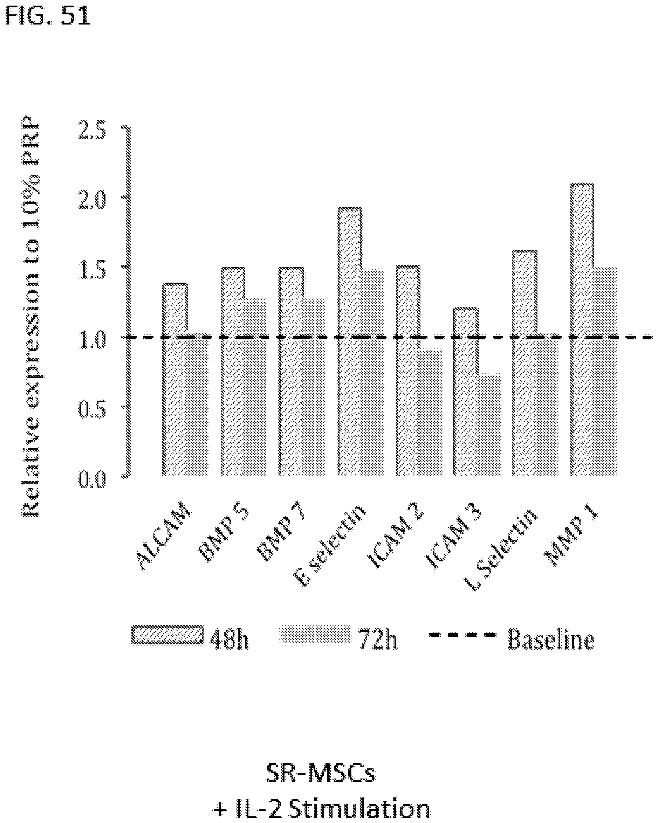

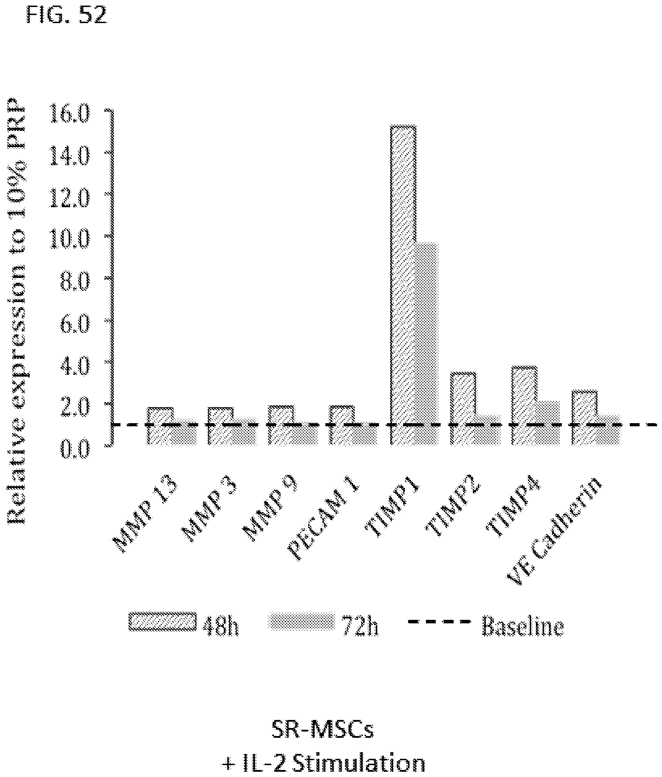

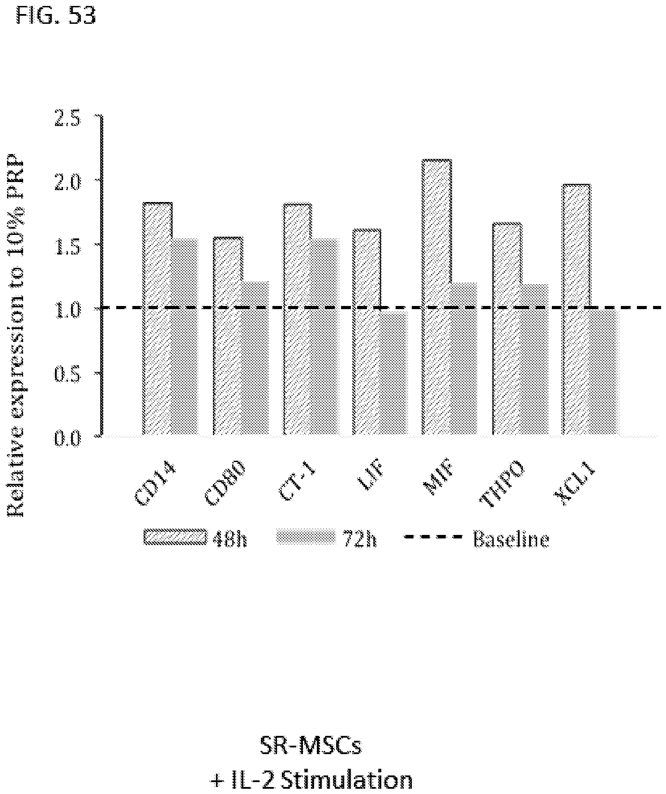

FIGS. 44-53 show the increase of the below named proteins (factors) from SR-hADSCs, 48 hours post stimulation with IL-2. Secretion levels are shown relative to the amount of the corresponding protein present at basal levels in the 10% PRP containing media, used for hADSC support. FIGS. 44 shows the increase in secretion of Interleukin 9 (IL9), Interleukin 11 (IL-11), Interleukin 12 alpha (IL12a), Interleukin 12 beta (IL12b), and Interleukin 18 binding protein alpha (IL18BPa). FIG. 45 shows the increase in secretion of Interleukin 1 receptor type I (IL1R1), Interleukin 1 receptor type II (IL1R2), Interleukin 1 receptor type IV (IL1R4), Interleukin 2 receptor beta (IL-2Rb), Interleukin 2 receptor gamma (IL-2Rg), Interleukin 5 receptor alpha (IL5Ra) Interleukin 10 receptor beta (IL10Rb), Interleukin 18 receptor beta (IL18Rb), and Interleukin 21 receptor (IL-21R). FIG. 46 shows the increase in secretion of Fibroblast growth factor 4 (FGF4), FGF9, MSP alpha/HGF like factor (HGF like), Insulin-like growth factor 1 (IGF1), IGF2, insulin-like growth factor-binding protein-6 (IGFBP6), LAP (TGF beta family), and platelet derived growth factor A alpha (PDGFAA). FIG. 47 shows the increase in secretion of platelet derived growth factor A beta (PDGFAB), platelet derived growth factor B beta (PDGFBB), Stromal Cell-Derived Factor-1 alpha (SDF1a), Sialic acid-binding Ig-like Lectin 5 (Siglec 5), Transforming growth factor alpha (TGFα), Transforming growth factor beta 2 (TGFb2), Vascular endothelial growth factor (VEGF), and Vascular endothelial growth factor D (VEGFD). FIG. 47 also shows the increase in secretion of DR6, Dtk, EGFR, Endoglin, ErbB3, Fas, Fas LG, and IGF1 sr. FIG. 48 shows the increase in secretion of Leptin (LEP), Leptin Receptor (LEPR), Macrophage colony-stimulating factor 1 receptor (MCSFR), Neurotrophin 4 (NT4), Osteoprotegerin (OPG), platelet-derived growth factor receptor alpha (PDGFRa), platelet-derived growth factor receptor beta (PDGFRb), and Prolactin (PRL). FIG. 49 shows the increase in secretion of Stem cell factor receptor (SCFR), Angiopoietin 1 receptor (TIE-1), Angiopoietin 1 receptor (TIE-2), TNF superfamily member 10C (TNFSF10C), TNF superfamily member 10D (TNFSF10D), TNF superfamily member 14 (TNFSF14), urokinase plasminogen activator receptor (uPAR), and Vascular endothelial growth factor receptor-2 (VEGFR2). FIG. 50 shows the increase in secretion of Chemokine (C-C motif) ligand 2 (CCL2), CCL3, CCL5, CCL8, CCL17, CCL20, CCL25, CXC chemokine ligand 5 (CXCL5), CXCL11, and CXCL16. FIG. 51 shows the increase in secretion of activated leukocyte cell adhesion molecule (ALCAM), Bone morphogenetic protein 5 (BMP5), BMP7, E selectin (endothelial cell adhesion molecule), Intercellular adhesion molecule 2 (ICAM2), ICAM3, L selectin (Leukocyte adhesion molecule), and matrix metalloproteinase 1 (MMP1). FIG. 52 shows the increase in secretion of matrix metalloproteinase 13 (MMP13), MMP3, MMP9, Platelet endothelial cell adhesion molecule (PECAM 1), Metalloproteinase inhibitors TIMP 1, TIMP 2, TIMP 4, and vascular epithelium (VE) Cadherin (calcium dependent cell adhesion protein). FIG. 53 shows the increase in secretion of monocyte differentiation antigen (CD14), cell differentiation antigen (CD80), Cardiotrophin-1 (CT-1), Leukemia inhibitory factor (LIF), Macrophage migration inhibitory factor (MIF), Thrombopoietin (THPO), and Lymphotactin (XCL1).

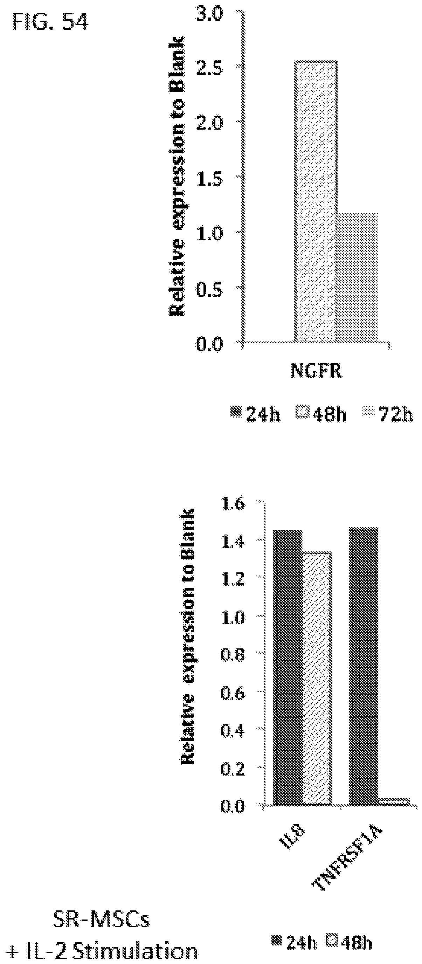

FIG. 54 shows the increase in secretion of Nerve growth factor receptor (NGFR) from SR-hADSCs, 24 or 48 hours post stimulation with IL-2. FIG. 54 also shows the increase in secretion of IL8 and TNFRSF1A. These factors in FIG. 54 were found to not be present in PRP.

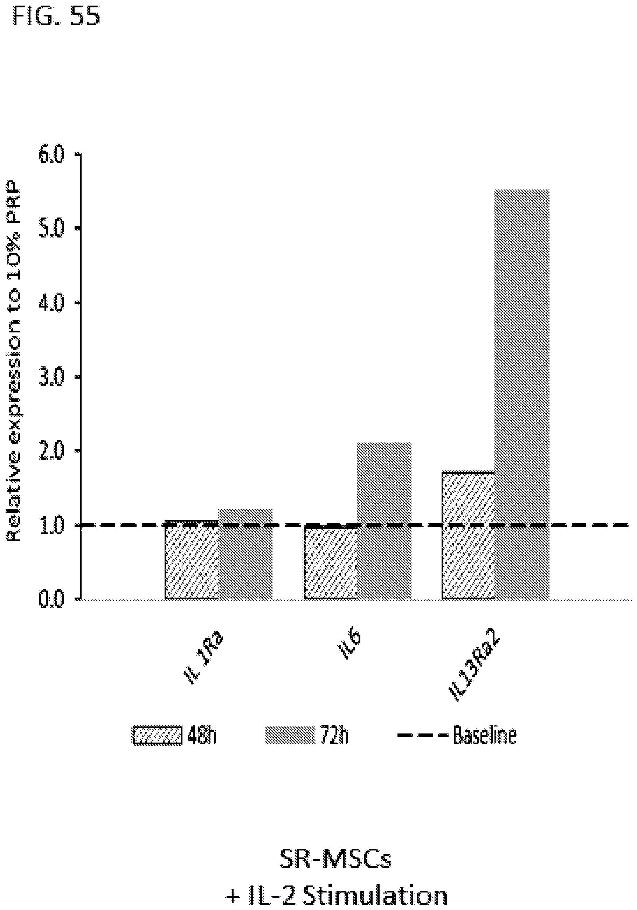

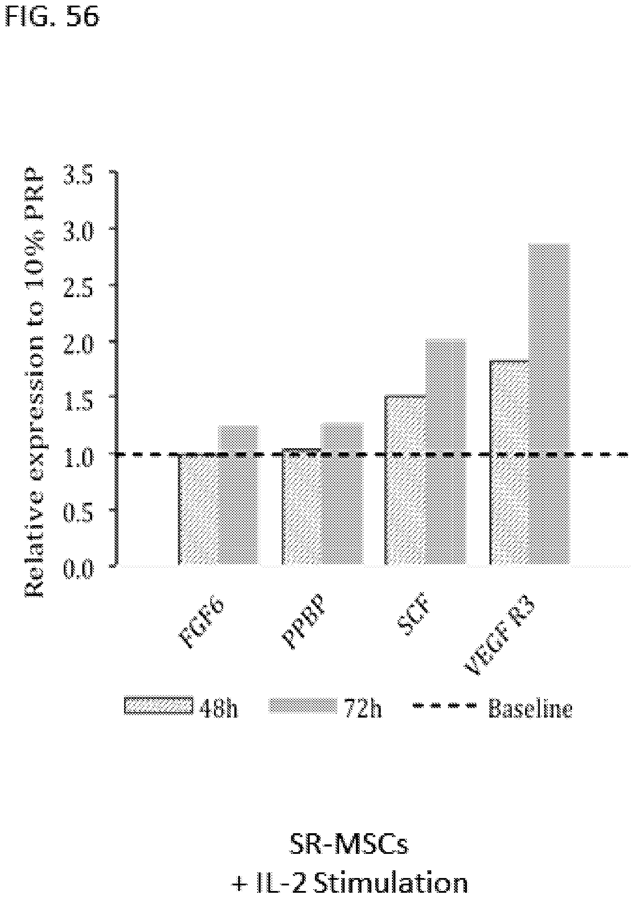

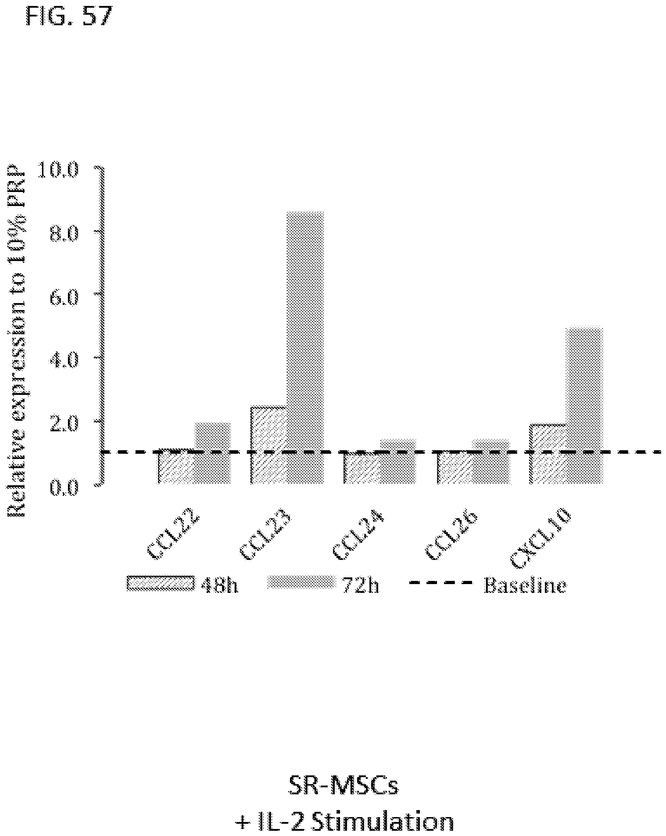

FIGS. 55-57 show the increase in the secretion of the below named proteins (factors) from SR-hADSCs, 72 hours post stimulation with IL-2. Secretion levels are shown relative to the amount of the corresponding protein present at basal levels in the 10% PRP containing media, used for hADSC support. FIG. 55 shows the increase in secretion of Interleukin 1 receptor alpha (IL1Ra), Interleukin 6 (IL6), and Interleukin-13 receptor subunit alpha-2 (IL13Ra2). FIG. 56 shows the increase in secretion of Fibroblast growth factor 6 (FGF6), pro-platelet basic protein (PPBP), stem cell factor (SCF), and Vascular endothelial growth factor receptor-3 (VEGFR3). FIG. 57 shows the increase in secretion of Chemokine (C-C motif) ligand 22 (CCL22), CCL23, CCL24, CCL26, and CXC chemokine ligand 10 (CXCL10).

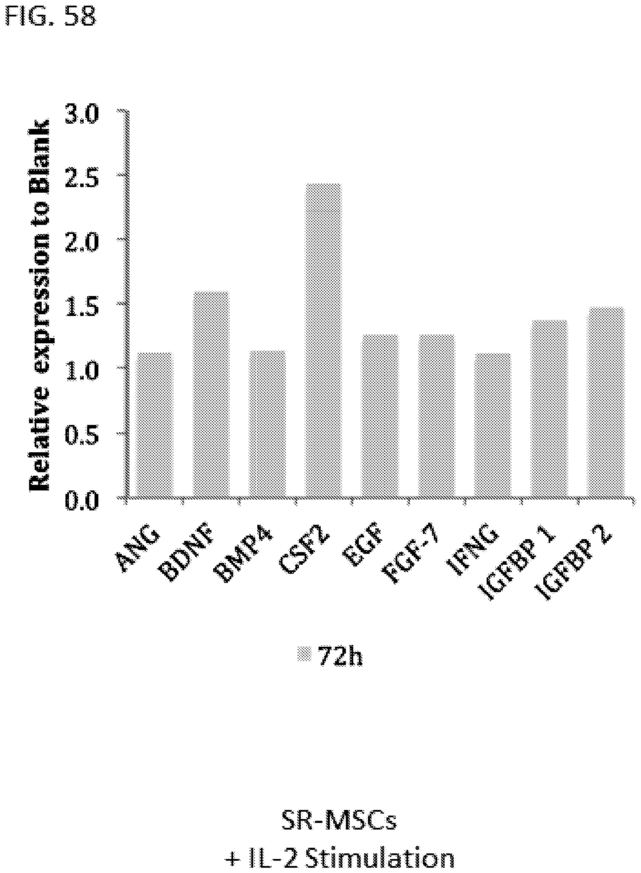

FIG. 58 shows the increase in secretion of Angiotensin (ANG), Brain-derived neurotrophic factor (BDNF), Bone morphogenetic protein 4 (BMP4), colony stimulating factor 2 (CSF2), Epidermal growth factor (EGF), Fibroblast growth factor 7 (FGF-7), Interferon gamma (IFNγ), insulin-like growth factor-binding protein-1 (IGFBP 1), and IGFBP 2 from SR-hADSCs, 72 hours post stimulation with IL-2. These factors were found to not be present in PRP.

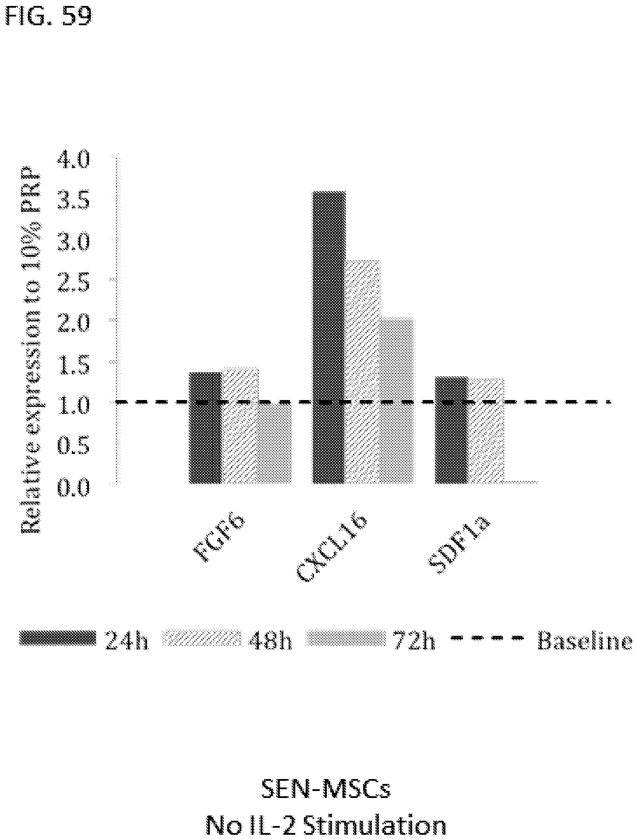

FIG. 59 shows the increase in secretion of Fibroblast growth factor 6 (FGF6), CXC chemokine ligand 16 (CXCL16), and Stromal Cell-Derived Factor-1 alpha (SDF1a) from SEN-hADSCs, 24 hours post incubation with 10% PRP alone (no IL-2 stimulation). Secretion levels are shown relative to the amount of the corresponding protein present at basal levels in the 10% PRP containing media, used for hADSC support.

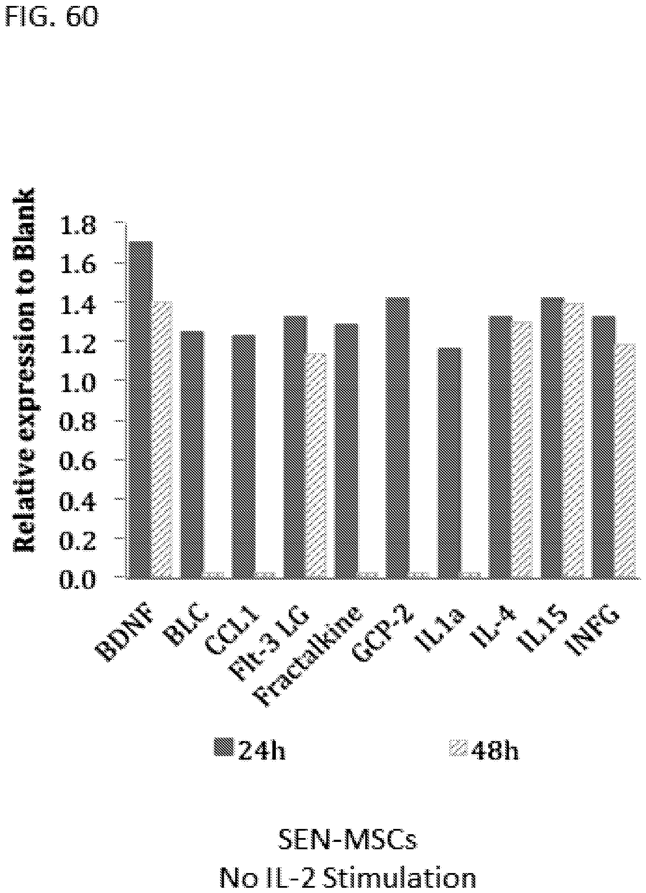

FIG. 60 shows the increase in secretion of Brain-derived neurotrophic factor (BDNF), B lymphocyte chemokine (CXCL13; BLC), Chemokine (C-C motif) ligand 1 (CCL1), Flt-3 LG (Fms-Related Tyrosine Kinase 3 Ligand), Fractalkine (T-cell chemokine CX3CL1), granulocyte chemotactic protein 2 (GCP-2)/CXCL6, Interleukin 1 alpha (IL1a), Interleukin 4 (IL4), IL15, and Interferon gamma (IFNγ) from SEN-hADSCs, 24 hours post incubation with 10% PRP alone (no IL-2 stimulation). These factors were found to not be present in PRP.

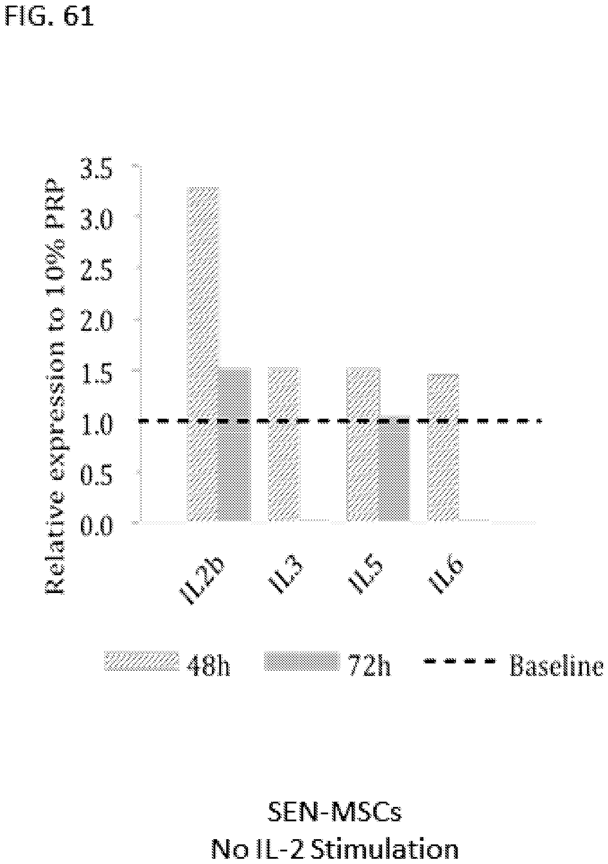

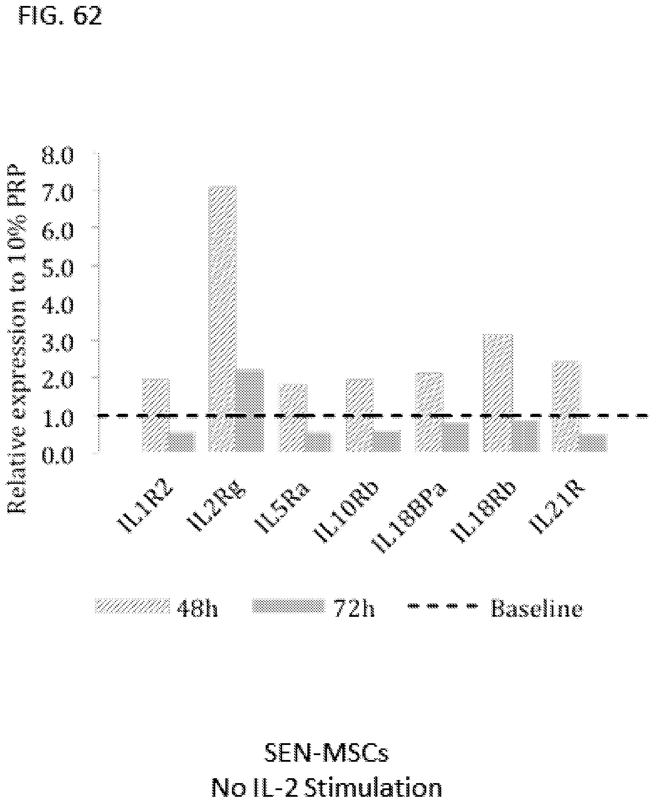

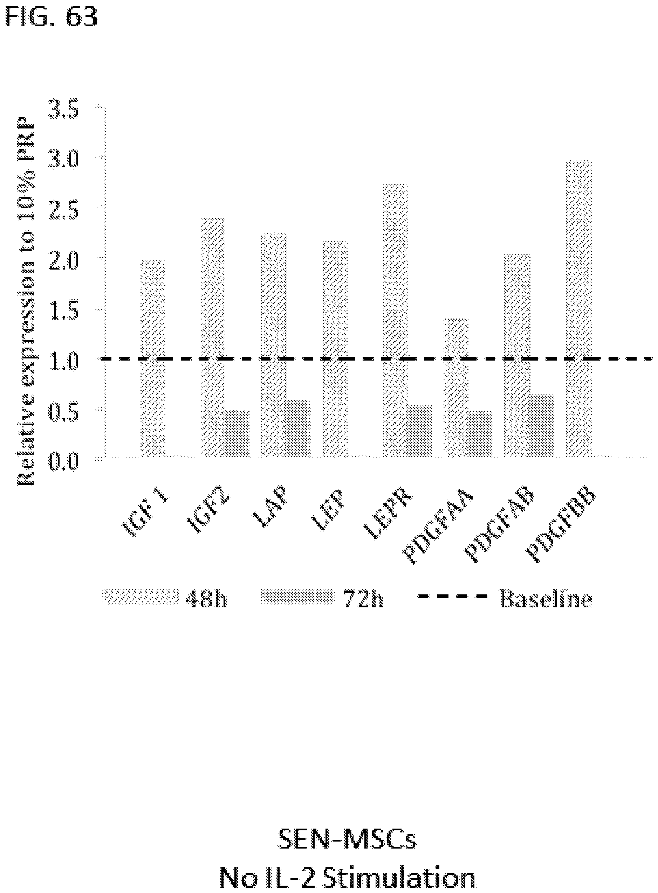

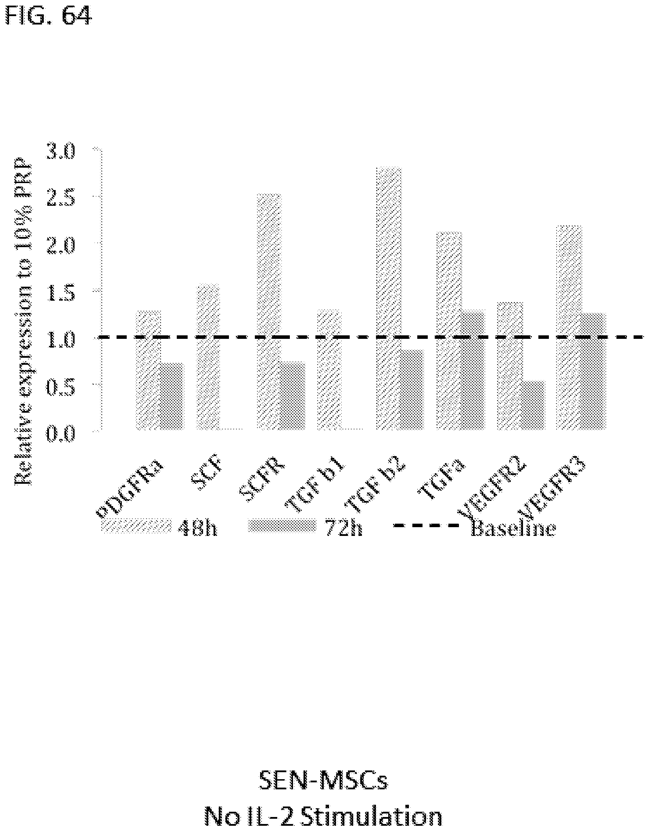

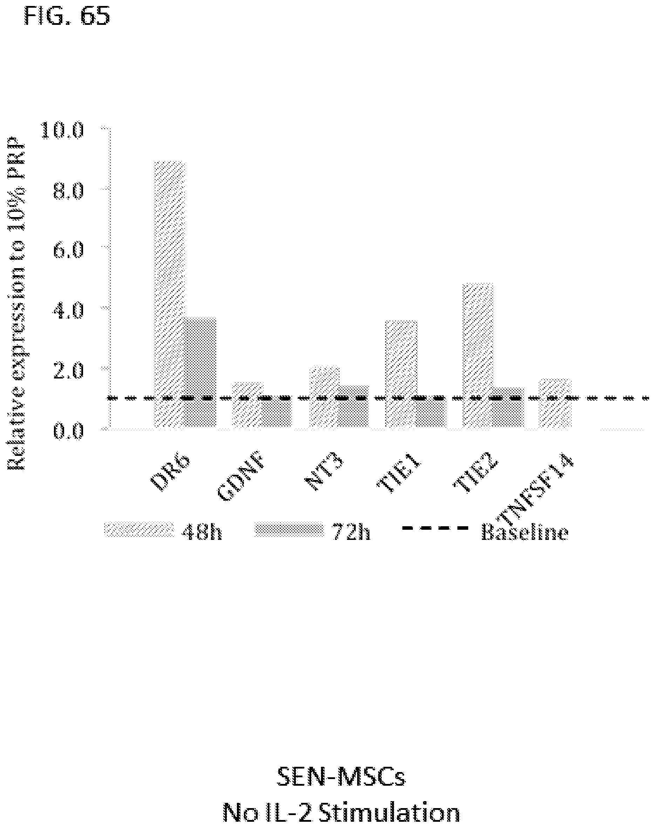

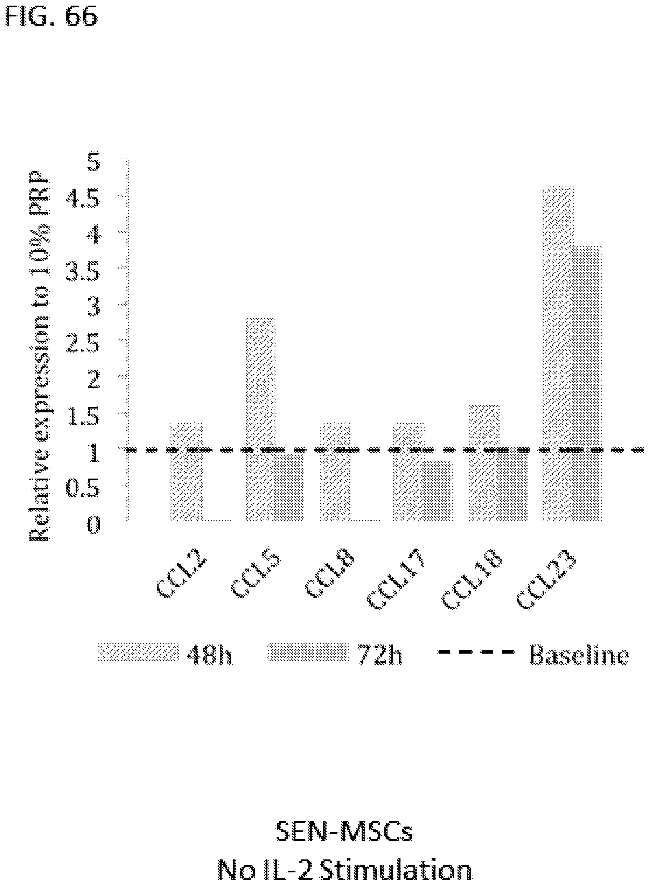

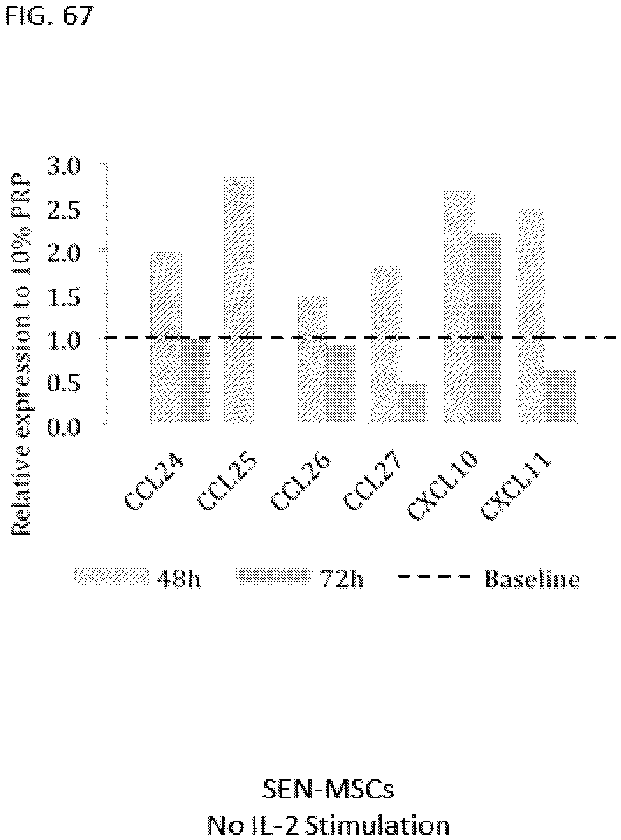

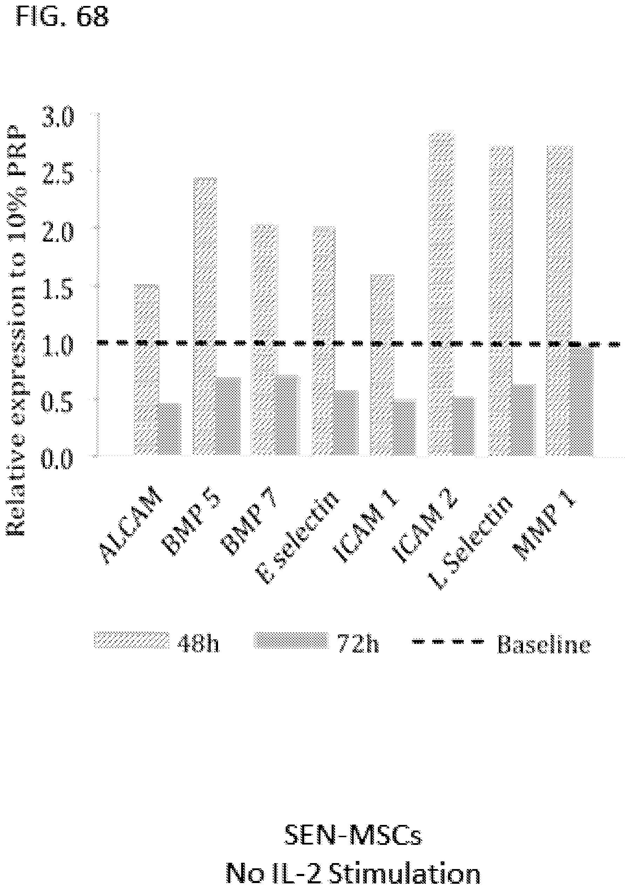

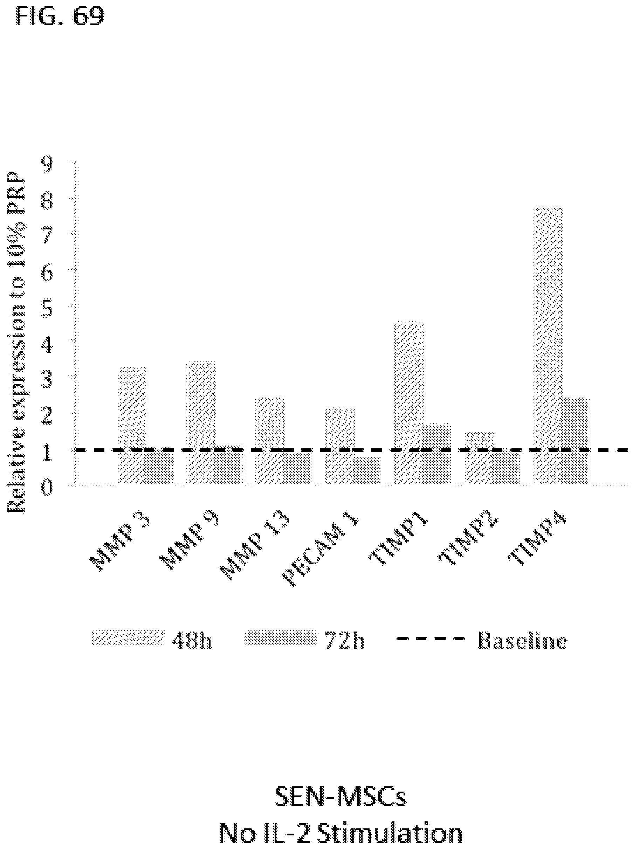

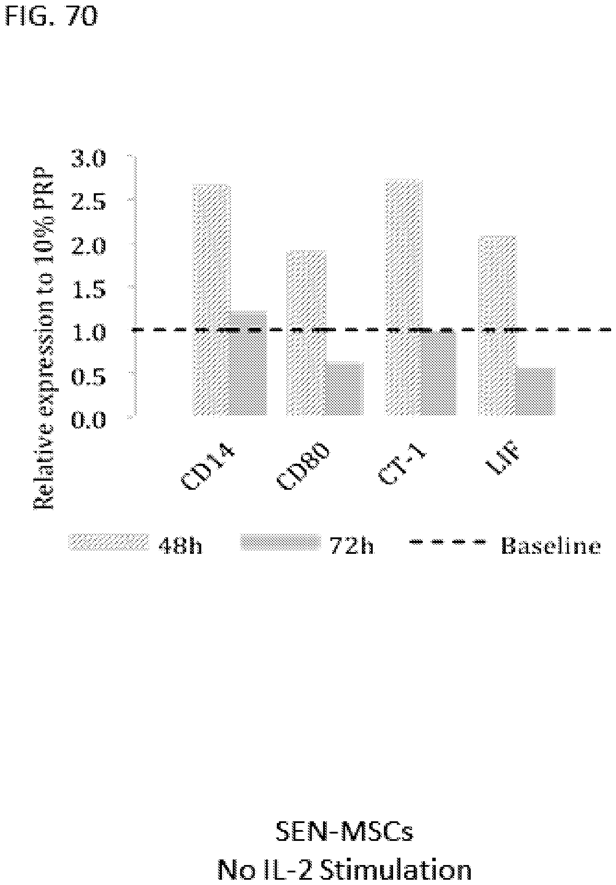

FIGS. 61-70 show the increase in the secretion of the below named proteins (factors) from SEN-hADSCs, 48 hours post incubation with 10% PRP alone (no IL-2 stimulation). Secretion levels are shown relative to the amount of the corresponding protein present at basal levels in the 10% PRP containing media, used for hADSC support. FIG. 61 shows the increase in secretion of Interleukin 2 beta (IL-2b), IL3, IL5, and IL6. FIG. 62 shows the increase in secretion of Interleukin 1 receptor type II (IL1R2), Interleukin 2 receptor gamma (IL-2Rg), Interleukin 5 receptor alpha (IL5Ra), Interleukin 10 receptor beta (IL10Rb), Interleukin 18 receptor binding protein alpha (IL18BPa), Interleukin 18 receptor beta (IL18Rb), and Interleukin 21 receptor (IL-21R).FIG. 63 shows the increase in secretion of Insulin-like growth factor 1 (IGF1), IGF2, LAP (TGF beta family), Leptin (LEP), Leptin Receptor (LEPR), platelet derived growth factor A alpha (PDGFAA), platelet derived growth factor A beta (PDGFAB), and platelet derived growth factor B beta (PDGFBB).FIG. 64 shows the increase in secretion of platelet-derived growth factor receptor alpha (PDGFRa), Stem cell factor (SCF), Stem cell factor receptor (SCFR), Transforming growth factor beta 1 (TGF b1), Transforming growth factor beta 2 (TGF b2), Transforming growth factor alpha (TGFα), Vascular endothelial growth factor receptor-2 (VEGFR2), and VEGFR3.FIG. 65 shows the increase in secretion of Death receptor 6 (DR6; TNF receptor superfamily member 21), Glial cell line-derived neurotrophic factor (GDNF), Neurotrophin 3 (NT3), Tyrosine kinase with immunoglobulin-like and EGF-like domains 1 (TIE-1), TIE-2, and TNF superfamily member 14 (TNFSF14).FIG. 66 shows the increase in secretion of Chemokine (C-C motif) ligand 2 (CCL2), CCL5, CCL8, CCL17, CCL18, and CCL23.FIG. 67 shows the increase in secretion of Chemokine (C-C motif) ligand 24 (CCL24), CCL25, CCL26, CCL27, CXC Chemokine ligand 10 (CXCL10), and CXCL11.FIG. 68 shows the increase in secretion of activated leukocyte cell adhesion molecule (ALCAM), Bone morphogenetic protein 5 (BMP5), BMP7, E selectin (endothelial cell adhesion molecule), Intercellular adhesion molecule 1 (ICAM1), ICAM2, L selectin (Leukocyte adhesion molecule), and matrix metalloproteinase 1 (MMP1).FIG. 69 shows the increase in secretion of matrix metalloproteinase 3 (MMP3), MMP9, MMP13, Platelet endothelial cell adhesion molecule (PECAM 1), Metalloproteinase inhibitors TIMP 1, TIMP 2, and TIMP 4.FIG. 70 shows the increase in secretion of monocyte differentiation antigen (CD14), monocyte differentiation antigen (CD80), Cardiotrophin-1 (CT-1), and Leukemia inhibitory factor (LIF).

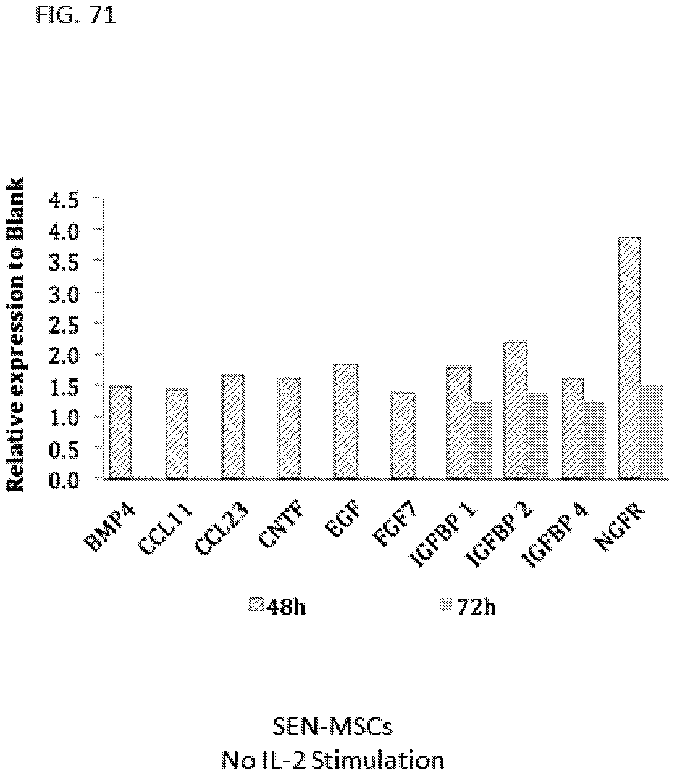

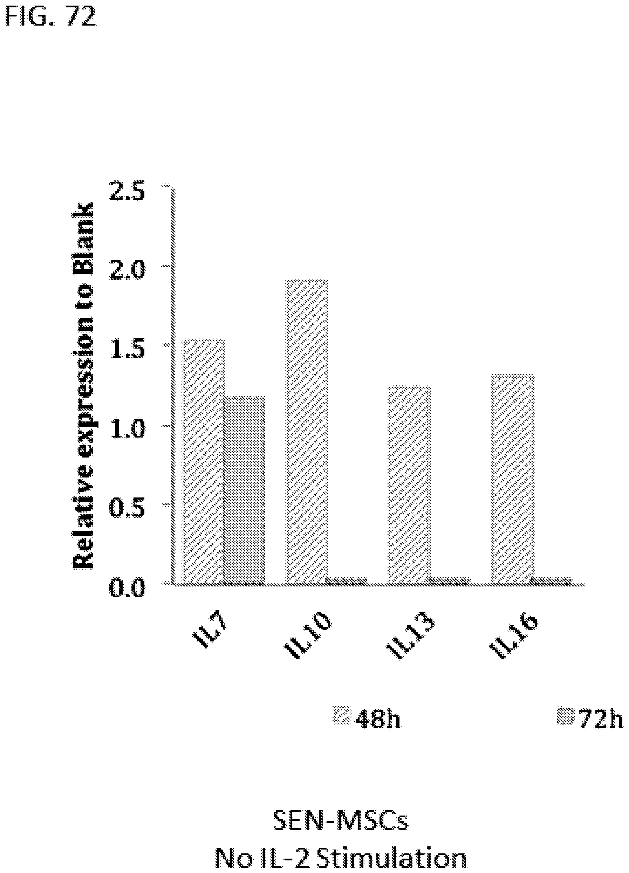

FIGS. 71-72 show the increase in the secretion the below named proteins (factors) from SEN-hADSCs, 48 hours post incubation with 10% PRP alone (no IL-2 stimulation). These factors were found to not he present in PRP. FIG. 71 shows the increase in secretion of Bone morphogenetic protein 4 (BMP4), Chemokine (C-C motif) ligand 11 (CCL11), CCL23, Ciliary neurotrophic factor (CNTF), Epidermal growth factor (EGF), Fibroblast growth factor 7 (FGF7), insulin-like growth factor-binding protein-1 (IGFBP1), IGFBP2, IGFBP4, and Nerve growth factor receptor (NGFR). FIG. 72 shows the increase in secretion of Interleukin 7 (IL7), IL10, IL13, and IL-16.

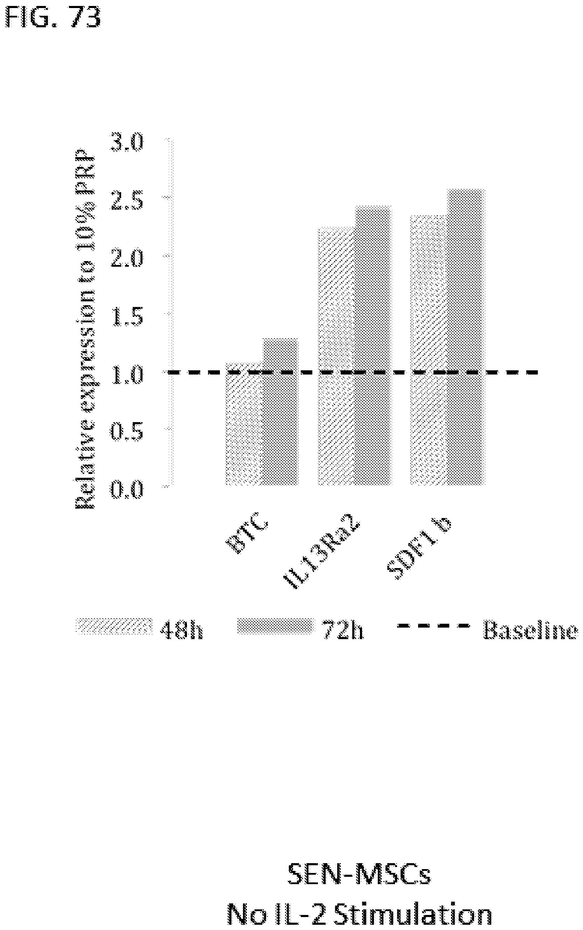

FIG. 73 shows the increase in secretion of Probetacellulin (BTC), Interleukin-13 receptor subunit alpha-2 (IL13Ra2), and Stromal Cell-Derived Factor-1 beta (SDF1b) from SEN-hADSCs, 72 hours post incubation with 10% PRP alone (no IL-2 stimulation). Secretion levels are shown relative to the amount of the corresponding protein present at basal levels in the 10% PRP containing media, used for hADSC support.

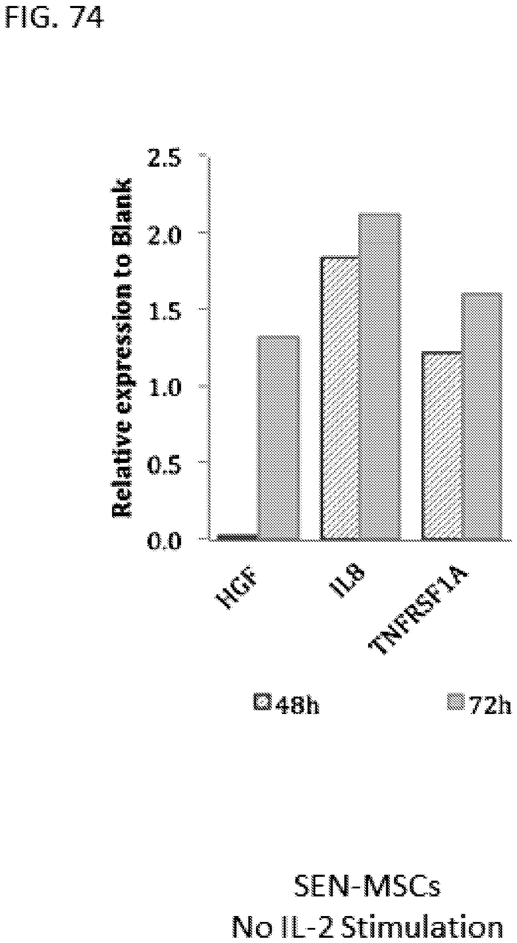

FIG. 74 shows the increase in secretion of Hepatocyte growth factor (HGF), Interleukin 8 (IL8), and TNFRSF1A (Tumor Necrosis Factor Receptor Superfamily, member 1A) from SEN-hADSCs, 72 hours post incubation with 10% PRP alone (no IL-2 stimulation). These factors were found to not be present in PRP.

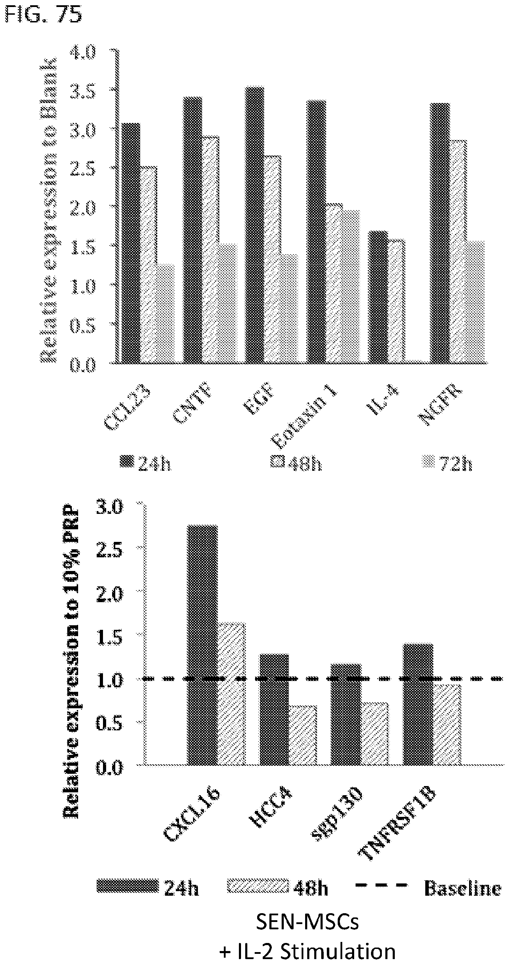

FIG. 75 shows the increase in secretion of Chemokine (C-C motif) ligand 23 (CCL23), Ciliary neurotrophic factor (CNTF), Epidermal growth factor (EGF), CCL11 (Eotaxin 1), IL4, and Nerve growth factor receptor (NGFR) from SEN-hADSCs, 24 hours post stimulation with IL-2. These factors were found to not be present in PRP.

FIG. 75 also shows the increase in secretion of CXCL16, HCC4, sgp130, and TNFRSF1B at 24h post IL-2 stimulation. Secretion levels are shown relative to the amount of the corresponding protein present at basal levels in the 10% PRP containing media.

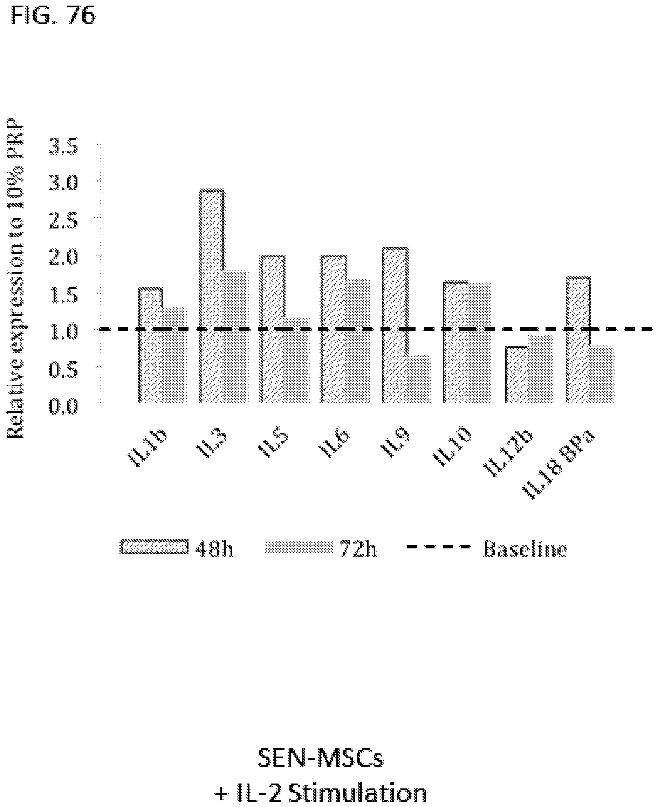

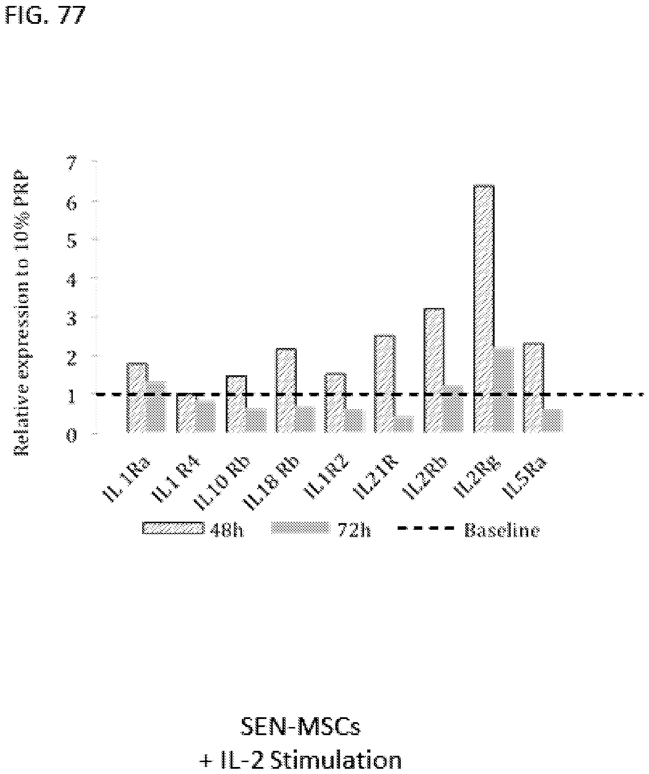

FIGS. 76-86 show the increase in the secretion of the below named proteins (factors) from SEN-hADSCs, 48 hours post stimulation with IL-2. Secretion levels are shown relative to the amount of the corresponding protein present at basal levels in the 10% PRP containing media, used for hADSC support. FIG. 76 shows the increase in secretion of Interleukin 1 beta (IL1β), IL3, IL5, IL6, IL9, IL10, IL12b, and Interleukin 18 binding protein alpha (IL18BPa). FIG. 77

30

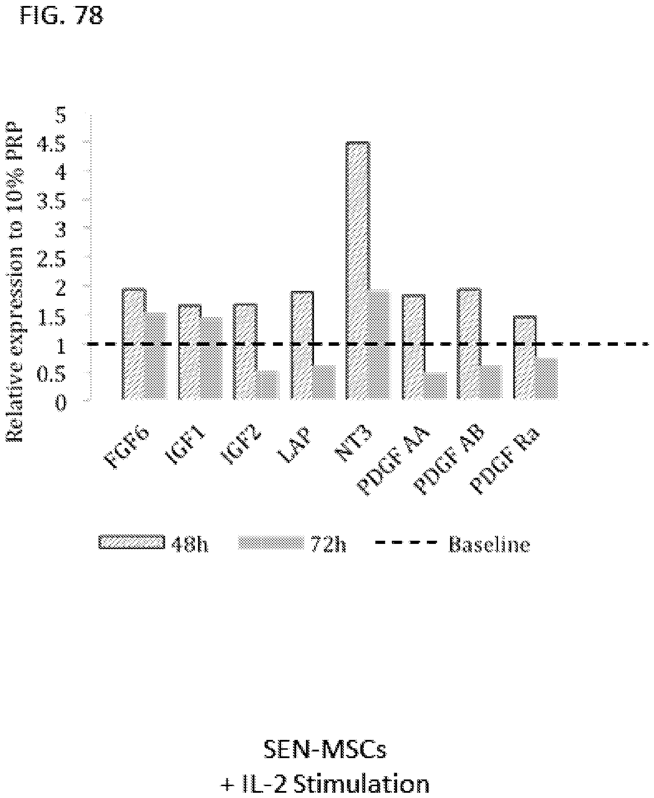

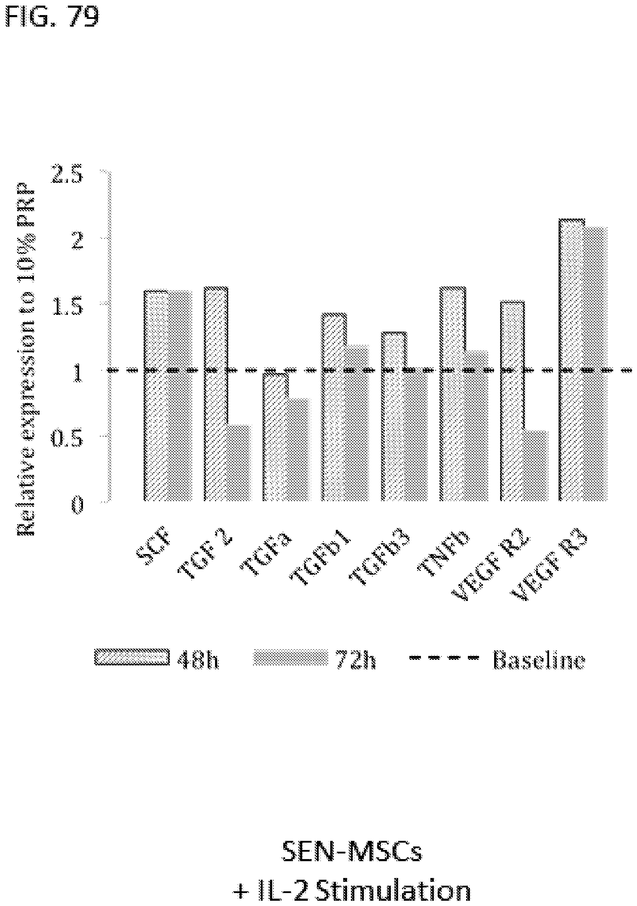

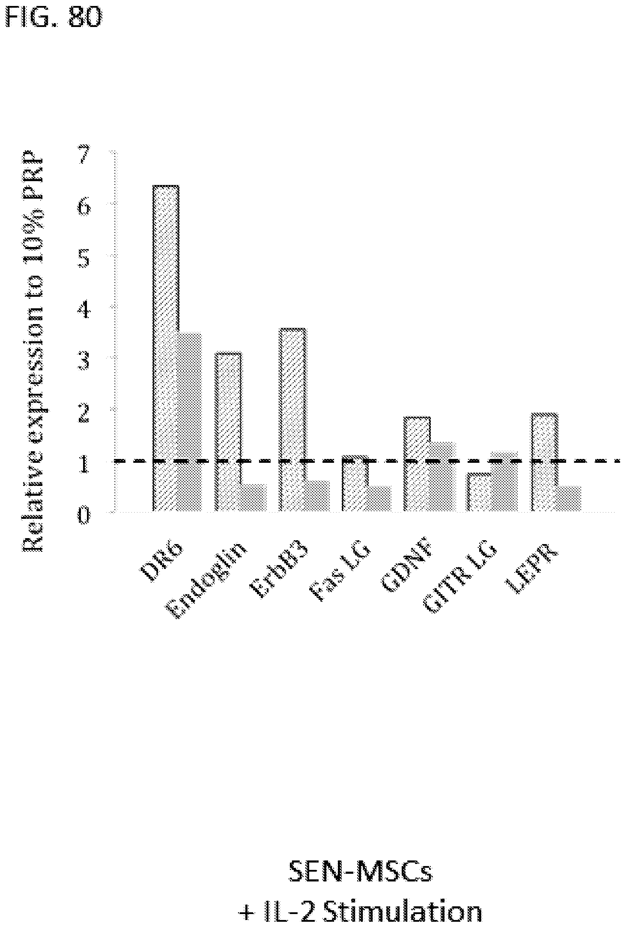

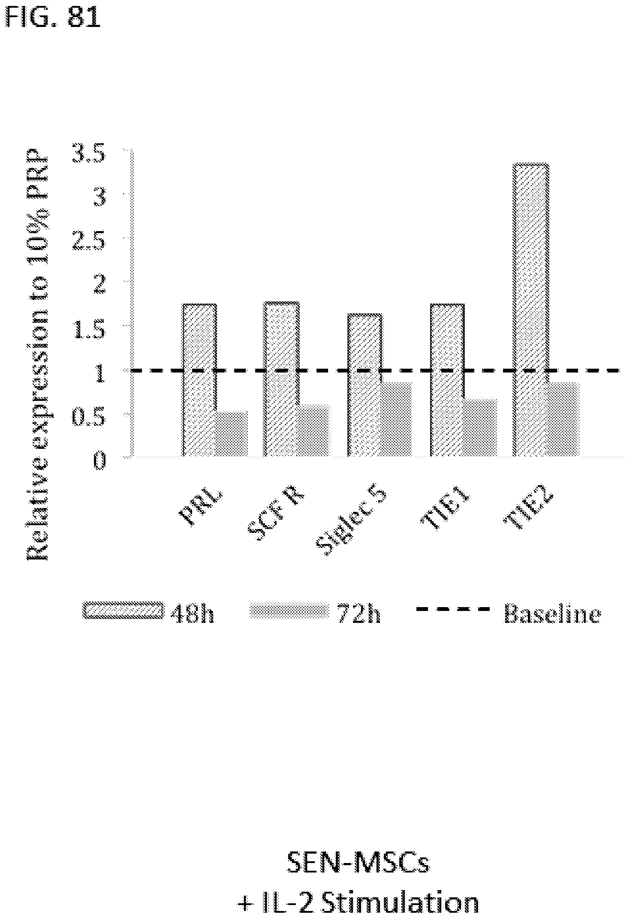

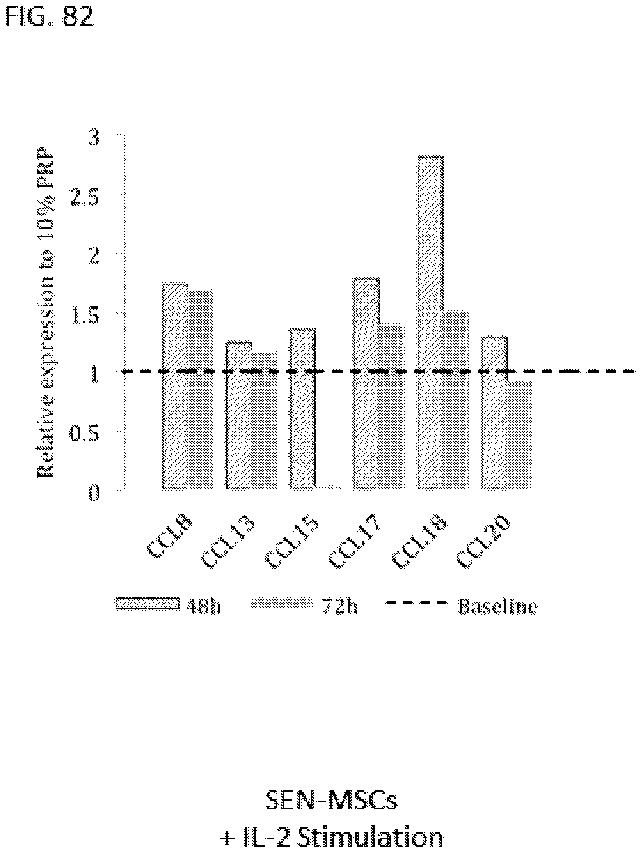

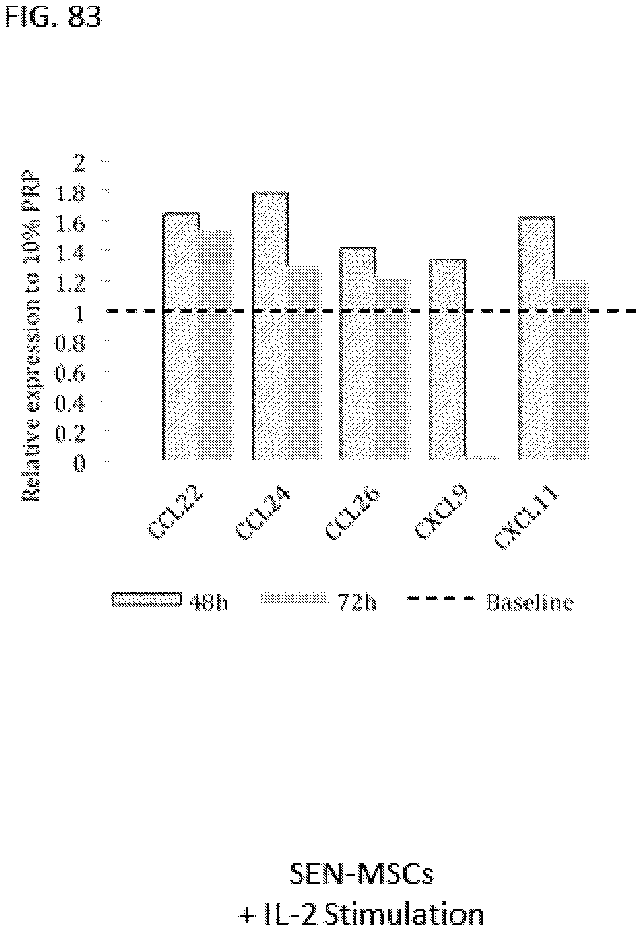

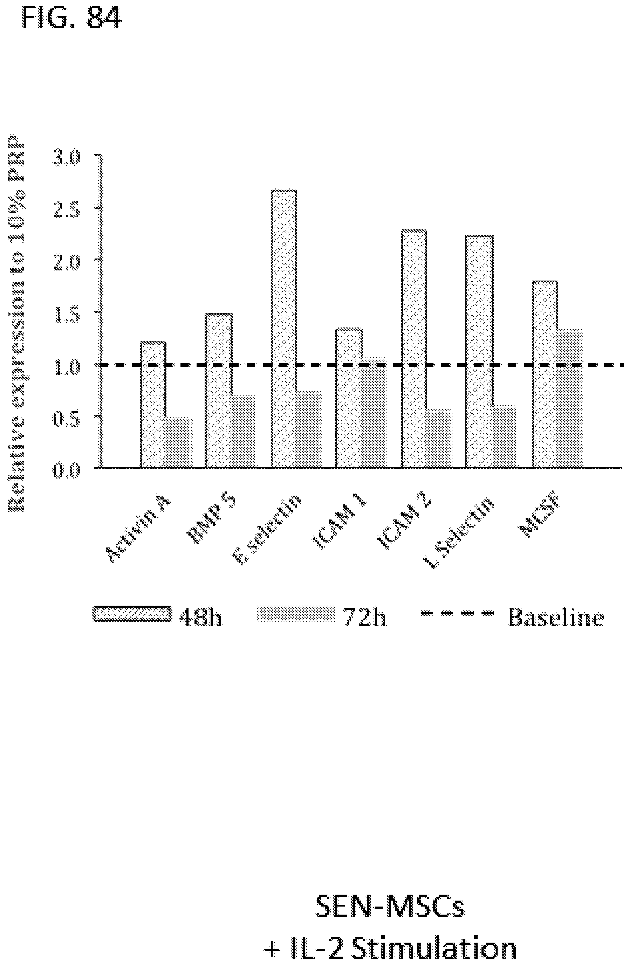

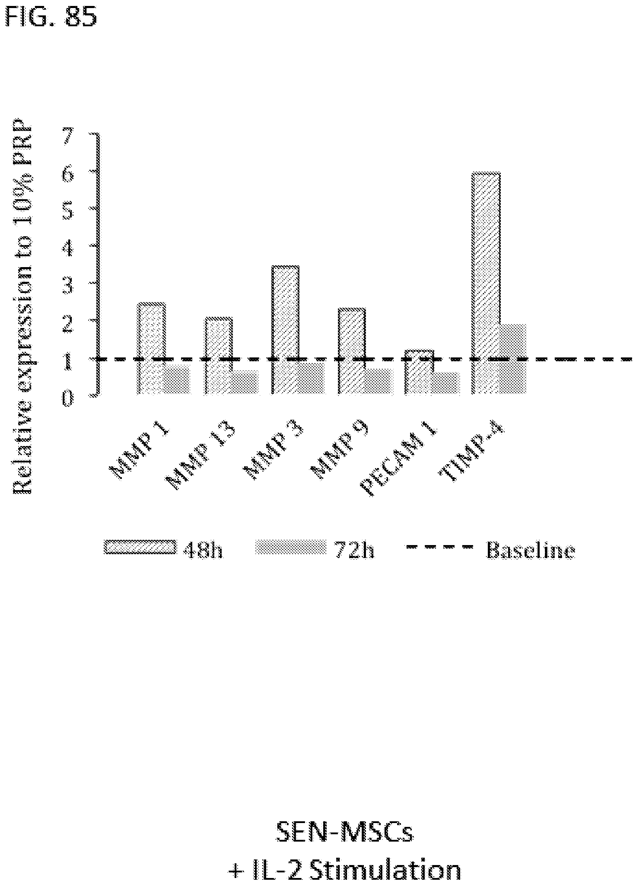

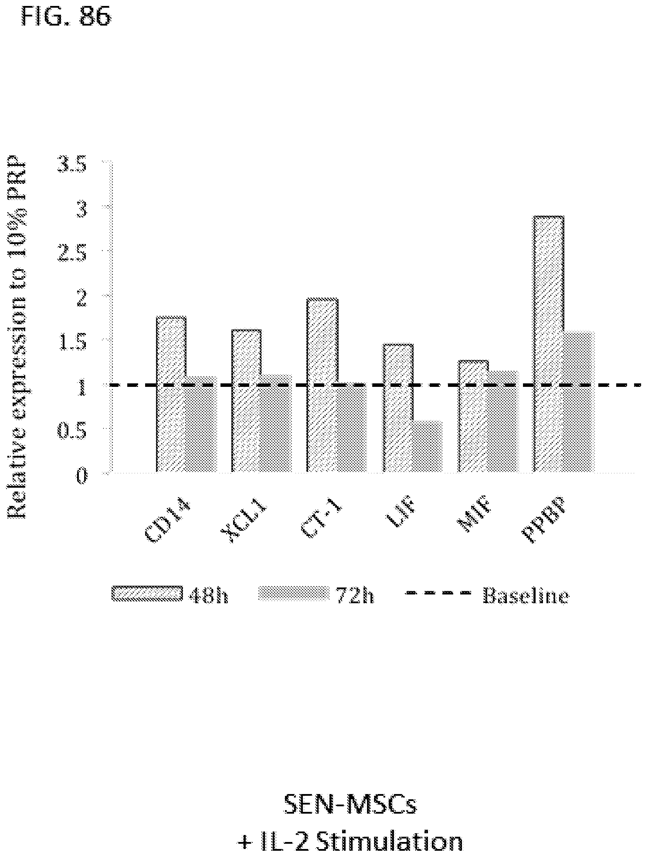

shows the increase in secretion of Interleukin 1 receptor alpha (IL1Ra), IL1R4, IL10Rb, IL18Rb, IL1R2, IL-21R, IL-2RP, IL-2Ry, and IL5Ra. FIG. 78 shows the increase in secretion of Fibroblast growth factor 6 (FGF6), insulin-like growth factors IGF1 and IGF2, LAP (TGF beta family), Neurotrophin 3 (NT3), platelet derived growth factor A alpha (PDGFAA), platelet derived growth factor A beta (PDGFAB), and platelet-derived growth factor receptor alpha (PDGFRa). FIG. 79 shows the increase in secretion of Stem cell factor (SCF), Transforming growth factor 2 (TGF2), TGFα, TGFβ1, TGFb3, Tumor necrosis factor beta (TNFb), Vascular endothelial growth factor receptor-2 (VEGF R2), and VEGF R3. FIG. 80 shows the increase in secretion of DR6 (TNF receptor superfamily member 21), Endoglin (ENG), Receptor tyrosine-protein kinase erbB-3 (ErbB3), Fas ligand (Fas LG), Glial cell line-derived neurotrophic factor (GDNF), GITR ligand (GITR LG), and Leptin receptor (LEPR). FIG. 81 shows the increase in secretion of Prolactin (PRL), Stem cell factor receptor (SCFR), Sialic acid-binding Ig-like Lectin 5 (Siglec 5), Angiopoietin 1 receptor (TIE-1), and Angiopoietin 1 receptor (TIE-2). FIG. 82 shows the increase in secretion of Chemokine (C-C motif) ligand 8 (CCL8), CCL13, CCL15, CCL17, CCL18, and CCL20. FIG. 83 shows the increase in secretion of Chemokine (C-C motif) ligand 22 (CCL22), CCL24, CCL26, CXC chemokine ligand 9 (CXCL9), and CXCL11. FIG. 84 shows the increase in secretion of Activin A (INHBA), Bone morphogenetic protein 5 (BMP5), E selectin (endothelial cell adhesion molecule), Intercellular adhesion molecule 1 (ICAM 1), ICAM 2, L selectin (Leukocyte adhesion molecule), and Macrophage colony-stimulating factor (MCSF). FIG. 85 shows the increase in secretion of matrix metalloproteinase 1 (MMP1), MMP13, MMP3, MMP9, Platelet endothelial cell adhesion molecule (PECAM 1), and Metalloproteinase inhibitor 4 (TIMP-4). FIG. 86 shows the increase in secretion of monocyte differentiation antigen (CD14), Lymphotactin (XCL1), Cardiotrophin-1 (CT-1), Leukemia inhibitory factor (LIF), Macrophage Migration Inhibitory Factor (MIF), and pro-platelet basic protein (PPBP).

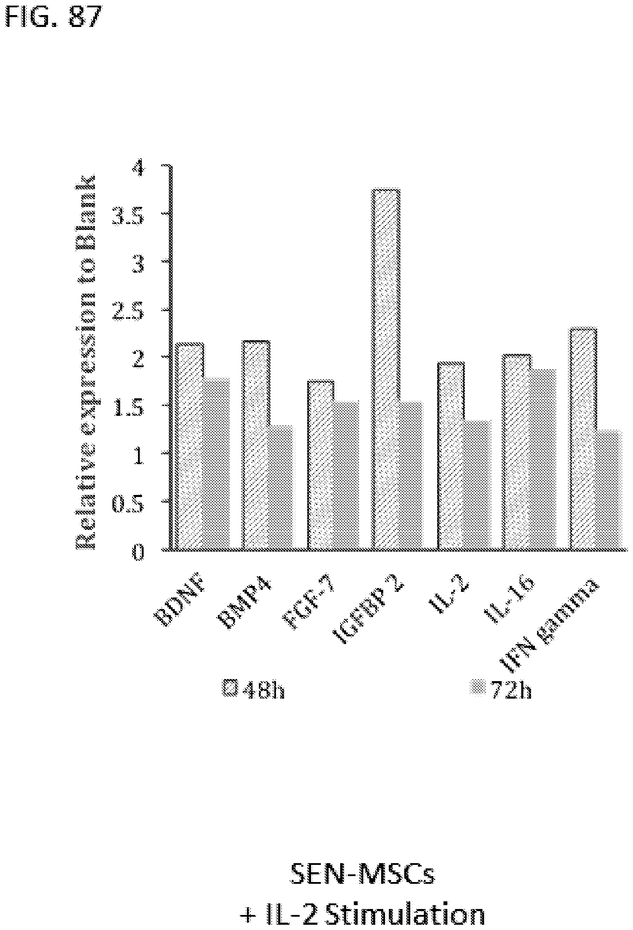

FIG. 87 shows the increase in secretion of Brain-derived neurotrophic factor (BDNF), Bone morphogenetic protein 4 (BMP4), Fibroblast growth factor 7 (FGF7), insulin-like growth factor-binding protein-2 (IGFBP2), IL-2, IL-16, and Interferon gamma (INF gamma) from SEN-hADSCs, 48 hours post stimulation with IL-2. These factors were found to not be present in PRP.

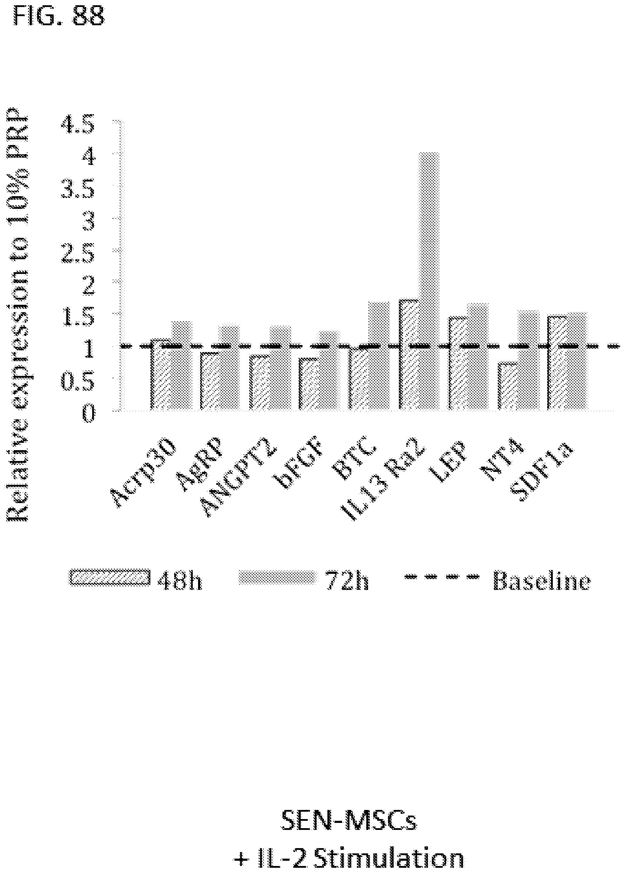

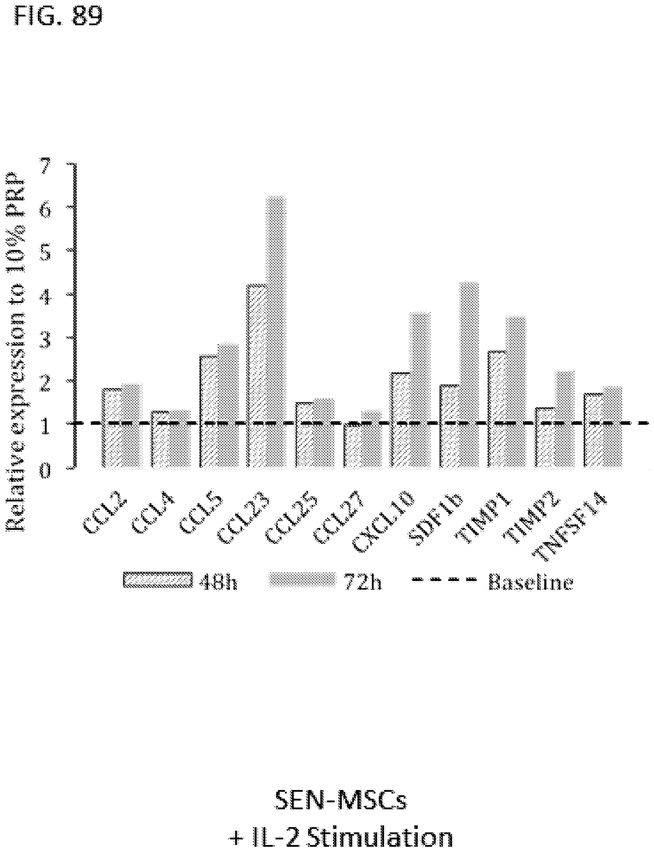

FIGS. 88-89 show the increase in secretion of the below named proteins (factors) from SEN-hADSCs, 72 hours post stimulation with IL-2. Secretion levels are shown relative to the amount of the corresponding protein present at basal levels in the 10% PRP containing media, used for hADSC support. FIG. 88 shows the increase in secretion of Adiponectin (Acrp30), Agouti-related protein (AgRP), ANGPT2 (Angiopoietin 2), basic-Fibroblast Growth Factor (bFGF), Probetacellulin (BTC), Interleukin-13 receptor subunit alpha-2 (IL13Ra2), Leptin (LEP), Neurotrophin 4 (NT4), and Stromal Cell-Derived Factor-1 alpha (SDF1a). FIG. 89 shows the increase in secretion of Chemokine (C-C motif) ligand 2 (CCL2), CCL4, CCL5, CCL23, CCL25, CCL27, CXC Chemokine ligand 10 (CXCL10), Stromal Cell-Derived Factor-1 beta (SDF1b), Metalloproteinase inhibitors 1 (TIMP1), TIMP2, and tumor necrosis factor superfamily member 14 (TNFSF14).

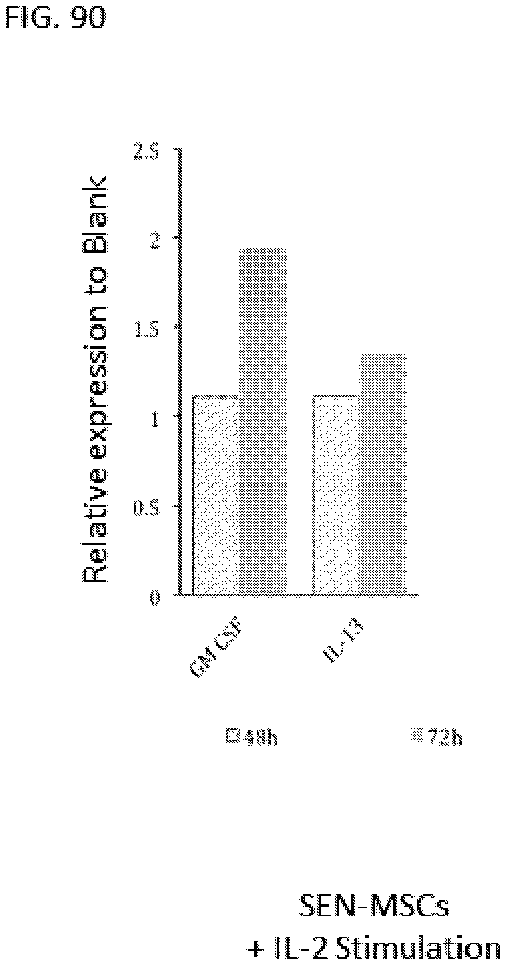

FIG. 90 shows the increase in secretion of Granulocyte-macrophage colony-stimulating factor (GM-CSF) and IL13 from SEN-hADSCs, 72 hours post stimulation with IL-2. These factors were found to not be present in PRP.

**Table 5A: Differentially Upregulated Genes in SR + IL-2**

| Table 5A: Differentially Upregulated Genes in SR + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2+ |
| NM_015665 | AAAS | 0.24 | 0.00 | 622 | 830 | 1129 | 1229 |
| NM_023928 | AACS | 0.09 | 0.00 | 863 | 1023 | 1254 | 1336 |
| NM_001286682 | AADAT | 0.15 | 0.00 | 59 | 96 | 283 | 275 |
| NM_001087 | AAMP | 0.20 | 0.00 | 2016 | 2486 | 3001 | 2832 |
| NM_015511 | AAR2 | 0.40 | 0.00 | 938 | 1367 | 1922 | 1835 |
| NM_020745 | AARS2 | 0.06 | -0.07 | 476 | 575 | 736 | 616 |
| NM_001261434 | AARSD1 | 0.16 | -0.03 | 274 | 369 | 518 | 436 |
| NM_001271696 | ABCB7 | 0.12 | -0.02 | 153 | 212 | 406 | 338 |
| NM_001198934 | ABCC10 | 0.07 | 0.00 | 734 | 865 | 1813 | 1673 |

(continued)

| Table 5A: Differentially Upregulated Genes in SR + IL-2 | | | | | | | |
|---|---|---|---|---|---|---|---|
| RefSeq ID | Gene Symbol | SR GFOLD | SEN GFOLD | SR IL- 2 - | SR IL-2 + | SEN IL-2 - | SEN IL-2+ |
| NM_005845 | ABCC4 | 0.19 | 0.00 | 731 | 935 | 1337 | 1362 |
| NM_020297 | ABCC9 | 0.23 | -0.22 | 721 | 949 | 2161 | 1723 |
| NM_001040876 | ABCE1 | 0.32 | -0.05 | 852 | 1178 | 1312 | 1152 |
| NM_007189 | ABCF2 | 0.06 | 0.00 | 1964 | 2207 | 3296 | 3251 |
| NM_032750 | ABHD14B | 0.17 | 0.00 | 611 | 779 | 1630 | 1558 |
| NM_005158 | ABL2 | 0.06 | 0.00 | 6606 | 7144 | 9240 | 9584 |
| NM_000019 | ACAT1 | 0.20 | 0.00 | 706 | 912 | 1884 | 1878 |
| NM_005891 | ACAT2 | 0.08 | 0.00 | 533 | 646 | 1362 | 1255 |
| NM_032360 | ACBD6 | 0.30 | 0.00 | 350 | 508 | 772 | 867 |
| NM_001082486 | ACD | 0.23 | 0.00 | 347 | 479 | 453 | 425 |
| NM_001098 | ACO2 | 0.34 | 0.00 | 1994 | 2706 | 4798 | 4689 |
| NM_001037171 | ACOT9 | 0.08 | -0.09 | 949 | 1114 | 1897 | 1645 |
| NM_003501 | ACOX3 | 0.25 | 0.00 | 344 | 483 | 973 | 878 |
| NM_016361 | ACP6 | 1.16 | 0.00 | 18 | 73 | 164 | 179 |
| NM_033068 | ACPT | 0.30 | 0.00 | 40 | 77 | 54 | 48 |
| NM_001288968 | ACSF2 | 0.43 | 0.00 | 37 | 79 | 200 | 214 |
| NM_001076552 | ACSS2 | 0.53 | 0.01 | 338 | 571 | 1373 | 1503 |
| NM_001100 | ACTA1 | 0.31 | -0.15 | 91 | 153 | 108 | 68 |
| NM_001141945 | ACTA2 | 0.28 | -0.30 | 3308 | 4240 | 4529 | 3486 |
| NM_005159 | ACTC1 | 0.52 | -0.29 | 1327 | 2069 | 807 | 582 |
| NM_001199954 | ACTG1 | 0.15 | -0.20 | 124973 | 139507 | 229718 | 199115 |
| NM_001024675 | ACTL10 | 0.47 | 0.00 | 40 | 86 | 37 | 41 |
| NM_004924 | ACTN4 | 0.34 | 0.00 | 18951 | 24599 | 25070 | 25242 |
| NM_005736 | ACTR1A | 0.38 | -0.10 | 2851 | 3921 | 8462 | 7590 |
| NM_005735 | ACTR1B | 0.09 | 0.00 | 2057 | 2353 | 2887 | 2987 |
| NM_005721 | ACTR3 | 0.40 | 0.00 | 2780 | 3875 | 7678 | 7468 |
| NM_024855 | ACTR5 | 0.10 | 0.00 | 159 | 217 | 241 | 211 |
| NM_022899 | ACTR8 | 0.20 | 0.00 | 752 | 968 | 1190 | 1218 |
| NM_000666 | ACY1 | 0.25 | -0.06 | 258 | 371 | 1171 | 1017 |

(continued)

| fSeq IDeR | Gene Symbol | SR GFOLD | SEN GFOLD | SR IL-2 - | SR IL-2 + | SEN IL-2 - | SEN IL-2 + |
|---|---|---|---|---|---|---|---|
| NM_203488 | ACYP1 | 0.16 | -0.15 | 55 | 91 | 133 | 87 |
| NM_000022 | ADA | 0.19 | 0.00 | 482 | 634 | 817 | 736 |
| NM_030955 | ADAMTS12 | 0.17 | 0.00 | 1480 | 1804 | 2313 | 2201 |
| NM_014244 | ADAMTS2 | 0.09 | -0.06 | 10108 | 11082 | 9512 | 8822 |
| NM_014243 | ADAMTS3 | 0.23 | -0.10 | 40 | 74 | 31 | 13 |
| NM_182920 | ADAMTS9 | 0.27 | 0.00 | 49 | 89 | 46 | 50 |
| NM_001025107 | ADAR | 0.17 | 0.00 | 5109 | 6000 | 8672 | 8943 |
| NM_182503 | ADAT2 | 0.03 | 0.00 | 99 | 137 | 166 | 163 |
| NM_052853 | ADCK2 | 0.79 | 0.00 | 269 | 552 | 514 | 462 |
| NM_000671 | ADH5 | 0.52 | -0.23 | 1369 | 2133 | 3629 | 2924 |
| NM_032804 | ADO | 0.65 | 0.00 | 367 | 671 | 894 | 884 |
| NM_001619 | ADRBK1 | 0.11 | 0.00 | 1607 | 1875 | 1924 | 2044 |
| NM_000026 | ADSL | 0.07 | -0.14 | 873 | 1019 | 1545 | 1288 |
| NM_001126 | ADSS | 0.40 | 0.00 | 373 | 576 | 1094 | 1081 |
| NM_022767 | AEN | 0.13 | -0.17 | 1828 | 2156 | 2358 | 1946 |
| NM_152406 | AFAP1L1 | 0.28 | 0.00 | 643 | 883 | 761 | 819 |
| NM_006796 | AFG3L2 | 0.60 | -0.12 | 965 | 1606 | 2423 | 2081 |
| NM_006076 | AGFG2 | 0.18 | 0.00 | 377 | 502 | 453 | 440 |
| NM_018238 | AGK | 0.28 | -0.03 | 238 | 351 | 551 | 466 |
| NM_024758 | AGMAT | 0.21 | 0.00 | 45 | 80 | 125 | 96 |
| NM_006411 | AGPAT1 | 0.29 | 0.00 | 1767 | 2318 | 3968 | 3865 |
| NM_006412 | AGPAT2 | 0.26 | 0.00 | 542 | 742 | 2096 | 2194 |
| NM_020133 | AGPAT4 | 0.74 | 0.00 | 103 | 227 | 626 | 599 |
| NM_178819 | AGPAT6 | 0.05 | 0.00 | 2204 | 2446 | 4257 | 4264 |
| NM_032717 | AGPAT9 | 0.54 | 0.01 | 28 | 68 | 129 | 170 |
| NM_000029 | AGT | 0.50 | 0.00 | 10 | 37 | 351 | 316 |
| NM_015446 | AHCTF1 | 0.02 | 0.00 | 782 | 887 | 1027 | 1000 |
| NM_000687 | AHCY | 0.03 | -0.15 | 1941 | 2136 | 3261 | 2765 |
| NM_020731 | AHRR | 0.12 | -0.27 | 687 | 842 | 1572 | 1192 |
| NM_012111 | AHSA1 | 0.31 | 0.00 | 1465 | 1974 | 2516 | 2567 |
| NM_001130847 | AIFM1 | 0.36 | 0.00 | 577 | 840 | 1350 | 1244 |
| NM_003977 | AIP | 0.10 | 0.00 | 1033 | 1221 | 1709 | 1671 |
| NM_032876 | AJUBA | 0.10 | 0.00 | 937 | 1113 | 799 | 823 |
| NM_174858 | AK5 | 0.35 | -0.61 | 837 | 1181 | 4461 | 2767 |
| NM_003488 | AKAP1 | 0.43 | 0.00 | 503 | 775 | 977 | 945 |
| NM_005100 | AKAP12 | 0.08 | -0.03 | 2308 | 2601 | 5209 | 4882 |

(continued)

| fSeq IDeR | Gene Symbol | SR GFOLD | SEN GFOLD | SR IL-2 - | SR IL-2 + | SEN IL-2 - | SEN IL-2 + |
|---|---|---|---|---|---|---|---|
| NM_001198656 | AKAP2 | 0.24 | -0.16 | 8623 | 10504 | 23286 | 20445 |
| NM_001202414 | AKR1A1 | 0.05 | 0.00 | 1332 | 1504 | 2165 | 2283 |
| NM_001628 | AKR1B1 | 0.30 | 0.00 | 3801 | 4907 | 10810 | 10662 |
| RefSeq ID | Gene Symbol | SR GFOLD | SEN GFOLD | SR IL-2 - | SR IL-2 + | SEN IL-2 - | SEN IL-2 + |
| NM_001353 | AKR1C1 | 0.43 | -0.40 | 11991 | 16663 | 17729 | 13053 |
| NM_001354 | AKR1C2 | 0.27 | -0.18 | 6380 | 7987 | 7310 | 6198 |
| NM_003739 | AKR1C3 | 0.61 | 0.00 | 1265 | 2095 | 2071 | 2009 |
| NM_001818 | AKR1C4 | 0.62 | 0.00 | 302 | 549 | 472 | 418 |
| NM_032375 | AKT1S1 | 0.08 | 0.01 | 1078 | 1251 | 1953 | 2115 |
| NM_000031 | ALAD | 0.17 | -0.02 | 404 | 530 | 1622 | 1473 |
| NM_000688 | ALAS1 | 0.07 | 0.00 | 2108 | 2372 | 3720 | 3920 |
| NM_000689 | ALDH1A1 | 0.54 | 0.00 | 19 | 51 | 0 | 0 |
| NM_000693 | ALDH1A3 | 0.16 | -0.69 | 502 | 645 | 4477 | 2617 |
| NM_000692 | ALDH1B1 | 0.44 | -0.42 | 1656 | 2418 | 2957 | 2067 |
| NM_001031806 | ALDH3A2 | 0.14 | 0.00 | 440 | 564 | 1175 | 1248 |
| NM_000694 | ALDH3B1 | 0.21 | -0.18 | 365 | 494 | 1870 | 1523 |
| NM_001182 | ALDH7A1 | 0.30 | 0.00 | 182 | 280 | 356 | 336 |
| NM_019109 | ALG1 | 0.21 | 0.00 | 446 | 598 | 1142 | 1039 |
| NM_032834 | ALG10 | 0.19 | 0.00 | 61 | 101 | 69 | 72 |
| NM_144988 | ALG14 | 0.89 | 0.00 | 46 | 127 | 252 | 238 |
| NM_013338 | ALG5 | 0.02 | 0.00 | 544 | 632 | 761 | 848 |
| NM_001007027 | ALG8 | 0.26 | -0.02 | 313 | 444 | 886 | 780 |
| NM_032306 | ALKBH7 | 0.26 | 0.00 | 295 | 421 | 630 | 601 |
| NM_052947 | ALPK2 | 0.16 | -0.16 | 3052 | 3605 | 4188 | 3558 |
| NM_020919 | ALS2 | 0.21 | 0.00 | 1107 | 1402 | 1678 | 1708 |
| NM_005782 | ALYREF | 0.15 | -0.12 | 1078 | 1312 | 1524 | 1285 |
| NM_001267782 | AMBRA1 | 0.32 | -0.08 | 686 | 959 | 1574 | 1370 |
| NM_001634 | AMD1 | 0.27 | -0.15 | 1591 | 2068 | 2543 | 2144 |
| NM_016201 | AMOTL2 | 0.26 | -0.51 | 7839 | 9743 | 6636 | 4447 |
| NM_001635 | AMPH | 0.27 | 0.00 | 103 | 166 | 241 | 266 |
| NM_000481 | AMT | 0.25 | 0.00 | 27 | 55 | 94 | 68 |
| NM_022662 | ANAPC1 | 0.15 | -0.04 | 985 | 1211 | 1460 | 1298 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001242374 | ANAPC13 | 0.55 | -0.08 | 611 | 1010 | 1291 | 1107 |
| NM_001278485 | ANAPC15 | 0.03 | 0.00 | 282 | 346 | 661 | 675 |
| NM_013366 | ANAPC2 | 0.11 | -0.06 | 740 | 899 | 1674 | 1481 |
| NM_001286756 | ANAPC4 | 0.57 | 0.00 | 200 | 363 | 584 | 608 |
| NM_016238 | ANAPC7 | 0.31 | -0.23 | 812 | 1118 | 1999 | 1577 |
| NM_015305 | ANGEL1 | 0.64 | 0.00 | 205 | 391 | 705 | 774 |
| NM_020987 | ANK3 | 0.44 | 0.00 | 18 | 46 | 89 | 81 |
| NM_001257999 | ANKFY1 | 0.25 | 0.00 | 1886 | 2414 | 4739 | 4961 |
| NM_020319 | ANKMY2 | 0.34 | 0.00 | 323 | 483 | 753 | 799 |
| NM_033121 | ANKRD13A | 0.20 | 0.00 | 3303 | 3994 | 4987 | 4900 |
| NM_030816 | ANKRD13C | 0.28 | -0.11 | 565 | 779 | 1029 | 854 |
| RefSeq ID | Gene Symbol | SR GFOLD | SEN GFOLD | SR IL- 2 - | SR IL-2 + | SEN IL-2- | SEN IL-2 + |
| NM_019046 | ANKRD16 | 0.27 | 0.00 | 97 | 158 | 164 | 177 |
| NM_032139 | ANKRD27 | 0.01 | 0.00 | 1123 | 1247 | 2080 | 1937 |
| NM_032290 | ANKRD32 | 0.31 | 0.00 | 109 | 179 | 154 | 120 |
| NM_144698 | ANKRD35 | 0.02 | 0.00 | 49 | 76 | 202 | 244 |
| NM_052855 | ANKRD40 | 0.53 | 0.00 | 1139 | 1801 | 2086 | 2185 |
| NM_017704 | ANKRD49 | 0.19 | 0.00 | 81 | 128 | 127 | 102 |
| NM_020337 | ANKRD50 | 0.13 | -0.05 | 1241 | 1488 | 2344 | 2116 |
| NM_173595 | ANKRD52 | 0.05 | -0.03 | 5569 | 6021 | 6208 | 5828 |
| NM_015245 | ANKS1A | 0.21 | 0.00 | 463 | 617 | 780 | 858 |
| NM_018685 | ANLN | 0.15 | -1.06 | 3260 | 3819 | 3309 | 1477 |
| NM_001286615 | ANO4 | 0.09 | -0.55 | 112 | 159 | 179 | 91 |
| NM_006305 | ANP32A | 0.09 | 0.00 | 500 | 613 | 1119 | 1198 |
| NM_006401 | ANP32B | 0.18 | -0.05 | 759 | 961 | 1002 | 869 |
| NM_012403 | ANP32C | 0.07 | 0.00 | 96 | 137 | 168 | 176 |
| NM_007193 | ANXA10 | 0.33 | -0.10 | 3 | 17 | 158 | 111 |
| NM_145869 | ANXA11 | 0.02 | -0.02 | 4548 | 4854 | 8306 | 7896 |
| NM_001283 | AP1S1 | 0.21 | 0.00 | 1496 | 1878 | 4669 | 4654 |
| NM_004069 | AP2S1 | 0.46 | -0.17 | 1784 | 2636 | 5133 | 4338 |
| NM_001271769 | AP3B1 | 0.36 | 0.00 | 1007 | 1422 | 2514 | 2688 |

(continued)

| RefSeq ID | Gene Symbol | SR GFOLD | SEN GFOLD | SR IL- 2 - | SR IL-2 + | SEN IL-2- | SEN IL-2 + |
|---|---|---|---|---|---|---|---|
| NM_001134296 | AP3M2 | 0.01 | 0.00 | 380 | 451 | 1198 | 1266 |
| NM_004722 | AP4M1 | 0.32 | -0.02 | 195 | 302 | 732 | 638 |
| NM_001204446 | AP5S1 | 0.09 | 0.00 | 264 | 340 | 464 | 518 |
| NM_013229 | APAF1 | 0.10 | 0.00 | 483 | 595 | 447 | 518 |
| NM_019043 | APBB1IP | 0.10 | 0.01 | 915 | 1089 | 942 | 1050 |
| NM_006051 | APBB3 | 0.03 | 0.00 | 159 | 207 | 279 | 275 |
| NM_014481 | APEX2 | 0.26 | -0.07 | 498 | 682 | 705 | 591 |
| NM_015957 | APIP | 0.14 | 0.00 | 61 | 98 | 160 | 161 |
| NM_001270517 | APITD1-CORT | 0.16 | 0.01 | 58 | 95 | 116 | 155 |
| NM_014508 | APOBEC3C | 0.44 | -0.29 | 317 | 509 | 1408 | 1050 |
| NM_001136540 | APOL1 | 0.33 | -0.35 | 246 | 374 | 713 | 488 |
| NM_030882 | APOL2 | 0.01 | -0.03 | 998 | 1114 | 1728 | 1560 |
| NM_001251904 | APPL2 | 0.16 | 0.00 | 2299 | 2747 | 3712 | 3926 |
| NM_000485 | APRT | 0.21 | 0.00 | 1326 | 1671 | 3213 | 3368 |
| NM_000044 | AR | 0.60 | 0.00 | 217 | 400 | 867 | 874 |
| NM_001039479 | AREL1 | 0.03 | 0.00 | 1435 | 1599 | 2941 | 2882 |
| NM_001024226 | ARF1 | 0.50 | -0.01 | 8861 | 12952 | 18255 | 17737 |
| NM_001662 | ARF5 | 0.28 | 0.00 | 1473 | 1936 | 3311 | 3335 |
| NM_032389 | ARFGAP2 | 0.23 | 0.00 | 1101 | 1419 | 2283 | 2144 |
| NM_001287432 | ARFIP1 | 0.07 | 0.00 | 469 | 570 | 1038 | 1107 |
| RefSeq ID | Gene Symbol | SR GFOLD | SEN GFOLD | SR IL- 2- | SR IL-2 + | SEN IL-2- | SEN IL-2 + |
| NM_018011 | ARGLU1 | 0.36 | -0.06 | 648 | 938 | 1169 | 1017 |
| NM_004308 | ARHGAP1 | 0.18 | -0.06 | 4080 | 4872 | 9533 | 8859 |
| NM_033515 | ARHGAP18 | 0.11 | 0.00 | 1154 | 1365 | 3357 | 3166 |
| NM_020809 | ARHGAP20 | 0.51 | 0.00 | 101 | 191 | 568 | 591 |
| NM_001025616 | ARHGAP24 | 0.13 | 0.00 | 1172 | 1402 | 2780 | 2719 |
| NM_004815 | ARHGAP29 | 0.31 | 0.00 | 1234 | 1674 | 3481 | 3619 |
| NM_020754 | ARHGAP31 | 0.33 | -0.29 | 2208 | 2953 | 4527 | 3510 |
| NM_001172630 | ARHGAP33 | 0.63 | 0.00 | 42 | 99 | 131 | 131 |

(continued)

| RefSeq ID | Gene Symbol | SR GFOLD | SEN GFOLD | SR IL-2- | SR IL-2 + | SEN IL-2- | SEN IL-2 + |
|---|---|---|---|---|---|---|---|
| NM_025251 | ARHGAP39 | 0.30 | 0.00 | 31 | 63 | 156 | 146 |
| NM_152432 | ARHGAP42 | 0.54 | 0.00 | 22 | 57 | 69 | 68 |
| NM_004309 | ARHGDIA | 0.21 | -0.09 | 7926 | 9529 | 12881 | 11758 |
| NM_199002 | ARHGEF1 | 0.17 | 0.00 | 1639 | 1993 | 1803 | 1834 |
| NM_018125 | ARHGEF10L | 0.67 | 0.00 | 363 | 674 | 1400 | 1495 |
| NM_153213 | ARHGEF19 | 0.23 | 0.00 | 133 | 202 | 79 | 79 |
| NM_001080479 | ARHGEF28 | 0.10 | 0.00 | 782 | 940 | 1291 | 1329 |
| NM_005435 | ARHGEF5 | 0.25 | 0.00 | 261 | 374 | 601 | 667 |
| NM_002892 | ARID4A | 0.16 | 0.00 | 144 | 207 | 435 | 490 |
| NM_001244638 | ARID5B | 0.19 | 0.00 | 2713 | 3296 | 4873 | 5026 |
| NM_006321 | ARIH2 | 0.16 | 0.00 | 919 | 1140 | 1813 | 1728 |
| NM_001667 | ARL2 | 0.31 | 0.00 | 892 | 1223 | 1622 | 1582 |
| NM_015161 | ARL6IP1 | 0.23 | -0.14 | 1327 | 1696 | 2084 | 1760 |
| NM_001199196 | ARMC6 | 0.60 | -0.33 | 506 | 873 | 1404 | 1018 |
| NM_001271466 | ARMC9 | 0.09 | -0.45 | 627 | 757 | 3232 | 2220 |
| NM_016608 | ARMCX1 | 0.18 | -0.02 | 570 | 735 | 1160 | 1037 |
| NM_016607 | ARMCX3 | 0.19 | 0.00 | 1186 | 1477 | 2986 | 3122 |
| NM_001168479 | ARMCX5 | 0.22 | 0.00 | 113 | 174 | 181 | 200 |
| NM_001199818 | ARMCX5-GPRASP2 | 0.06 | 0.00 | 213 | 274 | 709 | 686 |
| NM_001190996 | ARPC1A | 0.17 | 0.00 | 1262 | 1546 | 3159 | 3055 |
| NM_005718 | ARPC4 | 0.17 | -0.24 | 2515 | 3019 | 6199 | 5029 |
| NM_001198793 | ARPC4-TTLL3 | 0.30 | 0.00 | 1111 | 1497 | 3038 | 3057 |
| NM_001270439 | ARPC5 | 0.11 | 0.00 | 4798 | 5410 | 7245 | 7418 |
| NM_030978 | ARPC5L | 0.17 | -0.04 | 871 | 1087 | 1753 | 1569 |
| NM_182616 | ARPIN | 0.29 | -0.09 | 529 | 737 | 1248 | 1067 |
| NM_001257328 | ARRB2 | 0.25 | 0.00 | 186 | 275 | 216 | 229 |
| NM_152285 | ARRDC1 | 0.38 | 0.00 | 260 | 408 | 453 | 503 |
| NM_001079516 | ASAH2B | 0.29 | 0.00 | 26 | 55 | 60 | 57 |
| NM_001247996 | ASAP1 | 0.21 | -0.05 | 3120 | 3808 | 6549 | 6061 |

(continued)

| RefSeq ID | Gene Symbol | SR GFOLD | SEN GFOLD | SR IL- 2- | SR IL-2 + | SEN IL-2- | SEN IL-2 + |
|---|---|---|---|---|---|---|---|
| NM_ 001142733 | ASB14 | 1.05 | 0.00 | 37 | 118 | 112 | 129 |
| RefSeq ID | Gene Symbol | SR GFOLD | SEN GFOLD | SR IL-2- | SR IL-2 + | SEN IL-2- | SEN IL-2 + |
| NM_080863 | ASB16 | 0.27 | 0.00 | 37 | 71 | 102 | 118 |
| NM_017873 | ASB6 | 0.12 | -0.07 | 1758 | 2067 | 3271 | 2928 |
| NM_ 001198798 | ASCC1 | 0.47 | 0.00 | 171 | 296 | 522 | 486 |
| NM_032204 | ASCC2 | 0.22 | 0.00 | 1042 | 1333 | 2263 | 2299 |
| NM_004674 | ASH2L | 0.05 | 0.00 | 758 | 883 | 1206 | 1153 |
| NM_001095 | ASIC1 | 0.65 | -0.20 | 35 | 87 | 227 | 155 |
| NM_004192 | ASMTL | 0.11 | 0.00 | 637 | 781 | 1398 | 1392 |
| NM_004317 | ASNA1 | 0.15 | -0.17 | 1310 | 1586 | 3030 | 2521 |
| NM_ 001251888 | ASPSCR1 | 0.19 | 0.00 | 271 | 372 | 665 | 654 |
| NM_014010 | ASTN2 | 0.52 | 0.00 | 55 | 115 | 231 | 205 |
| NM_018164 | ASUN | 0.17 | -0.04 | 432 | 562 | 925 | 802 |
| NM_018188 | ATAD3A | 0.21 | 0.00 | 782 | 1010 | 913 | 906 |
| NM_031921 | ATAD3B | 0.42 | 0.00 | 448 | 692 | 574 | 590 |
| NM_024857 | ATAD5 | 0.02 | -0.20 | 230 | 287 | 125 | 78 |
| NM_ 001136153 | ATF6B | 0.05 | 0.00 | 1483 | 1671 | 3128 | 3257 |
| NM_ 001190266 | ATG16L1 | 0.29 | 0.00 | 658 | 909 | 1481 | 1394 |
| NM_052936 | ATG4A | 0.06 | 0.00 | 225 | 289 | 449 | 457 |
| NM_006395 | ATG7 | 0.17 | 0.00 | 1126 | 1388 | 2136 | 2103 |
| NM_004044 | ATIC | 0.59 | 0.00 | 705 | 1185 | 2059 | 2113 |
| NM_032827 | ATOH8 | 0.39 | 0.00 | 935 | 1348 | 1996 | 1978 |
| NM_004045 | ATOX1 | 0.13 | 0.00 | 486 | 611 | 1094 | 1148 |
| NM_024524 | ATP13A3 | 0.07 | 0.00 | 7540 | 8187 | 7289 | 7506 |
| NM_001677 | ATP1B1 | 0.06 | 0.00 | 1237 | 1408 | 1025 | 1098 |
| NM_ 001257335 | ATP5A1 | 0.33 | -0.34 | 3012 | 4000 | 7262 | 5506 |
| NM_001686 | ATP5B | 0.21 | -0.06 | 10702 | 12780 | 23492 | 21985 |
| NM_001687 | ATP5D | 0.25 | 0.00 | 587 | 790 | 1067 | 1055 |
| NM_ 001190329 | ATP5G3 | 0.56 | -0.32 | 1599 | 2539 | 4750 | 3614 |
| NM_006356 | ATP5H | 0.32 | -0.14 | 422 | 611 | 1458 | 1205 |
| NM_007100 | ATP5I | 0.30 | -0.13 | 656 | 910 | 1635 | 1372 |

(continued)

| RefSeq ID | Gene Symbol | SR GFOLD | SEN GFOLD | SR IL-2- | SR IL-2 + | SEN IL-2- | SEN IL-2 + |
|---|---|---|---|---|---|---|---|
| NM_001003703 | ATP5J | 0.20 | 0.00 | 513 | 676 | 1165 | 1205 |
| NM_001003713 | ATP5J2 | 0.38 | 0.00 | 364 | 555 | 1015 | 924 |
| NM_001697 | ATP50 | 0.04 | 0.00 | 654 | 761 | 1543 | 1438 |
| NM_018035 | ATP5SL | 0.41 | -0.09 | 561 | 846 | 1364 | 1172 |
| NM_001696 | ATP6V1E1 | 0.26 | 0.00 | 1730 | 2232 | 4195 | 4142 |
| NM_001198909 | ATP6V1F | 0.04 | -0.28 | 1068 | 1207 | 2660 | 2041 |
| NM_000053 | ATP7B | 0.24 | 0.00 | 219 | 317 | 422 | 492 |
| NM_005603 | ATP8B1 | 0.14 | -0.11 | 3192 | 3725 | 4369 | 3841 |
| NM_001184 | ATR | 0.29 | 0.00 | 571 | 795 | 1331 | 1231 |
| NM_130384 | ATRIP | 0.08 | 0.00 | 361 | 451 | 489 | 473 |
| RefSeq ID | Gene Symbol | SR GFOLD | SEN GFOLD | SR IL-2- | SR IL-2 + | SEN IL-2- | SEN IL-2 + |
| NM_001137675 | ATXN1L | 0.05 | 0.00 | 976 | 1117 | 1368 | 1399 |
| NM_153340 | A TXN7L2 | 0.18 | 0.00 | 86 | 134 | 121 | 116 |
| NM_001136262 | ATXN7L3B | 0.02 | 0.00 | 1192 | 1330 | 1516 | 1575 |
| NM_001287490 | AUNIP | 0.09 | 0.00 | 168 | 227 | 389 | 366 |
| NM_021913 | AXL | 0.26 | -0.30 | 24844 | 30431 | 25272 | 20136 |
| NM_152490 | B3GALNT2 | 0.20 | 0.00 | 496 | 655 | 794 | 854 |
| NM_194318 | B3GALTL | 0.03 | 0.00 | 216 | 272 | 368 | 399 |
| NM_001288722 | B3GAT3 | 0.34 | 0.00 | 390 | 576 | 1044 | 978 |
| NM_198540 | B3GNT8 | 0.62 | 0.00 | 14 | 44 | 83 | 67 |
| NM_033309 | B3GNT9 | 0.32 | 0.00 | 725 | 1015 | 1576 | 1562 |
| NM_212543 | B4GALT4 | 0.47 | 0.00 | 876 | 1344 | 1121 | 1201 |
| NM_001243475 | B9D1 | 0.32 | 0.00 | 73 | 128 | 212 | 213 |
| NM_001033549 | BABAM1 | 0.18 | 0.00 | 1026 | 1282 | 2525 | 2440 |
| NM_004322 | BAD | 0.30 | 0.00 | 398 | 569 | 1383 | 1353 |
| NM_001172415 | BAG1 | 0.25 | 0.00 | 310 | 439 | 645 | 726 |
| NM_004282 | BAG2 | 0.03 | -0.30 | 802 | 916 | 1375 | 1013 |
| NM_001188 | BAK1 | 0.30 | -0.15 | 395 | 566 | 747 | 591 |
| NM_003860 | BANF1 | 0.17 | -0.27 | 1226 | 1505 | 2250 | 1737 |

(continued)

| RefSeq ID | Gene Symbol | SR GFOLD | SEN GFOLD | SR IL-2- | SR IL-2 + | SEN IL-2- | SEN IL-2 + |
|---|---|---|---|---|---|---|---|
| NM_006317 | BASP1 | 0.46 | -0.03 | 3439 | 4992 | 5336 | 4993 |
| NM_031885 | BBS2 | 0.02 | 0.00 | 710 | 814 | 1612 | 1577 |
| NM_176824 | BBS7 | 0.04 | 0.00 | 264 | 328 | 699 | 769 |
| NM_001013257 | BCAM | 0.73 | 0.00 | 37 | 96 | 69 | 46 |
| NM_001170714 | BCAR1 | 0.09 | 0.00 | 2190 | 2500 | 3712 | 3656 |
| NM_003567 | BCAR3 | 0.15 | -0.06 | 3909 | 4555 | 6958 | 6407 |
| NM_078468 | BCCIP | 0.34 | 0.00 | 474 | 689 | 1096 | 1111 |
| NM_015367 | BCL2L13 | 0.34 | 0.00 | 986 | 1372 | 2922 | 3000 |
| NM_001199864 | BCL2L2-PABPN1 | 0.20 | 0.00 | 1113 | 1405 | 1926 | 2052 |
| NM_004765 | BCL7C | 0.29 | -0.04 | 232 | 345 | 668 | 567 |
| NM_182557 | BCL9L | 0.09 | 0.00 | 3336 | 3740 | 4629 | 4758 |
| NM_014739 | BCLAF1 | 0.06 | 0.00 | 1521 | 1726 | 3011 | 3096 |
| NM_020139 | BDH2 | 1.24 | -0.13 | 19 | 80 | 200 | 142 |
| NM_018429 | BDP1 | 0.29 | 0.00 | 718 | 981 | 1285 | 1331 |
| NM_005868 | BET1 | 0.07 | 0.00 | 351 | 434 | 514 | 530 |
| NM_018476 | BEX1 | 0.19 | 0.00 | 76 | 122 | 21 | 15 |
| NM_016561 | BFAR | 0.21 | 0.00 | 1199 | 1522 | 2731 | 2800 |
| NM_001003800 | BICD2 | 0.33 | -0.22 | 2183 | 2932 | 5180 | 4253 |
| NM_018688 | BIN3 | 0.28 | 0.00 | 362 | 514 | 1044 | 1140 |
| NM_017693 | BIVM | 0.20 | 0.00 | 437 | 580 | 1169 | 1187 |
| NM_001167820 | BLCAP | 0.28 | 0.00 | 958 | 1286 | 2375 | 2431 |
| NM_000057 | BLM | 0.31 | 0.00 | 143 | 227 | 62 | 87 |
| NM_001487 | BLOC1S1 | 0.15 | 0.00 | 479 | 614 | 1048 | 1059 |
| NM_001282436 | BLOC1S2 | 0.37 | 0.00 | 278 | 429 | 905 | 922 |
| NM_001253823 | BLVRA | 0.16 | -0.11 | 454 | 586 | 1341 | 1129 |
| NM_005180 | BMI1 | 0.13 | 0.00 | 568 | 708 | 1111 | 1227 |
| NM_133468 | BMPER | 0.06 | 0.00 | 1468 | 1659 | 1208 | 1103 |
| NM_013979 | BNIP1 | 0.03 | 0.00 | 249 | 309 | 333 | 359 |
| NM_138369 | BOD1 | 0.05 | 0.00 | 758 | 881 | 1077 | 1126 |
| NM_032515 | BOK | 0.81 | 0.00 | 597 | 1176 | 1770 | 1669 |
| NM_001286746 | BORA | 0.06 | -0.24 | 94 | 134 | 146 | 91 |

(continued)

| RefSeq ID | Gene Symbol | SR GFOLD | SEN GFOLD | SR IL-2- | SR IL-2 + | SEN IL-2- | SEN IL-2 + |
|---|---|---|---|---|---|---|---|
| NM_006085 | BPNT1 | 0.85 | -0.05 | 291 | 617 | 728 | 621 |
| NM_152743 | BRAT1 | 0.47 | -0.16 | 387 | 622 | 1329 | 1081 |
| NM_004899 | BRE | 0.66 | 0.00 | 163 | 321 | 842 | 924 |
| NM_001519 | BRF1 | 0.53 | 0.00 | 317 | 540 | 638 | 649 |
| NM_032043 | BRIP1 | 0.04 | -0.24 | 483 | 575 | 275 | 187 |
| NM_015399 | BRMS1 | 0.38 | 0.00 | 731 | 1064 | 1630 | 1573 |
| NM_017797 | BTBD2 | 0.32 | 0.00 | 1784 | 2394 | 3625 | 3725 |
| NM_001002860 | BTBD7 | 0.11 | 0.00 | 1091 | 1298 | 2032 | 2150 |
| NM_001037637 | BTF3 | 0.32 | 0.01 | 1453 | 1970 | 2656 | 2847 |
| NM_004336 | BUB1 | 0.07 | -1.05 | 926 | 1081 | 911 | 383 |
| NM_001211 | BUB1B | 0.03 | -0.98 | 602 | 698 | 514 | 216 |
| NM_004725 | BUB3 | 0.25 | -0.13 | 3362 | 4208 | 4245 | 3676 |
| NM_001261835 | BZRAP1 | 0.65 | 0.00 | 21 | 59 | 62 | 63 |
| NM_001159767 | BZW2 | 0.67 | -0.11 | 583 | 1049 | 1348 | 1137 |
| NM_014267 | C11orf58 | 0.24 | 0.00 | 377 | 522 | 965 | 972 |
| NM_001135635 | C11orf68 | 0.05 | -0.06 | 1741 | 1941 | 2379 | 2124 |
| NM_207645 | C11orf87 | 0.85 | 0.00 | 21 | 67 | 42 | 52 |
| NM_021640 | C12orf10 | 0.03 | 0.00 | 621 | 720 | 1061 | 1111 |
| NM_001286198 | C12orf43 | 0.85 | 0.00 | 151 | 341 | 370 | 349 |
| NM_152318 | C12orf45 | 0.31 | -0.38 | 79 | 136 | 256 | 155 |
| NM_024738 | C12orf49 | 0.09 | 0.00 | 1045 | 1228 | 1553 | 1691 |
| NM_138425 | C12orf57 | 0.13 | 0.00 | 937 | 1135 | 1445 | 1499 |
| NM_001135570 | C12orf73 | 0.43 | 0.00 | 75 | 141 | 164 | 163 |
| NM_001102366 | C14orf159 | 0.22 | -0.09 | 325 | 447 | 1275 | 1089 |
| NM_024644 | C14orf169 | 0.35 | 0.00 | 271 | 415 | 516 | 558 |
| NM_001199058 | C15orf38-AP3S2 | 0.14 | 0.00 | 895 | 1092 | 2206 | 2303 |
| NM_022744 | C16orf58 | 0.08 | 0.00 | 2197 | 2486 | 3294 | 3440 |
| NM_020314 | C16orf62 | 0.55 | 0.00 | 995 | 1602 | 3575 | 3429 |
| NM_001001436 | C16orf87 | 0.09 | 0.00 | 190 | 253 | 245 | 283 |

(continued)

| RefSeq ID | Gene Symbol | SR GFOLD | SEN GFOLD | SR IL-2- | SR IL-2 + | SEN IL-2- | SEN IL. 2 + |
|---|---|---|---|---|---|---|---|
| NM_001272051 | C16orf91 | 0.34 | 0.00 | 136 | 222 | 270 | 226 |
| NM_001193655 | C17orf62 | 0.04 | 0.00 | 737 | 853 | 1481 | 1571 |
| NM_017941 | C17orf80 | 0.01 | -0.06 | 241 | 297 | 418 | 338 |
| NM_001114118 | C17orf85 | 0.23 | 0.00 | 693 | 914 | 1366 | 1431 |
| NM_001201475 | C18orf21 | 0.08 | 0.00 | 171 | 229 | 285 | 255 |
| NM_017914 | C19orf24 | 0.51 | 0.00 | 266 | 454 | 736 | 828 |
| NM_152482 | C19orf25 | 0.56 | 0.00 | 118 | 227 | 387 | 340 |
| NM_032207 | C19orf44 | 0.33 | 0.00 | 100 | 168 | 193 | 181 |
| NM_001290149 | C19orf48 | 0.21 | -0.16 | 687 | 895 | 510 | 390 |
| NM_138358 | C19orf52 | 0.04 | 0.00 | 219 | 278 | 410 | 440 |
| NM_001100418 | C19orf60 | 0.26 | 0.00 | 153 | 233 | 412 | 427 |
| NM_199341 | C19orf68 | 0.16 | 0.00 | 52 | 87 | 96 | 104 |
| NM_205767 | C19orf70 | 0.38 | -0.34 | 248 | 390 | 923 | 647 |
| NM_017891 | C1ort159 | 0.62 | 0.00 | 49 | 112 | 177 | 153 |
| NM_207356 | C1ort174 | 0.34 | 0.00 | 379 | 561 | 624 | 695 |
| NM_152374 | Clorf216 | 0.12 | 0.00 | 317 | 411 | 1225 | 1237 |
| NM_024319 | Clorf35 | 0.31 | 0.00 | 218 | 331 | 270 | 220 |
| NM_001098616 | Clorf43 | 0.25 | 0.01 | 2556 | 3232 | 4914 | 5172 |
| NM_017860 | Clorf56 | 0.29 | 0.00 | 109 | 177 | 318 | 318 |
| NM_001282671 | Clorf86 | 0.03 | 0.00 | 249 | 310 | 545 | 593 |
| NM_001212 | C1QBP | 0.02 | -0.16 | 1402 | 1552 | 1888 | 1558 |
| NM_030968 | C1QTNF1 | 0.23 | 0.00 | 143 | 215 | 449 | 383 |
| NM_021254 | C21orf59 | 0.04 | 0.00 | 455 | 541 | 657 | 577 |
| NM_024627 | C22orf29 | 0.16 | 0.00 | 648 | 820 | 977 | 926 |
| NM_001286577 | C2CD3 | 0.40 | 0.00 | 345 | 535 | 851 | 867 |
| NM_001286174 | C2CD5 | 0.25 | 0.00 | 381 | 528 | 749 | 693 |
| NM_013310 | C2orf27A | 0.27 | 0.00 | 24 | 51 | 94 | 120 |
| NM_025203 | C2orf44 | 0.53 | 0.00 | 185 | 331 | 308 | 272 |
| NM_024520 | C2orf47 | 0.13 | 0.00 | 129 | 184 | 281 | 301 |

(continued)

| RefSeq ID | Gene Symbol | SR GFOLD | SEN GFOLD | SR IL-2- | SR IL-2 + | SEN IL-2- | SEN IL. 2 + |
|---|---|---|---|---|---|---|---|
| NM_001286537 | C2orf49 | 0.35 | 0.00 | 210 | 328 | 491 | 536 |
| NM_001145054 | C2orf81 | 0.77 | 0.00 | 50 | 125 | 266 | 253 |
| NM_032321 | C2orf88 | 0.37 | 0.00 | 329 | 502 | 838 | 876 |
| NM_020685 | C3orf14 | 0.25 | 0.00 | 29 | 58 | 168 | 179 |
| NM_173657 | C3orf33 | 0.32 | 0.00 | 36 | 72 | 110 | 105 |
| NM_001099777 | C3orf55 | 0.58 | 0.00 | 35 | 83 | 187 | 165 |
| NM_001206997 | C4orf22 | 0.32 | 0.00 | 40 | 78 | 92 | 113 |
| NM_018356 | C5orf22 | 0.40 | 0.00 | 300 | 469 | 493 | 564 |
| NM_033211 | C5orf30 | 0.06 | 0.00 | 2346 | 2608 | 5197 | 5346 |
| NM_001277348 | C5orf66 | 0.57 | 0.00 | 111 | 216 | 268 | 292 |
| RefSeq ID | Gene Symbol | SR GFOLD | SEN GFOLD | SR IL-2- | SR IL-2 + | SEN IL-2- | SEN IL. 2+ |
| NM_178508 | C6orf1 | 0.20 | -0.35 | 529 | 693 | 1647 | 1185 |
| NM_001161376 | C6orf136 | 0.87 | 0.00 | 120 | 283 | 277 | 327 |
| NM_021184 | C6orf47 | 0.24 | 0.00 | 320 | 448 | 559 | 536 |
| NM_145267 | C6orf57 | 0.94 | 0.00 | 19 | 66 | 69 | 91 |
| NM_024067 | C7orf26 | 0.06 | 0.00 | 501 | 602 | 632 | 662 |
| NM_001243749 | C7orf49 | 0.22 | 0.00 | 627 | 828 | 1160 | 1152 |
| NM_001134395 | C7orf50 | 0.06 | 0.00 | 511 | 613 | 1422 | 1299 |
| NM_197964 | C7orf55 | 0.55 | 0.00 | 99 | 193 | 287 | 253 |
| NM_001130929 | C7orf73 | 0.12 | 0.00 | 509 | 634 | 888 | 900 |
| NM_177965 | C8orf37 | 0.04 | -0.45 | 26 | 47 | 92 | 44 |
| NM_016390 | C9orf114 | 0.07 | -0.01 | 1047 | 1211 | 1720 | 1571 |
| NM_001193329 | C9orf3 | 0.47 | 0.00 | 362 | 587 | 859 | 852 |
| NM_017998 | C9orf40 | 0.17 | -0.55 | 195 | 272 | 314 | 172 |
| NM-032307 | C9orf64 | 0.32 | -0.06 | 335 | 494 | 603 | 501 |
| NM_032310 | C9orf89 | 0.25 | 0.00 | 478 | 653 | 1148 | 1065 |
| NM_198584 | CA13 | 0.71 | 0.00 | 20 | 59 | 54 | 59 |
| NM_016289 | CAB39 | 0.20 | 0.00 | 1085 | 1370 | 1797 | 1911 |
| NM_001080543 | CACTIN | 0.19 | -0.11 | 397 | 528 | 595 | 479 |

(continued)

| RefSeq ID | Gene Symbol | SR GFOLD | SEN GFOLD | SR IL-2- | SR IL-2 + | SEN IL-2- | SEN IL. 2+ |
|---|---|---|---|---|---|---|---|
| NM_004341 | CAD | 0.07 | -0.07 | 2925 | 3251 | 3519 | 3159 |
| NM_001009571 | CADPS2 | 0.33 | 0.00 | 50 | 94 | 17 | 9 |
| NM_033138 | CALD1 | 0.29 | -0.03 | 11161 | 14028 | 19721 | 18841 |
| NM_015916 | CALHM2 | 0.61 | 0.00 | 445 | 779 | 1662 | 1773 |
| NM_005184 | CALM3 | 0.11 | -0.15 | 6502 | 7321 | 12973 | 11353 |
| NM_001256139 | CAPG | 0.05 | 0.00 | 958 | 1097 | 3556 | 3530 |
| NM_005186 | CAPN1 | 0.33 | 0.00 | 4130 | 5456 | 9843 | 9636 |
| NM_024537 | CARS2 | 0.13 | -0.25 | 1424 | 1696 | 3508 | 2782 |
| NM_144508 | CASC5 | 0.15 | -0.86 | 529 | 674 | 670 | 314 |
| NM_020764 | CASKIN1 | 0.02 | 0.00 | 276 | 339 | 362 | 390 |
| NM_032982 | CASP2 | 0.42 | 0.00 | 625 | 945 | 925 | 924 |
| NM_004346 | CASP3 | 0.02 | 0.00 | 639 | 733 | 593 | 567 |
| NM_001225 | CASP4 | 0.46 | 0.00 | 578 | 900 | 1115 | 1017 |
| NM_001267056 | CASP7 | 0.14 | 0.00 | 316 | 413 | 445 | 425 |
| NM_001080125 | CASP8 | 0.47 | -0.04 | 86 | 162 | 272 | 214 |
| NM_001752 | CAT | 0.27 | 0.00 | 519 | 717 | 2510 | 2628 |
| NM_170662 | CBLB | 0.22 | 0.00 | 980 | 1257 | 2620 | 2540 |
| NM_001284291 | CBLL1 | 0.11 | 0.00 | 518 | 640 | 753 | 828 |
| NM_001286789 | CBR1 | 0.36 | 0.00 | 1619 | 2244 | 4163 | 4216 |
| NM_001127228 | CBX1 | 0.50 | 0.00 | 494 | 797 | 1067 | 1030 |
| NM_007276 | CBX3 | 0.10 | 0.00 | 821 | 984 | 1181 | 1201 |
| RefSeq ID | Gene Symbol | SR GFOLD | SEN GFOLD | SR IL-2 - | SR IL-2 + | SEN IL-2- | SEN IL-2 + |
| NM_014292 | CBX6 | 0.18 | -0.06 | 1274 | 1571 | 2851 | 2566 |
| NM_032449 | CC2D1B | 0.34 | 0.00 | 1019 | 1423 | 2246 | 2259 |
| NM_021174 | CCAR2 | 0.11 | -0.13 | 2194 | 2538 | 3438 | 2972 |
| NM_133459 | CCBE1 | 0.25 | -0.34 | 2421 | 3064 | 4280 | 3203 |
| NM_001008661 | CCBL2 | 0.45 | 0.00 | 162 | 278 | 391 | 421 |
| NM_013301 | CCDC 106 | 0.64 | 0.00 | 11 | 41 | 187 | 227 |
| NM_001163323 | CCDC120 | 0.69 | 0.00 | 35 | 89 | 60 | 85 |
| NM_176816 | CCDC125 | 0.05 | 0.00 | 94 | 133 | 260 | 261 |

(continued)

| RefSeq ID | Gene Symbol | SR GFOLD | SEN GFOLD | SR IL-2 - | SR IL-2 + | SEN IL-2- | SEN IL-2 + |
|---|---|---|---|---|---|---|---|
| NM_024821 | CCDC134 | 0.42 | 0.00 | 106 | 188 | 162 | 205 |
| NM_025004 | CCDC15 | 0.03 | -0.42 | 114 | 155 | 146 | 79 |
| NM_016474 | CCDC174 | 0.15 | 0.00 | 148 | 211 | 279 | 323 |
| NM_025057 | CCDC176 | 0.02 | 0.01 | 31 | 53 | 123 | 163 |
| NM_152492 | CCDC27 | 1.25 | 0.00 | 22 | 90 | 58 | 55 |
| NM_024296 | CCDC28B | 0.23 | -0.33 | 79 | 129 | 160 | 94 |
| NM_001256964 | CCDC51 | 0.20 | 0.00 | 191 | 273 | 327 | 375 |
| NM_001017928 | CCDC58 | 0.90 | 0.00 | 23 | 74 | 44 | 31 |
| NM_014167 | CCDC59 | 0.41 | 0.00 | 125 | 215 | 210 | 233 |
| NM_175884 | CCDC71L | 0.06 | -0.15 | 508 | 607 | 1117 | 909 |
| NM_138770 | CCDC74A | 0.88 | 0.00 | 14 | 52 | 381 | 431 |
| NM_001130147 | CCDC77 | 0.04 | 0.00 | 222 | 282 | 302 | 360 |
| NM_001080433 | CCDC85A | 0.50 | -0.58 | 335 | 558 | 603 | 346 |
| NM_006848 | CCDC85B | 0.42 | -0.18 | 669 | 1004 | 965 | 762 |
| NM_024098 | CCDC86 | 0.08 | -0.21 | 1184 | 1371 | 1310 | 1031 |
| NM_001135597 | CCDC88A | 0.03 | -0.06 | 1271 | 1424 | 2379 | 2122 |
| NM_152723 | CCDC89 | 1.11 | 0.00 | 13 | 58 | 67 | 65 |
| NM_018074 | CCDC94 | 0.29 | -0.02 | 239 | 354 | 435 | 364 |
| NM_052848 | CCDC97 | 0.14 | 0.00 | 438 | 561 | 628 | 579 |
| NM_001105564 | CCHCR1 | 0.38 | -0.08 | 500 | 743 | 988 | 837 |
| NM_002982 | CCL2 | 0.15 | -0.23 | 98 | 147 | 160 | 102 |
| NM_001237 | CCNA2 | 0.28 | -0.80 | 898 | 1208 | 778 | 388 |
| NM_031966 | CCNB1 | 0.24 | -0.93 | 1156 | 1499 | 1849 | 880 |
| NM_004701 | CCNB2 | 0.20 | -0.57 | 355 | 481 | 489 | 277 |
| NM_005190 | CCNC | 0.43 | 0.00 | 349 | 550 | 568 | 628 |
| NM_057749 | CCNE2 | 0.20 | 0.00 | 203 | 289 | 108 | 89 |
| NM_001761 | CCNF | 0.02 | 0.00 | 701 | 800 | 428 | 412 |
| NM_001199189 | CCNH | 0.12 | 0.00 | 476 | 595 | 774 | 728 |
| NM_014711 | CCP110 | 0.02 | 0.00 | 301 | 366 | 401 | 431 |
| NM_006431 | CCT2 | 0.15 | -0.02 | 2352 | 2781 | 3571 | 3327 |
| NM_006430 | CCT4 | 0.21 | -0.03 | 3016 | 3695 | 5648 | 5292 |

(continued)

| RefSeq ID | Gene Symbol | SR GFOLD | SEN GFOLD | SR IL-2- | SR IL-2 + | SEN IL-2- | SEN IL. 2 + |
|---|---|---|---|---|---|---|---|
| NM_012073 | CCT5 | 0.36 | -0.26 | 4545 | 6112 | 6971 | 5599 |
| NM_001762 | CCT6A | 0.32 | -0.05 | 1760 | 2369 | 3232 | 2937 |
| NM_001166285 | CCT7 | 0.19 | -0.08 | 3521 | 4249 | 7420 | 6747 |
| NM_001282907 | CCT8 | 0.13 | 0.00 | 1184 | 1417 | 1568 | 1630 |
| NM_133493 | CD109 | 0.03 | -0.05 | 1845 | 2033 | 8131 | 7582 |
| NM_020404 | CD248 | 0.02 | -0.68 | 5111 | 5422 | 9229 | 5532 |
| NM_014143 | CD274 | 0.08 | 0.00 | 329 | 414 | 168 | 166 |
| NM_001024736 | CD276 | 0.15 | 0.00 | 6196 | 7135 | 11178 | 11347 |
| NM_006110 | CD2BP2 | 0.10 | -0.08 | 1254 | 1468 | 2123 | 1871 |
| NM_030911 | CDADC1 | 0.14 | 0.00 | 100 | 149 | 189 | 176 |
| NM_138477 | CDAN1 | 0.07 | 0.00 | 457 | 557 | 734 | 826 |
| NM_006023 | CDC123 | 0.67 | 0.00 | 690 | 1227 | 1838 | 1880 |
| NM_003672 | CDC14A | 0.39 | 0.00 | 31 | 67 | 83 | 100 |
| NM_001255 | CDC20 | 0.11 | -1.31 | 886 | 1061 | 786 | 270 |
| NM_001789 | CDC25A | 0.15 | 0.00 | 547 | 692 | 160 | 126 |
| NM_139286 | CDC26 | 0.46 | 0.00 | 76 | 145 | 152 | 185 |
| NM_001114091 | CDC27 | 0.43 | 0.00 | 940 | 1396 | 1963 | 2022 |
| NM_004359 | CDC34 | 0.01 | 0.00 | 1345 | 1482 | 1645 | 1665 |
| NM_007065 | CDC37 | 0.16 | 0.00 | 2525 | 3002 | 4309 | 4490 |
| NM_017913 | CDC37L1 | 0.20 | 0.00 | 186 | 267 | 410 | 421 |
| NM_001039802 | CDC42 | 0.22 | -0.02 | 2680 | 3308 | 5632 | 5301 |
| NM_152243 | CDC42EP1 | 0.36 | -0.17 | 1763 | 2434 | 2699 | 2238 |
| NM_006779 | CDC42EP2 | 0.14 | 0.00 | 1151 | 1389 | 1212 | 1201 |
| NM_006449 | CDC42EP3 | 0.32 | -0.14 | 1544 | 2083 | 3261 | 2780 |
| NM_001038707 | CDC42SE1 | 0.04 | 0.00 | 2000 | 2209 | 3290 | 3364 |
| NM_001254 | CDC6 | 0.31 | 0.00 | 845 | 1162 | 223 | 229 |
| NM_152562 | CDCA2 | 0.36 | -0.21 | 336 | 508 | 626 | 471 |
| NM_031299 | CDCA3 | 0.19 | -0.91 | 344 | 463 | 445 | 194 |
| NM_017955 | CDCA4 | 0.31 | 0.00 | 648 | 906 | 713 | 734 |
| NM_080668 | CDCA5 | 0.10 | -0.72 | 803 | 965 | 753 | 397 |
| NM_001127370 | CDCA7L | 0.23 | -0.10 | 697 | 921 | 370 | 287 |
| NM_001796 | CDH8 | 0.24 | 0.00 | 234 | 336 | 258 | 266 |

(continued)

| RefSeq ID | Gene Symbol | SR GFOLD | SEN GFOLD | SR IL-2- | SR IL-2 + | SEN IL-2- | SEN IL. 2 + |
|---|---|---|---|---|---|---|---|
| NM_001786 | CDK1 | 0.08 | -0.42 | 349 | 437 | 314 | 189 |
| NM_052988 | CDK10 | 0.48 | 0.00 | 588 | 924 | 984 | 974 |
| NM_001798 | CDK2 | 0.30 | -0.34 | 1220 | 1639 | 1040 | 734 |
| NM_000075 | CDK4 | 0.28 | -0.29 | 1626 | 2130 | 3438 | 2643 |
| NM_004935 | CDK5 | 0.58 | 0.00 | 47 | 105 | 185 | 155 |
| NM_001278167 | CDK5RAP1 | 0.46 | -0.03 | 264 | 434 | 751 | 651 |
| NM_001278216 | CDK5RAP3 | 0.34 | 0.00 | 375 | 554 | 1011 | 1083 |
| RefSeq ID | Gene Symbol | SR GFOLD | SEN GFOLD | SR IL-2- | SR IL-2 + | SEN IL-2 | SEN IL-2 + |
| NM_001261 | CDK9 | 0.11 | 0.00 | 1139 | 1352 | 2211 | 2146 |
| NM_001220778 | CDKN1A | 0.13 | -0.03 | 13883 | 15626 | 32012 | 30770 |
| NM_004064 | CDKN1B | 0.08 | 0.00 | 434 | 534 | 426 | 442 |
| NM_017632 | CDKN2AIP | 0.25 | 0.00 | 248 | 358 | 389 | 407 |
| NM_080656 | CDKN2AIPNL | 0.03 | -0.03 | 189 | 242 | 343 | 279 |
| NM_001262 | CDKN2C | 0.22 | -0.33 | 430 | 579 | 339 | 220 |
| NM_005192 | CDKN3 | 0.03 | -0.99 | 275 | 339 | 331 | 131 |
| NM_001134422 | CDV3 | 0.27 | 0.00 | 3561 | 4532 | 3851 | 3970 |
| NM_152342 | CDYL2 | 0.08 | 0.00 | 446 | 545 | 674 | 721 |
| NM_005195 | CEBPD | 0.42 | -0.23 | 62 | 119 | 125 | 76 |
| NM_018689 | CEMIP | 0.17 | -0.38 | 9699 | 11231 | 3955 | 2869 |
| NM_001812 | CENPC | 0.58 | 0.00 | 76 | 157 | 135 | 152 |
| NM_024053 | CENPM | 0.55 | -0.91 | 230 | 407 | 329 | 139 |
| NM_001100624 | CENPN | 0.17 | -0.24 | 1035 | 1282 | 1179 | 900 |
| NM_001012267 | CENPP | 0.22 | 0.00 | 123 | 187 | 337 | 299 |
| NM_025082 | CENPT | 0.53 | -0.03 | 741 | 1193 | 1984 | 1806 |
| NM_024629 | CENPU | 0.44 | 0.00 | 140 | 243 | 191 | 152 |
| NM_181716 | CENPV | 0.02 | 0.00 | 299 | 363 | 316 | 316 |
| NM_001012507 | CENPW | 0.38 | -0.96 | 141 | 235 | 237 | 92 |
| NM_014956 | CEP164 | 0.12 | 0.00 | 806 | 980 | 1583 | 1510 |
| NM_032142 | CEP192 | 0.17 | 0.00 | 648 | 824 | 1102 | 1190 |
| NM_018718 | CEP41 | 0.38 | -0.04 | 141 | 235 | 618 | 525 |
| NM_015147 | CEP68 | 0.03 | 0.00 | 246 | 306 | 701 | 773 |

(continued)

| RefSeq ID | Gene Symbol | SR GFOLD | SEN GFOLD | SR IL-2- | SR IL-2 + | SEN IL-2 | SEN IL-2 + |
|---|---|---|---|---|---|---|---|
| NM_001098802 | CEP78 | 0.30 | -0.02 | 203 | 308 | 514 | 436 |
| NM_022778 | CEP85 | 0.48 | 0.00 | 542 | 856 | 564 | 645 |
| NM_032816 | CEP89 | 0.19 | -0.08 | 334 | 450 | 990 | 841 |
| NM_005507 | CFL1 | 0.17 | -0.09 | 15387 | 17812 | 29260 | 26863 |
| NM_001243645 | CFL2 | 0.32 | -0.13 | 1962 | 2633 | 4663 | 4042 |
| NM_033043 | CGB5 | 0.71 | 0.00 | 20 | 59 | 17 | 17 |
| NM_033183 | CGB8 | 0.48 | 0.00 | 21 | 53 | 27 | 33 |
| NM_001008390 | CGGBP1 | 0.13 | 0.00 | 1496 | 1774 | 2889 | 2937 |
| NM_138481 | CHADL | 0.94 | 0.00 | 13 | 52 | 110 | 83 |
| NM_005483 | CHAF1A | 0.09 | -0.13 | 1046 | 1225 | 707 | 566 |
| NM_005441 | CHAF1B | 0.19 | -0.34 | 332 | 447 | 272 | 172 |
| NM_001164144 | CHAMP 1 | 0.31 | 0.00 | 620 | 869 | 1000 | 915 |
| NM_203298 | CHCHD1 | 0.06 | 0.00 | 547 | 653 | 817 | 835 |
| NM_017812 | CHCHD3 | 0.07 | -0.03 | 540 | 649 | 1171 | 1035 |
| NM_001011667 | CHCHD7 | 0.41 | 0.00 | 121 | 210 | 356 | 318 |
| NM_001005735 | CHEK2 | 0.48 | 0.00 | 118 | 214 | 154 | 133 |
| RefSeq ID | Gene Symbol | SR GFOLD | SEN GFOLD | SR IL- 2 - | SR IL-2 + | SEN IL- 2- | SEN IL- 2 + |
| NM_014043 | CHMP2B | 0.19 | 0.00 | 580 | 752 | 759 | 797 |
| NM_001144073 | CHORDC1 | 0.09 | 0.00 | 231 | 301 | 258 | 244 |
| NM_019886 | CHST7 | 0.38 | 0.00 | 91 | 160 | 229 | 270 |
| NM_175856 | CHSY3 | 0.12 | 0.00 | 427 | 539 | 439 | 497 |
| NM_022092 | CHTF18 | 0.05 | 0.00 | 298 | 370 | 229 | 183 |
| NM_001039690 | CHTF8 | 0.26 | 0.00 | 2001 | 2575 | 3236 | 3281 |
| NM_001206612 | CHTOP | 0.02 | 0.00 | 1419 | 1572 | 1788 | 1895 |
| NM_001278 | CHUK | 0.09 | 0.00 | 591 | 718 | 938 | 876 |
| NM_144697 | CIART | 0.03 | 0.00 | 174 | 225 | 460 | 479 |
| NM_032630 | CINP | 0.17 | -0.08 | 411 | 539 | 1017 | 865 |
| NM_004882 | CIR1 | 0.03 | 0.00 | 181 | 232 | 399 | 396 |
| NM_018464 | CISD1 | 0.10 | 0.00 | 158 | 216 | 343 | 388 |

(continued)

| RefSeq ID | Gene Symbol | SR GFOLD | SEN GFOLD | SR IL-2 - | SR IL-2 + | SEN IL-2- | SEN IL-2 + |
|---|---|---|---|---|---|---|---|
| NM_ 001136498 | CISD3 | 0.12 | -0.22 | 716 | 875 | 1647 | 1299 |
| NM_013324 | CISH | 0.93 | -0.42 | 31 | 95 | 56 | 24 |
| NM_ 001206999 | CIT | 0.26 | -0.99 | 1141 | 1497 | 1739 | 791 |
| NM_ 001131016 | CIZ1 | 0.17 | 0.00 | 2897 | 3451 | 7302 | 7246 |
| NM_ 001098525 | CKAP2 | 0.02 | -0.03 | 967 | 1090 | 1115 | 985 |
| NM_152515 | CKAP2L | 0.05 | -0.42 | 621 | 729 | 493 | 311 |
| NM_ 001008938 | CKAP5 | 0.10 | 0.00 | 3832 | 4318 | 6114 | 5967 |
| NM_007056 | CLASRP | 0.13 | 0.00 | 610 | 758 | 622 | 617 |
| NM_ 001127899 | CLCN5 | 0.11 | 0.00 | 413 | 521 | 555 | 623 |
| NM_005602 | CLDN11 | 0.07 | -1.05 | 4182 | 4626 | 2909 | 1299 |
| NM_002975 | CLEC11A | 0.25 | -0.02 | 1071 | 1403 | 1601 | 1449 |
| NM_175060 | CLEC14A | 0.02 | -1.46 | 125 | 167 | 370 | 104 |
| NM_015226 | CLEC16A | 0.21 | 0.00 | 648 | 844 | 944 | 996 |
| NM_ 001004419 | CLEC2D | 0.08 | 0.00 | 193 | 255 | 349 | 305 |
| NM_ 001287594 | CLIC1 | 0.31 | -0.40 | 6592 | 8471 | 17949 | 13255 |
| NM_015526 | CLIP3 | 0.09 | -0.21 | 487 | 596 | 2493 | 2015 |
| NM_000086 | CLN3 | 0.15 | -0.09 | 666 | 836 | 1454 | 1250 |
| NM_001293 | CLNS1A | 0.75 | 0.00 | 323 | 636 | 740 | 787 |
| NM_006831 | CLP1 | 0.29 | 0.00 | 249 | 368 | 430 | 488 |
| NM_ 001258394 | CLPB | 0.19 | -0.13 | 743 | 953 | 1414 | 1181 |
| NM_006012 | CLPP | 0.31 | 0.00 | 986 | 1343 | 1406 | 1311 |
| NM_022111 | CLSPN | 0.14 | -0.26 | 892 | 1088 | 507 | 362 |
| NM_007096 | CLTA | 0.35 | 0.00 | 1734 | 2377 | 3829 | 3956 |
| NM_015229 | CLUH | 0.20 | 0.00 | 1725 | 2138 | 2556 | 2536 |
| NM_182523 | CMC1 | 0.43 | -0.27 | 40 | 84 | 141 | 85 |
| NM_032359 | CMSS1 | 0.58 | 0.00 | 228 | 414 | 628 | 689 |
| NM_017801 | CMTM6 | 0.04 | 0.00 | 1148 | 1295 | 2217 | 2359 |
| RefSeq ID | Gene Symbol | SR GFOLD | SEN GFOLD | SR IL-2- | SR IL-2 + | SEN IL-2- | SEN IL-2 + |
| NM_ 001099642 | CMTR2 | 0.37 | 0.00 | 236 | 371 | 507 | 573 |

(continued)

| RefSeq ID | Gene Symbol | SR GFOLD | SEN GFOLD | SR IL-2- | SR IL-2 + | SEN IL-2- | SEN IL-2 + |
|---|---|---|---|---|---|---|---|
| NM_018235 | CNDP2 | 0.01 | -0.17 | 1479 | 1621 | 2903 | 2418 |
| NM_001168647 | CNKSR2 | 0.27 | 0.00 | 33 | 65 | 56 | 68 |
| NM_173515 | CNKSR3 | 0.07 | -0.07 | 435 | 530 | 320 | 250 |
| NM_004368 | CNN2 | 0.11 | -0.61 | 2892 | 3309 | 5484 | 3425 |
| NM_033133 | CNP | 0.07 | 0.00 | 2696 | 3004 | 5243 | 5261 |
| NM_006586 | CNPY3 | 0.43 | 0.00 | 803 | 1200 | 2028 | 2176 |
| NM_015463 | CNRIP1 | 0.34 | 0.00 | 731 | 1037 | 1333 | 1299 |
| NM_152609 | CNST | 0.28 | 0.00 | 1781 | 2324 | 2105 | 2211 |
| NM_007018 | CNTRL | 0.04 | -0.33 | 273 | 340 | 443 | 296 |
| NM_001040431 | COA3 | 0.13 | 0.00 | 311 | 405 | 1181 | 1105 |
| NM_016565 | COA4 | 0.25 | -0.07 | 630 | 845 | 1198 | 1033 |
| NM_001206641 | COA6 | 1.93 | 0.00 | 36 | 208 | 204 | 203 |
| NM_023077 | COA7 | 0.45 | -0.13 | 292 | 474 | 611 | 484 |
| NM_015386 | COG4 | 0.31 | -0.02 | 924 | 1270 | 2489 | 2292 |
| NM_153603 | COG7 | 0.10 | 0.00 | 696 | 843 | 1160 | 1209 |
| NM_032382 | COG8 | 0.19 | 0.00 | 624 | 806 | 1248 | 1244 |
| NM_001854 | COL11A1 | 0.31 | 0.00 | 2126 | 2815 | 3910 | 3924 |
| NM_001849 | COL6A2 | 0.03 | 0.00 | 17602 | 18385 | 31346 | 31434 |
| NM_152516 | COMMD1 | 0.19 | -0.18 | 270 | 371 | 803 | 627 |
| NM_001287392 | COMMD6 | 0.18 | 0.00 | 218 | 304 | 699 | 651 |
| NM_144589 | COMTD1 | 0.50 | 0.00 | 17 | 46 | 87 | 65 |
| NM_007263 | COPE | 0.11 | 0.00 | 3180 | 3636 | 5840 | 6035 |
| NM_016128 | COPG1 | 0.30 | 0.00 | 8316 | 10594 | 13545 | 13268 |
| NM_012133 | COPG2 | 0.06 | 0.00 | 246 | 312 | 709 | 802 |
| NM_003653 | COPS3 | 0.28 | 0.00 | 738 | 1002 | 1314 | 1340 |
| NM_006833 | COPS6 | 0.03 | -0.16 | 1970 | 2160 | 4889 | 4159 |
| NM_001282950 | COPS7B | 0.02 | 0.00 | 749 | 851 | 1266 | 1153 |
| NM_001099337 | COQ10A | 0.07 | 0.00 | 97 | 138 | 148 | 144 |
| NM_016035 | COQ4 | 0.04 | -0.29 | 203 | 260 | 1023 | 750 |
| NM_001018070 | CORO1B | 0.18 | 0.00 | 1592 | 1956 | 3161 | 3129 |
| NM_014325 | CORO1C | 0.33 | 0.00 | 17437 | 22410 | 26274 | 25663 |
| NM_052820 | CORO2A | 0.04 | -0.12 | 201 | 257 | 383 | 294 |

(continued)

| RefSeq ID | Gene Symbol | SR GFOLD | SEN GFOLD | SR IL-2- | SR IL-2 + | SEN IL-2- | SEN IL-2 + |
|---|---|---|---|---|---|---|---|
| NM_024535 | CORO7 | 0.90 | 0.00 | 166 | 383 | 722 | 691 |
| NM_001201479 | CORO7-PAM16 | 0.54 | 0.00 | 348 | 590 | 1015 | 1004 |
| NM_001303 | COX10 | 0.22 | 0.00 | 244 | 346 | 331 | 375 |
| NM_004375 | COX11 | 0.18 | 0.00 | 293 | 398 | 566 | 579 |
| NM_078470 | COX15 | 0.16 | 0.00 | 562 | 715 | 1208 | 1150 |
| NM_001863 | COX6B1 | 0.40 | -0.12 | 1002 | 1459 | 3288 | 2848 |
| RefSeq ID | Gene Symbol | SR GFOLD | SN GFOLD | SR IL-2- | SR IL-2 + | SEN IL-2- | SEN IL-2 + |
| NM_001865 | COX7A2 | 0.28 | 0.00 | 732 | 996 | 1346 | 1229 |
| NM_004718 | COX7A2L | 0.79 | 0.00 | 775 | 1488 | 2092 | 1998 |
| NM_001866 | COX7B | 0.61 | 0.00 | 410 | 723 | 1011 | 985 |
| NM_001867 | COX7C | 0.05 | -0.03 | 662 | 776 | 1123 | 996 |
| NM_004074 | COX8A | 0.58 | -0.40 | 1081 | 1764 | 2993 | 2116 |
| NM_024913 | CPED1 | 0.04 | 0.00 | 1125 | 1268 | 1934 | 1911 |
| NM_003915 | CPNE1 | 0.20 | 0.00 | 3287 | 3981 | 5580 | 5490 |
| NM_152727 | CPNE2 | 0.11 | -0.07 | 599 | 734 | 851 | 719 |
| NM_001122633 | CPS1 | 0.28 | -0.13 | 102 | 166 | 320 | 240 |
| NM_013291 | CPSF1 | 0.05 | 0.00 | 2011 | 2238 | 3090 | 3209 |
| NM_017437 | CPSF2 | 0.14 | 0.00 | 1234 | 1487 | 2263 | 2340 |
| NM_016207 | CPSF3 | 0.37 | 0.00 | 452 | 675 | 1025 | 1103 |
| NM_001256456 | CPSF3L | 0.57 | -0.09 | 995 | 1625 | 2406 | 2107 |
| NM_007007 | CPSF6 | 0.24 | 0.00 | 1047 | 1356 | 1822 | 1830 |
| NM_001136040 | CPSF7 | 0.24 | 0.00 | 1257 | 1620 | 1537 | 1671 |
| NM_001876 | CPT1A | 0.04 | -0.06 | 2237 | 2467 | 4074 | 3697 |
| NM_001136052 | CPT1C | 0.51 | 0.00 | 83 | 162 | 208 | 207 |
| NM_000098 | CPT2 | 0.04 | 0.00 | 288 | 356 | 657 | 590 |
| NM_001029885 | CPTP | 0.18 | 0.00 | 351 | 470 | 757 | 745 |
| NM_198148 | CPXM2 | 0.07 | -1.27 | 73 | 109 | 237 | 72 |
| NM_001144958 | CRACR2A | 0.23 | -0.01 | 174 | 256 | 514 | 438 |
| NM_003805 | CRADD | 0.15 | 0.00 | 217 | 297 | 304 | 349 |
| NM_194071 | CREB3L2 | 0.25 | 0.00 | 9787 | 12023 | 11617 | 11621 |
| NM_015986 | CRLF3 | 0.06 | 0.00 | 388 | 475 | 428 | 486 |

(continued)

| RefSeq ID | Gene Symbol | SR GFOLD | SN GFOLD | SR IL-2- | SR IL-2 + | SEN IL-2- | SEN IL-2 + |
|---|---|---|---|---|---|---|---|
| NM_014675 | CROCC | 0.03 | -0.02 | 136 | 181 | 387 | 320 |
| NM_001098482 | CRTC1 | 0.08 | 0.00 | 260 | 333 | 534 | 538 |
| NM_001130042 | CRYZ | 0.22 | 0.00 | 311 | 432 | 1102 | 1018 |
| NM_004077 | CS | 0.13 | -0.15 | 1943 | 2282 | 3980 | 3408 |
| NM_001316 | CSE1L | 0.14 | -0.14 | 2065 | 2432 | 3818 | 3283 |
| NM_022048 | CSNK1G1 | 0.15 | -0.03 | 755 | 936 | 1425 | 1277 |
| NM_001897 | CSPG4 | 0.12 | 0.00 | 2905 | 3339 | 7803 | 7933 |
| NM_020536 | CSRP2BP | 0.29 | 0.00 | 465 | 655 | 998 | 980 |
| NM_001324 | CSTF1 | 0.08 | 0.00 | 517 | 628 | 759 | 795 |
| NM_001325 | CSTF2 | 0.21 | -0.06 | 333 | 455 | 709 | 599 |
| NM_015235 | CSTF2T | 0.18 | 0.00 | 534 | 690 | 882 | 854 |
| NM_001326 | CSTF3 | 0.17 | 0.00 | 314 | 421 | 568 | 616 |
| NM_001012614 | CTBP1 | 0.15 | 0.00 | 2861 | 3363 | 4706 | 4693 |
| NM_015343 | CTDNEP1 | 0.36 | -0.22 | 854 | 1217 | 1593 | 1250 |
| NM_001008392 | CTDSPL | 0.12 | 0.00 | 723 | 881 | 1235 | 1325 |
| RefSeq ID | Gene Symbol | SR GFOLD | SEN GFOLD | SR IL-2- | SR IL-2 + | SEN IL- 2- | SEN IL-2 + |
| NM_001085458 | CTNND1 | 0.23 | 0.00 | 2836 | 3534 | 5823 | 5965 |
| NM_175859 | CTPS2 | 0.33 | -0.04 | 240 | 365 | 620 | 525 |
| NM_001814 | CTSC | 0.19 | -0.35 | 921 | 1163 | 607 | 412 |
| NM_004079 | CTSS | 0.20 | 0.00 | 14 | 34 | 137 | 146 |
| NM_005231 | CTTN | 0.23 | -0.04 | 8090 | 9832 | 20056 | 19079 |
| NM_033427 | CTTNBP2 | 0.51 | 0.00 | 19 | 50 | 435 | 460 |
| NM_145232 | CTU1 | 0.24 | 0.00 | 46 | 83 | 46 | 59 |
| NM_024040 | CUEDC2 | 0.26 | -0.37 | 596 | 811 | 2075 | 1486 |
| NM_001079872 | CUL4B | 0.23 | -0.06 | 1410 | 1799 | 3558 | 3222 |
| NM_020943 | CWC22 | 0.02 | 0.00 | 236 | 293 | 412 | 453 |
| NM_199168 | CXCL12 | 0.04 | 0.00 | 3249 | 3535 | 3022 | 3081 |
| NM_001171907 | CXorf40A | 0.58 | 0.00 | 215 | 391 | 412 | 397 |
| NM_001013845 | CXorf40B | 0.26 | 0.00 | 392 | 549 | 678 | 599 |
| NM_001161454 | CYB561A3 | 0.02 | 0.00 | 952 | 1073 | 1932 | 1813 |

(continued)

| RefSeq ID | Gene Symbol | SR GFOLD | SEN GFOLD | SR IL-2- | SR IL-2 + | SEN IL- 2- | SEN IL-2 + |
|---|---|---|---|---|---|---|---|
| NM_001914 | CYB5A | 0.63 | -0.16 | 109 | 221 | 576 | 445 |
| NM_030579 | CYB5B | 0.47 | -0.06 | 890 | 1366 | 2360 | 2113 |
| NM_016229 | CYB5R2 | 0.28 | 0.00 | 136 | 213 | 287 | 259 |
| NM_032412 | CYSTM1 | 0.29 | -0.15 | 473 | 665 | 3088 | 2612 |
| NM_145056 | DACT3 | 0.29 | 0.00 | 47 | 87 | 250 | 237 |
| NM_033657 | DAP3 | 0.33 | 0.00 | 1061 | 1460 | 2579 | 2579 |
| NM_014326 | DAPK2 | 1.38 | 0.00 | 6 | 49 | 89 | 91 |
| NM_001348 | DAPK3 | 0.29 | -0.04 | 1889 | 2474 | 3371 | 3100 |
| NM_001141969 | DAXX | 0.30 | 0.00 | 1039 | 1411 | 2036 | 2081 |
| NM_018959 | DAZAP1 | 0.10 | -0.26 | 1886 | 2179 | 1612 | 1231 |
| NM_001178042 | DBI | 0.32 | 0.00 | 475 | 683 | 1142 | 1068 |
| NM_001048222 | DBNDD2 | 0.05 | 0.00 | 804 | 928 | 595 | 566 |
| NM_001014436 | DBNL | 0.45 | 0.00 | 1548 | 2279 | 6118 | 5946 |
| NM_025230 | DCAF11 | 0.14 | 0.00 | 851 | 1041 | 1932 | 1926 |
| NM_015397 | DCAF12 | 0.37 | 0.00 | 802 | 1151 | 1264 | 1368 |
| NM_015420 | DCAF13 | 0.44 | 0.00 | 418 | 657 | 969 | 900 |
| NM_017741 | DCAF16 | 0.26 | -0.04 | 492 | 678 | 747 | 643 |
| NM_005828 | DCAF7 | 0.17 | 0.00 | 2298 | 2766 | 3843 | 3797 |
| NM_000788 | DCK | 0.79 | 0.00 | 393 | 782 | 826 | 876 |
| NM_152640 | DCP1B | 0.22 | 0.00 | 183 | 267 | 543 | 553 |
| NM_001261412 | DCTN2 | 0.43 | -0.06 | 2286 | 3274 | 6249 | 5754 |
| NM_001281426 | DCTN3 | 0.30 | 0.00 | 510 | 716 | 1508 | 1536 |
| NM_001199743 | DCTN5 | 0.17 | 0.00 | 1795 | 2171 | 4047 | 4126 |
| NM_024096 | DCTPP1 | 0.57 | -0.17 | 422 | 723 | 809 | 636 |
| NM_032299 | DCUN1D5 | 0.30 | 0.00 | 511 | 722 | 751 | 797 |
| NM_001195218 | DCXR | 0.33 | 0.00 | 429 | 625 | 792 | 702 |
| NM_024050 | DDA1 | 0.01 | 0.00 | 739 | 837 | 1079 | 1168 |
| NM_013974 | DDAH2 | 0.16 | 0.00 | 794 | 994 | 1828 | 1911 |
| NM_000107 | DDB2 | 0.33 | 0.00 | 570 | 814 | 932 | 1000 |
| NM_032341 | DDI2 | 0.12 | 0.00 | 260 | 341 | 497 | 556 |
| NM_145244 | DDIT4L | 0.33 | 0.00 | 113 | 187 | 146 | 139 |

(continued)

| RefSeq ID | Gene Symbol | SR GFOLD | SEN GFOLD | SR IL-2- | SR IL-2 + | SEN IL- 2- | SEN IL-2 + |
|---|---|---|---|---|---|---|---|
| NM_ 001014796 | DDR2 | 0.10 | 0.00 | 3392 | 3842 | 10259 | 9911 |
| NM_023935 | DDRGK1 | 0.08 | 0.00 | 516 | 626 | 1383 | 1312 |
| NM_007242 | DDX19B | 0.02 | 0.00 | 621 | 717 | 998 | 1004 |
| NM_007204 | DDX20 | 0.24 | 0.00 | 366 | 508 | 476 | 469 |
| NM_004728 | DDX21 | 0.39 | -0.01 | 3021 | 4194 | 3937 | 3717 |
| NM_004818 | DDX23 | 0.18 | -0.01 | 1253 | 1549 | 2019 | 1863 |
| NM_022779 | DDX31 | 0.35 | 0.00 | 250 | 386 | 695 | 678 |
| NM_005804 | DDX39A | 0.23 | -0.58 | 1537 | 1946 | 2019 | 1238 |
| NM_004640 | DDX39B | 0.07 | -0.06 | 1352 | 1552 | 2489 | 2229 |
| NM_014829 | DDX46 | 0.01 | 0.00 | 1239 | 1369 | 1285 | 1320 |
| NM_ 001111322 | DDX54 | 0.05 | 0.00 | 1723 | 1922 | 2446 | 2327 |
| NM_020936 | DDX55 | 0.25 | 0.00 | 347 | 487 | 524 | 479 |
| NM_019082 | DDX56 | 0.14 | 0.00 | 1199 | 1445 | 1818 | 1885 |
| NM_014314 | DDX58 | 0.09 | 0.00 | 119 | 167 | 420 | 362 |
| NM_017631 | DDX60 | 0.23 | 0.00 | 120 | 185 | 281 | 235 |
| NM_032998 | DEDD | 0.20 | 0.00 | 661 | 854 | 1354 | 1270 |
| NM_020946 | DENND1A | 0.34 | 0.00 | 506 | 732 | 1142 | 1194 |
| NM_ 001195215 | DENND1B | 0.02 | 0.00 | 218 | 273 | 430 | 494 |
| NM_014957 | DENND3 | 0.13 | 0.00 | 485 | 612 | 720 | 704 |
| NM_014856 | DENND4B | 0.08 | 0.00 | 1043 | 1219 | 1237 | 1253 |
| NM_152678 | DENND6A | 0.09 | -0.17 | 227 | 297 | 347 | 255 |
| NM_018369 | DEPDC1B | 1.39 | 0.00 | 27 | 115 | 54 | 48 |
| NM_ 001077242 | DEPDC7 | 0.60 | 0.00 | 70 | 148 | 227 | 237 |
| NM_022783 | DEPTOR | 0.09 | -0.24 | 42 | 70 | 96 | 55 |
| NM_015954 | DERA | 0.29 | 0.00 | 407 | 578 | 749 | 717 |
| NM_001927 | DES | 0.43 | -0.07 | 362 | 569 | 135 | 94 |
| NM_213566 | DFFA | 0.09 | -0.01 | 886 | 1047 | 1840 | 1691 |
| NM_022719 | DGCR14 | 0.58 | 0.00 | 258 | 462 | 688 | 671 |
| NM_ 001199266 | DGKZ | 0.37 | 0.00 | 135 | 225 | 287 | 251 |
| NM_000791 | DHFR | 0.13 | 0.00 | 320 | 415 | 331 | 346 |
| NM_001930 | DHPS | 0.05 | 0.00 | 1104 | 1262 | 2159 | 2013 |
| NM_ 001136050 | DHRS1 | 0.57 | 0.00 | 153 | 286 | 1071 | 1046 |
| NM_024308 | DHRS11 | 0.74 | -0.17 | 35 | 92 | 154 | 102 |

(continued)

| RefSeq ID | Gene Symbol | SR GFOLD | SEN GFOLD | SR IL-2- | SR IL-2 + | SEN IL- 2- | SEN IL-2 + |
|---|---|---|---|---|---|---|---|
| NM_001193636 | DHRS4L2 | 0.17 | -0.42 | 103 | 156 | 308 | 185 |
| NM_001358 | DHX15 | 0.11 | -0.01 | 3010 | 3437 | 4321 | 4085 |
| NM_003587 | DHX16 | 0.40 | -0.03 | 1402 | 2005 | 2196 | 2006 |
| NM_019030 | DHX29 | 0.29 | 0.00 | 550 | 768 | 1117 | 1233 |
| NM_020162 | DHX33 | 0.25 | 0.00 | 687 | 921 | 880 | 893 |
| NM_014681 | DHX34 | 0.18 | 0.00 | 472 | 616 | 638 | 682 |
| NM_032656 | DHX37 | 0.30 | 0.00 | 865 | 1178 | 1468 | 1381 |
| NM_004941 | DHX8 | 0.13 | -0.16 | 1247 | 1494 | 2219 | 1852 |
| NM_005219 | DIAPH1 | 0.45 | 0.00 | 4232 | 6062 | 7185 | 7037 |
| NM_001042517 | DIAPH3 | 0.03 | -0.63 | 1363 | 1521 | 1050 | 603 |
| NM_014473 | DIMT1 | 0.08 | -0.02 | 680 | 809 | 1034 | 917 |
| NM_001931 | DLAT | 0.22 | 0.00 | 785 | 1023 | 1506 | 1444 |
| NM_006094 | DLC1 | 0.25 | 0.00 | 7520 | 9279 | 8412 | 8517 |
| NM_004405 | DLX2 | 0.74 | 0.00 | 19 | 58 | 125 | 155 |
| NM_019100 | DMAP1 | 0.52 | 0.00 | 315 | 534 | 1138 | 1148 |
| NM_001174116 | DMXL2 | 0.34 | 0.00 | 367 | 544 | 1333 | 1340 |
| NM_001080449 | DNA2 | 0.20 | 0.00 | 145 | 214 | 114 | 146 |
| NM_207437 | DNAH10 | 0.16 | 0.00 | 63 | 102 | 183 | 196 |
| NM_001002762 | DNAJB 12 | 0.06 | 0.00 | 1360 | 1542 | 2597 | 2547 |
| NM_018198 | DNAJC 11 | 0.09 | -0.06 | 1429 | 1652 | 2298 | 2055 |
| NM_018163 | DNAJC 17 | 0.15 | -0.01 | 146 | 208 | 266 | 213 |
| NM_152686 | DNAJC 18 | 0.20 | 0.00 | 118 | 178 | 441 | 449 |
| NM_145261 | DNAJC 19 | 0.38 | 0.00 | 29 | 63 | 116 | 92 |
| NM_003315 | DNAJC7 | 0.20 | 0.00 | 782 | 1003 | 1672 | 1608 |
| NM_014280 | DNAJC8 | 0.39 | 0.00 | 806 | 1174 | 1701 | 1634 |
| NM_015190 | DNAJC9 | 0.09 | 0.00 | 499 | 610 | 557 | 573 |
| NM_005740 | DNAL4 | 0.74 | 0.00 | 85 | 192 | 356 | 386 |
| NM_194249 | DND1 | 0.38 | 0.00 | 147 | 243 | 200 | 222 |
| NM_001278464 | DNM1L | 0.13 | -0.02 | 1825 | 2155 | 3107 | 2880 |
| NM_001130823 | DNMT1 | 0.04 | -0.19 | 3614 | 3923 | 2566 | 2102 |
| NM_006892 | DNMT3B | 0.97 | 0.00 | 52 | 148 | 96 | 96 |
| NM_006443 | DNPH1 | 0.36 | 0.00 | 177 | 284 | 720 | 778 |

(continued)

| RefSeq ID | Gene Symbol | SR GFOLD | SEN GFOLD | SR IL-2- | SR IL-2 + | SEN IL- 2- | SEN IL-2 + |
|---|---|---|---|---|---|---|---|
| NM_001282062 | DOC2A | 0.51 | -0.08 | 100 | 189 | 225 | 168 |
| NM_001290223 | DOCK1 | 0.20 | 0.00 | 2592 | 3172 | 5650 | 5560 |
| NM_144658 | DOCK11 | 0.17 | 0.00 | 128 | 188 | 474 | 506 |
| NM_001145165 | DOHH | 0.28 | 0.00 | 121 | 192 | 210 | 170 |
| NM_005128 | DOPEY2 | 0.03 | -0.13 | 917 | 1037 | 2926 | 2512 |
| NM_001384 | DPH2 | 0.10 | 0.00 | 538 | 659 | 636 | 689 |
| NM_080650 | DPH6 | 0.22 | 0.00 | 31 | 60 | 87 | 59 |
| NM_003863 | DPM2 | 0.42 | -0.18 | 544 | 826 | 1377 | 1103 |
| NM_153741 | DPM3 | 0.54 | -0.20 | 176 | 319 | 745 | 569 |
| NM_005700 | DPP3 | 0.02 | 0.00 | 818 | 926 | 1599 | 1595 |
| NM_004147 | DRG1 | 0.21 | -0.12 | 963 | 1227 | 1689 | 1427 |
| NM_001388 | DRG2 | 0.10 | -0.18 | 761 | 914 | 1564 | 1261 |
| NM_032160 | DSEL | 0.02 | -0.19 | 2349 | 2550 | 4151 | 3460 |
| NM_080820 | DTD1 | 0.08 | 0.00 | 785 | 926 | 1508 | 1623 |
| NM_080664 | DTD2 | 0.19 | -0.06 | 155 | 225 | 387 | 311 |
| NM_016448 | DTL | 0.28 | -0.37 | 839 | 1135 | 518 | 342 |
| NM_001271667 | DTNBP1 | 0.36 | 0.00 | 171 | 275 | 258 | 268 |
| NM_020892 | DTX2 | 0.21 | 0.00 | 459 | 614 | 564 | 616 |
| NM_138287 | DTX3L | 0.02 | 0.00 | 523 | 610 | 759 | 689 |
| NM_012145 | DTYMK | 0.19 | -0.05 | 705 | 901 | 1177 | 1028 |
| NM_022156 | DUS1L | 0.07 | 0.00 | 854 | 1001 | 1104 | 1105 |
| NM_020175 | DUS3L | 0.17 | -0.08 | 342 | 455 | 408 | 325 |
| NM_007240 | DUSP12 | 0.14 | 0.00 | 215 | 291 | 343 | 396 |
| NM_007026 | DUSP14 | 0.12 | -0.24 | 3052 | 3523 | 6002 | 4867 |
| NM_001142314 | DUSP19 | 1.19 | 0.00 | 10 | 57 | 52 | 65 |
| NM_001947 | DUSP7 | 0.10 | 0.00 | 937 | 1115 | 807 | 847 |
| NM_001271786 | DYNC1I2 | 0.55 | 0.00 | 334 | 575 | 1210 | 1122 |
| NM_016141 | DYNC1LI1 | 0.11 | 0.00 | 681 | 831 | 1121 | 1225 |
| NM_001281729 | DYNLRB1 | 0.09 | 0.00 | 1688 | 1939 | 3334 | 3384 |
| NM_006519 | DYNLT1 | 0.23 | -0.13 | 1405 | 1787 | 2627 | 2240 |
| NM_198968 | DZIP1 | 0.16 | 0.00 | 944 | 1165 | 1986 | 1887 |
| NM_001950 | E2F4 | 0.36 | -0.04 | 1548 | 2145 | 2394 | 2179 |

(continued)

| RefSeq ID | Gene Symbol | SR GFOLD | SEN GFOLD | SR IL-2- | SR IL-2 + | SEN IL- 2- | SEN IL-2 + |
|---|---|---|---|---|---|---|---|
| NM_022659 | EBF2 | 0.07 | 0.00 | 502 | 605 | 356 | 373 |
| NM_001398 | ECH1 | 0.09 | 0.00 | 1234 | 1439 | 1537 | 1625 |
| NM_ 001002030 | ECHDC 1 | 0.20 | 0.00 | 556 | 726 | 936 | 946 |
| NM_024693 | ECHDC3 | 0.44 | 0.00 | 42 | 88 | 31 | 48 |
| NM_ 001258316 | ECT2 | 0.35 | -0.01 | 599 | 862 | 940 | 835 |
| NM_ 001142443 | EDC3 | 0.26 | 0.00 | 1042 | 1369 | 1553 | 1475 |
| NM_018217 | EDEM2 | 0.48 | 0.00 | 334 | 548 | 1146 | 1131 |
| NM_001959 | EEF1B2 | 0.46 | -0.03 | 684 | 1058 | 2015 | 1835 |
| NM_032378 | EEF1D | 0.19 | -0.14 | 1974 | 2423 | 3321 | 2834 |
| NM_004280 | EEF1E1 | 0.90 | 0.00 | 80 | 202 | 173 | 214 |
| NM_001404 | EEF1G | 0.44 | -0.04 | 6878 | 9651 | 16581 | 15728 |
| NM_032437 | EFCAB7 | 0.34 | 0.00 | 16 | 40 | 79 | 111 |
| NM_015137 | EFR3A | 0.34 | 0.00 | 1394 | 1918 | 4394 | 4397 |
| NM_ 001258354 | EFTUD2 | 0.26 | -0.02 | 2650 | 3368 | 5207 | 4906 |
| NM_005228 | EGFR | 0.06 | -0.09 | 4980 | 5431 | 5971 | 5368 |
| NM_080732 | EGLN2 | 0.04 | -0.10 | 1313 | 1473 | 2365 | 2059 |
| NM_006795 | EHD1 | 0.24 | 0.00 | 2726 | 3424 | 3275 | 3390 |
| NM_139265 | EHD4 | 0.27 | 0.00 | 1874 | 2429 | 3729 | 3617 |
| NM_014335 | EID1 | 0.23 | 0.00 | 1313 | 1680 | 3411 | 3558 |
| NM_ 001008394 | EID3 | 0.19 | 0.00 | 47 | 82 | 108 | 120 |
| NM_ 001242481 | EIF1AD | 0.11 | 0.00 | 618 | 757 | 655 | 636 |
| NM_005875 | EIF1B | 0.14 | 0.00 | 526 | 662 | 576 | 599 |
| NM_032025 | EIF2A | 0.11 | 0.00 | 863 | 1034 | 1564 | 1597 |
| NM_002759 | EIF2AK2 | 0.02 | 0.00 | 751 | 854 | 1510 | 1408 |
| NM_ 001013703 | EIF2AK4 | 0.30 | 0.00 | 2757 | 3603 | 6578 | 6493 |
| NM_014239 | EIF2B2 | 0.06 | 0.00 | 919 | 1063 | 1807 | 1885 |
| NM_020365 | EIF2B3 | 0.17 | 0.00 | 505 | 653 | 1123 | 1128 |
| NM_006893 | EIF2D | 0.33 | -0.17 | 682 | 966 | 1481 | 1201 |
| NM_004094 | EIF2S1 | 0.09 | 0.00 | 1730 | 1983 | 2477 | 2615 |
| NM_001415 | EIF2S3 | 0.49 | 0.00 | 692 | 1090 | 1576 | 1619 |
| NM_003753 | EIF3D | 0.36 | 0.00 | 4345 | 5823 | 10710 | 10506 |
| NM_003754 | EIF3F | 0.22 | -0.28 | 748 | 977 | 1239 | 924 |

(continued)

| RefSeq ID | Gene Symbol | SR GFOLD | SEN GFOLD | SR IL-2- | SR IL-2 + | SEN IL- 2- | SEN IL-2 + |
|---|---|---|---|---|---|---|---|
| NM_003755 | EIF3G | 0.26 | -0.05 | 2266 | 2896 | 4078 | 3747 |
| NM_003756 | EIF3H | 0.49 | 0.00 | 1673 | 2530 | 3637 | 3623 |
| NM_003757 | EIF3I | 0.18 | -0.09 | 3964 | 4708 | 7709 | 6965 |
| NM_003758 | EIF3J | 0.38 | 0.00 | 456 | 684 | 659 | 640 |
| NM_013234 | EIF3K | 0.52 | -0.10 | 661 | 1064 | 2071 | 1793 |
| NM_016091 | EIF3L | 0.38 | -0.03 | 1911 | 2668 | 4835 | 4508 |
| NM_006360 | EIF3M | 0.03 | 0.01 | 1732 | 1908 | 2354 | 2529 |
| NM_001416 | EIF4A1 | 0.43 | -0.29 | 5254 | 7364 | 12022 | 9547 |
| NM_014740 | EIF4A3 | 0.01 | 0.00 | 2140 | 2314 | 1868 | 1926 |
| NM_001417 | EIF4B | 0.46 | -0.01 | 2393 | 3490 | 6037 | 5747 |
| NM_001130679 | EIF4E | 0.32 | 0.00 | 315 | 467 | 730 | 732 |
| NM_001282958 | EIF4E2 | 0.13 | 0.00 | 1109 | 1334 | 1242 | 1364 |
| NM_003732 | EIF4EBP3 | 0.18 | 0.00 | 44 | 77 | 212 | 213 |
| NM_001970 | EIF5A | 0.28 | -0.18 | 3606 | 4616 | 7495 | 6346 |
| NM_001099692 | EIF5AL1 | 0.08 | -0.01 | 4612 | 5115 | 8013 | 7662 |
| NM_002212 | EIF6 | 0.13 | -0.01 | 3082 | 3571 | 5636 | 5338 |
| NM_018127 | ELAC2 | 0.14 | 0.00 | 2123 | 2505 | 3606 | 3732 |
| NM_001114123 | ELK1 | 0.21 | 0.00 | 1292 | 1633 | 1452 | 1484 |
| NM_006532 | ELL | 0.23 | 0.00 | 481 | 649 | 990 | 891 |
| NM_025165 | ELL3 | 1.07 | 0.00 | 15 | 61 | 106 | 137 |
| NM_153702 | ELMOD2 | 0.25 | 0.00 | 250 | 361 | 611 | 664 |
| NM_001278939 | ELN | 0.50 | -0.41 | 2316 | 3480 | 641 | 416 |
| NM_203413 | ELP5 | 0.18 | 0.00 | 631 | 810 | 1038 | 935 |
| NM_006067 | EMC8 | 0.02 | 0.00 | 734 | 837 | 869 | 874 |
| NM_001257370 | EME2 | 0.64 | 0.00 | 8 | 37 | 98 | 131 |
| NM_001193268 | EML2 | 0.05 | 0.00 | 515 | 614 | 1027 | 945 |
| NM_001424 | EMP2 | 0.12 | -0.08 | 1343 | 1591 | 1533 | 1329 |
| NM_001426 | EN1 | 0.49 | -0.09 | 285 | 475 | 930 | 778 |
| NM_015036 | ENDOD1 | 0.05 | 0.00 | 1321 | 1493 | 4041 | 4142 |
| NM_004435 | ENDOG | 0.18 | 0.01 | 582 | 750 | 971 | 1081 |
| NM_000118 | ENG | 0.13 | -0.05 | 13055 | 14733 | 15252 | 14379 |
| NM_001977 | ENPEP | 0.51 | 0.00 | 28 | 67 | 31 | 41 |

(continued)

| RefSeq ID | Gene Symbol | SR GFOLD | SEN GFOLD | SR IL-2- | SR IL-2 + | SEN IL- 2- | SEN IL-2 + |
|---|---|---|---|---|---|---|---|
| NM_006209 | ENPP2 | 0.05 | -0.31 | 491 | 587 | 1547 | 1137 |
| NM_207043 | ENSA | 0.39 | 0.00 | 472 | 709 | 1044 | 1139 |
| NM_001098175 | ENTPD1 | 0.05 | 0.00 | 353 | 433 | 430 | 455 |
| NM_001128930 | ENTPD4 | 0.06 | 0.00 | 2349 | 2624 | 3051 | 3242 |
| NM_001247 | ENTPD6 | 0.17 | 0.00 | 1414 | 1735 | 2733 | 2850 |
| NM_001278689 | EOGT | 0.09 | 0.00 | 493 | 603 | 1175 | 1203 |
| NM_001430 | EPAS1 | 0.35 | 0.00 | 2929 | 3958 | 8874 | 9052 |
| NM_001431 | EPB41L2 | 0.16 | 0.00 | 1206 | 1479 | 2134 | 2205 |
| NM_018424 | EPB41L4B | 0.09 | 0.00 | 38 | 65 | 58 | 85 |
| NM_004431 | EPHA2 | 0.21 | 0.00 | 2827 | 3467 | 1283 | 1384 |
| NM_004444 | EPHB4 | 0.15 | -0.15 | 402 | 521 | 468 | 359 |
| NM_014805 | EPM2AIP1 | 0.36 | 0.00 | 341 | 516 | 840 | 928 |
| NM_001130072 | EPN1 | 0.09 | 0.00 | 1684 | 1935 | 2516 | 2643 |
| NM_001258374 | EPS15L1 | 0.07 | 0.00 | 817 | 961 | 1246 | 1368 |
| NM_004447 | EPS8 | 0.22 | -0.12 | 2317 | 2882 | 5800 | 5102 |
| NM_005702 | ERAL1 | 0.24 | -0.03 | 824 | 1084 | 1524 | 1372 |
| NM_004448 | ERBB2 | 0.18 | -0.01 | 1706 | 2090 | 3953 | 3726 |
| NM_000400 | ERCC2 | 0.21 | -0.18 | 2075 | 2576 | 4122 | 3460 |
| NM_000122 | ERCC3 | 0.09 | 0.00 | 1226 | 1424 | 1961 | 2039 |
| NM_005236 | ERCC4 | 0.27 | 0.00 | 293 | 421 | 497 | 477 |
| NM_001432 | EREG | 0.63 | 0.00 | 108 | 220 | 46 | 68 |
| NM_006494 | ERF | 0.18 | 0.00 | 826 | 1042 | 1578 | 1623 |
| NM_004450 | ERH | 0.07 | 0.00 | 306 | 383 | 634 | 597 |
| NM_006459 | ERLIN1 | 0.02 | 0.00 | 1609 | 1768 | 3257 | 3205 |
| NM_005238 | ETS1 | 0.19 | -0.06 | 4177 | 5002 | 4465 | 4065 |
| NM_005239 | ETS2 | 0.28 | 0.00 | 839 | 1133 | 769 | 758 |
| NM_001987 | ETV6 | 0.14 | 0.00 | 771 | 952 | 984 | 1063 |
| NM_001135032 | EVA1A | 0.32 | -0.09 | 2051 | 2744 | 3371 | 2991 |
| RefSeq ID | Gene Symbol | SR GFOLD | SENGFOLD | SR IL-2- | SR IL-2 + | SEN IL-2- | SEN IL-2 + |
| NM_018166 | EVA1B | 0.04 | -0.32 | 593 | 696 | 1160 | 839 |
| NM_013986 | EWSR1 | 0.40 | -0.34 | 1983 | 2806 | 3094 | 2296 |
| NM_017820 | EXD3 | 0.11 | -0.10 | 60 | 95 | 220 | 161 |

(continued)

| RefSeq ID | Gene Symbol | SR GFOLD | SENGFOLD | SR IL-2- | SR IL-2 + | SEN IL-2- | SEN IL-2 + |
|---|---|---|---|---|---|---|---|
| NM_130398 | EXO1 | 0.29 | -0.43 | 322 | 467 | 171 | 94 |
| NM_016046 | EXOSC1 | 0.42 | 0.00 | 261 | 418 | 903 | 835 |
| NM_001001998 | EXOSC10 | 0.08 | 0.00 | 1825 | 2073 | 2560 | 2429 |
| NM_014285 | EXOSC2 | 0.28 | -0.23 | 524 | 726 | 1002 | 762 |
| NM_058219 | EXOSC6 | 0.47 | -0.16 | 332 | 540 | 626 | 488 |
| NM_015004 | EXOSC7 | 0.22 | -0.07 | 373 | 510 | 780 | 656 |
| NM_001034194 | EXOSC9 | 0.27 | 0.00 | 812 | 1089 | 905 | 826 |
| NM_001440 | EXTL3 | 0.40 | -0.11 | 1829 | 2599 | 5143 | 4530 |
| NM_000137 | FAH | 0.36 | -0.30 | 302 | 460 | 2019 | 1519 |
| NM_016044 | FAHD2A | 0.02 | -0.25 | 249 | 307 | 620 | 451 |
| NM_001098832 | FAM104A | 0.14 | 0.00 | 534 | 671 | 1065 | 1030 |
| NM_001282696 | FAM107B | 0.04 | 0.00 | 249 | 312 | 46 | 55 |
| NM_014719 | FAM115A | 0.07 | 0.00 | 736 | 870 | 2560 | 2527 |
| NM_024556 | FAM118B | 0.09 | 0.00 | 357 | 447 | 682 | 669 |
| NM_001286722 | FAM120A | 0.27 | 0.00 | 6257 | 7828 | 11552 | 11833 |
| NM_198841 | FAM120AOS | 0.11 | 0.00 | 826 | 995 | 1728 | 1745 |
| NM_032448 | FAM120B | 0.13 | 0.00 | 901 | 1093 | 2213 | 2248 |
| NM_017848 | FAM120C | 0.41 | 0.00 | 143 | 242 | 299 | 309 |
| NM_138333 | FAM122A | 0.49 | 0.00 | 213 | 366 | 543 | 508 |
| NM_032581 | FAM126A | 0.13 | 0.00 | 1280 | 1533 | 2435 | 2368 |
| NM_001078172 | FAM127B | 0.16 | 0.00 | 464 | 597 | 884 | 933 |
| NM_001078173 | FAM127C | 0.42 | 0.00 | 591 | 897 | 1002 | 1011 |
| NM_052966 | FAM129A | 0.23 | -0.19 | 1922 | 2428 | 5659 | 4730 |
| NM_001031690 | FAM131B | 1.36 | 0.00 | 16 | 77 | 316 | 281 |
| NM_022749 | FAM160B2 | 0.26 | 0.00 | 1027 | 1353 | 2518 | 2510 |
| NM_001085480 | FAM162B | 0.04 | 0.00 | 49 | 77 | 2 | 9 |
| NM_032648 | FAM167B | 0.31 | 0.00 | 60 | 108 | 83 | 74 |
| NM_198475 | FAM171A2 | 1.17 | 0.00 | 10 | 56 | 195 | 201 |
| NM_023933 | FAM173A | 0.87 | 0.00 | 19 | 63 | 112 | 116 |
| NM_207446 | FAM174B | 0.01 | 0.00 | 39 | 63 | 64 | 79 |
| NM_139076 | FAM175A | 0.32 | 0.00 | 82 | 141 | 262 | 242 |

(continued)

| RefSeq ID | Gene Symbol | SR GFOLD | SENGFOLD | SR IL-2- | SR IL-2 + | SEN IL-2- | SEN IL-2 + |
|---|---|---|---|---|---|---|---|
| NM_032182 | FAM175B | 0.32 | 0.01 | 463 | 666 | 717 | 811 |
| NM_032222 | FAM188B | 0.07 | 0.00 | 24 | 45 | 152 | 128 |
| NM_006589 | FAM189B | 0.05 | 0.00 | 843 | 971 | 2088 | 2150 |
| NM_024946 | FAM192A | 0.13 | 0.00 | 433 | 550 | 940 | 876 |
| NM_207368 | FAM195B | 0.50 | -0.10 | 991 | 1540 | 3354 | 2952 |
| **RefSeq ID** | **Gene Symbol** | **SR GFOLD** | **GFOLD SEN** | **SR IL-2-** | **SR IL-2 +** | **SEN IL-2-** | **SEN IL-2 +** |
| NM_001129891 | FAM196B | 0.16 | 0.00 | 794 | 994 | 2011 | 2018 |
| NM_017832 | FAM206A | 0.47 | 0.00 | 116 | 210 | 399 | 383 |
| NM_001243746 | FAM20A | 0.30 | 0.00 | 44 | 83 | 58 | 67 |
| NM_020223 | FAM20C | 0.04 | 0.00 | 2522 | 2762 | 5790 | 6063 |
| NM_001005751 | FAM21A | 0.28 | 0.00 | 594 | 816 | 1585 | 1455 |
| NM_015262 | FAM21C | 0.20 | 0.00 | 612 | 795 | 1633 | 1571 |
| NM_001291328 | FAM228B | 0.67 | -0.20 | 100 | 211 | 618 | 466 |
| NM_001033564 | FAM229B | 0.22 | 0.00 | 46 | 82 | 164 | 176 |
| NM_152644 | FAM24B | 0.36 | 0.00 | 55 | 104 | 92 | 83 |
| NM_153711 | FAM26E | 0.46 | -0.16 | 270 | 443 | 942 | 752 |
| NM_014077 | FAM32A | 0.47 | 0.00 | 988 | 1500 | 2094 | 2205 |
| NM_019054 | FAM35A | 0.02 | 0.00 | 276 | 339 | 424 | 434 |
| NM_153690 | FAM43A | 0.16 | 0.00 | 189 | 264 | 245 | 214 |
| NM_052943 | FAM46B | 0.73 | -0.36 | 175 | 360 | 127 | 70 |
| NM_001256763 | FAM49B | 0.25 | 0.00 | 267 | 383 | 312 | 296 |
| NM_001013622 | FAM53A | 0.97 | -0.17 | 17 | 62 | 42 | 20 |
| NM_016605 | FAM53C | 0.25 | 0.00 | 925 | 1217 | 2061 | 2007 |
| NM_001130997 | FAM58A | 0.56 | 0.00 | 279 | 490 | 574 | 599 |
| NM_019013 | FAM64A | 0.16 | -1.32 | 173 | 244 | 245 | 72 |
| NM_001193523 | FAM65A | 0.43 | -0.02 | 2191 | 3147 | 4211 | 3944 |
| NM_001100910 | FAM72B | 0.19 | 0.00 | 29 | 56 | 56 | 41 |
| NM_032809 | FAM73B | 0.53 | -0.10 | 321 | 544 | 836 | 693 |
| NM_030919 | FAM83D | 0.12 | -0.70 | 450 | 566 | 545 | 283 |

(continued)

| RefSeq ID | Gene Symbol | SR GFOLD | GFOLD SEN | SR IL-2- | SR IL-2 + | SEN IL-2- | SEN IL-2 + |
|---|---|---|---|---|---|---|---|
| NM_201400 | FAM86A | 0.20 | 0.00 | 407 | 543 | 840 | 769 |
| NM_001083537 | FAM86B 1 | 0.06 | 0.00 | 22 | 42 | 100 | 94 |
| NM_001099653 | FAM86C1 | 0.18 | 0.00 | 104 | 158 | 231 | 190 |
| NM_144963 | FAM91A1 | 0.45 | 0.00 | 403 | 637 | 882 | 937 |
| NM_001289108 | FAM96A | 0.27 | 0.00 | 204 | 303 | 497 | 442 |
| NM_015475 | FAM98A | 0.18 | 0.00 | 1861 | 2266 | 2373 | 2501 |
| NM_000135 | FANCA | 0.17 | 0.00 | 845 | 1059 | 695 | 641 |
| NM_001018115 | FANCD2 | 0.16 | -0.38 | 317 | 421 | 327 | 205 |
| NM_001113378 | FANCI | 0.06 | 0.00 | 1065 | 1224 | 1165 | 1055 |
| NM_005766 | FARP1 | 0.16 | 0.00 | 3748 | 4406 | 5754 | 6026 |
| NM_006567 | FARS2 | 0.09 | 0.00 | 159 | 215 | 568 | 521 |
| NM_004461 | FARSA | 0.25 | 0.00 | 1422 | 1840 | 1722 | 1821 |
| NM_005687 | FARSB | 0.23 | -0.21 | 627 | 832 | 1518 | 1201 |
| NM_000043 | FAS | 0.18 | 0.00 | 502 | 653 | 1460 | 1462 |
| NM_001136193 | FASTKD2 | 0.26 | -0.12 | 649 | 879 | 1266 | 1054 |
| NM_024091 | FASTKD3 | 0.16 | 0.00 | 87 | 134 | 162 | 165 |
| RefSeq ID | Gene Symbol | SR GFOLD | SEN GFOLD | SR IL-2- | SR IL-2 + | SEN IL-2- | SEN IL-2+ |
| NM_021826 | FASTKD5 | 0.31 | 0.00 | 323 | 473 | 532 | 553 |
| NM_001291285 | FAT4 | 0.10 | -0.50 | 1098 | 1297 | 1512 | 974 |
| NM_001997 | FAU | 0.95 | 0.00 | 360 | 805 | 1069 | 1055 |
| NM_001080542 | FBF1 | 0.68 | 0.00 | 69 | 154 | 210 | 198 |
| NM_001436 | FBL | 0.34 | -0.01 | 1064 | 1480 | 1221 | 1098 |
| NM_017556 | FBLIM 1 | 0.40 | -0.02 | 968 | 1412 | 3271 | 3044 |
| NM_001004019 | FBLN2 | 0.16 | 0.00 | 6669 | 7750 | 2768 | 2769 |
| NM_017703 | FBXL12 | 0.38 | -0.01 | 232 | 367 | 686 | 597 |
| NM_001278716 | FBXL4 | 0.20 | 0.00 | 274 | 378 | 597 | 680 |
| NM_001193534 | FBXL5 | 0.16 | 0.00 | 1270 | 1552 | 2855 | 2874 |
| NM_032807 | FBX018 | 0.19 | 0.00 | 1782 | 2185 | 3860 | 3871 |
| NM_147188 | FBX022 | 0.11 | 0.00 | 412 | 519 | 930 | 919 |

(continued)

| RefSeq ID | Gene Symbol | SR GFOLD | SEN GFOLD | SR IL-2- | SR IL-2 + | SEN IL-2- | SEN IL-2+ |
|---|---|---|---|---|---|---|---|
| NM_012175 | FBXO3 | 0.16 | 0.00 | 302 | 404 | 690 | 780 |
| NM_030793 | FBXO38 | 0.28 | 0.00 | 721 | 983 | 1208 | 1316 |
| NM_018998 | FBXW5 | 0.30 | -0.15 | 2712 | 3547 | 5736 | 4928 |
| NM_153348 | FBXW8 | 0.43 | 0.01 | 459 | 710 | 1073 | 1190 |
| NM_001136019 | FCGRT | 0.11 | 0.00 | 764 | 923 | 2213 | 2346 |
| NM_001002901 | FCRLB | 0.08 | 0.00 | 61 | 94 | 171 | 139 |
| NM_001135821 | FDPS | 0.09 | 0.00 | 655 | 790 | 1437 | 1429 |
| NM_004109 | FDX1 | 0.06 | 0.00 | 61 | 93 | 179 | 166 |
| NM_001031734 | FDX1L | 0.14 | -0.13 | 258 | 345 | 838 | 677 |
| NM_001258013 | FDXR | 0.10 | -0.47 | 119 | 168 | 430 | 259 |
| NM_018708 | FEM1A | 0.20 | 0.00 | 1111 | 1401 | 1628 | 1634 |
| NM_004111 | FEN1 | 0.19 | -0.46 | 1865 | 2292 | 1339 | 880 |
| NM_001042548 | FEZ2 | 0.10 | 0.00 | 1599 | 1858 | 3404 | 3237 |
| NM_004463 | FGD1 | 0.18 | 0.00 | 700 | 890 | 1221 | 1318 |
| NM_004464 | FGF5 | 0.34 | -0.12 | 4322 | 5738 | 3329 | 2895 |
| NM_001113411 | FGGY | 0.34 | 0.00 | 38 | 76 | 258 | 262 |
| NM_000143 | FH | 0.29 | 0.00 | 1346 | 1793 | 1185 | 1091 |
| NM_004214 | FIBP | 0.49 | 0.00 | 801 | 1251 | 2421 | 2320 |
| NM_001080472 | FITM2 | 0.24 | 0.00 | 130 | 199 | 287 | 266 |
| NM_015258 | FKBP15 | 0.25 | -0.05 | 1391 | 1797 | 3111 | 2835 |
| NM_002014 | FKBP4 | 0.16 | 0.00 | 740 | 928 | 1395 | 1355 |
| NM_025207 | FLAD 1 | 0.10 | -0.08 | 426 | 532 | 1034 | 878 |
| NM_005803 | FLOT1 | 0.39 | -0.15 | 1488 | 2112 | 4993 | 4283 |
| NM_015033 | FNBP1 | 0.02 | 0.00 | 1181 | 1315 | 1526 | 1649 |
| NM_001024948 | FNBP1L | 0.28 | 0.00 | 164 | 252 | 374 | 436 |
| NM_002028 | FNTB | 0.16 | 0.00 | 833 | 1038 | 1456 | 1573 |
| NM_000804 | FOLR3 | 0.31 | 0.00 | 115 | 188 | 707 | 765 |
| RefSeq ID | Gene Symbol | SR GFOLD | SENGFOLD | SR IL-2- | SR IL-2 + | SEN IL-2- | SEN IL-2+ |
| NM_006732 | FOSB | 0.04 | 0.00 | 66 | 98 | 54 | 70 |
| NM_005438 | FOSL1 | 0.33 | -0.16 | 4479 | 5899 | 3947 | 3338 |

(continued)

| RefSeq ID | Gene Symbol | SR GFOLD | SENGFOLD | SR IL-2- | SR IL-2 + | SEN IL-2- | SEN IL-2+ |
|---|---|---|---|---|---|---|---|
| NM_005253 | FOSL2 | 0.13 | 0.00 | 3486 | 4019 | 5515 | 5296 |
| NM_004472 | FOXD1 | 0.14 | 0.00 | 1633 | 1950 | 2279 | 2442 |
| NM_005250 | FOXL1 | 0.06 | 0.00 | 662 | 779 | 1192 | 1311 |
| NM_202002 | FOXM1 | 0.26 | -0.86 | 2104 | 2690 | 2175 | 1098 |
| NM_001018078 | FPGS | 0.23 | -0.23 | 1094 | 1406 | 2521 | 2006 |
| NM_001193306 | FPR1 | 0.52 | 0.00 | 34 | 78 | 158 | 155 |
| NM_012083 | FRAT2 | 0.76 | 0.00 | 32 | 87 | 79 | 102 |
| NM_174938 | FRMD3 | 0.15 | -0.29 | 654 | 819 | 851 | 614 |
| NM_018027 | FRMD4A | 0.05 | 0.00 | 3154 | 3449 | 3822 | 4015 |
| NM_001267046 | FRMD6 | 0.24 | -0.05 | 10248 | 12522 | 23265 | 21989 |
| NM_014728 | FRMPD4 | 0.05 | 0.00 | 216 | 277 | 308 | 259 |
| NM_001013660 | FRRS1 | 0.29 | 0.00 | 51 | 93 | 237 | 222 |
| NM_006653 | FRS3 | 0.16 | 0.00 | 64 | 103 | 79 | 92 |
| NM_023037 | FRY | 0.10 | -0.22 | 263 | 342 | 674 | 503 |
| NM_003088 | FSCN1 | 0.12 | -0.22 | 5767 | 6530 | 6033 | 4967 |
| NM_013393 | FTSJ2 | 0.05 | 0.00 | 495 | 592 | 711 | 769 |
| NM_003902 | FUBP1 | 0.06 | -0.16 | 2579 | 2854 | 3157 | 2651 |
| NM_003934 | FUBP3 | 0.11 | 0.00 | 1631 | 1901 | 2955 | 2823 |
| NM_001170634 | FUS | 0.08 | -0.03 | 4400 | 4886 | 5644 | 5299 |
| NM_032664 | FUT10 | 0.06 | 0.00 | 219 | 282 | 433 | 499 |
| NM_001013439 | FXR1 | 0.06 | 0.00 | 1488 | 1685 | 2762 | 2885 |
| NM_024513 | FYCO1 | 0.24 | 0.00 | 694 | 921 | 2880 | 2808 |
| NM_003468 | FZD5 | 0.42 | 0.00 | 47 | 95 | 89 | 91 |
| NM_003508 | FZD9 | 0.91 | 0.00 | 13 | 51 | 10 | 15 |
| NM_005754 | G3BP1 | 0.55 | -0.06 | 1586 | 2498 | 2968 | 2675 |
| NM_007278 | GABARAP | 0.23 | -0.03 | 1187 | 1521 | 3402 | 3155 |
| NM_005254 | GABPB1 | 0.37 | 0.00 | 310 | 474 | 607 | 671 |
| NM_015675 | GADD45B | 0.26 | 0.00 | 2057 | 2633 | 1404 | 1416 |
| NM_052850 | GADD45GIP1 | 0.10 | 0.00 | 588 | 715 | 1447 | 1340 |
| NM_005255 | GAK | 0.17 | 0.00 | 1908 | 2302 | 3772 | 3750 |
| NM_024637 | GAL3ST4 | 0.17 | 0.00 | 136 | 198 | 493 | 434 |
| NM_000403 | GALE | 0.11 | -0.23 | 637 | 779 | 938 | 713 |

(continued)

| RefSeq ID | Gene Symbol | SR GFOLD | SENGFOLD | SR IL-2- | SR IL-2 + | SEN IL-2- | SEN IL-2+ |
|---|---|---|---|---|---|---|---|
| NM_001001556 | GALK2 | 0.02 | 0.00 | 350 | 419 | 522 | 475 |
| NM_054110 | GALNT15 | 0.36 | -0.65 | 79 | 140 | 749 | 414 |
| NM_004482 | GALNT3 | 0.50 | 0.00 | 91 | 174 | 54 | 79 |
| NM_000155 | GALT | 0.04 | -0.12 | 248 | 311 | 830 | 677 |
| NM_000156 | GAMT | 0.07 | -0.22 | 105 | 148 | 630 | 468 |
| RefSeq ID | Gene Symbol | SR GFOLD | SEN GFOLD | SR IL-2- | SR IL-2 + | SEN IL-2- | SEN IL-2+ |
| NM_198141 | GANC | 0.61 | 0.00 | 246 | 450 | 765 | 749 |
| NM_014364 | GAPDHS | 0.24 | -0.27 | 37 | 70 | 131 | 78 |
| NM_018983 | GAR1 | 0.08 | 0.00 | 130 | 179 | 148 | 126 |
| NM_001136005 | GART | 0.25 | 0.00 | 1086 | 1421 | 1803 | 1856 |
| NM_006478 | GAS2L1 | 0.09 | -0.01 | 612 | 738 | 1154 | 1035 |
| NM_174942 | GAS2L3 | 0.38 | -0.70 | 136 | 228 | 191 | 87 |
| NM_017660 | GATAD2A | 0.06 | 0.00 | 3058 | 3368 | 4147 | 4347 |
| NM_004564 | GATB | 0.26 | -0.29 | 303 | 433 | 853 | 619 |
| NM_001282629 | GBGT1 | 0.66 | -0.03 | 60 | 136 | 141 | 102 |
| NM_003203 | GCFC2 | 0.04 | 0.00 | 152 | 201 | 287 | 294 |
| NM_006836 | GCN1L1 | 0.11 | 0.00 | 7506 | 8432 | 12330 | 12454 |
| NM_004962 | GDF10 | 0.62 | 0.00 | 25 | 66 | 35 | 44 |
| NM_001288824 | GDF9 | 0.34 | 0.00 | 122 | 202 | 327 | 338 |
| NM_001494 | GDI2 | 0.22 | -0.02 | 4008 | 4895 | 6391 | 6041 |
| NM_001252156 | GEMIN5 | 0.11 | -0.09 | 910 | 1090 | 1439 | 1233 |
| NM_024775 | GEMIN6 | 0.04 | 0.00 | 123 | 167 | 241 | 209 |
| NM_017856 | GEMIN8 | 0.33 | 0.00 | 147 | 236 | 464 | 536 |
| NM_001130009 | GEN1 | 0.04 | 0.00 | 193 | 248 | 229 | 226 |
| NM_024996 | GFM1 | 0.30 | -0.01 | 961 | 1308 | 1832 | 1682 |
| NM_005110 | GFPT2 | 0.28 | 0.00 | 1557 | 2038 | 4055 | 4140 |
| NM_001099781 | GGT5 | 0.15 | -0.90 | 21 | 43 | 81 | 26 |
| NM_001080383 | GJC1 | 0.11 | -0.08 | 1437 | 1688 | 1720 | 1497 |
| NM_000169 | GLA | 0.35 | 0.00 | 416 | 615 | 1183 | 1250 |
| NM_001003722 | GLE1 | 0.21 | 0.00 | 705 | 917 | 1279 | 1198 |

(continued)

| RefSeq ID | Gene Symbol | SR GFOLD | SEN GFOLD | SR IL-2- | SR IL-2 + | SEN IL-2- | SEN IL-2+ |
|---|---|---|---|---|---|---|---|
| NM_005270 | GLI2 | 0.10 | 0.00 | 1090 | 1289 | 1707 | 1706 |
| NM_006708 | GLO1 | 0.37 | -0.09 | 892 | 1280 | 2770 | 2440 |
| NM_016080 | GLOD4 | 0.12 | 0.00 | 711 | 868 | 1389 | 1329 |
| NM_197962 | GLRX2 | 0.85 | 0.00 | 45 | 121 | 127 | 120 |
| NM_016417 | GLRX5 | 0.03 | 0.00 | 541 | 631 | 665 | 654 |
| NM_001256310 | GLS | 0.07 | 0.00 | 6267 | 6870 | 7611 | 7628 |
| NM_015710 | GLTSCR2 | 0.79 | -0.03 | 368 | 736 | 990 | 871 |
| NM_002065 | GLUL | 0.31 | -0.41 | 2045 | 2708 | 3789 | 2686 |
| NM_032569 | GLYR1 | 0.03 | 0.00 | 1637 | 1811 | 3136 | 2991 |
| NM_000405 | GM2A | 0.17 | 0.00 | 900 | 1125 | 3396 | 3416 |
| NM_013334 | GMPPB | 0.01 | 0.00 | 678 | 774 | 1106 | 1079 |
| NM_003875 | GMPS | 0.39 | 0.00 | 779 | 1141 | 1142 | 1192 |
| NM_002069 | GNAI1 | 0.22 | 0.00 | 820 | 1062 | 1460 | 1519 |
| NM_053004 | GNB1L | 0.27 | 0.00 | 130 | 204 | 198 | 176 |
| NM_006098 | GNB2L1 | 0.14 | -0.01 | 16273 | 18409 | 33137 | 32212 |
| NM_021629 | GNB4 | 0.20 | 0.00 | 2087 | 2568 | 2676 | 2530 |
| NM_004126 | GNG11 | 0.02 | -0.03 | 1274 | 1410 | 4480 | 4166 |
| NM_005274 | GNG5 | 0.53 | 0.00 | 246 | 429 | 767 | 808 |
| NM_013285 | GNL2 | 0.09 | 0.00 | 2079 | 2377 | 2252 | 2111 |
| NM_001184819 | GNL3L | 0.20 | 0.00 | 1299 | 1631 | 2048 | 1926 |
| NM_014236 | GNPAT | 0.38 | 0.00 | 575 | 851 | 2080 | 2159 |
| NM_005471 | GNPDA1 | 0.33 | -0.23 | 963 | 1332 | 2510 | 2000 |
| NM_002077 | GOLGA1 | 0.21 | 0.00 | 534 | 708 | 1325 | 1401 |
| NM_001174124 | GOLGA7 | 0.45 | 0.00 | 538 | 837 | 1302 | 1296 |
| NM_031899 | GORASP1 | 0.34 | 0.00 | 1191 | 1648 | 2331 | 2310 |
| NM_018559 | GPALPP1 | 0.03 | 0.00 | 128 | 171 | 360 | 336 |
| NM_018040 | GPATCH2 | 0.63 | 0.01 | 115 | 232 | 418 | 490 |
| NM_005708 | GPC6 | 0.13 | -0.30 | 505 | 636 | 1206 | 885 |
| NM_019593 | GPCPD1 | 0.37 | 0.00 | 228 | 358 | 643 | 688 |
| NM_001083112 | GPD2 | 0.20 | 0.00 | 774 | 995 | 1004 | 1020 |
| NM_015698 | GPKOW | 0.45 | 0.00 | 360 | 574 | 836 | 819 |
| NM_001080452 | GPR108 | 0.38 | 0.00 | 697 | 1014 | 2435 | 2512 |
| NM_153835 | GPR113 | 0.38 | 0.00 | 29 | 63 | 58 | 72 |

(continued)

| RefSeq ID | Gene Symbol | SR GFOLD | SEN GFOLD | SR IL-2- | SR IL-2 + | SEN IL-2- | SEN IL-2+ |
|---|---|---|---|---|---|---|---|
| NM_032777 | GPR124 | 0.05 | -0.16 | 1568 | 1763 | 2529 | 2115 |
| NM_001170880 | GPR137 | 0.23 | 0.00 | 345 | 477 | 821 | 869 |
| NM_207370 | GPR153 | 0.36 | 0.00 | 59 | 110 | 299 | 340 |
| NM_018969 | GPR173 | 0.13 | 0.00 | 45 | 76 | 204 | 176 |
| NM_005281 | GPR3 | 0.21 | 0.00 | 67 | 111 | 81 | 57 |
| NM_001508 | GPR39 | 0.32 | 0.00 | 332 | 490 | 85 | 113 |
| NM_001097613 | GPR89A | 0.18 | 0.00 | 210 | 293 | 383 | 401 |
| NM_016235 | GPRC5B | 0.08 | 0.00 | 70 | 106 | 77 | 105 |
| NM_212492 | GPS1 | 0.53 | 0.00 | 1473 | 2300 | 4053 | 4270 |
| NM_004489 | GPS2 | 0.45 | 0.00 | 213 | 356 | 476 | 499 |
| NM_001145638 | GPSM1 | 0.20 | 0.00 | 645 | 835 | 1154 | 1061 |
| NM_001276501 | GPSM3 | 0.23 | -0.02 | 80 | 130 | 243 | 192 |
| NM_201397 | GPX1 | 0.03 | -0.18 | 1172 | 1315 | 3188 | 2656 |
| NM_001039847 | GPX4 | 0.22 | 0.00 | 2486 | 3091 | 7584 | 7837 |
| NM_015696 | GPX7 | 0.47 | -0.07 | 677 | 1050 | 1373 | 1192 |
| NM_013372 | GREM1 | 0.14 | -0.22 | 183659 | 203725 | 239474 | 204292 |
| NM_012203 | GRHPR | 0.25 | -0.13 | 1219 | 1581 | 3575 | 3078 |
| NM_005308 | GRK5 | 0.25 | -0.07 | 394 | 546 | 499 | 407 |
| NM_001130006 | GSPT1 | 0.20 | 0.00 | 3743 | 4532 | 4760 | 4930 |
| NM_000637 | GSR | 0.36 | 0.00 | 966 | 1365 | 1890 | 2018 |
| NM_001143679 | GSTK1 | 0.81 | -0.19 | 767 | 1488 | 3488 | 2889 |
| NM_000848 | GSTM2 | 0.05 | 0.00 | 171 | 224 | 805 | 776 |
| NM_000850 | GSTM4 | 0.02 | 0.00 | 88 | 123 | 428 | 403 |
| NM_004832 | GSTO1 | 0.06 | -0.03 | 1983 | 2217 | 5045 | 4699 |
| NM_000853 | GSTT1 | 0.35 | 0.00 | 220 | 342 | 670 | 640 |
| NM_145870 | GSTZ1 | 0.24 | 0.00 | 90 | 145 | 335 | 327 |
| NM_002096 | GTF2F1 | 0.19 | 0.00 | 1527 | 1884 | 2373 | 2512 |
| NM_004128 | GTF2F2 | 0.06 | 0.00 | 249 | 315 | 410 | 416 |
| NM_001521 | GTF3C2 | 0.25 | 0.00 | 1050 | 1375 | 1888 | 1815 |
| NM_012086 | GTF3C3 | 0.08 | 0.00 | 678 | 810 | 1098 | 1041 |
| NM_001122823 | GTF3C5 | 0.31 | -0.09 | 1065 | 1449 | 2051 | 1786 |

(continued)

| RefSeq ID | Gene Symbol | SR GFOLD | SEN GFOLD | SR IL-2- | SR IL-2 + | SEN IL-2- | SEN IL-2+ |
|---|---|---|---|---|---|---|---|
| NM_001284251 | GUCD1 | 0.40 | 0.00 | 2120 | 2998 | 4222 | 4309 |
| NM_177925 | H2AFJ | 0.18 | -0.45 | 1315 | 1622 | 2263 | 1532 |
| NM_138635 | H2AFV | 0.34 | -0.05 | 873 | 1228 | 1414 | 1244 |
| NM_002105 | H2AFX | 0.42 | -0.26 | 946 | 1397 | 980 | 728 |
| NM_018649 | H2AFY2 | 0.59 | -0.01 | 112 | 220 | 410 | 344 |
| NM_002106 | H2AFZ | 0.28 | -0.53 | 1961 | 2558 | 2263 | 1445 |
| NM_014282 | HABP4 | 0.27 | 0.00 | 986 | 1309 | 1807 | 1699 |
| NM_001184705 | HADH | 0.45 | 0.00 | 442 | 695 | 1044 | 1078 |
| NM_002109 | HARS | 0.16 | 0.00 | 1532 | 1858 | 3159 | 3126 |
| NM_012208 | HARS2 | 0.11 | -0.01 | 309 | 398 | 824 | 726 |
| NM_005328 | HAS2 | 0.30 | -0.13 | 4581 | 5898 | 11051 | 9784 |
| NM_003642 | HAT1 | 0.19 | 0.00 | 564 | 734 | 794 | 871 |
| NM_138443 | HAUS1 | 0.43 | -0.03 | 133 | 231 | 227 | 176 |
| NM_018097 | HAUS2 | 0.14 | 0.00 | 340 | 444 | 786 | 773 |
| NM_001166269 | HAUS4 | 0.19 | -0.03 | 177 | 254 | 468 | 390 |
| NM_017645 | HAUS6 | 0.11 | 0.00 | 591 | 727 | 761 | 745 |
| NM_017518 | HAUS7 | 0.62 | 0.00 | 373 | 668 | 690 | 636 |
| NM_001945 | HBEGF | 0.27 | 0.00 | 203 | 303 | 541 | 591 |
| NM_006620 | HBS1L | 0.15 | 0.00 | 1205 | 1460 | 2493 | 2521 |
| NM_005334 | HCFC1 | 0.11 | 0.00 | 3594 | 4085 | 4839 | 5087 |
| NM_004964 | HDAC1 | 0.75 | 0.00 | 568 | 1074 | 2032 | 1891 |
| NM_032019 | HDAC10 | 0.12 | 0.00 | 159 | 220 | 474 | 538 |
| NM_178423 | HDAC9 | 0.51 | -0.08 | 165 | 295 | 620 | 510 |
| NM_016063 | HDDC2 | 0.17 | 0.00 | 274 | 370 | 643 | 665 |
| NM_004494 | HDGF | 0.52 | 0.00 | 2886 | 4378 | 4700 | 4473 |
| NM_001001520 | HDGFRP2 | 0.24 | 0.00 | 869 | 1139 | 1670 | 1577 |
| NM_001135565 | HDHD1 | 0.05 | 0.00 | 491 | 587 | 559 | 488 |
| NM_031219 | HDHD3 | 0.31 | 0.00 | 84 | 143 | 183 | 172 |
| NM_018072 | HEATR1 | 0.15 | 0.00 | 1584 | 1906 | 2862 | 2830 |
| NM_017802 | HEATR2 | 0.19 | 0.00 | 642 | 830 | 1183 | 1094 |
| NM_024602 | HECTD3 | 0.02 | -0.04 | 1182 | 1315 | 2425 | 2198 |
| NM_020760 | HECW2 | 0.05 | -0.09 | 672 | 784 | 1331 | 1137 |
| NM_001289067 | HELLS | 0.07 | -0.07 | 335 | 416 | 195 | 144 |

(continued)

| RefSeq ID | Gene Symbol | SR GFOLD | SEN GFOLD | SR IL-2- | SR IL-2 + | SEN IL-2- | SEN IL-2+ |
|---|---|---|---|---|---|---|---|
| NM_133636 | HELQ | 0.17 | 0.00 | 124 | 183 | 308 | 274 |
| NM_003922 | HERC1 | 0.06 | 0.00 | 2713 | 3001 | 5763 | 5924 |
| NM_022079 | HERC4 | 0.13 | -0.04 | 3367 | 3880 | 6079 | 5663 |
| NM_001165967 | HES7 | 2.24 | -0.39 | 35 | 249 | 225 | 133 |
| NM_016458 | HGH1 | 0.22 | 0.00 | 471 | 631 | 705 | 645 |
| NM_004712 | HGS | 0.25 | 0.00 | 3183 | 4013 | 5852 | 6109 |
| NM_022475 | HHIP | 0.10 | 0.00 | 3852 | 4357 | 1127 | 1179 |
| NM_032558 | HIATL1 | 0.35 | 0.00 | 1374 | 1903 | 3275 | 3353 |
| NM_014362 | HIBCH | 0.26 | 0.00 | 88 | 144 | 339 | 342 |
| NM_001098202 | HIC1 | 0.35 | 0.00 | 425 | 628 | 788 | 708 |
| NM_015094 | HIC2 | 0.03 | 0.00 | 169 | 219 | 164 | 185 |
| NM_001530 | HIF1A | 0.15 | 0.00 | 15128 | 17280 | 27056 | 26902 |
| NM_138820 | HIGD2A | 0.06 | -0.02 | 556 | 662 | 1333 | 1198 |
| NM_005322 | HIST1H1B | 0.07 | -0.68 | 7478 | 8154 | 3354 | 1956 |
| NM_003513 | HIST1H2AB | 0.08 | -0.13 | 513 | 622 | 297 | 220 |
| NM_003512 | HIST1H2AC | 0.36 | -0.06 | 2031 | 2792 | 3369 | 3055 |
| NM_021064 | HIST1H2AG | 0.52 | -0.25 | 1398 | 2168 | 665 | 486 |
| NM_080596 | HIST1H2AH | 0.12 | -0.06 | 1096 | 1309 | 476 | 390 |
| NM_003510 | HIST1H2AK | 0.67 | 0.00 | 487 | 882 | 412 | 359 |
| NM_021062 | HIST1H2BB | 0.25 | -0.52 | 874 | 1156 | 545 | 323 |
| NM_003526 | HIST1H2BC | 0.10 | -0.09 | 4518 | 5091 | 4074 | 3634 |
| NM_138720 | HIST1H2BD | 0.03 | 0.00 | 3040 | 3280 | 1689 | 1634 |
| NM_003522 | HIST1H2BF | 0.25 | 0.00 | 868 | 1144 | 412 | 372 |
| NM_003518 | HIST1H2BG | 0.40 | -0.09 | 1290 | 1848 | 703 | 580 |
| NM_003524 | HIST1H2BH | 0.12 | 0.00 | 1008 | 1213 | 64 | 74 |
| NM_003525 | HIST1H2BI | 0.36 | -0.47 | 1494 | 2076 | 784 | 494 |
| NM_021058 | HIST1H2BJ | 0.05 | 0.00 | 4017 | 4381 | 2130 | 1987 |
| NM_003519 | HIST1H2BL | 0.91 | -0.01 | 334 | 730 | 320 | 262 |
| NM_003521 | HIST1H2BM | 0.16 | -0.22 | 1063 | 1306 | 410 | 294 |
| NM_003520 | HIST1H2BN | 0.22 | -0.37 | 1498 | 1886 | 1468 | 1031 |
| NM_003537 | HIST1H3B | 0.11 | -0.54 | 6097 | 6875 | 2897 | 1859 |
| NM_003531 | HIST1H3C | 0.08 | -0.52 | 4759 | 5271 | 2009 | 1286 |
| NM_003534 | HIST1H3G | 0.33 | 0.00 | 1843 | 2490 | 651 | 588 |
| NM_003536 | HIST1H3H | 0.16 | -0.51 | 1688 | 2035 | 915 | 566 |
| NM_003538 | HIST1H4A | 0.74 | 0.00 | 395 | 761 | 220 | 203 |

(continued)

| RefSeq ID | Gene Symbol | SR GFOLD | SEN GFOLD | SR IL-2- | SR IL-2 + | SEN IL-2- | SEN IL-2+ |
|---|---|---|---|---|---|---|---|
| NM_003542 | HIST1H4C | 0.29 | -0.15 | 1357 | 1801 | 1050 | 848 |
| NM_175065 | HIST2H2AB | 0.22 | -0.27 | 458 | 617 | 406 | 281 |
| NM_001024599 | HIST2H2BF | 0.18 | 0.00 | 1377 | 1698 | 836 | 863 |
| NM_001005464 | HIST2H3A | 0.80 | -0.61 | 3293 | 6017 | 2466 | 1501 |
| NM_021059 | HIST2H3C | 0.80 | -0.61 | 3293 | 6017 | 2466 | 1501 |
| NM_001123375 | HIST2H3D | 0.28 | -0.54 | 659 | 902 | 266 | 144 |
| NM_001127714 | HIVEP3 | 0.06 | 0.00 | 475 | 572 | 1127 | 1152 |
| NM_018410 | HJURP | 0.22 | -0.76 | 757 | 987 | 562 | 281 |
| NM_002121 | HLA-DPB1 | 0.19 | 0.00 | 124 | 185 | 108 | 87 |
| NM_001098479 | HLA-F | 1.22 | 0.00 | 49 | 166 | 233 | 248 |
| NM_003071 | HLTF | 0.28 | 0.00 | 749 | 1015 | 1630 | 1571 |
| NM_000190 | HMBS | 0.21 | -0.17 | 545 | 719 | 1092 | 876 |
| NM_145901 | HMGA1 | 0.31 | -0.31 | 11513 | 14723 | 23733 | 18724 |
| NM_000191 | HMGCL | 0.27 | 0.00 | 439 | 612 | 1346 | 1375 |
| NM_031157 | HNRNPA1 | 0.18 | -0.20 | 1272 | 1569 | 2144 | 1726 |
| NM_031243 | HNRNPA2B 1 | 0.17 | -0.08 | 6298 | 7396 | 7674 | 6968 |
| NM_194247 | HNRNPA3 | 0.05 | 0.00 | 760 | 882 | 1017 | 1103 |
| NM_001013631 | HNRNPCL1 | 1.53 | 0.00 | 28 | 130 | 150 | 126 |
| NM_031370 | HNRNPD | 0.03 | -0.08 | 933 | 1056 | 695 | 575 |
| NM_001098206 | HNRNPF | 0.38 | -0.06 | 4775 | 6489 | 5786 | 5309 |
| NM_001257293 | HNRNPH1 | 0.04 | 0.00 | 2240 | 2465 | 2936 | 2915 |
| NM_001032393 | HNRNPH2 | 0.01 | 0.00 | 1291 | 1424 | 2479 | 2414 |
| NM_002140 | HNRNPK | 0.06 | -0.03 | 2537 | 2822 | 4536 | 4220 |
| NM_001533 | HNRNPL | 0.26 | -0.10 | 1383 | 1794 | 2633 | 2303 |
| NM_005968 | HNRNPM | 0.03 | -0.23 | 1954 | 2151 | 3130 | 2505 |
| NM_007040 | HNRNPUL1 | 0.19 | 0.00 | 4705 | 5639 | 6753 | 6815 |
| NM_018951 | HOXA10 | 0.32 | 0.00 | 255 | 385 | 576 | 575 |
| NM_002145 | HOXB2 | 0.52 | 0.00 | 263 | 453 | 957 | 874 |
| NM_002146 | HOXB3 | 0.28 | 0.00 | 190 | 286 | 641 | 697 |
| NM_014212 | HOXC11 | 0.43 | -0.29 | 37 | 79 | 347 | 233 |
| NM_014620 | HOXC4 | 0.31 | 0.00 | 191 | 294 | 385 | 407 |

(continued)

| RefSeq ID | Gene Symbol | SR GFOLD | SEN GFOLD | SR IL-2- | SR IL-2 + | SEN IL-2- | SEN IL-2+ |
|---|---|---|---|---|---|---|---|
| NM_153693 | HOXC6 | 0.15 | 0.00 | 595 | 749 | 1029 | 1139 |
| NM_022658 | HOXC8 | 0.27 | -0.07 | 545 | 751 | 1227 | 1057 |
| NM_000195 | HPS1 | 0.06 | -0.10 | 1026 | 1182 | 3644 | 3209 |
| NM_001009931 | HRNR | 0.11 | 0.00 | 113 | 162 | 289 | 274 |
| NM_005836 | HRSP12 | 0.06 | 0.00 | 69 | 103 | 256 | 214 |
| NM_022460 | HS1BP3 | 0.28 | -0.22 | 452 | 632 | 1986 | 1580 |
| NM_004807 | HS6ST1 | 0.06 | 0.00 | 939 | 1087 | 611 | 671 |
| NM_001537 | HSBP1 | 0.28 | 0.00 | 829 | 1123 | 2371 | 2466 |

| RefSeq ID | Gene Symbol | SRGFOLD | SEN GFOLD | SR IL-2- | SR IL-2 + | SEN IL-2- | SEN IL-2+ |
|---|---|---|---|---|---|---|---|
| NM_001206741 | HSD11B1 | 0.65 | 0.00 | 1 | 12 | 189 | 179 |
| NM_001037811 | HSD17B10 | 0.06 | -0.40 | 517 | 617 | 1054 | 712 |
| NM_016245 | HSD17B11 | 0.06 | 0.00 | 133 | 181 | 428 | 388 |
| NM_001199291 | HSD17B4 | 0.45 | 0.00 | 406 | 642 | 1316 | 1268 |
| NM_025193 | HSD3B7 | 0.24 | 0.00 | 1092 | 1420 | 2302 | 2429 |
| NM_005348 | HSP90AA1 | 0.29 | 0.00 | 11822 | 14867 | 16598 | 16161 |
| NM_001271969 | HSP90AB1 | 0.47 | -0.08 | 10026 | 14303 | 20597 | 19000 |
| NM_002154 | HSPA4 | 0.32 | 0.01 | 2589 | 3430 | 5064 | 5323 |
| NM_001541 | HSPB2 | 0.36 | -0.28 | 427 | 635 | 753 | 543 |
| NM_001130106 | HSPBP1 | 0.06 | -0.32 | 391 | 479 | 838 | 593 |
| NM_002156 | HSPD1 | 0.15 | -0.13 | 2386 | 2826 | 3288 | 2824 |
| NM_002157 | HSPE1 | 0.35 | 0.00 | 206 | 322 | 430 | 431 |
| NM_001202485 | HSPE1-MOB4 | 0.07 | 0.00 | 561 | 673 | 942 | 1031 |
| NM_000870 | HTR4 | 1.16 | 0.00 | 12 | 58 | 50 | 35 |
| NM_053044 | HTRA3 | 0.14 | 0.00 | 49 | 82 | 46 | 41 |
| NM_001040107 | HVCN1 | 0.03 | 0.00 | 40 | 65 | 279 | 327 |
| NM_033158 | HYAL2 | 0.15 | -0.46 | 910 | 1119 | 2830 | 1919 |
| NM_001243526 | HYI | 0.13 | -0.25 | 1583 | 1880 | 1148 | 871 |
| NM_001134793 | HYLS1 | 0.61 | -0.15 | 167 | 318 | 310 | 229 |
| NM_001039613 | IAH1 | 0.08 | 0.00 | 853 | 1004 | 1599 | 1617 |

(continued)

| RefSeq ID | Gene Symbol | SRGFOLD | SEN GFOLD | SR IL-2- | SR IL-2 + | SEN IL-2- | SEN IL-2+ |
|---|---|---|---|---|---|---|---|
| NM_013417 | IARS | 0.19 | 0.00 | 4327 | 5196 | 12781 | 13149 |
| NM_002167 | ID3 | 0.84 | 0.00 | 4478 | 8364 | 7682 | 7859 |
| NM_004969 | IDE | 0.16 | 0.00 | 627 | 791 | 1179 | 1107 |
| NM_002168 | IDH2 | 0.09 | 0.00 | 1063 | 1243 | 1044 | 989 |
| NM_033261 | IDI2 | 0.32 | 0.00 | 31 | 64 | 96 | 120 |
| NM_016097 | IER3IP1 | 0.25 | 0.00 | 693 | 925 | 1187 | 1207 |
| NM_016545 | IER5 | 0.20 | -0.03 | 1035 | 1306 | 1343 | 1196 |
| NM_005531 | IFI16 | 0.01 | 0.00 | 1667 | 1821 | 2358 | 2364 |
| NM_032036 | IFI27L2 | 0.10 | -0.01 | 420 | 525 | 1169 | 1048 |
| NM_005533 | IFI35 | 0.17 | -0.42 | 231 | 317 | 774 | 506 |
| NM_001547 | IFIT2 | 0.26 | 0.00 | 46 | 84 | 112 | 113 |
| NM_003641 | IFITM1 | 0.18 | -0.47 | 294 | 399 | 277 | 159 |
| NM_006435 | IFITM2 | 0.58 | -0.53 | 1733 | 2790 | 2548 | 1640 |
| NM_021034 | IFITM3 | 0.36 | -0.52 | 4256 | 5736 | 5401 | 3591 |
| NM_006764 | IFRD2 | 0.12 | 0.00 | 1188 | 1413 | 1370 | 1283 |
| NM_016004 | IFT52 | 0.41 | 0.00 | 170 | 282 | 557 | 519 |
| NM_001099223 | IFT74 | 0.60 | 0.00 | 37 | 88 | 189 | 216 |
| NM_001190241 | IFT80 | 0.22 | 0.00 | 120 | 184 | 582 | 664 |
| NM_001551 | IGBP1 | 0.15 | 0.00 | 497 | 635 | 1308 | 1268 |
| RefSeq ID | Gene Symbol | SR GFOLD | SEN GFOLD | SR IL-2- | SR IL-2 + | SEN IL-2- | SEN IL-2+ |
| NM_006546 | IGF2BP1 | 0.57 | 0.00 | 221 | 399 | 75 | 76 |
| NM_003640 | IKBKAP | 0.02 | -0.11 | 957 | 1075 | 2258 | 1941 |
| NM_014002 | IKBKE | 0.22 | -0.09 | 287 | 401 | 584 | 475 |
| NM_000628 | IL10RB | 0.18 | 0.00 | 323 | 435 | 792 | 874 |
| NM_000882 | IL12A | 0.29 | 0.00 | 14 | 36 | 94 | 91 |
| NM_001167928 | IL1RAP | 0.25 | 0.00 | 210 | 307 | 235 | 214 |
| NM_033439 | IL33 | 0.03 | 0.00 | 55 | 84 | 220 | 198 |
| NM_002185 | IL7R | 0.04 | -0.02 | 831 | 954 | 634 | 545 |
| NM_001267809 | ILF2 | 0.43 | -0.13 | 1344 | 1961 | 1878 | 1580 |
| NM_001014795 | ILK | 0.11 | -0.03 | 5437 | 6108 | 8647 | 8164 |
| NM_006844 | ILVBL | 0.19 | -0.04 | 902 | 1144 | 1811 | 1630 |
| NM_001100169 | IMMT | 0.38 | 0.00 | 1096 | 1558 | 2456 | 2503 |

(continued)

| RefSeq ID | Gene Symbol | SR GFOLD | SEN GFOLD | SR IL-2- | SR IL-2 + | SEN IL-2- | SEN IL-2+ |
|---|---|---|---|---|---|---|---|
| NM_018285 | IMP3 | 0.33 | 0.00 | 494 | 712 | 668 | 726 |
| NM_033416 | IMP4 | 0.28 | -0.07 | 1156 | 1539 | 1788 | 1573 |
| NM_000884 | IMPDH2 | 0.18 | -0.23 | 3398 | 4069 | 5087 | 4142 |
| NM_001040694 | INCENP | 0.14 | 0.00 | 846 | 1038 | 1009 | 919 |
| NM_001127582 | ING4 | 0.35 | 0.00 | 169 | 270 | 699 | 789 |
| NM_002193 | INHBB | 0.40 | 0.00 | 51 | 100 | 212 | 181 |
| NM_001098817 | INO80C | 0.23 | 0.00 | 288 | 403 | 595 | 553 |
| NM_173618 | INO80E | 0.51 | 0.00 | 433 | 709 | 767 | 706 |
| NM_005539 | INPP5 A | 0.28 | 0.00 | 1631 | 2131 | 2111 | 2116 |
| NM_005540 | INPP5B | 0.31 | 0.00 | 226 | 342 | 335 | 392 |
| NM_019892 | INPP5E | 0.17 | -0.16 | 332 | 442 | 730 | 575 |
| NM_020395 | INTS12 | 0.13 | -0.03 | 378 | 485 | 842 | 734 |
| NM_023015 | INTS3 | 0.27 | 0.00 | 766 | 1031 | 2562 | 2691 |
| NM_030628 | INTS5 | 0.15 | 0.00 | 859 | 1058 | 1553 | 1645 |
| NM_018250 | INTS9 | 0.34 | 0.00 | 174 | 275 | 460 | 418 |
| NM_015693 | INTU | 0.23 | 0.00 | 61 | 104 | 193 | 165 |
| NM_001005909 | IP6K2 | 0.01 | 0.00 | 659 | 753 | 1221 | 1268 |
| NM_014652 | IPO13 | 0.43 | 0.00 | 1050 | 1550 | 2943 | 2944 |
| NM_024658 | IPO4 | 0.39 | 0.00 | 746 | 1095 | 1915 | 1834 |
| NM_006391 | IPO7 | 0.07 | 0.00 | 3259 | 3607 | 4943 | 4920 |
| NM_022755 | IPPK | 0.14 | 0.00 | 404 | 521 | 967 | 911 |
| NM_001031715 | IQCH | 0.56 | 0.00 | 4 | 23 | 85 | 70 |
| NM_153208 | IQCK | 0.21 | 0.00 | 34 | 64 | 202 | 203 |
| NM_178229 | IQGAP3 | 0.13 | -0.96 | 1625 | 1927 | 1994 | 933 |
| NM_001569 | IRAK1 | 0.17 | 0.00 | 5587 | 6539 | 9009 | 9094 |
| NM_007199 | IRAK3 | 0.43 | -0.13 | 181 | 304 | 613 | 488 |
| NM_004136 | IREB2 | 0.10 | 0.00 | 877 | 1045 | 1158 | 1187 |
| NM_015649 | IRF2BP1 | 0.07 | 0.00 | 419 | 512 | 641 | 647 |
| NM_006084 | IRF9 | 0.06 | 0.00 | 413 | 504 | 1023 | 1017 |
| NM_024337 | IRX1 | 0.28 | 0.00 | 72 | 123 | 96 | 87 |
| NM_033267 | IRX2 | 0.25 | 0.00 | 68 | 115 | 56 | 67 |
| NM_194279 | ISCA2 | 0.20 | 0.00 | 89 | 140 | 198 | 155 |
| NM_014301 | ISCU | 0.14 | 0.00 | 828 | 1019 | 2414 | 2249 |

(continued)

| RefSeq ID | Gene Symbol | SR GFOLD | SEN GFOLD | SR IL-2- | SR IL-2 + | SEN IL-2- | SEN IL-2+ |
|---|---|---|---|---|---|---|---|
| NM_030980 | ISG20L2 | 0.20 | 0.00 | 478 | 633 | 720 | 710 |
| NM_016048 | ISOC1 | 0.36 | 0.00 | 104 | 178 | 104 | 135 |
| NM_024710 | ISOC2 | 0.66 | -0.03 | 527 | 944 | 1331 | 1190 |
| NM_001199469 | ISY1 | 0.10 | 0.00 | 558 | 684 | 846 | 800 |
| NM_016368 | ISYNA1 | 0.19 | 0.00 | 285 | 389 | 277 | 311 |
| NM_018463 | ITFG2 | 0.18 | -0.01 | 252 | 347 | 551 | 473 |
| NM_003637 | ITGA10 | 0.41 | 0.00 | 285 | 450 | 832 | 841 |
| NM_001291494 | ITGA8 | 0.10 | 0.00 | 27 | 50 | 79 | 68 |
| NM_002214 | ITGB8 | 0.25 | 0.00 | 25 | 52 | 42 | 30 |
| NM_001287241 | ITM2C | 0.13 | 0.00 | 734 | 904 | 790 | 791 |
| NM_033453 | ITPA | 0.17 | -0.30 | 340 | 453 | 994 | 721 |
| NM_014216 | ITPK1 | 0.43 | 0.00 | 846 | 1264 | 1312 | 1368 |
| NM_002224 | ITPR3 | 0.05 | 0.00 | 2835 | 3117 | 9429 | 9536 |
| NM_001034841 | ITPRIPL2 | 0.09 | 0.00 | 2686 | 3037 | 7119 | 7275 |
| NM_000214 | JAG1 | 0.16 | 0.00 | 688 | 866 | 534 | 482 |
| NM_175061 | JAZF1 | 0.37 | 0.00 | 308 | 473 | 813 | 815 |
| NM_023007 | JMJD4 | 0.38 | 0.00 | 369 | 561 | 994 | 924 |
| NM_002230 | JUP | 0.29 | 0.00 | 47 | 87 | 69 | 83 |
| NM_153186 | KANK1 | 0.16 | 0.00 | 2463 | 2938 | 4440 | 4571 |
| NM_001130089 | KARS | 0.16 | -0.12 | 1917 | 2303 | 3065 | 2656 |
| NM_006388 | KAT5 | 0.23 | 0.00 | 848 | 1107 | 1605 | 1699 |
| NM_007067 | KAT7 | 0.45 | 0.00 | 529 | 823 | 1242 | 1209 |
| NM_014867 | KBTBD11 | 0.32 | 0.00 | 36 | 72 | 25 | 33 |
| NM_016506 | KBTBD4 | 0.09 | 0.00 | 505 | 619 | 950 | 996 |
| NM_032505 | KBTBD8 | 0.23 | 0.00 | 177 | 261 | 96 | 122 |
| NM_013348 | KCNJ14 | 0.06 | 0.00 | 49 | 78 | 102 | 105 |
| NM_004982 | KCNJ8 | 0.24 | 0.00 | 402 | 552 | 539 | 508 |
| NM_001017424 | KCNK2 | 0.07 | -0.34 | 2934 | 3257 | 3970 | 2965 |
| NM_031954 | KCTD10 | 0.20 | 0.00 | 2066 | 2548 | 5010 | 5242 |
| NM_001282684 | KCTD17 | 0.28 | 0.00 | 283 | 410 | 886 | 824 |
| NM_173562 | KCTD20 | 0.08 | -0.12 | 5345 | 5895 | 9446 | 8412 |
| NM_016121 | KCTD3 | 0.12 | 0.00 | 1083 | 1291 | 1761 | 1628 |

(continued)

| RefSeq ID | Gene Symbol | SR GFOLD | SEN GFOLD | SR IL-2- | SR IL-2 + | SEN IL-2- | SEN IL-2+ |
|---|---|---|---|---|---|---|---|
| NM_153705 | KDELC2 | 0.46 | -0.17 | 892 | 1360 | 1329 | 1074 |
| NM_024773 | KDM8 | 1.45 | 0.00 | 25 | 113 | 119 | 111 |
| NM_006558 | KHDRBS3 | 0.14 | 0.00 | 345 | 449 | 214 | 187 |
| NM_003685 | KHSRP | 0.08 | -0.19 | 1516 | 1738 | 1874 | 1519 |
| NM_001145642 | KIAA0226 | 0.06 | 0.00 | 1223 | 1401 | 1895 | 1782 |
| NM_014734 | KIAA0247 | 0.21 | 0.00 | 852 | 1093 | 1225 | 1275 |
| NM_014732 | KIAA0513 | 0.13 | 0.00 | 656 | 811 | 1522 | 1590 |
| NM_001244189 | KIAA0586 | 0.10 | 0.00 | 323 | 412 | 528 | 457 |
| NM_001100425 | KIAA0895 | 0.19 | -0.42 | 118 | 177 | 135 | 72 |
| NM_015634 | KIAA1279 | 0.35 | 0.00 | 849 | 1198 | 1957 | 2094 |
| NM_015496 | KIAA1429 | 0.28 | 0.00 | 1089 | 1456 | 2637 | 2780 |
| NM_020844 | KIAA1456 | 0.86 | 0.00 | 17 | 58 | 4 | 13 |
| NM_020890 | KIAA1524 | 0.15 | -0.61 | 297 | 394 | 403 | 218 |
| NM_001105568 | KIF13A | 0.08 | 0.00 | 3630 | 4039 | 6129 | 6399 |
| NM_020242 | KIF15 | 0.43 | -0.60 | 131 | 228 | 179 | 87 |
| NM_001264573 | KIF18B | 0.14 | -1.16 | 719 | 891 | 520 | 192 |
| NM_005733 | KIF20A | 0.10 | -1.28 | 1309 | 1528 | 1258 | 458 |
| NM_001256269 | KIF22 | 0.15 | -0.48 | 963 | 1183 | 986 | 630 |
| NM_138555 | KIF23 | 0.05 | -0.54 | 1094 | 1251 | 1013 | 619 |
| NM_006845 | KIF2C | 0.22 | -0.59 | 734 | 959 | 913 | 536 |
| NM_002263 | KIFC1 | 0.10 | -0.36 | 1217 | 1429 | 643 | 434 |
| NM_001130100 | KIFC3 | 0.12 | -0.16 | 2078 | 2414 | 3321 | 2800 |
| NM_032531 | KIRREL3 | 0.08 | 0.00 | 2876 | 3236 | 1660 | 1715 |
| NM_000222 | KIT | 0.30 | -1.55 | 34 | 68 | 77 | 13 |
| NM_000899 | KITLG | 0.24 | -0.45 | 1768 | 2247 | 3831 | 2645 |
| NM_016270 | KLF2 | 0.14 | 0.00 | 667 | 831 | 223 | 192 |
| NM_016531 | KLF3 | 0.11 | 0.00 | 708 | 862 | 1069 | 1113 |
| NM_001206 | KLF9 | 0.28 | 0.01 | 592 | 813 | 1876 | 2033 |
| NM_173546 | KLHDC8B | 0.23 | -0.18 | 529 | 711 | 1464 | 1176 |
| NM_021633 | KLHL12 | 0.74 | 0.00 | 243 | 487 | 928 | 832 |
| NM_001007075 | KLHL5 | 0.18 | 0.00 | 420 | 553 | 1046 | 1026 |

(continued)

| RefSeq ID | Gene Symbol | SR GFOLD | SEN GFOLD | SR IL-2- | SR IL-2 + | SEN IL-2- | SEN IL-2+ |
|---|---|---|---|---|---|---|---|
| NM_018847 | KLHL9 | 0.24 | 0.00 | 869 | 1141 | 1601 | 1647 |
| NM_014708 | KNTC1 | 0.02 | -0.38 | 633 | 729 | 786 | 530 |
| NM_002266 | KPNA2 | 0.31 | -0.53 | 1865 | 2491 | 2932 | 1891 |
| NM_002265 | KPNB1 | 0.19 | 0.00 | 6752 | 8002 | 9013 | 9246 |
| NM_016618 | KRCC1 | 0.16 | 0.00 | 285 | 382 | 603 | 673 |
| NM_199187 | KRT18 | 0.63 | 0.00 | 2353 | 3873 | 980 | 1067 |
| NM_002277 | KRT31 | 0.81 | -0.45 | 46 | 120 | 403 | 244 |
| NM_002279 | KRT33B | 0.13 | 0.00 | 62 | 99 | 250 | 246 |
| NM_021013 | KRT34 | 0.47 | -0.05 | 1517 | 2268 | 7254 | 6743 |
| NM_030967 | KRTAP1-1 | 1.04 | -0.01 | 2 | 21 | 742 | 651 |
| NM_031957 | KRTAP1-5 | 0.14 | -0.26 | 328 | 430 | 2514 | 1956 |
| NM_173852 | KRTCAP2 | 0.39 | 0.00 | 362 | 554 | 1368 | 1484 |
| NM_014238 | KSR1 | 0.71 | 0.00 | 81 | 180 | 239 | 233 |
| NM_138417 | KTI12 | 0.56 | 0.00 | 474 | 799 | 736 | 791 |
| NM_001171948 | KXD1 | 0.51 | -0.01 | 1479 | 2270 | 4207 | 3969 |
| NM_144581 | L3HYPDH | 0.35 | 0.00 | 304 | 461 | 293 | 349 |
| NM_031488 | L3MBTL2 | 0.46 | 0.00 | 594 | 921 | 1385 | 1309 |
| NM_001105207 | LAMA4 | 0.13 | -0.90 | 1519 | 1805 | 7836 | 4002 |
| NM_002291 | LAMB1 | 0.02 | -0.20 | 5680 | 6023 | 17211 | 14556 |
| NM_017907 | LAMTOR1 | 0.33 | -0.23 | 1354 | 1855 | 3768 | 3030 |
| NM_001243736 | LAMTOR3 | 0.24 | 0.00 | 192 | 281 | 512 | 577 |
| NM_001008395 | LAMTOR4 | 0.14 | 0.00 | 693 | 859 | 1736 | 1678 |
| NM_006402 | LAMTOR5 | 0.40 | 0.00 | 505 | 760 | 1262 | 1384 |
| NM_015907 | LAP3 | 0.08 | -0.25 | 422 | 518 | 1215 | 921 |
| NM_004737 | LARGE | 0.13 | 0.00 | 844 | 1030 | 1955 | 2018 |
| NM_018078 | LARP1B | 0.14 | 0.00 | 285 | 376 | 272 | 240 |
| NM_052879 | LARP4 | 0.34 | 0.00 | 1078 | 1499 | 1506 | 1512 |
| NM_015155 | LARP4B | 0.13 | 0.00 | 2281 | 2662 | 2743 | 2874 |
| NM_031206 | LAS1L | 0.33 | -0.01 | 441 | 638 | 1034 | 924 |
| NM_001258390 | LAYN | 0.18 | -0.66 | 629 | 807 | 2171 | 1268 |
| NM_002296 | LBR | 0.27 | 0.00 | 543 | 744 | 439 | 394 |
| NM_001009812 | LBX2 | 0.63 | 0.00 | 19 | 54 | 116 | 116 |
| NM_014793 | LCMT2 | 0.36 | 0.00 | 306 | 465 | 430 | 464 |

(continued)

| RefSeq ID | Gene Symbol | SR GFOLD | SEN GFOLD | SR IL-2- | SR IL-2 + | SEN IL-2- | SEN IL-2+ |
|---|---|---|---|---|---|---|---|
| NM_001170765 | LCOR | 0.21 | -0.04 | 536 | 706 | 776 | 669 |
| NM_153686 | LCORL | 0.21 | -0.11 | 175 | 255 | 152 | 105 |
| NM_001174097 | LDHB | 0.20 | -0.18 | 2524 | 3087 | 5261 | 4423 |
| NM_174902 | LDLRAD3 | 0.28 | 0.00 | 167 | 255 | 393 | 410 |
| NM_012318 | LETM1 | 0.22 | 0.00 | 2328 | 2888 | 3635 | 3693 |
| NM_001243689 | LETMD1 | 0.11 | 0.00 | 333 | 425 | 657 | 688 |
| NM_005567 | LGALS3BP | 0.34 | -0.18 | 4250 | 5634 | 5890 | 4970 |
| NM_006859 | LIAS | 0.80 | 0.00 | 25 | 74 | 110 | 102 |
| NM_013975 | LIG3 | 0.10 | 0.00 | 702 | 848 | 1117 | 1076 |
| NM_001113547 | LIMA1 | 0.37 | 0.00 | 10458 | 13954 | 12813 | 12793 |
| NM_001024674 | LIN52 | 0.12 | 0.00 | 165 | 227 | 129 | 131 |
| NM_005357 | LIPE | 0.30 | 0.00 | 68 | 119 | 42 | 39 |
| NM_170707 | LMNA | 0.24 | -0.22 | 22240 | 26812 | 49065 | 41411 |
| NM_005573 | LMNB1 | 0.02 | -0.55 | 816 | 927 | 283 | 153 |
| NM_012134 | LMOD1 | 0.40 | -0.40 | 198 | 322 | 1522 | 1052 |
| RefSeq ID | Gene Symbol | SR GFOLD | SEN GFOLD | SR IL-2- | SR IL-2 + | SEN IL-2- | SEN IL-2+ |
| NM_001146181 | LOC649330 | 1.53 | 0.00 | 28 | 130 | 150 | 126 |
| NM_001199053 | LOC81691 | 0.79 | -0.07 | 63 | 154 | 160 | 115 |
| NM_153613 | LPCAT4 | 0.12 | 0.00 | 1208 | 1442 | 1535 | 1486 |
| NM_001164213 | LRCH1 | 0.03 | 0.00 | 606 | 702 | 1100 | 1092 |
| NM_153377 | LRIG3 | 0.22 | -0.23 | 120 | 183 | 225 | 150 |
| NM_002333 | LRP3 | 0.59 | 0.00 | 180 | 335 | 1250 | 1244 |
| NM_182492 | LRP5L | 0.19 | 0.00 | 43 | 76 | 56 | 76 |
| NM_133259 | LRPPRC | 0.27 | 0.00 | 1598 | 2087 | 3361 | 3220 |
| NM_024512 | LRRC2 | 0.19 | -0.47 | 288 | 393 | 208 | 115 |
| NM_001278211 | LRRC20 | 0.78 | -0.09 | 198 | 414 | 354 | 277 |
| NM_001143757 | LRRC27 | 2.14 | 0.00 | 9 | 104 | 189 | 227 |
| NM_001010847 | LRRC38 | 0.26 | 0.00 | 121 | 190 | 8 | 4 |
| NM_017768 | LRRC40 | 0.31 | 0.00 | 225 | 340 | 464 | 405 |

(continued)

| RefSeq ID | Gene Symbol | SR GFOLD | SEN GFOLD | SR IL-2- | SR IL-2 + | SEN IL-2- | SEN IL-2+ |
|---|---|---|---|---|---|---|---|
| NM_001256409 | LRRC42 | 0.48 | 0.00 | 684 | 1066 | 1316 | 1314 |
| NM_018509 | LRRC59 | 0.13 | -0.02 | 11496 | 12994 | 10752 | 10279 |
| NM_001113567 | LRRC75A | 0.16 | 0.00 | 193 | 269 | 252 | 262 |
| NM_001127244 | LRRC8A | 0.12 | -0.02 | 3710 | 4256 | 6709 | 6370 |
| NM_138361 | LRSAM1 | 0.05 | 0.00 | 348 | 425 | 701 | 793 |
| NM_152892 | LRWD1 | 0.22 | 0.00 | 352 | 483 | 697 | 619 |
| NM_002338 | LSAMP | 0.27 | 0.00 | 51 | 92 | 127 | 144 |
| NM_018385 | LSG1 | 0.14 | 0.00 | 751 | 926 | 1256 | 1244 |
| NM_032881 | LSM10 | 0.19 | 0.00 | 718 | 919 | 1198 | 1146 |
| NM_152344 | LSM12 | 0.05 | 0.00 | 763 | 889 | 888 | 983 |
| NM_021177 | LSM2 | 0.26 | -0.53 | 225 | 330 | 435 | 250 |
| NM_014463 | LSM3 | 0.82 | 0.00 | 63 | 157 | 212 | 190 |
| NM_016199 | LSM7 | 0.89 | -0.17 | 224 | 500 | 678 | 525 |
| NM_001260489 | LSR | 0.46 | 0.00 | 31 | 70 | 75 | 68 |
| NM_002342 | LTBR | 0.12 | -0.01 | 2724 | 3155 | 5584 | 5288 |
| NM_001127213 | LY6E | 0.39 | 0.00 | 1764 | 2491 | 3899 | 3939 |
| NM_021221 | LY6G5B | 0.22 | 0.00 | 37 | 69 | 116 | 150 |
| NM_144586 | LYPD1 | 0.32 | 0.00 | 425 | 613 | 89 | 100 |
| NM_001279358 | LYPLA1 | 0.14 | 0.00 | 251 | 336 | 318 | 364 |
| NM_138794 | LYPLAL1 | 0.29 | 0.00 | 70 | 121 | 264 | 277 |
| NM_020424 | LYRM1 | 0.07 | 0.00 | 121 | 167 | 343 | 318 |
| NM_001076680 | LYRM9 | 0.33 | 0.00 | 46 | 88 | 262 | 248 |
| NM_002355 | M6PR | 0.09 | 0.00 | 1658 | 1908 | 2828 | 2688 |
| NM_014067 | MACROD1 | 0.33 | 0.00 | 163 | 258 | 537 | 492 |
| NM_001013836 | MAD1L1 | 0.01 | 0.00 | 981 | 1096 | 975 | 887 |
| NM_002358 | MAD2L1 | 0.35 | -0.43 | 166 | 267 | 208 | 118 |
| RefSeq ID | Gene Symbol | SR GFOLD | SEN GFOLD | SR IL-2- | SR IL-2 + | SEN IL-2 - | SEN IL-2 + |
| NM_006341 | MAD2L2 | 0.36 | 0.00 | 393 | 586 | 1061 | 1070 |
| NM_001031804 | MAF | 0.46 | 0.00 | 22 | 54 | 44 | 44 |
| NM_005461 | MAFB | 0.93 | 0.00 | 79 | 204 | 455 | 390 |

(continued)

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_002360 | MAFK | 0.05 | 0.00 | 2105 | 2335 | 1354 | 1423 |
| NM_201222 | MAGED2 | 0.06 | 0.00 | 3885 | 4253 | 6574 | 6828 |
| NM_001272061 | MAGED4 | 0.34 | -0.08 | 635 | 904 | 730 | 606 |
| NM_001242362 | MAGED4B | 0.34 | -0.08 | 635 | 904 | 730 | 606 |
| NM_005491 | MAMLD1 | 0.21 | 0.00 | 391 | 529 | 439 | 397 |
| NM_001256494 | MAN2C1 | 0.04 | 0.00 | 864 | 991 | 1907 | 1946 |
| NM_022077 | MANBAL | 0.44 | 0.00 | 926 | 1384 | 2277 | 2207 |
| NM_019090 | MAP10 | 1.07 | 0.00 | 12 | 55 | 27 | 31 |
| NM_002419 | MAP3K11 | 0.11 | 0.00 | 852 | 1024 | 1323 | 1405 |
| NM_003954 | MAP3K14 | 0.55 | -0.08 | 275 | 480 | 607 | 499 |
| NM_004672 | MAP3K6 | 0.04 | 0.00 | 1036 | 1179 | 2204 | 2347 |
| NM_144578 | MAPK1IP1L | 0.17 | 0.00 | 2404 | 2877 | 3606 | 3763 |
| NM_001006617 | MAPKAP1 | 0.17 | -0.17 | 2344 | 2822 | 6879 | 5863 |
| NM_004759 | MAPKAPK2 | 0.07 | -0.04 | 3669 | 4067 | 4309 | 3976 |
| NM_001243925 | MAPKAPK3 | 0.31 | -0.02 | 455 | 648 | 1705 | 1555 |
| NM_012325 | MAPRE1 | 0.40 | 0.00 | 1761 | 2491 | 3814 | 3782 |
| NM_014268 | MAPRE2 | 0.36 | -0.02 | 526 | 772 | 1520 | 1375 |
| NM_017824 | MARCH5 | 0.45 | 0.00 | 667 | 1022 | 1040 | 1052 |
| NM_002356 | MARCKS | 0.07 | 0.00 | 3042 | 3389 | 4563 | 4499 |
| NM_138395 | MARS2 | 0.04 | 0.00 | 204 | 261 | 237 | 220 |
| NM_031484 | MARVELD1 | 0.48 | 0.00 | 1661 | 2486 | 2884 | 2791 |
| NM_001042539 | MAZ | 0.37 | -0.24 | 3022 | 4128 | 3492 | 2784 |
| NM_178496 | MB21D2 | 0.03 | -0.66 | 506 | 596 | 632 | 342 |
| NM_001144891 | MBIP | 0.36 | 0.00 | 147 | 241 | 264 | 257 |
| NM_203406 | MBLAC2 | 0.40 | -0.10 | 156 | 261 | 206 | 150 |
| NM_001080480 | MBOAT1 | 0.08 | 0.00 | 37 | 63 | 21 | 31 |
| NM_022132 | MCCC2 | 0.20 | -0.02 | 347 | 469 | 857 | 754 |
| NM_032601 | MCEE | 0.07 | 0.00 | 103 | 146 | 135 | 174 |
| NM_018518 | MCM10 | 0.51 | -0.75 | 474 | 772 | 193 | 85 |
| NM_004526 | MCM2 | 0.11 | -0.20 | 1864 | 2170 | 1389 | 1098 |
| NM_002388 | MCM3 | 0.30 | -0.33 | 2355 | 3087 | 1622 | 1177 |

(continued)

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_005914 | MCM4 | 0.53 | -0.22 | 2312 | 3545 | 2685 | 2157 |
| NM_006739 | MCM5 | 0.08 | -0.16 | 2710 | 3050 | 1868 | 1538 |
| NM_005915 | MCM6 | 0.17 | 0.00 | 1615 | 1969 | 1562 | 1471 |
| NM_001278595 | MCM7 | 0.02 | -0.31 | 3999 | 4282 | 2398 | 1795 |
| NM_001281520 | MCM8 | 0.36 | -0.01 | 279 | 427 | 362 | 299 |
| RefSeq ID | Gene Symbol | SR GFOLD | SEN GFOLD | SR IL-2- | SR IL-2 + | SEN IL-2 - | SEN IL-2 + |
| NM_006337 | MCRS1 | 0.41 | 0.00 | 1044 | 1526 | 1984 | 1922 |
| NM_014641 | MDC1 | 0.38 | 0.00 | 943 | 1357 | 1358 | 1364 |
| NM_005918 | MDH2 | 0.22 | -0.16 | 3961 | 4841 | 8106 | 6983 |
| NM_001199822 | MDP1 | 0.02 | 0.00 | 124 | 166 | 183 | 229 |
| NM_001161586 | ME3 | 0.36 | 0.00 | 142 | 234 | 568 | 603 |
| NM_001024732 | MECR | 0.01 | 0.00 | 269 | 329 | 678 | 638 |
| NM_005120 | MED12 | 0.01 | 0.00 | 1095 | 1216 | 2132 | 2074 |
| NM_015335 | MED13L | 0.05 | -0.03 | 3484 | 3802 | 5991 | 5623 |
| NM_005481 | MED16 | 0.26 | -0.19 | 994 | 1311 | 2308 | 1885 |
| NM_004268 | MED17 | 0.24 | 0.00 | 411 | 564 | 609 | 673 |
| NM_153450 | MED19 | 0.26 | 0.00 | 235 | 342 | 564 | 627 |
| NM_004830 | MED23 | 0.14 | 0.00 | 722 | 894 | 1077 | 1181 |
| NM_014815 | MED24 | 0.05 | 0.00 | 1735 | 1944 | 2972 | 2823 |
| NM_030973 | MED25 | 0.27 | 0.00 | 387 | 546 | 846 | 830 |
| NM_016060 | MED31 | 0.46 | 0.00 | 209 | 351 | 410 | 455 |
| NM_002398 | MEIS1 | 0.07 | -0.17 | 252 | 322 | 944 | 749 |
| NM_014791 | MELK | 0.10 | -0.20 | 647 | 783 | 593 | 445 |
| NM_015955 | MEMO1 | 0.11 | 0.00 | 135 | 189 | 318 | 311 |
| NM_002402 | MEST | 0.81 | -0.27 | 182 | 393 | 195 | 124 |
| NM_015143 | METAP1 | 0.14 | 0.00 | 1393 | 1669 | 2608 | 2525 |
| NM_199227 | METAP1D | 0.21 | 0.00 | 16 | 37 | 110 | 100 |
| NM_024042 | METRN | 0.13 | -0.01 | 271 | 358 | 900 | 797 |
| NM_015935 | METTL13 | 0.06 | -0.02 | 916 | 1064 | 1524 | 1383 |
| NM_020961 | METTL14 | 0.33 | 0.00 | 245 | 372 | 464 | 458 |
| NM_024109 | METTL22 | 0.22 | 0.00 | 485 | 649 | 1054 | 1129 |
| NM_001206983 | METTL23 | 0.04 | 0.00 | 260 | 324 | 443 | 381 |

(continued)

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_019852 | METTL3 | 0.25 | -0.10 | 1047 | 1369 | 1655 | 1420 |
| NM_014168 | METTL5 | 0.25 | 0.00 | 287 | 408 | 489 | 436 |
| NM_024770 | METTL8 | 0.07 | 0.00 | 234 | 300 | 566 | 567 |
| NM_001077180 | METTL9 | 0.21 | 0.00 | 1203 | 1527 | 3795 | 3922 |
| NM_001174118 | MEX3D | 0.06 | -0.18 | 529 | 634 | 855 | 667 |
| NM_005926 | MFAP1 | 0.12 | 0.00 | 410 | 519 | 572 | 599 |
| NM_001135248 | MFAP2 | 0.17 | 0.00 | 821 | 1032 | 1408 | 1534 |
| NM_033540 | MFN1 | 0.05 | 0.00 | 523 | 622 | 948 | 961 |
| NM_014874 | MFN2 | 0.19 | 0.00 | 2687 | 3253 | 6000 | 6035 |
| NM_001242534 | MFSD11 | 0.56 | 0.00 | 370 | 637 | 1123 | 1174 |
| NM_174983 | MFSD12 | 0.02 | 0.00 | 580 | 672 | 1250 | 1347 |
| NM_001136493 | MFSD2A | 0.48 | 0.00 | 130 | 233 | 154 | 189 |
| NM_001170790 | MFSD5 | 0.11 | 0.00 | 732 | 889 | 1231 | 1251 |
| RefSeq ID | Gene Symbol | SR GFOLD | SEN GFOLD | SR IL-2+ | SR IL-2 + | SEN IL-2 - | SEN IL-2 + |
| NM_052865 | MGME1 | 0.60 | 0.00 | 195 | 363 | 420 | 488 |
| NM_001260512 | MGST1 | 0.08 | 0.00 | 2082 | 2366 | 4717 | 4741 |
| NM_001204366 | MGST2 | 0.47 | 0.00 | 33 | 74 | 139 | 142 |
| NM_001286613 | MICAL1 | 0.08 | 0.00 | 758 | 896 | 1730 | 1595 |
| NM_033386 | MICALL1 | 0.23 | 0.00 | 3082 | 3833 | 2884 | 2924 |
| NM_182924 | MICALL2 | 0.35 | 0.00 | 231 | 357 | 630 | 567 |
| NM_001289160 | MICB | 0.30 | 0.00 | 514 | 725 | 632 | 604 |
| NM_001195518 | MICU1 | 0.09 | 0.00 | 1203 | 1406 | 3404 | 3571 |
| NM_152726 | MICU2 | 0.44 | 0.00 | 212 | 352 | 736 | 712 |
| NM_139162 | MIEF2 | 0.28 | 0.00 | 263 | 385 | 526 | 556 |
| NM_032339 | MIEN1 | 0.17 | -0.09 | 733 | 923 | 1522 | 1312 |
| NM_021933 | MIIP | 0.18 | 0.00 | 198 | 279 | 399 | 408 |
| NM_019005 | MIOS | 0.21 | 0.00 | 308 | 425 | 574 | 519 |
| NM_138798 | MITD1 | 0.09 | 0.00 | 192 | 255 | 358 | 336 |
| NM_017777 | MKS1 | 0.02 | 0.00 | 172 | 221 | 333 | 275 |

(continued)

| RefSeq ID | Gene Symbol | SR GFOLD | SEN GFOLD | SR IL-2+ | SR IL-2 + | SEN IL-2 - | SEN IL-2 + |
|---|---|---|---|---|---|---|---|
| NM_001242702 | MKX | 0.57 | 0.00 | 31 | 75 | 229 | 211 |
| NM_001130157 | MLF1 | 0.85 | 0.00 | 30 | 88 | 202 | 165 |
| NM_000249 | MLH1 | 0.16 | 0.00 | 502 | 646 | 984 | 1020 |
| NM_005937 | MLLT6 | 0.10 | 0.00 | 1278 | 1494 | 2003 | 2085 |
| NM_170607 | MLX | 0.24 | -0.12 | 1716 | 2180 | 3198 | 2763 |
| NM_002421 | MMP1 | 0.50 | -0.04 | 11316 | 16457 | 6268 | 5830 |
| NM_002425 | MMP10 | 0.51 | 0.00 | 21 | 54 | 114 | 104 |
| NM_002426 | MMP12 | 0.05 | -0.23 | 14 | 31 | 98 | 57 |
| NM_002428 | MMP15 | 0.02 | 0.00 | 214 | 268 | 551 | 582 |
| NM_005941 | MMP16 | 0.24 | 0.00 | 172 | 256 | 374 | 322 |
| NM_002422 | MMP3 | 0.15 | -0.04 | 4384 | 5116 | 17523 | 16658 |
| NM_022362 | MMS19 | 0.22 | 0.00 | 1704 | 2139 | 3124 | 3298 |
| NM_002430 | MN1 | 0.42 | 0.00 | 326 | 513 | 998 | 1037 |
| NM_018221 | MOB1A | 0.13 | 0.00 | 741 | 911 | 1393 | 1403 |
| NM_130807 | MOB3A | 0.04 | 0.00 | 1554 | 1730 | 3213 | 3410 |
| NM_014226 | MOK | 0.14 | 0.00 | 2765 | 3236 | 1385 | 1318 |
| NM_014940 | MON1B | 0.03 | 0.00 | 678 | 781 | 973 | 1013 |
| NM_024657 | MORC4 | 0.09 | 0.00 | 439 | 541 | 1038 | 1037 |
| NM_206839 | MORF4L1 | 0.80 | 0.00 | 887 | 1699 | 1884 | 2033 |
| NM_001286072 | MOV10 | 0.07 | -0.09 | 809 | 947 | 2161 | 1889 |
| NM_002434 | MPG | 0.54 | -0.04 | 617 | 1013 | 1460 | 1301 |
| NM_022782 | MPHOSPH9 | 0.09 | 0.00 | 340 | 429 | 480 | 455 |
| NM_002435 | MPI | 0.29 | 0.00 | 606 | 837 | 1368 | 1434 |
| NM_001130517 | MPST | 0.32 | 0.00 | 315 | 466 | 520 | 455 |
| RefSeq ID | Gene Symbol | SR GFOLD | SEN GFOLD | SR IL-2 - | SR IL-2 + | SEN IL-2 - | SEN IL-2 + |
| NM_002437 | MPV17 | 0.07 | 0.00 | 675 | 801 | 1429 | 1388 |
| NM_005590 | MRE11A | 0.37 | -0.24 | 162 | 264 | 389 | 274 |
| NM_001272054 | MRFAP1 | 0.02 | -0.12 | 5828 | 6145 | 9279 | 8268 |
| NM_001098515 | MRGPRF | 0.46 | -0.20 | 249 | 412 | 638 | 482 |
| NM_001031727 | MRI1 | 0.12 | 0.00 | 508 | 634 | 1121 | 1150 |
| NM_032450 | MROH1 | 0.46 | 0.00 | 655 | 1012 | 1992 | 2091 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_020236 | MRPL1 | 0.24 | 0.00 | 112 | 175 | 231 | 242 |
| NM_002949 | MRPL12 | 0.19 | -0.30 | 858 | 1089 | 1410 | 1042 |
| NM_017840 | MRPL16 | 0.05 | -0.43 | 420 | 507 | 1044 | 693 |
| NM_014161 | MRPL18 | 0.16 | 0.00 | 989 | 1222 | 1734 | 1654 |
| NM_017971 | MRPL20 | 0.45 | -0.03 | 451 | 710 | 1204 | 1067 |
| NM_181514 | MRPL21 | 0.03 | -0.11 | 587 | 684 | 932 | 773 |
| NM_014180 | MRPL22 | 0.11 | 0.00 | 285 | 370 | 562 | 510 |
| NM_021134 | MRPL23 | 0.54 | -0.15 | 490 | 815 | 1129 | 919 |
| NM_007208 | MRPL3 | 0.11 | 0.00 | 579 | 713 | 796 | 824 |
| NM_004891 | MRPL33 | 0.05 | -0.27 | 475 | 570 | 747 | 542 |
| NM_016622 | MRPL35 | 0.48 | 0.00 | 347 | 566 | 830 | 823 |
| NM_032479 | MRPL36 | 0.12 | -0.04 | 647 | 793 | 1140 | 1002 |
| NM_003776 | MRPL40 | 0.31 | -0.31 | 214 | 326 | 786 | 556 |
| NM_032477 | MRPL41 | 0.13 | -0.17 | 463 | 585 | 1381 | 1118 |
| NM_020409 | MRPL47 | 0.40 | -0.01 | 259 | 412 | 562 | 484 |
| NM_016497 | MRPL51 | 0.14 | -0.05 | 974 | 1189 | 1770 | 1579 |
| NM_181304 | MRPL52 | 0.15 | -0.14 | 650 | 816 | 1616 | 1342 |
| NM_181462 | MRPL55 | 0.53 | -0.14 | 245 | 427 | 848 | 682 |
| NM_024026 | MRPL57 | 0.44 | -0.31 | 302 | 485 | 890 | 636 |
| NM_022839 | MRPS 11 | 0.29 | -0.28 | 512 | 715 | 1239 | 924 |
| NM_033363 | MRPS12 | 0.38 | -0.15 | 483 | 719 | 932 | 749 |
| NM_031280 | MRPS15 | 0.08 | -0.04 | 976 | 1140 | 2225 | 2015 |
| NM_015969 | MRPS17 | 0.38 | 0.00 | 203 | 326 | 391 | 333 |
| NM_018135 | MRPS18A | 0.34 | -0.14 | 387 | 572 | 838 | 673 |
| NM_016070 | MRPS23 | 0.08 | 0.00 | 499 | 608 | 1129 | 1102 |
| NM_032014 | MRPS24 | 0.24 | -0.01 | 1109 | 1439 | 1633 | 1495 |
| NM_030811 | MRPS26 | 0.06 | 0.00 | 425 | 515 | 570 | 536 |
| NM_005830 | MRPS31 | 0.20 | 0.00 | 161 | 235 | 327 | 355 |
| NM_016071 | MRPS33 | 0.48 | 0.00 | 112 | 205 | 347 | 312 |
| NM_023936 | MRPS34 | 0.08 | -0.22 | 964 | 1131 | 1934 | 1534 |
| NM_001286264 | MRS2 | 0.52 | 0.00 | 170 | 305 | 482 | 462 |
| NM_016183 | MRTO4 | 0.03 | -0.20 | 718 | 824 | 1185 | 932 |
| NM_130385 | MRVI1 | 0.05 | -0.99 | 657 | 768 | 3103 | 1455 |
| NM_024631 | MSANTD2 | 0.72 | 0.00 | 24 | 68 | 85 | 102 |
| NM_032424 | MSANTD4 | 0.16 | 0.00 | 433 | 561 | 695 | 656 |
| NM_000251 | MSH2 | 0.43 | 0.00 | 381 | 599 | 449 | 518 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_078629 | MSL3 | 0.51 | 0.00 | 134 | 245 | 414 | 442 |
| NM_001193460 | MSRB3 | 0.41 | 0.00 | 1555 | 2237 | 3275 | 3307 |
| NM_001256532 | MSTO1 | 0.65 | -0.15 | 255 | 480 | 903 | 723 |
| NM_004739 | MTA2 | 0.01 | 0.00 | 2380 | 2563 | 2679 | 2856 |
| NM_002451 | MTAP | 0.03 | 0.00 | 696 | 799 | 1316 | 1381 |
| NM_014342 | MTCH2 | 0.02 | 0.00 | 952 | 1068 | 1772 | 1723 |
| NM_001018025 | MTCP1 | 0.46 | 0.00 | 35 | 77 | 143 | 135 |
| NM_001164391 | MTF2 | 0.11 | 0.00 | 168 | 229 | 181 | 194 |
| NM_139242 | MTFMT | 0.32 | 0.00 | 173 | 270 | 399 | 425 |
| NM_014637 | MTFR1 | 0.07 | 0.00 | 583 | 699 | 911 | 1013 |
| NM_001099625 | MTFR1L | 0.35 | -0.03 | 833 | 1177 | 2535 | 2320 |
| NM_015666 | MTG2 | 0.03 | 0.00 | 486 | 571 | 699 | 693 |
| NM_005956 | MTHFD1 | 0.16 | -0.29 | 1020 | 1258 | 1957 | 1475 |
| NM_001005369 | MTIF2 | 0.36 | 0.00 | 352 | 529 | 861 | 793 |
| NM_017762 | MTMR10 | 0.21 | 0.00 | 904 | 1159 | 1225 | 1211 |
| NM_001243571 | MTMR2 | 0.12 | 0.00 | 1706 | 2003 | 2504 | 2617 |
| NM_004687 | MTMR4 | 0.34 | 0.00 | 783 | 1105 | 1343 | 1408 |
| NM_004685 | MTMR6 | 0.13 | 0.00 | 729 | 896 | 1381 | 1462 |
| NM_004294 | MTRF1 | 1.28 | 0.00 | 14 | 67 | 79 | 109 |
| NM_019041 | MTRF1L | 0.14 | 0.00 | 252 | 336 | 505 | 494 |
| NM_001190708 | MTRNR2L10 | 1.48 | 0.00 | 132 | 464 | 2026 | 2159 |
| NM_001190487 | MTRNR2L6 | 2.14 | -0.03 | 56 | 344 | 1701 | 1536 |
| NM_152793 | MTURN | 0.27 | 0.00 | 287 | 415 | 1146 | 1259 |
| NM_002455 | MTX1 | 0.03 | 0.00 | 524 | 616 | 988 | 911 |
| NM_025128 | MUS81 | 0.64 | -0.04 | 571 | 1003 | 1215 | 1074 |
| NM_000255 | MUT | 0.31 | 0.00 | 386 | 559 | 767 | 776 |
| NM_138401 | MVB12A | 0.08 | 0.00 | 359 | 446 | 736 | 717 |
| NM_002461 | MVD | 0.46 | -0.16 | 276 | 453 | 824 | 654 |
| NM_001144925 | MX1 | 0.63 | 0.00 | 37 | 90 | 62 | 83 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001142935 | MXD3 | 0.55 | 0.00 | 772 | 1259 | 2086 | 1972 |
| NM_032348 | MXRA8 | 0.26 | 0.00 | 5628 | 7036 | 10088 | 10194 |
| NM_014520 | MYBBP1A | 0.20 | 0.01 | 2029 | 2492 | 3369 | 3586 |
| NM_002466 | MYBL2 | 0.48 | -1.15 | 958 | 1471 | 327 | 115 |
| NM_002467 | MYC | 0.25 | 0.00 | 2140 | 2715 | 3305 | 3377 |
| NM_012333 | MYCBP | 0.28 | 0.00 | 150 | 232 | 410 | 442 |
| NM_001172567 | MYD88 | 0.21 | 0.00 | 693 | 901 | 1481 | 1479 |
| NM_002473 | MYH9 | 0.19 | 0.00 | 29788 | 34631 | 57483 | 58380 |
| NM_021019 | MYL6 | 0.44 | -0.08 | 10866 | 15214 | 22801 | 21125 |
| NM_006097 | MYL9 | 0.83 | -0.21 | 10584 | 19391 | 38407 | 32665 |
| NM_001185118 | MYNN | 0.43 | 0.00 | 180 | 302 | 362 | 370 |
| NM_078471 | MYO18A | 0.29 | 0.00 | 871 | 1178 | 4059 | 4151 |
| NM_001163735 | MYO19 | 0.18 | -0.25 | 1563 | 1921 | 1988 | 1543 |
| NM_004998 | MYO1E | 0.18 | -0.13 | 2962 | 3547 | 8920 | 7887 |
| NM_001080527 | MYO7B | 0.04 | 0.00 | 171 | 223 | 54 | 41 |
| NM_001146312 | MYOCD | 0.10 | 0.00 | 691 | 836 | 4 | 13 |
| NM_013451 | MYOF | 0.11 | -0.13 | 16695 | 18456 | 32033 | 28645 |
| NM_001012643 | MYPOP | 0.61 | 0.00 | 82 | 171 | 223 | 201 |
| NM_001071775 | MZT1 | 0.14 | 0.00 | 241 | 323 | 275 | 316 |
| NM_001256120 | NAA10 | 0.19 | 0.00 | 1145 | 1432 | 2221 | 2259 |
| NM_024635 | NAA35 | 0.31 | 0.00 | 325 | 475 | 803 | 900 |
| NM_025146 | NAA50 | 0.12 | 0.00 | 2337 | 2712 | 3217 | 3274 |
| NM_005468 | NAALADL1 | 0.14 | 0.00 | 170 | 237 | 366 | 359 |
| NM_024068 | NABP2 | 0.02 | 0.00 | 436 | 514 | 782 | 762 |
| NM_199290 | NACA2 | 0.04 | -0.20 | 1980 | 2190 | 3529 | 2900 |
| NM_001146334 | NACAD | 0.54 | 0.00 | 78 | 156 | 243 | 296 |
| NM_052876 | NACC1 | 0.05 | 0.00 | 3329 | 3642 | 5792 | 5863 |
| NM_144653 | NACC2 | 0.05 | -0.06 | 1557 | 1744 | 2776 | 2493 |
| NM_018161 | NADSYN1 | 0.31 | 0.00 | 807 | 1114 | 2059 | 2129 |
| NM_138386 | NAF1 | 0.68 | 0.00 | 112 | 234 | 189 | 213 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_000262 | NAGA | 0.12 | 0.00 | 994 | 1197 | 2441 | 2379 |
| NM_197956 | NAIF1 | 0.48 | 0.00 | 118 | 214 | 314 | 296 |
| NM_018946 | NANS | 0.20 | -0.33 | 2987 | 3623 | 3030 | 2264 |
| NM_005969 | NAP1L4 | 0.20 | -0.08 | 1768 | 2187 | 3635 | 3248 |
| NM_003827 | NAPA | 0.49 | -0.07 | 1716 | 2597 | 4852 | 4392 |
| NM_022493 | NARFL | 0.65 | 0.00 | 277 | 516 | 821 | 800 |
| NM_001256281 | NARR | 0.34 | -0.05 | 290 | 437 | 757 | 647 |
| NM_024678 | NARS2 | 0.27 | -0.35 | 250 | 365 | 403 | 264 |
| NM_002482 | NASP | 0.35 | -0.29 | 578 | 835 | 564 | 396 |
| NM_001160170 | NAT1 | 0.18 | 0.00 | 39 | 70 | 150 | 161 |
| NM_024662 | NAT10 | 0.24 | -0.03 | 1273 | 1642 | 1716 | 1545 |
| NM_001200016 | NAT6 | 0.40 | 0.00 | 154 | 258 | 345 | 294 |
| NM_015654 | NAT9 | 0.02 | 0.00 | 533 | 621 | 982 | 1055 |
| NM_014865 | NCAPD2 | 0.33 | -0.50 | 1673 | 2269 | 2612 | 1717 |
| NM_015261 | NCAPD3 | 0.05 | -0.17 | 1334 | 1506 | 1429 | 1161 |
| NM_022346 | NCAPG | 0.13 | -0.86 | 696 | 855 | 846 | 405 |
| NM_001281932 | NCAPG2 | 0.04 | -0.72 | 1148 | 1294 | 1323 | 721 |
| NM_001185011 | NCAPH2 | 0.17 | 0.00 | 1459 | 1783 | 2473 | 2333 |
| NM_002486 | NCBP1 | 0.10 | -0.23 | 681 | 826 | 1680 | 1312 |
| NM_007362 | NCBP2 | 0.22 | 0.00 | 936 | 1207 | 1689 | 1828 |
| NM_001146276 | NCEH1 | 0.04 | 0.00 | 2579 | 2823 | 2691 | 2715 |
| NM_003581 | NCK2 | 0.22 | 0.00 | 533 | 708 | 857 | 906 |
| NM_001145260 | NCOA4 | 0.45 | 0.01 | 2385 | 3458 | 6322 | 6617 |
| NM_020967 | NCOA5 | 0.32 | 0.00 | 571 | 809 | 855 | 902 |
| NM_014286 | NCS1 | 0.08 | -0.09 | 4139 | 4613 | 10953 | 9972 |
| NM_001143979 | NDE1 | 0.08 | 0.00 | 687 | 820 | 634 | 715 |
| NM_024574 | NDNF | 0.33 | 0.00 | 124 | 203 | 218 | 251 |
| NM_138704 | NDNL2 | 0.48 | 0.00 | 372 | 604 | 780 | 815 |
| NM_001242835 | NDRG4 | 1.38 | -0.11 | 24 | 105 | 162 | 113 |
| NM_004544 | NDUFA10 | 0.05 | -0.02 | 1363 | 1542 | 2968 | 2749 |
| NM_014222 | NDUFA8 | 0.25 | -0.01 | 827 | 1095 | 2046 | 1893 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_005002 | NDUFA9 | 0.25 | 0.00 | 416 | 575 | 1083 | 980 |
| NM_005003 | NDUFAB1 | 0.03 | -0.25 | 564 | 658 | 1017 | 765 |
| NM_174889 | NDUFAF2 | 0.24 | 0.00 | 184 | 272 | 507 | 451 |
| NM_199069 | NDUFAF3 | 0.13 | 0.00 | 919 | 1114 | 2541 | 2586 |
| NM_004545 | NDUFB1 | 0.03 | 0.00 | 121 | 163 | 306 | 305 |
| NM_004546 | NDUFB2 | 0.55 | 0.00 | 556 | 920 | 1996 | 1998 |
| NM_004146 | NDUFB7 | 0.23 | -0.11 | 824 | 1075 | 2019 | 1732 |
| NM_005006 | NDUFS1 | 0.14 | 0.00 | 1635 | 1949 | 3005 | 3163 |
| NM_001184979 | NDUFS5 | 0.34 | 0.00 | 500 | 726 | 1398 | 1407 |
| NM_024407 | NDUFS7 | 0.15 | 0.00 | 478 | 611 | 794 | 834 |
| NM_002496 | NDUFS8 | 0.20 | -0.33 | 1361 | 1705 | 3783 | 2848 |
| NM_022351 | NECAB 1 | 0.50 | 0.00 | 64 | 129 | 112 | 100 |
| NM_015509 | NECAP1 | 0.11 | 0.00 | 472 | 588 | 817 | 747 |
| NM_018090 | NECAP2 | 0.29 | 0.00 | 2176 | 2849 | 3839 | 3630 |
| NM_001144966 | NEDD4L | 0.41 | -0.14 | 517 | 782 | 455 | 349 |
| NM_006156 | NEDD8 | 0.18 | -0.17 | 977 | 1224 | 2460 | 2039 |
| NM_001199823 | NEDD8-MDP1 | 0.46 | 0.00 | 272 | 447 | 591 | 579 |
| NM_006403 | NEDD9 | 0.15 | -0.01 | 2617 | 3093 | 2123 | 1956 |
| NM_001199397 | NEK1 | 0.01 | 0.00 | 326 | 391 | 742 | 789 |
| NM_003157 | NEK4 | 0.33 | 0.00 | 241 | 367 | 562 | 547 |
| NM_001166171 | NEK6 | 0.23 | -0.38 | 1956 | 2469 | 6370 | 4682 |
| NM_033116 | NEK9 | 0.29 | 0.00 | 993 | 1337 | 3232 | 3373 |
| NM_005663 | NELFA | 0.04 | 0.00 | 412 | 495 | 410 | 460 |
| NM_002904 | NELFE | 0.16 | 0.00 | 1154 | 1416 | 3278 | 3094 |
| RefSeq ID | Gene Symbol | SR GFOLD | SEN GFOLD | SR IL-2 - | SR IL-2 + | SEN IL-2 - | SEN IL-2 + |
| NM_001201477 | NETO2 | 1.15 | 0.00 | 53 | 169 | 220 | 216 |
| NM_006656 | NEU3 | 0.24 | 0.00 | 112 | 175 | 250 | 270 |
| NM_001142651 | NEURL1B | 0.02 | -0.96 | 115 | 155 | 116 | 39 |
| NM_181832 | NF2 | 0.01 | -0.15 | 6567 | 6905 | 7713 | 6671 |
| NM_001278669 | NFATC1 | 0.22 | 0.00 | 123 | 188 | 343 | 384 |
| NM_032815 | NFATC2IP | 0.03 | 0.00 | 665 | 769 | 1040 | 1037 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_020529 | NFKBIA | 0.09 | 0.00 | 770 | 918 | 769 | 689 |
| NM_004556 | NFKBIE | 0.36 | 0.00 | 81 | 143 | 139 | 148 |
| NM_001142588 | NFYC | 0.30 | 0.00 | 528 | 743 | 848 | 828 |
| NM_002507 | NGFR | 0.49 | 0.00 | 7 | 31 | 81 | 111 |
| NM_206915 | NGFRAP1 | 0.06 | -0.04 | 1257 | 1438 | 4527 | 4177 |
| NM_024782 | NHEJ1 | 0.22 | 0.00 | 185 | 269 | 322 | 344 |
| NM_005008 | NHP2L1 | 0.21 | 0.00 | 1447 | 1818 | 2512 | 2490 |
| NM_001144060 | NHSL1 | 0.06 | 0.00 | 781 | 909 | 1940 | 1887 |
| NM_002508 | NIDI | 0.10 | 0.00 | 4300 | 4853 | 3452 | 3427 |
| NM_021824 | NIF3L1 | 0.37 | 0.00 | 291 | 448 | 426 | 484 |
| NM_004148 | NINJ1 | 0.06 | 0.00 | 299 | 373 | 957 | 902 |
| NM_025176 | NINL | 0.02 | 0.00 | 170 | 218 | 306 | 349 |
| NM_003634 | NIPSNAP1 | 0.09 | -0.11 | 517 | 631 | 1435 | 1214 |
| NM_020202 | NIT2 | 0.06 | 0.00 | 341 | 420 | 928 | 937 |
| NM_001144929 | NKIRAS2 | 0.19 | 0.00 | 1236 | 1539 | 1863 | 1996 |
| NM_001014445 | NLE1 | 0.58 | -0.17 | 275 | 489 | 611 | 471 |
| NM_020726 | NLN | 0.02 | 0.00 | 921 | 1041 | 1649 | 1654 |
| NM_032206 | NLRC5 | 0.09 | 0.00 | 745 | 893 | 2034 | 1959 |
| NM_021077 | NMB | 0.23 | -0.14 | 79 | 129 | 3752 | 3216 |
| NM_002512 | NME2 | 0.38 | -0.21 | 2409 | 3329 | 5715 | 4713 |
| NM_001286438 | NME4 | 0.16 | -0.21 | 1706 | 2056 | 4852 | 3985 |
| NM_005793 | NME6 | 0.03 | 0.00 | 292 | 358 | 354 | 370 |
| NM_020677 | NMRAL1 | 0.68 | 0.00 | 388 | 718 | 1133 | 1028 |
| NM_021079 | NMT1 | 0.21 | 0.00 | 3401 | 4163 | 7004 | 7061 |
| NM_005386 | NNAT | 0.40 | 0.00 | 25 | 57 | 56 | 37 |
| NM_006169 | NNMT | 0.47 | 0.00 | 1726 | 2574 | 3814 | 3706 |
| NM_032313 | NOA1 | 0.43 | 0.00 | 250 | 406 | 724 | 662 |
| NM_014062 | NOB1 | 0.47 | 0.00 | 680 | 1058 | 1244 | 1353 |
| NM_015658 | NOC2L | 0.47 | -0.16 | 1143 | 1730 | 2408 | 2006 |
| NM_015462 | NOL11 | 0.53 | -0.20 | 205 | 363 | 416 | 305 |
| NM_022917 | NOL6 | 0.19 | -0.40 | 1820 | 2227 | 2803 | 1985 |
| NM_001284388 | NOLC1 | 0.31 | 0.00 | 2062 | 2743 | 2090 | 2120 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001145408 | NONO | 0.44 | -0.12 | 2593 | 3736 | 5299 | 4662 |
| RefSeq ID | Gene Symbol | SR GFOLD | SEN GFOLD | SR IL-2 - | SR IL-2 + | SEN IL-2 - | SEN IL-2 + |
| NM_016391 | NOP16 | 0.04 | -0.29 | 713 | 826 | 1179 | 869 |
| NM_001033714 | NOP2 | 0.04 | -0.01 | 1240 | 1396 | 1318 | 1194 |
| NM_174913 | NOP9 | 0.16 | 0.00 | 1572 | 1904 | 2693 | 2741 |
| NM_000603 | NOS3 | 0.19 | 0.00 | 48 | 83 | 137 | 142 |
| NM_002514 | NOV | 0.43 | -0.19 | 457 | 710 | 216 | 148 |
| NM_001291926 | NOX4 | 0.57 | -0.12 | 22 | 58 | 166 | 115 |
| NM_002519 | NPAT | 0.29 | 0.00 | 365 | 522 | 568 | 540 |
| NM_017921 | NPLOC4 | 0.22 | 0.00 | 4577 | 5567 | 7996 | 8283 |
| NM_006993 | NPM3 | 0.26 | -0.47 | 270 | 388 | 730 | 458 |
| NM_000903 | NQO1 | 0.63 | -0.08 | 5148 | 8328 | 15747 | 14464 |
| NM_001290221 | NQO2 | 0.03 | 0.00 | 558 | 651 | 992 | 1098 |
| NM_007121 | NR1H2 | 0.10 | 0.00 | 1800 | 2086 | 3448 | 3347 |
| NM_021005 | NR2F2 | 0.13 | -0.14 | 2093 | 2448 | 3047 | 2590 |
| NM_005234 | NR2F6 | 0.42 | 0.00 | 313 | 494 | 753 | 747 |
| NM_000901 | NR3C2 | 0.50 | -0.15 | 612 | 974 | 1996 | 1664 |
| NM_198465 | NRK | 0.27 | -0.46 | 98 | 159 | 385 | 231 |
| NM_024958 | NRSN2 | 0.43 | 0.00 | 575 | 877 | 2683 | 2684 |
| NM_014886 | NSA2 | 0.02 | 0.00 | 319 | 385 | 595 | 556 |
| NM_022455 | NSD1 | 0.22 | -0.01 | 3201 | 3934 | 4633 | 4390 |
| NM_001206736 | NSFL1C | 0.15 | 0.00 | 1194 | 1452 | 2562 | 2599 |
| NM_001287763 | NSG1 | 1.10 | 0.00 | 19 | 73 | 75 | 83 |
| NM_001042549 | NSL1 | 0.24 | 0.00 | 238 | 341 | 534 | 545 |
| NM_145080 | NSMCE1 | 0.49 | 0.00 | 378 | 619 | 1589 | 1469 |
| NM_173685 | NSMCE2 | 0.39 | 0.00 | 153 | 254 | 354 | 375 |
| NM_001199104 | NT5C1B-RDH14 | 0.13 | 0.00 | 241 | 321 | 516 | 545 |
| NM_012229 | NT5C2 | 0.11 | 0.00 | 997 | 1191 | 1722 | 1824 |
| NM_006179 | NTF4 | 0.11 | 0.00 | 34 | 60 | 54 | 44 |
| NM_002528 | NTHL1 | 0.41 | 0.00 | 108 | 190 | 337 | 368 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001286798 | NTMT1 | 0.18 | -0.05 | 1671 | 2042 | 2113 | 1893 |
| NM_030952 | NUAK2 | 0.14 | -0.90 | 477 | 607 | 443 | 194 |
| NM_002484 | NUBP1 | 0.14 | 0.00 | 302 | 397 | 476 | 438 |
| NM_025152 | NUBPL | 0.43 | -0.25 | 42 | 87 | 239 | 159 |
| NM_022731 | NUCKS1 | 0.02 | 0.00 | 4521 | 4815 | 6599 | 6800 |
| NM_006600 | NUDC | 0.49 | 0.00 | 502 | 803 | 1079 | 1024 |
| NM_032869 | NUDCD1 | 0.10 | 0.00 | 636 | 772 | 570 | 508 |
| NM_198948 | NUDT1 | 0.28 | 0.00 | 279 | 407 | 466 | 399 |
| NM_031438 | NUDT12 | 0.70 | 0.00 | 58 | 135 | 247 | 226 |
| NM_001105570 | NUDT19 | 0.17 | 0.00 | 291 | 393 | 447 | 410 |
| NM_001161 | NUDT2 | 0.44 | 0.00 | 196 | 328 | 641 | 662 |
| RefSeq ID | Gene Symbol | SR GFOLD | SEN GFOLD | SR IL-2 - | SR IL-2 + | SEN IL-2 - | SEN IL-2 + |
| NM_145697 | NUF2 | 0.19 | -0.84 | 215 | 302 | 254 | 109 |
| NM_012345 | NUFIP1 | 0.02 | 0.00 | 67 | 98 | 139 | 166 |
| NM_018230 | NUP133 | 0.65 | -0.07 | 508 | 904 | 1275 | 1105 |
| NM_004298 | NUP155 | 0.16 | -0.18 | 879 | 1091 | 1433 | 1152 |
| NM_015231 | NUP160 | 0.16 | 0.00 | 773 | 964 | 1025 | 1085 |
| NM_015354 | NUP188 | 0.29 | -0.27 | 3261 | 4208 | 5817 | 4597 |
| NM_015135 | NUP205 | 0.27 | 0.00 | 1503 | 1956 | 2365 | 2251 |
| NM_198887 | NUP43 | 0.28 | 0.00 | 541 | 750 | 973 | 1076 |
| NM_007172 | NUP50 | 0.21 | 0.00 | 766 | 988 | 1381 | 1383 |
| NM_017426 | NUP54 | 0.41 | 0.00 | 436 | 670 | 680 | 750 |
| NM_024844 | NUP85 | 0.63 | -0.12 | 635 | 1103 | 1144 | 950 |
| NM_014669 | NUP93 | 0.37 | -0.06 | 1342 | 1885 | 2206 | 1965 |
| NM_016320 | NUP98 | 0.18 | 0.00 | 3468 | 4143 | 5586 | 5358 |
| NM_001045477 | NUTM2G | 1.25 | 0.00 | 8 | 54 | 60 | 44 |
| NM_002533 | NVL | 0.26 | 0.00 | 266 | 383 | 566 | 529 |
| NM_022463 | NXN | 0.03 | -0.02 | 1270 | 1418 | 1400 | 1266 |
| NM_013248 | NXT1 | 0.36 | -0.01 | 262 | 404 | 406 | 340 |
| NM_178507 | OAF | 0.10 | -0.13 | 2203 | 2531 | 4962 | 4310 |
| NM_006187 | OAS3 | 0.01 | -0.27 | 144 | 188 | 162 | 100 |
| NM_000274 | OAT | 0.02 | -0.20 | 2509 | 2712 | 4908 | 4072 |
| NM_002540 | ODF2 | 0.36 | -0.03 | 1086 | 1529 | 2003 | 1815 |
| NM_003611 | OFD1 | 0.21 | 0.00 | 150 | 221 | 270 | 322 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_002541 | OGDH | 0.14 | 0.00 | 5119 | 5883 | 9822 | 9516 |
| NM_007346 | OGFR | 0.13 | 0.00 | 377 | 483 | 772 | 738 |
| NM_024576 | OGFRL1 | 0.32 | 0.00 | 463 | 665 | 674 | 752 |
| NM_016828 | OGG1 | 0.02 | 0.00 | 293 | 357 | 796 | 706 |
| NM_001011708 | OLA1 | 0.32 | 0.00 | 189 | 293 | 566 | 501 |
| NM_015441 | OLFML2B | 0.05 | -0.73 | 299 | 372 | 772 | 405 |
| NM_001286352 | OLFML3 | 0.06 | -0.13 | 1102 | 1261 | 3444 | 2969 |
| NM_130837 | OPA1 | 0.24 | 0.00 | 962 | 1253 | 1938 | 1798 |
| NM_182647 | OPRL1 | 0.13 | 0.00 | 7 | 25 | 94 | 65 |
| NM_001004462 | OR10G4 | 0.80 | 0.00 | 41 | 109 | 131 | 128 |
| NM_001190818 | ORC1 | 0.49 | 0.00 | 337 | 555 | 154 | 120 |
| NM_012381 | ORC3 | 0.06 | -0.26 | 167 | 221 | 501 | 355 |
| NM_016467 | ORMDL1 | 0.34 | 0.00 | 348 | 516 | 624 | 671 |
| NM_001201480 | OSBPL6 | 0.24 | 0.00 | 394 | 544 | 622 | 636 |
| NM_145798 | OSBPL7 | 0.07 | 0.00 | 85 | 124 | 164 | 152 |
| NM_017807 | OSGEP | 0.26 | -0.36 | 263 | 380 | 732 | 497 |
| NM_182981 | OSGIN1 | 0.04 | 0.00 | 681 | 791 | 416 | 455 |
| NM_005015 | OXA1L | 0.03 | 0.00 | 1703 | 1880 | 3433 | 3516 |
| NM_000436 | OXCT1 | 0.16 | -0.17 | 473 | 609 | 1169 | 939 |
| NM_001198533 | OXR1 | 0.27 | 0.00 | 404 | 568 | 1279 | 1231 |
| NM_000916 | OXTR | 0.07 | -1.20 | 13965 | 15094 | 10394 | 4346 |
| NM_006191 | PA2G4 | 0.35 | -0.50 | 1583 | 2177 | 2375 | 1555 |
| NM_001267803 | PAAF1 | 0.76 | 0.00 | 55 | 135 | 327 | 316 |
| NM_004643 | PABPN1 | 0.14 | 0.00 | 957 | 1169 | 1547 | 1508 |
| NM_001100913 | PACS2 | 0.13 | 0.00 | 1191 | 1429 | 3727 | 3673 |
| NM_019088 | PAF1 | 0.21 | -0.05 | 756 | 977 | 1368 | 1209 |
| NM_000430 | PAFAH1B1 | 0.37 | 0.00 | 2101 | 2902 | 4274 | 4286 |
| NM_002573 | PAFAH1B3 | 0.07 | -0.08 | 314 | 392 | 830 | 697 |
| NM_006451 | PAIP1 | 0.54 | 0.00 | 218 | 386 | 458 | 488 |
| NM_001128620 | PAK1 | 0.26 | 0.00 | 1024 | 1347 | 2668 | 2845 |
| NM_002577 | PAK2 | 0.33 | 0.00 | 1666 | 2255 | 2641 | 2763 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_007203 | PALM2-AKAP2 | 0.22 | -0.15 | 8867 | 10707 | 24263 | 21369 |
| NM_016069 | PAM16 | 0.04 | 0.00 | 197 | 253 | 399 | 373 |
| NM_148977 | PANK1 | 0.19 | 0.00 | 77 | 123 | 121 | 137 |
| NM_024594 | PANK3 | 0.05 | 0.00 | 472 | 564 | 811 | 860 |
| NM_152911 | PAOX | 0.39 | 0.00 | 52 | 101 | 127 | 139 |
| NM_001040284 | PAPD5 | 0.29 | 0.00 | 445 | 628 | 589 | 608 |
| NM_173462 | PAPLN | 0.99 | 0.00 | 11 | 51 | 160 | 146 |
| NM_022894 | PAPOLG | 0.08 | 0.01 | 276 | 352 | 406 | 477 |
| NM_002581 | PAPPA | 0.09 | 0.00 | 1798 | 2060 | 10906 | 10872 |
| NM_133367 | PAQR8 | 0.17 | 0.00 | 26 | 51 | 143 | 163 |
| NM_198504 | PAQR9 | 1.49 | 0.00 | 18 | 91 | 29 | 46 |
| NM_001184785 | PARD3 | 0.66 | 0.00 | 336 | 620 | 1291 | 1364 |
| NM_018622 | PARL | 0.27 | 0.00 | 146 | 225 | 458 | 477 |
| NM_001618 | PARP1 | 0.24 | -0.36 | 1308 | 1681 | 1682 | 1201 |
| NM_022750 | PARP12 | 0.34 | 0.00 | 1094 | 1523 | 1023 | 970 |
| NM_017554 | PARP14 | 0.07 | 0.00 | 624 | 745 | 828 | 847 |
| NM_001042618 | PARP2 | 0.32 | 0.00 | 372 | 542 | 487 | 555 |
| NM_006437 | PARP4 | 0.21 | 0.00 | 1502 | 1882 | 3766 | 3954 |
| NM_031458 | PARP9 | 0.46 | -0.14 | 146 | 256 | 520 | 405 |
| NM_001252120 | PASK | 0.19 | 0.00 | 182 | 259 | 318 | 351 |
| NM_152716 | PATL1 | 0.19 | 0.00 | 1136 | 1424 | 1379 | 1325 |
| NM_006195 | PBX3 | 0.40 | 0.00 | 716 | 1058 | 1751 | 1660 |
| NM_001040716 | PC | 0.31 | 0.00 | 608 | 850 | 1231 | 1325 |
| NM_000532 | PCCB | 0.31 | 0.00 | 445 | 636 | 950 | 978 |
| NM_019035 | PCDH18 | 0.49 | -0.39 | 1973 | 2962 | 6738 | 4915 |
| NM_001173523 | PCDH7 | 0.10 | 0.00 | 265 | 344 | 374 | 347 |
| NM_022760 | PCED1A | 0.21 | 0.00 | 276 | 384 | 566 | 593 |
| NM_002592 | PCNA | 0.40 | -0.21 | 1467 | 2090 | 1543 | 1222 |
| NM_032223 | PCNXL3 | 0.15 | 0.00 | 2228 | 2651 | 3870 | 3835 |
| NM_002593 | PCOLCE | 0.10 | -0.23 | 1538 | 1782 | 3084 | 2462 |
| NM_004716 | PCSK7 | 0.40 | -0.21 | 2203 | 3109 | 4952 | 4072 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001184917 | PCYT2 | 0.05 | -0.05 | 448 | 538 | 1678 | 1492 |
| NM_001199462 | PDCD2 | 0.23 | 0.00 | 243 | 345 | 499 | 458 |
| NM_032346 | PDCD2L | 0.30 | 0.00 | 88 | 148 | 125 | 120 |
| NM_182612 | PDDC1 | 0.06 | 0.00 | 877 | 1015 | 2458 | 2384 |
| NM_177966 | PDE12 | 0.37 | 0.00 | 753 | 1085 | 1229 | 1172 |
| NM_000921 | PDE3A | 0.09 | 0.00 | 664 | 796 | 796 | 741 |
| NM_001111308 | PDE4A | 0.31 | 0.00 | 476 | 678 | 1077 | 1161 |
| NM_002607 | PDGFA | 0.32 | -0.04 | 612 | 866 | 790 | 682 |
| NM_006206 | PDGFRA | 0.15 | -0.63 | 3877 | 4533 | 5085 | 3109 |
| NM_001173454 | PDHA1 | 0.18 | -0.03 | 1069 | 1328 | 1770 | 1604 |
| NM_006457 | PDLIM5 | 0.06 | 0.00 | 7107 | 7674 | 8695 | 8976 |
| NM_001161779 | PDP1 | 0.30 | 0.00 | 839 | 1145 | 1845 | 1808 |
| NM_006474 | PDPN | 0.11 | 0.00 | 97 | 142 | 40 | 55 |
| NM_014317 | PDSS1 | 0.17 | 0.00 | 24 | 48 | 77 | 76 |
| NM_020381 | PDSS2 | 0.27 | 0.00 | 330 | 470 | 836 | 898 |
| NM_016484 | PDZD 11 | 0.56 | 0.00 | 203 | 367 | 928 | 994 |
| NM_001080471 | PEAR1 | 0.21 | -1.16 | 1926 | 2387 | 510 | 187 |
| NM_001040152 | PEG10 | 0.14 | -0.32 | 1116 | 1351 | 616 | 425 |
| NM_001135690 | PENK | 0.60 | -1.15 | 4042 | 6409 | 3625 | 1525 |
| NM_000285 | PEPD | 0.42 | -0.23 | 1234 | 1795 | 2797 | 2238 |
| NM_001243225 | PES1 | 0.05 | 0.00 | 1792 | 2005 | 2469 | 2368 |
| NM_001164811 | PET117 | 0.25 | 0.00 | 212 | 310 | 347 | 383 |
| NM_000466 | PEX1 | 0.18 | 0.00 | 173 | 247 | 430 | 457 |
| NM_003846 | PEX11B | 0.19 | 0.00 | 249 | 344 | 794 | 887 |
| NM_004565 | PEX14 | 0.29 | 0.00 | 240 | 356 | 414 | 451 |
| NM_000318 | PEX2 | 0.75 | 0.00 | 254 | 509 | 630 | 643 |
| NM_012393 | PFAS | 0.36 | -0.47 | 716 | 1026 | 1198 | 778 |
| NM_002622 | PFDN1 | 0.68 | -0.15 | 484 | 884 | 1651 | 1366 |
| NM_053024 | PFN2 | 0.13 | 0.00 | 2363 | 2751 | 5906 | 5865 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_ 001170543 | PGAM5 | 0.28 | 0.00 | 1065 | 1425 | 1373 | 1312 |
| NM_033419 | PGAP3 | 0.10 | -0.08 | 118 | 167 | 528 | 431 |
| NM_152595 | PGBD4 | 0.52 | 0.00 | 24 | 60 | 56 | 59 |
| NM_002631 | PGD | 0.33 | 0.00 | 985 | 1367 | 4920 | 4845 |

**Table 5A: Differentially Upregulated Genes in SR +IL-2**

| RefSeq ID | Gene Symbol | SR GFOLD | SEN GFOLD | SR IL-2- | SR IL-2 + | SEN IL- 2 - | SEN IL-2 + |
|---|---|---|---|---|---|---|---|
| NM_005023 | PGGT1B | 0.37 | 0.00 | 95 | 165 | 229 | 237 |
| NM_012088 | PGLS | 0.17 | 0.00 | 983 | 1222 | 2464 | 2445 |
| NM_018290 | PGM2 | 0.02 | 0.00 | 928 | 1045 | 1840 | 1872 |
| NM_024419 | PGS1 | 0.06 | 0.00 | 473 | 570 | 1023 | 1041 |
| NM_ 001048183 | PHACTR4 | 0.08 | -0.06 | 620 | 743 | 859 | 732 |
| NM_ 001281496 | PHB | 0.05 | -0.21 | 1356 | 1529 | 2793 | 2257 |
| NM_ 001144831 | PHB2 | 0.13 | -0.25 | 2884 | 3352 | 5103 | 4100 |
| NM_004426 | PHC1 | 0.11 | 0.00 | 485 | 604 | 948 | 874 |
| NM_015651 | PHF19 | 0.33 | -0.03 | 1010 | 1397 | 1291 | 1152 |
| NM_005392 | PHF2 | 0.18 | -0.02 | 1074 | 1334 | 1778 | 1614 |
| NM_032458 | PHF6 | 0.10 | 0.00 | 461 | 572 | 713 | 660 |
| NM_000292 | PHKA2 | 0.25 | 0.00 | 439 | 604 | 834 | 835 |
| NM_000294 | PHKG2 | 0.41 | 0.00 | 410 | 630 | 803 | 738 |
| NM_ 001134438 | PHLDB2 | 0.31 | -0.13 | 783 | 1079 | 2263 | 1928 |
| NM_015020 | PHLPP2 | 0.07 | 0.00 | 679 | 807 | 873 | 970 |
| NM_006214 | PHYH | 0.40 | -0.01 | 251 | 398 | 1019 | 913 |
| NM_ 001100876 | PHYHD1 | 0.48 | -0.50 | 39 | 85 | 239 | 131 |
| NM_018323 | PI4K2B | 0.02 | 0.00 | 359 | 429 | 643 | 617 |
| NM_058004 | PI4KA | 0.17 | -0.08 | 2111 | 2551 | 6416 | 5828 |
| NM_080476 | PIGU | 0.39 | -0.13 | 231 | 367 | 580 | 460 |
| NM_ 001042616 | PIGY | 0.09 | 0.00 | 555 | 675 | 834 | 896 |
| NM_014602 | PIK3R4 | 0.26 | 0.00 | 627 | 851 | 1163 | 1131 |
| NM_ 001193621 | PINLYP | 0.07 | -0.24 | 38 | 64 | 89 | 50 |
| NM_017884 | PINX1 | 0.17 | 0.00 | 251 | 343 | 314 | 285 |
| NM_005028 | PIP4K2A | 0.24 | 0.00 | 1236 | 1599 | 2597 | 2445 |

(continued)

| Table 5A: Differentially Upregulated Genes in SR +IL-2 | | | | | | | |
|---|---|---|---|---|---|---|---|
| RefSeq ID | Gene Symbol | SR GFOLD | SEN GFOLD | SR IL-2- | SR IL-2 + | SEN IL- 2 - | SEN IL-2 + |
| NM_001135638 | PIP5K1A | 0.31 | -0.13 | 1294 | 1751 | 1531 | 1277 |
| NM_014338 | PISD | 0.10 | -0.13 | 728 | 875 | 1321 | 1100 |
| NM_001284278 | PITPNB | 0.07 | 0.00 | 1291 | 1484 | 1913 | 1989 |
| NM_138370 | PKDCC | 0.13 | 0.00 | 207 | 281 | 183 | 179 |
| NM_181805 | PKIG | 0.25 | -0.17 | 599 | 809 | 1275 | 1028 |
| NM_007069 | PLA2G16 | 0.38 | 0.00 | 155 | 255 | 275 | 327 |
| NM_003560 | PLA2G6 | 0.69 | 0.00 | 31 | 81 | 175 | 194 |
| NM_001012973 | PLAC9 | 0.93 | 0.00 | 128 | 310 | 277 | 274 |
| NM_000932 | PLCB3 | 0.18 | 0.00 | 1655 | 2030 | 3302 | 3353 |
| NM_001243108 | PLD2 | 0.15 | 0.00 | 428 | 551 | 1281 | 1207 |
| NM_024310 | PLEKHF1 | 0.42 | 0.00 | 87 | 159 | 208 | 233 |
| NM_024613 | PLEKHF2 | 0.56 | 0.00 | 78 | 158 | 191 | 233 |
| NM_022835 | PLEKHG2 | 0.46 | -0.51 | 431 | 683 | 1470 | 939 |
| NM_015432 | PLEKHG4 | 0.22 | -0.41 | 528 | 704 | 1414 | 963 |
| RefSeq ID | Gene Symbol | SR GFOLD | SEN GFOLD | SR IL-2- | SR IL-2 + | SEN IL-2- | SEN IL-2+ |
| NM_172069 | PLEKHH2 | 0.51 | -0.08 | 48 | 102 | 364 | 287 |
| NM_018049 | PLEKHJ1 | 0.06 | 0.00 | 502 | 602 | 838 | 791 |
| NM_015164 | PLEKHM2 | 0.18 | 0.00 | 5242 | 6193 | 9103 | 9425 |
| NM_016274 | PLEKHO1 | 0.12 | 0.00 | 1454 | 1717 | 2708 | 2769 |
| NM_025201 | PLEKHO2 | 0.14 | 0.00 | 1008 | 1228 | 2427 | 2521 |
| NM_001164189 | PLIN3 | 0.14 | -0.04 | 7305 | 8341 | 16793 | 15924 |
| NM_005030 | PLK1 | 0.39 | -1.00 | 957 | 1382 | 1177 | 521 |
| NM_002669 | PLRG1 | 0.10 | 0.00 | 1048 | 1238 | 2086 | 2128 |
| NM_021105 | PLSCR1 | 0.32 | 0.00 | 208 | 320 | 381 | 410 |
| NM_021127 | PMAIP1 | 0.43 | -0.26 | 200 | 332 | 204 | 131 |
| NM_001199653 | PMF1 | 0.01 | -0.24 | 412 | 486 | 934 | 702 |
| NM_033238 | PML | 0.16 | 0.00 | 1114 | 1365 | 2236 | 2377 |
| NM_002676 | PMM1 | 0.43 | -0.06 | 732 | 1104 | 2978 | 2688 |
| NM_000303 | PMM2 | 0.13 | 0.00 | 1167 | 1403 | 1599 | 1490 |
| NM_001281455 | PMP22 | 0.20 | 0.00 | 1867 | 2309 | 3192 | 3152 |

(continued)

| RefSeq ID | Gene Symbol | SR GFOLD | SEN GFOLD | SR IL-2- | SR IL-2 + | SEN IL-2- | SEN IL-2+ |
|---|---|---|---|---|---|---|---|
| NM_001282946 | PMPCA | 0.03 | -0.08 | 1069 | 1207 | 2352 | 2079 |
| NM_000535 | PMS2 | 0.29 | 0.00 | 191 | 290 | 516 | 462 |
| NM_006556 | PMVK | 0.03 | -0.08 | 370 | 444 | 973 | 824 |
| NM_001077399 | PNKD | 0.65 | -0.37 | 83 | 177 | 487 | 318 |
| NM_020143 | PNO1 | 0.18 | 0.00 | 293 | 396 | 410 | 445 |
| NM_018129 | PNPO | 0.13 | 0.00 | 344 | 444 | 1160 | 1055 |
| NM_001199781 | POC1B-GALNT4 | 0.11 | 0.00 | 255 | 334 | 566 | 610 |
| NM_001099271 | POC5 | 0.18 | 0.00 | 82 | 129 | 137 | 118 |
| NM_017542 | POGK | 0.06 | 0.00 | 1187 | 1355 | 1466 | 1349 |
| NM_002689 | POLA2 | 0.62 | -0.14 | 547 | 950 | 728 | 579 |
| NM_001256879 | POLD2 | 0.02 | 0.00 | 940 | 1058 | 2213 | 2079 |
| NM_015584 | POLDIP2 | 0.26 | -0.01 | 1667 | 2158 | 3858 | 3639 |
| NM_001278657 | POLDIP3 | 0.10 | 0.00 | 1431 | 1672 | 2531 | 2545 |
| NM_001278255 | POLE3 | 0.53 | -0.02 | 782 | 1257 | 1229 | 1100 |
| NM_006502 | POLH | 0.13 | -0.08 | 1379 | 1641 | 2244 | 1978 |
| NM_015425 | POLR1A | 0.18 | -0.12 | 1411 | 1739 | 3067 | 2660 |
| NM_004805 | POLR2D | 0.50 | 0.00 | 465 | 756 | 761 | 830 |
| NM_002696 | POLR2G | 0.43 | 0.00 | 547 | 836 | 1329 | 1338 |
| NM_006233 | POLR2I | 0.48 | 0.00 | 444 | 713 | 905 | 924 |
| NM_006234 | POLR2J | 0.07 | 0.00 | 493 | 598 | 919 | 830 |
| NM_021128 | POLR2L | 0.40 | -0.45 | 2046 | 2889 | 5698 | 3989 |
| NM_006467 | POLR3G | 0.21 | 0.00 | 63 | 105 | 135 | 135 |
| NM_032305 | POLR3GL | 0.90 | 0.00 | 61 | 161 | 320 | 342 |
| NM_001018050 | POLR3H | 0.31 | 0.00 | 1275 | 1725 | 3001 | 3030 |

**Table 5A: Differentially Upregulated Genes in SR+IL-2**

| RefSeq ID | Gene Symbol | SR GFOLD | SEN GFOLD | SR IL-2- | SR IL-2 + | SEN IL-2- | SEN IL-2 + |
|---|---|---|---|---|---|---|---|
| NM_005035 | POLRMT | 0.34 | 0.00 | 391 | 576 | 865 | 854 |
| NM_015932 | POMP | 0.10 | 0.00 | 1162 | 1366 | 2352 | 2516 |
| NM_013382 | POMT2 | 0.23 | 0.00 | 355 | 490 | 1264 | 1344 |
| NM_012230 | POMZP3 | 0.11 | 0.00 | 296 | 383 | 491 | 518 |
| NM_006627 | POP4 | 0.42 | 0.00 | 420 | 652 | 930 | 941 |

(continued)

| Table 5A: Differentially Upregulated Genes in SR+IL-2 | | | | | | | |
|---|---|---|---|---|---|---|---|
| RefSeq ID | Gene Symbol | SR GFOLD | SEN GFOLD | SR IL-2- | SR IL-2 + | SEN IL-2- | SEN IL-2 + |
| NM_000941 | POR | 0.19 | 0.00 | 1425 | 1763 | 3192 | 3340 |
| NM_001277406 | POTEI | 0.69 | 0.00 | 550 | 1006 | 478 | 551 |
| NM_021129 | PPA1 | 0.54 | 0.00 | 237 | 416 | 468 | 429 |
| NM_176869 | PPA2 | 0.71 | -0.22 | 182 | 368 | 1079 | 834 |
| NM_003713 | PPAP2B | 0.48 | -0.45 | 1703 | 2546 | 2327 | 1579 |
| NM_203453 | PPAPDC2 | 0.64 | -0.17 | 58 | 130 | 260 | 185 |
| NM_024299 | PPDPF | 0.22 | 0.00 | 2125 | 2641 | 4344 | 4429 |
| NM_006112 | PPIE | 0.35 | 0.00 | 567 | 819 | 1242 | 1270 |
| NM_005729 | PPIF | 0.01 | 0.00 | 3731 | 3965 | 2138 | 2155 |
| NM_006347 | PPIH | 0.39 | -0.19 | 84 | 151 | 252 | 176 |
| NM_014337 | PPIL2 | 0.17 | 0.00 | 806 | 1012 | 1504 | 1627 |
| NM_014634 | PPM1F | 0.23 | -0.11 | 1853 | 2335 | 3575 | 3139 |
| NM_177983 | PPM1G | 0.10 | 0.00 | 2059 | 2360 | 2441 | 2549 |
| NM_001122764 | PPOX | 0.16 | 0.00 | 169 | 239 | 534 | 530 |
| NM_002710 | PPP1CC | 0.28 | 0.00 | 1316 | 1734 | 1514 | 1457 |
| NM_002714 | PPP1R10 | 0.11 | 0.00 | 2033 | 2347 | 2456 | 2495 |
| NM_021959 | PPP1R11 | 0.30 | -0.01 | 676 | 936 | 2075 | 1913 |
| NM_002480 | PPP1R12A | 0.30 | 0.00 | 2181 | 2869 | 3635 | 3503 |
| NM_001271618 | PPP1R12C | 0.10 | 0.00 | 987 | 1170 | 1938 | 2024 |
| NM_138689 | PPP1R14B | 0.15 | -0.22 | 782 | 971 | 1585 | 1250 |
| NM_014811 | PPP1R26 | 0.22 | 0.00 | 344 | 471 | 809 | 847 |
| NM_145030 | PPP1R35 | 0.03 | -0.09 | 207 | 262 | 302 | 231 |
| NM_006242 | PPP1R3D | 0.16 | 0.00 | 239 | 325 | 256 | 229 |
| NM_001276318 | PPP1R3E | 0.29 | 0.00 | 52 | 95 | 114 | 131 |
| NM_014225 | PPP2R1A | 0.55 | 0.00 | 5745 | 8742 | 18057 | 17800 |
| NM_002717 | PPP2R2A | 0.14 | 0.00 | 652 | 810 | 880 | 969 |
| NM_002718 | PPP2R3A | 0.37 | 0.00 | 773 | 1112 | 1252 | 1285 |
| NM_013239 | PPP2R3B | 0.56 | 0.00 | 183 | 334 | 420 | 475 |
| NM_017917 | PPP2R3C | 0.10 | 0.00 | 270 | 349 | 624 | 686 |
| NM_178001 | PPP2R4 | 0.48 | -0.15 | 1780 | 2666 | 5669 | 4867 |
| NM_006247 | PPP5C | 0.10 | 0.00 | 1822 | 2107 | 2728 | 2553 |
| NM_001123355 | PPP6C | 0.15 | 0.00 | 621 | 780 | 986 | 956 |

(continued)

| Table 5A: Differentially Upregulated Genes in SR+IL-2 | | | | | | | |
|---|---|---|---|---|---|---|---|
| RefSeq ID | Gene Symbol | SR GFOLD | SEN GFOLD | SR IL-2- | SR IL-2 + | SEN IL-2- | SEN IL-2 + |
| NM_ 001242898 | PPP6R2 | 0.18 | 0.00 | 1142 | 1419 | 2548 | 2710 |
| NM_015062 | PPRC1 | 0.04 | 0.00 | 2162 | 2379 | 2498 | 2643 |
| Table 5A: Differentially Upregulated Genes in SR + IL-2 | | | | | | | |
| RefSeq ID | Gene Symbol | SR GFOLD | SENGFOLD | SR IL-2- | SR IL-2 + | SEN IL-2- | SEN IL-2 + |
| NM_000310 | PPT1 | 0.18 | -0.01 | 1067 | 1330 | 2017 | 1854 |
| NM_ 001278926 | PPWD1 | 0.16 | 0.00 | 276 | 371 | 497 | 560 |
| NM_025078 | PQLC1 | 0.14 | 0.00 | 859 | 1056 | 1370 | 1492 |
| NM_ 001040125 | PQLC2 | 0.02 | 0.00 | 619 | 715 | 867 | 896 |
| NM_007213 | PRAF2 | 0.05 | 0.00 | 885 | 1017 | 1940 | 1978 |
| NM_003981 | PRC1 | 0.03 | -0.98 | 2695 | 2934 | 2502 | 1170 |
| NM_199418 | PRCP | 0.04 | 0.00 | 948 | 1082 | 1759 | 1706 |
| NM_ 001282934 | PRDM15 | 0.10 | 0.01 | 344 | 437 | 489 | 567 |
| NM_006793 | PRDX3 | 0.02 | 0.00 | 1190 | 1325 | 2662 | 2573 |
| NM_013388 | PREB | 0.30 | 0.00 | 1521 | 2030 | 1895 | 1824 |
| NM_013237 | PRELID1 | 0.49 | -0.06 | 1259 | 1927 | 3151 | 2850 |
| NM_006150 | PRICKLE3 | 0.07 | 0.00 | 168 | 224 | 258 | 224 |
| NM_000946 | PRIM1 | 0.04 | 0.00 | 106 | 146 | 58 | 50 |
| NM_000947 | PRIM2 | 0.37 | -0.14 | 362 | 549 | 460 | 355 |
| NM_206907 | PRKAA1 | 0.07 | -0.02 | 994 | 1156 | 2136 | 1957 |
| NM_002732 | PRKACG | 0.14 | 0.00 | 133 | 190 | 381 | 444 |
| NM_ 001206710 | PRKAG1 | 0.06 | 0.00 | 1255 | 1432 | 2350 | 2216 |
| NM_ 001040633 | PRKAG2 | 0.10 | 0.00 | 679 | 819 | 967 | 991 |
| NM_ 001164760 | PRKAR1B | 0.04 | 0.00 | 204 | 260 | 397 | 377 |
| NM_145040 | PRKCDBP | 0.05 | -0.27 | 975 | 1117 | 2261 | 1743 |
| NM_005400 | PRKCE | 0.07 | 0.00 | 316 | 396 | 534 | 527 |
| NM_006255 | PRKCH | 0.91 | 0.00 | 13 | 51 | 85 | 78 |
| NM_002742 | PRKD1 | 0.04 | 0.00 | 1354 | 1515 | 2042 | 2061 |
| NM_006904 | PRKDC | 0.05 | -0.04 | 6452 | 6943 | 13025 | 12318 |
| NM_005788 | PRMT3 | 0.09 | 0.00 | 197 | 261 | 354 | 311 |
| NM_ 001039619 | PRMT5 | 0.31 | 0.00 | 1286 | 1740 | 1913 | 1815 |

(continued)

| Table 5A: Differentially Upregulated Genes in SR + IL-2 | | | | | | | |
|---|---|---|---|---|---|---|---|
| RefSeq ID | Gene Symbol | SR GFOLD | SENGFOLD | SR IL-2- | SR IL-2 + | SEN IL-2- | SEN IL-2 + |
| NM_018137 | PRMT6 | 0.12 | 0.00 | 230 | 306 | 302 | 279 |
| NM_153256 | PROSER2 | 0.32 | 0.00 | 186 | 288 | 141 | 172 |
| NM_014502 | PRPF19 | 0.02 | -0.04 | 2027 | 2207 | 2051 | 1858 |
| NM_015629 | PRPF31 | 0.21 | 0.00 | 982 | 1256 | 1680 | 1632 |
| NM_017922 | PRPF39 | 0.18 | 0.00 | 255 | 349 | 356 | 388 |
| NM_001244926 | PRPF4 | 0.21 | 0.00 | 903 | 1155 | 1605 | 1510 |
| NM_017892 | PRPF40A | 0.09 | 0.00 | 1577 | 1813 | 2525 | 2638 |
| NM_006445 | PRPF8 | 0.32 | 0.00 | 10139 | 13025 | 18796 | 19088 |
| NM_002764 | PRPS1 | 0.38 | -0.37 | 2762 | 3797 | 4731 | 3486 |
| NM_002766 | PRPSAP1 | 0.36 | 0.00 | 452 | 667 | 1125 | 1207 |
| NM_020719 | PRR12 | 0.25 | 0.00 | 664 | 888 | 747 | 688 |
| NM_018457 | PRR13 | 0.33 | -0.09 | 395 | 577 | 1553 | 1338 |
| NM_024031 | PRR14 | 0.23 | 0.00 | 422 | 573 | 574 | 610 |
| RefSeq ID | Gene Symbol | SR GFOLD | SEN GFOLD | SR IL- 2 - | SR IL-2 + | SEN IL-2- | SEN IL-2 + |
| NM_173566 | PRR14L | 0.15 | 0.00 | 1221 | 1487 | 1843 | 1933 |
| NM_016644 | PRR16 | 0.18 | -0.21 | 413 | 545 | 424 | 309 |
| NM_199285 | PRR19 | 0.07 | 0.00 | 58 | 90 | 227 | 203 |
| NM_025263 | PRR3 | 0.34 | 0.00 | 89 | 153 | 177 | 157 |
| NM_004638 | PRRC2A | 0.18 | -0.12 | 8554 | 10007 | 12164 | 10817 |
| NM_013318 | PRRC2B | 0.05 | 0.01 | 6737 | 7247 | 11648 | 12055 |
| NM_001173489 | PRRG1 | 0.19 | 0.00 | 306 | 415 | 374 | 372 |
| NM_016307 | PRRX2 | 0.10 | -0.27 | 482 | 597 | 776 | 566 |
| NM_172341 | PSENEN | 0.29 | 0.00 | 254 | 375 | 799 | 787 |
| NM_001184825 | PSG1 | 0.36 | -0.04 | 110 | 187 | 204 | 155 |
| NM_031246 | PSG2 | 0.16 | 0.00 | 209 | 289 | 299 | 287 |
| NM_002780 | PSG4 | 0.34 | -0.07 | 1589 | 2168 | 3350 | 3018 |
| NM_001130167 | PSG8 | 0.16 | 0.00 | 52 | 87 | 108 | 140 |
| NM_006742 | PSKH1 | 0.21 | 0.00 | 1274 | 1609 | 2868 | 2950 |
| NM_002786 | PSMA1 | 0.23 | 0.00 | 1402 | 1782 | 2159 | 2026 |
| NM_002787 | PSMA2 | 0.85 | 0.00 | 353 | 741 | 747 | 669 |
| NM_002790 | PSMA5 | 0.23 | -0.19 | 1051 | 1360 | 1915 | 1547 |
| NM_001282232 | PSMA6 | 0.34 | -0.07 | 730 | 1034 | 1458 | 1268 |

(continued)

| RefSeq ID | Gene Symbol | SR GFOLD | SEN GFOLD | SR IL- 2 - | SR IL-2 + | SEN IL-2- | SEN IL-2 + |
|---|---|---|---|---|---|---|---|
| NM_002792 | PSMA7 | 0.10 | -0.11 | 3420 | 3876 | 6549 | 5802 |
| NM_002793 | PSMB1 | 0.57 | 0.00 | 1808 | 2887 | 4767 | 4935 |
| NM_002794 | PSMB2 | 0.24 | 0.00 | 1859 | 2361 | 3161 | 3240 |
| NM_002795 | PSMB3 | 0.19 | -0.10 | 1288 | 1607 | 3259 | 2872 |
| NM_002796 | PSMB4 | 0.08 | 0.00 | 3718 | 4146 | 6961 | 6780 |
| NM_001144932 | PSMB5 | 0.37 | -0.06 | 3103 | 4225 | 6906 | 6342 |
| NM_001270481 | PSMB6 | 0.13 | -0.05 | 2176 | 2554 | 4586 | 4218 |
| NM_004159 | PSMB8 | 0.17 | -0.19 | 420 | 549 | 861 | 669 |
| NM_002803 | PSMC2 | 0.10 | -0.12 | 880 | 1046 | 2063 | 1760 |
| NM_002804 | PSMC3 | 0.22 | -0.21 | 1655 | 2085 | 4143 | 3397 |
| NM_006503 | PSMC4 | 0.22 | 0.00 | 1804 | 2260 | 3280 | 3371 |
| NM_002807 | PSMD1 | 0.16 | -0.01 | 2277 | 2720 | 5162 | 4882 |
| NM_001270482 | PSMD11 | 0.29 | -0.01 | 2736 | 3551 | 4349 | 4118 |
| NM_002817 | PSMD13 | 0.41 | -0.05 | 2208 | 3125 | 4723 | 4344 |
| NM_002810 | PSMD4 | 0.27 | 0.00 | 571 | 783 | 1435 | 1388 |
| NM_005047 | PSMD5 | 0.06 | -0.06 | 911 | 1056 | 2073 | 1845 |
| NM_001271779 | PSMD6 | 0.15 | 0.00 | 1102 | 1340 | 1870 | 1800 |
| NM_002811 | PSMD7 | 0.59 | 0.00 | 624 | 1053 | 1308 | 1240 |
| NM_002813 | PSMD9 | 0.06 | -0.09 | 1517 | 1712 | 2795 | 2460 |
| NM_002818 | PSME2 | 0.46 | -0.17 | 131 | 232 | 430 | 322 |
| NM_001267045 | PSME3 | 0.30 | -0.05 | 2495 | 3266 | 4166 | 3811 |
| RefSeq ID | Gene Symbol | SR GFOLD | SEN GFOLD | SR IL-2- | SR IL-2 + | SEN IL-2 - | SEN IL-2 + |
| NM_006814 | PSMF1 | 0.14 | 0.00 | 1791 | 2124 | 3569 | 3697 |
| NM_001261824 | PSMG1 | 0.39 | 0.00 | 429 | 650 | 857 | 773 |
| NM_020232 | PSMG2 | 0.15 | 0.00 | 436 | 563 | 722 | 780 |
| NM_032302 | PSMG3 | 0.30 | -0.37 | 315 | 460 | 510 | 336 |
| NM_001128592 | PSMG4 | 0.17 | -0.01 | 129 | 189 | 212 | 165 |
| NM_001032291 | PSRC1 | 0.78 | -0.66 | 138 | 300 | 304 | 153 |
| NM_024430 | PSTPIP2 | 0.41 | -0.09 | 237 | 382 | 428 | 340 |
| NM_002819 | PTBP1 | 0.12 | -0.04 | 4837 | 5496 | 5499 | 5122 |

(continued)

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_001163788 | PTBP3 | 0.19 | 0.00 | 991 | 1252 | 1477 | 1566 |
| NM_017952 | PTCD3 | 0.11 | 0.00 | 515 | 637 | 830 | 850 |
| NM_001166292 | PTCH2 | 1.39 | -0.50 | 1767 | 4937 | 3298 | 2185 |
| NM_025072 | PTGES2 | 0.33 | 0.00 | 521 | 746 | 1778 | 1699 |
| NM_001136042 | PTGES3L-AARSD1 | 0.33 | -0.04 | 259 | 391 | 532 | 445 |
| NM_000960 | PTGIR | 0.33 | 0.00 | 282 | 424 | 732 | 717 |
| NM_012212 | PTGR1 | 0.30 | 0.00 | 518 | 731 | 1680 | 1734 |
| NM_152444 | PTGR2 | 0.15 | 0.00 | 35 | 63 | 139 | 116 |
| NM_002824 | PTMS | 1.00 | -0.05 | 2432 | 5150 | 3406 | 3107 |
| NM_001253830 | PTPDC1 | 0.02 | 0.00 | 308 | 373 | 630 | 717 |
| NM_014241 | PTPLA | 0.24 | 0.00 | 228 | 328 | 437 | 396 |
| NM_002834 | PTPN11 | 0.26 | 0.00 | 3195 | 4054 | 6097 | 6270 |
| NM_014369 | PTPN18 | 0.18 | 0.00 | 701 | 893 | 801 | 752 |
| NM_015466 | PTPN23 | 0.36 | 0.00 | 1835 | 2523 | 3074 | 3018 |
| NM_002833 | PTPN9 | 0.27 | 0.00 | 1019 | 1354 | 1986 | 2085 |
| NM_002837 | PTPRB | 0.09 | 0.00 | 1488 | 1715 | 2427 | 2440 |
| NM_002842 | PTPRH | 0.04 | 0.00 | 94 | 132 | 54 | 67 |
| NM_001291981 | PTPRK | 0.28 | 0.00 | 2721 | 3504 | 4095 | 4155 |
| NM_000317 | PTS | 0.01 | 0.00 | 337 | 403 | 609 | 643 |
| NM_001282382 | PTTG1 | 0.27 | -1.35 | 389 | 548 | 595 | 192 |
| NM_002852 | PTX3 | 0.51 | -0.41 | 8141 | 11999 | 2774 | 1952 |
| NM_001271098 | PUF60 | 0.03 | 0.00 | 3073 | 3315 | 4353 | 4279 |
| NM_019042 | PUS7 | 0.17 | 0.00 | 345 | 458 | 507 | 499 |
| NM_153339 | PUSL1 | 0.35 | 0.00 | 183 | 291 | 322 | 287 |
| NM_002855 | PVRL1 | 0.62 | 0.01 | 195 | 368 | 378 | 447 |
| NM_002856 | PVRL2 | 0.15 | 0.00 | 1510 | 1822 | 2290 | 2405 |
| NM_001243286 | PVRL3 | 0.33 | 0.00 | 596 | 846 | 1098 | 1176 |
| NM_005049 | PWP2 | 0.15 | 0.00 | 931 | 1146 | 1133 | 1074 |
| NM_007238 | PXMP4 | 0.25 | 0.00 | 217 | 316 | 597 | 597 |
| NM_138300 | PYGO2 | 0.10 | 0.00 | 502 | 618 | 942 | 865 |
| NM_032906 | PYURF | 0.09 | 0.00 | 555 | 675 | 834 | 896 |

(continued)

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_018292 | QRSL1 | 0.03 | -0.22 | 174 | 224 | 393 | 281 |
| NM_031209 | QTRT1 | 0.05 | 0.00 | 356 | 436 | 709 | 682 |
| NM_024638 | QTRTD1 | 0.10 | 0.00 | 408 | 511 | 485 | 497 |
| NM_138774 | R3HDM4 | 0.13 | 0.00 | 725 | 894 | 1194 | 1142 |
| NM_016322 | RAB14 | 0.04 | 0.00 | 1942 | 2150 | 3479 | 3553 |
| NM_016277 | RAB23 | 0.33 | 0.00 | 730 | 1028 | 1568 | 1676 |
| NM_004163 | RAB27B | 0.02 | 0.00 | 22 | 41 | 1059 | 1087 |
| NM_006834 | RAB32 | 0.85 | -0.08 | 1109 | 2178 | 2263 | 1987 |
| NM_001144942 | RAB34 | 0.28 | 0.00 | 1770 | 2312 | 3893 | 3991 |
| NM_013401 | RAB3IL1 | 0.15 | -0.05 | 256 | 343 | 1073 | 932 |
| NM_201434 | RAB5C | 0.48 | 0.00 | 3380 | 4951 | 6567 | 6523 |
| NM_012197 | RABGAP1 | 0.17 | 0.00 | 1061 | 1311 | 2365 | 2285 |
| NM_014857 | RABGAP1L | 0.46 | 0.00 | 222 | 372 | 992 | 991 |
| NM_004581 | RABGGTA | 0.67 | 0.00 | 789 | 1396 | 1886 | 1920 |
| NM_002871 | RABIF | 0.10 | 0.00 | 415 | 519 | 663 | 671 |
| NM_002853 | RAD1 | 0.33 | 0.00 | 329 | 488 | 395 | 453 |
| NM_006265 | RAD21 | 0.01 | 0.00 | 2135 | 2307 | 2539 | 2384 |
| NM_001270362 | RAD23A | 0.06 | -0.18 | 3990 | 4387 | 6726 | 5700 |
| NM_002874 | RAD23B | 0.29 | 0.00 | 2683 | 3483 | 5006 | 5041 |
| NM_002875 | RAD51 | 0.07 | -0.44 | 380 | 469 | 297 | 176 |
| NM_003610 | RAE1 | 0.45 | 0.00 | 532 | 826 | 876 | 885 |
| NM_001001788 | RAET1G | 1.70 | 0.00 | 2 | 32 | 94 | 67 |
| NM_002880 | RAF1 | 0.47 | 0.00 | 1945 | 2894 | 3858 | 4015 |
| NM_016732 | RALY | 0.33 | 0.00 | 2075 | 2782 | 4163 | 4055 |
| NM_001278639 | RANBP1 | 0.34 | -0.21 | 347 | 517 | 624 | 468 |
| NM_020850 | RANBP10 | 0.04 | 0.00 | 368 | 447 | 792 | 885 |
| NM_003624 | RANBP3 | 0.17 | 0.00 | 1260 | 1550 | 2086 | 1959 |
| NM_012294 | RAPGEF5 | 0.87 | 0.00 | 19 | 63 | 31 | 24 |
| NM_001042728 | RARG | 0.18 | 0.00 | 930 | 1169 | 1493 | 1577 |
| NM_002887 | RARS | 0.44 | 0.00 | 1354 | 1996 | 2473 | 2421 |
| NM_007368 | RASA3 | 0.23 | 0.00 | 3051 | 3785 | 5563 | 5562 |
| NM_017805 | RASIP1 | 0.29 | -0.90 | 334 | 482 | 863 | 401 |
| NM_170713 | RASSF1 | 0.16 | 0.00 | 1440 | 1753 | 1614 | 1497 |

(continued)

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_178169 | RASSF3 | 0.20 | -0.43 | 903 | 1148 | 2117 | 1449 |
| NM_133452 | RAVER1 | 0.04 | 0.00 | 1284 | 1446 | 1144 | 1129 |
| NM_006606 | RBBP9 | 0.76 | 0.00 | 191 | 397 | 821 | 745 |
| NM_006047 | RBM12 | 0.06 | -0.20 | 2660 | 2953 | 2425 | 1974 |
| NM_006328 | RBM14 | 0.04 | -0.06 | 1616 | 1800 | 1778 | 1571 |
| NM_001291780 | RBM38 | 0.30 | 0.00 | 116 | 188 | 162 | 207 |
| NM_002896 | RBM4 | 0.10 | 0.00 | 1401 | 1636 | 2485 | 2632 |
| NM_024321 | RBM42 | 0.05 | 0.00 | 1455 | 1635 | 1832 | 1741 |
| NM_005777 | RBM6 | 0.15 | 0.00 | 895 | 1100 | 1385 | 1309 |
| NM_005105 | RBM8A | 0.19 | 0.00 | 459 | 605 | 724 | 795 |
| NM_001251974 | RCAN2 | 0.06 | 0.00 | 87 | 125 | 152 | 118 |
| NM_001268 | RCBTB2 | 0.07 | 0.00 | 75 | 111 | 285 | 329 |
| NM_002901 | RCN1 | 0.26 | 0.00 | 3097 | 3931 | 6362 | 6102 |
| NM_020905 | RDH14 | 0.31 | 0.00 | 216 | 328 | 482 | 527 |
| NM_001048205 | REC8 | 0.49 | 0.00 | 23 | 57 | 77 | 100 |
| NM_004260 | RECQL4 | 0.02 | 0.00 | 370 | 443 | 443 | 381 |
| NM_004259 | RECQL5 | 0.17 | 0.00 | 167 | 238 | 293 | 342 |
| NM_025232 | REEP4 | 0.04 | -0.21 | 478 | 569 | 616 | 462 |
| NM_005045 | RELN | 0.17 | -0.29 | 2490 | 2989 | 925 | 671 |
| NM_002910 | RENBP | 0.04 | 0.00 | 78 | 113 | 285 | 251 |
| NM_013400 | REPIN1 | 0.12 | 0.00 | 721 | 878 | 984 | 933 |
| NM_001278791 | RFC2 | 0.21 | -0.03 | 457 | 611 | 493 | 414 |
| NM_002916 | RFC4 | 0.07 | -0.17 | 422 | 516 | 318 | 231 |
| NM_018339 | RFK | 0.23 | -0.02 | 374 | 513 | 788 | 688 |
| NM_015150 | RFTN1 | 0.32 | 0.00 | 1639 | 2210 | 4062 | 3974 |
| NM_022457 | RFWD2 | 0.11 | 0.00 | 249 | 326 | 489 | 484 |
| NM_018124 | RFWD3 | 0.11 | -0.18 | 1265 | 1495 | 1346 | 1081 |
| NM_000449 | RFX5 | 0.23 | 0.00 | 414 | 565 | 1140 | 1166 |
| NM_003721 | RFXANK | 0.32 | -0.26 | 606 | 853 | 1395 | 1057 |
| NM_001012761 | RGMB | 0.44 | -0.12 | 6096 | 8610 | 9602 | 8534 |
| NM_001282848 | RGN | 0.39 | -0.49 | 31 | 67 | 195 | 105 |
| NM_002926 | RGS12 | 0.18 | 0.00 | 369 | 489 | 697 | 750 |
| NM_006480 | RGS14 | 0.73 | 0.00 | 68 | 158 | 206 | 174 |

(continued)

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_001282773 | RGS7 | 0.37 | 0.00 | 52 | 100 | 33 | 31 |
| NM_001242359 | RHOBTB1 | 0.57 | 0.00 | 297 | 521 | 799 | 736 |
| NM_014578 | RHOD | 0.41 | -0.21 | 193 | 317 | 670 | 505 |
| NM_001665 | RHOG | 0.21 | 0.00 | 1659 | 2070 | 2188 | 2264 |
| NM_138769 | RHOT2 | 0.10 | 0.00 | 1041 | 1231 | 1364 | 1423 |
| NM_033103 | RHPN2 | 0.49 | 0.00 | 37 | 82 | 19 | 33 |
| NM_021932 | RIC8A | 0.25 | 0.00 | 5016 | 6222 | 8539 | 8504 |
| NM_018157 | RIC8B | 0.19 | 0.01 | 291 | 397 | 510 | 591 |
| NM_018151 | RIF1 | 0.09 | 0.00 | 1405 | 1632 | 1649 | 1784 |
| NM_145058 | RILPL2 | 0.21 | 0.00 | 192 | 277 | 198 | 205 |
| NM_004292 | RIN1 | 0.36 | 0.00 | 245 | 381 | 480 | 551 |
| NM_024832 | RIN3 | 0.08 | 0.00 | 963 | 1130 | 1774 | 1867 |
| NM_018343 | RIOK2 | 0.07 | 0.00 | 439 | 537 | 634 | 710 |
| NM_016033 | RMDN1 | 0.72 | 0.00 | 180 | 365 | 790 | 841 |
| NM_152308 | RMI2 | 0.01 | 0.00 | 74 | 106 | 73 | 70 |
| NM_032193 | RNASEH2C | 0.66 | 0.00 | 515 | 926 | 1470 | 1590 |
| NM_022064 | RNF123 | 0.05 | 0.00 | 1076 | 1231 | 2215 | 2379 |
| NM_001184992 | RNF135 | 0.14 | 0.00 | 287 | 379 | 584 | 647 |
| NM_016422 | RNF141 | 0.18 | 0.00 | 463 | 607 | 1046 | 1035 |
| NM_001242844 | RNF146 | 0.37 | 0.00 | 263 | 409 | 722 | 708 |
| NM_016494 | RNF181 | 0.57 | 0.00 | 331 | 575 | 1242 | 1227 |
| NM_001256071 | RNF213 | 0.12 | 0.00 | 6897 | 7790 | 17419 | 17770 |
| NM_024546 | RNF219 | 0.17 | 0.00 | 270 | 365 | 343 | 362 |
| NM_022453 | RNF25 | 0.13 | 0.00 | 376 | 484 | 932 | 850 |
| NM_002938 | RNF4 | 0.51 | 0.00 | 859 | 1357 | 1834 | 1957 |
| NM_001207033 | RNF40 | 0.41 | 0.00 | 2057 | 2925 | 4484 | 4628 |
| NM_006913 | RNF5 | 0.04 | 0.00 | 345 | 421 | 510 | 564 |
| NM_203387 | RNH1 | 0.25 | -0.01 | 5557 | 6898 | 14959 | 14456 |
| NM_001286625 | RNPS1 | 0.43 | 0.00 | 1377 | 2012 | 2209 | 2116 |
| NM_022370 | ROBO3 | 0.51 | 0.00 | 72 | 143 | 110 | 124 |
| NM_002945 | RPA1 | 0.32 | 0.00 | 1625 | 2182 | 2523 | 2569 |

(continued)

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_001286076 | RPA2 | 0.23 | -0.06 | 400 | 547 | 532 | 440 |
| NM_002947 | RPA3 | 1.09 | 0.00 | 18 | 70 | 79 | 68 |
| NM_001033002 | RPAIN | 0.26 | 0.00 | 590 | 803 | 905 | 948 |
| NM_015540 | RPAP1 | 0.46 | 0.00 | 538 | 842 | 1233 | 1320 |
| NM_001278283 | RPE | 0.26 | 0.00 | 258 | 374 | 755 | 684 |
| NM_006013 | RPL10 | 0.15 | -0.08 | 4357 | 5056 | 9288 | 8468 |
| NM_007104 | RPL10A | 0.27 | -0.39 | 1480 | 1938 | 3232 | 2307 |
| NM_000975 | RPL11 | 0.07 | -0.12 | 4723 | 5204 | 8961 | 7965 |
| NM_000976 | RPL12 | 1.00 | -0.11 | 317 | 741 | 1013 | 843 |
| NM_033251 | RPL13 | 0.41 | -0.38 | 4442 | 6194 | 10648 | 7885 |
| NM_012423 | RPL13A | 0.44 | -0.22 | 2671 | 3849 | 5987 | 4915 |
| NM_001253382 | RPL15 | 0.26 | -0.24 | 6011 | 7518 | 14955 | 12327 |
| NM_000985 | RPL17 | 1.05 | 0.00 | 996 | 2258 | 2716 | 2896 |
| NM_001199356 | RPL17-C18orf32 | 0.43 | 0.00 | 562 | 861 | 1291 | 1416 |
| NM_000979 | RPL18 | 0.11 | -0.29 | 4194 | 4757 | 7699 | 6039 |
| NM_000980 | RPL18A | 0.49 | -0.41 | 1354 | 2065 | 3525 | 2503 |
| NM_000981 | RPL19 | 0.64 | -0.23 | 1380 | 2332 | 3506 | 2817 |
| NM_000982 | RPL21 | 0.51 | 0.00 | 93 | 178 | 177 | 148 |
| NM_000983 | RPL22 | 0.19 | -0.01 | 1208 | 1507 | 2658 | 2466 |
| NM_000984 | RPL23A | 0.24 | -0.12 | 1690 | 2158 | 3078 | 2671 |
| RefSeq ID | Gene Symbol | SR GFOLD | SEN GFOLD | SR IL-2 - | SR IL-2 + | SEN IL-2 - | SEN IL-2 + |
| NM_000986 | RPL24 | 1.00 | -0.16 | 345 | 801 | 980 | 784 |
| NM_000987 | RPL26 | 0.31 | -0.09 | 1272 | 1720 | 3084 | 2725 |
| NM_000990 | RPL27 A | 0.12 | -0.03 | 2132 | 2480 | 3388 | 3122 |
| NM_001136134 | RPL28 | 0.69 | -0.02 | 4317 | 7259 | 9013 | 8553 |
| NM_000967 | RPL3 | 0.45 | -0.28 | 6338 | 8994 | 11900 | 9482 |
| NM_000989 | RPL30 | 0.04 | 0.00 | 4147 | 4492 | 5987 | 5970 |
| NM_001099693 | RPL31 | 0.43 | -0.13 | 380 | 597 | 1027 | 843 |
| NM_001007074 | RPL32 | 0.97 | -0.46 | 3916 | 8037 | 68665 | 49312 |
| NM_033625 | RPL34 | 0.33 | -0.20 | 374 | 548 | 772 | 593 |
| NM_007209 | RPL35 | 0.07 | -0.22 | 2140 | 2415 | 5538 | 4532 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_033643 | RPL36 | 0.80 | -0.35 | 822 | 1579 | 2196 | 1593 |
| NM_021029 | RPL36A | 0.13 | -0.52 | 69 | 108 | 166 | 85 |
| NM_001199973 | RPL36A-HNRNPH2 | 0.09 | 0.00 | 1240 | 1441 | 2543 | 2377 |
| NM_001001 | RPL36AL | 0.18 | 0.00 | 1475 | 1817 | 2217 | 2327 |
| NM_052969 | RPL39L | 1.09 | -0.17 | 82 | 234 | 302 | 218 |
| NM_000968 | RPL4 | 0.31 | -0.31 | 4844 | 6298 | 10964 | 8527 |
| NM_000969 | RPL5 | 0.22 | -0.12 | 2280 | 2836 | 5160 | 4514 |
| NM_000971 | RPL7 | 0.39 | -0.16 | 506 | 757 | 1111 | 893 |
| NM_000972 | RPL7A | 0.36 | -0.45 | 2918 | 3971 | 7740 | 5431 |
| NM_001024921 | RPL9 | 1.18 | -0.12 | 389 | 1013 | 1096 | 906 |
| NM_053275 | RPLP0 | 0.31 | -0.17 | 10422 | 13300 | 18261 | 15881 |
| NM_001003 | RPLP1 | 0.70 | -0.34 | 1964 | 3408 | 3157 | 2338 |
| NM_001004 | RPLP2 | 1.11 | -0.03 | 1117 | 2624 | 2797 | 2577 |
| NM_148179 | RPP25L | 0.42 | 0.00 | 144 | 246 | 383 | 421 |
| NM_183005 | RPP38 | 0.13 | 0.00 | 302 | 395 | 420 | 431 |
| NM_021215 | RPRD1B | 0.38 | 0.00 | 558 | 824 | 1108 | 1172 |
| NM_001203245 | RPS10 | 0.40 | 0.00 | 753 | 1108 | 1425 | 1353 |
| NM_001202470 | RPS 10-NUDT3 | 0.38 | 0.00 | 998 | 1430 | 1903 | 1991 |
| NM_001015 | RPS11 | 0.68 | 0.00 | 3469 | 5838 | 8223 | 7904 |
| NM_001016 | RPS12 | 0.44 | 0.00 | 1065 | 1584 | 1911 | 1810 |
| NM_001017 | RPS13 | 0.04 | -0.17 | 752 | 870 | 1281 | 1035 |
| NM_001025070 | RPS14 | 0.87 | -0.48 | 1170 | 2319 | 4280 | 2911 |
| NM_001018 | RPS15 | 0.38 | -0.55 | 1636 | 2301 | 4648 | 3017 |
| NM_001019 | RPS15A | 0.79 | 0.00 | 314 | 638 | 703 | 684 |
| NM_001020 | RPS16 | 0.46 | -0.24 | 2732 | 3995 | 5147 | 4159 |
| NM_022551 | RPS18 | 0.90 | -0.26 | 517 | 1090 | 1845 | 1416 |
| NM_194326 | RPS19BP1 | 0.08 | 0.00 | 859 | 1012 | 1391 | 1357 |
| NM_002952 | RPS2 | 0.18 | -0.35 | 4327 | 5142 | 7042 | 5303 |
| NM_001023 | RPS20 | 0.37 | 0.00 | 2165 | 2985 | 3625 | 3506 |
| NM_001024 | RPS21 | 0.21 | 0.00 | 273 | 380 | 605 | 593 |
| NM_001025 | RPS23 | 0.60 | -0.38 | 1525 | 2500 | 3660 | 2652 |
| NM_001030 | RPS27 | 0.19 | 0.00 | 93 | 144 | 256 | 235 |
| NM_015920 | RPS27L | 0.16 | -0.13 | 1071 | 1313 | 3228 | 2773 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001032 | RPS29 | 1.27 | 0.00 | 116 | 359 | 495 | 529 |
| NM_001260506 | RPS3 | 0.23 | -0.35 | 5032 | 6168 | 8541 | 6445 |
| NM_001007 | RPS4X | 0.20 | -0.17 | 3478 | 4204 | 6929 | 5928 |
| NM_001009 | RPS5 | 0.54 | -0.29 | 1682 | 2634 | 3764 | 2915 |
| NM_001010 | RPS6 | 0.22 | -0.25 | 9254 | 11144 | 18727 | 15381 |
| NM_021135 | RPS6KA2 | 0.19 | -0.20 | 4011 | 4813 | 7445 | 6236 |
| NM_001006944 | RPS6KA4 | 0.26 | -0.32 | 994 | 1311 | 1805 | 1327 |
| NM_001011 | RPS7 | 0.53 | -0.62 | 972 | 1548 | 2452 | 1477 |
| NM_001012 | RPS8 | 0.24 | -0.28 | 5557 | 6843 | 12336 | 9848 |
| NM_032795 | RPUSD4 | 0.15 | -0.22 | 615 | 775 | 977 | 749 |
| NM_001271634 | RQCD1 | 0.16 | -0.26 | 1830 | 2205 | 2227 | 1728 |
| NM_016656 | RRAGB | 0.03 | 0.00 | 150 | 197 | 225 | 266 |
| NM_012250 | RRAS2 | 0.17 | 0.00 | 587 | 751 | 1135 | 1190 |
| NM_001033 | RRM1 | 0.28 | -0.11 | 1840 | 2399 | 2573 | 2224 |
| NM_015997 | RRNAD1 | 0.16 | 0.00 | 236 | 321 | 291 | 333 |
| NM_016052 | RRP15 | 0.06 | -0.03 | 356 | 439 | 451 | 375 |
| NM_033112 | RRP36 | 0.68 | -0.15 | 522 | 950 | 1720 | 1427 |
| NM_015703 | RRP7A | 0.25 | -0.08 | 892 | 1180 | 1539 | 1338 |
| NM_004704 | RRP9 | 0.35 | -0.12 | 505 | 734 | 690 | 558 |
| NM_018364 | RSBN1 | 0.07 | 0.00 | 314 | 393 | 381 | 416 |
| NM_001130841 | RTCA | 0.22 | 0.00 | 467 | 625 | 1341 | 1366 |
| NM_014306 | RTCB | 0.17 | 0.00 | 2224 | 2682 | 3248 | 3447 |
| NM_001283035 | RTFDC1 | 0.19 | 0.00 | 1564 | 1931 | 3427 | 3495 |
| NM_032730 | RTN4IP1 | 0.33 | -0.10 | 35 | 71 | 135 | 92 |
| NM_173630 | RTTN | 0.21 | 0.00 | 454 | 605 | 537 | 490 |
| NM_001037442 | RUFY3 | 0.14 | 0.00 | 96 | 144 | 331 | 305 |
| NM_001105203 | RUSC1 | 0.10 | -0.05 | 970 | 1148 | 1809 | 1614 |
| NM_006666 | RUVBL2 | 0.37 | -0.23 | 1628 | 2263 | 2928 | 2342 |
| NM_016104 | RWDD1 | 0.25 | -0.06 | 443 | 610 | 973 | 839 |
| NM_016940 | RWDD2B | 0.29 | 0.00 | 264 | 388 | 489 | 564 |
| NM_001278248 | RWDD3 | 1.42 | 0.00 | 37 | 151 | 177 | 150 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001005861 | RYK | 0.29 | 0.00 | 496 | 693 | 1250 | 1213 |
| NM_002960 | S100A3 | 0.28 | -0.11 | 50 | 91 | 162 | 113 |
| NM_014624 | S100A6 | 0.52 | 0.00 | 7410 | 10971 | 22491 | 22164 |
| RefSeq ID | Gene Symbol | SR GFOLD | SEN GFOLD | SR IL-2- | SR IL-2 + | SEN IL-2 - | SEN IL-2 + |
| NM_001400 | S1PR1 | 0.05 | -0.14 | 1060 | 1212 | 799 | 641 |
| NM_004230 | S1PR2 | 0.14 | 0.00 | 615 | 769 | 717 | 799 |
| NM_013299 | SAC3D1 | 0.61 | 0.00 | 74 | 156 | 125 | 118 |
| NM_005500 | SAE1 | 0.38 | -0.20 | 1853 | 2596 | 3022 | 2466 |
| NM_138352 | SAMD1 | 0.30 | 0.00 | 249 | 372 | 312 | 368 |
| NM_017827 | SARS2 | 0.68 | -0.24 | 168 | 334 | 553 | 403 |
| NM_015278 | SASH1 | 0.18 | 0.00 | 2596 | 3123 | 4754 | 4811 |
| NM_133491 | SAT2 | 0.30 | -0.28 | 639 | 890 | 1325 | 989 |
| NM_001195470 | SATB1 | 0.07 | 0.00 | 118 | 164 | 584 | 632 |
| NM_018322 | SAYSD1 | 0.39 | 0.00 | 82 | 148 | 345 | 331 |
| NM_014963 | SBNO2 | 0.18 | 0.00 | 1577 | 1933 | 1932 | 2026 |
| NM_005698 | SCAMP3 | 0.22 | -0.14 | 1598 | 2012 | 3818 | 3286 |
| NM_012235 | SCAP | 0.15 | 0.00 | 1395 | 1680 | 2034 | 1896 |
| NM_153334 | SCARF2 | 0.05 | 0.00 | 1973 | 2191 | 2835 | 2976 |
| NM_016106 | SCFD1 | 0.12 | 0.00 | 824 | 998 | 1714 | 1739 |
| NM_152540 | SCFD2 | 0.18 | -0.56 | 1264 | 1559 | 3440 | 2189 |
| NM_016510 | SCLY | 0.09 | 0.00 | 126 | 176 | 247 | 227 |
| NM_001172218 | SCMH1 | 0.07 | 0.00 | 669 | 791 | 1052 | 1057 |
| NM_001037 | SCN1B | 0.03 | 0.00 | 67 | 99 | 331 | 307 |
| NM_001204856 | SCNM1 | 0.33 | 0.00 | 312 | 466 | 668 | 677 |
| NM_001169111 | SCO2 | 0.02 | 0.00 | 687 | 788 | 1377 | 1466 |
| NM_002979 | SCP2 | 0.26 | 0.00 | 976 | 1287 | 2350 | 2397 |
| NM_007281 | SCRG1 | 0.52 | -0.14 | 77 | 153 | 44 | 22 |
| NM_001288984 | SDAD1 | 0.22 | 0.00 | 344 | 472 | 684 | 725 |
| NM_002999 | SDC4 | 0.29 | -0.08 | 2244 | 2921 | 3165 | 2823 |
| NM_006923 | SDF2 | 0.37 | 0.00 | 685 | 992 | 1466 | 1516 |
| NM_022044 | SDF2L1 | 0.02 | 0.00 | 282 | 344 | 399 | 434 |
| NM_017841 | SDHAF2 | 0.14 | 0.00 | 439 | 561 | 846 | 902 |

(continued)

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_003000 | SDHB | 0.39 | 0.00 | 922 | 1333 | 2142 | 2225 |
| NM_003001 | SDHC | 0.07 | 0.00 | 605 | 720 | 1722 | 1691 |
| NM_001276506 | SDHD | 0.12 | 0.00 | 399 | 506 | 707 | 719 |
| NM_001136026 | SEC13 | 0.08 | 0.00 | 5442 | 5997 | 5634 | 5462 |
| NM_014866 | SEC16A | 0.22 | 0.01 | 4629 | 5653 | 8462 | 8802 |
| NM_001172746 | SEC23B | 0.14 | 0.00 | 1617 | 1927 | 2125 | 2244 |
| NM_007190 | SEC23IP | 0.22 | 0.00 | 1236 | 1576 | 1965 | 2017 |
| NM_004922 | SEC24C | 0.08 | 0.00 | 4174 | 4647 | 6630 | 6647 |
| NM_006808 | SEC61B | 0.91 | -0.21 | 318 | 701 | 1125 | 876 |
| NM_001282688 | SECISBP2 | 0.05 | 0.00 | 395 | 478 | 923 | 845 |
| NM_003004 | SECTM1 | 0.04 | 0.00 | 57 | 87 | 48 | 28 |

**Table 5A: Differentially Upregulated genes in SR + IL-2**

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_031216 | SEH1L | 0.04 | 0.00 | 866 | 994 | 1204 | 1311 |
| NM_001258289 | SELENBP1 | 0.19 | -0.77 | 36 | 66 | 204 | 89 |
| NM_016275 | SELT | 0.08 | 0.00 | 836 | 985 | 1730 | 1835 |
| NM_006080 | SEMA3A | 0.21 | 0.00 | 228 | 323 | 308 | 261 |
| NM_017893 | SEMA4G | 0.06 | 0.00 | 107 | 150 | 189 | 231 |
| NM_153617 | SEMA6D | 0.16 | -0.29 | 234 | 320 | 58 | 28 |
| NM_015670 | SENP3 | 0.05 | 0.00 | 1874 | 2086 | 2699 | 2806 |
| NM_012247 | SEPHS1 | 0.14 | 0.00 | 517 | 653 | 720 | 652 |
| NM_020451 | SEPN1 | 0.18 | 0.00 | 2972 | 3576 | 6002 | 6281 |
| NM_003009 | SEPW1 | 0.56 | 0.00 | 328 | 569 | 1689 | 1680 |
| NM_004155 | SERPINB9 | 0.24 | -0.74 | 217 | 315 | 258 | 120 |
| NM_002615 | SERPINF1 | 0.34 | -0.56 | 229 | 353 | 1210 | 734 |
| NM_001207014 | SERPINH1 | 0.28 | -0.06 | 8513 | 10678 | 10523 | 9763 |
| NM_003011 | SET | 0.52 | 0.00 | 1750 | 2687 | 3696 | 3560 |
| NM_001160305 | SETD6 | 0.17 | 0.00 | 142 | 206 | 293 | 333 |
| NM_020382 | SETD8 | 0.18 | 0.00 | 646 | 829 | 1258 | 1262 |
| NM_001145415 | SETDB1 | 0.18 | 0.00 | 550 | 711 | 772 | 858 |

(continued)

| Table 5A: Differentially Upregulated genes in SR + IL-2 | | | | | | | |
|---|---|---|---|---|---|---|---|
| RefSeq ID | Gene Symbol | SR GFOLD | SEN GFOLD | SR IL-2- | SR IL-2 + | SEN IL-2 - | SEN IL-2 + |
| NM_001287737 | SETSIP | 0.67 | 0.00 | 104 | 218 | 223 | 220 |
| NM_007165 | SF3A2 | 0.38 | 0.00 | 697 | 1014 | 586 | 628 |
| NM_006802 | SF3A3 | 0.32 | 0.00 | 616 | 869 | 925 | 882 |
| NM_012426 | SF3B3 | 0.31 | -0.12 | 3833 | 4988 | 6451 | 5689 |
| NM_005850 | SF3B4 | 0.10 | -0.07 | 1271 | 1488 | 1522 | 1325 |
| NM_031287 | SF3B5 | 0.21 | -0.02 | 1359 | 1706 | 2859 | 2654 |
| NM_016047 | SF3B6 | 0.26 | 0.00 | 634 | 860 | 1431 | 1420 |
| NM_005066 | SFPQ | 0.33 | -0.05 | 2459 | 3294 | 2512 | 2274 |
| NM_001261411 | SFSWAP | 0.28 | -0.17 | 602 | 829 | 952 | 754 |
| NM_030971 | SFXN3 | 0.34 | 0.00 | 1235 | 1701 | 2595 | 2664 |
| NM_213649 | SFXN4 | 0.28 | 0.00 | 109 | 175 | 204 | 205 |
| NM_144579 | SFXN5 | 0.20 | 0.00 | 186 | 267 | 616 | 654 |
| NM_005627 | SGK1 | 0.05 | -0.06 | 8304 | 8922 | 3589 | 3244 |
| NM_147156 | SGMS1 | 0.22 | 0.00 | 681 | 894 | 917 | 976 |
| NM_015705 | SGSM3 | 0.10 | 0.00 | 1076 | 1266 | 2236 | 2187 |
| NM_001103161 | SH2D5 | 0.08 | -0.50 | 729 | 869 | 738 | 453 |
| NM_031469 | SH3BGRL2 | 2.14 | 0.00 | 1 | 31 | 77 | 83 |
| NM_018957 | SH3BP1 | 0.83 | 0.00 | 52 | 135 | 112 | 137 |
| NM_014521 | SH3BP4 | 0.28 | -0.06 | 4207 | 5375 | 6443 | 5944 |
| NM_001009555 | SH3D19 | 0.17 | 0.00 | 1624 | 1971 | 2970 | 3066 |
| NM_003025 | SH3GL1 | 0.05 | 0.00 | 3189 | 3500 | 3802 | 3719 |
| NM_020870 | SH3RF1 | 0.27 | 0.00 | 3964 | 5024 | 4773 | 4810 |
| NM_024745 | SHCBP1 | 0.05 | -0.64 | 929 | 1066 | 782 | 436 |
| NM_138356 | SHF | 1.28 | -2.03 | 243 | 699 | 524 | 100 |
| NM_001272067 | SHISA5 | 0.12 | -0.29 | 2286 | 2655 | 4887 | 3789 |
| NM_004169 | SHMT1 | 0.39 | 0.00 | 252 | 399 | 285 | 253 |
| NM_013276 | SHPK | 0.42 | 0.00 | 397 | 615 | 736 | 776 |
| NM_018130 | SHQ1 | 0.25 | 0.00 | 302 | 428 | 412 | 447 |
| NM_020859 | SHROOM3 | 0.25 | -0.21 | 1335 | 1723 | 2408 | 1939 |
| NM_001199922 | SIAE | 0.13 | 0.00 | 173 | 239 | 749 | 760 |
| NM_198567 | SIMC1 | 0.71 | 0.00 | 105 | 226 | 125 | 129 |
| NM_015073 | SIPA1L3 | 0.07 | 0.00 | 5341 | 5852 | 4359 | 4264 |

(continued)

| Table 5A: Differentially Upregulated genes in SR + IL-2 | | | | | | |
|---|---|---|---|---|---|---|
| RefSeq ID | Gene Symbol | SR GFOLD | SEN GFOLD | SR IL-2- | SR IL-2 + | SEN IL-2 - | SEN IL-2 + |
| NM_012237 | SIRT2 | 0.11 | -0.06 | 862 | 1036 | 2057 | 1837 |
| NM_012241 | SIRT5 | 0.04 | -0.32 | 183 | 237 | 489 | 331 |
| NM_016539 | SIRT6 | 0.43 | -0.11 | 207 | 341 | 532 | 423 |
| NM_006427 | SIVA1 | 0.31 | -0.20 | 410 | 591 | 674 | 512 |
| NM_005982 | SIX1 | 0.22 | 0.00 | 209 | 301 | 532 | 593 |
| NM_182620 | SKA2 | 0.17 | -0.02 | 338 | 448 | 485 | 410 |
| NM_005983 | SKP2 | 0.07 | -0.45 | 390 | 480 | 539 | 336 |
| NM_005072 | SLC12A4 | 0.04 | 0.00 | 3320 | 3620 | 5682 | 5896 |
| NM_012450 | SLC13A4 | 0.10 | 0.00 | 40 | 68 | 141 | 150 |
| NM_004172 | SLC1A3 | 0.27 | 0.00 | 224 | 330 | 279 | 309 |
| NM_006749 | SLC20A2 | 0.20 | 0.00 | 2527 | 3093 | 6457 | 6471 |
| NM_003562 | SLC25A11 | 0.38 | 0.00 | 1106 | 1578 | 2063 | 2116 |
| NM_001160210 | SLC25A13 | 0.12 | 0.00 | 263 | 345 | 464 | 433 |
| NM_014252 | SLC25A15 | 0.22 | 0.00 | 171 | 251 | 343 | 298 |
| NM_021734 | SLC25A19 | 0.67 | 0.00 | 119 | 246 | 293 | 268 |
| NM_000387 | SLC25A20 | 0.11 | 0.00 | 197 | 265 | 684 | 738 |
| NM_024698 | SLC25A22 | 0.29 | -0.07 | 665 | 918 | 898 | 763 |
| NM_013386 | SLC25A24 | 0.14 | -0.01 | 984 | 1196 | 1714 | 1569 |
| NM_213611 | SLC25A3 | 0.24 | -0.22 | 5866 | 7201 | 10822 | 8978 |
| NM_032315 | SLC25A33 | 0.75 | 0.00 | 40 | 103 | 96 | 87 |
| NM_017875 | SLC25A38 | 0.20 | 0.00 | 395 | 531 | 709 | 706 |
| NM_018843 | SLC25A40 | 0.22 | -0.03 | 173 | 254 | 287 | 229 |
| NM_145305 | SLC25A43 | 0.32 | 0.00 | 453 | 650 | 1198 | 1198 |
| NM_001286184 | SLC25A44 | 0.54 | 0.00 | 573 | 944 | 1183 | 1170 |
| NM_001152 | SLC25A5 | 0.25 | -0.46 | 2798 | 3536 | 4390 | 3018 |
| NM_001166347 | SLC26A11 | 0.15 | 0.00 | 203 | 279 | 497 | 444 |
| NM_005094 | SLC27A4 | 0.08 | -0.19 | 934 | 1093 | 2874 | 2357 |
| NM_012254 | SLC27A5 | 0.05 | -0.22 | 34 | 58 | 79 | 44 |
| NM_001174098 | SLC29A3 | 0.26 | 0.00 | 123 | 193 | 275 | 333 |
| RefSeq ID | Gene Symbol | SR GFOLD | SEN GFOLD | SR IL-2 - | SR IL-2 + | SEN IL-2 - | SEN IL-2 + |
| NM_020062 | SLC2A4RG | 0.22 | 0.00 | 889 | 1149 | 1886 | 1859 |
| NM_021194 | SLC30A1 | 0.25 | 0.00 | 348 | 488 | 534 | 556 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_080670 | SLC35A4 | 0.07 | -0.22 | 1472 | 1678 | 2512 | 2018 |
| NM_015139 | SLC35D1 | 0.23 | 0.00 | 439 | 594 | 1092 | 1144 |
| NM_001164277 | SLC37A4 | 0.04 | 0.00 | 384 | 462 | 618 | 566 |
| NM_173514 | SLC38A9 | 0.09 | 0.00 | 100 | 144 | 247 | 283 |
| NM_001271957 | SLC39A1 | 0.44 | -0.22 | 1202 | 1783 | 2593 | 2079 |
| NM_001039960 | SLC4A8 | 0.85 | 0.00 | 28 | 83 | 366 | 420 |
| NM_003041 | SLC5A2 | 0.07 | 0.00 | 448 | 545 | 720 | 726 |
| NM_012244 | SLC7A8 | 0.29 | 0.00 | 75 | 128 | 2556 | 2510 |
| NM_001260491 | SLC9A8 | 0.38 | 0.00 | 186 | 300 | 724 | 717 |
| NM_001289009 | SLFN12 | 0.12 | 0.00 | 133 | 188 | 262 | 262 |
| NM_144682 | SLFN13 | 0.30 | 0.00 | 121 | 195 | 87 | 113 |
| NM_004787 | SLIT2 | 0.22 | -0.20 | 2629 | 3250 | 3479 | 2859 |
| NM_001014999 | SLX1A | 0.34 | 0.00 | 639 | 912 | 672 | 603 |
| NM_024044 | SLX1B | 0.34 | 0.00 | 639 | 912 | 672 | 603 |
| NM_005900 | SMAD1 | 0.74 | 0.00 | 125 | 269 | 401 | 381 |
| NM_005902 | SMAD3 | 0.42 | -0.27 | 984 | 1446 | 2394 | 1852 |
| NM_001031628 | SMAGP | 0.67 | 0.00 | 178 | 351 | 695 | 730 |
| NM_022733 | SMAP2 | 0.05 | -0.44 | 1083 | 1234 | 2159 | 1475 |
| NM_003069 | SMARCA1 | 0.04 | -0.31 | 765 | 885 | 1770 | 1312 |
| NM_003076 | SMARCD1 | 0.19 | 0.00 | 1179 | 1471 | 1583 | 1715 |
| NM_001098426 | SMARCD2 | 0.06 | 0.00 | 1363 | 1553 | 1853 | 1861 |
| NM_003079 | SMARCE1 | 0.51 | 0.00 | 673 | 1074 | 1470 | 1405 |
| NM_015110 | SMC5 | 0.19 | 0.00 | 696 | 892 | 1144 | 1037 |
| NM_020179 | SMCO4 | 0.35 | 0.00 | 149 | 241 | 121 | 133 |
| NM_033318 | SMDT1 | 0.37 | 0.00 | 226 | 355 | 497 | 464 |
| NM_001124767 | SMIM4 | 0.12 | -0.27 | 132 | 187 | 597 | 427 |
| NM_005871 | SMNDC1 | 0.27 | 0.00 | 366 | 517 | 570 | 636 |
| NM_018225 | SMU1 | 0.22 | 0.00 | 462 | 621 | 859 | 932 |
| NM_020197 | SMYD2 | 0.43 | 0.00 | 618 | 942 | 1425 | 1549 |
| NM_006062 | SMYD5 | 0.59 | 0.00 | 591 | 1006 | 1221 | 1192 |
| NM_003068 | SNAI2 | 0.72 | -0.20 | 777 | 1415 | 3885 | 3205 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_053052 | SNAP47 | 0.28 | 0.00 | 746 | 1015 | 1364 | 1455 |
| NM_003082 | SNAPC1 | 0.07 | 0.00 | 755 | 888 | 690 | 701 |
| NM_012437 | SNAPIN | 0.06 | 0.00 | 270 | 340 | 747 | 811 |
| NM_007241 | SNF8 | 0.16 | -0.03 | 516 | 659 | 1348 | 1203 |
| NM_003498 | SNN | 0.10 | 0.00 | 325 | 414 | 799 | 884 |
| NM_022717 | SNRNP35 | 0.35 | 0.00 | 174 | 277 | 366 | 396 |
| RefSeq ID | Gene Symbol | SR GFOLD | SEN GFOLD | SR IL-2- | SR IL-2 + | SEN IL-2 - | SEN IL-2 + |
| NM_003090 | SNRPA1 | 0.47 | 0.00 | 91 | 170 | 125 | 129 |
| NM_003091 | SNRPB | 0.01 | -0.19 | 5364 | 5663 | 5415 | 4543 |
| NM_003093 | SNRPC | 0.19 | -0.03 | 448 | 593 | 851 | 741 |
| NM_177542 | SNRPD2 | 0.10 | -0.02 | 2155 | 2468 | 4706 | 4427 |
| NM_006750 | SNTB2 | 0.07 | 0.00 | 1911 | 2159 | 3463 | 3335 |
| NM_005701 | SNUPN | 0.47 | 0.00 | 380 | 614 | 928 | 996 |
| NM_001256188 | SNX12 | 0.12 | 0.00 | 1524 | 1797 | 2647 | 2680 |
| NM_013306 | SNX15 | 0.10 | 0.00 | 383 | 480 | 487 | 542 |
| NM_001102575 | SNX18 | 0.03 | 0.00 | 689 | 796 | 1944 | 1821 |
| NM_014758 | SNX19 | 0.45 | 0.00 | 1816 | 2667 | 4739 | 4930 |
| NM_001278199 | SNX2 | 0.06 | 0.00 | 715 | 839 | 1753 | 1808 |
| NM_014426 | SNX5 | 0.63 | 0.00 | 300 | 549 | 676 | 638 |
| NM_021249 | SNX6 | 0.11 | 0.00 | 1178 | 1395 | 2789 | 2806 |
| NM_015976 | SNX7 | 0.22 | 0.00 | 287 | 399 | 842 | 900 |
| NM_013321 | SNX8 | 0.27 | 0.00 | 708 | 957 | 2003 | 1863 |
| NM_003101 | SOAT1 | 0.13 | 0.00 | 991 | 1196 | 1730 | 1671 |
| NM_018013 | SOBP | 0.42 | 0.00 | 147 | 251 | 258 | 255 |
| NM_014598 | SOCS7 | 0.24 | 0.01 | 641 | 853 | 813 | 913 |
| NM_005775 | SORBS3 | 0.24 | 0.00 | 2230 | 2814 | 3367 | 3203 |
| NM_006943 | SOX12 | 0.40 | 0.00 | 288 | 452 | 699 | 712 |
| NM_005686 | SOX13 | 0.02 | 0.00 | 326 | 393 | 198 | 220 |
| NM_001025436 | SPAG16 | 0.53 | 0.00 | 55 | 116 | 171 | 159 |
| NM_006461 | SPAG5 | 0.19 | -0.74 | 1406 | 1748 | 1310 | 706 |
| NM_006038 | SPATA2 | 0.48 | 0.00 | 200 | 343 | 302 | 362 |
| NM_024063 | SPATA5L1 | 0.50 | 0.00 | 100 | 188 | 235 | 214 |
| NM_018418 | SPATA7 | 0.64 | 0.00 | 41 | 98 | 137 | 133 |

EP 3 303 636 B1

(continued)

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_001100423 | SPATS2L | 0.06 | 0.00 | 2522 | 2798 | 5170 | 5007 |
| NM_182513 | SPC24 | 0.62 | -0.83 | 97 | 199 | 235 | 100 |
| NM_020675 | SPC25 | 0.49 | -0.65 | 87 | 166 | 133 | 59 |
| NM_001004351 | SPDYE3 | 0.65 | 0.00 | 24 | 65 | 96 | 76 |
| NM_025137 | SPG11 | 0.08 | 0.00 | 836 | 989 | 2498 | 2628 |
| NM_016630 | SPG21 | 0.29 | 0.00 | 967 | 1309 | 2277 | 2216 |
| NM_003119 | SPG7 | 0.17 | 0.00 | 1539 | 1877 | 3621 | 3739 |
| NM_182965 | SPHK1 | 0.01 | -0.47 | 5455 | 5745 | 7699 | 5351 |
| NM_001080394 | SPIDR | 0.07 | -0.14 | 461 | 559 | 986 | 799 |
| NM_001010862 | SPIN3 | 0.78 | 0.00 | 38 | 101 | 181 | 214 |
| NM_144569 | SPOCD1 | 0.10 | -0.54 | 830 | 995 | 3261 | 2103 |
| NM_001007226 | SPOP | 0.16 | 0.00 | 728 | 910 | 1096 | 1037 |
| NM_001077238 | SPPL2B | 0.04 | 0.00 | 535 | 632 | 1002 | 1091 |
| NM_207344 | SPRYD4 | 0.32 | 0.00 | 138 | 221 | 260 | 279 |
| NM_020456 | SPRYD7 | 0.69 | 0.00 | 146 | 298 | 453 | 442 |
| NM_001281303 | SPTLC1 | 0.60 | 0.00 | 851 | 1424 | 2154 | 2174 |
| NM_021199 | SQRDL | 0.34 | 0.00 | 684 | 973 | 2263 | 2227 |
| NM_001047 | SRD5A1 | 0.06 | 0.00 | 91 | 130 | 360 | 396 |
| NM_001033117 | SRGAP3 | 0.12 | 0.00 | 47 | 78 | 183 | 205 |
| NM_003132 | SRM | 0.17 | 0.00 | 2324 | 2795 | 3034 | 3091 |
| NM_001130440 | SRP9 | 0.15 | 0.00 | 756 | 940 | 1318 | 1392 |
| NM_003137 | SRPK1 | 0.12 | 0.00 | 1360 | 1615 | 2693 | 2808 |
| NM_182692 | SRPK2 | 0.35 | 0.00 | 594 | 856 | 1252 | 1264 |
| NM_005839 | SRRM1 | 0.20 | -0.07 | 1724 | 2133 | 2466 | 2201 |
| NM_001078166 | SRSF1 | 0.02 | 0.00 | 2911 | 3131 | 3510 | 3529 |
| NM_001191005 | SRSF10 | 0.08 | 0.00 | 533 | 644 | 784 | 811 |
| NM_003016 | SRSF2 | 0.23 | 0.00 | 2213 | 2780 | 2381 | 2316 |
| NM_001031684 | SRSF7 | 0.03 | 0.00 | 1723 | 1905 | 1655 | 1638 |

(continued)

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_001009998 | SSBP4 | 0.02 | 0.00 | 118 | 159 | 281 | 270 |
| NM_018984 | SSH1 | 0.25 | -0.09 | 5186 | 6444 | 7975 | 7198 |
| NM_017857 | SSH3 | 0.29 | -0.10 | 153 | 238 | 780 | 641 |
| NM_003731 | SSNA1 | 0.57 | -0.07 | 722 | 1197 | 1926 | 1693 |
| NM_003145 | SSR2 | 0.29 | -0.08 | 3942 | 5062 | 6441 | 5854 |
| NM_007107 | SSR3 | 0.07 | 0.00 | 8407 | 9159 | 11186 | 11562 |
| NM_003146 | SSRP1 | 0.19 | -0.05 | 2681 | 3257 | 3741 | 3414 |
| NM_001278589 | ST13 | 0.15 | 0.00 | 934 | 1151 | 2161 | 2022 |
| NM_174963 | ST3GAL3 | 0.02 | 0.00 | 693 | 792 | 1083 | 1129 |
| NM_213618 | ST5 | 0.17 | -0.01 | 440 | 572 | 984 | 876 |
| NM_173216 | ST6GAL1 | 0.43 | 0.00 | 9 | 35 | 121 | 122 |
| NM_175039 | ST6GALNAC4 | 0.78 | -0.20 | 396 | 782 | 1572 | 1251 |
| NM_001286999 | ST6GALNAC6 | 0.09 | 0.00 | 1482 | 1708 | 4756 | 4991 |
| NM_021908 | ST7 | 0.24 | 0.00 | 366 | 506 | 638 | 706 |
| NM_015136 | STAB1 | 0.69 | -0.20 | 210 | 412 | 724 | 551 |
| NM_005862 | STAG1 | 0.05 | 0.00 | 1440 | 1623 | 1739 | 1837 |
| NM_001013841 | STAP2 | 0.23 | -0.24 | 57 | 98 | 133 | 81 |
| NM_178006 | STARD13 | 0.12 | 0.00 | 618 | 760 | 917 | 952 |
| NM_032016 | STARD3NL | 0.66 | -0.08 | 347 | 641 | 1040 | 882 |
| NM_001142503 | STARD8 | 0.19 | 0.00 | 290 | 395 | 599 | 606 |
| NM_007315 | STAT1 | 0.42 | -0.20 | 2776 | 3945 | 6961 | 5798 |
| NM_207342 | STEAP1B | 0.21 | -0.04 | 259 | 363 | 327 | 262 |
| NM_182915 | STEAP3 | 0.26 | -0.23 | 849 | 1128 | 3350 | 2682 |
| NM_001282936 | STIL | 0.02 | -0.20 | 517 | 604 | 485 | 359 |
| NM_006819 | STIP1 | 0.13 | 0.00 | 2986 | 3456 | 3945 | 3869 |
| NM_005990 | STK10 | 0.15 | 0.00 | 2498 | 2947 | 3369 | 3538 |
| NM_006281 | STK3 | 0.02 | 0.00 | 370 | 442 | 584 | 623 |
| NM_007271 | STK38 | 0.23 | 0.00 | 1143 | 1470 | 2600 | 2726 |
| NM_005563 | STMN1 | 0.10 | -0.54 | 2797 | 3175 | 3413 | 2203 |
| NM_001199214 | STMN2 | 0.54 | -1.62 | 5 | 26 | 946 | 261 |
| NM_004099 | STOM | 0.02 | -0.50 | 4540 | 4816 | 6430 | 4331 |

(continued)

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_001256677 | STOML1 | 0.24 | 0.00 | 293 | 412 | 828 | 924 |
| NM_013442 | STOML2 | 0.14 | -0.37 | 1807 | 2150 | 4018 | 2932 |
| NM_001271006 | STRA13 | 0.23 | -0.31 | 621 | 826 | 955 | 686 |
| NM_007178 | STRAP | 0.18 | 0.00 | 1721 | 2097 | 2812 | 2939 |
| NM_016930 | STX18 | 0.29 | -0.09 | 201 | 304 | 624 | 510 |
| NM_052874 | STX1B | 0.06 | 0.00 | 27 | 49 | 241 | 231 |
| NM_004853 | STX8 | 0.28 | 0.00 | 349 | 497 | 928 | 1000 |
| NM_178509 | STXBP4 | 0.03 | 0.00 | 99 | 137 | 241 | 279 |
| NM_018423 | STYK1 | 0.13 | 0.00 | 40 | 69 | 71 | 92 |
| NM_016086 | STYXL1 | 0.40 | 0.00 | 152 | 254 | 549 | 505 |
| NM_006713 | SUB1 | 0.10 | 0.00 | 568 | 694 | 1190 | 1152 |
| NM_003850 | SUCLA2 | 0.09 | 0.00 | 329 | 416 | 645 | 641 |
| NM_003849 | SUCLG1 | 0.42 | 0.00 | 457 | 703 | 1256 | 1187 |
| NM_003848 | SUCLG2 | 0.15 | -0.18 | 509 | 648 | 1065 | 847 |
| NM_001193311 | SUGCT | 0.22 | 0.00 | 51 | 89 | 297 | 342 |
| NM_001017392 | SUGP2 | 0.21 | -0.10 | 1536 | 1918 | 3361 | 2950 |
| NM_001128205 | SULF1 | 0.13 | -0.12 | 6706 | 7605 | 21270 | 19087 |
| NM_000456 | SUOX | 0.49 | -0.36 | 211 | 362 | 641 | 433 |
| NM_007192 | SUPT16H | 0.11 | 0.00 | 1709 | 1987 | 2450 | 2355 |
| NM_001280787 | SURF1 | 0.44 | 0.00 | 92 | 168 | 403 | 445 |
| NM_001278942 | SURF6 | 0.10 | -0.08 | 463 | 574 | 693 | 577 |
| NM_003173 | SUV39H1 | 0.11 | -0.32 | 353 | 448 | 391 | 261 |
| NM_001193424 | SUV39H2 | 0.04 | 0.00 | 161 | 211 | 210 | 196 |
| NM_001040011 | SWI5 | 0.03 | 0.00 | 453 | 537 | 1052 | 1044 |
| NM_033025 | SYDE1 | 0.31 | -0.16 | 2110 | 2793 | 2691 | 2253 |
| NM_030786 | SYNC | 0.11 | -0.10 | 2376 | 2729 | 6052 | 5397 |
| NM_001159677 | SYNCRIP | 0.19 | -0.11 | 1592 | 1967 | 2518 | 2183 |
| NM_003898 | SYNJ2 | 0.02 | 0.00 | 4019 | 4294 | 9271 | 9325 |
| NM_001242394 | SYTL3 | 0.19 | 0.00 | 581 | 754 | 1115 | 1092 |

(continued)

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_001114600 | SZRD1 | 0.37 | -0.05 | 4090 | 5541 | 6802 | 6320 |
| NM_015284 | SZT2 | 0.13 | 0.00 | 2763 | 3218 | 3357 | 3248 |
| NM_006342 | TACC3 | 0.25 | -0.66 | 815 | 1078 | 821 | 455 |
| NM_001488 | TADA2A | 0.22 | 0.00 | 194 | 280 | 345 | 298 |
| NM_006354 | TADA3 | 0.34 | -0.05 | 1229 | 1697 | 3296 | 3005 |
| NM_006284 | TAF10 | 0.14 | 0.00 | 657 | 817 | 2146 | 2017 |
| NM_153809 | TAF1L | 0.13 | 0.00 | 195 | 266 | 468 | 521 |
| NM_014409 | TAF5L | 0.54 | -0.10 | 370 | 627 | 653 | 532 |
| NM_001190415 | TAF6 | 0.47 | -0.13 | 763 | 1179 | 1568 | 1311 |
| NM_015975 | TAF9B | 0.28 | 0.00 | 139 | 217 | 225 | 264 |
| NM_001001522 | TAGLN | 0.57 | -0.44 | 34290 | 51712 | 62715 | 45611 |
| NM_006755 | TALDO1 | 0.09 | -0.09 | 6037 | 6707 | 11030 | 10065 |
| NM_001145909 | TANC1 | 0.24 | -0.04 | 1163 | 1505 | 3687 | 3403 |
| NM_024562 | TANGO6 | 0.57 | -0.10 | 648 | 1079 | 1491 | 1275 |
| NM_016151 | TAOK2 | 0.16 | 0.00 | 1416 | 1715 | 2934 | 2900 |
| NM_000116 | TAZ | 0.72 | 0.00 | 198 | 398 | 478 | 451 |
| NM_001204240 | TBC1D10A | 0.51 | 0.00 | 285 | 484 | 682 | 734 |
| NM_015527 | TBC1D10B | 0.21 | 0.00 | 1291 | 1629 | 2009 | 1983 |
| NM_015188 | TBC1D12 | 0.03 | 0.00 | 163 | 212 | 227 | 250 |
| NM_018201 | TBC1D13 | 0.28 | 0.00 | 1086 | 1447 | 2575 | 2673 |
| NM_019020 | TBC1D16 | 0.19 | 0.00 | 1250 | 1556 | 3488 | 3407 |
| NM_016495 | TBC1D7 | 0.59 | 0.00 | 368 | 644 | 965 | 985 |
| NM_015130 | TBC1D9 | 0.18 | 0.01 | 1294 | 1598 | 2323 | 2499 |
| NM_004607 | TBCA | 0.41 | 0.00 | 180 | 297 | 364 | 384 |
| NM_001079515 | TBCE | 0.27 | 0.00 | 419 | 585 | 1002 | 1044 |
| NM_001163435 | TBCK | 0.23 | 0.00 | 225 | 323 | 838 | 787 |
| NM_001139466 | TBL1X | 0.11 | -0.04 | 715 | 870 | 1666 | 1494 |
| NM_003194 | TBP | 0.97 | 0.00 | 197 | 469 | 478 | 497 |
| NM_004865 | TBPL1 | 0.58 | 0.00 | 87 | 176 | 204 | 192 |
| NM_001261834 | TBRG4 | 0.28 | 0.00 | 1437 | 1895 | 2338 | 2327 |

(continued)

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_001080508 | TBX18 | 0.19 | 0.00 | 524 | 684 | 570 | 636 |
| NM_153333 | TCEAL8 | 0.07 | -0.10 | 576 | 691 | 1304 | 1103 |
| NM_007108 | TCEB2 | 0.61 | 0.00 | 1166 | 1939 | 4860 | 4684 |
| NM_007109 | TCF19 | 0.09 | -0.37 | 1654 | 1905 | 1316 | 921 |
| NM_198392 | TCF21 | 1.22 | -1.91 | 17 | 73 | 69 | 6 |
| NM_003200 | TCF3 | 0.02 | 0.00 | 931 | 1048 | 1023 | 1116 |
| NM_006602 | TCFL5 | 0.55 | 0.00 | 168 | 308 | 655 | 593 |
| NM_030752 | TCP1 | 0.47 | 0.00 | 1439 | 2150 | 3103 | 2930 |
| NM_022171 | TCTA | 0.08 | 0.00 | 585 | 705 | 1666 | 1536 |
| NM_001146071 | TDRD3 | 0.39 | 0.00 | 141 | 236 | 351 | 372 |
| NM_001256661 | TEAD2 | 0.16 | -0.10 | 545 | 696 | 1065 | 895 |
| RefSeq ID | Gene Symbol | SR GFOLD | SEN GFOLD | SR IL-2 - | SR IL-2 + | SEN IL-2 - | SEN IL-2 + |
| NM_003213 | TEAD4 | 0.50 | -0.39 | 384 | 632 | 233 | 139 |
| NM_014844 | TECPR2 | 0.08 | 0.00 | 878 | 1030 | 2333 | 2216 |
| NM_138501 | TECR | 0.37 | 0.00 | 281 | 434 | 641 | 601 |
| NM_024683 | TEFM | 0.29 | 0.00 | 106 | 172 | 237 | 268 |
| NM_170754 | TENC1 | 0.13 | -0.08 | 597 | 744 | 1603 | 1388 |
| NM_017746 | TEX10 | 0.06 | -0.16 | 532 | 637 | 1298 | 1054 |
| NM_144582 | TEX261 | 0.09 | 0.00 | 1652 | 1899 | 3369 | 3444 |
| NM_001278195 | TEX264 | 0.78 | 0.00 | 641 | 1233 | 1672 | 1739 |
| NM_003201 | TFAM | 0.05 | 0.00 | 277 | 346 | 320 | 311 |
| NM_022366 | TFB2M | 0.14 | 0.00 | 192 | 263 | 310 | 370 |
| NM_007111 | TFDP1 | 0.15 | -0.01 | 1415 | 1708 | 1857 | 1702 |
| NM_016521 | TFDP3 | 0.76 | 0.00 | 30 | 83 | 96 | 76 |
| NM_007162 | TFEB | 0.51 | 0.00 | 38 | 85 | 123 | 144 |
| NM_015927 | TGFB1I1 | 0.25 | -0.16 | 1804 | 2303 | 3088 | 2590 |
| NM_001199514 | TGIF2 | 0.28 | 0.00 | 197 | 297 | 287 | 259 |
| NM_001199535 | TGIF2-C20orf24 | 0.64 | 0.00 | 598 | 1050 | 1564 | 1682 |
| NM_004613 | TGM2 | 0.13 | 0.00 | 2758 | 3199 | 4020 | 4135 |
| NM_001083953 | THADA | 0.43 | 0.00 | 541 | 831 | 1535 | 1449 |
| NM_018105 | THAP1 | 0.29 | 0.00 | 192 | 291 | 395 | 442 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_138350 | THAP3 | 0.17 | -0.20 | 485 | 629 | 844 | 651 |
| NM_015963 | THAP4 | 0.42 | 0.00 | 1020 | 1499 | 2477 | 2407 |
| NM_144721 | THAP6 | 0.05 | 0.00 | 111 | 153 | 243 | 287 |
| NM_001008695 | THAP7 | 0.24 | 0.00 | 209 | 305 | 316 | 294 |
| NM_152658 | THAP8 | 0.93 | 0.00 | 32 | 97 | 119 | 122 |
| NM_000361 | THBD | 0.30 | -0.06 | 381 | 547 | 2575 | 2307 |
| NM_003247 | THBS2 | 0.26 | -0.03 | 13115 | 16124 | 32251 | 31038 |
| NM_016647 | THEM6 | 0.18 | -0.01 | 147 | 214 | 239 | 190 |
| NM_017872 | THG1L | 0.09 | 0.00 | 142 | 195 | 218 | 220 |
| NM_024838 | THNSL1 | 0.30 | 0.00 | 40 | 77 | 148 | 172 |
| NM_032361 | THOC3 | 0.16 | 0.00 | 371 | 487 | 541 | 584 |
| NM_024339 | THOC6 | 0.18 | 0.00 | 365 | 487 | 339 | 390 |
| NM_003249 | THOP1 | 0.08 | -0.29 | 1244 | 1441 | 1965 | 1482 |
| NM_001190919 | THRA | 0.30 | 0.00 | 657 | 911 | 1456 | 1381 |
| NM_024817 | THSD4 | 0.02 | -0.53 | 790 | 897 | 1707 | 1081 |
| NM_024328 | THTPA | 0.25 | 0.00 | 142 | 217 | 422 | 377 |
| NM_017736 | THUMPD1 | 0.02 | 0.00 | 461 | 540 | 668 | 712 |
| NM_022173 | TIA1 | 0.03 | 0.00 | 371 | 447 | 734 | 673 |
| NM_004740 | TIAF1 | 0.36 | 0.00 | 123 | 206 | 803 | 871 |
| NM_012454 | TIAM2 | 0.20 | -0.31 | 456 | 607 | 1483 | 1091 |
| NM_182919 | TICAM1 | 0.63 | -0.38 | 443 | 786 | 961 | 658 |
| NM_012456 | TIMM10 | 0.46 | 0.00 | 264 | 435 | 551 | 534 |
| NM_001167947 | TIMM17B | 0.32 | -0.37 | 239 | 363 | 595 | 397 |
| NM_014177 | TIMM21 | 0.44 | 0.00 | 321 | 514 | 468 | 481 |
| NM_013337 | TIMM22 | 0.14 | 0.00 | 559 | 704 | 1177 | 1067 |
| NM_004085 | TIMM8A | 0.24 | 0.00 | 139 | 212 | 208 | 205 |
| NM_012460 | TIMM9 | 0.28 | 0.00 | 158 | 243 | 293 | 246 |
| NM_001146018 | TJAP1 | 0.21 | 0.00 | 463 | 617 | 690 | 675 |
| NM_003258 | TK1 | 0.31 | -0.43 | 1595 | 2129 | 1042 | 688 |
| NM_001135055 | TKT | 0.06 | -0.15 | 13833 | 14856 | 17057 | 14972 |
| NM_005077 | TLE1 | 0.08 | 0.00 | 332 | 417 | 853 | 945 |
| NM_001282748 | TLE4 | 0.01 | -0.10 | 1903 | 2067 | 1104 | 930 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001284333 | TLK2 | 0.12 | 0.00 | 249 | 328 | 476 | 499 |
| NM_015059 | TLN2 | 0.09 | -0.10 | 2058 | 2352 | 4839 | 4309 |
| NM_003266 | TLR4 | 0.04 | 0.00 | 1633 | 1822 | 1639 | 1697 |
| NM_078474 | TM2D3 | 0.05 | 0.00 | 271 | 338 | 638 | 699 |
| NM_014858 | TMCC2 | 0.15 | 0.00 | 159 | 225 | 272 | 253 |
| NM_181719 | TMCO4 | 0.63 | -0.34 | 323 | 586 | 537 | 362 |
| NM_018502 | TMCO6 | 0.34 | 0.00 | 106 | 178 | 191 | 205 |
| NM_001143842 | TMEM106C | 0.48 | 0.00 | 380 | 618 | 1142 | 1142 |
| NM_032354 | TMEM107 | 1.49 | -0.03 | 1693 | 5070 | 5099 | 4754 |
| NM_007024 | TMEM115 | 0.04 | 0.00 | 1570 | 1747 | 2570 | 2575 |
| NM_001193531 | TMEM116 | 0.41 | 0.00 | 5 | 24 | 77 | 107 |
| NM_052932 | TMEM123 | 0.06 | 0.00 | 2153 | 2406 | 2704 | 2869 |
| NM_001127266 | TMEM129 | 0.27 | 0.00 | 790 | 1059 | 2161 | 2322 |
| NM_052907 | TMEM132B | 0.61 | 0.00 | 30 | 75 | 210 | 205 |
| NM_001198670 | TMEM136 | 0.15 | 0.00 | 144 | 206 | 318 | 353 |
| NM_016464 | TMEM138 | 0.16 | 0.00 | 457 | 592 | 890 | 813 |
| NM_032928 | TMEM141 | 0.47 | -0.12 | 314 | 515 | 1210 | 1009 |
| NM_001242597 | TMEM147 | 0.53 | 0.00 | 824 | 1321 | 2514 | 2678 |
| NM_024789 | TMEM180 | 0.08 | 0.00 | 31 | 55 | 64 | 74 |
| NM_138391 | TMEM183A | 0.47 | 0.00 | 224 | 378 | 526 | 510 |
| NM_001079809 | TMEM183B | 0.33 | 0.00 | 222 | 341 | 499 | 481 |
| NM_001097620 | TMEM184A | 0.25 | 0.00 | 167 | 251 | 148 | 135 |
| NM_018279 | TMEM19 | 0.24 | 0.00 | 276 | 391 | 761 | 830 |
| NM_013390 | TMEM2 | 0.05 | 0.00 | 1787 | 1996 | 2396 | 2377 |
| NM_053045 | TMEM203 | 0.17 | -0.01 | 639 | 814 | 1414 | 1281 |
| NM_024600 | TMEM204 | 0.18 | 0.00 | 454 | 593 | 776 | 841 |
| NM_198536 | TMEM205 | 0.75 | 0.00 | 117 | 255 | 503 | 506 |
| NM_001258245 | TMEM218 | 0.05 | 0.00 | 79 | 115 | 289 | 311 |
| NM_001083613 | TMEM219 | 0.19 | 0.00 | 850 | 1080 | 2365 | 2401 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001009924 | TMEM230 | 0.04 | 0.00 | 1036 | 1177 | 2286 | 2445 |
| NM_019118 | TMEM234 | 0.09 | 0.00 | 120 | 169 | 399 | 347 |
| NM_001098621 | TMEM251 | 0.11 | 0.00 | 119 | 170 | 166 | 205 |
| NM_017799 | TMEM260 | 0.27 | 0.00 | 104 | 168 | 435 | 492 |
| NM_018112 | TMEM38B | 0.42 | 0.00 | 333 | 524 | 1000 | 1035 |
| NM_018056 | TMEM39B | 0.36 | -0.05 | 354 | 534 | 711 | 603 |
| NM_024334 | TMEM43 | 0.09 | 0.00 | 2900 | 3280 | 5139 | 5029 |
| NM_001136218 | TMEM51 | 0.49 | 0.00 | 94 | 177 | 351 | 388 |
| NM_024587 | TMEM53 | 0.38 | -0.01 | 40 | 81 | 285 | 231 |
| NM_018710 | TMEM55A | 0.51 | 0.00 | 232 | 400 | 622 | 636 |
| NM_001199691 | TMEM56-RWDD3 | 0.89 | 0.00 | 33 | 97 | 121 | 102 |
| NM_194291 | TMEM65 | 0.02 | 0.00 | 439 | 518 | 433 | 499 |
| NM_001286657 | TMEM68 | 0.40 | 0.00 | 142 | 239 | 279 | 335 |
| NM_014573 | TMEM97 | 0.40 | -0.08 | 219 | 353 | 482 | 388 |
| NM_015544 | TMEM98 | 0.09 | 0.00 | 513 | 627 | 1872 | 1987 |
| NM_014548 | TMOD2 | 0.37 | 0.00 | 285 | 439 | 1988 | 1954 |
| NM_001032283 | TMPO | 0.22 | -0.50 | 619 | 815 | 539 | 323 |
| NM_021109 | TMSB4X | 0.45 | -0.08 | 10426 | 14703 | 30300 | 28081 |
| NM_032813 | TMTC4 | 0.71 | -0.03 | 171 | 348 | 349 | 283 |
| NM_002160 | TNC | 0.26 | -1.03 | 3327 | 4199 | 13501 | 6370 |
| NM_021137 | TNFAIP1 | 0.24 | -0.18 | 2185 | 2763 | 3569 | 2981 |
| NM_006291 | TNFAIP2 | 0.19 | 0.00 | 645 | 832 | 1553 | 1519 |
| NM_152362 | TNFAIP8L1 | 0.81 | -0.10 | 54 | 137 | 185 | 133 |
| NM_001204848 | TNFAIP8L2-SCNM1 | 0.46 | 0.00 | 251 | 415 | 589 | 590 |
| NM_002546 | TNFRSF11B | 0.01 | -0.49 | 5506 | 5798 | 1795 | 1168 |
| NM_016639 | TNFRSF12A | 0.05 | -0.10 | 6207 | 6707 | 6863 | 6142 |
| NM_001065 | TNFRSF1A | 0.10 | 0.00 | 3288 | 3732 | 6480 | 6456 |
| NM_014452 | TNFRSF21 | 0.16 | 0.00 | 244 | 331 | 636 | 616 |
| NM_003823 | TNFRSF6B | 0.01 | 0.00 | 32 | 54 | 60 | 50 |
| NM_001161560 | TNIK | 0.23 | 0.00 | 133 | 202 | 318 | 377 |
| NM_022748 | TNS3 | 0.24 | 0.00 | 293 | 412 | 1556 | 1590 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_016272 | TOB2 | 0.13 | 0.00 | 1232 | 1476 | 2082 | 1996 |
| NM_020243 | TOMM22 | 0.34 | -0.34 | 731 | 1034 | 1884 | 1375 |
| NM_001134484 | TOMM5 | 0.45 | -0.04 | 479 | 749 | 911 | 791 |
| NM_001134493 | TOMM6 | 0.03 | -0.08 | 1043 | 1174 | 2389 | 2107 |
| NM_013432 | TONSL | 0.04 | 0.00 | 270 | 335 | 266 | 255 |
| RefSeq ID | Gene Symbol | SR GFOLD | SEN GFOLD | SR IL-2 - | SR IL-2 + | SEN IL-2 - | SEN IL-2 + |
| NM_007027 | TOPBP1 | 0.17 | 0.00 | 940 | 1173 | 1027 | 998 |
| NM_000113 | TOR1A | 0.27 | 0.01 | 1088 | 1437 | 2826 | 3017 |
| NM_130459 | TOR2A | 0.42 | 0.00 | 77 | 143 | 158 | 135 |
| NM_022371 | TOR3A | 0.31 | 0.00 | 273 | 405 | 539 | 617 |
| NM_014729 | TOX | 0.13 | 0.00 | 57 | 92 | 52 | 44 |
| NM_014828 | TOX4 | 0.30 | 0.00 | 1187 | 1601 | 2321 | 2353 |
| NM_001141979 | TP53BP1 | 0.42 | 0.00 | 1016 | 1490 | 3442 | 3641 |
| NM_001258320 | TP53I11 | 0.24 | 0.00 | 1071 | 1387 | 543 | 580 |
| NM_004881 | TP53I3 | 0.50 | -0.40 | 387 | 634 | 2269 | 1599 |
| NM_033550 | TP53RK | 0.35 | 0.00 | 238 | 367 | 661 | 593 |
| NM_033513 | TPGS1 | 0.66 | 0.00 | 19 | 55 | 56 | 63 |
| NM_213674 | TPM2 | 0.18 | -0.13 | 16559 | 19170 | 30082 | 26996 |
| NM_001278189 | TPM3 | 0.29 | -0.04 | 4631 | 5922 | 9286 | 8747 |
| NM_001145160 | TPM4 | 0.19 | -0.27 | 26570 | 30835 | 63316 | 51679 |
| NM_000367 | TPMT | 0.64 | 0.00 | 114 | 231 | 331 | 281 |
| NM_001136053 | TPRA1 | 0.14 | 0.00 | 1416 | 1693 | 1766 | 1749 |
| NM_001128228 | TPRN | 0.04 | 0.00 | 164 | 214 | 316 | 320 |
| NM_003595 | TPST2 | 0.38 | 0.00 | 961 | 1381 | 2263 | 2307 |
| NM_012112 | TPX2 | 0.41 | -1.02 | 1714 | 2454 | 2354 | 1068 |
| NM_001282759 | TRA2A | 0.18 | 0.00 | 534 | 693 | 1699 | 1621 |
| NM_021138 | TRAF2 | 0.09 | -0.15 | 380 | 476 | 422 | 320 |
| NM_032271 | TRAF7 | 0.17 | 0.00 | 3201 | 3802 | 5499 | 5647 |
| NM_005879 | TRAIP | 0.54 | -0.24 | 91 | 178 | 189 | 122 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001042646 | TRAK1 | 0.25 | -0.02 | 2483 | 3133 | 2190 | 2007 |
| NM_012288 | TRAM2 | 0.02 | 0.00 | 15850 | 16507 | 14745 | 14369 |
| NM_014831 | TRANK1 | 0.13 | 0.00 | 457 | 579 | 1250 | 1137 |
| NM_016292 | TRAP1 | 0.25 | 0.00 | 1934 | 2464 | 3363 | 3200 |
| NM_021210 | TRAPPC1 | 0.50 | 0.00 | 1033 | 1607 | 2866 | 3054 |
| NM_016030 | TRAPPC12 | 0.15 | 0.00 | 1136 | 1388 | 2125 | 2262 |
| NM_001011658 | TRAPPC2 | 0.25 | 0.00 | 75 | 125 | 146 | 163 |
| NM_001270896 | TRAPPC3 | 0.18 | 0.00 | 1202 | 1494 | 2152 | 2296 |
| NM_016146 | TRAPPC4 | 0.03 | 0.00 | 949 | 1074 | 1362 | 1292 |
| NM_001042461 | TRAPPC5 | 0.09 | 0.00 | 556 | 674 | 1574 | 1664 |
| NM_001160372 | TRAPPC9 | 0.39 | -0.01 | 409 | 623 | 1154 | 1039 |
| NM_145214 | TRIM 11 | 0.35 | 0.00 | 427 | 629 | 480 | 444 |
| NM_001037330 | TRIM16L | 0.24 | 0.00 | 88 | 142 | 345 | 394 |
| NM_003141 | TRIM21 | 0.31 | -0.23 | 353 | 513 | 684 | 510 |
| NM_001656 | TRIM23 | 0.18 | 0.00 | 386 | 511 | 576 | 654 |
| NM_003449 | TRIM26 | 0.02 | 0.00 | 1071 | 1196 | 1716 | 1747 |
| NM_006510 | TRIM27 | 0.30 | 0.00 | 1491 | 1994 | 2269 | 2290 |
| NM_001003827 | TRIM34 | 0.15 | 0.00 | 155 | 219 | 356 | 312 |
| NM_171982 | TRIM35 | 0.20 | 0.00 | 526 | 689 | 890 | 863 |
| NM_033092 | TRIM5 | 0.26 | 0.00 | 686 | 923 | 595 | 566 |
| NM_001003819 | TRIM6-TRIM34 | 0.08 | -0.03 | 276 | 352 | 545 | 462 |
| NM_173547 | TRIM65 | 0.24 | -0.02 | 410 | 563 | 497 | 421 |
| NM_018073 | TRIM68 | 0.06 | -0.14 | 259 | 328 | 433 | 331 |
| NM_003302 | TRIP6 | 0.18 | -0.42 | 1387 | 1710 | 2899 | 2020 |
| NM_017819 | TRMT10C | 0.47 | 0.00 | 152 | 267 | 370 | 408 |
| NM_001286082 | TRMT112 | 0.38 | -0.30 | 753 | 1097 | 1699 | 1270 |
| NM_017956 | TRMT12 | 0.23 | 0.00 | 156 | 233 | 285 | 303 |
| NM_001257994 | TRMT2A | 0.43 | -0.04 | 621 | 944 | 1300 | 1150 |
| NM_152307 | TRMT61A | 0.10 | 0.00 | 279 | 359 | 439 | 453 |
| NM_017910 | TRMT61B | 0.25 | -0.23 | 149 | 226 | 308 | 214 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_018006 | TRMU | 0.37 | 0.00 | 290 | 447 | 420 | 445 |
| NM_001013642 | TRNP1 | 0.19 | 0.00 | 345 | 464 | 1219 | 1146 |
| NM_003306 | TRPC4 | 0.30 | -1.13 | 948 | 1285 | 1004 | 399 |
| NM_016113 | TRPV2 | 0.37 | -0.33 | 2156 | 2966 | 9808 | 7508 |
| NM_021625 | TRPV4 | 0.30 | 0.00 | 130 | 207 | 437 | 486 |
| NM_000368 | TSC1 | 0.13 | -0.08 | 1217 | 1458 | 3363 | 3009 |
| NM_030935 | TSC22D4 | 0.40 | 0.00 | 588 | 878 | 1034 | 963 |
| NM_025265 | TSEN2 | 0.45 | 0.00 | 58 | 115 | 210 | 201 |
| NM_207346 | TSEN54 | 0.05 | -0.05 | 229 | 292 | 220 | 168 |
| NM_020856 | TSHZ3 | 0.20 | 0.00 | 1448 | 1801 | 2408 | 2377 |
| NM_130783 | TSPAN18 | 0.95 | 0.00 | 29 | 91 | 37 | 54 |
| NM_021648 | TSPYL4 | 0.14 | 0.00 | 554 | 697 | 1021 | 996 |
| NM_003310 | TSSC1 | 0.25 | 0.00 | 410 | 568 | 1065 | 1009 |
| NM_005706 | TSSC4 | 0.26 | 0.00 | 370 | 518 | 761 | 704 |
| NM_052841 | TSSK3 | 0.44 | -0.08 | 24 | 57 | 48 | 26 |
| NM_003313 | TSTA3 | 0.03 | -0.06 | 918 | 1041 | 1624 | 1434 |
| NM_001282500 | TTC1 | 0.27 | -0.07 | 710 | 960 | 1583 | 1381 |
| NM_017868 | TTC12 | 0.54 | 0.00 | 18 | 49 | 162 | 142 |
| NM_001288615 | TTC23 | 0.03 | 0.00 | 378 | 454 | 593 | 678 |
| NM_024926 | TTC26 | 0.15 | 0.00 | 53 | 88 | 208 | 179 |
| NM_017735 | TTC27 | 0.07 | 0.00 | 374 | 463 | 576 | 547 |
| NM_001145418 | TTC28 | 0.22 | 0.00 | 798 | 1036 | 1736 | 1702 |
| NM_001008237 | TTC32 | 0.05 | 0.00 | 31 | 54 | 35 | 33 |
| NM_001080494 | TTC39A | 2.49 | 0.00 | 1 | 39 | 133 | 146 |
| NM_004623 | TTC4 | 0.21 | 0.00 | 443 | 592 | 894 | 810 |

| Table 5A: Differentially Upregulated Genes in SR + IL-2 | | | | | | | |
|---|---|---|---|---|---|---|---|
| RefSeq ID | Gene Symbol | SR GFOLD | SEN GFOLD | SR IL- 2 - | SR IL-2 + | SEN IL-2 - | SEN IL-2 + |
| NM_138376 | TTC5 | 0.66 | 0.00 | 125 | 254 | 422 | 444 |
| NM_173810 | TTC9C | 0.07 | 0.00 | 477 | 579 | 759 | 719 |
| NM_003594 | TTF2 | 0.08 | -0.20 | 631 | 754 | 728 | 555 |
| NM_014657 | TTI1 | 0.11 | 0.00 | 520 | 645 | 736 | 817 |
| NM_025115 | TTI2 | 0.18 | 0.00 | 551 | 712 | 645 | 645 |

(continued)

| | Table 5A: Differentially Upregulated Genes in SR + IL-2 | | | | | | |
|---|---|---|---|---|---|---|---|
| RefSeq ID | Gene Symbol | SR GFOLD | SEN GFOLD | SR IL- 2 - | SR IL-2 + | SEN IL-2 - | SEN IL-2 + |
| NM_001166691 | TTK | 0.15 | -0.92 | 255 | 343 | 335 | 140 |
| NM_015140 | TTLL12 | 0.06 | 0.00 | 1332 | 1511 | 1957 | 1811 |
| NM_014640 | TTLL4 | 0.61 | 0.00 | 402 | 707 | 530 | 481 |
| NM_025250 | TTYH3 | 0.19 | -0.32 | 2562 | 3105 | 3136 | 2364 |
| NM_001270399 | TUBA1A | 0.67 | -0.43 | 5700 | 9409 | 17311 | 12504 |
| NM_006082 | TUBA1B | 0.49 | -0.64 | 24029 | 34465 | 32484 | 20447 |
| NM_032704 | TUBA1C | 0.39 | -0.64 | 15529 | 20794 | 21822 | 13643 |
| NM_178014 | TUBB | 0.48 | -0.35 | 30537 | 43320 | 56651 | 43657 |
| NM_001069 | TUBB2A | 0.27 | -0.19 | 2069 | 2675 | 4157 | 3462 |
| NM_178012 | TUBB2B | 0.56 | -0.43 | 541 | 903 | 1657 | 1126 |
| NM_006086 | TUBB3 | 0.19 | -0.39 | 1065 | 1341 | 8198 | 6004 |
| NM_001289130 | TUBB4A | 1.77 | -0.90 | 13 | 89 | 116 | 41 |
| NM_006088 | TUBB4B | 0.30 | -0.52 | 12969 | 16445 | 15350 | 10364 |
| NM_032525 | TUBB6 | 0.42 | -0.36 | 17083 | 23402 | 33459 | 25606 |
| NM_001070 | TUBG1 | 0.75 | -0.17 | 964 | 1778 | 2236 | 1850 |
| NM_052903 | TUBGCP5 | 0.08 | 0.00 | 598 | 717 | 832 | 880 |
| NM_001004125 | TUSC1 | 0.65 | 0.00 | 154 | 304 | 262 | 288 |
| NM_022830 | TUT1 | 0.13 | 0.00 | 320 | 417 | 439 | 442 |
| NM_007284 | TWF2 | 0.03 | 0.00 | 1467 | 1625 | 2795 | 2813 |
| NM_001271893 | TWIST2 | 0.51 | 0.00 | 407 | 671 | 998 | 978 |
| NM_001002926 | TWISTNB | 0.10 | -0.01 | 310 | 396 | 568 | 488 |
| NM_012473 | TXN2 | 0.23 | -0.06 | 642 | 853 | 1587 | 1401 |
| NM_001160047 | TXNDC16 | 0.15 | 0.00 | 56 | 92 | 152 | 133 |
| NM_005783 | TXNDC9 | 0.10 | 0.00 | 308 | 393 | 668 | 747 |
| NM_017853 | TXNL4B | 0.21 | 0.00 | 138 | 206 | 210 | 207 |
| NM_006293 | TYRO3 | 0.03 | 0.00 | 302 | 369 | 890 | 948 |
| NM_001145440 | TYW1B | 0.23 | 0.00 | 52 | 91 | 106 | 128 |
| NM_001012478 | U2AF2 | 0.10 | 0.00 | 2784 | 3175 | 5555 | 5506 |
| NM_207309 | UAP1L1 | 0.21 | 0.00 | 844 | 1086 | 2410 | 2257 |
| NM_016172 | UBAC1 | 0.30 | -0.05 | 715 | 985 | 1994 | 1786 |

(continued)

| Table 5A: Differentially Upregulated Genes in SR + IL-2 | | | | | | | |
|---|---|---|---|---|---|---|---|
| RefSeq ID | Gene Symbol | SR GFOLD | SEN GFOLD | SR IL- 2 - | SR IL-2 + | SEN IL-2 - | SEN IL-2 + |
| NM_018449 | UBAP2 | 0.20 | -0.04 | 1613 | 2004 | 2304 | 2092 |
| NM_014847 | UBAP2L | 0.08 | -0.03 | 4869 | 5372 | 7433 | 6989 |
| NM_032873 | UBASH3B | 0.05 | -0.01 | 1408 | 1585 | 1501 | 1368 |
| NM_018955 | UBB | 0.85 | 0.00 | 4272 | 8061 | 21607 | 21136 |
| NM_181838 | UBE2D2 | 0.32 | 0.00 | 746 | 1041 | 1514 | 1584 |
| NM_181886 | UBE2D3 | 0.20 | 0.00 | 2310 | 2834 | 4839 | 4909 |
| NM_015983 | UBE2D4 | 0.11 | -0.05 | 186 | 252 | 622 | 523 |
| NM_194458 | UBE2J2 | 0.01 | 0.00 | 737 | 836 | 903 | 867 |
| NM_003969 | UBE2M | 0.24 | 0.00 | 424 | 579 | 688 | 699 |
| NM_003348 | UBE2N | 0.54 | 0.00 | 743 | 1208 | 1526 | 1495 |
| NM_001012989 | UBE2NL | 0.80 | 0.00 | 66 | 161 | 204 | 240 |
| NM_014501 | UBE2S | 0.37 | -0.50 | 597 | 871 | 761 | 468 |
| NM_014176 | UBE2T | 0.18 | -0.41 | 255 | 350 | 297 | 179 |
| NM_001204077 | UBE4A | 0.12 | 0.00 | 1268 | 1510 | 2924 | 3030 |
| NM_001267584 | UBOX5 | 0.29 | 0.00 | 230 | 342 | 489 | 471 |
| NM_175748 | UBR7 | 0.51 | 0.00 | 494 | 801 | 913 | 821 |
| NM_015853 | UBXN1 | 0.38 | 0.00 | 742 | 1079 | 1872 | 1954 |
| NM_183008 | UBXN11 | 0.42 | 0.00 | 120 | 209 | 264 | 226 |
| NM_014607 | UBXN4 | 0.03 | 0.00 | 1176 | 1319 | 1932 | 2042 |
| NM_006002 | UCHL3 | 0.33 | 0.00 | 327 | 484 | 830 | 889 |
| NM_001261450 | UCK1 | 0.10 | -0.06 | 461 | 572 | 977 | 843 |
| NM_012474 | UCK2 | 0.25 | 0.00 | 1534 | 1973 | 1938 | 2022 |
| NM_001040697 | UEVLD | 0.32 | 0.00 | 195 | 301 | 520 | 534 |
| NM_001048201 | UHRF1 | 0.15 | -0.71 | 1218 | 1481 | 547 | 283 |
| NM_017754 | UHRF1BP1 | 0.34 | 0.01 | 470 | 686 | 680 | 773 |
| NM_001199297 | UIMC1 | 0.05 | 0.00 | 436 | 523 | 455 | 447 |
| NM_001039675 | UNC45A | 0.08 | 0.00 | 2434 | 2751 | 5430 | 5419 |
| NM_001080419 | UNK | 0.66 | -0.21 | 225 | 430 | 672 | 506 |
| NM_001114403 | UPK3BL | 0.13 | 0.00 | 118 | 170 | 285 | 287 |

(continued)

| Table 5A: Differentially Upregulated Genes in SR + IL-2 | | | | | | | |
|---|---|---|---|---|---|---|---|
| RefSeq ID | Gene Symbol | SR GFOLD | SEN GFOLD | SR IL- 2 - | SR IL-2 + | SEN IL-2 - | SEN IL-2 + |
| NM_001003684 | UQCR10 | 0.10 | -0.29 | 792 | 952 | 1955 | 1471 |
| NM_006830 | UQCR11 | 0.25 | 0.00 | 769 | 1023 | 1811 | 1856 |
| NM_003365 | UQCRC1 | 0.20 | 0.00 | 2817 | 3437 | 6975 | 6798 |
| NM_003366 | UQCRC2 | 0.22 | -0.01 | 1675 | 2101 | 3250 | 3039 |
| NM_006003 | UQCRFS1 | 0.51 | 0.00 | 900 | 1411 | 1840 | 1817 |
| NM_006004 | UQCRH | 0.53 | 0.00 | 458 | 760 | 1056 | 1122 |
| NM_014402 | UQCRQ | 0.50 | 0.00 | 949 | 1477 | 2365 | 2495 |
| NM_001077663 | URGCP | 0.02 | 0.00 | 1186 | 1321 | 1562 | 1532 |
| NM_001204871 | URGCP-MRPS24 | 0.24 | 0.00 | 1171 | 1512 | 1685 | 1567 |
| NM_001265582 | URM1 | 0.33 | -0.36 | 925 | 1282 | 2562 | 1863 |
| NM_024598 | USB1 | 0.10 | 0.00 | 855 | 1024 | 1206 | 1139 |
| NM_001272075 | USP10 | 0.03 | -0.01 | 1256 | 1401 | 2250 | 2081 |
| NM_004651 | USP11 | 0.24 | 0.00 | 1374 | 1767 | 2290 | 2233 |
| NM_001252078 | USP15 | 0.08 | 0.00 | 625 | 748 | 1248 | 1305 |
| NM_001014443 | USP21 | 0.13 | 0.00 | 295 | 386 | 399 | 394 |
| NM_020886 | USP28 | 0.10 | 0.00 | 399 | 499 | 661 | 627 |
| NM_020718 | USP31 | 0.08 | 0.00 | 1300 | 1498 | 1215 | 1296 |
| NM_025090 | USP36 | 0.10 | 0.00 | 1651 | 1919 | 2556 | 2586 |
| NM_001290326 | USP38 | 0.22 | 0.00 | 767 | 998 | 1314 | 1327 |
| NM_018218 | USP40 | 0.05 | -0.10 | 483 | 577 | 1510 | 1294 |
| NM_032236 | USP48 | 0.12 | 0.00 | 775 | 941 | 1310 | 1364 |
| NM_001098536 | USP5 | 0.25 | 0.00 | 2373 | 3009 | 6711 | 6870 |
| NM_152586 | USP54 | 0.32 | 0.00 | 101 | 169 | 399 | 420 |
| NM_001128610 | USP8 | 0.16 | 0.00 | 710 | 890 | 1200 | 1209 |
| NM_006649 | UTP14A | 0.34 | -0.25 | 273 | 415 | 526 | 379 |
| NM_032175 | UTP15 | 0.11 | -0.15 | 320 | 410 | 534 | 412 |
| NM_016001 | UTP18 | 0.35 | -0.16 | 171 | 274 | 416 | 314 |
| NM_014503 | UTP20 | 0.03 | -0.16 | 862 | 978 | 1356 | 1107 |
| NM_018428 | UTP6 | 0.26 | 0.00 | 452 | 623 | 794 | 850 |

(continued)

| Table 5A: Differentially Upregulated Genes in SR + IL-2 | | | | | | | |
| --- | --- | --- | --- | --- | --- | --- | --- |
| RefSeq ID | Gene Symbol | SR GFOLD | SEN GFOLD | SR IL- 2 - | SR IL-2 + | SEN IL-2 - | SEN IL-2 + |
| NM_003369 | UVRAG | 0.63 | 0.00 | 357 | 643 | 986 | 893 |
| NM_153477 | UXT | 0.32 | 0.00 | 559 | 792 | 1233 | 1281 |
| NM_018052 | VAC14 | 0.33 | 0.00 | 2101 | 2826 | 6308 | 6305 |
| NM_199245 | VAMP1 | 0.59 | 0.00 | 89 | 181 | 235 | 237 |
| NM_001185127 | VAMP4 | 0.56 | 0.00 | 140 | 263 | 428 | 482 |
| NM_001172411 | VANGL1 | 0.04 | 0.00 | 1244 | 1405 | 1637 | 1680 |
| NM_006295 | VARS | 0.20 | 0.00 | 2673 | 3256 | 5255 | 5464 |
| NM_001167734 | VARS2 | 0.26 | 0.00 | 443 | 613 | 774 | 832 |
| NM_003370 | VASP | 0.47 | -0.20 | 1454 | 2178 | 3876 | 3200 |
| NM_001134398 | VAV2 | 0.13 | 0.00 | 994 | 1200 | 1913 | 1859 |
| NM_016206 | VGLL3 | 0.06 | 0.00 | 6394 | 6947 | 5734 | 5865 |
| NM_001128219 | VGLL4 | 0.24 | 0.00 | 1072 | 1394 | 1379 | 1482 |
| NM_001193314 | VIPAS39 | 0.32 | 0.00 | 273 | 408 | 715 | 747 |
| NM_001017921 | VMAC | 0.15 | 0.00 | 13 | 32 | 79 | 61 |
| NM_017684 | VPS13C | 0.01 | 0.00 | 1315 | 1450 | 3918 | 3871 |
| NM_015378 | VPS13D | 0.14 | 0.00 | 1884 | 2226 | 4571 | 4724 |
| NM_020857 | VPS18 | 0.39 | -0.01 | 1586 | 2238 | 3702 | 3492 |
| NM_016208 | VPS28 | 0.13 | 0.00 | 1166 | 1398 | 2901 | 2880 |
| NM_001282168 | VPS36 | 0.14 | 0.00 | 439 | 560 | 1031 | 946 |
| NM_152415 | VPS37A | 0.64 | 0.00 | 566 | 994 | 1158 | 1211 |
| NM_007259 | VPS45 | 0.18 | 0.00 | 476 | 623 | 1273 | 1196 |
| NM_013265 | VPS51 | 0.11 | 0.00 | 774 | 938 | 1381 | 1423 |
| NM_001271087 | VPS72 | 0.07 | -0.28 | 892 | 1042 | 1352 | 1011 |
| NM_016440 | VRK3 | 0.39 | -0.14 | 439 | 664 | 1029 | 839 |
| NM_019086 | VSIG10 | 0.23 | 0.00 | 359 | 493 | 655 | 728 |
| NM_016485 | VTA1 | 0.18 | 0.00 | 912 | 1142 | 1520 | 1399 |
| NM_015058 | VWA8 | 0.04 | 0.00 | 368 | 445 | 1217 | 1183 |
| NM_003931 | WASF1 | 0.02 | 0.00 | 338 | 406 | 476 | 464 |
| NM_016312 | WBP11 | 0.09 | 0.00 | 1241 | 1446 | 1379 | 1266 |

(continued)

| Table 5A: Differentially Upregulated Genes in SR + IL-2 | | | | | | | |
|---|---|---|---|---|---|---|---|
| RefSeq ID | Gene Symbol | SR GFOLD | SEN GFOLD | SR IL- 2 - | SR IL-2 + | SEN IL-2 - | SEN IL-2 + |
| NM_001083913 | WBP1L | 0.35 | 0.00 | 1202 | 1670 | 2527 | 2656 |
| NM_152559 | WBSCR27 | 0.15 | -0.47 | 62 | 100 | 164 | 87 |
| NM_020830 | WDFY1 | 0.16 | -0.16 | 2012 | 2405 | 3664 | 3098 |
| NM_052950 | WDFY2 | 0.64 | 0.00 | 609 | 1067 | 1393 | 1420 |
| NM_015910 | WDPCP | 0.80 | 0.00 | 55 | 138 | 189 | 216 |
| NM_024100 | WDR18 | 0.18 | -0.40 | 293 | 397 | 626 | 410 |
| NM_018383 | WDR33 | 0.15 | 0.00 | 973 | 1192 | 1402 | 1442 |
| NM_052844 | WDR34 | 0.48 | -0.43 | 276 | 459 | 969 | 641 |
| NM_001260474 | WDR4 | 0.22 | -0.14 | 445 | 598 | 510 | 396 |
| NM_001029896 | WDR45 | 0.16 | 0.00 | 310 | 412 | 894 | 900 |
| NM_017588 | WDR5 | 0.14 | -0.20 | 616 | 769 | 994 | 776 |
| NM_001083961 | WDR62 | 0.49 | -0.16 | 439 | 710 | 462 | 351 |
| NM_001178003 | WDR66 | 0.13 | 0.00 | 318 | 413 | 110 | 129 |
| NM_015285 | WDR7 | 0.04 | 0.00 | 722 | 835 | 1348 | 1440 |
| NM_032856 | WDR73 | 0.09 | 0.00 | 174 | 233 | 453 | 518 |
| NM_032168 | WDR75 | 0.33 | 0.00 | 547 | 783 | 892 | 911 |
| NM_024102 | WDR77 | 0.33 | -0.06 | 746 | 1049 | 1911 | 1702 |
| NM_001099737 | WDR83 | 0.23 | 0.00 | 409 | 559 | 1044 | 1091 |
| NM_001258272 | WDR89 | 0.05 | 0.00 | 115 | 158 | 127 | 155 |
| NM_133330 | WHSC1 | 0.10 | -0.26 | 4227 | 4755 | 3920 | 3096 |
| NM_001077269 | WIPF1 | 0.14 | 0.00 | 1912 | 2268 | 3045 | 3168 |
| NM_003882 | WISP1 | 0.87 | 0.00 | 38 | 107 | 175 | 146 |
| NM_032387 | WNK4 | 0.12 | -0.18 | 3386 | 3883 | 2441 | 2007 |
| NM_017818 | WRAP73 | 0.24 | 0.00 | 293 | 413 | 505 | 473 |
| NM_001080436 | WTIP | 0.45 | -0.04 | 684 | 1050 | 821 | 710 |
| NM_015691 | WWC3 | 0.03 | 0.00 | 1602 | 1771 | 1406 | 1433 |
| NM_020196 | XAB2 | 0.12 | 0.00 | 1500 | 1764 | 1909 | 1994 |
| NM_004628 | XPC | 0.23 | 0.00 | 921 | 1200 | 1678 | 1678 |
| NM_022459 | XPO4 | 0.25 | 0.00 | 457 | 628 | 984 | 945 |
| NM_006297 | XRCC1 | 0.15 | 0.00 | 535 | 680 | 828 | 738 |

(continued)

| Table 5A: Differentially Upregulated Genes in SR + IL-2 | | | | | | | |
|---|---|---|---|---|---|---|---|
| RefSeq ID | Gene Symbol | SR GFOLD | SEN GFOLD | SR IL- 2 - | SR IL-2 + | SEN IL-2 - | SEN IL-2 + |
| NM_021141 | XRCC5 | 0.06 | -0.13 | 5681 | 6182 | 9774 | 8660 |
| NM_001288976 | XRCC6 | 0.15 | 0.00 | 3089 | 3626 | 5241 | 5074 |
| NM_022167 | XYLT2 | 0.24 | -0.03 | 1661 | 2111 | 2818 | 2588 |
| NM_020192 | YAE1D1 | 0.78 | 0.00 | 58 | 143 | 129 | 157 |
| RefSeq ID | Gene Symbol | SR GFOLD | SEN GFOLD | SR IL-2- | SR IL-2 + | SEN IL-2 - | SEN IL-2 + |
| NM_001190979 | YAF2 | 0.14 | 0.00 | 229 | 308 | 493 | 495 |
| NM_003680 | YARS | 0.18 | 0.00 | 2791 | 3358 | 5796 | 6033 |
| NM_001017964 | YDJC | 0.22 | 0.00 | 162 | 239 | 329 | 362 |
| NM_020470 | YIF1A | 0.47 | 0.00 | 1473 | 2207 | 3228 | 3298 |
| NM_019589 | YLPM1 | 0.06 | 0.00 | 1861 | 2094 | 2808 | 2900 |
| NM_024640 | YRDC | 0.35 | 0.00 | 437 | 645 | 557 | 481 |
| NM_006761 | YWHAE | 0.34 | -0.06 | 6442 | 8456 | 13305 | 12358 |
| NM_175907 | ZADH2 | 0.57 | 0.00 | 347 | 603 | 938 | 845 |
| NM_001287603 | ZBTB17 | 0.11 | 0.00 | 579 | 714 | 732 | 786 |
| NM_006352 | ZBTB18 | 0.13 | 0.00 | 572 | 713 | 772 | 769 |
| NM_020861 | ZBTB2 | 0.27 | 0.01 | 475 | 658 | 742 | 839 |
| NM_020924 | ZBTB26 | 0.97 | 0.00 | 22 | 75 | 89 | 111 |
| NM_014830 | ZBTB39 | 0.02 | 0.00 | 285 | 348 | 343 | 397 |
| NM_006626 | ZBTB6 | 0.28 | 0.00 | 187 | 282 | 310 | 336 |
| NM_016010 | ZC2HC1A | 0.57 | 0.00 | 95 | 189 | 349 | 340 |
| NM_033390 | ZC3H12C | 0.17 | 0.00 | 267 | 362 | 665 | 688 |
| NM_001160103 | ZC3H14 | 0.09 | 0.00 | 1181 | 1380 | 2360 | 2508 |
| NM_015117 | ZC3H3 | 0.35 | 0.00 | 319 | 482 | 740 | 652 |
| NM_001282190 | ZC3HC1 | 0.35 | 0.00 | 274 | 419 | 543 | 490 |
| NM_017665 | ZCCHC10 | 0.70 | 0.00 | 79 | 175 | 129 | 137 |
| NM_024617 | ZCCHC6 | 0.04 | 0.00 | 962 | 1093 | 1778 | 1778 |
| NM_017612 | ZCCHC8 | 0.10 | 0.00 | 320 | 407 | 391 | 384 |
| NM_032280 | ZCCHC9 | 0.37 | 0.00 | 171 | 276 | 299 | 259 |
| NM_033114 | ZCRB1 | 0.26 | 0.00 | 97 | 157 | 329 | 381 |
| NM_024630 | ZDHHC14 | 0.01 | 0.00 | 165 | 212 | 360 | 312 |
| NM_032283 | ZDHHC18 | 0.09 | 0.00 | 1202 | 1400 | 1194 | 1309 |

(continued)

| RefSeq ID | Gene Symbol | SR GFOLD | SEN GFOLD | SR IL-2- | SR IL-2 + | SEN IL-2 - | SEN IL-2 + |
|---|---|---|---|---|---|---|---|
| NM_207340 | ZDHHC24 | 0.54 | 0.00 | 77 | 155 | 499 | 451 |
| NM_001134387 | ZDHHC4 | 0.35 | 0.00 | 354 | 528 | 913 | 932 |
| NM_001170796 | ZFAND1 | 0.38 | 0.00 | 149 | 247 | 241 | 288 |
| NM_001282905 | ZFAND4 | 0.49 | 0.00 | 61 | 123 | 135 | 139 |
| NM_153688 | ZFP1 | 0.62 | 0.00 | 52 | 117 | 212 | 218 |
| NM_003407 | ZFP36 | 0.01 | -0.41 | 1190 | 1319 | 1102 | 745 |
| NM_153813 | ZFPM1 | 0.10 | 0.00 | 66 | 102 | 98 | 129 |
| NM_001281734 | ZFYVE1 | 0.13 | 0.00 | 651 | 807 | 1643 | 1671 |
| NM_001105251 | ZFYVE16 | 0.11 | 0.00 | 425 | 535 | 955 | 1026 |
| NM_001077268 | ZFYVE19 | 0.44 | 0.01 | 407 | 639 | 676 | 769 |
| NM_015346 | ZFYVE26 | 0.31 | 0.00 | 1428 | 1918 | 3049 | 3231 |
| NM_001204180 | ZHX1-C8orf76 | 0.16 | 0.00 | 241 | 327 | 416 | 414 |
| NM_003412 | ZIC1 | 0.29 | -0.28 | 188 | 285 | 437 | 303 |
| RefSeq ID | Gene Symbol | SR GFOLD | SEN GFOLD | SR IL-2 - | SR IL-2 + | SEN IL-2 - | SEN IL-2 + |
| NM_001012981 | ZKSCAN2 | 0.22 | 0.00 | 200 | 288 | 441 | 379 |
| NM_001242894 | ZKSCAN3 | 0.21 | 0.00 | 97 | 152 | 206 | 194 |
| NM_005857 | ZMPSTE24 | 0.17 | 0.00 | 769 | 971 | 1888 | 1981 |
| NM_001289091 | ZMYM1 | 0.52 | 0.00 | 171 | 305 | 435 | 440 |
| NM_201599 | ZMYM3 | 0.17 | 0.00 | 845 | 1058 | 1747 | 1778 |
| NM_005095 | ZMYM4 | 0.04 | 0.00 | 1062 | 1203 | 2419 | 2329 |
| NM_173531 | ZNF100 | 0.88 | 0.00 | 34 | 99 | 81 | 68 |
| NM_016265 | ZNF12 | 0.57 | 0.00 | 159 | 297 | 430 | 464 |
| NM_003434 | ZNF133 | 0.25 | -0.23 | 244 | 351 | 510 | 372 |
| NM_021030 | ZNF14 | 0.25 | 0.00 | 27 | 55 | 98 | 109 |
| NM_144680 | ZNF18 | 0.28 | 0.00 | 106 | 171 | 266 | 287 |
| NM_007149 | ZNF184 | 0.24 | 0.00 | 94 | 151 | 185 | 189 |
| NM_197977 | ZNF189 | 0.04 | 0.00 | 171 | 223 | 503 | 569 |
| NM_001242841 | ZNF195 | 0.33 | 0.00 | 130 | 211 | 285 | 266 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001017396 | ZNF2 | 0.47 | 0.00 | 66 | 130 | 225 | 185 |
| NM_001203250 | ZNF20 | 0.14 | 0.00 | 24 | 47 | 154 | 148 |
| NM_001278158 | ZNF205 | 0.17 | 0.00 | 141 | 205 | 225 | 209 |
| NM_001098507 | ZNF207 | 0.10 | 0.00 | 1814 | 2093 | 2747 | 2900 |
| NM_013359 | ZNF221 | 0.19 | 0.00 | 25 | 50 | 89 | 98 |
| NM_013362 | ZNF225 | 0.35 | 0.00 | 53 | 100 | 152 | 196 |
| NM_001099282 | ZNF239 | 0.37 | -0.02 | 52 | 100 | 214 | 166 |
| NM_001278677 | ZNF254 | 0.58 | 0.00 | 64 | 136 | 191 | 226 |
| NM_001265588 | ZNF267 | 0.33 | 0.00 | 157 | 250 | 200 | 207 |
| NM_152943 | ZNF268 | 0.10 | 0.00 | 231 | 303 | 453 | 519 |
| NM_152287 | ZNF276 | 0.12 | 0.00 | 451 | 566 | 470 | 523 |
| NM_001037813 | ZNF284 | 0.03 | 0.00 | 56 | 85 | 87 | 87 |
| NM_020653 | ZNF287 | 0.03 | 0.00 | 129 | 173 | 341 | 340 |
| NM_014347 | ZNF324 | 0.02 | 0.00 | 156 | 203 | 312 | 349 |
| NM_024620 | ZNF329 | 0.10 | 0.00 | 162 | 221 | 428 | 466 |
| NM_014487 | ZNF330 | 0.26 | -0.04 | 747 | 1003 | 990 | 863 |
| NM_001286769 | ZNF34 | 0.20 | 0.00 | 28 | 55 | 104 | 87 |
| NM_001282933 | ZNF341 | 0.33 | -0.03 | 147 | 236 | 160 | 118 |
| NM_001282497 | ZNF343 | 0.51 | 0.00 | 201 | 351 | 468 | 458 |
| NM_012279 | ZNF346 | 0.25 | 0.00 | 207 | 304 | 439 | 388 |
| NM_003420 | ZNF35 | 0.07 | 0.00 | 156 | 209 | 229 | 242 |
| NM_018083 | ZNF358 | 0.09 | 0.00 | 626 | 754 | 1343 | 1359 |
| NM_152493 | ZNF362 | 0.34 | 0.00 | 245 | 375 | 493 | 494 |
| NM_015481 | ZNF385A | 0.01 | 0.00 | 43 | 68 | 141 | 122 |
| NM_020781 | ZNF398 | 0.51 | 0.01 | 449 | 737 | 923 | 1030 |
| NM_024741 | ZNF408 | 0.70 | 0.00 | 182 | 364 | 437 | 486 |
| NM_001242924 | ZNF410 | 0.21 | 0.00 | 856 | 1100 | 1452 | 1449 |
| NM_015069 | ZNF423 | 0.50 | 0.00 | 69 | 137 | 50 | 46 |
| NM_024106 | ZNF426 | 0.16 | 0.00 | 166 | 235 | 324 | 342 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001164276 | ZNF44 | 0.07 | 0.00 | 83 | 121 | 160 | 185 |
| NM_017908 | ZNF446 | 0.29 | 0.00 | 55 | 99 | 297 | 255 |
| NM_021224 | ZNF462 | 0.19 | -0.19 | 513 | 671 | 1410 | 1128 |
| NM_001006656 | ZNF473 | 0.19 | -0.03 | 210 | 295 | 318 | 257 |
| NM_020713 | ZNF512B | 0.16 | 0.00 | 313 | 416 | 684 | 675 |
| NM_001278524 | ZNF518A | 0.31 | 0.00 | 147 | 233 | 254 | 255 |
| NM_145287 | ZNF519 | 0.52 | 0.00 | 23 | 58 | 29 | 30 |
| NM_015461 | ZNF521 | 0.05 | 0.00 | 52 | 81 | 256 | 240 |
| NM_153219 | ZNF524 | 0.36 | 0.00 | 59 | 110 | 119 | 144 |
| NM_133444 | ZNF526 | 0.13 | -0.05 | 758 | 930 | 1183 | 1037 |
| NM_020880 | ZNF530 | 0.38 | 0.00 | 58 | 110 | 87 | 83 |
| NM_001044387 | ZNF557 | 0.37 | 0.00 | 153 | 250 | 287 | 344 |
| NM_144693 | ZNF558 | 0.94 | 0.00 | 65 | 174 | 314 | 336 |
| NM_001042474 | ZNF565 | 0.02 | -0.49 | 75 | 108 | 173 | 91 |
| NM_152484 | ZNF569 | 0.23 | 0.00 | 43 | 78 | 166 | 183 |
| NM_152360 | ZNF573 | 0.35 | 0.00 | 32 | 67 | 92 | 74 |
| NM_022752 | ZNF574 | 0.41 | -0.15 | 508 | 772 | 886 | 708 |
| NM_152600 | ZNF579 | 0.57 | 0.00 | 82 | 166 | 154 | 131 |
| NM_016202 | ZNF580 | 1.73 | 0.00 | 32 | 165 | 183 | 181 |
| NM_001159860 | ZNF583 | 0.40 | 0.00 | 25 | 57 | 162 | 183 |
| NM_173548 | ZNF584 | 0.61 | 0.00 | 106 | 213 | 320 | 366 |
| NM_001204814 | ZNF586 | 0.33 | 0.00 | 63 | 114 | 177 | 179 |
| NM_015871 | ZNF593 | 0.52 | 0.00 | 166 | 298 | 383 | 412 |
| NM_001007248 | ZNF599 | 0.41 | 0.00 | 31 | 68 | 121 | 94 |
| NM_001031721 | ZNF613 | 0.65 | 0.00 | 23 | 63 | 89 | 104 |
| NM_133374 | ZNF618 | 0.05 | -0.03 | 1493 | 1678 | 2710 | 2484 |
| NM_014789 | ZNF623 | 0.24 | 0.00 | 285 | 402 | 605 | 593 |
| NM_033113 | ZNF628 | 0.18 | 0.00 | 85 | 133 | 181 | 179 |
| NM_001080417 | ZNF629 | 0.16 | -0.07 | 833 | 1036 | 1788 | 1571 |
| NM_014699 | ZNF646 | 0.05 | 0.00 | 809 | 937 | 1025 | 1020 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001083956 | ZNF655 | 0.23 | 0.00 | 372 | 512 | 745 | 691 |
| NM_001204299 | ZNF664-FAM101A | 0.04 | 0.00 | 32 | 55 | 104 | 85 |
| NM_024833 | ZNF671 | 0.11 | 0.00 | 65 | 101 | 216 | 250 |
| NM_182609 | ZNF677 | 0.49 | 0.00 | 51 | 106 | 87 | 85 |
| NM_021915 | ZNF69 | 0.52 | 0.00 | 13 | 40 | 89 | 83 |
| NM_030895 | ZNF696 | 0.19 | 0.00 | 63 | 104 | 123 | 104 |
| NM_025069 | ZNF703 | 0.21 | -0.16 | 285 | 396 | 433 | 327 |
| NM_001267708 | ZNF706 | 0.19 | 0.00 | 230 | 320 | 393 | 366 |
| NM_023931 | ZNF747 | 0.17 | 0.00 | 130 | 190 | 158 | 200 |
| NM_153028 | ZNF75A | 0.21 | 0.00 | 72 | 118 | 237 | 253 |
| NM_024671 | ZNF768 | 0.13 | 0.00 | 748 | 915 | 986 | 950 |
| NM_001201407 | ZNF778 | 0.63 | 0.00 | 103 | 211 | 299 | 329 |
| NM_001286697 | ZNF79 | 0.46 | 0.00 | 94 | 174 | 324 | 301 |
| NM_001013659 | ZNF793 | 0.34 | 0.00 | 16 | 40 | 98 | 131 |
| NM_178835 | ZNF827 | 0.36 | 0.00 | 421 | 627 | 1242 | 1335 |
| NM_001136501 | ZNF844 | 0.21 | 0.00 | 70 | 115 | 179 | 222 |
| NM_138374 | ZNF845 | 0.23 | 0.00 | 128 | 196 | 216 | 264 |
| NM_001195605 | ZNF865 | 0.22 | -0.02 | 185 | 269 | 283 | 227 |
| NM_001145434 | ZNF880 | 0.40 | 0.00 | 110 | 191 | 356 | 416 |
| NM_001101338 | ZNF883 | 0.50 | 0.00 | 10 | 37 | 121 | 126 |
| NM_001277291 | ZNF891 | 2.33 | 0.00 | 4 | 71 | 35 | 37 |
| NM_001287533 | ZNF92 | 0.06 | 0.00 | 76 | 112 | 85 | 65 |
| NM_014205 | ZNHIT2 | 0.96 | -0.14 | 48 | 137 | 220 | 157 |
| NM_147128 | ZNRF2 | 0.54 | 0.00 | 56 | 118 | 85 | 105 |
| NM_080603 | ZSWIM1 | 0.10 | 0.00 | 255 | 331 | 478 | 523 |
| NM_001287821 | ZWILCH | 0.26 | -0.21 | 310 | 440 | 497 | 366 |
| NM_032997 | ZWINT | 0.47 | -0.53 | 607 | 947 | 819 | 497 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001010972 | ZYX | 0.20 | 0.00 | 7940 | 9431 | 14755 | 14447 |

### Table 5B: Differentially Upregulated Genes in SEN + IL-2

| Table 5B: Differentially Upregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_005954 | MT3 | 0.00 | 6.09 | 4 | 1 | 0 | 453 |
| NM_000425 | L1CAM | 0.00 | 4.29 | 9 | 10 | 12 | 469 |
| NM_199296 | ISM2 | 0.00 | 4.24 | 2 | 8 | 10 | 433 |
| NM_001216 | CA9 | 0.00 | 3.88 | 22 | 26 | 12 | 355 |
| NM_182762 | MACC1 | 0.00 | 3.66 | 2 | 1 | 2 | 118 |
| NM_198516 | GALNT1 8 | 0.00 | 3.56 | 46 | 40 | 33 | 584 |
| NM_006771 | KRT38 | 0.00 | 3.51 | 0 | 5 | 2 | 107 |
| NM_001039130 | ALOX15 B | 0.00 | 3.33 | 1 | 6 | 4 | 139 |
| NM_004112 | FGF11 | 0.00 | 3.09 | 67 | 48 | 10 | 198 |
| NM_001144994 | C2orf72 | 0.00 | 3.07 | 25 | 10 | 4 | 116 |
| NM_017413 | APLN | 0.00 | 2.97 | 277 | 249 | 83 | 845 |
| NM_006426 | DPYSL4 | 0.00 | 2.83 | 49 | 56 | 35 | 373 |
| NM_003122 | SPINK1 | -0.01 | 2.70 | 8 | 1 | 6 | 116 |
| NM_001958 | EEF1A2 | 0.00 | 2.50 | 2 | 11 | 8 | 122 |
| NM_019058 | DDIT4 | 0.00 | 2.46 | 532 | 480 | 720 | 4338 |
| NM_000230 | LEP | 0.00 | 2.41 | 76 | 83 | 56 | 412 |
| NM_001128850 | RRAD | -0.66 | 2.39 | 47 | 15 | 54 | 396 |
| NM_002246 | KCNK3 | 0.00 | 2.35 | 7 | 12 | 150 | 939 |
| NM_004052 | BNIP3 | 0.00 | 2.17 | 2262 | 2243 | 1083 | 5257 |
| NM_004967 | IBSP | 0.00 | 2.14 | 2 | 2 | 21 | 157 |
| NM_000201 | ICAM1 | 0.00 | 2.14 | 295 | 325 | 487 | 2408 |
| NM_181726 | ANKRD3 7 | -0.06 | 2.14 | 339 | 269 | 50 | 312 |
| NM_006516 | SLC2A1 | -0.01 | 2.05 | 6356 | 6044 | 1329 | 5907 |
| NM_022834 | VWA1 | 0.00 | 2.04 | 22 | 33 | 23 | 157 |
| NM_001142386 | PDK3 | 0.00 | 2.02 | 191 | 157 | 270 | 1277 |
| NM_005165 | ALDOC | 0.00 | 2.00 | 534 | 478 | 516 | 2316 |
| NM_002820 | PTHLH | 0.00 | 1.98 | 46 | 38 | 52 | 290 |
| NM_001002814 | RAB11FI P1 | 0.00 | 1.98 | 42 | 60 | 6 | 72 |
| NM_020311 | ACKR3 | 0.00 | 1.96 | 40 | 39 | 27 | 168 |
| NM_007036 | ESM1 | -0.34 | 1.94 | 2562 | 1887 | 1863 | 7612 |
| NM_009590 | AOC2 | 0.00 | 1.93 | 21 | 35 | 15 | 107 |

(continued)

| Table 5B: Differentially Upregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_032727 | INA | 0.00 | 1.89 | 17 | 31 | 21 | 133 |
| NM_015053 | PPFIA4 | 0.00 | 1.88 | 235 | 232 | 129 | 593 |
| NM_001142617 | STRA6 | 0.00 | 1.88 | 2 | 4 | 25 | 150 |
| NM_019105 | TNXB | 0.00 | 1.81 | 2575 | 2620 | 129 | 567 |
| NM_000316 | PTH1R | 0.00 | 1.79 | 0 | 4 | 21 | 124 |
| NM_001025366 | VEGFA | 0.00 | 1.78 | 5690 | 5914 | 5781 | 20606 |
| NM_206966 | C5orf46 | 0.00 | 1.72 | 21 | 13 | 27 | 144 |
| NM_000094 | COL7A1 | 0.00 | 1.71 | 376 | 398 | 1416 | 4952 |
| NM_021219 | JAM2 | 0.00 | 1.68 | 37 | 26 | 48 | 222 |
| NM_001145106 | FIBCD1 | 0.00 | 1.68 | 352 | 369 | 69 | 301 |
| NM_170744 | UNC5B | 0.00 | 1.68 | 1077 | 1011 | 1404 | 4828 |
| NM_003391 | WNT2 | 0.00 | 1.68 | 7 | 13 | 19 | 107 |
| NM_016953 | PDE11A | 0.00 | 1.65 | 22 | 24 | 245 | 911 |
| NM_001286485 | RGS11 | 0.00 | 1.65 | 14 | 13 | 21 | 113 |
| NM_016588 | NRN1 | 0.00 | 1.64 | 455 | 447 | 37 | 174 |
| NM_001282349 | OSCAR | 0.00 | 1.63 | 53 | 40 | 60 | 259 |
| NM_001855 | COL15A1 | 0.00 | 1.63 | 171 | 210 | 1196 | 4004 |
| NM_000962 | PTGS1 | 0.00 | 1.63 | 1202 | 1233 | 2595 | 8456 |
| NM_000299 | PKP1 | -0.33 | 1.61 | 196 | 119 | 8 | 68 |
| NM_001089 | ABCA3 | 0.00 | 1.60 | 37 | 32 | 87 | 347 |
| NM_005545 | ISLR | 0.00 | 1.59 | 1165 | 1067 | 2007 | 6429 |
| NM_030949 | PPP1R14 C | 0.00 | 1.58 | 25 | 33 | 27 | 131 |
| NM_003278 | CLEC3B | 0.00 | 1.54 | 58 | 81 | 52 | 216 |
| NM_001166222 | CARNS1 | 0.00 | 1.54 | 8 | 24 | 21 | 105 |
| NM_002023 | FMOD | 0.00 | 1.53 | 68 | 74 | 173 | 610 |
| NM_001012706 | C14orfl82 | 0.00 | 1.51 | 10 | 15 | 42 | 177 |
| NM_000291 | PGK1 | 0.00 | 1.48 | 6933 | 7191 | 8146 | 23458 |
| NM_001039570 | KREMEN 1 | 0.00 | 1.46 | 115 | 130 | 308 | 983 |
| NM_001146079 | CLDN14 | -0.07 | 1.45 | 868 | 734 | 164 | 551 |
| NM_001193582 | NRCAM | 0.00 | 1.44 | 24 | 25 | 37 | 153 |
| NM_018192 | LEPREL1 | 0.00 | 1.42 | 947 | 1050 | 1508 | 4338 |
| NM_001185080 | CLDN15 | 0.00 | 1.41 | 61 | 78 | 44 | 172 |
| NM_148672 | CCL28 | -0.20 | 1.39 | 68 | 37 | 44 | 170 |
| NM_000923 | PDE4C | 0.00 | 1.36 | 18 | 31 | 31 | 126 |
| NM_001277307 | MAGEB1 7 | 0.00 | 1.36 | 280 | 284 | 50 | 185 |
| NM_014367 | FAM162 A | 0.00 | 1.36 | 593 | 570 | 761 | 2148 |
| NM_004997 | MYBPH | 0.00 | 1.34 | 27 | 26 | 204 | 623 |

(continued)

| Table 5B: Differentially Upregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_005562 | LAMC2 | 0.00 | 1.33 | 360 | 327 | 470 | 1336 |
| NM_001290294 | SORBS 1 | 0.00 | 1.33 | 23 | 27 | 69 | 237 |
| NM_014936 | ENPP4 | 0.00 | 1.30 | 2 | 6 | 29 | 115 |
| NM_001199989 | RASD1 | 0.00 | 1.30 | 58 | 48 | 69 | 233 |
| NM_000441 | SLC26A4 | 0.00 | 1.30 | 16 | 22 | 64 | 218 |
| NM_006813 | PNRC1 | 0.00 | 1.29 | 572 | 634 | 759 | 2055 |
| NM_001164507 | NEB | 0.00 | 1.29 | 60 | 48 | 123 | 383 |
| NM_173354 | SIK1 | 0.00 | 1.29 | 249 | 265 | 505 | 1392 |
| NM_000636 | SOD2 | 0.00 | 1.27 | 1297 | 1180 | 1483 | 3828 |
| NM_001040619 | ATF3 | 0.00 | 1.26 | 167 | 164 | 83 | 266 |
| NM_134262 | RORA | 0.00 | 1.26 | 640 | 706 | 713 | 1887 |
| NM_144717 | IL20RB | 0.00 | 1.24 | 27 | 24 | 35 | 128 |
| NM_001012631 | IL32 | 0.00 | 1.24 | 404 | 422 | 526 | 1397 |
| NM_001099952 | ITPR1 | 0.00 | 1.24 | 166 | 167 | 559 | 1479 |
| NM_001838 | CCR7 | 0.00 | 1.23 | 44 | 43 | 27 | 104 |
| NM_004102 | FABP3 | 0.00 | 1.22 | 98 | 72 | 310 | 845 |
| NM_001040167 | LFNG | 0.00 | 1.22 | 117 | 153 | 67 | 216 |
| NM_002317 | LOX | 0.00 | 1.22 | 42411 | 42652 | 50535 | 119408 |
| NM_014650 | ZNF432 | 0.00 | 1.22 | 88 | 62 | 164 | 471 |
| NM_001195541 | GS1-259H13.2 | -0.37 | 1.22 | 66 | 31 | 35 | 126 |
| NM_001164665 | KIAA154 9 | 0.00 | 1.22 | 60 | 81 | 200 | 562 |
| NM_007037 | ADAMTS 8 | 0.00 | 1.19 | 16 | 25 | 29 | 107 |
| NM_001082967 | FAM19A 5 | -0.37 | 1.19 | 407 | 262 | 106 | 314 |
| NM_003355 | UCP2 | 0.00 | 1.19 | 84 | 77 | 77 | 237 |
| NM_000888 | ITGB6 | 0.00 | 1.18 | 1 | 1 | 37 | 129 |
| NM_001253845 | ADM2 | 0.00 | 1.18 | 142 | 165 | 185 | 512 |
| NM_005962 | MXI1 | -0.08 | 1.17 | 598 | 491 | 778 | 1932 |
| NM_004864 | GDF15 | 0.00 | 1.16 | 4509 | 4526 | 2219 | 5268 |
| NM_001166239 | CGREF1 | 0.00 | 1.15 | 60 | 77 | 137 | 384 |
| NM_080552 | SLC32A1 | 0.00 | 1.15 | 11 | 24 | 29 | 104 |
| NM_020125 | SLAMF8 | 0.00 | 1.14 | 15 | 22 | 35 | 120 |
| NM_001042784 | CCDC158 | 0.00 | 1.14 | 2 | 3 | 31 | 109 |
| NM_005704 | PTPRU | 0.00 | 1.14 | 109 | 108 | 318 | 817 |
| NM_032148 | SLC41A2 | 0.00 | 1.13 | 153 | 182 | 449 | 1122 |
| NM_014399 | TSPAN13 | 0.00 | 1.13 | 368 | 379 | 726 | 1765 |
| NM_006167 | NKX3-1 | 0.00 | 1.13 | 289 | 242 | 218 | 575 |
| NM_006871 | RIPK3 | 0.00 | 1.12 | 130 | 124 | 220 | 573 |

(continued)

| Table 5B: Differentially Upregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_139125 | MASP1 | 0.00 | 1.11 | 1205 | 1205 | 229 | 593 |
| NM_001161572 | MAFF | 0.00 | 1.11 | 1915 | 1861 | 1491 | 3460 |
| NM_004115 | FGF14 | 0.00 | 1.11 | 61 | 69 | 89 | 255 |
| NM_030957 | ADAMTS 10 | -0.50 | 1.11 | 139 | 70 | 87 | 250 |
| NM_032532 | FNDC1 | 0.00 | 1.10 | 123 | 160 | 343 | 854 |
| NM_024787 | RNF122 | 0.00 | 1.10 | 115 | 141 | 54 | 166 |
| NM_025107 | MYCT1 | 0.00 | 1.10 | 260 | 270 | 6 | 41 |
| NM_031866 | FZD8 | 0.00 | 1.09 | 445 | 452 | 339 | 837 |
| NM_152701 | ABCA13 | 0.00 | 1.09 | 19 | 25 | 247 | 625 |
| NM_001130026 | FAM115C | 0.00 | 1.09 | 115 | 105 | 44 | 139 |
| NM_001946 | DUSP6 | 0.00 | 1.08 | 133 | 144 | 1589 | 3606 |
| NM_001161352 | KCNMA1 | 0.00 | 1.08 | 252 | 251 | 2115 | 4750 |
| NM_001243784 | PDE2A | 0.00 | 1.07 | 31 | 30 | 62 | 183 |
| NM_022818 | MAP1LC 3B | -0.01 | 1.07 | 2676 | 2492 | 2859 | 6325 |
| NM_018004 | TMEM45 A | 0.00 | 1.07 | 437 | 380 | 441 | 1055 |
| NM_001033045 | GPR155 | 0.00 | 1.07 | 166 | 127 | 320 | 782 |
| NM_144665 | SESN3 | 0.00 | 1.06 | 212 | 192 | 378 | 911 |
| NM_002245 | KCNK1 | -0.34 | 1.06 | 158 | 92 | 60 | 177 |
| NM_020309 | SLC17A7 | -0.08 | 1.06 | 44 | 23 | 50 | 152 |
| NM_016133 | INSIG2 | 0.00 | 1.06 | 287 | 334 | 266 | 656 |
| NM_004748 | CCPG1 | 0.00 | 1.06 | 1702 | 1722 | 2731 | 5998 |
| NM_033518 | SLC38A5 | 0.00 | 1.06 | 1760 | 1793 | 1824 | 4048 |
| NM_133494 | NEK7 | 0.00 | 1.05 | 7431 | 7614 | 7711 | 16495 |
| NM_198276 | TMEM17 | 0.00 | 1.05 | 23 | 25 | 64 | 185 |
| NM_001159643 | MCTP2 | 0.00 | 1.04 | 2 | 6 | 114 | 303 |
| NM_001282192 | RNASE4 | 0.00 | 1.04 | 356 | 367 | 686 | 1571 |
| NM_001004439 | ITGA11 | 0.00 | 1.04 | 6800 | 6553 | 10423 | 22038 |
| NM_002250 | KCNN4 | 0.00 | 1.04 | 69 | 70 | 842 | 1904 |
| NM_014331 | SLC7A11 | -0.07 | 1.04 | 854 | 722 | 1227 | 2728 |
| NM_005202 | COL8A2 | 0.00 | 1.03 | 353 | 400 | 900 | 2024 |
| NM_013332 | HILPDA | 0.00 | 1.03 | 416 | 443 | 237 | 580 |
| NM_002061 | GCLM | 0.00 | 1.03 | 1509 | 1517 | 1350 | 2981 |
| NM_024896 | ERMP1 | 0.00 | 1.03 | 331 | 301 | 580 | 1327 |
| NM_001077619 | UBXN2B | 0.00 | 1.03 | 28 | 21 | 106 | 281 |
| NM_012328 | DNAJB9 | -0.09 | 1.02 | 487 | 390 | 327 | 774 |
| NM_020689 | SLC24A3 | 0.00 | 1.02 | 498 | 528 | 156 | 394 |
| NM_000093 | COL5A1 | -0.07 | 1.02 | 14317 | 13263 | 13133 | 27299 |

(continued)

| Table 5B: Differentially Upregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_001040084 | ANXA8 | 0.00 | 1.01 | 131 | 161 | 58 | 166 |
| NM_017817 | RAB20 | 0.00 | 1.00 | 99 | 103 | 40 | 122 |
| NM_001759 | CCND2 | 0.00 | 1.00 | 122 | 126 | 1279 | 2771 |
| NM_153360 | APCDD1 L | -0.03 | 1.00 | 2412 | 2212 | 6576 | 13626 |
| NM_006072 | CCL26 | 0.00 | 1.00 | 108 | 131 | 154 | 384 |
| NM_001081 | CUBN | 0.00 | 1.00 | 11 | 27 | 146 | 366 |
| NM_005220 | DLX3 | 0.00 | 1.00 | 19 | 19 | 35 | 109 |
| NM_007325 | GRIA3 | -0.24 | 0.99 | 429 | 306 | 1040 | 2257 |
| NM_001130987 | DYSF | -0.20 | 0.99 | 695 | 530 | 2392 | 5033 |
| NM_018000 | MREG | -0.53 | 0.99 | 109 | 51 | 106 | 274 |
| NM_181079 | IL21R | 0.00 | 0.99 | 71 | 64 | 19 | 68 |
| NM_000392 | ABCC2 | 0.00 | 0.98 | 37 | 55 | 37 | 113 |
| NM_005211 | CSF1R | -0.27 | 0.98 | 80 | 43 | 189 | 455 |
| NM_001001794 | DENND6 B | 0.00 | 0.98 | 24 | 20 | 79 | 211 |
| NM_001039966 | GPER1 | 0.00 | 0.98 | 302 | 336 | 247 | 580 |
| NM_152666 | PLD5 | -0.36 | 0.98 | 49 | 21 | 54 | 153 |
| NM_152772 | TCP11L2 | 0.00 | 0.97 | 270 | 219 | 387 | 872 |
| NM_001252100 | KIF21B | 0.00 | 0.97 | 103 | 88 | 35 | 107 |
| NM_016577 | RAB6B | 0.00 | 0.96 | 61 | 75 | 279 | 643 |
| NM_001199619 | NCOA7 | 0.00 | 0.96 | 510 | 554 | 582 | 1275 |
| NM_001098845 | ANXA8L 1 | 0.00 | 0.96 | 67 | 95 | 37 | 111 |
| NM_002275 | KRT15 | -0.21 | 0.96 | 83 | 47 | 181 | 431 |
| NM_001286423 | GLB1L | 0.00 | 0.96 | 347 | 342 | 1025 | 2170 |
| NM_000379 | XDH | 0.00 | 0.95 | 58 | 84 | 52 | 146 |
| NM_025218 | ULBP1 | 0.00 | 0.95 | 280 | 263 | 385 | 858 |
| NM_017694 | MFSD6 | 0.00 | 0.95 | 96 | 105 | 243 | 560 |
| NM_002658 | PLAU | 0.00 | 0.95 | 246 | 268 | 1709 | 3525 |
| NM_194296 | SPATA24 | 0.00 | 0.94 | 46 | 29 | 25 | 81 |
| NM_021111 | RECK | 0.01 | 0.93 | 3270 | 3477 | 5422 | 10732 |
| NM_153006 | NAGS | 0.00 | 0.92 | 79 | 58 | 67 | 177 |
| NM_181481 | LDLRAD 4 | 0.00 | 0.92 | 21 | 15 | 100 | 248 |
| NM_004598 | SPOCK1 | 0.00 | 0.91 | 10414 | 10436 | 9246 | 17944 |
| NM_001134338 | RNF24 | 0.00 | 0.91 | 1086 | 1189 | 1422 | 2887 |
| NM_152405 | JMY | 0.00 | 0.91 | 197 | 206 | 381 | 828 |
| NM_004936 | CDKN2B | 0.00 | 0.91 | 81 | 99 | 287 | 636 |
| NM_001042410 | ANKZF1 | 0.00 | 0.91 | 380 | 372 | 316 | 695 |
| NM_032623 | MGARP | 0.00 | 0.91 | 37 | 31 | 46 | 128 |

(continued)

| Table 5B: Differentially Upregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_022817 | PER2 | 0.00 | 0.90 | 188 | 177 | 198 | 451 |
| NM_007332 | TRPA1 | 0.00 | 0.90 | 145 | 167 | 225 | 505 |
| NM_001145115 | PPP1R3G | 0.00 | 0.89 | 142 | 132 | 106 | 257 |
| NM_001785 | CDA | -0.23 | 0.89 | 220 | 146 | 293 | 640 |
| NM_001170820 | IFITM10 | 0.00 | 0.89 | 110 | 108 | 418 | 889 |
| NM_000451 | SHOX | 0.00 | 0.89 | 4 | 14 | 121 | 288 |

| Table 5B: Differentially Upregulated Genes in SEN + IL- 2 | | | | | | | |
|---|---|---|---|---|---|---|---|
| RefSeq ID | Gene Symbol | SR GFOLD | SEN GFOLD | SR IL-2 - | SR IL-2 + | SEN IL-2- | SEN IL-2+ |
| NM_001135219 | PIP5KL1 | 0.00 | 0.89 | 18 | 29 | 75 | 190 |
| NM_001001791 | C10orf55 | 0.00 | 0.88 | 88 | 90 | 618 | 1275 |
| NM_001136537 | BTBD19 | 0.00 | 0.87 | 71 | 97 | 156 | 357 |
| NM_015012 | TMEM41 B | 0.00 | 0.87 | 609 | 592 | 892 | 1795 |
| NM_001323 | CST6 | 0.00 | 0.87 | 25 | 33 | 37 | 105 |
| NM_006208 | ENPP1 | 0.00 | 0.87 | 545 | 553 | 1406 | 2774 |
| NM_022051 | EGLN1 | 0.00 | 0.87 | 1466 | 1523 | 1217 | 2407 |
| NM_004717 | DGKI | 0.00 | 0.86 | 1118 | 1195 | 688 | 1396 |
| NM_003660 | PPFIA3 | 0.00 | 0.86 | 64 | 82 | 62 | 159 |
| NM_003486 | SLC7A5 | 0.00 | 0.86 | 3975 | 4108 | 1884 | 3647 |
| NM_000955 | PTGER1 | 0.00 | 0.86 | 9 | 19 | 37 | 104 |
| NM_031464 | RPS6KL1 | 0.00 | 0.85 | 69 | 94 | 42 | 115 |
| NM_001113498 | MDGA2 | 0.00 | 0.85 | 2 | 8 | 139 | 318 |
| NM_001206995 | PPP2R2C | 0.00 | 0.85 | 102 | 132 | 433 | 896 |
| NM_012068 | ATF5 | 0.00 | 0.85 | 903 | 922 | 1670 | 3225 |
| NM_000202 | IDS | 0.00 | 0.84 | 2240 | 2352 | 4409 | 8259 |
| NM_000640 | IL13RA2 | 0.00 | 0.84 | 50 | 73 | 349 | 728 |
| NM_001792 | CDH2 | 0.00 | 0.84 | 12840 | 13233 | 12338 | 22695 |
| NM_033656 | BRWD1 | 0.00 | 0.84 | 730 | 734 | 948 | 1863 |
| NM_001001891 | ANO7 | 0.00 | 0.84 | 64 | 53 | 50 | 131 |
| NM_178554 | KY | 0.00 | 0.84 | 102 | 91 | 177 | 390 |
| NM_153610 | CMYA5 | 0.00 | 0.84 | 31 | 25 | 166 | 368 |
| NM_004978 | KCNC4 | 0.00 | 0.83 | 352 | 347 | 2136 | 4050 |
| NM_181712 | KANK4 | 0.00 | 0.83 | 3 | 5 | 110 | 255 |
| NM_005233 | EPHA3 | 0.00 | 0.83 | 22 | 10 | 54 | 139 |
| NM_002923 | RGS2 | 0.00 | 0.83 | 144 | 126 | 233 | 497 |
| NM_016599 | MYOZ2 | 0.00 | 0.83 | 33 | 26 | 85 | 203 |
| NM_013231 | FLRT2 | 0.00 | 0.82 | 2909 | 2973 | 4702 | 8682 |
| NM_001030273 | ARNTL | 0.00 | 0.82 | 335 | 335 | 297 | 619 |

(continued)

| Table 5B: Differentially Upregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_015482 | SLC22A2 3 | 0.00 | 0.82 | 389 | 406 | 809 | 1580 |
| NM_198390 | CMIP | 0.00 | 0.82 | 1503 | 1435 | 1289 | 2460 |
| NM_001099773 | CYP11A1 | 0.00 | 0.81 | 19 | 18 | 119 | 270 |
| NM_018012 | KIF26B | 0.00 | 0.81 | 348 | 390 | 661 | 1298 |
| NM_025225 | PNPLA3 | 0.00 | 0.81 | 10 | 24 | 85 | 201 |
| NM_001010858 | RNF187 | 0.00 | 0.81 | 2966 | 3079 | 4421 | 8089 |
| NM_017680 | ASPN | 0.00 | 0.81 | 28 | 41 | 114 | 259 |
| NM_032812 | PLXDC2 | 0.00 | 0.81 | 32 | 22 | 401 | 808 |
| NM_024841 | PRR5L | 0.00 | 0.81 | 389 | 404 | 428 | 858 |
| RefSeq ID | Gene Symbol | SR GFOLD | SEN GFOLD | SR IL-2 - | SR IL-2 + | SEN IL-2- | SEN IL-2+ |
| NM_015193 | ARC | -0.93 | 0.80 | 68 | 20 | 21 | 65 |
| NM_030788 | DCSTAM P | 0.00 | 0.80 | 0 | 2 | 94 | 218 |
| NM_005099 | ADAMTS 4 | 0.00 | 0.80 | 578 | 603 | 1135 | 2155 |
| NM_015672 | RIMBP3 | 0.00 | 0.80 | 37 | 59 | 25 | 74 |
| NM_145648 | SLC15A4 | 0.00 | 0.80 | 912 | 921 | 1163 | 2205 |
| NM_001199383 | RNF145 | 0.00 | 0.80 | 1750 | 1651 | 1337 | 2519 |
| NM_001199723 | CRABP2 | 0.00 | 0.80 | 79 | 90 | 206 | 436 |
| NM_001195144 | ANKRD4 4 | -0.20 | 0.80 | 387 | 280 | 618 | 1205 |
| NM_006520 | DYNLT3 | 0.00 | 0.80 | 318 | 332 | 778 | 1497 |
| NM_023016 | SOWAHC | -0.04 | 0.79 | 445 | 368 | 428 | 852 |
| NM_003979 | GPRC5A | 0.00 | 0.79 | 961 | 1002 | 351 | 706 |
| NM_003897 | IER3 | -0.14 | 0.79 | 6943 | 6033 | 3138 | 5700 |
| NM_144599 | NIPA1 | 0.00 | 0.79 | 761 | 856 | 1061 | 1998 |
| NM_001270764 | CHST15 | 0.00 | 0.78 | 89 | 76 | 227 | 471 |
| NM_181784 | SPRED2 | 0.00 | 0.78 | 577 | 661 | 1031 | 1939 |
| NM_014840 | NUAK1 | 0.00 | 0.78 | 1562 | 1609 | 1379 | 2560 |
| NM_001170330 | C4orf3 | -0.17 | 0.78 | 1159 | 932 | 1479 | 2737 |
| NM_138371 | PCED1B | 0.00 | 0.78 | 180 | 197 | 156 | 335 |
| NM_021999 | ITM2B | 0.00 | 0.77 | 2699 | 2733 | 3966 | 7100 |
| NM_005069 | SIM2 | 0.00 | 0.77 | 130 | 98 | 175 | 370 |
| NM_005734 | HIPK3 | 0.00 | 0.77 | 2724 | 2760 | 3621 | 6488 |
| NM_000152 | GAA | -0.40 | 0.77 | 591 | 384 | 1387 | 2556 |
| NM_015444 | TMEM15 8 | 0.00 | 0.77 | 325 | 291 | 2148 | 3898 |
| NM_001204108 | BCL2L11 | -0.22 | 0.77 | 69 | 37 | 35 | 94 |
| NM_005629 | SLC6A8 | -0.01 | 0.77 | 1157 | 1039 | 1462 | 2682 |
| NM_001823 | CKB | 0.00 | 0.77 | 1022 | 1040 | 1383 | 2542 |
| NM_003896 | ST3GAL5 | 0.00 | 0.77 | 171 | 179 | 225 | 462 |

(continued)

| RefSeq ID | Gene Symbol | SR GFOLD | SEN GFOLD | SR IL-2 - | SR IL-2 + | SEN IL-2- | SEN IL-2+ |
|---|---|---|---|---|---|---|---|
| NM_005524 | HES1 | 0.00 | 0.76 | 103 | 89 | 52 | 129 |
| NM_030647 | KDM7A | 0.00 | 0.76 | 335 | 354 | 368 | 725 |
| NM_001100912 | BEND7 | 0.00 | 0.76 | 95 | 92 | 81 | 187 |
| NM_005640 | TAF4B | 0.00 | 0.76 | 108 | 104 | 69 | 163 |
| NM_020957 | PCDHB16 | 0.00 | 0.76 | 13 | 14 | 40 | 104 |
| NM_001731 | BTG1 | 0.00 | 0.76 | 1170 | 1284 | 1250 | 2298 |
| NM_015015 | KDM4B | -0.12 | 0.76 | 1017 | 839 | 1138 | 2096 |
| NM_012309 | SHANK2 | 0.00 | 0.76 | 81 | 69 | 137 | 294 |
| NM_003272 | GPR137B | 0.00 | 0.76 | 171 | 194 | 501 | 961 |
| NM_007224 | NXPH4 | -0.30 | 0.75 | 147 | 87 | 73 | 170 |
| NM_004669 | CLIC3 | 0.00 | 0.75 | 97 | 93 | 218 | 445 |
| NM_003532 | HIST1H3 E | -0.65 | 0.75 | 794 | 440 | 52 | 128 |
| NM_001178106 | ZNF185 | 0.00 | 0.75 | 487 | 540 | 89 | 201 |
| NM_001206951 | SLC16A3 | -0.06 | 0.75 | 9943 | 9200 | 11315 | 19558 |
| NM_000290 | PGAM2 | 0.00 | 0.75 | 28 | 29 | 77 | 177 |
| NM_006636 | MTHFD2 | 0.00 | 0.74 | 1635 | 1720 | 869 | 1606 |
| NM_001161546 | PROB1 | 0.00 | 0.74 | 133 | 137 | 58 | 139 |
| NM_145658 | SPESP1 | 0.00 | 0.74 | 76 | 78 | 58 | 139 |
| NM_152680 | TMEM15 4 | 0.00 | 0.74 | 89 | 91 | 283 | 560 |
| NM_173208 | TGIF1 | 0.00 | 0.74 | 863 | 948 | 834 | 1536 |
| NM_002890 | RASA1 | 0.00 | 0.74 | 1300 | 1342 | 2957 | 5196 |
| NM_020777 | SORCS2 | 0.00 | 0.74 | 335 | 291 | 1360 | 2449 |
| NM_018964 | SLC37A1 | 0.00 | 0.73 | 80 | 53 | 37 | 96 |
| NM_153365 | TAPT1 | 0.00 | 0.73 | 288 | 305 | 366 | 706 |
| NM_001135216 | ZSCAN31 | 0.00 | 0.73 | 10 | 14 | 40 | 102 |
| NM_019050 | USP53 | 0.00 | 0.73 | 1457 | 1427 | 1878 | 3333 |
| NM_153487 | MDGA1 | 0.00 | 0.73 | 332 | 380 | 921 | 1680 |
| NM_001270691 | SMOX | -0.10 | 0.73 | 664 | 540 | 549 | 1028 |
| NM_017514 | PLXNA3 | -0.03 | 0.73 | 2184 | 1984 | 4999 | 8625 |
| NM_198581 | ZC3H6 | 0.00 | 0.73 | 400 | 347 | 343 | 662 |
| NM_001172646 | PLCB4 | 0.00 | 0.72 | 809 | 816 | 1506 | 2682 |
| NM_001433 | ERN1 | 0.00 | 0.72 | 257 | 306 | 462 | 871 |
| NM_001135556 | DYNC1I1 | 0.00 | 0.72 | 31 | 48 | 362 | 693 |
| NM_001105244 | PTPRM | 0.01 | 0.72 | 3725 | 3953 | 5035 | 8628 |
| NM_003183 | ADAM17 | 0.00 | 0.72 | 747 | 786 | 703 | 1288 |
| NM_005127 | CLEC2B | 0.00 | 0.71 | 208 | 190 | 177 | 359 |
| NM_001128588 | SLC14A1 | 0.00 | 0.71 | 75 | 68 | 385 | 730 |
| NM_203394 | E2F7 | 0.00 | 0.71 | 1629 | 1635 | 2914 | 5039 |

(continued)

| RefSeq ID | Gene Symbol | SR GFOLD | SEN GFOLD | SR IL-2 - | SR IL-2 + | SEN IL-2- | SEN IL-2+ |
|---|---|---|---|---|---|---|---|
| NM_020485 | RHCE | 0.00 | 0.71 | 47 | 59 | 60 | 140 |
| NM_001098722 | GNG4 | 0.00 | 0.71 | 27 | 35 | 60 | 140 |
| NM_019107 | C19orf10 | 0.00 | 0.71 | 6232 | 6065 | 8560 | 14429 |
| NM_001130144 | LTBP3 | 0.00 | 0.71 | 2082 | 1944 | 3101 | 5333 |
| NM_024592 | SRD5A3 | 0.00 | 0.70 | 115 | 142 | 403 | 758 |
| NM_032530 | ZNF594 | 0.00 | 0.70 | 43 | 52 | 52 | 124 |
| NM_018227 | UBA6 | 0.00 | 0.70 | 1163 | 1231 | 2250 | 3898 |
| NM_001160148 | DDHD1 | 0.00 | 0.70 | 526 | 563 | 745 | 1349 |
| NM_001258379 | SLC4A7 | 0.00 | 0.70 | 1922 | 1933 | 2674 | 4603 |
| NM_012334 | MYO10 | 0.01 | 0.70 | 5198 | 5465 | 6671 | 11249 |
| NM_181486 | TBX5 | 0.00 | 0.70 | 86 | 89 | 37 | 94 |
| NM_198159 | MITF | 0.00 | 0.70 | 167 | 167 | 206 | 408 |
| NM_007350 | PHLDA1 | 0.00 | 0.70 | 1860 | 1978 | 6904 | 11608 |
| NM_001336 | CTSZ | -0.02 | 0.70 | 6070 | 5747 | 10047 | 16785 |
| NM_032642 | WNT5B | 0.00 | 0.70 | 5099 | 5198 | 10641 | 17759 |
| NM_002840 | PTPRF | 0.00 | 0.70 | 13060 | 12751 | 11579 | 19301 |
| NM_017649 | CNNM2 | 0.00 | 0.70 | 97 | 124 | 183 | 366 |
| NM_002632 | PGF | 0.00 | 0.69 | 471 | 471 | 842 | 1503 |
| NM_004947 | DOCK3 | 0.00 | 0.69 | 40 | 29 | 44 | 107 |
| NM_015194 | MYOID | 0.00 | 0.69 | 1674 | 1646 | 3841 | 6480 |
| NM_178518 | TMEM10 2 | -0.24 | 0.69 | 211 | 139 | 85 | 185 |
| NM_001204397 | PITX2 | 0.00 | 0.68 | 128 | 142 | 466 | 856 |
| NM_001012361 | WDR31 | 0.00 | 0.68 | 10 | 6 | 83 | 181 |
| NM_173200 | NR4A3 | 0.00 | 0.68 | 161 | 184 | 75 | 166 |
| NM_001145671 | SORBS2 | 0.00 | 0.68 | 86 | 102 | 114 | 238 |
| NM_018941 | CLN8 | 0.00 | 0.68 | 1538 | 1657 | 1882 | 3229 |
| NM_002517 | NPAS1 | 0.00 | 0.68 | 116 | 154 | 168 | 335 |
| NM_020975 | RET | 0.00 | 0.68 | 25 | 35 | 60 | 137 |
| NM_014287 | NOMO1 | 0.00 | 0.67 | 5419 | 5437 | 8036 | 13259 |
| NM_017580 | ZRANB1 | 0.00 | 0.67 | 1238 | 1329 | 944 | 1656 |
| NM_024303 | ZSCAN5 A | 0.00 | 0.67 | 48 | 63 | 58 | 133 |
| NM_182487 | OLFML2 A | 0.00 | 0.67 | 16 | 30 | 58 | 133 |
| NM_000959 | PTGFR | 0.00 | 0.67 | 306 | 317 | 437 | 799 |
| NM_019053 | EXOC6 | 0.00 | 0.67 | 133 | 101 | 73 | 161 |
| NM_001151 | SLC25A4 | -0.22 | 0.67 | 2057 | 1631 | 1237 | 2137 |
| NM_001042496 | SLC12A6 | 0.00 | 0.67 | 1214 | 1263 | 1996 | 3388 |
| NM_005892 | FMNL1 | 0.00 | 0.67 | 67 | 71 | 166 | 329 |
| NM_005139 | ANXA3 | 0.00 | 0.67 | 51 | 37 | 10 | 41 |

(continued)

| RefSeq ID | Gene Symbol | SR GFOLD | SEN GFOLD | SR IL-2 - | SR IL-2 + | SEN IL-2- | SEN IL-2+ |
|---|---|---|---|---|---|---|---|
| NM_017644 | KLHL24 | 0.00 | 0.67 | 508 | 493 | 686 | 1216 |
| NM_001031680 | RUNX3 | -0.20 | 0.67 | 188 | 125 | 69 | 153 |
| NM_003749 | IRS2 | 0.00 | 0.67 | 851 | 872 | 747 | 1318 |
| NM_004457 | ACSL3 | 0.00 | 0.66 | 955 | 974 | 1967 | 3322 |
| NM_015419 | MXRA5 | 0.00 | 0.66 | 273 | 296 | 1148 | 1978 |
| NM_001077624 | ZNF846 | 0.00 | 0.66 | 49 | 50 | 137 | 275 |
| NM_031463 | HSDL1 | 0.01 | 0.66 | 512 | 593 | 767 | 1342 |
| NM_001242804 | LOC1005 05549 | 0.00 | 0.65 | 88 | 110 | 81 | 174 |
| NM_001142550 | WDR47 | 0.00 | 0.65 | 601 | 540 | 697 | 1225 |
| NM_001121 | ADD3 | 0.00 | 0.65 | 1620 | 1573 | 2325 | 3887 |
| NM_024006 | VKORC1 | 0.01 | 0.65 | 1455 | 1590 | 3573 | 5902 |
| NM_003395 | WNT9A | 0.00 | 0.65 | 34 | 55 | 85 | 181 |
| NM_002210 | ITGAV | 0.01 | 0.65 | 6797 | 7110 | 10656 | 17227 |
| NM_001714 | BICD1 | 0.00 | 0.65 | 767 | 758 | 988 | 1702 |
| NM_032409 | PINK1 | 0.00 | 0.65 | 2952 | 3068 | 6759 | 10996 |
| NM_030627 | CPEB4 | 0.00 | 0.65 | 1268 | 1267 | 1069 | 1832 |
| NM_005560 | LAMA5 | -0.23 | 0.65 | 347 | 243 | 129 | 259 |
| NM_002556 | OSBP | 0.00 | 0.64 | 2109 | 2232 | 2313 | 3843 |
| NM_148893 | SVIP | 0.00 | 0.64 | 81 | 91 | 127 | 255 |
| NM_033254 | BOC | 0.00 | 0.64 | 460 | 498 | 745 | 1294 |
| NM_017789 | SEMA4C | 0.00 | 0.64 | 472 | 477 | 374 | 677 |
| NM_001258210 | TSKU | 0.00 | 0.64 | 977 | 971 | 838 | 1444 |
| NM_001281731 | CERS5 | 0.00 | 0.64 | 868 | 944 | 1522 | 2553 |
| NM_006517 | SLC16A2 | 0.00 | 0.64 | 1926 | 1872 | 2870 | 4715 |
| NM_001178007 | BBS12 | 0.00 | 0.64 | 74 | 49 | 135 | 268 |
| NM_001278376 | MPP2 | 0.00 | 0.64 | 215 | 254 | 277 | 512 |
| NM_005493 | RANBP9 | 0.00 | 0.64 | 1164 | 1240 | 1111 | 1885 |
| NM_005149 | TBX19 | -0.63 | 0.64 | 74 | 29 | 56 | 126 |
| NM_173552 | C3orf58 | 0.00 | 0.63 | 336 | 399 | 208 | 394 |
| NM_006091 | CORO2B | 0.00 | 0.63 | 436 | 470 | 1483 | 2481 |
| NM_001200 | BMP2 | 0.00 | 0.63 | 10 | 18 | 60 | 133 |
| NM_001005291 | SREBF1 | 0.00 | 0.63 | 1122 | 1091 | 1323 | 2220 |
| NM_182752 | TPRG1L | 0.00 | 0.63 | 3275 | 3097 | 4968 | 8016 |
| NM_012168 | FBXO2 | 0.00 | 0.63 | 7 | 9 | 52 | 118 |
| NM_016127 | SARAF | 0.00 | 0.63 | 1623 | 1546 | 4026 | 6521 |
| NM_004093 | EFNB2 | 0.00 | 0.63 | 106 | 82 | 29 | 74 |
| NM_007214 | SEC63 | -0.01 | 0.63 | 526 | 451 | 747 | 1283 |
| NM_198542 | ZNF773 | 0.00 | 0.63 | 118 | 112 | 154 | 299 |

(continued)

| RefSeq ID | Gene Symbol | SR GFOLD | SEN GFOLD | SR IL-2 - | SR IL-2 + | SEN IL-2- | SEN IL-2+ |
|---|---|---|---|---|---|---|---|
| NM_152445 | FAM161B | 0.00 | 0.62 | 127 | 166 | 345 | 621 |
| NM_001708 | OPN1SW | 0.00 | 0.62 | 225 | 261 | 133 | 262 |
| NM_022763 | FNDC3B | 0.00 | 0.62 | 3339 | 3164 | 4392 | 7068 |
| NM_001039548 | KLHL35 | 0.00 | 0.62 | 69 | 91 | 87 | 181 |
| NM_001025077 | CELF2 | 0.00 | 0.62 | 249 | 304 | 339 | 610 |
| NM_001104647 | SLC25A3 6 | 0.00 | 0.62 | 754 | 795 | 948 | 1601 |
| NM_001199039 | SERINC2 | 0.01 | 0.62 | 2806 | 2994 | 6622 | 10540 |
| NM_012317 | LDOC1 | 0.00 | 0.62 | 252 | 308 | 166 | 318 |
| NM_177552 | SULT1A3 | 0.00 | 0.62 | 198 | 200 | 183 | 347 |
| NM_001017390 | SULT1A4 | 0.00 | 0.62 | 198 | 200 | 183 | 347 |
| NM_003185 | TAF4 | 0.00 | 0.62 | 245 | 197 | 162 | 311 |
| NM_004415 | DSP | 0.00 | 0.62 | 24219 | 24316 | 29861 | 46568 |
| NM_002047 | GARS | 0.00 | 0.61 | 6352 | 6573 | 8996 | 14209 |
| NM_006219 | PIK3CB | 0.00 | 0.61 | 400 | 389 | 468 | 819 |
| NM_005399 | PRKAB2 | 0.00 | 0.61 | 442 | 500 | 1190 | 1981 |
| NM_003956 | CH25H | 0.00 | 0.61 | 10 | 22 | 227 | 420 |
| NM_002846 | PTPRN | 0.00 | 0.61 | 193 | 232 | 185 | 349 |
| NM_030671 | PTPRO | 0.00 | 0.61 | 0 | 3 | 67 | 144 |
| NM_182961 | SYNE1 | 0.00 | 0.61 | 12665 | 12456 | 8828 | 13905 |
| NM_017510 | TMED9 | -0.17 | 0.61 | 10691 | 9190 | 11866 | 18591 |
| NM_001195202 | LOC1001 29636 | 0.00 | 0.61 | 58 | 72 | 60 | 131 |
| NM_001166050 | APBB2 | 0.00 | 0.61 | 1688 | 1688 | 1892 | 3083 |
| NM_001040667 | HSF4 | 0.00 | 0.61 | 62 | 68 | 123 | 242 |
| NM_001957 | EDNRA | 0.00 | 0.61 | 84 | 79 | 310 | 556 |
| NM_058238 | WNT7B | 0.00 | 0.60 | 18 | 11 | 256 | 466 |
| NM_016445 | PLEK2 | 0.00 | 0.60 | 291 | 304 | 410 | 719 |
| NM_004984 | KIF5A | -0.43 | 0.60 | 36 | 11 | 46 | 105 |
| NM_006472 | TXNIP | 0.00 | 0.60 | 832 | 819 | 3409 | 5444 |
| NM_022465 | IKZF4 | 0.00 | 0.60 | 151 | 193 | 181 | 340 |
| NM_019112 | ABCA7 | 0.00 | 0.60 | 35 | 50 | 283 | 510 |
| NM_198181 | GOLGA6 L9 | 0.00 | 0.60 | 52 | 42 | 21 | 57 |
| NM_201380 | PLEC | -0.10 | 0.60 | 12686 | 11469 | 21412 | 33088 |
| NM_031449 | ZMIZ2 | -0.21 | 0.60 | 1482 | 1171 | 1581 | 2575 |
| NM_001201380 | CNTNAP 3B | 0.00 | 0.60 | 94 | 118 | 110 | 218 |
| NM_032134 | QRICH2 | 0.00 | 0.59 | 133 | 146 | 212 | 390 |
| NM_032801 | JAM3 | 0.00 | 0.59 | 945 | 956 | 3045 | 4848 |
| NM_032867 | MICALC L | 0.00 | 0.59 | 118 | 120 | 156 | 296 |
| NM_152495 | CNIH3 | 0.00 | 0.59 | 24 | 39 | 1512 | 2460 |

(continued)

| RefSeq ID | Gene Symbol | SR GFOLD | SEN GFOLD | SR IL-2 - | SR IL-2 + | SEN IL-2- | SEN IL-2+ |
|---|---|---|---|---|---|---|---|
| NM_152703 | SAMD9L | 0.00 | 0.59 | 271 | 261 | 774 | 1296 |
| NM_031435 | THAP2 | 0.00 | 0.59 | 112 | 134 | 100 | 200 |
| NM_005098 | MSC | 0.00 | 0.59 | 757 | 818 | 649 | 1096 |
| NM_025160 | WDR26 | 0.00 | 0.59 | 2963 | 2876 | 3612 | 5712 |
| NM_015061 | KDM4C | 0.00 | 0.59 | 561 | 593 | 701 | 1177 |
| NM_015833 | ADARB1 | 0.00 | 0.59 | 731 | 723 | 455 | 784 |
| NM_001199771 | RDH5 | 0.00 | 0.59 | 122 | 104 | 195 | 360 |
| NM_001102603 | ZNF160 | 0.00 | 0.59 | 366 | 422 | 507 | 867 |
| NM_025081 | NYNRIN | 0.00 | 0.58 | 1139 | 1257 | 1714 | 2760 |
| NM_001144903 | TM6SF1 | -0.44 | 0.58 | 96 | 47 | 60 | 129 |
| NM_007257 | PNMA2 | 0.00 | 0.58 | 480 | 554 | 216 | 394 |
| NM_020801 | ARRDC3 | 0.00 | 0.58 | 365 | 405 | 455 | 782 |
| NM_004031 | IRF7 | 0.00 | 0.58 | 94 | 119 | 73 | 152 |
| NM_152527 | SLC16A1 4 | 0.00 | 0.58 | 6 | 9 | 54 | 118 |
| NM_001001713 | SH3BGR | 0.00 | 0.58 | 3 | 9 | 54 | 118 |
| NM_001258205 | TRPV3 | -0.24 | 0.58 | 99 | 57 | 54 | 118 |
| NM_015650 | TRAF3IP 1 | 0.00 | 0.58 | 234 | 261 | 283 | 503 |
| NM_022374 | ATL2 | 0.00 | 0.58 | 416 | 473 | 372 | 647 |
| NM_015448 | DPCD | 0.00 | 0.58 | 156 | 189 | 345 | 603 |
| NM_198935 | SS18L1 | -0.12 | 0.58 | 456 | 355 | 289 | 512 |
| NM_203314 | BDH1 | 0.00 | 0.58 | 70 | 56 | 35 | 83 |
| NM_015513 | CRELD1 | 0.00 | 0.58 | 1032 | 926 | 1951 | 3111 |
| NM_004612 | TGFBR1 | 0.00 | 0.58 | 2002 | 2082 | 3583 | 5612 |
| NM_001118887 | ANGPT2 | -0.47 | 0.58 | 430 | 259 | 89 | 179 |
| NM_006896 | HOXA7 | 0.00 | 0.57 | 213 | 221 | 195 | 357 |
| NM_030790 | ITFG1 | 0.00 | 0.57 | 1067 | 1164 | 2398 | 3793 |
| NM_002228 | JUN | 0.00 | 0.57 | 5158 | 5103 | 4862 | 7553 |
| NM_001284341 | FKBP9 | 0.00 | 0.57 | 5348 | 5604 | 8333 | 12809 |
| NM_013309 | SLC30A4 | 0.00 | 0.57 | 293 | 342 | 441 | 754 |
| NM_004671 | PIAS2 | 0.00 | 0.57 | 418 | 406 | 578 | 970 |
| NM_174899 | FBXO36 | 0.00 | 0.57 | 85 | 101 | 175 | 323 |
| NM_017623 | CNNM3 | 0.00 | 0.57 | 576 | 632 | 435 | 743 |
| NM_001128303 | LACC1 | 0.00 | 0.57 | 131 | 167 | 512 | 865 |
| NM_022897 | RANBP17 | 0.00 | 0.57 | 56 | 71 | 160 | 298 |
| NM_145799 | SEPT6 | 0.00 | 0.57 | 587 | 643 | 1321 | 2124 |
| NM_000690 | ALDH2 | 0.00 | 0.57 | 326 | 370 | 622 | 1037 |
| NM_001013732 | PTCHD4 | 0.00 | 0.57 | 10 | 8 | 116 | 224 |
| NM_001004067 | NOMO3 | 0.00 | 0.57 | 4823 | 4775 | 7054 | 10830 |

(continued)

| RefSeq ID | Gene Symbol | SR GFOLD | SEN GFOLD | SR IL-2 - | SR IL-2 + | SEN IL-2- | SEN IL-2+ |
|---|---|---|---|---|---|---|---|
| NM_006252 | PRKAA2 | 0.00 | 0.57 | 92 | 89 | 175 | 322 |
| NM_001042544 | LTBP4 | 0.00 | 0.57 | 201 | 217 | 92 | 183 |
| NM_001220488 | CDH13 | 0.00 | 0.56 | 4350 | 4553 | 7104 | 10887 |
| NM_178450 | MARCH3 | -0.01 | 0.56 | 369 | 307 | 154 | 287 |
| NM_018590 | CSGALN ACT2 | 0.01 | 0.56 | 1369 | 1500 | 1233 | 1983 |
| NM_001242828 | ELOVL5 | 0.00 | 0.56 | 2034 | 2092 | 2437 | 3821 |
| NM_014028 | OSTM1 | 0.00 | 0.56 | 1259 | 1352 | 2335 | 3665 |
| NM_005272 | GNAT2 | 0.00 | 0.56 | 29 | 15 | 56 | 120 |
| NM_001130858 | PPIP5K1 | 0.00 | 0.56 | 250 | 205 | 903 | 1469 |
| NM_003337 | UBE2B | 0.00 | 0.56 | 466 | 481 | 693 | 1142 |
| NM_000165 | GJA1 | 0.00 | 0.56 | 5578 | 5681 | 5773 | 8845 |
| NM_023071 | SPATS2 | -0.04 | 0.56 | 1173 | 1035 | 1352 | 2157 |
| NM_198463 | C3orf67 | 0.00 | 0.56 | 70 | 85 | 98 | 192 |
| NM_001242801 | ZNF790 | 0.00 | 0.56 | 40 | 63 | 98 | 192 |
| NM_001146254 | PODNL1 | 0.00 | 0.56 | 108 | 121 | 279 | 488 |
| NM_001024807 | APLP1 | 0.00 | 0.56 | 172 | 199 | 455 | 767 |
| NM_030759 | NRBF2 | 0.00 | 0.56 | 588 | 584 | 755 | 1235 |
| NM_001282716 | STAG3 | 0.00 | 0.56 | 94 | 121 | 166 | 305 |
| NM_138448 | ACYP2 | -0.17 | 0.55 | 112 | 70 | 146 | 272 |
| NM_001285878 | CEBPB | -0.05 | 0.55 | 2040 | 1825 | 2240 | 3503 |
| NM_015016 | MAST3 | 0.00 | 0.55 | 305 | 271 | 497 | 832 |
| NM_173614 | NOMO2 | 0.00 | 0.55 | 5918 | 5975 | 8678 | 13144 |
| NM_024615 | PARP8 | 0.00 | 0.55 | 152 | 169 | 547 | 909 |
| NM_001135036 | TVP23C | 0.00 | 0.55 | 205 | 183 | 243 | 429 |
| NM_001083617 | RB1CC1 | 0.00 | 0.55 | 1220 | 1285 | 1890 | 2965 |
| NM_004040 | RHOB | 0.00 | 0.55 | 4756 | 4726 | 3720 | 5723 |
| NM_003235 | TG | 0.00 | 0.55 | 27 | 16 | 48 | 105 |
| NM_199054 | MKNK2 | 0.00 | 0.55 | 1813 | 1878 | 1647 | 2591 |
| NM_015052 | HECW1 | 0.00 | 0.55 | 10 | 24 | 131 | 246 |
| NM_080660 | ZC3HAV 1L | 0.00 | 0.55 | 182 | 198 | 191 | 344 |
| NM_000428 | LTBP2 | 0.00 | 0.55 | 21013 | 20804 | 44866 | 66470 |
| NM_001204478 | TVP23C-CDRT4 | 0.00 | 0.55 | 190 | 202 | 252 | 442 |
| NM_177924 | ASAH1 | 0.00 | 0.55 | 565 | 629 | 2683 | 4148 |
| NM_000875 | IGF1R | 0.00 | 0.54 | 2519 | 2666 | 3180 | 4891 |
| NM_007266 | GPN1 | 0.00 | 0.54 | 739 | 807 | 1025 | 1640 |
| NM_001134878 | KIF9 | 0.00 | 0.54 | 75 | 81 | 112 | 214 |
| NM_015274 | MAN2B2 | 0.00 | 0.54 | 1855 | 2002 | 3870 | 5922 |
| NM_012139 | SERGEF | 0.00 | 0.54 | 140 | 156 | 220 | 390 |

(continued)

| RefSeq ID | Gene Symbol | SR GFOLD | SEN GFOLD | SR IL-2 - | SR IL-2 + | SEN IL-2- | SEN IL-2+ |
|---|---|---|---|---|---|---|---|
| NM_021244 | RRAGD | 0.00 | 0.54 | 43 | 45 | 356 | 606 |
| NM_002332 | LRP1 | -0.08 | 0.54 | 60577 | 56575 | 116461 | 171215 |
| NM_020702 | KIAA116 1 | 0.00 | 0.54 | 156 | 200 | 270 | 469 |
| NM_001287491 | TET3 | 0.00 | 0.54 | 34 | 52 | 29 | 70 |
| NM_001080423 | GRIP2 | 0.00 | 0.54 | 62 | 83 | 29 | 70 |
| NM_001114634 | PLAG1 | 0.00 | 0.54 | 91 | 80 | 141 | 261 |
| NM_015407 | ABHD14 A | 0.00 | 0.54 | 282 | 314 | 518 | 856 |
| NM_001184940 | FAM219 A | 0.00 | 0.54 | 1318 | 1383 | 1360 | 2140 |
| NM_001172223 | MOB2 | 0.00 | 0.54 | 378 | 347 | 391 | 658 |
| NM_001190274 | FBXO11 | 0.00 | 0.54 | 973 | 876 | 809 | 1301 |
| NM_003060 | SLC22A5 | 0.00 | 0.53 | 61 | 67 | 171 | 309 |
| NM_001172624 | NEO1 | 0.00 | 0.53 | 910 | 983 | 2462 | 3787 |
| NM_024501 | HOXD1 | 0.00 | 0.53 | 144 | 114 | 541 | 889 |
| NM_152402 | TRAM1L 1 | 0.00 | 0.53 | 225 | 183 | 279 | 481 |
| NM_001248008 | SKIL | 0.00 | 0.53 | 1951 | 1985 | 2410 | 3706 |
| NM_024496 | IRF2BPL | 0.00 | 0.53 | 347 | 406 | 1048 | 1662 |
| NM_001718 | BMP6 | 0.00 | 0.53 | 1403 | 1344 | 977 | 1553 |
| NM_014797 | ZBTB24 | 0.00 | 0.53 | 363 | 315 | 358 | 604 |
| NM_000577 | IL1RN | 0.00 | 0.53 | 28 | 23 | 79 | 157 |
| NM_052868 | IGSF8 | 0.00 | 0.53 | 267 | 288 | 591 | 963 |
| NM_001214909 | ZNF48 | 0.00 | 0.53 | 193 | 210 | 168 | 303 |
| NM_001244808 | FOXP1 | 0.00 | 0.53 | 3787 | 3900 | 4001 | 6052 |
| NM_001013657 | GDPGP1 | 0.00 | 0.53 | 63 | 48 | 46 | 100 |
| NM_144629 | RFTN2 | 0.00 | 0.53 | 144 | 131 | 141 | 259 |
| NM_001288748 | P4HA3 | 0.00 | 0.53 | 174 | 195 | 900 | 1431 |
| NM_172016 | TRIM39 | 0.00 | 0.53 | 411 | 422 | 376 | 630 |
| NM_001286067 | C11orf54 | 0.00 | 0.53 | 167 | 184 | 116 | 218 |
| NM_033411 | RWDD2A | 0.00 | 0.52 | 101 | 106 | 104 | 198 |
| NM_004186 | SEMA3F | 0.00 | 0.52 | 897 | 952 | 389 | 649 |
| NM_018366 | BLOC1S4 | 0.00 | 0.52 | 276 | 232 | 243 | 421 |
| NM_001256267 | MYPN | -0.03 | 0.52 | 603 | 514 | 630 | 1018 |
| NM_001199861 | KCNAB2 | 0.00 | 0.52 | 267 | 266 | 493 | 808 |
| NM_025147 | COQ10B | -0.11 | 0.52 | 1232 | 1037 | 1073 | 1684 |
| NM_032875 | FBXL20 | 0.00 | 0.52 | 582 | 554 | 1009 | 1586 |
| NM_001627 | ALCAM | 0.00 | 0.52 | 9874 | 10091 | 13823 | 20299 |
| NM_001114 | ADCY7 | 0.00 | 0.52 | 2753 | 2901 | 4111 | 6166 |
| NM_001130690 | PDE10A | 0.00 | 0.52 | 213 | 191 | 179 | 318 |

(continued)

| RefSeq ID | Gene Symbol | SR GFOLD | SEN GFOLD | SR IL-2 - | SR IL-2 + | SEN IL-2- | SEN IL-2+ |
|---|---|---|---|---|---|---|---|
| NM_021943 | ZFAND3 | 0.00 | 0.52 | 1601 | 1734 | 1801 | 2762 |
| NM_001030055 | ARHGAP 5 | 0.00 | 0.51 | 1760 | 1725 | 2069 | 3157 |
| NM_002149 | HPCAL1 | 0.00 | 0.51 | 686 | 631 | 1701 | 2612 |
| NM_173557 | RNF152 | 0.00 | 0.51 | 354 | 322 | 314 | 529 |
| NM_016472 | GSKIP | 0.00 | 0.51 | 302 | 342 | 611 | 983 |
| NM_020354 | ENTPD7 | -0.07 | 0.51 | 1171 | 1008 | 1362 | 2107 |
| NM_173074 | PIGF | 0.00 | 0.51 | 439 | 436 | 801 | 1268 |
| NM_002731 | PRKACB | 0.00 | 0.51 | 344 | 358 | 643 | 1030 |
| NM_014960 | ARSG | 0.00 | 0.51 | 43 | 40 | 139 | 253 |
| NM_001408 | CELSR2 | -0.18 | 0.51 | 88 | 52 | 27 | 65 |
| NM_001145536 | C17orf107 | 0.00 | 0.51 | 52 | 53 | 27 | 65 |
| NM_002835 | PTPN12 | 0.00 | 0.51 | 1499 | 1594 | 2431 | 3678 |
| NM_000052 | ATP7A | 0.00 | 0.51 | 288 | 300 | 485 | 789 |
| NM_005929 | MFI2 | 0.00 | 0.51 | 279 | 332 | 316 | 530 |
| NM_013399 | CDIP1 | 0.00 | 0.51 | 951 | 970 | 1208 | 1871 |
| NM_173174 | PTK2B | 0.00 | 0.51 | 275 | 256 | 191 | 335 |
| NM_003255 | TIMP2 | 0.00 | 0.51 | 23515 | 23998 | 28668 | 41461 |
| NM_001130080 | IFI27 | 0.00 | 0.51 | 187 | 150 | 1007 | 1571 |
| NM_022164 | TINAGL1 | 0.00 | 0.51 | 4555 | 4721 | 2271 | 3434 |
| NM_006772 | SYNGAP 1 | 0.00 | 0.51 | 379 | 362 | 557 | 896 |
| NM_181093 | SCYL3 | 0.00 | 0.50 | 176 | 205 | 229 | 394 |
| NM_005402 | RALA | 0.00 | 0.50 | 1646 | 1781 | 2991 | 4482 |
| NM_000203 | IDUA | -0.38 | 0.50 | 329 | 206 | 559 | 898 |
| NM_001282511 | NPIPA8 | 0.00 | 0.50 | 370 | 341 | 389 | 640 |
| NM_001282507 | NPIPA7 | 0.00 | 0.50 | 370 | 341 | 389 | 640 |
| NM_001025389 | AMPD3 | 0.00 | 0.50 | 503 | 509 | 1258 | 1939 |
| NM_007157 | ZXDB | 0.00 | 0.50 | 306 | 294 | 395 | 649 |
| NM_001779 | CD58 | 0.00 | 0.50 | 139 | 142 | 214 | 370 |
| NM_198404 | KCTD4 | 0.00 | 0.50 | 45 | 30 | 25 | 61 |
| NM_004642 | CDK2AP 1 | 0.00 | 0.50 | 2854 | 2983 | 3529 | 5255 |
| NM_145257 | CCSAP | 0.00 | 0.50 | 329 | 301 | 227 | 390 |
| NM_014498 | GOLIM4 | -0.08 | 0.50 | 1160 | 994 | 1595 | 2431 |
| NM_000100 | CSTB | -0.15 | 0.50 | 3010 | 2546 | 7004 | 10268 |
| NM_152230 | IPMK | 0.00 | 0.50 | 127 | 141 | 114 | 211 |
| NM_015626 | WSB1 | 0.00 | 0.50 | 1027 | 1083 | 1266 | 1946 |
| NM_001204 | BMPR2 | 0.00 | 0.50 | 3312 | 3506 | 6528 | 9571 |
| NM_022151 | MOAP1 | 0.00 | 0.50 | 352 | 330 | 503 | 810 |
| NM_001164496 | CFAP44 | 0.00 | 0.50 | 70 | 89 | 143 | 257 |

(continued)

| RefSeq ID | Gene Symbol | SR GFOLD | SEN GFOLD | SR IL-2 - | SR IL-2 + | SEN IL-2- | SEN IL-2+ |
|---|---|---|---|---|---|---|---|
| NM_020651 | PELI1 | 0.00 | 0.50 | 141 | 158 | 89 | 170 |
| NM_139025 | ADAMTS 13 | -0.13 | 0.50 | 41 | 20 | 46 | 98 |
| NM_018490 | LGR4 | 0.00 | 0.49 | 445 | 499 | 980 | 1519 |
| NM_173485 | TSHZ2 | 0.00 | 0.49 | 13 | 10 | 214 | 368 |
| NM_001198568 | ADCY4 | 0.00 | 0.49 | 293 | 316 | 264 | 445 |
| NM_173519 | CLVS1 | 0.00 | 0.49 | 310 | 293 | 630 | 998 |
| NM_000351 | STS | 0.00 | 0.49 | 858 | 951 | 1774 | 2680 |
| NM_022740 | HIPK2 | 0.00 | 0.49 | 6503 | 6325 | 11039 | 15965 |
| NM_001710 | CFB | 0.00 | 0.49 | 69 | 53 | 64 | 128 |
| NM_024686 | TTLL7 | 0.00 | 0.49 | 41 | 40 | 166 | 292 |
| NM_001145306 | CDK6 | 0.00 | 0.49 | 2799 | 2933 | 2683 | 3991 |
| NM_001039886 | ZNF808 | 0.00 | 0.49 | 223 | 244 | 327 | 540 |
| NM_015680 | CNPPD1 | 0.00 | 0.49 | 1507 | 1493 | 2489 | 3710 |
| NM_001037582 | SCD5 | -0.04 | 0.49 | 284 | 224 | 378 | 617 |
| NM_001039703 | NBPF10 | -0.04 | 0.49 | 669 | 570 | 1042 | 1603 |
| NM_001079526 | IKZF2 | 0.00 | 0.49 | 73 | 64 | 44 | 94 |
| NM_022365 | DNAJC1 | 0.00 | 0.49 | 830 | 891 | 936 | 1447 |
| NM_000514 | GDNF | 0.00 | 0.49 | 2980 | 3166 | 1782 | 2682 |
| NM_153607 | CREBRF | 0.00 | 0.49 | 225 | 278 | 526 | 839 |
| NM_002127 | HLA-G | 0.00 | 0.49 | 392 | 454 | 630 | 994 |
| NM_005252 | FOS | 0.00 | 0.49 | 109 | 122 | 54 | 111 |
| NM_001080424 | KDM6B | 0.00 | 0.49 | 448 | 425 | 358 | 586 |
| NM_001136499 | ZNF841 | 0.00 | 0.49 | 211 | 229 | 717 | 1122 |
| NM_001278267 | NBPF20 | -0.23 | 0.49 | 834 | 631 | 1190 | 1817 |
| NM_003437 | ZNF136 | 0.00 | 0.49 | 135 | 120 | 114 | 209 |
| NM_133466 | ZFP82 | 0.00 | 0.49 | 171 | 136 | 143 | 255 |
| NM_006547 | IGF2BP3 | 0.00 | 0.48 | 22 | 34 | 200 | 344 |
| NM_001103184 | FMN1 | 0.00 | 0.48 | 755 | 738 | 873 | 1351 |
| NM_144690 | ZNF582 | 0.00 | 0.48 | 8 | 13 | 50 | 104 |
| NM_005070 | SLC4A3 | 0.00 | 0.48 | 93 | 65 | 260 | 436 |
| NM_003287 | TPD52L1 | 0.00 | 0.48 | 139 | 128 | 264 | 442 |
| NM_000697 | ALOX12 | 0.00 | 0.48 | 47 | 36 | 62 | 124 |
| NM_080836 | STK35 | 0.00 | 0.48 | 1060 | 1161 | 1431 | 2163 |
| NM_001113482 | MANEAL | -0.40 | 0.48 | 40 | 15 | 62 | 124 |
| NM_001769 | CD9 | 0.00 | 0.48 | 1309 | 1388 | 3637 | 5336 |
| NM_032788 | ZNF514 | 0.00 | 0.48 | 195 | 219 | 275 | 458 |
| NM_002350 | LYN | -0.03 | 0.48 | 223 | 172 | 312 | 514 |
| NM_001014447 | CPZ | 0.00 | 0.48 | 6 | 17 | 81 | 155 |

(continued)

| RefSeq ID | Gene Symbol | SR GFOLD | SEN GFOLD | SR IL-2 - | SR IL-2 + | SEN IL-2- | SEN IL-2+ |
|---|---|---|---|---|---|---|---|
| NM_144736 | NDUFAF 7 | 0.00 | 0.48 | 203 | 165 | 372 | 604 |
| NM_021818 | SAV1 | 0.00 | 0.48 | 675 | 704 | 609 | 957 |
| NM_015348 | TMEM13 1 | 0.00 | 0.48 | 1577 | 1708 | 3893 | 5691 |
| NM_017918 | CCDC109 B | 0.00 | 0.48 | 261 | 270 | 304 | 501 |
| NM_033429 | CALML4 | 0.00 | 0.48 | 153 | 167 | 123 | 222 |
| NM_001271441 | C21orf2 | 0.00 | 0.48 | 183 | 206 | 200 | 342 |
| NM_012413 | QPCT | 0.00 | 0.48 | 300 | 270 | 539 | 852 |
| NM_024336 | IRX3 | 0.00 | 0.48 | 228 | 223 | 297 | 490 |
| NM_032017 | STK40 | 0.00 | 0.48 | 848 | 906 | 851 | 1311 |
| NM_032854 | CORO6 | 0.00 | 0.47 | 153 | 185 | 341 | 556 |
| NM_007202 | AKAP10 | 0.00 | 0.47 | 581 | 570 | 801 | 1237 |
| NM_032383 | HPS3 | 0.00 | 0.47 | 752 | 809 | 1025 | 1564 |
| NM_001278740 | TSPAN6 | 0.00 | 0.47 | 258 | 216 | 526 | 832 |
| NM_007285 | GABARA PL2 | 0.00 | 0.47 | 513 | 546 | 903 | 1386 |
| NM_004183 | BEST1 | -0.03 | 0.47 | 165 | 123 | 424 | 680 |
| NM_016032 | ZDHHC9 | 0.00 | 0.47 | 1352 | 1460 | 2820 | 4146 |
| NM_198335 | GANAB | 0.00 | 0.47 | 14444 | 14592 | 20102 | 28519 |
| NM_001172655 | ZNF701 | 0.00 | 0.47 | 93 | 120 | 150 | 264 |
| NM_005013 | NUCB2 | 0.00 | 0.47 | 1298 | 1218 | 1429 | 2146 |
| NM_003999 | OSMR | 0.00 | 0.47 | 4665 | 4563 | 4334 | 6286 |
| NM_030768 | ILKAP | 0.00 | 0.47 | 420 | 396 | 393 | 632 |
| NM_001008401 | ZNF761 | 0.00 | 0.47 | 220 | 220 | 254 | 423 |
| NM_017659 | QPCTL | 0.00 | 0.47 | 532 | 555 | 1017 | 1547 |
| NM_203371 | FIBIN | 0.00 | 0.47 | 57 | 64 | 345 | 560 |
| NM_032927 | TMEM12 8 | 0.00 | 0.47 | 203 | 201 | 218 | 368 |
| NM_004272 | HOMER1 | 0.00 | 0.47 | 203 | 244 | 218 | 368 |
| NM_020123 | TM9SF3 | 0.00 | 0.47 | 4186 | 4396 | 5561 | 8015 |
| NM_015044 | GGA2 | 0.00 | 0.47 | 1200 | 1265 | 1701 | 2532 |
| NM_004714 | DYRK1B | 0.00 | 0.47 | 183 | 167 | 289 | 475 |
| NM_012110 | CHIC2 | 0.00 | 0.47 | 419 | 462 | 435 | 693 |
| NM_024848 | MORN1 | 0.00 | 0.47 | 39 | 35 | 67 | 131 |
| NM_021255 | PELI2 | 0.00 | 0.47 | 119 | 125 | 131 | 233 |
| NM_012142 | CCNDBP 1 | 0.00 | 0.47 | 1075 | 974 | 1718 | 2551 |
| NM_001024845 | SLC6A9 | 0.00 | 0.47 | 611 | 587 | 308 | 503 |
| NM_001289125 | IFNAR2 | 0.00 | 0.47 | 344 | 333 | 381 | 612 |
| NM_001282116 | RFX3 | 0.00 | 0.46 | 103 | 78 | 46 | 96 |
| NM_178140 | PDZD2 | 0.00 | 0.46 | 54 | 41 | 46 | 96 |
| NM_145276 | ZNF563 | 0.00 | 0.46 | 35 | 45 | 58 | 116 |

(continued)

| RefSeq ID | Gene Symbol | SR GFOLD | SEN GFOLD | SR IL-2 - | SR IL-2 + | SEN IL-2- | SEN IL-2+ |
|---|---|---|---|---|---|---|---|
| NM_001099434 | DCDC2B | 0.00 | 0.46 | 36 | 28 | 58 | 116 |
| NM_172037 | RDH10 | 0.00 | 0.46 | 573 | 613 | 543 | 850 |
| NM_080491 | GAB2 | 0.00 | 0.46 | 1265 | 1338 | 1818 | 2686 |
| NM_020699 | GATAD2 B | 0.00 | 0.46 | 1123 | 1082 | 1215 | 1823 |
| NM_176814 | ZNF800 | 0.00 | 0.46 | 568 | 621 | 724 | 1113 |
| NM_001284274 | HECTD2 | 0.00 | 0.46 | 82 | 73 | 158 | 274 |
| NM_016085 | ATRAID | 0.00 | 0.46 | 1220 | 1326 | 2186 | 3207 |
| NM_001271928 | KIF27 | 0.00 | 0.46 | 38 | 21 | 54 | 109 |
| NM_001195608 | PLEKHA 1 | 0.00 | 0.46 | 716 | 676 | 713 | 1096 |
| NM_018842 | BAIAP2L 1 | -0.15 | 0.46 | 588 | 457 | 595 | 924 |
| NM_001001664 | SPOPL | 0.00 | 0.46 | 357 | 356 | 329 | 532 |
| NM_006565 | CTCF | 0.00 | 0.46 | 972 | 902 | 859 | 1307 |
| NM_000527 | LDLR | 0.00 | 0.46 | 2516 | 2501 | 4045 | 5828 |
| NM_001040457 | RHBDD2 | 0.00 | 0.46 | 979 | 942 | 2649 | 3858 |
| NM_005957 | MTHFR | -0.01 | 0.46 | 680 | 594 | 821 | 1251 |
| NM_001291860 | HSPG2 | -0.13 | 0.46 | 26212 | 23496 | 31269 | 43654 |
| NM_145021 | MARCH8 | 0.00 | 0.46 | 511 | 552 | 1015 | 1530 |
| NM_030777 | SLC2A10 | 0.00 | 0.46 | 1958 | 1892 | 2154 | 3152 |
| NM_002312 | LIG4 | 0.00 | 0.45 | 184 | 216 | 272 | 445 |
| NM_014636 | RALGPS1 | 0.00 | 0.45 | 30 | 20 | 89 | 165 |
| NM_021089 | ZNF8 | 0.00 | 0.45 | 187 | 235 | 295 | 479 |
| NM_001005404 | YPEL2 | 0.00 | 0.45 | 159 | 196 | 559 | 867 |
| NM_016498 | MTFP1 | 0.00 | 0.45 | 180 | 218 | 58 | 115 |
| NM_172027 | ABTB1 | 0.00 | 0.45 | 235 | 279 | 586 | 906 |
| NM_001195263 | PDZD7 | -0.42 | 0.45 | 223 | 129 | 231 | 383 |
| NM_001009955 | SSBP3 | 0.00 | 0.45 | 373 | 373 | 530 | 824 |
| NM_001167856 | SBNO1 | 0.00 | 0.45 | 1973 | 2010 | 2283 | 3322 |
| NM_006260 | DNAJC3 | 0.00 | 0.45 | 2248 | 2403 | 2872 | 4146 |
| NM_006045 | ATP9A | 0.00 | 0.45 | 1685 | 1807 | 5043 | 7177 |
| NM_001276435 | KCNJ15 | 0.00 | 0.44 | 38 | 46 | 150 | 259 |
| NM_001204883 | RAB43 | 0.00 | 0.44 | 779 | 754 | 838 | 1264 |
| NM_004261 | SEP15 | 0.00 | 0.44 | 1539 | 1644 | 2227 | 3229 |
| NM_004538 | NAP1L3 | 0.00 | 0.44 | 11 | 14 | 48 | 98 |
| NM_001134707 | SARDH | -0.72 | 0.44 | 199 | 90 | 19 | 48 |
| NM_002917 | RFNG | 0.00 | 0.44 | 964 | 1019 | 1075 | 1601 |
| NM_004388 | CTBS | -0.09 | 0.44 | 389 | 307 | 591 | 908 |
| NM_018462 | BRK1 | 0.00 | 0.44 | 1676 | 1595 | 2618 | 3774 |
| NM_001101802 | PHF21A | 0.00 | 0.44 | 478 | 512 | 930 | 1394 |

(continued)

| RefSeq ID | Gene Symbol | SR GFOLD | SEN GFOLD | SR IL-2 - | SR IL-2 + | SEN IL-2- | SEN IL-2+ |
|---|---|---|---|---|---|---|---|
| NM_181333 | PRR5 | 0.00 | 0.44 | 54 | 35 | 64 | 124 |
| NM_000629 | IFNAR1 | -0.07 | 0.44 | 1096 | 942 | 1778 | 2593 |
| NM_007218 | RNF139 | 0.00 | 0.44 | 553 | 577 | 566 | 871 |
| NM_014232 | VAMP2 | 0.00 | 0.44 | 879 | 840 | 888 | 1333 |
| NM_001166345 | MDFIC | 0.00 | 0.44 | 1120 | 1138 | 2236 | 3235 |
| NM_052888 | LRRC37B | 0.00 | 0.44 | 112 | 143 | 185 | 311 |
| NM_020310 | MNT | 0.00 | 0.44 | 600 | 668 | 919 | 1375 |
| NM_012330 | KAT6B | 0.00 | 0.44 | 761 | 764 | 1111 | 1647 |
| NM_181453 | GCC2 | 0.00 | 0.44 | 622 | 601 | 1004 | 1495 |
| NM_018023 | YEATS2 | 0.00 | 0.44 | 2162 | 2291 | 2225 | 3211 |
| NM_207333 | ZNF320 | 0.00 | 0.44 | 167 | 189 | 293 | 471 |
| NM_033280 | SEC11C | 0.00 | 0.44 | 165 | 141 | 287 | 462 |
| NM_030665 | RAI1 | 0.00 | 0.44 | 791 | 829 | 1152 | 1702 |
| NM_001291415 | KDM6A | 0.01 | 0.43 | 551 | 635 | 572 | 876 |
| NM_033426 | CIPC | 0.00 | 0.43 | 214 | 240 | 462 | 717 |
| NM_014812 | CEP170 | 0.00 | 0.43 | 8327 | 8099 | 11240 | 15626 |
| NM_182491 | ZFAND2 A | -0.01 | 0.43 | 505 | 430 | 553 | 848 |
| NM_177938 | P4HTM | 0.00 | 0.43 | 302 | 359 | 809 | 1214 |
| NM_006734 | HIVEP2 | 0.00 | 0.43 | 2739 | 2790 | 3876 | 5495 |
| NM_014999 | RAB21 | 0.00 | 0.43 | 1433 | 1359 | 1518 | 2214 |
| NM_001172700 | SHROOM 1 | 0.00 | 0.43 | 172 | 153 | 310 | 495 |
| NM_001123392 | TBC1D3 H | 0.00 | 0.43 | 180 | 169 | 214 | 353 |
| NM_032258 | TBC1D3F | 0.00 | 0.43 | 178 | 167 | 210 | 347 |
| NM_001003940 | BMF | 0.00 | 0.43 | 16 | 21 | 139 | 240 |
| NM_170753 | PGBD3 | 0.00 | 0.43 | 393 | 349 | 341 | 540 |
| NM_032561 | C22orf23 | 0.00 | 0.43 | 895 | 818 | 616 | 937 |
| NM_002850 | PTPRS | 0.00 | 0.43 | 1513 | 1624 | 4130 | 5835 |
| NM_024312 | GNPTAB | 0.00 | 0.43 | 1067 | 1117 | 2082 | 2996 |
| NM_014117 | C 16orf72 | 0.00 | 0.43 | 1360 | 1438 | 2647 | 3780 |
| NM_003107 | SOX4 | 0.00 | 0.43 | 420 | 440 | 767 | 1150 |
| NM_001134655 | ZNF213 | 0.00 | 0.43 | 300 | 288 | 335 | 530 |
| NM_001040445 | ASB1 | 0.00 | 0.43 | 2965 | 2938 | 3827 | 5407 |
| NM_014044 | UNC50 | 0.00 | 0.43 | 596 | 517 | 740 | 1111 |
| NM_030802 | FAM117 A | 0.00 | 0.43 | 42 | 52 | 112 | 198 |
| NM_001163922 | VSIG10L | -0.30 | 0.43 | 119 | 68 | 112 | 198 |
| NM_018215 | PNMAL1 | 0.00 | 0.43 | 111 | 148 | 299 | 477 |
| NM_004454 | ETV5 | 0.00 | 0.43 | 1619 | 1624 | 2689 | 3832 |
| NM_016433 | GLTP | 0.00 | 0.43 | 2313 | 2364 | 2760 | 3930 |

(continued)

| RefSeq ID | Gene Symbol | SR GFOLD | SEN GFOLD | SR IL-2 - | SR IL-2 + | SEN IL-2- | SEN IL-2+ |
|---|---|---|---|---|---|---|---|
| NM_001252127 | AP4E1 | 0.00 | 0.43 | 629 | 585 | 755 | 1131 |
| NM_005665 | EVI5 | 0.00 | 0.42 | 893 | 874 | 894 | 1327 |
| NM_004369 | COL6A3 | -0.11 | 0.42 | 31491 | 28566 | 126573 | 171367 |
| NM_001015880 | PAPSS2 | 0.00 | 0.42 | 7619 | 7633 | 6316 | 8809 |
| NM_001003795 | GTF2IRD 2B | 0.00 | 0.42 | 142 | 127 | 195 | 323 |

| RefSeq ID | Gene Symbol | SR GFOLD | SEN GFOLD | SR IL-2 | SR IL-2 + | SEN IL-2 - | SEN IL-2 + |
|---|---|---|---|---|---|---|---|
| NM_024784 | ZBTB3 | 0.00 | 0.42 | 153 | 164 | 177 | 296 |
| NM_001291462 | LOC1010 60321 | 0.00 | 0.42 | 179 | 169 | 214 | 351 |
| NM_020765 | UBR4 | 0.01 | 0.42 | 14337 | 14831 | 24696 | 33768 |
| NM_080489 | SDCBP2 | -0.08 | 0.42 | 103 | 68 | 100 | 179 |
| NM_152778 | MFSD8 | 0.00 | 0.42 | 160 | 165 | 202 | 333 |
| NM_173822 | FAM126B | -0.14 | 0.42 | 271 | 198 | 324 | 512 |
| NM_176891 | IFNE | 0.00 | 0.42 | 9 | 11 | 121 | 211 |
| NM_020729 | ODF2L | -0.10 | 0.42 | 309 | 235 | 345 | 542 |
| NM_024541 | C10orf76 | 0.00 | 0.42 | 416 | 397 | 630 | 950 |
| NM_016049 | EMC9 | 0.00 | 0.42 | 178 | 190 | 191 | 316 |
| NM_001014283 | DCUN1D 2 | 0.00 | 0.42 | 293 | 293 | 364 | 569 |
| NM_025106 | SPSB1 | 0.00 | 0.42 | 487 | 555 | 497 | 760 |
| NM_032995 | ARHGEF 4 | 0.00 | 0.42 | 172 | 137 | 349 | 547 |
| NM_080669 | SLC46A1 | 0.00 | 0.42 | 440 | 493 | 853 | 1264 |
| NM_005981 | TSPAN31 | 0.00 | 0.42 | 366 | 399 | 1059 | 1553 |
| NM_001755 | CBFB | 0.00 | 0.42 | 1496 | 1606 | 1947 | 2786 |
| NM_018378 | FBXL8 | 0.00 | 0.42 | 67 | 54 | 83 | 152 |
| NM_001018116 | MURC | 0.00 | 0.42 | 48 | 58 | 83 | 152 |
| NM_018197 | ZFP64 | 0.00 | 0.42 | 400 | 426 | 530 | 806 |
| NM_001256619 | R3HCC1L | -0.14 | 0.42 | 414 | 315 | 306 | 484 |
| NM_004747 | DLG5 | 0.00 | 0.42 | 2751 | 2780 | 4972 | 6930 |
| NM_002668 | PLP2 | -0.01 | 0.42 | 3124 | 2912 | 4382 | 6124 |
| NM_001963 | EGF | 0.00 | 0.42 | 28 | 30 | 64 | 122 |
| NM_015021 | ZNF292 | 0.00 | 0.42 | 798 | 821 | 859 | 1270 |
| NM_001135575 | SMIM13 | 0.00 | 0.42 | 347 | 384 | 374 | 582 |
| NM_001896 | CSNK2A 2 | 0.00 | 0.42 | 824 | 730 | 678 | 1015 |
| NM_000854 | GSTT2 | 0.00 | 0.42 | 178 | 174 | 356 | 556 |
| NM_001080843 | GSTT2B | 0.00 | 0.42 | 178 | 174 | 356 | 556 |
| NM_022158 | FN3K | 0.00 | 0.42 | 67 | 95 | 146 | 248 |
| NM_019072 | SGTB | -0.04 | 0.41 | 1257 | 1111 | 1493 | 2152 |
| NM_016530 | RAB8B | 0.00 | 0.41 | 821 | 780 | 1470 | 2120 |
| NM_020971 | SPTBN4 | 0.00 | 0.41 | 42 | 40 | 89 | 161 |

(continued)

| RefSeq ID | Gene Symbol | SR GFOLD | SEN GFOLD | SR IL-2 | SR IL-2 + | SEN IL-2 - | SEN IL-2 + |
|---|---|---|---|---|---|---|---|
| NM_001148 | ANK2 | 0.00 | 0.41 | 100 | 120 | 1822 | 2606 |
| NM_004490 | GRB14 | 0.00 | 0.41 | 46 | 31 | 177 | 294 |
| NM_001042483 | NUPR1 | -0.04 | 0.41 | 1550 | 1379 | 2785 | 3930 |
| NM_018216 | PANK4 | 0.00 | 0.41 | 1151 | 1070 | 780 | 1157 |
| NM_152271 | LONRF1 | 0.00 | 0.41 | 184 | 197 | 106 | 187 |
| NM_022900 | CASD1 | 0.00 | 0.41 | 124 | 117 | 135 | 231 |
| NM_001136265 | IFFO2 | -0.05 | 0.41 | 1768 | 1576 | 1618 | 2322 |
| NM_012421 | RLF | 0.00 | 0.41 | 612 | 646 | 686 | 1024 |
| NM_001040182 | CLDND1 | -0.03 | 0.41 | 1181 | 1047 | 942 | 1383 |
| NM_148894 | BOD1L1 | -0.01 | 0.41 | 1437 | 1307 | 1971 | 2808 |
| NM_015102 | NPHP4 | 0.00 | 0.41 | 376 | 405 | 603 | 906 |
| NM_014106 | ZNF770 | 0.00 | 0.41 | 807 | 848 | 842 | 1242 |
| NM_014649 | SAFB2 | 0.00 | 0.41 | 617 | 568 | 470 | 717 |
| NM_017983 | WIPI1 | 0.00 | 0.41 | 638 | 661 | 1242 | 1798 |
| NM_003801 | GPAA1 | 0.00 | 0.41 | 1613 | 1646 | 2589 | 3651 |
| NM_001165958 | GSDMB | 0.00 | 0.41 | 67 | 85 | 154 | 259 |
| NM_014315 | KLHDC2 | 0.01 | 0.41 | 544 | 627 | 728 | 1081 |
| NM_014506 | TOR1B | 0.00 | 0.41 | 1792 | 1732 | 3662 | 5115 |
| NM_025179 | PLXNA2 | -0.01 | 0.41 | 3653 | 3428 | 5203 | 7209 |
| NM_006454 | MXD4 | 0.00 | 0.41 | 1138 | 1246 | 2566 | 3617 |
| NM_001144757 | SCG5 | 0.00 | 0.41 | 589 | 583 | 992 | 1449 |
| NM_001286398 | RNF217 | 0.00 | 0.41 | 305 | 326 | 358 | 556 |
| NM_014746 | RNF144A | 0.00 | 0.41 | 15 | 16 | 264 | 420 |
| NM_144964 | TRMT10 B | 0.00 | 0.41 | 102 | 138 | 127 | 218 |
| NM_004854 | CHST10 | 0.00 | 0.41 | 252 | 285 | 223 | 360 |
| NM_000801 | FKBP1A | -0.03 | 0.41 | 4437 | 4137 | 9144 | 12506 |
| NM_014322 | OPN3 | 0.00 | 0.41 | 339 | 359 | 208 | 338 |
| NM_138426 | GLCCI1 | 0.00 | 0.40 | 401 | 387 | 281 | 444 |
| NM_014938 | MLXIP | -0.05 | 0.40 | 2329 | 2103 | 2959 | 4140 |
| NM_001271872 | SRGAP2 C | 0.00 | 0.40 | 1012 | 1093 | 1410 | 2022 |
| NM_001278535 | NEURL2 | -0.74 | 0.40 | 71 | 25 | 44 | 89 |
| NM_001270384 | FAM73A | 0.00 | 0.40 | 667 | 730 | 1254 | 1806 |
| NM_005645 | TAF13 | 0.00 | 0.40 | 530 | 489 | 439 | 669 |
| NM_000296 | PKD1 | 0.00 | 0.40 | 2780 | 2693 | 3305 | 4603 |
| NM_053042 | ZNF518B | -0.05 | 0.40 | 940 | 815 | 1044 | 1514 |
| NM_002839 | PTPRD | 0.00 | 0.40 | 136 | 163 | 510 | 769 |
| NM_017658 | KLHL28 | 0.00 | 0.40 | 343 | 287 | 252 | 401 |
| NM_024756 | MMRN2 | 0.00 | 0.40 | 97 | 80 | 141 | 238 |

(continued)

| RefSeq ID | Gene Symbol | SR GFOLD | SEN GFOLD | SR IL-2 | SR IL-2 + | SEN IL-2 - | SEN IL-2 + |
|-----------|-------------|----------|-----------|---------|-----------|------------|------------|
| NM_021090 | MTMR3 | -0.14 | 0.40 | 1277 | 1055 | 1705 | 2421 |
| NM_006463 | STAMBP | 0.00 | 0.40 | 488 | 500 | 435 | 662 |
| NM_022110 | FKBPL | 0.00 | 0.40 | 106 | 138 | 79 | 144 |
| NM_018379 | FAM63A | 0.00 | 0.40 | 150 | 153 | 308 | 481 |
| NM_013995 | LAMP2 | 0.00 | 0.40 | 2337 | 2446 | 3452 | 4793 |
| NM_006699 | MAN1A2 | 0.00 | 0.40 | 922 | 888 | 1545 | 2200 |
| NM_212481 | ARID5A | 0.00 | 0.40 | 255 | 267 | 175 | 288 |
| NM_003129 | SQLE | 0.00 | 0.40 | 603 | 679 | 1643 | 2333 |
| NM_014935 | PLEKHA 6 | 0.00 | 0.40 | 78 | 57 | 150 | 251 |
| NM_001006612 | WBP5 | 0.00 | 0.40 | 1521 | 1555 | 1840 | 2601 |
| NM_138962 | MSI2 | 0.00 | 0.40 | 84 | 75 | 239 | 381 |
| NM_016248 | AKAP11 | 0.00 | 0.40 | 1624 | 1699 | 2976 | 4142 |
| NM_001129896 | SYTL4 | 0.00 | 0.40 | 350 | 342 | 378 | 580 |
| NM_004446 | EPRS | 0.00 | 0.40 | 3716 | 3604 | 6108 | 8357 |
| NM_001242809 | ANKRD6 | 0.00 | 0.40 | 234 | 199 | 537 | 804 |
| NM_001291832 | FAM132B | -0.07 | 0.39 | 91 | 59 | 258 | 408 |
| NM_001001872 | C14orf37 | 0.00 | 0.39 | 176 | 192 | 593 | 882 |
| NM_153188 | TNPO1 | 0.00 | 0.39 | 3316 | 3417 | 5555 | 7604 |
| NM_016353 | ZDHHC2 | -0.03 | 0.39 | 1262 | 1122 | 1312 | 1874 |
| NM_032423 | ZNF528 | 0.00 | 0.39 | 104 | 137 | 412 | 627 |
| NM_015092 | SMG1 | 0.00 | 0.39 | 2891 | 2989 | 4675 | 6418 |
| NM_018169 | KIAA155 1 | 0.00 | 0.39 | 410 | 359 | 420 | 638 |
| NM_024089 | KDELC1 | 0.00 | 0.39 | 207 | 252 | 391 | 597 |
| NM_005512 | LRRC32 | 0.00 | 0.39 | 4065 | 4076 | 12915 | 17410 |
| NM_001902 | CTH | 0.00 | 0.39 | 172 | 203 | 64 | 120 |
| NM_001285549 | ZDBF2 | 0.00 | 0.39 | 243 | 227 | 364 | 558 |
| NM_014230 | SRP68 | -0.11 | 0.39 | 3007 | 2624 | 2801 | 3889 |
| NM_001008211 | OPTN | 0.01 | 0.39 | 5063 | 5327 | 8454 | 11456 |
| NM_031886 | KCNA7 | 0.00 | 0.39 | 215 | 189 | 198 | 320 |
| NM_006609 | MAP3K2 | 0.00 | 0.39 | 1321 | 1391 | 1843 | 2591 |
| NM_020717 | SHROOM 4 | 0.00 | 0.39 | 204 | 223 | 518 | 774 |
| NM_015349 | GLTSCR1 L | 0.01 | 0.39 | 353 | 419 | 693 | 1017 |
| NM_153240 | NPHP3 | -0.21 | 0.39 | 593 | 442 | 832 | 1209 |
| NM_017851 | PARP16 | 0.00 | 0.39 | 154 | 197 | 158 | 261 |
| NM_024561 | NAA16 | 0.00 | 0.39 | 168 | 205 | 139 | 233 |
| NM_004460 | FAP | 0.00 | 0.39 | 1810 | 1767 | 4594 | 6279 |
| NM_001204817 | ZNF587 | -0.05 | 0.39 | 523 | 434 | 607 | 896 |
| NM_012135 | FAM50B | 0.00 | 0.39 | 279 | 242 | 241 | 381 |

(continued)

| RefSeq ID | Gene Symbol | SR GFOLD | SEN GFOLD | SR IL-2 | SR IL-2 + | SEN IL-2 - | SEN IL-2 + |
|---|---|---|---|---|---|---|---|
| NM_080607 | VSTM2L | 0.00 | 0.38 | 36 | 44 | 56 | 107 |
| NM_002114 | HIVEP1 | 0.00 | 0.38 | 1142 | 1217 | 1447 | 2046 |
| NM_001202560 | WBSCR2 2 | 0.01 | 0.38 | 821 | 924 | 1506 | 2126 |
| NM_000147 | FUCA1 | 0.00 | 0.38 | 111 | 84 | 387 | 588 |
| NM_031946 | AGAP3 | 0.00 | 0.38 | 1926 | 2019 | 2797 | 3867 |
| NM_001171816 | RNF166 | 0.00 | 0.38 | 188 | 216 | 208 | 333 |
| NM_000132 | F8 | 0.00 | 0.38 | 30 | 45 | 166 | 272 |
| NM_005938 | FOX04 | -0.01 | 0.38 | 308 | 251 | 343 | 525 |
| NM_001007523 | F8A2 | 0.00 | 0.38 | 389 | 342 | 460 | 689 |
| NM_001007524 | F8A3 | 0.00 | 0.38 | 389 | 342 | 460 | 689 |
| NM_001005463 | EBF3 | 0.00 | 0.38 | 502 | 520 | 780 | 1131 |
| NM_018434 | RNF130 | 0.00 | 0.38 | 324 | 386 | 934 | 1342 |
| NM_003908 | EIF2S2 | 0.00 | 0.38 | 1607 | 1689 | 2360 | 3270 |
| NM_005781 | TNK2 | 0.00 | 0.38 | 582 | 624 | 813 | 1176 |
| NM_001136234 | SUPT20H L1 | 0.00 | 0.38 | 17 | 22 | 73 | 133 |
| NM_031913 | ESYT3 | 0.00 | 0.38 | 75 | 95 | 150 | 248 |
| NM_133496 | SLC30A7 | 0.00 | 0.38 | 1042 | 1077 | 1217 | 1726 |
| NM_001291694 | NR2C2 | 0.00 | 0.38 | 1625 | 1559 | 1747 | 2442 |
| NM_014952 | BAHD1 | 0.00 | 0.38 | 386 | 394 | 520 | 771 |
| NM_001304 | CPD | -0.08 | 0.38 | 1422 | 1232 | 2868 | 3946 |
| NM_001271870 | SRGAP2 B | 0.00 | 0.38 | 988 | 1050 | 1346 | 1900 |
| NM_001277945 | ZNF83 | 0.00 | 0.38 | 267 | 304 | 455 | 680 |
| NM_001924 | GADD45 A | -0.32 | 0.38 | 1772 | 1300 | 1375 | 1937 |
| NM_006768 | BRAP | 0.00 | 0.38 | 419 | 483 | 636 | 930 |
| NM_001001330 | REEP3 | 0.00 | 0.38 | 1559 | 1474 | 1828 | 2547 |
| NM_013313 | YPEL1 | 0.00 | 0.38 | 87 | 97 | 158 | 259 |
| NM_024560 | ACSS3 | 0.00 | 0.38 | 25 | 29 | 158 | 259 |
| NM_024725 | CCDC82 | 0.00 | 0.38 | 354 | 344 | 416 | 625 |
| NM_001040185 | ZNF765 | 0.00 | 0.38 | 135 | 138 | 187 | 301 |
| NM_001079537 | TRAPPC6 B | 0.00 | 0.37 | 594 | 596 | 834 | 1200 |
| NM_001993 | F3 | 0.00 | 0.37 | 268 | 261 | 451 | 673 |
| NM_001006607 | LRRC37A 2 | 0.00 | 0.37 | 212 | 199 | 370 | 560 |
| NM_003162 | STRN | 0.00 | 0.37 | 821 | 916 | 1113 | 1579 |
| NM_025075 | THOC7 | 0.00 | 0.37 | 383 | 389 | 480 | 713 |
| NM_013272 | SLCO3A1 | -0.25 | 0.37 | 672 | 493 | 961 | 1372 |
| NM_175922 | PRR18 | -0.41 | 0.37 | 54 | 23 | 37 | 76 |
| NM_003799 | RNMT | 0.00 | 0.37 | 696 | 768 | 1059 | 1505 |
| NM_020928 | ZSWIM6 | 0.00 | 0.37 | 953 | 994 | 1079 | 1532 |

(continued)

| RefSeq ID | Gene Symbol | SR GFOLD | SEN GFOLD | SR IL-2 | SR IL-2 + | SEN IL-2 - | SEN IL-2 + |
|---|---|---|---|---|---|---|---|
| NM_006876 | B3GNT1 | 0.00 | 0.37 | 369 | 347 | 848 | 1218 |
| NM_005713 | COL4A3 BP | 0.00 | 0.37 | 602 | 668 | 1177 | 1664 |
| NM_014157 | CCDC113 | -0.04 | 0.37 | 82 | 53 | 175 | 283 |
| NM_198557 | RBM43 | 0.00 | 0.37 | 85 | 110 | 175 | 283 |
| NM_001098402 | ZBTB21 | 0.00 | 0.37 | 466 | 499 | 638 | 930 |
| NM_022458 | LMBR1 | 0.00 | 0.37 | 474 | 526 | 774 | 1116 |
| NM_018426 | TMEM63 B | 0.00 | 0.37 | 1331 | 1389 | 2600 | 3569 |
| NM_024744 | CARF | 0.00 | 0.37 | 87 | 88 | 160 | 261 |
| NM_014600 | EHD3 | 0.00 | 0.37 | 582 | 633 | 813 | 1168 |
| NM_024580 | EFTUD1 | 0.00 | 0.37 | 910 | 948 | 1510 | 2109 |
| NM_001204477 | CDRT4 | 0.00 | 0.37 | 150 | 149 | 183 | 294 |
| NM_145265 | CCDC127 | 0.00 | 0.37 | 309 | 364 | 530 | 780 |
| NM_025219 | DNAJC5 | -0.12 | 0.37 | 3547 | 3082 | 3833 | 5196 |
| NM_025191 | EDEM3 | 0.00 | 0.37 | 1097 | 1212 | 2182 | 3005 |
| NM_014155 | ZBTB44 | 0.00 | 0.37 | 475 | 526 | 613 | 893 |
| NM_015621 | CCDC69 | 0.00 | 0.37 | 61 | 67 | 137 | 227 |
| NM_020974 | SCUBE2 | 0.00 | 0.37 | 43 | 38 | 81 | 144 |
| NM_005450 | NOG | 0.00 | 0.37 | 149 | 122 | 135 | 224 |
| NM_032494 | ZC3H8 | 0.00 | 0.37 | 46 | 62 | 62 | 115 |
| NM_052911 | ESCO1 | 0.00 | 0.37 | 281 | 273 | 260 | 403 |
| NM_015038 | KIAA075 4 | 0.00 | 0.37 | 1854 | 1893 | 3361 | 4566 |
| NM_001278503 | STT3A | 0.00 | 0.37 | 5046 | 5237 | 6800 | 9094 |
| NM_001282903 | TRPS1 | 0.00 | 0.37 | 505 | 501 | 896 | 1277 |
| NM_000709 | BCKDHA | 0.00 | 0.36 | 673 | 665 | 782 | 1122 |
| NM_001278940 | GTF2A1 | 0.00 | 0.36 | 1071 | 1155 | 1054 | 1490 |
| NM_025164 | SIK3 | -0.02 | 0.36 | 749 | 653 | 1739 | 2408 |
| NM_032242 | PLXNA1 | 0.00 | 0.36 | 5485 | 5596 | 9523 | 12647 |
| NM_001012454 | FAM71F2 | 0.00 | 0.36 | 51 | 40 | 106 | 181 |
| NM_015966 | ERGIC3 | 0.00 | 0.36 | 3835 | 3977 | 6971 | 9299 |
| NM_001144769 | DST | -0.22 | 0.36 | 9290 | 7703 | 22150 | 29075 |
| NM_012151 | F8A1 | 0.00 | 0.36 | 612 | 575 | 753 | 1081 |
| NM_001256486 | GOLGB1 | 0.00 | 0.36 | 2455 | 2390 | 4488 | 6039 |
| NM_001284201 | JKAMP | 0.00 | 0.36 | 892 | 893 | 1566 | 2174 |
| NM_173542 | PLBD2 | -0.03 | 0.36 | 883 | 773 | 2909 | 3957 |
| NM_001271837 | RCN2 | 0.00 | 0.36 | 1133 | 1136 | 1984 | 2730 |
| NM_152313 | SLC36A4 | -0.06 | 0.36 | 368 | 295 | 424 | 630 |
| NM_130468 | CHST14 | 0.00 | 0.36 | 782 | 755 | 709 | 1020 |
| NM_003635 | NDST2 | 0.00 | 0.36 | 701 | 765 | 1054 | 1486 |

(continued)

| RefSeq ID | Gene Symbol | SR GFOLD | SEN GFOLD | SR IL-2 | SR IL-2 + | SEN IL-2 - | SEN IL-2 + |
|---|---|---|---|---|---|---|---|
| NM_020528 | PCBP3 | 0.00 | 0.36 | 61 | 38 | 6 | 26 |
| NM_018259 | TTC17 | 0.00 | 0.36 | 1086 | 1031 | 1508 | 2092 |
| NM_152914 | NATD1 | 0.00 | 0.36 | 357 | 351 | 406 | 604 |
| NM_001134651 | EIF4E3 | 0.00 | 0.36 | 126 | 101 | 349 | 525 |
| NM_001017969 | KIAA202 6 | 0.00 | 0.36 | 587 | 513 | 857 | 1218 |
| NM_198279 | CXorf23 | 0.00 | 0.36 | 119 | 126 | 268 | 412 |
| NM_032779 | CCDC142 | -0.05 | 0.36 | 279 | 219 | 316 | 479 |
| NM_001130726 | CCDC149 | 0.00 | 0.36 | 491 | 481 | 844 | 1200 |
| NM_001080496 | RGP1 | 0.00 | 0.36 | 566 | 501 | 501 | 734 |
| NM_004562 | PARK2 | -0.19 | 0.36 | 21 | 5 | 73 | 131 |
| NM_001136022 | NFATC4 | 0.00 | 0.36 | 1204 | 1237 | 2889 | 3915 |
| NM_021242 | MID1IP1 | 0.00 | 0.36 | 590 | 624 | 813 | 1157 |
| NM_001409 | MEGF6 | 0.00 | 0.36 | 2221 | 2211 | 2404 | 3274 |
| NM_001199260 | TOR1AIP 2 | 0.00 | 0.36 | 2900 | 2998 | 4159 | 5580 |
| NM_001360 | DHCR7 | 0.00 | 0.35 | 1147 | 1042 | 2475 | 3366 |
| NM_153605 | CRYBG3 | 0.00 | 0.35 | 1109 | 1082 | 1316 | 1830 |
| NM_001144013 | RGPD3 | 0.00 | 0.35 | 191 | 165 | 216 | 338 |
| NM_001288608 | ADAMTS L4 | 0.00 | 0.35 | 233 | 245 | 576 | 835 |
| NM_001040653 | ZXDC | 0.00 | 0.35 | 862 | 920 | 919 | 1298 |
| NM_006405 | TM9SF1 | 0.00 | 0.35 | 1614 | 1712 | 3111 | 4198 |
| NM_016651 | DACT1 | 0.00 | 0.35 | 802 | 850 | 1086 | 1521 |
| NM_001172659 | ZFYVE28 | 0.00 | 0.35 | 73 | 74 | 193 | 305 |
| NM_003966 | SEMA5A | 0.00 | 0.35 | 1095 | 1144 | 1691 | 2325 |
| NM_001281495 | CTNNBL 1 | 0.00 | 0.35 | 701 | 632 | 730 | 1042 |
| NM_001282167 | PORCN | 0.00 | 0.35 | 595 | 568 | 1452 | 2007 |
| NM_138463 | TLCD1 | 0.00 | 0.35 | 147 | 111 | 131 | 216 |
| NM_001284209 | MED6 | 0.00 | 0.35 | 439 | 384 | 403 | 597 |
| NM_001025100 | MBP | 0.00 | 0.35 | 450 | 476 | 476 | 697 |
| NM_015101 | COLGAL T2 | 0.00 | 0.35 | 185 | 202 | 545 | 791 |
| NM_005920 | MEF2D | 0.00 | 0.35 | 2638 | 2623 | 3086 | 4159 |
| NM_001135844 | SIRPB1 | 0.00 | 0.35 | 155 | 190 | 243 | 375 |
| NM_003112 | SP4 | -0.31 | 0.35 | 243 | 154 | 116 | 194 |
| NM_021942 | TRAPPC1 1 | 0.00 | 0.35 | 519 | 564 | 986 | 1384 |
| NM_025140 | CCDC92 | 0.00 | 0.35 | 1157 | 1194 | 2119 | 2885 |
| NM_002379 | MATN1 | -0.79 | 0.35 | 377 | 177 | 125 | 207 |
| NM_001010898 | SLC6A17 | 0.00 | 0.35 | 166 | 178 | 599 | 863 |
| NM_001199179 | ATP2C1 | 0.00 | 0.35 | 2410 | 2577 | 4294 | 5730 |
| NM_032312 | YIPF4 | -0.13 | 0.35 | 412 | 317 | 482 | 704 |

(continued)

| RefSeq ID | Gene Symbol | SR GFOLD | SEN GFOLD | SR IL-2 | SR IL-2 + | SEN IL-2 - | SEN IL-2 + |
|---|---|---|---|---|---|---|---|
| NM_003620 | PPM1D | 0.00 | 0.35 | 306 | 303 | 212 | 331 |
| NM_199186 | BPGM | -0.15 | 0.35 | 435 | 330 | 688 | 983 |
| NM_152221 | CSNK1E | -0.04 | 0.35 | 3499 | 3209 | 3105 | 4176 |
| NM_001172509 | SATB2 | 0.00 | 0.35 | 728 | 738 | 834 | 1179 |
| NM_016542 | MST4 | 0.00 | 0.35 | 148 | 122 | 139 | 227 |
| NM_001166111 | PNPLA6 | 0.01 | 0.35 | 2934 | 3132 | 5447 | 7225 |
| NM_194356 | STX2 | 0.00 | 0.35 | 999 | 1028 | 1402 | 1935 |
| NM_133631 | ROB01 | 0.00 | 0.35 | 1216 | 1309 | 2275 | 3083 |
| NM_001204519 | P2RX5 | 0.00 | 0.35 | 156 | 196 | 780 | 1105 |
| NM_001101426 | ISPD | -0.03 | 0.34 | 75 | 48 | 79 | 139 |
| NM_001286606 | CRACR2 B | -0.15 | 0.34 | 37 | 17 | 79 | 139 |
| NM_024980 | GPR157 | 0.00 | 0.34 | 104 | 96 | 75 | 133 |
| NM_101395 | DYRK1A | 0.00 | 0.34 | 1558 | 1572 | 1689 | 2310 |
| NM_022061 | MRPL17 | 0.00 | 0.34 | 2604 | 2784 | 3259 | 4366 |
| NM_023072 | ZSWIM4 | 0.00 | 0.34 | 336 | 317 | 424 | 623 |
| NM_002489 | NDUFA4 | 0.00 | 0.34 | 639 | 613 | 1029 | 1436 |
| NM_207123 | GAB1 | 0.00 | 0.34 | 211 | 235 | 235 | 362 |
| NM_006029 | PNMA1 | -0.02 | 0.34 | 1372 | 1238 | 1868 | 2543 |
| NM_016018 | PHF20L1 | 0.00 | 0.34 | 812 | 893 | 1393 | 1917 |
| NM_015512 | DNAH1 | 0.00 | 0.34 | 305 | 326 | 618 | 885 |
| NM_015974 | CRYL1 | 0.00 | 0.34 | 268 | 217 | 836 | 1177 |
| NM_001282913 | TCAIM | 0.00 | 0.34 | 162 | 134 | 287 | 434 |
| NM_001258 | CDK3 | 0.00 | 0.34 | 54 | 52 | 56 | 104 |
| NM_001194958 | KCNJ18 | 0.00 | 0.34 | 35 | 22 | 56 | 104 |
| NM_001290322 | DMXL1 | 0.00 | 0.34 | 818 | 821 | 1736 | 2366 |
| NM_006829 | ADIRF | -0.07 | 0.34 | 883 | 748 | 1138 | 1577 |
| NM_198507 | FAM174 A | 0.00 | 0.34 | 186 | 194 | 270 | 410 |
| NM_001178085 | ZFX | 0.00 | 0.34 | 1452 | 1410 | 2036 | 2758 |
| NM_203459 | CAMSAP 2 | 0.00 | 0.34 | 1534 | 1510 | 1999 | 2708 |
| NM_020422 | TMEM15 9 | 0.00 | 0.34 | 554 | 607 | 1192 | 1647 |
| NM_032870 | PNISR | 0.00 | 0.34 | 1197 | 1229 | 1468 | 2011 |
| NM_181836 | TMED7 | 0.00 | 0.34 | 3008 | 3107 | 4698 | 6214 |
| NM_015144 | ZCCHC14 | 0.00 | 0.34 | 839 | 876 | 1160 | 1604 |
| NM_022095 | ZNF335 | 0.00 | 0.34 | 951 | 879 | 1015 | 1412 |
| NM_004273 | CHST3 | 0.00 | 0.34 | 2867 | 2935 | 2766 | 3706 |
| NM_020840 | FNIP2 | 0.00 | 0.34 | 1420 | 1534 | 2283 | 3076 |
| **RefSeq ID** | **Gene Symbol** | **SR GFOLD** | **SEN GFOLD** | **SR IL-2 -** | **SR IL-2 +** | **SEN IL-2 -** | **SEN IL-2 +** |
| NM_006455 | LEPREL4 | 0.00 | 0.34 | 2600 | 2651 | 2211 | 2981 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001190737 | NFIB | 0.00 | 0.34 | 1079 | 1131 | 231 | 355 |
| NM_014714 | IFT140 | 0.00 | 0.34 | 227 | 252 | 705 | 998 |
| NM_001172671 | ZNF430 | 0.00 | 0.34 | 133 | 119 | 89 | 153 |
| NM_020978 | AMY2B | 0.00 | 0.34 | 10 | 26 | 102 | 172 |
| NM_001278299 | WIPI2 | 0.00 | 0.34 | 2119 | 2216 | 3926 | 5205 |
| NM_014971 | EFR3B | 0.00 | 0.34 | 130 | 145 | 443 | 645 |
| NM_001084 | PLOD3 | 0.00 | 0.34 | 4858 | 5093 | 9525 | 12403 |
| NM_197941 | ADAMTS 6 | 0.00 | 0.34 | 1589 | 1625 | 1096 | 1516 |
| NM_005107 | EXOG | 0.00 | 0.34 | 247 | 201 | 368 | 543 |
| NM_006682 | FGL2 | 0.00 | 0.33 | 265 | 230 | 177 | 279 |
| NM_005575 | LNPEP | 0.00 | 0.33 | 956 | 1024 | 1805 | 2447 |
| NM_001286527 | ATP2C2 | 0.00 | 0.33 | 92 | 84 | 83 | 144 |
| NM_178314 | RILPL1 | 0.00 | 0.33 | 657 | 728 | 873 | 1220 |
| NM_020778 | ALPK3 | 0.00 | 0.33 | 155 | 127 | 225 | 346 |
| NM_018351 | FGD6 | 0.00 | 0.33 | 419 | 411 | 749 | 1055 |
| NM_001040202 | PAQR3 | 0.00 | 0.33 | 432 | 429 | 555 | 795 |
| NM_014872 | ZBTB5 | 0.00 | 0.33 | 442 | 456 | 308 | 460 |
| NM_001075098 | MOCS1 | 0.00 | 0.33 | 162 | 136 | 154 | 246 |
| NM_001135057 | LRRC15 | 0.00 | 0.33 | 479 | 517 | 1917 | 2588 |
| NM_001412 | EIF1AX | -0.05 | 0.33 | 1150 | 1003 | 1400 | 1911 |
| NM_001135643 | DCTN4 | 0.00 | 0.33 | 1427 | 1558 | 3629 | 4802 |
| NM_001004127 | ALG11 | 0.00 | 0.33 | 709 | 661 | 715 | 1007 |
| NM_001040108 | MLH3 | 0.00 | 0.33 | 433 | 474 | 915 | 1272 |
| NM_000117 | EMD | 0.00 | 0.33 | 922 | 1002 | 1260 | 1726 |
| NM_052928 | SMYD4 | 0.00 | 0.33 | 490 | 479 | 547 | 782 |
| NM_003392 | WNT5A | 0.00 | 0.33 | 1101 | 994 | 2789 | 3712 |
| NM_001130677 | C 17orf96 | 0.00 | 0.33 | 72 | 50 | 21 | 48 |
| NM_003812 | ADAM23 | 0.00 | 0.33 | 318 | 306 | 1221 | 1673 |
| NM_001039693 | TYW5 | 0.00 | 0.33 | 90 | 124 | 127 | 207 |
| NM_031301 | APH1B | 0.00 | 0.33 | 258 | 300 | 747 | 1048 |
| NM_002611 | PDK2 | 0.00 | 0.33 | 415 | 462 | 1142 | 1569 |
| NM_194283 | DNAJC21 | -0.04 | 0.33 | 792 | 683 | 971 | 1344 |
| NM_001289121 | ZCCHC7 | 0.00 | 0.33 | 287 | 345 | 528 | 756 |
| NM_004481 | GALNT2 | 0.00 | 0.33 | 6321 | 6564 | 9071 | 11758 |
| NM_024086 | METTL16 | 0.00 | 0.33 | 489 | 491 | 582 | 828 |
| NM_031419 | NFKBIZ | 0.00 | 0.33 | 508 | 436 | 304 | 453 |
| NM_194455 | KRIT1 | 0.00 | 0.33 | 428 | 478 | 695 | 978 |
| NM_001033503 | SAR1B | 0.00 | 0.33 | 1248 | 1279 | 1626 | 2200 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_000215 | JAK3 | 0.00 | 0.33 | 157 | 194 | 166 | 262 |
| NM_001040020 | FAM3C | -0.02 | 0.33 | 1297 | 1162 | 1518 | 2059 |
| NM_015375 | DSTYK | 0.00 | 0.33 | 1005 | 920 | 1851 | 2492 |
| NM_015455 | CNOT6 | 0.00 | 0.33 | 491 | 450 | 420 | 610 |
| NM_022720 | DGCR8 | 0.00 | 0.33 | 1837 | 1861 | 1285 | 1754 |
| NM_004507 | HUS1 | 0.00 | 0.33 | 339 | 374 | 406 | 591 |
| NM_138782 | FCHO2 | 0.00 | 0.33 | 460 | 486 | 630 | 891 |
| NM_145246 | FRA10AC 1 | 0.00 | 0.33 | 177 | 196 | 189 | 294 |
| NM_007246 | KLHL2 | 0.00 | 0.33 | 428 | 427 | 514 | 736 |
| NM_018956 | C9orf9 | 0.00 | 0.33 | 65 | 56 | 241 | 366 |
| NM_012120 | CD2AP | 0.00 | 0.32 | 548 | 558 | 772 | 1079 |
| NM_007039 | PTPN21 | 0.01 | 0.32 | 1183 | 1306 | 1984 | 2662 |
| NM_020245 | TULP4 | 0.00 | 0.32 | 1812 | 1911 | 2354 | 3139 |
| NM_001570 | IRAK2 | 0.00 | 0.32 | 197 | 168 | 329 | 486 |
| NM_001134671 | DERL1 | 0.00 | 0.32 | 1908 | 2033 | 2645 | 3514 |
| NM_005257 | GATA6 | 0.00 | 0.32 | 337 | 342 | 185 | 288 |
| NM_001429 | EP300 | 0.00 | 0.32 | 3034 | 3190 | 3691 | 4858 |
| NM_001145313 | FSD1L | 0.00 | 0.32 | 79 | 105 | 175 | 274 |
| NM_001106 | ACVR2B | 0.00 | 0.32 | 73 | 90 | 79 | 137 |
| NM_198892 | BMP2K | 0.00 | 0.32 | 430 | 417 | 651 | 917 |
| NM_001145853 | WFS1 | 0.00 | 0.32 | 1983 | 1969 | 4561 | 5970 |
| NM_177533 | NUDT14 | 0.00 | 0.32 | 43 | 28 | 141 | 226 |
| NM_001171894 | MEF2A | 0.00 | 0.32 | 1198 | 1245 | 2498 | 3320 |
| NM_004563 | PCK2 | 0.00 | 0.32 | 807 | 770 | 1077 | 1477 |
| NM_001080825 | TMEM12 0B | 0.00 | 0.32 | 772 | 836 | 1090 | 1494 |
| NM_001256126 | CERS6 | 0.00 | 0.32 | 699 | 755 | 1117 | 1529 |
| NM_016612 | SLC25A3 7 | 0.00 | 0.32 | 624 | 634 | 657 | 924 |
| NM_004897 | MINPP1 | 0.00 | 0.32 | 486 | 510 | 599 | 847 |
| NM_025136 | OPA3 | 0.00 | 0.32 | 766 | 842 | 1069 | 1466 |
| NM_173569 | UBN2 | 0.00 | 0.32 | 461 | 432 | 474 | 680 |
| NM_153261 | CNEP1R1 | 0.00 | 0.32 | 200 | 217 | 272 | 407 |
| NM_015310 | PSD3 | 0.00 | 0.32 | 499 | 545 | 1926 | 2577 |
| NM_007114 | TMF1 | 0.00 | 0.32 | 1431 | 1375 | 1693 | 2275 |
| NM_144975 | SLFN5 | 0.00 | 0.32 | 1442 | 1476 | 3720 | 4880 |
| NM_003549 | HYAL3 | 0.00 | 0.32 | 157 | 143 | 162 | 255 |
| NM_018360 | TXLNG | 0.00 | 0.32 | 437 | 464 | 354 | 518 |
| NM_016579 | CD320 | -0.29 | 0.32 | 1068 | 782 | 990 | 1360 |
| NM_001859 | SLC31A1 | 0.00 | 0.32 | 2394 | 2244 | 3088 | 4068 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_003466 | PAX8 | 0.00 | 0.32 | 218 | 268 | 478 | 684 |
| NM_001204897 | TM4SF19 | 0.00 | 0.32 | 61 | 52 | 416 | 601 |
| NM_001267550 | TTN | 0.00 | 0.32 | 672 | 694 | 1553 | 2091 |
| NM_138797 | ANKRD5 4 | 0.00 | 0.32 | 443 | 501 | 539 | 765 |
| NM_030807 | SLC2A11 | 0.00 | 0.32 | 82 | 56 | 148 | 235 |
| NM_001498 | GCLC | 0.01 | 0.32 | 392 | 462 | 720 | 1004 |
| NM_152357 | ZNF440 | 0.00 | 0.32 | 150 | 172 | 247 | 372 |
| NM_001136041 | HDAC11 | 0.00 | 0.32 | 273 | 230 | 562 | 795 |
| NM_080622 | ABHD16 B | 0.00 | 0.32 | 55 | 66 | 96 | 161 |
| NM_153367 | ZCCHC24 | 0.00 | 0.32 | 1798 | 1882 | 3429 | 4497 |
| NM_002600 | PDE4B | -0.14 | 0.31 | 252 | 182 | 364 | 530 |
| NM_145294 | WDR90 | -0.03 | 0.31 | 267 | 211 | 208 | 318 |
| NM_005401 | PTPN14 | 0.00 | 0.31 | 7452 | 7306 | 12409 | 15861 |
| NM_007220 | CA5B | 0.00 | 0.31 | 261 | 239 | 293 | 434 |
| NM_001197104 | KMT2A | 0.00 | 0.31 | 2487 | 2534 | 4151 | 5412 |
| NM_007225 | NXPH3 | 0.00 | 0.31 | 121 | 148 | 362 | 527 |
| NM_031905 | ARMC10 | -0.01 | 0.31 | 270 | 217 | 362 | 527 |
| NM_003470 | USP7 | 0.00 | 0.31 | 2230 | 2284 | 2743 | 3614 |
| NM_001142853 | HES6 | -0.21 | 0.31 | 64 | 34 | 10 | 33 |
| NM_030965 | ST6GAL NAC5 | 0.00 | 0.31 | 109 | 107 | 35 | 70 |
| NM_032246 | MEX3B | 0.00 | 0.31 | 313 | 263 | 35 | 70 |
| NM_003796 | URI1 | 0.00 | 0.31 | 1158 | 1165 | 1493 | 2007 |
| NM_001145664 | RFX8 | 0.00 | 0.31 | 158 | 183 | 836 | 1153 |
| NM_012287 | ACAP2 | 0.01 | 0.31 | 1262 | 1388 | 2402 | 3174 |
| NM_001037131 | AGAP1 | 0.00 | 0.31 | 1452 | 1565 | 1915 | 2549 |
| NM_001277325 | NPIPA5 | 0.00 | 0.31 | 115 | 123 | 160 | 251 |
| NM_182488 | USP12 | 0.00 | 0.31 | 624 | 675 | 988 | 1351 |
| NM_001144000 | AGAP5 | -0.03 | 0.31 | 70 | 44 | 58 | 105 |
| NM_032340 | UQCC2 | 0.00 | 0.31 | 923 | 996 | 1410 | 1898 |
| NM_003808 | TNFSF13 | 0.00 | 0.31 | 69 | 55 | 104 | 172 |
| NM_001943 | DSG2 | 0.00 | 0.31 | 970 | 1077 | 5157 | 6678 |
| NM_025079 | ZC3H12A | 0.00 | 0.31 | 243 | 295 | 287 | 425 |
| NM_182551 | LCLAT1 | 0.00 | 0.31 | 606 | 669 | 865 | 1190 |
| NM_014899 | RHOBTB 3 | 0.00 | 0.31 | 3414 | 3569 | 6950 | 8944 |
| NM_001268284 | LRRC8E | -0.02 | 0.31 | 743 | 645 | 982 | 1342 |
| NM_020846 | SLAIN2 | 0.00 | 0.31 | 1602 | 1721 | 2005 | 2662 |
| NM_004459 | BPTF | 0.00 | 0.31 | 2298 | 2324 | 2627 | 3458 |
| NM_001291466 | LOC1027 23859 | 0.00 | 0.31 | 143 | 106 | 143 | 227 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001291459 | LOC1010 60376 | -0.01 | 0.31 | 144 | 106 | 143 | 227 |
| NM_001123391 | TBC1D3 | -0.01 | 0.31 | 144 | 106 | 143 | 227 |
| NM_001291464 | LOC1010 60351 | 0.00 | 0.31 | 143 | 106 | 143 | 227 |
| NM_001291463 | LOC1027 24862 | 0.00 | 0.31 | 143 | 106 | 143 | 227 |
| NM_001291465 | LOC1010 60389 | 0.00 | 0.31 | 143 | 106 | 143 | 227 |
| NM_018143 | KLHL11 | 0.00 | 0.31 | 288 | 313 | 393 | 567 |
| NM_001144892 | FGF1 | 0.00 | 0.31 | 287 | 237 | 524 | 741 |
| NM_004992 | MECP2 | 0.00 | 0.31 | 1723 | 1623 | 2096 | 2776 |
| NM_001001484 | PTER | 0.00 | 0.31 | 136 | 109 | 146 | 231 |
| NM_001039771 | CBLN3 | 0.00 | 0.31 | 40 | 36 | 141 | 224 |
| NM_001025930 | TTLL3 | 0.00 | 0.31 | 335 | 375 | 888 | 1218 |
| NM_018999 | CCSER2 | 0.00 | 0.31 | 1278 | 1384 | 2427 | 3198 |
| NM_152387 | KCTD18 | 0.00 | 0.31 | 357 | 357 | 557 | 784 |
| NM_012234 | RYBP | 0.00 | 0.31 | 565 | 612 | 688 | 956 |
| NM_001010935 | RAP1A | 0.00 | 0.31 | 609 | 654 | 780 | 1076 |
| NM_016174 | CERCAM | 0.00 | 0.31 | 6943 | 7119 | 12879 | 16369 |
| NM_005261 | GEM | 0.00 | 0.31 | 416 | 415 | 869 | 1192 |
| NM_014178 | STXBP6 | 0.00 | 0.31 | 229 | 246 | 67 | 118 |
| NM_032836 | FIZ1 | 0.00 | 0.31 | 124 | 153 | 67 | 118 |
| NM_001042522 | SPRED3 | 0.00 | 0.31 | 85 | 86 | 364 | 527 |
| NM_001207025 | POU2F2 | 0.00 | 0.31 | 124 | 121 | 160 | 250 |
| NM_173517 | VKORC1 L1 | 0.00 | 0.31 | 1597 | 1556 | 1874 | 2486 |
| NM_014709 | USP34 | 0.00 | 0.30 | 2922 | 3017 | 4960 | 6403 |
| NM_018691 | FAM114 A2 | 0.00 | 0.30 | 365 | 381 | 699 | 969 |
| NM_020807 | ZNF319 | 0.00 | 0.30 | 431 | 482 | 499 | 706 |
| NM_033393 | FHDC1 | -0.03 | 0.30 | 44 | 24 | 19 | 44 |
| NM_016072 | GOLT1B | 0.00 | 0.30 | 1346 | 1436 | 1414 | 1895 |
| NM_001270974 | HYDIN | 0.00 | 0.30 | 13 | 18 | 208 | 316 |
| NM_001018100 | MYZAP | 0.00 | 0.30 | 100 | 131 | 208 | 316 |
| NM_178526 | SLC25A4 2 | 0.00 | 0.30 | 144 | 153 | 272 | 403 |
| NM_145235 | FANK1 | 0.00 | 0.30 | 64 | 52 | 85 | 144 |
| NM_138496 | CYHR1 | 0.00 | 0.30 | 422 | 382 | 790 | 1087 |
| NM_001079533 | CPEB1 | -1.04 | 0.30 | 107 | 33 | 514 | 725 |
| RefSeq ID | Gene Symbol | SR GFOLD | SEN GFOLD | SR IL-2 - | SR IL-2 + | SEN IL-2 - | SEN IL-2+ |
| NM_014881 | DCLRE1 A | 0.00 | 0.30 | 106 | 136 | 143 | 226 |
| NM_001173452 | TFCP2 | 0.00 | 0.30 | 670 | 587 | 684 | 948 |
| NM_006885 | ZFHX3 | -0.11 | 0.30 | 2389 | 2061 | 2092 | 2760 |
| NM_004177 | STX3 | 0.00 | 0.30 | 678 | 760 | 626 | 872 |

(continued)

| RefSeq ID | Gene Symbol | SR GFOLD | SEN GFOLD | SR IL-2 - | SR IL-2 + | SEN IL-2 - | SEN IL-2+ |
|---|---|---|---|---|---|---|---|
| NM_000512 | GALNS | 0.00 | 0.30 | 661 | 666 | 890 | 1216 |
| NM_001288668 | HCFC1R1 | 0.00 | 0.30 | 549 | 495 | 784 | 1078 |
| NM_018362 | LIN7C | 0.00 | 0.30 | 1294 | 1273 | 1572 | 2094 |
| NM_015077 | SARM1 | 0.00 | 0.30 | 387 | 380 | 776 | 1067 |
| NM_004804 | CIAO1 | 0.00 | 0.30 | 1425 | 1460 | 2483 | 3255 |
| NM_004620 | TRAF6 | 0.00 | 0.30 | 556 | 543 | 562 | 787 |
| NM_021167 | GATAD1 | -0.26 | 0.30 | 694 | 505 | 705 | 974 |
| NM_001144823 | DENND4 A | 0.00 | 0.30 | 678 | 654 | 1029 | 1394 |
| NM_032452 | JPH4 | 0.00 | 0.30 | 43 | 35 | 79 | 135 |
| NM_001286581 | PHRF1 | 0.00 | 0.30 | 1387 | 1447 | 1227 | 1649 |
| NM_001025108 | AFF3 | 0.00 | 0.30 | 88 | 111 | 250 | 372 |
| NM_173791 | PDZD8 | 0.00 | 0.30 | 1055 | 1153 | 1747 | 2314 |
| NM_020911 | PLXNA4 | 0.00 | 0.30 | 36 | 35 | 262 | 388 |
| NM_006224 | PITPNA | -0.10 | 0.30 | 1858 | 1604 | 2724 | 3554 |
| NM_139280 | ORMDL3 | 0.00 | 0.30 | 1525 | 1594 | 2433 | 3185 |
| NM_020399 | GOPC | 0.00 | 0.30 | 994 | 946 | 1404 | 1874 |
| NM_145169 | SFT2D1 | 0.00 | 0.30 | 443 | 443 | 599 | 834 |
| NM_001137552 | LRRFIP1 | -0.39 | 0.30 | 1023 | 697 | 1206 | 1619 |
| NM_001199161 | USP19 | 0.00 | 0.30 | 1321 | 1384 | 1657 | 2196 |
| NM_199336 | FAHD2B | -0.15 | 0.30 | 143 | 95 | 135 | 214 |
| NM_207644 | LRRC75B | 0.00 | 0.30 | 57 | 64 | 125 | 200 |
| NM_003718 | CDK13 | 0.00 | 0.30 | 1200 | 1192 | 1526 | 2028 |
| NM_001127615 | ENOX1 | 0.00 | 0.30 | 19 | 18 | 96 | 159 |
| NM_198449 | EMB | 0.00 | 0.30 | 265 | 226 | 826 | 1128 |
| NM_001001890 | RUNX1 | 0.00 | 0.30 | 1982 | 1889 | 3872 | 4996 |
| NM_001012398 | AKTIP | 0.00 | 0.30 | 172 | 197 | 329 | 477 |
| NM_138389 | FAM114 A1 | 0.00 | 0.30 | 2607 | 2719 | 4099 | 5281 |
| NM_003477 | PDHX | 0.00 | 0.30 | 806 | 853 | 892 | 1213 |
| NM_182767 | SLC6A15 | 0.00 | 0.30 | 65 | 94 | 146 | 229 |
| NM_001256667 | FAM193 A | 0.00 | 0.30 | 967 | 1014 | 1123 | 1510 |
| NM_032832 | LRP11 | 0.00 | 0.29 | 1024 | 1018 | 1934 | 2545 |
| NM_001287349 | ZNF852 | 0.00 | 0.29 | 86 | 74 | 50 | 92 |
| NM_003070 | SMARCA | 0.00 | 0.29 | 1601 | 1688 | 2288 | 2993 |
| **RefSeq ID** | **Gene Symbol** | **SR GFOLD** | **SEN GFOLD** | **SR IL-2 -** | **SR IL-2 +** | **SEN IL-2 -** | **SEN IL-2 +** |
| | 2 | | | | | | |
| NM_024790 | CSPP1 | 0.00 | 0.29 | 166 | 164 | 189 | 288 |
| NM_001271938 | MEGF8 | 0.00 | 0.29 | 635 | 717 | 1618 | 2142 |
| NM_001288645 | ZNF286A | 0.00 | 0.29 | 112 | 141 | 195 | 296 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001164315 | ANKRD3 6 | 0.00 | 0.29 | 383 | 357 | 510 | 715 |
| NM_001277090 | ZNF550 | -0.04 | 0.29 | 160 | 117 | 160 | 248 |
| NM_003367 | USF2 | 0.00 | 0.29 | 1860 | 1778 | 2296 | 3000 |
| NM_024753 | TTC21B | 0.00 | 0.29 | 450 | 454 | 543 | 758 |
| NM_001039590 | USP9X | 0.00 | 0.29 | 4589 | 4606 | 9009 | 11399 |
| NM_018714 | COG1 | 0.00 | 0.29 | 1846 | 1974 | 2810 | 3647 |
| NM_001001795 | C8orf82 | 0.00 | 0.29 | 181 | 169 | 327 | 473 |
| NM_198076 | COX20 | -0.42 | 0.29 | 483 | 305 | 474 | 667 |
| NM_000124 | ERCC6 | 0.00 | 0.29 | 1586 | 1640 | 2044 | 2678 |
| NM_001282569 | ZCCHC17 | 0.00 | 0.29 | 864 | 905 | 1206 | 1612 |
| NM_025040 | ZNF614 | 0.00 | 0.29 | 204 | 223 | 191 | 290 |
| NM_001001418 | TBC1D3C | -0.04 | 0.29 | 146 | 106 | 148 | 231 |
| NM_001278277 | E2F6 | 0.00 | 0.29 | 174 | 217 | 175 | 268 |
| NM_003170 | SUPT6H | -0.02 | 0.29 | 2072 | 1894 | 4132 | 5298 |
| NM_053055 | THEM4 | 0.00 | 0.29 | 88 | 119 | 220 | 329 |
| NM_138367 | ZNF251 | 0.00 | 0.29 | 252 | 233 | 543 | 756 |
| NM_032270 | LRRC8C | 0.00 | 0.29 | 1041 | 983 | 491 | 688 |
| NM_017933 | PID1 | 0.00 | 0.29 | 270 | 294 | 264 | 388 |
| NM_001271675 | LOC4411 55 | 0.00 | 0.29 | 716 | 796 | 836 | 1135 |
| NM_024529 | CDC73 | 0.00 | 0.29 | 985 | 989 | 1383 | 1834 |
| NM_001024210 | S100A13 | 0.00 | 0.29 | 2237 | 2366 | 5430 | 6913 |
| NM_025054 | VCPIP1 | 0.00 | 0.29 | 946 | 939 | 1495 | 1976 |
| NM_152305 | POGLUT 1 | 0.00 | 0.29 | 320 | 274 | 582 | 806 |
| NM_138571 | HINT3 | -0.15 | 0.29 | 171 | 117 | 356 | 510 |
| NM_002298 | LCP1 | 0.00 | 0.29 | 15 | 22 | 277 | 405 |
| NM_014713 | LAPTM4 A | 0.00 | 0.29 | 6061 | 5946 | 9618 | 12105 |
| NM_004996 | ABCC1 | 0.01 | 0.29 | 3233 | 3438 | 4744 | 6048 |
| NM_002392 | MDM2 | 0.00 | 0.28 | 1487 | 1553 | 2849 | 3678 |
| NM_001001481 | UBE2W | 0.00 | 0.28 | 448 | 489 | 786 | 1068 |
| NM_152272 | CHMP7 | 0.00 | 0.28 | 976 | 1007 | 1578 | 2076 |
| NM_014931 | PPP6R1 | 0.00 | 0.28 | 3188 | 3176 | 3619 | 4638 |
| NM_012192 | TIMM10 B | 0.00 | 0.28 | 616 | 682 | 1262 | 1675 |
| NM_017864 | INTS8 | 0.00 | 0.28 | 384 | 426 | 661 | 906 |
| NM_001111125 | IQSEC2 | 0.00 | 0.28 | 475 | 433 | 638 | 876 |
| NM_006854 | KDELR2 | 0.00 | 0.28 | 10362 | 10647 | 8911 | 11201 |
| NM_144666 | DNHD1 | 0.00 | 0.28 | 237 | 223 | 445 | 625 |
| NM_005054 | RGPD5 | 0.00 | 0.28 | 1425 | 1330 | 2003 | 2608 |
| NM_016374 | ARID4B | -0.04 | 0.28 | 367 | 297 | 401 | 567 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_015234 | GPR116 | 0.00 | 0.28 | 427 | 462 | 817 | 1105 |
| NM_024830 | LPCAT1 | -0.20 | 0.28 | 2873 | 2349 | 2354 | 3046 |
| NM_020812 | DOCK6 | 0.00 | 0.28 | 413 | 469 | 1192 | 1582 |
| NM_002485 | NBN | 0.00 | 0.28 | 576 | 648 | 950 | 1274 |
| NM_001145855 | SH3BP2 | 0.00 | 0.28 | 717 | 728 | 990 | 1325 |
| NM_004999 | MY06 | 0.00 | 0.28 | 1095 | 1167 | 1635 | 2142 |
| NM_001170637 | SRGAP2 | 0.00 | 0.28 | 1757 | 1869 | 2770 | 3566 |
| NM_006031 | PCNT | 0.00 | 0.28 | 2036 | 2017 | 2396 | 3098 |
| NM_024527 | ABHD8 | 0.00 | 0.28 | 195 | 232 | 256 | 375 |
| NM_004330 | BNIP2 | 0.00 | 0.28 | 931 | 973 | 1508 | 1981 |
| NM_017423 | GALNT7 | 0.01 | 0.28 | 379 | 448 | 774 | 1048 |
| NM_001116 | ADCY9 | 0.00 | 0.28 | 2359 | 2217 | 3519 | 4497 |
| NM_001130720 | AHCYL2 | 0.00 | 0.28 | 346 | 290 | 586 | 806 |
| NM_001042463 | TMEM80 | 0.00 | 0.28 | 187 | 224 | 495 | 688 |
| NM_030964 | SPRY4 | 0.00 | 0.28 | 1431 | 1518 | 3061 | 3924 |
| NM_134269 | SMTN | 0.00 | 0.28 | 6625 | 6496 | 7645 | 9602 |
| NM_007372 | DDX42 | 0.00 | 0.28 | 2000 | 2130 | 3537 | 4516 |
| NM_001288805 | ZNF707 | 0.00 | 0.28 | 177 | 200 | 237 | 349 |
| NM_006675 | TSPAN9 | 0.00 | 0.28 | 442 | 441 | 690 | 939 |
| NM_001195192 | EFCAB13 | 0.00 | 0.28 | 53 | 79 | 162 | 248 |
| NM_173647 | RNF149 | 0.00 | 0.28 | 560 | 639 | 1142 | 1514 |
| NM_001256182 | ANKRD1 1 | -0.09 | 0.28 | 2578 | 2261 | 3677 | 4684 |
| NM_152556 | C7orf60 | 0.00 | 0.27 | 284 | 292 | 351 | 499 |
| NM_138714 | NFAT5 | -0.08 | 0.27 | 3955 | 3550 | 3901 | 4957 |
| NM_020244 | CHPT1 | -0.12 | 0.27 | 702 | 568 | 1175 | 1553 |
| NM_019034 | RHOF | 0.00 | 0.27 | 174 | 217 | 308 | 442 |
| NM_001291163 | EPHX1 | 0.00 | 0.27 | 865 | 967 | 2269 | 2924 |
| NM_152517 | TTC30B | 0.00 | 0.27 | 62 | 44 | 73 | 124 |
| NM_019087 | ARL15 | 0.00 | 0.27 | 205 | 190 | 187 | 281 |
| NM_016282 | AK3 | 0.00 | 0.27 | 610 | 674 | 1982 | 2564 |
| NM_013284 | POLM | 0.00 | 0.27 | 451 | 426 | 397 | 558 |
| NM_001122838 | NAPEPL D | 0.00 | 0.27 | 115 | 92 | 241 | 353 |
| NM_020199 | C5orf15 | 0.00 | 0.27 | 800 | 866 | 1593 | 2076 |
| NM_144982 | ZFC3H1 | 0.00 | 0.27 | 1959 | 1870 | 1788 | 2320 |
| NM_001166263 | MTIF3 | -0.01 | 0.27 | 544 | 467 | 763 | 1028 |
| NM_001288654 | DCAKD | 0.00 | 0.27 | 401 | 448 | 959 | 1277 |
| NM_015525 | IBTK | 0.00 | 0.27 | 1549 | 1479 | 1996 | 2579 |
| NM_015384 | NIPBL | 0.00 | 0.27 | 1446 | 1544 | 1824 | 2364 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_183075 | CYP2U1 | 0.01 | 0.27 | 350 | 417 | 830 | 1113 |
| NM_173537 | GTF2IRD 2 | 0.00 | 0.27 | 223 | 249 | 416 | 582 |
| NM_001787 | CDK11B | -0.04 | 0.27 | 267 | 209 | 258 | 375 |
| NM_014616 | ATP11B | 0.00 | 0.27 | 1790 | 1884 | 2213 | 2847 |
| NM_005619 | RTN2 | 0.00 | 0.27 | 136 | 120 | 341 | 484 |
| NM_021645 | UTP14C | 0.00 | 0.27 | 1229 | 1156 | 1589 | 2068 |
| NM_012193 | FZD4 | 0.00 | 0.27 | 3636 | 3831 | 2048 | 2641 |
| NM_152457 | ZNF597 | 0.00 | 0.27 | 59 | 62 | 81 | 135 |
| NM_001283043 | RALGAP A1 | 0.00 | 0.27 | 157 | 200 | 291 | 418 |
| NM_001252335 | CGNL1 | 0.00 | 0.27 | 11 | 22 | 67 | 115 |
| NM_017750 | RETSAT | 0.00 | 0.27 | 1488 | 1552 | 3169 | 4028 |
| NM_014654 | SDC3 | 0.00 | 0.27 | 1726 | 1775 | 4989 | 6270 |
| NM_005749 | TOB1 | 0.00 | 0.27 | 866 | 914 | 1025 | 1357 |
| NM_001281294 | ZNF281 | 0.00 | 0.27 | 1184 | 1266 | 2828 | 3603 |
| NM_001145045 | ZNF286B | 0.00 | 0.26 | 52 | 76 | 94 | 153 |
| NM_024518 | ULBP3 | 0.00 | 0.26 | 66 | 47 | 89 | 146 |
| NM_004711 | SYNGR1 | 0.00 | 0.26 | 92 | 109 | 185 | 277 |
| NM_020233 | ADPRM | -0.01 | 0.26 | 139 | 102 | 185 | 277 |
| NM_033083 | EAF1 | 0.00 | 0.26 | 811 | 809 | 1004 | 1329 |
| NM_003917 | AP1G2 | 0.00 | 0.26 | 316 | 268 | 372 | 523 |
| NM_001143819 | TPCN1 | 0.00 | 0.26 | 799 | 855 | 2127 | 2732 |
| NM_015205 | ATP11A | 0.00 | 0.26 | 1178 | 1273 | 1791 | 2314 |
| NM_002093 | GSK3B | -0.01 | 0.26 | 1943 | 1793 | 1982 | 2551 |
| NM_001123363 | RGPD6 | 0.00 | 0.26 | 963 | 938 | 1395 | 1819 |
| NM_144775 | SMCR8 | 0.00 | 0.26 | 1424 | 1447 | 2067 | 2656 |
| NM_024011 | CDK11A | -0.02 | 0.26 | 210 | 163 | 177 | 266 |
| NM_001289986 | TIPIN | 0.00 | 0.26 | 124 | 123 | 92 | 150 |
| NM_006977 | ZBTB25 | -0.02 | 0.26 | 110 | 77 | 154 | 235 |
| NM_152281 | GORAB | -0.16 | 0.26 | 208 | 145 | 183 | 274 |
| NM_000127 | EXT1 | 0.00 | 0.26 | 9045 | 8841 | 10344 | 12798 |
| NM_001178138 | TFDP2 | 0.00 | 0.26 | 413 | 456 | 1042 | 1375 |
| NM_032871 | RELT | 0.00 | 0.26 | 629 | 549 | 331 | 469 |
| RefSeq ID | Gene Symbol | SR GFOLD | SEN GFOLD | SR IL-2 - | SR IL-2 + | SEN IL-2- | SEN IL-2+ |
| NM_001251 | CD68 | -0.02 | 0.26 | 3830 | 3590 | 9144 | 11331 |
| NM_000836 | GRIN2D | 0.00 | 0.26 | 299 | 256 | 351 | 495 |
| NM_138783 | ZNF653 | 0.00 | 0.26 | 123 | 132 | 100 | 161 |
| NM_025077 | TOE1 | 0.00 | 0.26 | 338 | 369 | 299 | 427 |
| NM_001347 | DGKQ | 0.00 | 0.26 | 495 | 489 | 730 | 980 |

(continued)

| RefSeq ID | Gene Symbol | SR GFOLD | SEN GFOLD | SR IL-2 - | SR IL-2 + | SEN IL-2- | SEN IL-2+ |
|---|---|---|---|---|---|---|---|
| NM_198458 | ZNF497 | -0.10 | 0.26 | 202 | 146 | 393 | 549 |
| NM_002737 | PRKCA | 0.00 | 0.26 | 3139 | 3258 | 4215 | 5298 |
| NM_020435 | GJC2 | 0.00 | 0.26 | 39 | 56 | 21 | 46 |
| NM_015157 | PHLDB1 | 0.00 | 0.26 | 3020 | 3044 | 5719 | 7140 |
| NM_001289402 | ZNF135 | 0.00 | 0.26 | 82 | 115 | 333 | 471 |
| NM_022129 | PBLD | 0.00 | 0.26 | 144 | 160 | 291 | 416 |
| NM_001242546 | ANAPC 1 6 | 0.00 | 0.26 | 454 | 493 | 809 | 1079 |
| NM_004577 | PSPH | 0.00 | 0.26 | 252 | 291 | 412 | 573 |
| NM_015884 | MBTPS2 | 0.00 | 0.26 | 657 | 602 | 1077 | 1416 |
| NM_153042 | KDM1B | 0.00 | 0.26 | 290 | 335 | 616 | 834 |
| NM_020315 | PDXP | 0.00 | 0.26 | 650 | 735 | 616 | 834 |
| NM_014909 | VASH1 | 0.00 | 0.26 | 225 | 183 | 250 | 362 |
| NM_018602 | DNAJA4 | 0.00 | 0.26 | 333 | 332 | 389 | 543 |
| NM_001042573 | ENGASE | 0.00 | 0.26 | 227 | 215 | 414 | 575 |
| NM_001080 | ALDH5A 1 | 0.00 | 0.26 | 28 | 24 | 189 | 281 |
| NM_001271803 | REEP2 | 0.00 | 0.26 | 92 | 109 | 408 | 567 |
| NM_032453 | ZNF527 | 0.00 | 0.26 | 67 | 58 | 96 | 155 |
| NM_014454 | SESN1 | 0.00 | 0.26 | 155 | 187 | 360 | 505 |
| NM_003312 | TST | 0.00 | 0.26 | 530 | 543 | 672 | 904 |
| NM_002198 | IRF1 | 0.00 | 0.26 | 290 | 307 | 547 | 745 |
| NM_203487 | PCDH9 | 0.00 | 0.26 | 219 | 184 | 447 | 617 |
| NM_152783 | D2HGDH | 0.00 | 0.26 | 395 | 360 | 347 | 488 |
| NM_001291813 | SLC25A2 9 | 0.00 | 0.26 | 119 | 139 | 258 | 372 |
| NM_022494 | ZDHHC6 | 0.00 | 0.26 | 599 | 549 | 986 | 1299 |
| NM_147161 | ACOT11 | 0.00 | 0.26 | 191 | 185 | 166 | 250 |
| NM_024759 | NIPAL2 | 0.00 | 0.26 | 184 | 164 | 638 | 860 |
| NM_001278438 | FNDC3A | 0.00 | 0.26 | 1565 | 1636 | 2479 | 3150 |
| NM_015055 | SWAP70 | 0.00 | 0.26 | 1838 | 1890 | 1907 | 2444 |
| NM_021228 | SCAF1 | 0.00 | 0.26 | 913 | 1004 | 1237 | 1612 |
| NM_145119 | PJA1 | 0.00 | 0.25 | 544 | 520 | 647 | 871 |
| NM_003566 | EEA1 | -0.11 | 0.25 | 1335 | 1129 | 2159 | 2754 |
| NM_002221 | ITPKB | 0.00 | 0.25 | 56 | 82 | 339 | 477 |
| NM_001031701 | NT5DC3 | 0.00 | 0.25 | 647 | 719 | 948 | 1250 |
| RefSeq ID | Gene Symbol | SR GFOLD | SEN GFOLD | SR IL-2 | SR IL-2 + | SEN IL-2 - | SEN IL-2 + |
| NM_001286631 | RBM26 | 0.00 | 0.25 | 604 | 609 | 720 | 963 |
| NM_005999 | TSNAX | 0.00 | 0.25 | 454 | 407 | 898 | 1187 |
| NM_022456 | RAB3IP | -0.47 | 0.25 | 152 | 80 | 79 | 131 |
| NM_005860 | FSTL3 | -0.05 | 0.25 | 3553 | 3252 | 1549 | 1998 |

(continued)

| RefSeq ID | Gene Symbol | SR GFOLD | SEN GFOLD | SR IL-2 | SR IL-2 + | SEN IL-2 - | SEN IL-2 + |
|---|---|---|---|---|---|---|---|
| NM_001277710 | CD99 | 0.00 | 0.25 | 6948 | 7069 | 13830 | 16928 |
| NM_001307 | CLDN7 | 0.00 | 0.25 | 37 | 52 | 141 | 216 |
| NM_020240 | CDC42SE 2 | 0.00 | 0.25 | 633 | 635 | 636 | 856 |
| NM_003324 | TULP3 | 0.00 | 0.25 | 988 | 979 | 1115 | 1457 |
| NM_020135 | WRNIP1 | 0.00 | 0.25 | 1043 | 1092 | 1233 | 1604 |
| NM_033549 | TRIM41 | 0.00 | 0.25 | 498 | 555 | 796 | 1057 |
| NM_016628 | WAC | -0.07 | 0.25 | 3135 | 2804 | 2914 | 3675 |
| NM_133264 | WIPF2 | 0.00 | 0.25 | 1751 | 1735 | 1655 | 2126 |
| NM_004442 | EPHB2 | 0.00 | 0.25 | 28 | 44 | 360 | 503 |
| NM_031407 | HUWE1 | 0.00 | 0.25 | 11361 | 11092 | 18197 | 22166 |
| NM_014964 | EPN2 | 0.00 | 0.25 | 1334 | 1350 | 1543 | 1987 |
| NM_001184781 | DGCR2 | -0.08 | 0.25 | 2247 | 1979 | 2161 | 2749 |
| NM_015124 | GRAMD4 | 0.00 | 0.25 | 205 | 245 | 522 | 710 |
| NM_001007189 | IGIP | -0.02 | 0.25 | 314 | 255 | 589 | 795 |
| NM_001240 | CCNT1 | 0.00 | 0.25 | 1083 | 1192 | 1075 | 1405 |
| NM_018952 | HOXB6 | 0.00 | 0.25 | 369 | 338 | 372 | 518 |
| NM_001458 | FLNC | -0.07 | 0.25 | 135215 | 127692 | 109037 | 130883 |
| NM_001128212 | WDSUB1 | 0.00 | 0.25 | 67 | 83 | 85 | 139 |
| NM_001143674 | MPC2 | 0.00 | 0.25 | 1203 | 1260 | 1668 | 2139 |
| NM_001099220 | ZNF862 | 0.00 | 0.25 | 238 | 279 | 543 | 736 |
| NM_003790 | TNFRSF2 5 | 0.00 | 0.25 | 61 | 68 | 56 | 98 |
| NM_001013693 | LDLRAD 2 | -0.17 | 0.25 | 2048 | 1684 | 2498 | 3157 |
| NM_005534 | IFNGR2 | 0.00 | 0.25 | 1396 | 1509 | 2317 | 2935 |
| NM_001782 | CD72 | -0.06 | 0.25 | 61 | 36 | 73 | 122 |
| NM_000404 | GLB1 | 0.00 | 0.25 | 2424 | 2563 | 6484 | 7998 |
| NM_001046 | SLC12A2 | 0.00 | 0.25 | 969 | 920 | 851 | 1122 |
| NM_032368 | LZIC | 0.00 | 0.25 | 326 | 388 | 586 | 789 |
| NM_147780 | CTSB | 0.00 | 0.25 | 28365 | 28109 | 115877 | 138711 |
| NM_003216 | TEF | 0.00 | 0.25 | 427 | 399 | 973 | 1274 |
| NM_001114132 | NBEAL1 | 0.00 | 0.25 | 1017 | 938 | 2011 | 2556 |
| NM_032806 | POMGNT 2 | 0.00 | 0.25 | 356 | 350 | 925 | 1214 |
| NM_001025579 | NDEL1 | 0.00 | 0.25 | 2371 | 2466 | 2150 | 2726 |
| NM_024015 | HOXB4 | 0.00 | 0.25 | 332 | 340 | 595 | 800 |
| **RefSeq ID** | **Gene Symbol** | **SR GFOLD** | **SEN GFOLD** | **SR IL-2 -** | **SR IL-2 +** | **SEN IL-2 -** | **SEN IL-2 +** |
| NM_000437 | PAFAH2 | 0.00 | 0.25 | 144 | 153 | 406 | 560 |
| NM_004419 | DUSP5 | 0.00 | 0.25 | 1102 | 1158 | 1443 | 1856 |
| NM_001242784 | HLCS | 0.00 | 0.25 | 719 | 733 | 1516 | 1946 |
| NM_194328 | RNF38 | 0.00 | 0.25 | 669 | 694 | 747 | 991 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001242885 | LOC1002 87036 | 0.00 | 0.24 | 87 | 89 | 81 | 133 |
| NM_016094 | COMMD 2 | 0.00 | 0.24 | 432 | 494 | 578 | 778 |
| NM_001290256 | KHNYN | 0.00 | 0.24 | 2109 | 2113 | 2421 | 3055 |
| NM_016447 | MPP6 | 0.00 | 0.24 | 391 | 441 | 470 | 641 |
| NM_001083893 | STRN3 | 0.00 | 0.24 | 881 | 872 | 1038 | 1353 |
| NM_001256695 | PRDM11 | 0.00 | 0.24 | 49 | 31 | 35 | 67 |
| NM_001369 | DNAH5 | 0.00 | 0.24 | 34 | 45 | 119 | 185 |
| NM_001085384 | ZNF154 | 0.00 | 0.24 | 85 | 87 | 148 | 224 |
| NM_152542 | PPM1K | 0.00 | 0.24 | 294 | 297 | 366 | 508 |
| NM_176782 | FAM151 A | 0.00 | 0.24 | 50 | 55 | 50 | 89 |
| NM_007124 | UTRN | -0.03 | 0.24 | 3589 | 3322 | 7699 | 9442 |
| NM_003338 | UBE2D1 | 0.00 | 0.24 | 163 | 208 | 374 | 518 |
| NM_024595 | AKIRIN1 | 0.00 | 0.24 | 1333 | 1434 | 1533 | 1963 |
| NM_024665 | TBL1XR1 | 0.00 | 0.24 | 989 | 1077 | 1899 | 2412 |
| NM_001166692 | C11orf91 | 0.00 | 0.24 | 182 | 140 | 131 | 201 |
| NM_001261459 | ZNF484 | 0.00 | 0.24 | 137 | 149 | 214 | 311 |
| NM_002841 | PTPRG | 0.00 | 0.24 | 1560 | 1584 | 2350 | 2963 |
| NM_001037633 | SIL1 | 0.00 | 0.24 | 1142 | 1138 | 2756 | 3458 |
| NM_024947 | PHC3 | 0.00 | 0.24 | 1500 | 1600 | 2213 | 2795 |
| NM_058181 | YBEY | 0.00 | 0.24 | 70 | 75 | 158 | 237 |
| NM_030821 | PLA2G12 A | 0.00 | 0.24 | 189 | 224 | 597 | 800 |
| NM_016614 | TDP2 | 0.00 | 0.24 | 542 | 596 | 1000 | 1303 |
| NM_001127605 | LIPA | 0.00 | 0.24 | 392 | 447 | 923 | 1207 |
| NM_138430 | ADPRHL 1 | 0.00 | 0.24 | 22 | 30 | 123 | 190 |
| NM_032430 | BRSK1 | 0.00 | 0.24 | 98 | 122 | 123 | 190 |
| NM_205842 | NCKAP1 | 0.00 | 0.24 | 5174 | 5258 | 9119 | 11131 |
| NM_203437 | AFTPH | 0.00 | 0.24 | 565 | 596 | 713 | 945 |
| NM_014818 | TRIM66 | 0.00 | 0.24 | 147 | 150 | 245 | 351 |
| NM_005649 | ZNF354A | 0.00 | 0.24 | 165 | 150 | 193 | 283 |
| NM_014752 | SPCS2 | 0.00 | 0.24 | 291 | 286 | 314 | 440 |
| NM_015430 | PAMR1 | -0.05 | 0.24 | 1386 | 1224 | 5906 | 7260 |
| NM_000081 | LYST | 0.00 | 0.24 | 518 | 544 | 1537 | 1963 |
| NM_001037501 | NBPF8 | -0.17 | 0.24 | 982 | 778 | 996 | 1296 |
| NM_017614 | BHMT2 | 0.00 | 0.24 | 475 | 487 | 2048 | 2588 |
| NM_031477 | YPEL3 | 0.00 | 0.24 | 207 | 225 | 510 | 689 |
| NM_024835 | GGNBP2 | -0.04 | 0.24 | 1386 | 1232 | 1714 | 2179 |
| NM_004850 | ROCK2 | 0.00 | 0.24 | 2014 | 2149 | 2171 | 2737 |
| NM_012090 | MACF1 | 0.00 | 0.24 | 11306 | 10967 | 23899 | 28761 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_015630 | EPC2 | 0.00 | 0.24 | 288 | 265 | 410 | 562 |
| NM_024956 | TMEM62 | 0.00 | 0.24 | 244 | 247 | 489 | 662 |
| NM_024641 | MANEA | 0.00 | 0.24 | 357 | 310 | 543 | 730 |
| NM_015275 | KIAA103 3 | 0.00 | 0.24 | 1294 | 1406 | 2263 | 2847 |
| NM_145167 | PIGM | 0.00 | 0.24 | 215 | 239 | 310 | 434 |
| NM_173475 | DCUN1D 3 | 0.00 | 0.24 | 638 | 667 | 1077 | 1394 |
| NM_000663 | ABAT | 0.00 | 0.24 | 94 | 76 | 83 | 135 |
| NM_001170760 | SRPK3 | -0.04 | 0.24 | 72 | 45 | 83 | 135 |
| NM_007167 | ZMYM6 | 0.00 | 0.24 | 380 | 347 | 545 | 732 |
| NM_017836 | SLC41A3 | 0.00 | 0.24 | 862 | 960 | 2400 | 3011 |
| NM_001134831 | AHI1 | 0.00 | 0.24 | 775 | 794 | 828 | 1085 |
| NM_004776 | B4GALT5 | 0.00 | 0.24 | 1871 | 1918 | 1755 | 2225 |
| NM_013446 | MKRN1 | 0.00 | 0.24 | 880 | 897 | 1271 | 1632 |
| NM_001470 | GABBR1 | 0.00 | 0.23 | 276 | 309 | 466 | 632 |
| NM_021259 | TMEM8A | 0.00 | 0.23 | 2352 | 2395 | 4663 | 5745 |
| NM_144694 | ZNF570 | 0.00 | 0.23 | 108 | 77 | 125 | 192 |
| NM_181809 | BMP8A | -1.00 | 0.23 | 79 | 23 | 40 | 74 |
| NM_006264 | PTPN13 | 0.00 | 0.23 | 706 | 772 | 1445 | 1845 |
| NM_198147 | ABHD15 | 0.00 | 0.23 | 287 | 310 | 528 | 710 |
| NM_183050 | BCKDHB | 0.00 | 0.23 | 152 | 177 | 256 | 364 |
| NM_004973 | JARID2 | 0.00 | 0.23 | 828 | 890 | 1441 | 1839 |
| NM_152564 | VPS13B | 0.00 | 0.23 | 1062 | 1098 | 2356 | 2954 |
| NM_014577 | BRD1 | 0.00 | 0.23 | 816 | 838 | 796 | 1044 |
| NM_001267698 | CD63 | -0.15 | 0.23 | 12326 | 10792 | 27628 | 33094 |
| NM_153029 | N4BP1 | 0.00 | 0.23 | 696 | 754 | 1094 | 1412 |
| NM_015027 | PDXDC1 | 0.00 | 0.23 | 3261 | 3457 | 3629 | 4492 |
| NM_002744 | PRKCZ | 0.00 | 0.23 | 137 | 134 | 181 | 266 |
| NM_033637 | BTRC | 0.00 | 0.23 | 490 | 550 | 844 | 1103 |
| NM_024642 | GALNT1 2 | 0.00 | 0.23 | 275 | 236 | 840 | 1098 |
| NM_001172713 | GOLGA4 | -0.42 | 0.23 | 4504 | 3197 | 3872 | 4784 |
| NM_014159 | SETD2 | 0.00 | 0.23 | 1467 | 1578 | 1853 | 2340 |
| NM_016463 | CXXC5 | 0.00 | 0.23 | 720 | 798 | 1709 | 2164 |
| NM_001286767 | USP46 | -0.12 | 0.23 | 394 | 304 | 541 | 725 |
| NM_002972 | SBF1 | 0.00 | 0.23 | 3413 | 3449 | 5384 | 6597 |
| NM_005385 | NKTR | 0.00 | 0.23 | 1017 | 1029 | 1219 | 1564 |
| NM_003292 | TPR | 0.00 | 0.23 | 2607 | 2702 | 3465 | 4288 |
| NM_138465 | GLI4 | 0.00 | 0.23 | 112 | 125 | 160 | 238 |
| NM_022041 | GAN | 0.00 | 0.23 | 416 | 392 | 447 | 606 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_145059 | FUK | 0.00 | 0.23 | 113 | 106 | 173 | 255 |
| NM_015076 | CDK19 | 0.00 | 0.23 | 446 | 500 | 736 | 967 |
| NM_173825 | RABL3 | 0.00 | 0.23 | 401 | 405 | 626 | 830 |
| NM_025196 | GRPEL1 | 0.00 | 0.23 | 1670 | 1551 | 1481 | 1882 |
| NM_001160302 | SYNJ1 | 0.00 | 0.23 | 400 | 406 | 555 | 741 |
| NM_032042 | FAM172 A | 0.00 | 0.23 | 316 | 320 | 1050 | 1353 |
| NM_001099922 | ALG13 | 0.00 | 0.23 | 422 | 397 | 401 | 547 |
| NM_001288661 | CRMP1 | 0.00 | 0.23 | 168 | 209 | 401 | 547 |
| NM_023926 | ZSCAN18 | 0.00 | 0.23 | 277 | 307 | 861 | 1120 |
| NM_002267 | KPNA3 | 0.00 | 0.23 | 1422 | 1542 | 1986 | 2493 |
| NM_203390 | RBM12B | 0.00 | 0.23 | 373 | 379 | 327 | 453 |
| NM_001204456 | RBAK | 0.00 | 0.23 | 385 | 373 | 368 | 505 |
| NM_003655 | CBX4 | 0.00 | 0.23 | 233 | 237 | 324 | 449 |
| NM_012097 | ARL5A | 0.00 | 0.23 | 271 | 283 | 310 | 431 |
| NM_032587 | CARD6 | 0.00 | 0.23 | 526 | 589 | 807 | 1052 |
| NM_198580 | SLC27A1 | 0.00 | 0.23 | 648 | 592 | 807 | 1052 |
| NM_016002 | SCCPDH | 0.00 | 0.23 | 589 | 545 | 846 | 1100 |
| NM_001290205 | DCP1A | -0.07 | 0.23 | 1188 | 1028 | 855 | 1111 |
| NM_014720 | SLK | -0.03 | 0.22 | 1020 | 901 | 1497 | 1896 |
| NM_020533 | MCOLN1 | 0.00 | 0.22 | 1087 | 1125 | 1782 | 2242 |
| NM_015191 | SIK2 | 0.00 | 0.22 | 930 | 991 | 1568 | 1981 |
| NM_006312 | NCOR2 | -0.07 | 0.22 | 4358 | 3934 | 4960 | 6055 |
| NM_000286 | PEX12 | 0.00 | 0.22 | 196 | 195 | 187 | 272 |
| NM_005776 | CNIH1 | 0.00 | 0.22 | 1168 | 1117 | 2315 | 2882 |
| NM_173550 | CCDC171 | 0.00 | 0.22 | 49 | 33 | 89 | 142 |
| NM_001127178 | PIGG | 0.00 | 0.22 | 776 | 743 | 1460 | 1847 |
| NM_207328 | GPAT2 | 0.00 | 0.22 | 380 | 434 | 586 | 776 |
| NM_057177 | PARD3B | 0.00 | 0.22 | 255 | 233 | 322 | 445 |
| NM_001136116 | ZNF879 | 0.00 | 0.22 | 32 | 35 | 73 | 120 |
| NM_001080744 | DGKG | 0.00 | 0.22 | 11 | 17 | 81 | 131 |
| NM_001204858 | TCEB1 | 0.00 | 0.22 | 475 | 480 | 755 | 985 |
| NM_003647 | DGKE | 0.00 | 0.22 | 171 | 161 | 152 | 226 |
| NM_001005851 | ZNF780B | 0.00 | 0.22 | 94 | 95 | 208 | 299 |
| NM_004926 | ZFP36L1 | 0.00 | 0.22 | 1831 | 1911 | 5016 | 6111 |
| NM_024076 | KCTD15 | -0.07 | 0.22 | 999 | 854 | 676 | 887 |
| NM_001284 | AP3S1 | 0.00 | 0.22 | 558 | 506 | 844 | 1094 |
| NM_181336 | LEMD2 | 0.00 | 0.22 | 1761 | 1731 | 1917 | 2397 |
| NM_032802 | SPPL2A | 0.00 | 0.22 | 460 | 440 | 761 | 991 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_005840 | SPRY3 | 0.00 | 0.22 | 60 | 57 | 87 | 139 |
| NM_032514 | MAP1LC 3A | 0.00 | 0.22 | 511 | 456 | 659 | 865 |
| NM_015060 | AVL9 | 0.00 | 0.22 | 742 | 781 | 898 | 1159 |
| NM_001135553 | MKNK1 | -0.01 | 0.22 | 493 | 421 | 613 | 808 |
| NM_001017916 | CYB561 | 0.00 | 0.22 | 931 | 951 | 2579 | 3192 |
| NM_013262 | MYLIP | 0.00 | 0.22 | 108 | 96 | 27 | 54 |
| NM_001145033 | C11orf96 | 0.00 | 0.22 | 257 | 216 | 322 | 444 |
| NM_005160 | ADRBK2 | 0.00 | 0.22 | 231 | 185 | 285 | 397 |
| NM_001229 | CASP9 | 0.00 | 0.22 | 151 | 131 | 200 | 288 |
| NM_182931 | KMT2E | -0.07 | 0.22 | 956 | 814 | 1237 | 1571 |
| NM_000876 | IGF2R | 0.00 | 0.22 | 4634 | 4625 | 13873 | 16565 |
| NM_006218 | PIK3CA | 0.00 | 0.22 | 519 | 597 | 720 | 939 |
| NM_007045 | FGFR1OP | 0.00 | 0.22 | 165 | 192 | 218 | 311 |
| NM_002141 | HOXA4 | 0.00 | 0.22 | 61 | 56 | 102 | 159 |
| NM_004559 | YBX1 | 0.00 | 0.22 | 10140 | 10047 | 9439 | 11327 |
| NM_001128827 | DLG4 | 0.00 | 0.22 | 711 | 797 | 1007 | 1290 |
| NM_001102654 | NTF3 | 0.00 | 0.22 | 357 | 335 | 254 | 357 |
| NM_001164463 | RGPD8 | 0.00 | 0.22 | 463 | 402 | 607 | 799 |
| NM_182603 | ANKRD4 2 | 0.00 | 0.22 | 294 | 286 | 422 | 569 |
| NM_001136029 | DEPDC5 | 0.00 | 0.22 | 402 | 391 | 495 | 660 |
| NM_018358 | ABCF3 | 0.00 | 0.22 | 1726 | 1753 | 2502 | 3092 |
| NM_001995 | ACSL1 | 0.00 | 0.21 | 996 | 1061 | 2059 | 2560 |
| NM_033212 | CCDC102 A | 0.00 | 0.21 | 91 | 111 | 185 | 268 |
| NM_012329 | MMD | 0.00 | 0.21 | 121 | 123 | 185 | 268 |
| NM_024165 | PHF1 | 0.00 | 0.21 | 1738 | 1611 | 2096 | 2604 |
| NM_181705 | LYRM7 | 0.00 | 0.21 | 177 | 137 | 420 | 566 |
| NM_183238 | ZNF605 | 0.00 | 0.21 | 174 | 199 | 543 | 719 |
| NM_015264 | KIAA093 0 | 0.00 | 0.21 | 1509 | 1641 | 3205 | 3930 |
| NM_017709 | FAM46C | 0.00 | 0.21 | 19 | 24 | 64 | 107 |
| NM_001543 | NDST1 | 0.01 | 0.21 | 9336 | 9720 | 14978 | 17828 |
| NM_005786 | TSHZ1 | 0.00 | 0.21 | 436 | 498 | 848 | 1094 |
| NM_145206 | VTI1A | 0.00 | 0.21 | 672 | 693 | 1175 | 1492 |
| NM_003036 | SKI | 0.01 | 0.21 | 2084 | 2251 | 2936 | 3606 |
| NM_018555 | ZNF331 | 0.00 | 0.21 | 308 | 269 | 275 | 383 |
| NM_032930 | C11orf70 | 0.00 | 0.21 | 13 | 24 | 83 | 133 |
| NM_017653 | DYM | 0.00 | 0.21 | 767 | 819 | 1535 | 1926 |
| NM_153033 | KCTD7 | 0.00 | 0.21 | 379 | 331 | 769 | 996 |
| NM_001185060 | AQP1 | 0.00 | 0.21 | 16 | 16 | 67 | 111 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_173471 | SLC25A2 6 | 0.00 | 0.21 | 155 | 178 | 376 | 510 |
| NM_173681 | ATG9B | 0.00 | 0.21 | 37 | 50 | 62 | 104 |
| NM_022735 | ACBD3 | -0.16 | 0.21 | 720 | 564 | 996 | 1272 |
| NM_001286587 | AIG1 | 0.00 | 0.21 | 204 | 234 | 778 | 1006 |
| NM_020440 | PTGFRN | -0.08 | 0.21 | 1827 | 1591 | 4571 | 5541 |
| NM_001277204 | P2RY6 | 0.00 | 0.21 | 42 | 54 | 98 | 153 |
| NM_001042551 | SMC2 | 0.00 | 0.21 | 957 | 887 | 1123 | 1425 |
| NM_001287054 | ZKSCAN 1 | 0.00 | 0.21 | 1519 | 1497 | 2789 | 3421 |
| NM_020141 | TMEM16 7B | 0.00 | 0.21 | 1103 | 1083 | 1549 | 1937 |
| NM_004398 | DDX10 | 0.00 | 0.21 | 448 | 507 | 426 | 571 |
| NM_001145312 | ETV3 | 0.00 | 0.21 | 432 | 467 | 809 | 1042 |
| NM_006049 | SNAPC5 | 0.00 | 0.21 | 505 | 545 | 661 | 861 |
| NM_175902 | OGFOD3 | 0.00 | 0.21 | 604 | 678 | 988 | 1259 |
| NM_022078 | GPATCH 3 | 0.00 | 0.21 | 217 | 268 | 277 | 384 |
| NM_004874 | BAG4 | 0.00 | 0.21 | 642 | 715 | 786 | 1013 |
| NM_001145548 | ZDHHC7 | 0.00 | 0.21 | 2633 | 2694 | 3388 | 4126 |
| NM_020644 | TMEM9B | 0.00 | 0.21 | 753 | 752 | 1551 | 1937 |
| NM_015296 | DOCK9 | 0.00 | 0.21 | 401 | 382 | 715 | 926 |
| NM_003341 | UBE2E1 | 0.00 | 0.21 | 1014 | 961 | 2231 | 2749 |
| NM_024704 | KIF16B | 0.00 | 0.21 | 439 | 395 | 601 | 786 |
| NM_000489 | ATRX | -0.35 | 0.21 | 842 | 583 | 1543 | 1926 |
| NM_001005746 | CACNB4 | 0.00 | 0.20 | 242 | 235 | 121 | 183 |
| NM_032408 | BAZ1B | 0.00 | 0.20 | 2931 | 3030 | 3816 | 4627 |
| NM_152511 | DUSP18 | 0.00 | 0.20 | 108 | 117 | 291 | 401 |
| NM_001098272 | HMGCS1 | 0.00 | 0.20 | 511 | 539 | 1350 | 1693 |
| NM_017636 | TRPM4 | 0.00 | 0.20 | 804 | 792 | 1830 | 2268 |
| NM_032632 | PAPOLA | 0.00 | 0.20 | 2105 | 2230 | 3354 | 4079 |
| NM_001043318 | ELMSAN 1 | 0.00 | 0.20 | 1199 | 1303 | 1223 | 1540 |
| NM_015520 | MAGI1 | 0.00 | 0.20 | 1339 | 1356 | 2306 | 2835 |
| NM_005067 | SIAH2 | 0.00 | 0.20 | 824 | 895 | 570 | 747 |
| NM_001166339 | SPDYE2B | 0.00 | 0.20 | 91 | 105 | 106 | 163 |
| NM_006166 | NFYB | -0.01 | 0.20 | 195 | 150 | 106 | 163 |
| NM_001031618 | SPDYE2 | 0.00 | 0.20 | 91 | 105 | 106 | 163 |
| NM_001243186 | PIM1 | 0.01 | 0.20 | 281 | 341 | 106 | 163 |
| NM_005751 | AKAP9 | -0.04 | 0.20 | 1762 | 1585 | 2059 | 2540 |
| NM_001135178 | ZNF397 | 0.00 | 0.20 | 85 | 104 | 141 | 209 |
| NM_006577 | B3GNT2 | 0.00 | 0.20 | 463 | 473 | 638 | 830 |
| NM_005341 | ZBTB48 | 0.00 | 0.20 | 146 | 146 | 324 | 442 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001270 | CHD1 | 0.00 | 0.20 | 1022 | 950 | 1040 | 1318 |
| NM_025132 | WDR19 | 0.00 | 0.20 | 201 | 246 | 653 | 848 |
| NM_022491 | SUDS3 | 0.00 | 0.20 | 512 | 553 | 884 | 1129 |
| NM_020156 | C1GALT1 | 0.00 | 0.20 | 1196 | 1162 | 1510 | 1882 |
| NM_030819 | GFOD2 | 0.00 | 0.20 | 212 | 247 | 366 | 494 |
| NM_138444 | KCTD12 | 0.00 | 0.20 | 2071 | 2025 | 2695 | 3292 |
| NM_183416 | KIF1B | 0.00 | 0.20 | 2111 | 2210 | 2556 | 3127 |
| NM_001291964 | MLLT4 | 0.00 | 0.20 | 859 | 871 | 911 | 1161 |
| NM_001800 | CDKN2D | -0.10 | 0.20 | 404 | 317 | 354 | 479 |
| NM_004993 | ATXN3 | 0.00 | 0.20 | 290 | 322 | 616 | 802 |
| NM_006738 | AKAP13 | 0.01 | 0.20 | 1679 | 1824 | 3943 | 4765 |
| NM_012326 | MAPRE3 | -0.10 | 0.20 | 186 | 133 | 657 | 852 |
| NM_000548 | TSC2 | 0.00 | 0.20 | 1727 | 1836 | 4342 | 5233 |
| NM_015089 | CUL9 | 0.00 | 0.20 | 716 | 795 | 1277 | 1601 |
| NM_006915 | RP2 | 0.00 | 0.20 | 822 | 765 | 747 | 961 |
| NM_015483 | KBTBD2 | 0.00 | 0.20 | 983 | 1041 | 1133 | 1427 |
| NM_004249 | RAB28 | 0.00 | 0.20 | 162 | 183 | 202 | 287 |
| NM_016609 | SLC22A1 7 | 0.00 | 0.20 | 245 | 261 | 374 | 503 |
| NM_016570 | ERGIC2 | 0.00 | 0.20 | 643 | 611 | 884 | 1126 |
| NM_001284338 | NEDD4 | 0.01 | 0.20 | 2996 | 3199 | 2545 | 3107 |
| NM_033201 | C16orf45 | 0.00 | 0.20 | 669 | 696 | 1568 | 1946 |
| NM_021241 | WIZ | 0.00 | 0.20 | 746 | 780 | 1171 | 1471 |
| NM_001145437 | LSS | 0.00 | 0.20 | 2263 | 2346 | 6006 | 7174 |
| NM_014738 | KIAA019 5 | 0.00 | 0.20 | 666 | 715 | 1389 | 1732 |
| NM_015409 | EP400 | -0.10 | 0.20 | 2486 | 2167 | 2891 | 3514 |
| NM_004839 | HOMER2 | 0.00 | 0.20 | 117 | 136 | 279 | 384 |
| NM_015208 | ANKRD1 2 | -0.09 | 0.20 | 1047 | 884 | 1346 | 1680 |
| NM_001607 | ACAA1 | -0.06 | 0.20 | 741 | 627 | 1568 | 1945 |
| NM_001185072 | CLDN12 | 0.00 | 0.20 | 925 | 961 | 842 | 1074 |
| NM_001099401 | SGCE | 0.00 | 0.20 | 762 | 693 | 1171 | 1469 |
| NM_001278547 | MAPK8 | 0.00 | 0.20 | 843 | 901 | 593 | 771 |
| NM_001161499 | ZNF611 | 0.00 | 0.20 | 163 | 190 | 333 | 451 |
| NM_006010 | MANF | 0.00 | 0.20 | 1647 | 1547 | 1722 | 2126 |
| NM_001081563 | DMPK | -0.05 | 0.20 | 877 | 754 | 1666 | 2059 |
| NM_001146 | ANGPT1 | 0.00 | 0.20 | 386 | 406 | 1069 | 1346 |
| NM_001080481 | USP45 | 0.00 | 0.19 | 178 | 196 | 262 | 362 |
| NM_152699 | SENP5 | 0.00 | 0.19 | 1291 | 1380 | 2019 | 2477 |
| NM_001167 | XIAP | 0.00 | 0.19 | 954 | 928 | 1720 | 2122 |

(continued)

| RefSeq ID | Gene Symbol | SR GFOLD | SEN GFOLD | SR IL-2 - | SR IL-2 + | SEN IL-2 - | SEN IL-2 + |
|-----------|-------------|----------|-----------|-----------|-----------|------------|------------|
| NM_001124756 | PABPC1L | 0.00 | 0.19 | 102 | 112 | 166 | 240 |
| NM_016423 | ZNF219 | 0.00 | 0.19 | 144 | 117 | 166 | 240 |
| NM_001008707 | EML1 | 0.00 | 0.19 | 680 | 692 | 1847 | 2272 |
| NM_144709 | PUS10 | 0.00 | 0.19 | 74 | 52 | 94 | 146 |
| NM_001122770 | ZBTB37 | 0.00 | 0.19 | 120 | 120 | 156 | 227 |
| NM_002505 | NFYA | 0.00 | 0.19 | 1086 | 1126 | 1262 | 1575 |
| NM_033271 | BTBD6 | 0.00 | 0.19 | 916 | 1020 | 1491 | 1848 |
| NM_003377 | VEGFB | 0.00 | 0.19 | 989 | 1070 | 1832 | 2253 |
| NM_001008224 | UACA | -0.01 | 0.19 | 2500 | 2317 | 6185 | 7357 |
| NM_017791 | FLVCR2 | 0.00 | 0.19 | 49 | 30 | 60 | 100 |
| NM_001134233 | C1orf204 | 0.00 | 0.19 | 39 | 51 | 60 | 100 |
| NM_006511 | RSC1A1 | 0.00 | 0.19 | 212 | 250 | 241 | 335 |
| NM_021626 | SCPEP1 | 0.00 | 0.19 | 661 | 594 | 1614 | 1993 |
| NM_013349 | NENF | 0.00 | 0.19 | 630 | 702 | 1574 | 1945 |
| NM_016481 | C9orf156 | 0.00 | 0.19 | 131 | 150 | 200 | 283 |
| NM_024831 | TGS1 | 0.00 | 0.19 | 433 | 447 | 472 | 621 |
| NM_018109 | MTPAP | 0.00 | 0.19 | 481 | 521 | 595 | 771 |
| NM_080677 | DYNLL2 | 0.00 | 0.19 | 968 | 1063 | 1202 | 1501 |
| NM_005476 | GNE | 0.00 | 0.19 | 893 | 921 | 1108 | 1388 |
| NM_001044264 | MSMP | 0.00 | 0.19 | 231 | 284 | 299 | 407 |
| NM_001286522 | PARP11 | 0.00 | 0.19 | 81 | 78 | 158 | 229 |
| NM_004586 | RPS6KA3 | 0.00 | 0.19 | 1431 | 1541 | 1934 | 2368 |
| NM_020645 | NRIP3 | 0.00 | 0.19 | 1169 | 1198 | 1986 | 2429 |
| NM_057169 | GIT2 | -0.04 | 0.19 | 1688 | 1511 | 2406 | 2924 |
| NM_030762 | BHLHE41 | 0.00 | 0.19 | 416 | 434 | 609 | 787 |
| NM_001278529 | ARHGEF 40 | 0.00 | 0.19 | 2216 | 2079 | 4193 | 5018 |
| NM_000600 | IL6 | 0.00 | 0.19 | 434 | 502 | 782 | 996 |
| NM_001040628 | NCALD | 0.01 | 0.19 | 314 | 377 | 851 | 1079 |
| NM_024685 | BBS10 | 0.00 | 0.19 | 204 | 221 | 329 | 444 |
| NM_007043 | KRR1 | 0.00 | 0.19 | 825 | 916 | 1547 | 1909 |
| NM_033070 | CECR5 | 0.00 | 0.19 | 608 | 688 | 559 | 726 |
| NM_001040177 | AKR1E2 | 0.00 | 0.19 | 213 | 249 | 206 | 290 |
| NM_004453 | ETFDH | 0.00 | 0.19 | 337 | 329 | 663 | 852 |
| NM_025195 | TRIB1 | 0.00 | 0.19 | 535 | 595 | 491 | 643 |
| NM_001109662 | HECTD4 | 0.00 | 0.19 | 1699 | 1825 | 3088 | 3723 |
| NM_005038 | PPID | 0.00 | 0.19 | 464 | 424 | 726 | 928 |
| NM_182838 | SLC35E2 | 0.00 | 0.19 | 693 | 718 | 1296 | 1610 |
| NM_001042750 | STAG2 | 0.00 | 0.19 | 1272 | 1274 | 1799 | 2205 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_181715 | CRTC2 | 0.00 | 0.19 | 831 | 849 | 923 | 1164 |
| NM_014949 | KIAA090 7 | 0.00 | 0.19 | 588 | 668 | 848 | 1074 |
| NM_018140 | CEP72 | 0.00 | 0.19 | 107 | 142 | 75 | 120 |
| NM_001077690 | ALG9 | -0.08 | 0.19 | 694 | 576 | 1069 | 1338 |
| NM_016263 | FZR1 | 0.00 | 0.19 | 1169 | 1207 | 1736 | 2129 |
| NM_001282659 | USP47 | 0.00 | 0.19 | 1311 | 1204 | 2213 | 2691 |
| NM_001009881 | ZCCHC11 | 0.00 | 0.19 | 432 | 462 | 684 | 876 |
| NM_032772 | ZNF503 | -0.22 | 0.19 | 273 | 188 | 867 | 1096 |
| NM_001198777 | CUL2 | 0.00 | 0.19 | 877 | 971 | 1418 | 1752 |
| NM_001113575 | CDKL3 | 0.00 | 0.19 | 28 | 35 | 64 | 105 |
| NM_022072 | NSUN3 | 0.00 | 0.18 | 81 | 85 | 106 | 161 |
| NM_014982 | PCNX | 0.00 | 0.18 | 2292 | 2240 | 5180 | 6150 |
| NM_002199 | IRF2 | 0.00 | 0.18 | 393 | 430 | 322 | 434 |
| NM_006037 | HDAC4 | -0.07 | 0.18 | 502 | 410 | 499 | 651 |
| NM_003054 | SLC18A2 | 0.00 | 0.18 | 76 | 91 | 179 | 255 |
| NM_004691 | ATP6V0D 1 | 0.00 | 0.18 | 2855 | 2818 | 4910 | 5834 |
| NM_001277058 | ERCC6-PGBD3 | 0.00 | 0.18 | 1044 | 962 | 1065 | 1331 |
| NM_032027 | TM2D1 | -0.13 | 0.18 | 478 | 371 | 844 | 1067 |
| NM_133447 | AGAP11 | 0.00 | 0.18 | 88 | 65 | 100 | 153 |
| NM_020371 | AVEN | 0.00 | 0.18 | 645 | 689 | 1056 | 1320 |
| NM_005809 | PRDX2 | 0.00 | 0.18 | 1009 | 1091 | 2302 | 2791 |
| NM_015312 | KIAA110 9 | 0.00 | 0.18 | 1917 | 1857 | 5303 | 6288 |
| NM_015344 | LEPROT L1 | 0.00 | 0.18 | 374 | 383 | 595 | 767 |
| NM_004667 | HERC2 | -0.11 | 0.18 | 4379 | 3858 | 6971 | 8220 |
| NM_015221 | DNMBP | 0.00 | 0.18 | 2235 | 2202 | 4407 | 5244 |
| NM_015001 | SPEN | -0.09 | 0.18 | 2365 | 2078 | 2479 | 2996 |
| NM_020640 | DCUN1D 1 | 0.00 | 0.18 | 318 | 342 | 551 | 713 |
| NM_014765 | TOMM20 | 0.00 | 0.18 | 2334 | 2210 | 3284 | 3935 |
| NM_030937 | CCNL2 | -0.03 | 0.18 | 1650 | 1482 | 2202 | 2669 |
| NM_032846 | RAB2B | 0.00 | 0.18 | 559 | 552 | 784 | 993 |
| NM_017916 | PIH1D1 | 0.00 | 0.18 | 626 | 707 | 903 | 1135 |
| NM_001172681 | ZNF641 | 0.00 | 0.18 | 210 | 211 | 216 | 301 |
| NM_014041 | SPCS1 | 0.00 | 0.18 | 892 | 820 | 1593 | 1952 |
| NM_012455 | PSD4 | -0.03 | 0.18 | 520 | 437 | 499 | 649 |
| NM_016078 | TVP23B | -0.12 | 0.18 | 486 | 381 | 499 | 649 |
| NM_007249 | KLF12 | 0.00 | 0.18 | 734 | 771 | 1113 | 1384 |
| NM_003030 | SHOX2 | 0.00 | 0.18 | 302 | 288 | 372 | 494 |
| NM_001291965 | WASF3 | 0.00 | 0.18 | 764 | 679 | 961 | 1203 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_020959 | ANO8 | 0.00 | 0.18 | 297 | 247 | 233 | 322 |
| NM_001136037 | LIMS2 | 0.00 | 0.18 | 1252 | 1364 | 1194 | 1479 |
| NM_206825 | GNL3 | 0.00 | 0.18 | 1761 | 1767 | 1435 | 1763 |
| NM_001694 | ATP6V0C | -0.10 | 0.18 | 3092 | 2708 | 4147 | 4926 |
| NM_015994 | ATP6V1D | -0.05 | 0.18 | 1908 | 1710 | 2487 | 2996 |
| NM_000328 | RPGR | 0.00 | 0.18 | 79 | 67 | 102 | 155 |
| NM_014320 | HEBP2 | 0.00 | 0.18 | 634 | 616 | 1936 | 2351 |
| NM_020328 | ACVR1B | -0.09 | 0.18 | 874 | 731 | 1059 | 1318 |
| NM_032164 | ZNF394 | 0.00 | 0.18 | 376 | 383 | 443 | 580 |
| NM_001282531 | ADNP | 0.00 | 0.18 | 2120 | 1993 | 2325 | 2806 |
| NM_001042683 | SHPRH | 0.00 | 0.18 | 342 | 347 | 366 | 486 |
| NM_006442 | DRAP1 | 0.00 | 0.18 | 5589 | 5634 | 7776 | 9113 |
| NM_001076675 | ZNF626 | 0.00 | 0.18 | 72 | 67 | 195 | 274 |
| NM_031296 | RAB33B | 0.00 | 0.18 | 240 | 243 | 195 | 274 |
| NM_004486 | GOLGA2 | 0.00 | 0.18 | 5127 | 5348 | 5603 | 6606 |
| NM_024874 | KIAA031 9L | 0.00 | 0.18 | 862 | 954 | 2350 | 2834 |
| NM_014646 | LPIN2 | 0.00 | 0.18 | 1762 | 1689 | 2132 | 2579 |
| NM_001256865 | DNAJC6 | 0.00 | 0.18 | 98 | 105 | 293 | 396 |
| NM_153257 | ZNF461 | 0.00 | 0.18 | 86 | 75 | 119 | 177 |
| NM_024116 | TAF1D | 0.00 | 0.18 | 733 | 717 | 564 | 726 |
| NM_014015 | DEXI | 0.00 | 0.18 | 790 | 836 | 1206 | 1490 |
| NM_018477 | ACTR10 | -0.04 | 0.18 | 678 | 580 | 1751 | 2131 |
| NM_000332 | ATXN1 | 0.00 | 0.18 | 1049 | 1027 | 1734 | 2111 |
| NM_032373 | PCGF5 | 0.00 | 0.17 | 1612 | 1703 | 2321 | 2797 |
| NM_001161841 | SULF2 | 0.00 | 0.17 | 58 | 41 | 166 | 237 |
| NM_031431 | COG3 | 0.00 | 0.17 | 514 | 540 | 815 | 1026 |
| NM_014643 | ZNF516 | 0.00 | 0.17 | 891 | 861 | 1508 | 1845 |
| NM_033063 | MAP6 | 0.00 | 0.17 | 99 | 132 | 453 | 591 |
| NM_015562 | UBXN7 | 0.00 | 0.17 | 598 | 580 | 1113 | 1379 |
| NM_015322 | FEM1B | 0.00 | 0.17 | 1692 | 1830 | 2581 | 3098 |
| NM_198150 | ARSK | 0.00 | 0.17 | 445 | 461 | 530 | 684 |
| NM_003205 | TCF12 | 0.00 | 0.17 | 1776 | 1818 | 2710 | 3248 |
| NM_001198862 | TMEM20 6 | 0.00 | 0.17 | 385 | 431 | 335 | 447 |
| NM_015962 | FCF1 | 0.00 | 0.17 | 265 | 298 | 426 | 558 |
| NM_033215 | PPP1R3F | 0.00 | 0.17 | 87 | 86 | 127 | 187 |
| NM_001077472 | NR1I3 | 0.00 | 0.17 | 70 | 93 | 110 | 165 |
| NM_032888 | COL27A1 | 0.00 | 0.17 | 464 | 471 | 437 | 571 |
| NM_172159 | KCNAB1 | -0.01 | 0.17 | 186 | 143 | 206 | 287 |

(continued)

| RefSeq ID | Gene Symbol | SR GFOLD | SEN GFOLD | SR IL-2 - | SR IL-2 + | SEN IL-2 - | SEN IL-2 + |
|-----------|-------------|----------|-----------|-----------|-----------|------------|------------|
| NM_000617 | SLC11A2 | -0.15 | 0.17 | 705 | 556 | 1077 | 1335 |
| NM_001198961 | ECHDC2 | 0.00 | 0.17 | 110 | 99 | 131 | 192 |
| NM_031492 | RBM4B | 0.00 | 0.17 | 348 | 377 | 424 | 555 |
| NM_001079839 | OCIAD1 | 0.00 | 0.17 | 946 | 1022 | 1753 | 2129 |
| NM_033512 | TSPYL5 | 0.00 | 0.17 | 463 | 482 | 549 | 706 |
| NM_007110 | TEP1 | 0.00 | 0.17 | 1816 | 1748 | 2429 | 2917 |
| NM_018453 | EAPP | 0.00 | 0.17 | 435 | 378 | 634 | 808 |
| NM_030930 | UNC93B 1 | -0.21 | 0.17 | 399 | 288 | 453 | 590 |
| NM_017870 | TMEM13 2A | 0.00 | 0.17 | 257 | 277 | 374 | 494 |
| NM_032575 | GLIS2 | 0.01 | 0.17 | 543 | 625 | 482 | 625 |
| NM_138421 | SAAL1 | 0.00 | 0.17 | 144 | 168 | 104 | 157 |
| NM_001145343 | ZNF566 | 0.00 | 0.17 | 80 | 79 | 104 | 157 |
| NM_015575 | GIGYF2 | 0.00 | 0.17 | 2081 | 2145 | 2882 | 3442 |
| NM_020834 | HOMEZ | 0.00 | 0.17 | 187 | 188 | 312 | 418 |
| NM_018439 | IMPACT | 0.00 | 0.17 | 285 | 319 | 611 | 780 |
| NM_001278593 | ALDH6A 1 | 0.00 | 0.17 | 183 | 205 | 412 | 540 |
| NM_007282 | RNF13 | 0.00 | 0.17 | 357 | 350 | 493 | 638 |
| NM_003786 | ABCC3 | 0.00 | 0.17 | 282 | 328 | 1431 | 1750 |
| NM_022737 | LPPR2 | 0.00 | 0.17 | 1280 | 1344 | 2209 | 2658 |
| NM_001040099 | MOSPD3 | 0.00 | 0.17 | 145 | 158 | 418 | 547 |
| NM_078476 | BTN2A1 | 0.00 | 0.17 | 281 | 261 | 524 | 675 |
| NM_006726 | LRBA | -0.12 | 0.17 | 912 | 747 | 1747 | 2118 |
| NM_001242739 | ZNF691 | 0.00 | 0.17 | 104 | 112 | 158 | 226 |
| NM_001018055 | BRCC3 | 0.00 | 0.17 | 144 | 152 | 364 | 481 |
| NM_016038 | SBDS | 0.00 | 0.17 | 3520 | 3446 | 4658 | 5486 |
| NM_001243773 | GNG2 | 0.00 | 0.17 | 25 | 31 | 541 | 695 |
| NM_001077195 | ZNF436 | 0.01 | 0.17 | 246 | 302 | 566 | 725 |
| NM_178040 | ERC1 | 0.00 | 0.17 | 1744 | 1662 | 3438 | 4079 |
| NM_182958 | KAT8 | 0.00 | 0.17 | 463 | 455 | 547 | 702 |
| NM_025015 | HSPA12A | -0.03 | 0.17 | 1144 | 1012 | 2161 | 2599 |
| NM_002336 | LRP6 | 0.00 | 0.17 | 1354 | 1366 | 2223 | 2671 |
| NM_018017 | CCDC186 | 0.00 | 0.17 | 305 | 309 | 356 | 471 |
| NM_002843 | PTPRJ | 0.00 | 0.17 | 1790 | 1748 | 1655 | 2009 |
| NM_003592 | CUL1 | 0.00 | 0.17 | 1515 | 1494 | 1739 | 2107 |
| NM_004914 | RAB36 | 0.00 | 0.17 | 30 | 20 | 133 | 194 |
| NM_017842 | SLC48A1 | 0.00 | 0.17 | 367 | 365 | 1181 | 1453 |
| NM_013302 | EEF2K | 0.00 | 0.17 | 1168 | 1169 | 1791 | 2166 |
| NM_020897 | HCN3 | 0.00 | 0.17 | 70 | 87 | 148 | 213 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_017450 | BAIAP2 | 0.00 | 0.17 | 272 | 327 | 844 | 1055 |
| NM_024599 | RHBDF2 | 0.00 | 0.17 | 311 | 288 | 331 | 440 |
| NM_001136157 | OTUD5 | 0.00 | 0.17 | 1295 | 1405 | 2100 | 2525 |
| NM_001162501 | TNRC6B | -0.01 | 0.17 | 1842 | 1691 | 3768 | 4453 |
| NM_015510 | DHRS7B | 0.00 | 0.17 | 344 | 359 | 547 | 701 |
| NM_145725 | TRAF3 | 0.00 | 0.17 | 1473 | 1384 | 2315 | 2774 |
| NM_001135218 | TAF12 | 0.00 | 0.17 | 419 | 388 | 624 | 793 |
| NM_005631 | SMO | 0.00 | 0.17 | 26 | 27 | 83 | 129 |
| NM_153223 | CEP120 | 0.00 | 0.17 | 420 | 475 | 703 | 887 |
| NM_003473 | STAM | 0.00 | 0.17 | 1601 | 1604 | 1512 | 1839 |
| NM_022736 | MFSD1 | 0.00 | 0.17 | 877 | 964 | 2117 | 2543 |
| NM_030640 | DUSP16 | 0.00 | 0.17 | 445 | 461 | 441 | 573 |
| NM_001128325 | SPON2 | 0.00 | 0.17 | 752 | 706 | 4993 | 5856 |
| NM_018438 | FBXO6 | 0.00 | 0.17 | 73 | 70 | 333 | 442 |
| NM_001171741 | C3orf18 | 0.00 | 0.17 | 254 | 284 | 786 | 985 |
| NM_030581 | WDR59 | -0.19 | 0.17 | 656 | 500 | 824 | 1030 |
| NM_016281 | TAOK3 | 0.00 | 0.17 | 1291 | 1196 | 2144 | 2573 |
| NM_031288 | INO80B | 0.00 | 0.16 | 294 | 271 | 541 | 693 |
| NM_001018072 | BTBD11 | 0.00 | 0.16 | 43 | 27 | 71 | 113 |
| NM_001278141 | NBPF12 | 0.00 | 0.16 | 234 | 230 | 385 | 505 |
| NM_000534 | PMS1 | -0.08 | 0.16 | 339 | 266 | 289 | 388 |
| NM_001100397 | RAPGEF4 | 0.00 | 0.16 | 487 | 548 | 414 | 540 |
| NM_153026 | PRICKLE 1 | 0.00 | 0.16 | 156 | 184 | 474 | 612 |
| NM_001145447 | ZNF200 | 0.00 | 0.16 | 140 | 149 | 154 | 220 |
| NM_015106 | RAD54L2 | 0.00 | 0.16 | 999 | 1098 | 1029 | 1270 |
| NM_001001521 | UGP2 | 0.00 | 0.16 | 1664 | 1579 | 3275 | 3872 |
| NM_003190 | TAPBP | 0.00 | 0.16 | 3127 | 3266 | 5648 | 6591 |
| NM_018094 | GSPT2 | 0.00 | 0.16 | 809 | 846 | 1011 | 1248 |
| NM_001281433 | ZNHIT3 | 0.00 | 0.16 | 382 | 411 | 433 | 562 |
| NM_014991 | WDFY3 | 0.00 | 0.16 | 2133 | 2177 | 4066 | 4780 |
| NM_015202 | KIAA055 | 0.00 | 0.16 | 646 | 661 | 1387 | 1688 |
|  | 6 |  |  |  |  |  |  |
| NM_003087 | SNCG | 0.00 | 0.16 | 51 | 37 | 125 | 183 |
| NM_020317 | RSRP1 | 0.00 | 0.16 | 758 | 774 | 961 | 1189 |
| NM_001113407 | LDB1 | 0.00 | 0.16 | 1296 | 1361 | 2144 | 2567 |
| NM_032410 | HOOK3 | 0.00 | 0.16 | 2021 | 2119 | 2857 | 3390 |
| NM_021103 | TMSB10 | -0.20 | 0.16 | 41209 | 35245 | 46245 | 52478 |
| NM_001256732 | SSBP2 | 0.00 | 0.16 | 212 | 206 | 406 | 529 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001142640 | TNRC6C | 0.00 | 0.16 | 332 | 288 | 187 | 261 |
| NM_080652 | TMEM41 A | 0.00 | 0.16 | 392 | 395 | 537 | 686 |
| NM_005811 | GDF11 | 0.00 | 0.16 | 655 | 694 | 1541 | 1865 |
| NM_001135642 | INPP5K | 0.00 | 0.16 | 634 | 715 | 1004 | 1238 |
| NM_013233 | STK39 | 0.00 | 0.16 | 1047 | 1084 | 1115 | 1368 |
| NM_006912 | RIT1 | 0.00 | 0.16 | 784 | 836 | 824 | 1026 |
| NM_014892 | SCAF8 | 0.00 | 0.16 | 1290 | 1304 | 1718 | 2070 |
| NM_001042681 | RERE | 0.00 | 0.16 | 1313 | 1222 | 1422 | 1726 |
| NM_001660 | ARF4 | 0.00 | 0.16 | 5936 | 5927 | 7098 | 8231 |
| NM_014008 | CCDC22 | 0.00 | 0.16 | 493 | 477 | 932 | 1153 |
| NM_014377 | DNAJC2 | 0.00 | 0.16 | 347 | 337 | 428 | 555 |
| NM_001257137 | ITCH | 0.01 | 0.16 | 1751 | 1903 | 2828 | 3351 |
| NM_001032280 | TFAP2A | 0.00 | 0.16 | 44 | 43 | 27 | 52 |
| NM_213613 | SLC26A1 | 0.00 | 0.16 | 127 | 103 | 168 | 237 |
| NM_015042 | ZNF609 | 0.00 | 0.16 | 1587 | 1646 | 1788 | 2150 |
| NM_001277 | CHKA | 0.00 | 0.16 | 419 | 401 | 310 | 412 |
| NM_182715 | SYPL1 | -0.16 | 0.16 | 1654 | 1362 | 2568 | 3050 |
| NM_024090 | ELOVL6 | 0.00 | 0.16 | 999 | 1055 | 1260 | 1536 |
| NM_030806 | C1orf21 | 0.00 | 0.16 | 857 | 946 | 998 | 1229 |
| NM_001080503 | CCDC159 | -0.09 | 0.16 | 73 | 44 | 181 | 253 |
| NM_006635 | ZNF460 | 0.00 | 0.16 | 770 | 856 | 1237 | 1508 |
| NM_194285 | SPTY2D1 | 0.01 | 0.16 | 874 | 978 | 942 | 1163 |
| NM_001282755 | MTA3 | -0.06 | 0.16 | 387 | 312 | 717 | 898 |
| NM_014290 | TDRD7 | 0.00 | 0.16 | 273 | 270 | 453 | 584 |
| NM_198467 | RSBN1L | 0.00 | 0.16 | 193 | 217 | 202 | 279 |
| NM_005530 | IDH3A | 0.00 | 0.16 | 982 | 1057 | 1524 | 1841 |
| NM_001007527 | LMBRD2 | 0.00 | 0.16 | 284 | 258 | 489 | 627 |
| NM_030912 | TRIM8 | 0.00 | 0.16 | 2173 | 2295 | 3552 | 4174 |
| NM_023070 | ZFP69B | 0.00 | 0.16 | 69 | 80 | 73 | 115 |
| NM_014834 | LRRC37A | 0.00 | 0.16 | 176 | 174 | 241 | 327 |
| NM_007159 | SLMAP | 0.00 | 0.16 | 921 | 954 | 1223 | 1490 |
| NM_006329 | FBLN5 | 0.00 | 0.15 | 5879 | 6117 | 8801 | 10137 |

**Table 5B: Differetially Upregulated Genes in SEN + IL-2**

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_173811 | HARBI1 | 0.00 | 0.15 | 45 | 59 | 110 | 163 |
| NM_001272034 | STXBP2 | 0.00 | 0.15 | 650 | 603 | 472 | 606 |
| NM_001288718 | STAT5A | 0.00 | 0.15 | 562 | 633 | 803 | 998 |
| NM_018064 | AKIRIN2 | 0.00 | 0.15 | 504 | 537 | 653 | 821 |

(continued)

| Table 5B: Differetially Upregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_001134369 | LRRFIP2 | 0.00 | 0.15 | 1869 | 1941 | 2340 | 2780 |
| NM_032145 | FBXO30 | 0.00 | 0.15 | 573 | 634 | 1073 | 1314 |
| NM_013376 | SERTAD 1 | 0.00 | 0.15 | 530 | 587 | 665 | 835 |
| NM_015622 | CCZ1 | 0.00 | 0.15 | 769 | 789 | 898 | 1109 |
| NM_007212 | RNF2 | 0.00 | 0.15 | 314 | 360 | 408 | 529 |
| NM_033504 | TMEM54 | 0.00 | 0.15 | 252 | 226 | 547 | 695 |
| NM_001190983 | COQ7 | 0.00 | 0.15 | 159 | 147 | 243 | 329 |
| NM_080916 | DGUOK | 0.00 | 0.15 | 469 | 528 | 672 | 843 |
| NM_139015 | SPPL3 | 0.00 | 0.15 | 477 | 485 | 553 | 702 |
| NM_001173487 | NKRF | 0.00 | 0.15 | 293 | 317 | 293 | 390 |
| NM_152519 | KANSL1 L | 0.00 | 0.15 | 255 | 250 | 345 | 453 |
| NM_001286276 | KIAA158 6 | 0.00 | 0.15 | 194 | 214 | 266 | 357 |
| NM_015141 | GPD1L | 0.00 | 0.15 | 100 | 136 | 460 | 591 |
| NM_001029882 | AHDC1 | 0.00 | 0.15 | 515 | 559 | 713 | 891 |
| NM_001271641 | MTCH1 | 0.00 | 0.15 | 5598 | 5417 | 8622 | 9920 |
| NM_018121 | FAM178 A | 0.00 | 0.15 | 933 | 879 | 1289 | 1564 |
| NM_015358 | MORC3 | 0.00 | 0.15 | 887 | 831 | 1237 | 1503 |
| NM_007195 | POLI | 0.00 | 0.15 | 174 | 191 | 416 | 538 |
| NM_138459 | NUS1 | 0.00 | 0.15 | 714 | 786 | 830 | 1028 |
| NM_004232 | SOCS6 | 0.00 | 0.15 | 795 | 758 | 1250 | 1518 |
| NM_201444 | DGKA | 0.00 | 0.15 | 1303 | 1400 | 2202 | 2617 |
| NM_018120 | ARMC1 | 0.00 | 0.15 | 611 | 644 | 819 | 1015 |
| NM_138492 | PRELID2 | 0.00 | 0.15 | 114 | 135 | 104 | 155 |
| NM_003342 | UBE2G1 | 0.00 | 0.15 | 1199 | 1182 | 1258 | 1527 |
| NM_013379 | DPP7 | 0.00 | 0.15 | 1303 | 1265 | 3589 | 4203 |
| NM_001290259 | PHF3 | 0.00 | 0.15 | 1200 | 1153 | 1680 | 2015 |
| NM_012094 | PRDX5 | 0.00 | 0.15 | 2093 | 2093 | 3887 | 4542 |
| NM_000140 | FECH | 0.00 | 0.15 | 559 | 584 | 1079 | 1318 |
| NM_004239 | TRIP11 | -0.06 | 0.15 | 986 | 851 | 1190 | 1447 |
| NM_024933 | ANKRD5 3 | 0.00 | 0.15 | 61 | 69 | 94 | 142 |
| NM_006026 | H1FX | 0.00 | 0.15 | 2012 | 2162 | 930 | 1144 |
| NM_021930 | RINT1 | 0.00 | 0.15 | 555 | 628 | 813 | 1007 |
| NM_001042601 | TTC14 | 0.00 | 0.15 | 466 | 418 | 722 | 900 |
| NM_024989 | PGAP1 | 0.00 | 0.15 | 201 | 200 | 283 | 377 |
| NM_004285 | H6PD | 0.00 | 0.15 | 2069 | 2212 | 5199 | 6029 |
| NM_032582 | USP32 | 0.00 | 0.15 | 2316 | 2338 | 2984 | 3508 |
| NM_015383 | NBPF14 | -0.09 | 0.15 | 476 | 382 | 834 | 1031 |

(continued)

| Table 5B: Differetially Upregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_001173539 | BANP | 0.00 | 0.15 | 436 | 446 | 247 | 333 |
| NM_021738 | SVIL | 0.00 | 0.15 | 1117 | 1070 | 2521 | 2978 |
| NM_014247 | RAPGEF2 | 0.00 | 0.15 | 978 | 1062 | 1753 | 2096 |
| NM_001500 | GMDS | 0.00 | 0.15 | 255 | 236 | 337 | 442 |
| NM_203351 | MAP3K3 | 0.00 | 0.15 | 1932 | 1896 | 2283 | 2704 |
| NM_001135586 | C5orf24 | 0.00 | 0.15 | 1800 | 1701 | 2500 | 2952 |
| NM_014547 | TMOD3 | 0.00 | 0.15 | 3025 | 3063 | 2579 | 3042 |
| NM_017926 | GPATCH 2L | 0.01 | 0.15 | 824 | 927 | 1801 | 2150 |
| NM_021008 | DEAF1 | 0.00 | 0.15 | 1036 | 1020 | 1346 | 1625 |
| NM_053043 | RBM33 | 0.00 | 0.15 | 977 | 991 | 1264 | 1530 |
| NM_015104 | ATG2A | 0.00 | 0.15 | 817 | 819 | 1046 | 1277 |
| NM_005955 | MTF1 | 0.00 | 0.15 | 383 | 365 | 562 | 710 |
| NM_001025091 | ABCF1 | 0.00 | 0.15 | 2545 | 2399 | 3180 | 3726 |
| NM_175063 | EMC10 | -0.04 | 0.15 | 1293 | 1143 | 1508 | 1811 |
| NM_032169 | ACAD11 | 0.00 | 0.15 | 213 | 209 | 537 | 680 |
| NM_004738 | VAPB | 0.00 | 0.15 | 1399 | 1511 | 1801 | 2148 |
| NM_022574 | GIGYF1 | 0.00 | 0.15 | 887 | 937 | 942 | 1155 |
| NM_001017930 | DCAF8L1 | 0.00 | 0.15 | 56 | 68 | 92 | 139 |
| NM_001173990 | TMEM21 6 | 0.00 | 0.15 | 98 | 108 | 92 | 139 |
| NM_001013627 | NHSL2 | 0.00 | 0.15 | 83 | 69 | 92 | 139 |
| NM_001039671 | YIF1B | 0.00 | 0.15 | 1828 | 1758 | 2084 | 2471 |
| NM_007034 | DNAJB4 | 0.00 | 0.15 | 2631 | 2621 | 7077 | 8131 |
| NM_005359 | SMAD4 | 0.00 | 0.15 | 1975 | 2035 | 2219 | 2625 |
| NM_015203 | RPRD2 | 0.00 | 0.15 | 1511 | 1536 | 1868 | 2222 |
| NM_001024455 | RGAG4 | 0.00 | 0.14 | 260 | 281 | 435 | 558 |
| NM_001278173 | ZNF33A | 0.00 | 0.14 | 325 | 301 | 358 | 466 |
| NM_033198 | PIGS | 0.00 | 0.14 | 1770 | 1760 | 2776 | 3259 |
| NM_001080475 | PLEKHM 3 | 0.00 | 0.14 | 370 | 369 | 809 | 998 |
| NM_001098484 | SLC4A4 | 0.00 | 0.14 | 2533 | 2684 | 2252 | 2660 |
| NM_002897 | RBMS1 | 0.00 | 0.14 | 807 | 861 | 1258 | 1519 |
| NM_017420 | SIX4 | 0.00 | 0.14 | 229 | 261 | 426 | 547 |
| NM_001204890 | ISY1-RAB43 | 0.00 | 0.14 | 1036 | 1148 | 1366 | 1643 |
| NM_030791 | SGPP1 | 0.00 | 0.14 | 1023 | 1117 | 1077 | 1309 |
| NM_016024 | RBMX2 | -0.40 | 0.14 | 326 | 200 | 231 | 312 |
| NM_005654 | NR2F1 | 0.00 | 0.14 | 246 | 259 | 520 | 658 |
| NM_005426 | TP53BP2 | 0.00 | 0.14 | 1071 | 1141 | 1140 | 1381 |
| NM_181725 | METTL2 A | 0.00 | 0.14 | 622 | 692 | 670 | 834 |

(continued)

| Table 5B: Differetially Upregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_032268 | ZNRF1 | 0.00 | 0.14 | 485 | 479 | 441 | 564 |
| NM_017924 | C14orf119 | 0.00 | 0.14 | 1461 | 1370 | 2092 | 2473 |
| NM_001113324 | TEN1 | 0.00 | 0.14 | 389 | 438 | 784 | 967 |
| NM_015325 | ICE1 | 0.00 | 0.14 | 1076 | 1127 | 1755 | 2087 |
| NM_001185039 | ACOX1 | 0.00 | 0.14 | 1430 | 1392 | 3086 | 3603 |
| NM_001291586 | TMEM10 6A | 0.00 | 0.14 | 481 | 494 | 996 | 1213 |
| NM_182703 | ANKDD1 A | 0.00 | 0.14 | 109 | 131 | 206 | 281 |
| NM_176787 | PIGN | 0.00 | 0.14 | 418 | 383 | 751 | 928 |
| NM_207310 | CCDC74 B | 0.00 | 0.14 | 27 | 19 | 64 | 102 |
| NM_005486 | TOM1L1 | 0.00 | 0.14 | 106 | 124 | 64 | 102 |
| NM_015046 | SETX | 0.00 | 0.14 | 1160 | 1114 | 2808 | 3285 |
| NM_001289999 | NFIL3 | 0.00 | 0.14 | 606 | 528 | 913 | 1116 |
| NM_024884 | L2HGDH | 0.00 | 0.14 | 91 | 80 | 58 | 94 |
| NM_001102564 | IFT43 | 0.00 | 0.14 | 264 | 250 | 649 | 808 |
| NM_001002857 | ANXA2 | -0.03 | 0.14 | 14766 | 14085 | 27335 | 30694 |
| NM_018341 | ERMARD | -0.17 | 0.14 | 273 | 195 | 327 | 427 |
| NM_000674 | ADORA1 | 0.00 | 0.14 | 52 | 66 | 98 | 146 |
| NM_001167614 | LEMD3 | 0.00 | 0.14 | 556 | 576 | 790 | 972 |
| NM_024623 | OGFOD2 | 0.00 | 0.14 | 416 | 376 | 572 | 717 |
| NM_020823 | TMEM18 1 | 0.00 | 0.14 | 1863 | 1882 | 3196 | 3719 |
| NM_007198 | PROSC | 0.00 | 0.14 | 422 | 493 | 643 | 800 |
| NM_007349 | PAXIP1 | 0.00 | 0.14 | 501 | 472 | 349 | 453 |
| NM_015909 | NBAS | 0.00 | 0.14 | 2372 | 2506 | 3862 | 4469 |
| NM_032549 | IMMP2L | 0.00 | 0.14 | 55 | 67 | 141 | 200 |
| NM_178836 | PLD6 | 0.00 | 0.14 | 180 | 196 | 133 | 190 |
| NM_001195296 | MIPOL1 | 0.00 | 0.14 | 90 | 83 | 133 | 190 |
| NM_003590 | CUL3 | 0.00 | 0.14 | 1024 | 979 | 1034 | 1253 |
| NM_153369 | KIAA191 9 | 0.00 | 0.14 | 99 | 105 | 166 | 231 |
| NM_032286 | MED10 | 0.00 | 0.14 | 972 | 1041 | 1314 | 1575 |
| NM_001277444 | NBPF9 | -0.02 | 0.14 | 905 | 799 | 1335 | 1599 |
| NM_001199135 | TANK | 0.00 | 0.14 | 546 | 590 | 757 | 932 |
| NM_032861 | SERAC1 | 0.00 | 0.14 | 356 | 390 | 532 | 669 |
| NM_018474 | KIZ | 0.00 | 0.14 | 162 | 140 | 410 | 525 |
| NM_001023563 | ZNF805 | 0.00 | 0.14 | 433 | 464 | 655 | 813 |
| NM_001284280 | SMEK1 | 0.00 | 0.13 | 659 | 746 | 848 | 1037 |
| NM_194271 | RNF34 | 0.00 | 0.13 | 668 | 674 | 880 | 1074 |
| NM_203462 | MRFAP1 L1 | 0.00 | 0.13 | 934 | 953 | 1061 | 1283 |

(continued)

| Table 5B: Differetially Upregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_020345 | NKIRAS1 | -0.15 | 0.13 | 434 | 331 | 412 | 527 |
| NM_001282161 | UBE2A | 0.00 | 0.13 | 1929 | 1813 | 3342 | 3876 |
| NM_001008397 | GPX8 | 0.00 | 0.13 | 1677 | 1806 | 3392 | 3932 |
| NM_017436 | A4GALT | 0.00 | 0.13 | 737 | 782 | 815 | 998 |
| NM_199344 | SFT2D2 | 0.00 | 0.13 | 319 | 300 | 514 | 647 |
| NM_024663 | NPEPL1 | 0.00 | 0.13 | 606 | 672 | 1210 | 1453 |
| NM_019094 | NUDT4 | -0.12 | 0.13 | 1386 | 1162 | 1418 | 1691 |
| NM_015097 | CLASP2 | 0.01 | 0.13 | 1326 | 1458 | 1926 | 2270 |
| NM_003214 | TEAD3 | 0.00 | 0.13 | 1031 | 1096 | 1412 | 1684 |
| NM_001193552 | ZNF850 | 0.00 | 0.13 | 163 | 158 | 168 | 233 |
| NM_016333 | SRRM2 | 0.00 | 0.13 | 9495 | 9624 | 14510 | 16336 |
| NM_019001 | XRN1 | 0.00 | 0.13 | 929 | 969 | 1670 | 1978 |
| NM_144973 | DENND5 B | 0.00 | 0.13 | 499 | 508 | 936 | 1137 |
| NM_001093 | ACACB | 0.00 | 0.13 | 40 | 33 | 119 | 172 |
| NM_133372 | FNIP1 | 0.01 | 0.13 | 1223 | 1348 | 2267 | 2656 |
| NM_002907 | RECQL | 0.00 | 0.13 | 1670 | 1633 | 2067 | 2429 |
| NM_001084393 | DDTL | 0.00 | 0.13 | 285 | 320 | 630 | 782 |
| NM_173545 | APLF | 0.00 | 0.13 | 47 | 72 | 131 | 187 |
| NM_058230 | ZNF354B | 0.00 | 0.13 | 130 | 100 | 156 | 218 |
| NM_015534 | ZZZ3 | 0.00 | 0.13 | 946 | 990 | 1431 | 1704 |
| NM_001164000 | MECOM | 0.00 | 0.13 | 51 | 52 | 343 | 444 |
| NM_005482 | PIGK | 0.00 | 0.13 | 716 | 700 | 996 | 1205 |
| NM_024771 | NAA40 | 0.00 | 0.13 | 510 | 516 | 599 | 745 |
| NM_020368 | UTP3 | 0.00 | 0.13 | 656 | 696 | 921 | 1118 |
| NM_001244766 | MOB1B | 0.00 | 0.13 | 1371 | 1400 | 2075 | 2434 |
| NM_002027 | FNTA | -0.05 | 0.13 | 542 | 451 | 693 | 854 |
| NM_001120 | MFSD10 | 0.00 | 0.13 | 564 | 641 | 1229 | 1471 |
| NM_014061 | MAGEH1 | 0.00 | 0.13 | 612 | 622 | 320 | 416 |
| NM_001033568 | RHOT1 | 0.00 | 0.13 | 683 | 600 | 842 | 1026 |
| NM_006380 | APPBP2 | 0.00 | 0.13 | 837 | 872 | 1102 | 1325 |
| NM_003668 | MAPKAP K5 | 0.00 | 0.13 | 761 | 806 | 888 | 1079 |
| NM_004505 | USP6 | 0.00 | 0.13 | 51 | 77 | 60 | 96 |
| NM_199464 | KCNRG | -0.34 | 0.13 | 79 | 40 | 94 | 140 |
| NM_006773 | DDX18 | 0.00 | 0.13 | 643 | 711 | 703 | 865 |
| NM_001171606 | PREPL | 0.00 | 0.13 | 693 | 747 | 2439 | 2843 |
| NM_015172 | PRRC2C | 0.00 | 0.13 | 6142 | 6339 | 7967 | 9026 |
| NM_004228 | CYTH2 | 0.00 | 0.13 | 1182 | 1190 | 1639 | 1937 |

(continued)

| Table 5B: Differetially Upregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_006696 | BRD8 | 0.00 | 0.13 | 624 | 622 | 699 | 860 |
| NM_006946 | SPTBN2 | 0.00 | 0.13 | 31 | 31 | 121 | 174 |
| NM_004430 | EGR3 | 0.00 | 0.13 | 43 | 45 | 125 | 179 |
| NM_033632 | FBXW7 | 0.00 | 0.13 | 419 | 396 | 559 | 697 |
| NM_001127510 | APC | 0.00 | 0.13 | 1907 | 1873 | 3198 | 3695 |
| NM_020466 | LYRM2 | 0.00 | 0.13 | 543 | 490 | 957 | 1157 |
| NM_130809 | PRRC1 | 0.00 | 0.13 | 2388 | 2295 | 2672 | 3103 |
| NM_016100 | NAA20 | 0.00 | 0.13 | 904 | 896 | 1389 | 1651 |
| NM_032731 | TXNDC1 7 | 0.00 | 0.13 | 642 | 673 | 1637 | 1933 |
| NM_022087 | GALNT1 1 | 0.00 | 0.13 | 893 | 870 | 2258 | 2636 |
| NM_001281504 | ATG4D | 0.00 | 0.13 | 446 | 491 | 372 | 477 |
| NM_172002 | HSCB | 0.00 | 0.13 | 93 | 114 | 106 | 155 |
| NM_001281297 | EDF1 | 0.00 | 0.13 | 2922 | 2781 | 4586 | 5248 |
| NM_004229 | MED14 | 0.00 | 0.13 | 1548 | 1518 | 1949 | 2286 |
| NM_016480 | PAIP2 | 0.00 | 0.13 | 838 | 917 | 940 | 1137 |
| NM_001935 | DPP4 | -0.05 | 0.13 | 356 | 285 | 1689 | 1991 |
| NM_024907 | FBXO17 | -0.01 | 0.13 | 629 | 547 | 863 | 1048 |
| NM_153214 | FBLN7 | 0.00 | 0.13 | 201 | 195 | 268 | 353 |
| NM_178191 | ATPIF1 | -0.14 | 0.13 | 862 | 695 | 1832 | 2152 |
| NM_001252612 | ZNF638 | 0.00 | 0.13 | 1513 | 1475 | 2242 | 2615 |
| NM_015360 | SKIV2L2 | 0.00 | 0.13 | 1263 | 1362 | 2103 | 2458 |
| NM_018032 | LUC7L | 0.00 | 0.13 | 279 | 272 | 239 | 318 |
| NM_006282 | STK4 | 0.00 | 0.13 | 979 | 1040 | 1456 | 1725 |
| NM_001098512 | PRKG1 | 0.00 | 0.12 | 814 | 873 | 1096 | 1314 |
| NM_006743 | RBM3 | 0.00 | 0.12 | 3427 | 3529 | 4494 | 5137 |
| NM_001184889 | NIPA2 | 0.00 | 0.12 | 1191 | 1119 | 1840 | 2159 |
| NM_173664 | ARL10 | 0.00 | 0.12 | 289 | 310 | 451 | 569 |
| NM_004863 | SPTLC2 | 0.00 | 0.12 | 2945 | 3014 | 3901 | 4473 |
| NM_001349 | DARS | 0.00 | 0.12 | 1625 | 1589 | 2217 | 2584 |
| NM_175918 | CRIPAK | 0.00 | 0.12 | 73 | 50 | 123 | 176 |
| NM_000263 | NAGLU | 0.00 | 0.12 | 1008 | 1007 | 2051 | 2396 |
| NM_001039465 | SRSF5 | 0.00 | 0.12 | 2117 | 2168 | 3178 | 3662 |
| NM_015132 | SNX13 | 0.00 | 0.12 | 594 | 535 | 996 | 1198 |
| RefSeq ID | Gene Symbol | SR GFOLD | SEN GFOLD | SR IL-2 | SR IL-2 + | SEN IL-2 - | SEN IL-2 + |
| NM_014051 | TMEM14 A | 0.00 | 0.12 | 103 | 130 | 247 | 327 |
| NM_001166005 | EPB41 | 0.00 | 0.12 | 241 | 275 | 455 | 573 |
| NM_016406 | UFC1 | 0.00 | 0.12 | 716 | 735 | 1233 | 1468 |

(continued)

| RefSeq ID | Gene Symbol | SR GFOLD | SEN GFOLD | SR IL-2 | SR IL-2 + | SEN IL-2 - | SEN IL-2 + |
|---|---|---|---|---|---|---|---|
| NM_020429 | SMURF1 | 0.00 | 0.12 | 1834 | 1891 | 2084 | 2431 |
| NM_032711 | MAFG | 0.00 | 0.12 | 1779 | 1789 | 2007 | 2344 |
| NM_024836 | ZNF672 | 0.00 | 0.12 | 1110 | 1022 | 1063 | 1274 |
| NM_001287036 | KIAA195 8 | 0.00 | 0.12 | 96 | 109 | 89 | 133 |
| NM_005163 | AKT1 | 0.00 | 0.12 | 5878 | 5999 | 7884 | 8893 |
| NM_176812 | CHMP4B | 0.00 | 0.12 | 1274 | 1351 | 1824 | 2137 |
| NM_005897 | IPP | 0.00 | 0.12 | 192 | 163 | 433 | 547 |
| NM_004429 | EFNB1 | 0.00 | 0.12 | 115 | 151 | 289 | 377 |
| NM_001162371 | LOC7283 92 | -0.07 | 0.12 | 590 | 486 | 493 | 617 |
| NM_020817 | KIAA140 7 | 0.00 | 0.12 | 11 | 22 | 75 | 115 |
| NM_001286554 | USP49 | 0.00 | 0.12 | 128 | 120 | 154 | 214 |
| NM_021994 | ZNF277 | 0.00 | 0.12 | 155 | 124 | 214 | 287 |
| NM_001143909 | RPEL1 | 0.00 | 0.12 | 237 | 290 | 366 | 468 |
| NM_014014 | SNRNP20 0 | 0.01 | 0.12 | 6901 | 7213 | 8972 | 10089 |
| NM_015154 | MESDC2 | 0.00 | 0.12 | 1005 | 1034 | 1244 | 1479 |
| NM_006355 | TRIM38 | 0.00 | 0.12 | 659 | 646 | 1009 | 1211 |
| NM_015091 | FAM179B | 0.00 | 0.12 | 194 | 231 | 690 | 845 |
| NM_001005271 | CHD3 | -0.07 | 0.12 | 2412 | 2149 | 3502 | 4015 |
| NM_032256 | TMEM11 7 | 0.00 | 0.12 | 174 | 135 | 235 | 312 |
| NM_004344 | CETN2 | 0.00 | 0.12 | 365 | 364 | 753 | 917 |
| NM_001134415 | TBCCD1 | 0.00 | 0.12 | 264 | 247 | 341 | 438 |
| NM_001242797 | ZNF322 | 0.00 | 0.12 | 216 | 244 | 293 | 381 |
| NM_001281723 | BTD | -0.02 | 0.12 | 416 | 347 | 697 | 852 |
| NM_016424 | LUC7L3 | 0.00 | 0.12 | 1047 | 1079 | 1279 | 1516 |
| NM_001033561 | PHF12 | 0.00 | 0.12 | 839 | 880 | 1067 | 1275 |
| NM_004782 | SNAP29 | 0.00 | 0.12 | 907 | 882 | 1485 | 1749 |
| NM_025222 | WDR82 | 0.00 | 0.12 | 2483 | 2580 | 2656 | 3065 |
| NM_001105079 | FBRS | 0.00 | 0.12 | 740 | 824 | 794 | 963 |
| NM_152653 | UBE2E2 | 0.00 | 0.12 | 535 | 519 | 1025 | 1227 |
| NM_152316 | ARL14EP | 0.00 | 0.12 | 156 | 170 | 316 | 408 |
| NM_001040280 | CD83 | 0.00 | 0.12 | 19 | 27 | 69 | 107 |
| NM_002830 | PTPN4 | 0.00 | 0.12 | 191 | 197 | 264 | 346 |
| NM_016120 | RLIM | 0.00 | 0.12 | 2471 | 2611 | 3708 | 4235 |
| RefSeq ID | Gene Symbol | SR GFOLD | SEN GFOLD | SR IL-2 - | SR IL-2 + | SEN IL-2 - | SEN IL-2 + |
| NM_032590 | KDM2B | 0.00 | 0.12 | 654 | 604 | 620 | 762 |
| NM_024672 | THAP9 | 0.00 | 0.12 | 64 | 68 | 44 | 74 |
| NM_003458 | BSN | 0.00 | 0.12 | 45 | 30 | 44 | 74 |
| NM_005406 | ROCK1 | 0.00 | 0.12 | 2280 | 2326 | 2662 | 3068 |

(continued)

| RefSeq ID | Gene Symbol | SR GFOLD | SEN GFOLD | SR IL-2 - | SR IL-2 + | SEN IL-2 - | SEN IL-2 + |
|-----------|-------------|----------|-----------|-----------|-----------|------------|------------|
| NM_001441 | FAAH | -0.49 | 0.12 | 44 | 16 | 33 | 59 |
| NM_019059 | TOMM7 | 0.00 | 0.11 | 607 | 643 | 1146 | 1362 |
| NM_017520 | MPHOSP H8 | 0.00 | 0.11 | 279 | 296 | 443 | 556 |
| NM_006540 | NCOA2 | 0.00 | 0.11 | 511 | 583 | 1250 | 1479 |
| NM_001288588 | ZNF280D | 0.00 | 0.11 | 256 | 260 | 420 | 529 |
| NM_152551 | SNRNP48 | 0.00 | 0.11 | 206 | 254 | 252 | 331 |
| NM_130464 | NPIPB3 | -0.02 | 0.11 | 544 | 463 | 526 | 652 |
| NM_003430 | ZNF91 | 0.00 | 0.11 | 143 | 152 | 374 | 475 |
| NM_015542 | UPF2 | 0.00 | 0.11 | 672 | 700 | 988 | 1181 |
| NM_001099270 | ZBTB34 | 0.00 | 0.11 | 241 | 228 | 335 | 429 |
| NM_138363 | CEP95 | 0.00 | 0.11 | 164 | 203 | 335 | 429 |
| NM_005433 | YES 1 | 0.00 | 0.11 | 999 | 1078 | 1545 | 1810 |
| NM_001144950 | SSC5D | 0.00 | 0.11 | 798 | 871 | 1757 | 2048 |
| NM_001112808 | FPGT-TNNI3K | 0.00 | 0.11 | 63 | 43 | 112 | 161 |
| NM_017950 | CCDC40 | 0.00 | 0.11 | 15 | 25 | 67 | 104 |
| NM_003362 | UNG | 0.00 | 0.11 | 418 | 487 | 591 | 726 |
| NM_031208 | FAHD1 | 0.00 | 0.11 | 254 | 306 | 632 | 773 |
| NM_004267 | CHST2 | 0.00 | 0.11 | 285 | 281 | 293 | 379 |
| NM_001008727 | ZNF121 | 0.00 | 0.11 | 243 | 210 | 266 | 347 |
| NM_144600 | FOPNL | 0.00 | 0.11 | 364 | 424 | 593 | 728 |
| NM_024570 | RNASEH 2B | 0.00 | 0.11 | 190 | 218 | 245 | 322 |
| NM_031925 | TMEM12 0A | 0.00 | 0.11 | 739 | 728 | 1705 | 1987 |
| NM_153687 | IKBIP | 0.00 | 0.11 | 865 | 879 | 867 | 1041 |
| NM_001258373 | SPATA20 | 0.00 | 0.11 | 357 | 393 | 1092 | 1296 |
| NM_003400 | XPO1 | 0.00 | 0.11 | 2982 | 3093 | 3910 | 4438 |
| NM_003450 | ZNF174 | 0.00 | 0.11 | 207 | 242 | 358 | 455 |
| NM_016533 | NINJ2 | 0.00 | 0.11 | 55 | 49 | 139 | 194 |
| NM_031452 | FAM103 A1 | 0.00 | 0.11 | 97 | 129 | 148 | 205 |
| NM_001136534 | TMEM23 3 | 0.00 | 0.11 | 79 | 65 | 48 | 79 |
| NM_012098 | ANGPTL 2 | 0.00 | 0.11 | 1241 | 1362 | 4927 | 5560 |
| NM_177441 | TMUB2 | 0.00 | 0.11 | 756 | 699 | 1348 | 1584 |
| NM_003169 | SUPT5H | -0.01 | 0.11 | 2766 | 2574 | 4255 | 4815 |
| NM_005319 | HIST1H1 C | -0.11 | 0.11 | 15716 | 14224 | 8543 | 9530 |
| NM 015517 | HINFP | 0.00 | 0.11 | 226 | 268 | 385 | 486 |
| NM_147189 | FAM110B | 0.00 | 0.11 | 458 | 470 | 281 | 364 |
| NM_021033 | RAP2A | 0.00 | 0.11 | 901 | 935 | 1256 | 1479 |
| NM_001042358 | PTPN20B | 0.00 | 0.11 | 35 | 41 | 202 | 270 |
| NM_001242823 | C4B_2 | 0.00 | 0.11 | 131 | 105 | 127 | 179 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_007293 | C4A | 0.00 | 0.11 | 131 | 105 | 127 | 179 |
| NM_001002029 | C4B | 0.00 | 0.11 | 131 | 105 | 127 | 179 |
| NM_001031712 | TRMT11 | 0.00 | 0.11 | 164 | 157 | 168 | 229 |
| NM_001001419 | SMAD5 | 0.00 | 0.11 | 1677 | 1729 | 2317 | 2665 |
| NM_001286509 | SLC35B2 | 0.00 | 0.11 | 2909 | 2922 | 6280 | 7039 |
| NM_032679 | ZNF577 | 0.00 | 0.11 | 66 | 64 | 123 | 174 |
| NM_015087 | SPG20 | 0.00 | 0.11 | 1720 | 1677 | 2290 | 2634 |
| NM_004661 | CDC23 | 0.00 | 0.11 | 671 | 731 | 903 | 1079 |
| NM_021156 | TMX4 | 0.00 | 0.11 | 624 | 567 | 1514 | 1767 |
| NM_172089 | TNFSF12-TNFSF13 | 0.00 | 0.11 | 268 | 259 | 464 | 577 |
| NM_001199281 | CABIN1 | 0.00 | 0.11 | 1786 | 1919 | 2664 | 3048 |
| NM_001114137 | DMTN | 0.00 | 0.11 | 28 | 38 | 83 | 124 |
| NM_002613 | PDPK1 | 0.00 | 0.11 | 1497 | 1525 | 2739 | 3131 |
| NM_021216 | ZNF71 | 0.00 | 0.11 | 271 | 322 | 510 | 630 |
| NM_182565 | UBALD2 | 0.00 | 0.11 | 431 | 394 | 570 | 699 |
| NM_003737 | DCHS1 | 0.00 | 0.10 | 116 | 134 | 179 | 242 |
| NM_032287 | LDOC1L | -0.23 | 0.10 | 2009 | 1583 | 2078 | 2396 |
| NM_032472 | PPIL3 | 0.00 | 0.10 | 145 | 165 | 241 | 316 |
| NM_001334 | CTSO | 0.01 | 0.10 | 222 | 275 | 499 | 617 |
| NM_000108 | DLD | -0.05 | 0.10 | 943 | 817 | 1370 | 1604 |
| NM_012239 | SIRT3 | 0.00 | 0.10 | 332 | 281 | 474 | 588 |
| NM_014467 | SRPX2 | 0.00 | 0.10 | 694 | 726 | 2227 | 2560 |
| NM_001032372 | ZNF226 | 0.00 | 0.10 | 162 | 187 | 356 | 451 |
| NM_015659 | RSL1D1 | 0.00 | 0.10 | 2078 | 2209 | 2951 | 3362 |
| NM_001172687 | GGA1 | 0.00 | 0.10 | 1312 | 1362 | 2063 | 2377 |
| NM_014255 | CNPY2 | -0.15 | 0.10 | 867 | 696 | 1680 | 1950 |
| NM_152622 | MIER3 | 0.00 | 0.10 | 293 | 353 | 412 | 516 |
| NM_001031617 | COX19 | 0.00 | 0.10 | 326 | 345 | 339 | 431 |
| NM_001012732 | DCTD | 0.00 | 0.10 | 2432 | 2439 | 3498 | 3965 |
| NM_175872 | ZNF792 | 0.00 | 0.10 | 49 | 51 | 152 | 209 |
| NM_001160094 | ACOT13 | -0.01 | 0.10 | 307 | 250 | 705 | 852 |
| NM_032024 | C10orf11 | 0.00 | 0.10 | 23 | 29 | 181 | 244 |
| NM_006980 | MTERF1 | 0.00 | 0.10 | 86 | 103 | 125 | 176 |
| NM_024646 | ZYG11B | 0.00 | 0.10 | 1428 | 1508 | 2161 | 2484 |
| NM_016021 | UBE2J1 | 0.00 | 0.10 | 2773 | 2600 | 2450 | 2804 |
| NM_018229 | AP5M1 | 0.00 | 0.10 | 569 | 609 | 971 | 1153 |
| NM_020121 | UGGT2 | 0.00 | 0.10 | 590 | 629 | 917 | 1092 |
| NM_017940 | NBPF1 | 0.00 | 0.10 | 475 | 507 | 774 | 930 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_173500 | TTBK2 | 0.00 | 0.10 | 340 | 394 | 422 | 527 |
| NM_001272004 | EPC1 | 0.00 | 0.10 | 339 | 390 | 534 | 656 |
| NM_001282640 | SUSD1 | 0.00 | 0.10 | 879 | 820 | 1774 | 2052 |
| NM_001919 | ECI1 | 0.00 | 0.10 | 320 | 332 | 555 | 680 |
| NM_013354 | CNOT7 | 0.00 | 0.10 | 560 | 642 | 734 | 884 |
| NM_032023 | RASSF4 | 0.00 | 0.10 | 114 | 126 | 158 | 216 |
| NM_174869 | IDH3G | 0.00 | 0.10 | 1635 | 1657 | 3182 | 3610 |
| NM_001271977 | STK25 | -0.01 | 0.10 | 3098 | 2887 | 5840 | 6529 |
| NM_018313 | PBRM1 | 0.00 | 0.10 | 1869 | 1852 | 2600 | 2967 |
| NM_153045 | C9orf91 | -0.05 | 0.10 | 244 | 188 | 362 | 457 |
| NM_017654 | SAMD9 | 0.00 | 0.10 | 433 | 421 | 693 | 837 |
| NM_002015 | FOXO1 | 0.00 | 0.10 | 140 | 106 | 297 | 381 |
| NM_002067 | GNA11 | 0.00 | 0.10 | 3554 | 3352 | 4053 | 4566 |
| NM_016951 | CKLF | 0.00 | 0.10 | 368 | 324 | 703 | 848 |
| NM_017785 | SPDL1 | 0.00 | 0.10 | 639 | 588 | 824 | 985 |
| NM_001127649 | PEX26 | 0.00 | 0.10 | 786 | 851 | 1697 | 1963 |
| NM_004775 | B4GALT6 | 0.00 | 0.10 | 257 | 228 | 605 | 736 |
| NM_001201338 | SAFB | -0.03 | 0.10 | 565 | 479 | 605 | 736 |
| NM_006544 | EXOC5 | 0.00 | 0.10 | 1086 | 1133 | 1878 | 2164 |
| NM_003679 | KMO | 0.00 | 0.10 | 120 | 133 | 89 | 131 |
| NM_001172679 | ZNF764 | 0.00 | 0.10 | 60 | 79 | 89 | 131 |
| NM_005851 | CDK2AP 2 | 0.00 | 0.10 | 411 | 476 | 495 | 610 |
| NM_001310 | CREBL2 | 0.00 | 0.10 | 547 | 565 | 1231 | 1442 |
| NM_001289824 | FURIN | 0.00 | 0.10 | 4833 | 4901 | 7499 | 8329 |
| NM_001099666 | PTAR1 | 0.00 | 0.10 | 1272 | 1214 | 2258 | 2584 |
| NM_001129993 | KIAA184 1 | 0.00 | 0.10 | 145 | 171 | 324 | 412 |
| NM_001289166 | ZNF227 | 0.00 | 0.10 | 177 | 177 | 277 | 357 |
| NM_020320 | RARS2 | 0.00 | 0.10 | 609 | 644 | 1269 | 1484 |
| NM_002814 | PSMD10 | 0.00 | 0.10 | 382 | 406 | 638 | 773 |
| NM_032683 | MPV17L2 | 0.00 | 0.10 | 570 | 560 | 576 | 702 |
| NM_000454 | SOD1 | 0.00 | 0.10 | 2838 | 2726 | 5251 | 5869 |
| NM_006377 | UNC13B | 0.00 | 0.10 | 1028 | 1123 | 2876 | 3264 |
| NM_024909 | ATAT1 | 0.00 | 0.10 | 96 | 123 | 137 | 190 |
| NM_001195150 | LOC1001 30705 | 0.00 | 0.10 | 70 | 89 | 27 | 50 |
| NM_017619 | RNPC3 | 0.00 | 0.10 | 120 | 132 | 191 | 255 |
| NM_015852 | ZNF117 | -0.24 | 0.10 | 106 | 62 | 191 | 255 |
| NM_001080821 | ZNF799 | 0.00 | 0.10 | 56 | 54 | 94 | 137 |
| NM_177476 | LYNX1 | -0.12 | 0.10 | 178 | 125 | 616 | 747 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_032718 | MFSD9 | -0.21 | 0.10 | 127 | 79 | 133 | 185 |
| NM_213590 | TRIM 13 | 0.00 | 0.10 | 394 | 406 | 668 | 806 |
| NM_025185 | TANC2 | 0.00 | 0.10 | 2352 | 2463 | 5332 | 5952 |
| NM_015960 | CUTC | 0.00 | 0.10 | 249 | 250 | 304 | 388 |
| NM_182498 | ZNF428 | 0.00 | 0.10 | 251 | 267 | 385 | 482 |
| NM_002513 | NME3 | 0.00 | 0.09 | 231 | 219 | 512 | 628 |
| NM_001078177 | SLC29A1 | 0.00 | 0.09 | 609 | 611 | 693 | 834 |
| NM_001002878 | THOC5 | 0.00 | 0.09 | 1187 | 1293 | 1857 | 2135 |
| NM_153812 | PHF13 | 0.00 | 0.09 | 968 | 963 | 975 | 1152 |
| NM_001177387 | ATXN7 | 0.00 | 0.09 | 814 | 895 | 1067 | 1255 |
| NM_016006 | ABHD5 | 0.00 | 0.09 | 854 | 947 | 1279 | 1492 |
| NM_015694 | ZNF777 | 0.00 | 0.09 | 637 | 652 | 665 | 802 |
| NM_006285 | TESK1 | 0.00 | 0.09 | 1056 | 1139 | 1360 | 1582 |
| NM_002016 | FLG | 0.00 | 0.09 | 128 | 124 | 312 | 397 |
| NM_001918 | DBT | 0.00 | 0.09 | 326 | 329 | 586 | 712 |
| NM_005188 | CBL | 0.00 | 0.09 | 1941 | 1829 | 2365 | 2695 |
| NM_000033 | ABCD1 | 0.00 | 0.09 | 463 | 471 | 713 | 856 |
| NM_002079 | GOT1 | 0.00 | 0.09 | 702 | 781 | 1031 | 1214 |
| NM_014988 | LIMCH1 | 0.00 | 0.09 | 966 | 1050 | 2728 | 3094 |
| NM_014880 | CD302 | -0.28 | 0.09 | 175 | 109 | 173 | 233 |
| NM_001013620 | ALG10B | 0.01 | 0.09 | 93 | 128 | 79 | 118 |
| NM_020884 | MYH7B | 0.00 | 0.09 | 98 | 69 | 79 | 118 |
| NM_022166 | XYLT1 | 0.00 | 0.09 | 5763 | 5923 | 4407 | 4933 |
| NM_024309 | TNIP2 | 0.00 | 0.09 | 742 | 792 | 905 | 1072 |
| NM_199337 | TMEM17 9B | 0.00 | 0.09 | 645 | 715 | 1285 | 1497 |
| NM_018316 | KLHL26 | 0.00 | 0.09 | 197 | 199 | 239 | 311 |
| NM_001012301 | ARSI | 0.00 | 0.09 | 616 | 629 | 2015 | 2305 |
| NM_003502 | AXIN1 | 0.00 | 0.09 | 559 | 503 | 676 | 813 |
| NM_001040454 | SLC26A6 | 0.00 | 0.09 | 326 | 372 | 480 | 590 |
| NM_006623 | PHGDH | -0.01 | 0.09 | 2003 | 1851 | 1942 | 2224 |
| NM_003196 | TCEA3 | -0.27 | 0.09 | 267 | 177 | 724 | 867 |
| NM_001197026 | PLEKHA 8 | 0.00 | 0.09 | 330 | 334 | 281 | 360 |
| NM_014932 | NLGN1 | 0.00 | 0.09 | 207 | 193 | 229 | 299 |
| NM_016396 | CTDSPL2 | 0.00 | 0.09 | 441 | 434 | 401 | 499 |
| NM_153713 | LIX1L | 0.00 | 0.09 | 1588 | 1535 | 1512 | 1747 |
| NM_001567 | INPPL1 | 0.00 | 0.09 | 3955 | 4079 | 7447 | 8229 |
| NM_005994 | TBX2 | 0.00 | 0.09 | 856 | 922 | 1150 | 1344 |
| NM_001007531 | NKAPL | 0.00 | 0.09 | 124 | 102 | 127 | 177 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_182476 | COQ6 | 0.00 | 0.09 | 326 | 326 | 715 | 856 |
| NM_032510 | PARD6G | 0.00 | 0.09 | 8 | 14 | 146 | 200 |
| NM_006721 | ADK | 0.00 | 0.09 | 724 | 726 | 1129 | 1320 |
| NM_003432 | ZNF131 | 0.00 | 0.09 | 466 | 510 | 493 | 604 |
| NM_015295 | SMCHD1 | 0.00 | 0.09 | 928 | 1012 | 1229 | 1431 |
| NM_005223 | DNASE1 | 0.00 | 0.09 | 105 | 124 | 214 | 281 |
| NM_006875 | PIM2 | 0.00 | 0.09 | 252 | 248 | 214 | 281 |
| NM_016373 | WWOX | -0.01 | 0.09 | 59 | 36 | 137 | 189 |
| NM_001170580 | HHAT | 0.00 | 0.09 | 55 | 47 | 123 | 172 |
| NM_152705 | POLR1D | 0.00 | 0.09 | 431 | 461 | 778 | 926 |
| NM_024818 | UBA5 | 0.00 | 0.09 | 369 | 409 | 512 | 625 |
| NM_015878 | AZIN1 | 0.00 | 0.09 | 2620 | 2604 | 3045 | 3429 |
| NM_002973 | ATXN2 | -0.13 | 0.09 | 2071 | 1760 | 1882 | 2152 |
| NM_015503 | SH2B1 | -0.24 | 0.09 | 1035 | 783 | 975 | 1146 |
| NM_030798 | WBSCR1 6 | 0.00 | 0.09 | 853 | 886 | 1106 | 1292 |
| NM_004278 | PIGL | 0.00 | 0.09 | 108 | 139 | 189 | 251 |
| NM_024544 | MUL1 | 0.00 | 0.09 | 1013 | 1057 | 1749 | 2004 |
| NM_012255 | XRN2 | 0.00 | 0.09 | 2056 | 2106 | 2970 | 3344 |
| NM_017719 | SNRK | 0.00 | 0.09 | 696 | 674 | 861 | 1018 |
| NM_022915 | MRPL44 | 0.00 | 0.09 | 519 | 523 | 599 | 723 |
| NM_001028 | RPS25 | 0.00 | 0.09 | 2882 | 2849 | 3858 | 4312 |
| NM_002730 | PRKACA | -0.03 | 0.09 | 1849 | 1675 | 2647 | 2989 |
| NM_018992 | KCTD5 | 0.00 | 0.09 | 1341 | 1336 | 1581 | 1817 |
| NM_014007 | ZBTB43 | 0.00 | 0.08 | 585 | 606 | 819 | 970 |
| NM_001287757 | DCUN1D 4 | 0.00 | 0.08 | 473 | 435 | 699 | 835 |
| NM_001163280 | HTATSF1 | 0.00 | 0.08 | 730 | 719 | 1071 | 1251 |
| NM_004225 | MFHAS1 | 0.00 | 0.08 | 646 | 657 | 774 | 919 |
| NM_001003801 | SMARCD 3 | 0.00 | 0.08 | 492 | 505 | 934 | 1098 |
| NM_153267 | MAMDC 2 | 0.00 | 0.08 | 445 | 494 | 466 | 571 |
| NM_022913 | GPBP1 | 0.00 | 0.08 | 917 | 956 | 919 | 1081 |
| NM_001005407 | CACNA1 H | 0.00 | 0.08 | 60 | 70 | 23 | 44 |
| NM_006357 | UBE2E3 | 0.00 | 0.08 | 240 | 270 | 453 | 556 |
| NM_001204055 | NDUFC2 | 0.00 | 0.08 | 1151 | 1130 | 2265 | 2566 |
| NM_006468 | POLR3C | 0.00 | 0.08 | 790 | 820 | 1329 | 1536 |
| NM_001278346 | PHYKPL | 0.00 | 0.08 | 444 | 509 | 763 | 906 |
| NM_004337 | OSGIN2 | -0.32 | 0.08 | 827 | 586 | 1048 | 1224 |
| NM_139126 | PPIL4 | 0.00 | 0.08 | 286 | 330 | 462 | 566 |
| NM_003631 | PARG | 0.00 | 0.08 | 387 | 402 | 605 | 728 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_206962 | PRMT2 | 0.00 | 0.08 | 1832 | 1969 | 3754 | 4188 |
| NM_001040450 | FAM63B | 0.01 | 0.08 | 252 | 309 | 545 | 660 |
| NM_181783 | TMTC3 | 0.00 | 0.08 | 995 | 972 | 1978 | 2249 |
| NM_001025248 | DUT | 0.00 | 0.08 | 298 | 274 | 347 | 434 |
| NM_181742 | ORC4 | 0.00 | 0.08 | 264 | 297 | 347 | 434 |
| NM_181808 | POLN | 0.00 | 0.08 | 14 | 17 | 67 | 102 |
| NM_001282515 | ZFP37 | 0.00 | 0.08 | 39 | 44 | 67 | 102 |
| NM_001031689 | PLAA | 0.00 | 0.08 | 976 | 960 | 1483 | 1704 |
| NM_152345 | ANKRD1 3B | 0.00 | 0.08 | 121 | 88 | 104 | 148 |
| NM_001455 | FOXO3 | 0.00 | 0.08 | 2006 | 1908 | 1990 | 2261 |
| NM_173632 | ZNF776 | 0.00 | 0.08 | 250 | 281 | 474 | 579 |
| NM_000070 | CAPN3 | 0.00 | 0.08 | 34 | 39 | 83 | 122 |
| NM_001271 | CHD2 | 0.00 | 0.08 | 2018 | 1946 | 3288 | 3675 |
| NM_022350 | ERAP2 | 0.00 | 0.08 | 792 | 817 | 1470 | 1688 |
| NM_178833 | SLC9B2 | 0.00 | 0.08 | 478 | 472 | 980 | 1146 |
| NM_018367 | ACER3 | -0.10 | 0.08 | 313 | 239 | 364 | 453 |
| NM_023008 | KRI1 | 0.00 | 0.08 | 347 | 288 | 351 | 438 |
| NM_138418 | FAM195 A | 0.00 | 0.08 | 102 | 92 | 133 | 183 |
| NM_001082537 | TCTN1 | 0.00 | 0.08 | 133 | 160 | 372 | 462 |
| NM_021012 | KCNJ12 | 0.00 | 0.08 | 59 | 83 | 148 | 201 |
| NM_022165 | LIN7B | -0.05 | 0.08 | 141 | 101 | 225 | 292 |
| NM_018051 | WDR60 | 0.00 | 0.08 | 127 | 105 | 231 | 299 |
| NM_018086 | FIGN | 0.00 | 0.08 | 157 | 144 | 37 | 63 |
| NM_004822 | NTN1 | -0.96 | 0.08 | 67 | 19 | 6 | 22 |
| NM_021627 | SENP2 | 0.00 | 0.08 | 705 | 754 | 1154 | 1338 |
| NM_001082968 | TOM1L2 | 0.00 | 0.08 | 2030 | 2097 | 4438 | 4917 |
| NM_016538 | SIRT7 | 0.00 | 0.08 | 362 | 377 | 489 | 595 |
| NM_014974 | DIP2C | 0.00 | 0.08 | 2396 | 2336 | 4465 | 4946 |
| NM_001057 | TACR2 | 0.00 | 0.08 | 40 | 41 | 64 | 98 |
| NM_005027 | PIK3R2 | 0.00 | 0.08 | 1580 | 1600 | 3236 | 3614 |
| NM_001080495 | TNRC18 | 0.00 | 0.08 | 1905 | 2037 | 2398 | 2702 |
| NM_152279 | ZNF585B | 0.00 | 0.08 | 270 | 319 | 665 | 793 |
| NM_001037165 | FOXK1 | 0.00 | 0.08 | 2187 | 2156 | 3282 | 3662 |
| NM_207012 | AP3M1 | 0.01 | 0.08 | 1133 | 1253 | 1882 | 2137 |
| NM_001204193 | SRP19 | 0.00 | 0.08 | 286 | 325 | 270 | 344 |
| NM_022126 | LHPP | 0.00 | 0.08 | 289 | 324 | 693 | 824 |
| NM_001292018 | CYTH1 | 0.00 | 0.08 | 397 | 418 | 532 | 643 |
| NM_001856 | COL16A1 | -0.12 | 0.08 | 3354 | 2903 | 3032 | 3388 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_014296 | CAPN7 | 0.00 | 0.08 | 518 | 549 | 757 | 895 |
| NM_006693 | CPSF4 | 0.00 | 0.08 | 659 | 723 | 836 | 983 |
| NM_012115 | CASP8AP 2 | 0.00 | 0.08 | 418 | 476 | 422 | 518 |
| NM_032936 | TMEM60 | 0.00 | 0.08 | 231 | 213 | 331 | 414 |
| NM_012302 | LPHN2 | 0.00 | 0.08 | 1798 | 1775 | 1653 | 1884 |
| NM_001114394 | PAPD4 | 0.00 | 0.08 | 588 | 642 | 1038 | 1207 |
| NM_000068 | CACNA1 A | 0.00 | 0.08 | 94 | 102 | 318 | 399 |
| NM_001080425 | BEX4 | 0.00 | 0.08 | 156 | 165 | 229 | 296 |
| NM_001142300 | CCNYL1 | 0.00 | 0.08 | 401 | 403 | 453 | 553 |
| NM_032291 | SGIP1 | 0.00 | 0.08 | 162 | 199 | 618 | 739 |
| NM_012154 | AGO2 | 0.00 | 0.07 | 1586 | 1721 | 1537 | 1756 |
| NM_015885 | PCF11 | 0.00 | 0.07 | 910 | 862 | 799 | 941 |
| NM_015617 | PYGO1 | 0.00 | 0.07 | 255 | 275 | 333 | 416 |
| NM_001173425 | DFNB31 | 0.00 | 0.07 | 150 | 193 | 341 | 425 |
| NM_014939 | TRAPPC8 | 0.00 | 0.07 | 705 | 795 | 1375 | 1577 |
| NM_007245 | ATXN2L | 0.00 | 0.07 | 2178 | 2072 | 1709 | 1943 |
| NM_199203 | TMEM18 9-UBE2V1 | 0.00 | 0.07 | 3016 | 3143 | 5326 | 5858 |
| NM_001284267 | EFCAB 11 | 0.00 | 0.07 | 46 | 58 | 69 | 104 |
| NM_015401 | HDAC7 | 0.00 | 0.07 | 3460 | 3495 | 3286 | 3656 |
| NM_001282291 | ABCB8 | 0.00 | 0.07 | 475 | 545 | 836 | 981 |
| NM_033449 | FCHSD1 | 0.00 | 0.07 | 847 | 940 | 1585 | 1806 |
| NM_006135 | CAPZA1 | 0.00 | 0.07 | 2616 | 2781 | 5089 | 5599 |
| NM_030624 | KLHL15 | 0.00 | 0.07 | 342 | 351 | 316 | 396 |
| NM_001146110 | MIER1 | 0.00 | 0.07 | 916 | 845 | 1287 | 1479 |
| NM_004468 | FHL3 | 0.00 | 0.07 | 487 | 561 | 705 | 835 |
| NM_001288767 | ARMC5 | 0.00 | 0.07 | 690 | 668 | 416 | 510 |
| NM_001257964 | BOD1L2 | 0.00 | 0.07 | 66 | 88 | 108 | 152 |
| NM_134442 | CREB1 | 0.00 | 0.07 | 741 | 806 | 1135 | 1311 |
| NM_173829 | SREK1IP 1 | 0.00 | 0.07 | 323 | 315 | 551 | 662 |
| NM_024097 | C1orf50 | -0.31 | 0.07 | 118 | 67 | 123 | 170 |
| NM_002657 | PLAGL2 | 0.00 | 0.07 | 451 | 444 | 391 | 481 |
| NM_004491 | ARHGAP 35 | 0.01 | 0.07 | 4907 | 5163 | 7435 | 8103 |
| NM_004215 | EBAG9 | -0.05 | 0.07 | 216 | 164 | 223 | 288 |
| NM_001695 | ATP6V1C 1 | 0.00 | 0.07 | 1161 | 1178 | 2078 | 2340 |
| NM_173474 | NTAN1 | -0.16 | 0.07 | 590 | 457 | 1092 | 1262 |
| NM_007041 | ATE1 | 0.00 | 0.07 | 462 | 468 | 755 | 889 |
| NM_001271592 | LOC1001 29361 | 0.00 | 0.07 | 442 | 396 | 659 | 782 |
| NM_001193511 | MAP3K1 2 | 0.00 | 0.07 | 512 | 475 | 932 | 1085 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_181708 | BCDIN3D | 0.00 | 0.07 | 27 | 47 | 139 | 189 |
| NM_001143936 | C15orf61 | 0.00 | 0.07 | 132 | 168 | 119 | 165 |
| NM_007371 | BRD3 | 0.00 | 0.07 | 934 | 902 | 1102 | 1272 |
| NM_000722 | CACNA2 D1 | 0.00 | 0.07 | 466 | 460 | 826 | 967 |
| NM_001099670 | C8orf59 | -0.15 | 0.07 | 261 | 189 | 195 | 255 |
| NM_001145029 | ANKRD3 0B | 0.00 | 0.07 | 299 | 296 | 195 | 255 |
| NM_001008388 | CISD2 | 0.00 | 0.07 | 439 | 433 | 613 | 730 |
| NM_001194937 | POGZ | 0.00 | 0.07 | 1501 | 1464 | 1816 | 2052 |
| NM_001135243 | TCOF1 | 0.00 | 0.07 | 1369 | 1413 | 1306 | 1495 |
| NM_152720 | NEK3 | 0.00 | 0.07 | 111 | 119 | 156 | 209 |
| NM_178502 | DTX3 | 0.00 | 0.07 | 333 | 379 | 730 | 860 |
| NM_001145641 | SRRM5 | 0.00 | 0.07 | 150 | 158 | 214 | 277 |
| NM_000635 | RFX2 | 0.00 | 0.07 | 334 | 286 | 485 | 586 |
| NM_020706 | SCAF4 | 0.00 | 0.07 | 1118 | 1218 | 1098 | 1266 |
| NM_032970 | SEC22C | 0.00 | 0.07 | 1010 | 1034 | 2163 | 2427 |
| NM_152424 | AMER1 | 0.00 | 0.07 | 139 | 161 | 272 | 344 |
| NM_007130 | ZNF41 | 0.00 | 0.07 | 160 | 196 | 306 | 383 |
| NM_001039887 | PROSER3 | 0.00 | 0.07 | 228 | 207 | 327 | 407 |
| NM_001100874 | SLC9B1 | 0.00 | 0.07 | 51 | 34 | 79 | 116 |
| NM_021145 | DMTF1 | 0.00 | 0.07 | 620 | 689 | 1021 | 1181 |
| NM_000341 | SLC3A1 | -0.09 | 0.07 | 149 | 104 | 335 | 416 |
| NM_006267 | RANBP2 | -0.26 | 0.07 | 1632 | 1249 | 3059 | 3394 |
| NM_000552 | VWF | 0.00 | 0.07 | 52 | 54 | 75 | 111 |
| NM_006642 | SDCCAG 8 | 0.00 | 0.07 | 749 | 690 | 1553 | 1763 |
| **Table 5B: Differentially Upregulated Genes in SEN + IL-2** | | | | | | | |
| **RefSeq ID** | **Gene Symbol** | **SR GFOLD** | **SEN GFOLD** | **SR IL-2 -** | **SR IL-2 +** | **SEN IL-2 -** | **SEN IL-2 +** |
| NM_020825 | CRAMP1 L | -0.04 | 0.07 | 836 | 724 | 859 | 1002 |
| NM_006349 | ZNHIT1 | 0.00 | 0.07 | 1287 | 1221 | 2903 | 3225 |
| NM_032172 | USP42 | 0.00 | 0.07 | 397 | 358 | 322 | 401 |
| NM_001099678 | LRRC58 | 0.00 | 0.07 | 1075 | 1083 | 1452 | 1652 |
| NM_033081 | DIDO1 | 0.00 | 0.07 | 1338 | 1389 | 2073 | 2327 |
| NM_003769 | SRSF9 | -0.07 | 0.07 | 1723 | 1509 | 2157 | 2418 |
| NM_025194 | ITPKC | 0.00 | 0.07 | 511 | 495 | 826 | 965 |
| NM_001098787 | BET1L | 0.00 | 0.07 | 2029 | 2169 | 2335 | 2610 |
| NM_017686 | GDAP2 | 0.00 | 0.06 | 421 | 454 | 955 | 1107 |
| NM_005321 | HIST1H1 E | 0.00 | 0.06 | 7602 | 7405 | 2945 | 3268 |
| NM_001098638 | RNF169 | 0.00 | 0.06 | 1399 | 1502 | 1732 | 1956 |
| NM_001136036 | ZNF692 | 0.00 | 0.06 | 279 | 322 | 324 | 403 |

(continued)

| Table 5B: Differentially Upregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_145728 | SYNM | 0.00 | 0.06 | 391 | 367 | 414 | 505 |
| NM_020676 | ABHD6 | 0.00 | 0.06 | 305 | 277 | 422 | 514 |
| NM_001007561 | IRGQ | 0.00 | 0.06 | 1049 | 1044 | 1425 | 1621 |
| NM_001260 | CDK8 | 0.00 | 0.06 | 291 | 345 | 447 | 542 |
| NM_005791 | MPHOSP H10 | 0.00 | 0.06 | 326 | 308 | 447 | 542 |
| NM_016271 | RNF138 | 0.00 | 0.06 | 160 | 139 | 98 | 139 |
| NM_015045 | WAPAL | 0.00 | 0.06 | 1428 | 1516 | 1747 | 1970 |
| NM_138446 | MALSU1 | 0.00 | 0.06 | 395 | 378 | 672 | 793 |
| NM_014946 | SPAST | 0.00 | 0.06 | 336 | 381 | 426 | 518 |
| NM_001285439 | RICTOR | -0.03 | 0.06 | 878 | 765 | 1354 | 1542 |
| NM_194259 | UBE2I | 0.00 | 0.06 | 2177 | 2178 | 3134 | 3466 |
| NM_024045 | DDX50 | 0.00 | 0.06 | 678 | 635 | 788 | 921 |
| NM_020993 | BCL7A | 0.00 | 0.06 | 1682 | 1655 | 761 | 891 |
| NM_014313 | TMEM50 A | 0.00 | 0.06 | 3446 | 3481 | 4777 | 5224 |
| NM_005389 | PCMT1 | 0.01 | 0.06 | 1977 | 2137 | 3136 | 3466 |
| NM_012430 | SEC22A | 0.00 | 0.06 | 431 | 416 | 520 | 623 |
| NM_024899 | CEP76 | 0.00 | 0.06 | 180 | 195 | 150 | 201 |
| NM_001514 | GTF2B | 0.00 | 0.06 | 348 | 391 | 497 | 597 |
| NM_024697 | ZNF385D | 0.00 | 0.06 | 32 | 39 | 81 | 118 |
| NM_001288781 | TTC8 | 0.00 | 0.06 | 258 | 273 | 722 | 847 |
| NM_001135993 | TTC39C | -0.12 | 0.06 | 261 | 193 | 441 | 534 |
| NM_144703 | LSM14B | 0.00 | 0.06 | 1155 | 1152 | 1545 | 1747 |
| NM_020137 | GRIPAP1 | 0.00 | 0.06 | 548 | 610 | 821 | 956 |
| NM_032830 | CIRH1A | 0.00 | 0.06 | 1287 | 1338 | 1736 | 1954 |
| NM_014663 | KDM4A | -0.07 | 0.06 | 553 | 454 | 774 | 904 |
| NM_001005408 | NEURL4 | 0.01 | 0.06 | 498 | 577 | 624 | 738 |
| NM_173611 | FAM98B | 0.00 | 0.06 | 581 | 547 | 599 | 710 |
| NM_000410 | HFE | 0.00 | 0.06 | 189 | 168 | 420 | 510 |
| NM_032352 | BRMS1L | 0.00 | 0.06 | 105 | 130 | 193 | 251 |
| NM_030755 | TMX1 | 0.00 | 0.06 | 561 | 619 | 884 | 1024 |
| NM_001282231 | ARMCX2 | 0.00 | 0.06 | 672 | 751 | 1612 | 1817 |
| NM_006287 | TFPI | -0.01 | 0.06 | 2382 | 2212 | 2884 | 3187 |
| NM_144597 | C 15orf40 | 0.00 | 0.06 | 118 | 149 | 206 | 266 |
| NM_172003 | CBWD2 | 0.00 | 0.06 | 255 | 280 | 324 | 401 |
| NM_212550 | BLOC1S3 | 0.00 | 0.06 | 488 | 500 | 362 | 444 |
| NM_018368 | LMBRD1 | 0.00 | 0.06 | 97 | 108 | 370 | 453 |
| NM_006785 | MALT1 | 0.00 | 0.06 | 1114 | 1102 | 1352 | 1534 |

(continued)

| Table 5B: Differentially Upregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_001620 | AHNAK | -0.01 | 0.06 | 110432 | 108601 | 198713 | 208233 |
| NM_001253875 | UXS1 | 0.00 | 0.06 | 889 | 978 | 1811 | 2031 |
| NM_001270485 | CAMKK2 | 0.00 | 0.06 | 1229 | 1305 | 2473 | 2743 |
| NM_005230 | ELK3 | 0.00 | 0.06 | 2347 | 2403 | 3400 | 3736 |
| NM_018053 | XKR8 | 0.01 | 0.06 | 515 | 595 | 593 | 702 |
| NM_001288563 | WDR63 | 0.00 | 0.06 | 19 | 10 | 121 | 166 |
| NM_001745 | CAMLG | 0.00 | 0.06 | 521 | 559 | 1069 | 1225 |
| NM_014859 | ARHGAP 44 | 0.00 | 0.06 | 85 | 69 | 25 | 46 |
| NM_000322 | PRPH2 | 0.00 | 0.06 | 65 | 58 | 25 | 46 |
| NM_001195156 | ZMYM6N B | -0.27 | 0.06 | 587 | 419 | 975 | 1122 |
| NM_144607 | CYB5D1 | 0.00 | 0.06 | 176 | 211 | 472 | 567 |
| NM_001270401 | RXRB | 0.00 | 0.06 | 1084 | 1147 | 1564 | 1762 |
| NM_018989 | RBM27 | 0.00 | 0.06 | 738 | 774 | 1079 | 1235 |
| NM_024509 | LRFN3 | 0.00 | 0.05 | 174 | 152 | 250 | 316 |
| NM_007187 | WBP4 | 0.00 | 0.05 | 394 | 347 | 460 | 553 |
| NM_001076786 | QSER1 | -0.06 | 0.05 | 821 | 700 | 873 | 1009 |
| NM_013450 | BAZ2B | -0.12 | 0.05 | 532 | 421 | 730 | 852 |
| NM_153638 | PANK2 | 0.00 | 0.05 | 499 | 576 | 853 | 987 |
| NM_002380 | MATN2 | -0.33 | 0.05 | 528 | 359 | 643 | 756 |
| NM_020998 | MST1 | 0.00 | 0.05 | 67 | 57 | 79 | 115 |
| NM_004760 | STK17A | 0.00 | 0.05 | 827 | 912 | 1749 | 1959 |
| NM_003927 | MBD2 | 0.00 | 0.05 | 1286 | 1278 | 1510 | 1701 |
| NM_001037172 | PXYLP1 | 0.00 | 0.05 | 82 | 93 | 218 | 279 |
| NM_001142648 | SARIA | 0.00 | 0.05 | 3206 | 3378 | 5432 | 5887 |
| NM_003847 | PEX11A | 0.00 | 0.05 | 57 | 52 | 123 | 168 |
| NM_152341 | PAQR4 | 0.00 | 0.05 | 615 | 609 | 281 | 351 |
| NM_030792 | GDPD5 | 0.00 | 0.05 | 333 | 344 | 482 | 577 |
| NM_006100 | ST3GAL6 | 0.00 | 0.05 | 124 | 98 | 212 | 272 |
| NM_014572 | LATS2 | 0.00 | 0.05 | 2975 | 3090 | 2466 | 2728 |
| NM_001143935 | CROT | 0.00 | 0.05 | 124 | 162 | 406 | 492 |
| NM_003681 | PDXK | -0.02 | 0.05 | 6372 | 6019 | 6653 | 7177 |
| NM_031935 | HMCN1 | 0.00 | 0.05 | 290 | 269 | 2570 | 2839 |
| NM_018079 | SRBD1 | 0.00 | 0.05 | 187 | 228 | 289 | 360 |
| NM_001101654 | CXXC1 | 0.00 | 0.05 | 1000 | 1066 | 886 | 1022 |
| NM_201269 | ZNF644 | 0.00 | 0.05 | 544 | 587 | 751 | 874 |
| NM_001286416 | SUM03 | 0.00 | 0.05 | 2581 | 2702 | 4413 | 4802 |
| NM_001531 | MR1 | 0.00 | 0.05 | 600 | 538 | 1431 | 1614 |

(continued)

| Table 5B: Differentially Upregulated Genes in SEN + IL-2 | | | | | | | |
|---|---|---|---|---|---|---|---|
| **RefSeq ID** | **Gene Symbol** | **SR GFOLD** | **SEN GFOLD** | **SR IL-2 -** | **SR IL-2 +** | **SEN IL-2 -** | **SEN IL-2 +** |
| NM_000866 | HTR1F | 0.00 | 0.05 | 135 | 107 | 485 | 580 |
| NM_001190826 | FAM217B | 0.00 | 0.05 | 261 | 210 | 175 | 229 |
| NM_001198759 | LY75-CD302 | -0.23 | 0.05 | 175 | 113 | 175 | 229 |
| NM_001258213 | PCID2 | 0.00 | 0.05 | 853 | 873 | 1088 | 1242 |
| NM_138400 | NOM1 | 0.00 | 0.05 | 610 | 688 | 624 | 734 |
| NM_001284304 | TBC1D22 A | 0.00 | 0.05 | 734 | 714 | 1325 | 1499 |
| NM_032595 | PPP1R9B | 0.00 | 0.05 | 2155 | 2228 | 3051 | 3351 |
| NM_003171 | SUPV3L1 | 0.00 | 0.05 | 447 | 501 | 745 | 867 |
| NM_004589 | SCO1 | 0.00 | 0.05 | 537 | 491 | 705 | 823 |
| NM_152905 | NEDD1 | 0.00 | 0.05 | 281 | 330 | 291 | 362 |
| NM_001145358 | SIN3A | 0.01 | 0.05 | 1155 | 1274 | 1682 | 1884 |
| NM_018288 | PHF10 | 0.00 | 0.05 | 263 | 253 | 306 | 379 |
| NM_016352 | CPA4 | 0.00 | 0.05 | 36 | 41 | 214 | 274 |
| NM_001261406 | MPDZ | 0.01 | 0.05 | 1860 | 2012 | 3700 | 4041 |
| NM_019095 | CRLS1 | 0.00 | 0.05 | 532 | 580 | 562 | 665 |
| NM_001077654 | TNFAIP8 | 0.00 | 0.05 | 109 | 101 | 67 | 100 |
| NM_138809 | CMBL | 0.00 | 0.05 | 368 | 362 | 975 | 1118 |
| NM_181706 | DNAJC24 | 0.00 | 0.05 | 224 | 230 | 370 | 451 |
| NM_014023 | WDR37 | 0.00 | 0.05 | 1037 | 1010 | 1439 | 1621 |
| NM_025134 | CHD9 | 0.00 | 0.05 | 1535 | 1514 | 2780 | 3059 |
| NM_001099660 | LRRN3 | 0.00 | 0.05 | 240 | 246 | 114 | 157 |
| NM_018047 | RBM22 | 0.00 | 0.05 | 654 | 709 | 1013 | 1159 |
| NM_152289 | ZNF561 | 0.00 | 0.05 | 216 | 239 | 397 | 481 |
| NM_002395 | ME1 | 0.00 | 0.05 | 487 | 524 | 1452 | 1634 |
| NM_014673 | EMC2 | 0.00 | 0.05 | 318 | 307 | 657 | 769 |
| NM_014182 | ORMDL2 | 0.00 | 0.05 | 496 | 463 | 1104 | 1257 |
| NM_138558 | PPP1R8 | 0.00 | 0.05 | 770 | 812 | 1000 | 1144 |
| **RefSeq ID** | **Gene Symbol** | **SR GFOLD** | **SEN GFOLD** | **SR IL-2 -** | **IL-2 +** | **SEN IL-2 -** | **SEN IL-2 +** |
| NM_080671 | KCNE4 | 0.00 | 0.05 | 1172 | 1105 | 834 | 963 |
| NM_032811 | TBRG1 | 0.00 | 0.05 | 976 | 1060 | 1287 | 1455 |
| NM_152617 | RNF168 | 0.00 | 0.05 | 960 | 933 | 1227 | 1390 |
| NM_000051 | ATM | 0.00 | 0.05 | 907 | 856 | 1633 | 1828 |
| NM_014516 | CNOT3 | 0.00 | 0.05 | 1025 | 1057 | 936 | 1074 |
| NM_001100814 | TMEM55 B | 0.00 | 0.05 | 787 | 743 | 1123 | 1277 |
| NM_030818 | CCDC130 | 0.00 | 0.05 | 473 | 542 | 661 | 773 |
| NM_018046 | AGGF1 | 0.00 | 0.05 | 456 | 519 | 651 | 762 |
| NM_015120 | ALMS1 | 0.00 | 0.05 | 728 | 715 | 1042 | 1189 |

(continued)

| RefSeq ID | Gene Symbol | SR GFOLD | SEN GFOLD | SR IL-2 - | IL-2 + | SEN IL-2 - | SEN IL-2 + |
|---|---|---|---|---|---|---|---|
| NM_001204504 | DGKH | 0.00 | 0.05 | 378 | 440 | 445 | 534 |
| NM_001170791 | RMDN2 | 0.00 | 0.05 | 62 | 41 | 110 | 152 |
| NM_015355 | SUZ12 | 0.00 | 0.05 | 694 | 744 | 811 | 937 |
| NM_001256163 | BIRC2 | 0.00 | 0.05 | 2499 | 2566 | 2697 | 2965 |
| NM_003080 | SMPD2 | 0.00 | 0.05 | 75 | 69 | 173 | 226 |
| NM_152581 | MOSPD2 | 0.00 | 0.05 | 921 | 889 | 969 | 1109 |
| NM_003028 | SHB | 0.00 | 0.05 | 967 | 984 | 1343 | 1514 |
| NM_006111 | ACAA2 | 0.00 | 0.05 | 520 | 554 | 1314 | 1482 |
| NM_001251845 | TRPC1 | 0.00 | 0.05 | 187 | 199 | 406 | 490 |
| NM_003478 | CUL5 | 0.00 | 0.05 | 784 | 801 | 1156 | 1311 |
| NM_014727 | KMT2B | 0.00 | 0.05 | 946 | 894 | 932 | 1068 |
| NM_006458 | TRIM3 | 0.00 | 0.05 | 550 | 603 | 682 | 795 |
| NM_018233 | OGFOD1 | 0.00 | 0.05 | 1183 | 1193 | 1581 | 1769 |
| NM_152449 | LYSMD4 | 0.00 | 0.05 | 170 | 162 | 200 | 257 |
| NM_001287391 | C9orf131 | -0.48 | 0.05 | 62 | 26 | 60 | 91 |
| NM_003218 | TERF1 | 0.00 | 0.05 | 109 | 141 | 187 | 242 |
| NM_020753 | CASKIN2 | 0.00 | 0.05 | 275 | 318 | 416 | 501 |
| NM_001143835 | NFRKB | 0.00 | 0.05 | 947 | 1017 | 1260 | 1423 |
| NM_001001433 | STX16 | 0.00 | 0.05 | 1160 | 1251 | 2121 | 2347 |
| NM_020832 | ZNF687 | 0.00 | 0.05 | 736 | 715 | 1054 | 1200 |
| NM_001164501 | EIF4ENIF 1 | 0.00 | 0.05 | 688 | 730 | 1081 | 1229 |
| NM_001257133 | COX14 | 0.00 | 0.05 | 306 | 361 | 586 | 689 |
| NM_005559 | LAMA1 | 0.00 | 0.04 | 2812 | 2685 | 4313 | 4673 |
| NM_005086 | SSPN | 0.00 | 0.04 | 300 | 335 | 811 | 935 |
| NM_014804 | KIAA075 3 | 0.00 | 0.04 | 278 | 260 | 503 | 597 |
| NM_001195563 | CAMTA1 | 0.00 | 0.04 | 302 | 332 | 345 | 421 |
| NM_020888 | KIAA152 2 | 0.00 | 0.04 | 567 | 624 | 1183 | 1338 |
| NM_152245 | CPT1B | 0.00 | 0.04 | 29 | 32 | 69 | 102 |
| **RefSeq ID** | **Gene Symbol** | **SR GFOLD** | **SEN GFOLD** | **SR IL-2 -** | **SR IL-2 +** | **SEN IL-2 -** | **SEN IL-2 +** |
| NM_024852 | AGO3 | 0.00 | 0.04 | 437 | 507 | 626 | 732 |
| NM_153265 | EML3 | 0.00 | 0.04 | 843 | 876 | 1493 | 1671 |
| NM_170692 | RASAL2 | 0.00 | 0.04 | 2394 | 2373 | 4240 | 4590 |
| NM_012477 | WBP1 | 0.00 | 0.04 | 241 | 237 | 547 | 645 |
| NM_003006 | SELPLG | 0.00 | 0.04 | 105 | 132 | 339 | 414 |
| NM_005146 | SART1 | 0.00 | 0.04 | 1640 | 1745 | 2225 | 2453 |
| NM_002387 | MCC | 0.00 | 0.04 | 159 | 194 | 435 | 521 |
| NM_015365 | AMMEC R1 | 0.00 | 0.04 | 263 | 290 | 239 | 301 |
| NM_018318 | CCDC91 | -0.29 | 0.04 | 255 | 165 | 445 | 532 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_015323 | UFL1 | 0.00 | 0.04 | 919 | 860 | 1192 | 1346 |
| NM_005273 | GNB2 | 0.00 | 0.04 | 4944 | 5151 | 6204 | 6654 |
| NM_004541 | NDUFA1 | -0.23 | 0.04 | 667 | 495 | 888 | 1017 |
| NM_001144936 | C11orf95 | 0.01 | 0.04 | 270 | 329 | 466 | 555 |
| NM_007044 | KATNA1 | 0.00 | 0.04 | 105 | 124 | 212 | 270 |
| NM_080430 | SELM | 0.00 | 0.04 | 1399 | 1490 | 3854 | 4177 |
| NM_139159 | DPP9 | 0.00 | 0.04 | 3465 | 3378 | 4091 | 4427 |
| NM_001039885 | FKRP | 0.00 | 0.04 | 490 | 531 | 647 | 754 |
| NM_012207 | HNRNPH 3 | 0.00 | 0.04 | 2045 | 2036 | 2175 | 2397 |
| NM_000252 | MTM1 | 0.00 | 0.04 | 280 | 268 | 422 | 506 |
| NM_004506 | HSF2 | 0.00 | 0.04 | 289 | 236 | 220 | 279 |
| NM_015905 | TRIM24 | 0.00 | 0.04 | 401 | 468 | 451 | 538 |
| NM_022098 | XPNPEP3 | 0.00 | 0.04 | 484 | 488 | 705 | 817 |
| NM_001099415 | POM121C | 0.00 | 0.04 | 1921 | 1902 | 1828 | 2026 |
| NM_080821 | FAM210B | 0.00 | 0.04 | 564 | 490 | 1512 | 1688 |
| NM_019008 | MIEF1 | 0.00 | 0.04 | 1574 | 1640 | 2439 | 2676 |
| NM_032840 | SPRYD3 | 0.00 | 0.04 | 1329 | 1309 | 2539 | 2782 |
| NM_002033 | FUT4 | 0.00 | 0.04 | 488 | 448 | 408 | 490 |
| NM_019044 | CCDC93 | 0.01 | 0.04 | 1515 | 1654 | 2824 | 3083 |
| NM_013361 | ZNF223 | 0.00 | 0.04 | 59 | 66 | 104 | 144 |
| NM_001040157 | CEP44 | 0.00 | 0.04 | 258 | 209 | 237 | 298 |
| NM_003748 | ALDH4A 1 | 0.00 | 0.04 | 530 | 500 | 876 | 1002 |
| NM_006227 | PLTP | 0.00 | 0.04 | 709 | 752 | 2219 | 2440 |
| NM_001991 | EZH1 | 0.00 | 0.04 | 794 | 818 | 1728 | 1917 |
| NM_001203260 | NDUFC2-KCTD14 | 0.00 | 0.04 | 824 | 882 | 1714 | 1902 |
| NM_006568 | CGRRF1 | 0.00 | 0.04 | 82 | 85 | 195 | 250 |
| NM_033107 | GTPBP10 | -0.03 | 0.04 | 270 | 214 | 412 | 494 |
| NM_020195 | SDR39U1 | 0.00 | 0.04 | 700 | 659 | 857 | 981 |
| NM_018452 | TMEM24 2 | 0.00 | 0.04 | 298 | 353 | 478 | 567 |
| NM_003993 | CLK2 | -0.08 | 0.04 | 404 | 322 | 368 | 445 |
| NM_018328 | MBD5 | 0.00 | 0.04 | 183 | 197 | 622 | 725 |
| NM_001242491 | FAM156 A | 0.00 | 0.04 | 637 | 650 | 593 | 693 |
| NM_003482 | KMT2D | -0.27 | 0.04 | 4235 | 3338 | 4357 | 4695 |
| NM_015627 | LDLRAP 1 | 0.00 | 0.04 | 905 | 822 | 988 | 1122 |
| NM_001165258 | TMEM14 C | 0.01 | 0.04 | 770 | 868 | 1620 | 1800 |
| NM_001001563 | TIMM50 | 0.00 | 0.04 | 1348 | 1292 | 1685 | 1869 |
| NM_022170 | EIF4H | 0.00 | 0.04 | 6228 | 6195 | 9733 | 10318 |
| NM_024316 | LENG1 | 0.00 | 0.04 | 130 | 153 | 177 | 229 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_017415 | KLHL3 | 0.00 | 0.04 | 28 | 37 | 337 | 410 |
| NM_024731 | KLHL36 | 0.00 | 0.04 | 859 | 867 | 1350 | 1510 |
| NM_033505 | EPT1 | 0.00 | 0.04 | 695 | 706 | 1092 | 1233 |
| NM_006366 | CAP2 | 0.00 | 0.04 | 739 | 809 | 1603 | 1780 |
| NM_004041 | ARRB1 | 0.00 | 0.04 | 566 | 590 | 1042 | 1179 |
| NM_005680 | TAF1B | 0.00 | 0.04 | 302 | 351 | 668 | 774 |
| NM_005068 | SIM1 | 0.00 | 0.04 | 97 | 109 | 306 | 375 |
| NM_014997 | KLHDC1 0 | 0.00 | 0.04 | 674 | 711 | 919 | 1046 |
| NM_145207 | SPATA5 | 0.00 | 0.03 | 515 | 562 | 522 | 614 |
| NM_021934 | ATG101 | 0.00 | 0.03 | 1647 | 1548 | 1840 | 2031 |
| NM_005643 | TAF11 | 0.00 | 0.03 | 207 | 253 | 308 | 377 |
| NM_198243 | ASB7 | 0.01 | 0.03 | 356 | 423 | 493 | 582 |
| NM_172195 | EIF2B4 | 0.00 | 0.03 | 694 | 742 | 1071 | 1209 |
| NM_018244 | UQCC1 | 0.00 | 0.03 | 553 | 528 | 1019 | 1153 |
| NM_080742 | B3GAT2 | 0.00 | 0.03 | 97 | 102 | 112 | 153 |
| NM_001199107 | TBC1D24 | 0.00 | 0.03 | 877 | 948 | 1242 | 1392 |
| NM_013398 | ZNF224 | 0.00 | 0.03 | 150 | 178 | 285 | 351 |
| NM_001355 | DDT | 0.00 | 0.03 | 278 | 302 | 636 | 738 |
| NM_001099684 | FAM156B | 0.00 | 0.03 | 611 | 642 | 595 | 693 |
| NM_000297 | PKD2 | 0.00 | 0.03 | 3107 | 3270 | 4010 | 4316 |
| NM_018648 | NOP10 | 0.00 | 0.03 | 2266 | 2381 | 3438 | 3715 |
| NM_145166 | ZBTB47 | 0.00 | 0.03 | 1921 | 1951 | 3342 | 3614 |
| NM_024047 | NUDT9 | 0.00 | 0.03 | 479 | 484 | 957 | 1085 |
| NM_005896 | IDH1 | 0.00 | 0.03 | 493 | 529 | 1922 | 2115 |
| NM_152267 | RNF185 | 0.00 | 0.03 | 1375 | 1483 | 2444 | 2667 |
| NM_001198807 | MSANTD 3 | 0.00 | 0.03 | 2155 | 2177 | 2753 | 2993 |
| NM_001407 | CELSR3 | 0.00 | 0.03 | 156 | 153 | 175 | 226 |
| NM_006528 | TFPI2 | -0.12 | 0.03 | 18837 | 16887 | 45731 | 47463 |
| NM_001143769 | ZNF438 | -0.08 | 0.03 | 189 | 138 | 225 | 283 |
| NM_022761 | C11orf1 | 0.00 | 0.03 | 46 | 67 | 119 | 161 |
| NM_004383 | CSK | 0.00 | 0.03 | 1583 | 1625 | 1464 | 1627 |
| NM_133473 | ZNF431 | 0.00 | 0.03 | 91 | 89 | 92 | 129 |
| NM_001040662 | VCPKMT | 0.00 | 0.03 | 70 | 58 | 92 | 129 |
| NM_016544 | DNAJC27 | 0.00 | 0.03 | 113 | 82 | 92 | 129 |
| NM_006212 | PFKFB2 | 0.00 | 0.03 | 266 | 309 | 788 | 902 |
| NM_001278559 | ZNF316 | 0.00 | 0.03 | 407 | 435 | 601 | 699 |
| NM_015492 | C15orf39 | 0.00 | 0.03 | 936 | 1016 | 980 | 1109 |
| NM_001040715 | KIAA089 5L | -1.15 | 0.03 | 1032 | 407 | 547 | 640 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_182515 | ZNF714 | -0.53 | 0.03 | 249 | 135 | 125 | 168 |
| NM_001242659 | C1orf233 | 0.01 | 0.03 | 75 | 107 | 108 | 148 |
| NM_015138 | RTF1 | 0.00 | 0.03 | 1491 | 1589 | 2217 | 2425 |
| NM_001128928 | INPP1 | 0.00 | 0.03 | 571 | 566 | 734 | 843 |
| NM_018036 | ATG2B | 0.00 | 0.03 | 918 | 858 | 1870 | 2057 |
| NM_001382 | DPAGT1 | 0.00 | 0.03 | 1001 | 1059 | 1695 | 1871 |
| NM_001184743 | PGBD1 | 0.00 | 0.03 | 204 | 223 | 268 | 331 |
| NM_001271918 | SEC11A | -0.03 | 0.03 | 985 | 866 | 1104 | 1240 |
| NM_144601 | CMTM3 | 0.00 | 0.03 | 464 | 410 | 925 | 1048 |
| NM_001005920 | JMJD8 | 0.00 | 0.03 | 2700 | 2653 | 5521 | 5882 |
| NM_176795 | HRAS | 0.00 | 0.03 | 604 | 604 | 996 | 1124 |
| NM_001286611 | REPS1 | 0.00 | 0.03 | 570 | 628 | 917 | 1039 |
| NM_001142405 | SLMO1 | 0.00 | 0.03 | 64 | 89 | 133 | 177 |
| NM_001161425 | ZNF610 | 0.00 | 0.03 | 29 | 24 | 127 | 170 |
| NM_002439 | MSH3 | 0.00 | 0.03 | 285 | 344 | 497 | 584 |
| NM_001184970 | PACSIN2 | 0.00 | 0.03 | 2428 | 2567 | 3650 | 3926 |
| NM_004888 | ATP6V1G 1 | 0.00 | 0.03 | 1435 | 1401 | 2506 | 2725 |
| NM_014633 | CTR9 | 0.00 | 0.03 | 1288 | 1376 | 1861 | 2044 |
| NM_001170755 | NBPF15 | 0.00 | 0.03 | 656 | 658 | 1102 | 1237 |
| NM_001136264 | DAZAP2 | -0.04 | 0.03 | 3305 | 3031 | 3841 | 4124 |
| NM_020207 | ERCC6L2 | 0.00 | 0.03 | 411 | 443 | 940 | 1063 |
| NM_001143942 | RBM24 | 0.00 | 0.03 | 327 | 342 | 545 | 636 |
| NM_198291 | SRC | 0.00 | 0.03 | 1196 | 1165 | 2362 | 2571 |
| NM_001267039 | LARP7 | 0.00 | 0.03 | 459 | 439 | 593 | 688 |
| NM_020453 | ATP10D | 0.00 | 0.03 | 1061 | 1142 | 2263 | 2466 |
| NM_001190810 | AGAP9 | 0.00 | 0.03 | 107 | 129 | 154 | 201 |
| NM_014291 | GCAT | 0.00 | 0.03 | 271 | 315 | 283 | 347 |
| NM_020933 | ZNF317 | 0.00 | 0.03 | 629 | 716 | 1258 | 1401 |
| NM_022039 | FBXW4 | 0.00 | 0.02 | 257 | 311 | 580 | 673 |
| NM_001136574 | LANCL1 | 0.00 | 0.02 | 832 | 897 | 1855 | 2033 |
| NM_007342 | NUPL2 | 0.00 | 0.02 | 258 | 271 | 349 | 420 |
| NM_012213 | MLYCD | 0.01 | 0.02 | 283 | 343 | 439 | 519 |
| NM_173690 | SCAI | 0.00 | 0.02 | 221 | 206 | 304 | 370 |
| NM_002481 | PPP1R12 B | 0.00 | 0.02 | 195 | 200 | 399 | 475 |
| NM_145298 | APOBEC 3F | 0.00 | 0.02 | 82 | 107 | 198 | 251 |
| NM_005173 | ATP2A3 | 0.00 | 0.02 | 54 | 61 | 198 | 251 |
| NM_014071 | NCOA6 | 0.00 | 0.02 | 1485 | 1567 | 2048 | 2235 |
| NM_031917 | ANGPTL 6 | -0.50 | 0.02 | 339 | 195 | 279 | 342 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_032792 | ZBTB45 | 0.00 | 0.02 | 585 | 557 | 541 | 630 |
| NM_001170629 | CHD8 | 0.00 | 0.02 | 2690 | 2666 | 3976 | 4253 |
| NM_144970 | CXorf38 | 0.00 | 0.02 | 568 | 622 | 649 | 747 |
| NM_001100819 | MOB4 | 0.00 | 0.02 | 413 | 458 | 674 | 774 |
| NM_001199302 | CNOT2 | 0.00 | 0.02 | 976 | 952 | 1506 | 1662 |
| NM_001198595 | STON1 | 0.00 | 0.02 | 98 | 114 | 403 | 479 |
| NM_012100 | DNPEP | -0.13 | 0.02 | 1509 | 1265 | 1886 | 2063 |
| NM_001039374 | CCDC183 | 0.00 | 0.02 | 36 | 37 | 96 | 133 |
| NM_017671 | FERMT1 | 0.00 | 0.02 | 76 | 70 | 264 | 325 |
| NM_052951 | DNTTIP1 | -0.13 | 0.02 | 519 | 406 | 607 | 701 |
| NM_001145309 | LRTOMT | 0.00 | 0.02 | 285 | 299 | 549 | 638 |
| NM_015490 | SEC31B | 0.00 | 0.02 | 108 | 100 | 158 | 205 |
| NM_001932 | MPP3 | -0.09 | 0.02 | 313 | 241 | 225 | 281 |
| NM_002567 | PEBP1 | -0.06 | 0.02 | 2519 | 2266 | 5501 | 5830 |
| NM_004641 | MLLT10 | 0.00 | 0.02 | 713 | 787 | 796 | 904 |
| NM_198273 | LYSMD3 | 0.00 | 0.02 | 533 | 610 | 410 | 486 |
| NM_015189 | EXOC6B | 0.00 | 0.02 | 439 | 477 | 1400 | 1547 |
| NM_024029 | YIPF2 | -0.02 | 0.02 | 1419 | 1278 | 1720 | 1885 |
| NM_003632 | CNTNAP 1 | 0.00 | 0.02 | 2055 | 2094 | 2543 | 2749 |
| NM_024511 | HAUS3 | 0.00 | 0.02 | 234 | 229 | 166 | 214 |
| NM_001080415 | U2SURP | 0.00 | 0.02 | 1055 | 1105 | 1254 | 1392 |
| NM_004831 | MED26 | -0.10 | 0.02 | 387 | 303 | 412 | 488 |
| NM_006709 | EHMT2 | -0.05 | 0.02 | 1521 | 1347 | 2021 | 2201 |
| NM_001104587 | SLFN11 | 0.00 | 0.02 | 879 | 970 | 647 | 743 |
| NM_002894 | RBBP8 | 0.00 | 0.02 | 422 | 427 | 470 | 551 |
| NM_003136 | SRP54 | 0.00 | 0.02 | 1385 | 1513 | 1755 | 1920 |
| NM_001287585 | NUP35 | 0.00 | 0.02 | 129 | 95 | 87 | 122 |
| NM_005837 | POP7 | 0.00 | 0.02 | 737 | 821 | 761 | 865 |
| NM_001198801 | EIF4G3 | 0.00 | 0.02 | 2683 | 2675 | 5914 | 6246 |
| NM_022483 | C5orf28 | 0.00 | 0.02 | 222 | 213 | 347 | 416 |
| NM_024573 | C6orf211 | 0.00 | 0.02 | 517 | 468 | 593 | 684 |
| NM_007313 | ABL1 | 0.00 | 0.02 | 5902 | 5847 | 6875 | 7236 |
| NM_001164407 | TLCD2 | -0.26 | 0.02 | 380 | 264 | 349 | 418 |
| NM_001949 | E2F3 | 0.00 | 0.02 | 598 | 656 | 755 | 858 |
| NM_032140 | ENKD1 | 0.00 | 0.02 | 138 | 146 | 231 | 287 |
| NM_001272071 | AP1S2 | 0.00 | 0.02 | 311 | 309 | 401 | 475 |
| NM_001127208 | TET2 | 0.00 | 0.02 | 444 | 451 | 445 | 523 |
| NM_012118 | CCRN4L | 0.00 | 0.02 | 462 | 475 | 312 | 377 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_032127 | FAM160 A2 | 0.00 | 0.02 | 543 | 550 | 724 | 824 |
| NM_024095 | ASB8 | 0.00 | 0.02 | 561 | 636 | 928 | 1042 |
| NM_001221 | CAMK2D | 0.00 | 0.02 | 2453 | 2623 | 4245 | 4510 |
| NM_020191 | MRPS22 | 0.00 | 0.02 | 463 | 445 | 701 | 799 |
| NM_005116 | SLC23A2 | -0.05 | 0.02 | 693 | 589 | 1691 | 1848 |
| NM_014284 | NCDN | 0.00 | 0.01 | 1039 | 982 | 2194 | 2375 |
| NM_145036 | CEP112 | 0.00 | 0.01 | 83 | 80 | 316 | 381 |
| NM_030929 | KAZALD 1 | 0.00 | 0.01 | 195 | 214 | 100 | 137 |
| NM_001478 | B4GALN T1 | 0.00 | 0.01 | 136 | 121 | 514 | 597 |
| NM_003151 | STAT4 | 0.00 | 0.01 | 43 | 62 | 106 | 144 |
| NM_014813 | LRIG2 | 0.00 | 0.01 | 290 | 265 | 470 | 549 |
| NM_022757 | CCDC14 | 0.00 | 0.01 | 448 | 497 | 772 | 874 |
| NM_194313 | KIF24 | 0.00 | 0.01 | 231 | 207 | 291 | 353 |
| NM_198535 | ZNF699 | 0.00 | 0.01 | 99 | 87 | 166 | 213 |
| NM_152558 | IQCE | 0.00 | 0.01 | 466 | 491 | 1383 | 1521 |
| NM_001204062 | COMMD 3-BMI1 | 0.00 | 0.01 | 764 | 837 | 1422 | 1562 |
| NM_001131034 | RNF212 | 0.00 | 0.01 | 66 | 77 | 119 | 159 |
| NM_005732 | RAD50 | 0.00 | 0.01 | 920 | 857 | 1834 | 1994 |
| NM_152420 | C9orf41 | 0.00 | 0.01 | 176 | 205 | 430 | 505 |
| NM_005017 | PCYT1A | 0.00 | 0.01 | 317 | 354 | 801 | 904 |
| NM_022353 | OSGEPL1 | -0.21 | 0.01 | 160 | 103 | 146 | 190 |
| NM_030891 | LRRC3 | 0.00 | 0.01 | 45 | 35 | 60 | 89 |
| NM_004501 | HNRNPU | 0.00 | 0.01 | 4140 | 4232 | 4480 | 4739 |
| NM_002547 | OPHN1 | 0.00 | 0.01 | 1223 | 1336 | 1260 | 1390 |
| RefSeq ID | Gene Symbol | SR GFOLD | SEN GFOLD | SR IL-2 - | SR IL-2 + | SEN IL-2- | SEN IL-2 + |
| NM_005772 | RCL1 | 0.00 | 0.01 | 174 | 207 | 250 | 307 |
| NM_001195087 | GGACT | -0.04 | 0.01 | 151 | 110 | 108 | 146 |
| NM_001281453 | MBD3 | 0.00 | 0.01 | 1837 | 1942 | 1892 | 2052 |

## Table 5C: Differentially Downregulated Genes in SR + IL-2

| Table 5C: Differentially Downregulated Genes in SR + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_138340 | ABHD3 | -0.04 | 0.00 | 161 | 118 | 110 | 109 |
| NM_001607 | ACAA1 | -0.06 | 0.20 | 741 | 627 | 1568 | 1945 |
| NM_014716 | ACAP1 | -0.03 | 0.00 | 116 | 81 | 87 | 94 |
| NM_022735 | ACBD3 | -0.16 | 0.21 | 720 | 564 | 996 | 1272 |
| NM_018367 | ACER3 | -0.10 | 0.08 | 313 | 239 | 364 | 453 |

(continued)

| Table 5C: Differentially Downregulated Genes in SR + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_001160094 | ACOT13 | -0.01 | 0.10 | 307 | 250 | 705 | 852 |
| NM_018477 | ACTR10 | -0.04 | 0.18 | 678 | 580 | 1751 | 2131 |
| NM_020445 | ACTR3B | -0.31 | 0.00 | 326 | 215 | 241 | 224 |
| NM_020328 | ACVR1B | -0.09 | 0.18 | 874 | 731 | 1059 | 1318 |
| NM_138448 | ACYP2 | -0.17 | 0.55 | 112 | 70 | 146 | 272 |
| NM_030957 | ADAMTS 10 | -0.50 | 1.11 | 139 | 70 | 87 | 250 |
| NM_139025 | ADAMTS 13 | -0.13 | 0.50 | 41 | 20 | 46 | 98 |
| NM_001102470 | ADH6 | -1.02 | 0.00 | 67 | 18 | 12 | 20 |
| NM_006829 | ADIRF | -0.07 | 0.34 | 883 | 748 | 1138 | 1577 |
| NM_020233 | ADPRM | -0.01 | 0.26 | 139 | 102 | 185 | 277 |
| NM_000679 | ADRA1B | -0.04 | 0.00 | 82 | 53 | 46 | 68 |
| NM_001144000 | AGAP5 | -0.03 | 0.31 | 70 | 44 | 58 | 105 |
| NM_005751 | AKAP9 | -0.04 | 0.20 | 1762 | 1585 | 2059 | 2540 |
| NM_005787 | ALG3 | -0.04 | 0.00 | 844 | 730 | 1004 | 974 |
| NM_001077690 | ALG9 | -0.08 | 0.19 | 694 | 576 | 1069 | 1338 |
| NM_001145374 | ALKBH2 | -0.30 | 0.00 | 269 | 175 | 185 | 190 |
| NM_138775 | ALKBH8 | -0.08 | 0.00 | 206 | 152 | 281 | 279 |
| NM_025144 | ALPK1 | -0.10 | 0.00 | 97 | 62 | 198 | 203 |
| NM_001118887 | ANGPT2 | -0.47 | 0.58 | 430 | 259 | 89 | 179 |
| NM_031917 | ANGPTL 6 | -0.50 | 0.02 | 339 | 195 | 279 | 342 |
| NM_001256182 | ANKRD1 1 | -0.09 | 0.28 | 2578 | 2261 | 3677 | 4684 |
| NM_015208 | ANKRD1 2 | -0.09 | 0.20 | 1047 | 884 | 1346 | 1680 |
| NM_001256053 | ANKRD2 6 | -0.15 | 0.00 | 332 | 246 | 356 | 388 |
| NM_025190 | ANKRD3 6B | -0.02 | 0.00 | 276 | 221 | 339 | 366 |
| NM_181726 | ANKRD3 7 | -0.06 | 2.14 | 339 | 269 | 50 | 312 |
| NM_001195144 | ANKRD4 4 | -0.20 | 0.80 | 387 | 280 | 618 | 1205 |
| NM_001002857 | ANXA2 | -0.03 | 0.14 | 14766 | 14085 | 27335 | 30694 |
| RefSeq ID | Gene Symbol | SR GFOLD | SEN GFOLD | SR IL-2 | SR IL-2 + | SR IL-2 - | SEN IL-2 + |
| NM_001130524 | AP1M1 | -0.12 | 0.00 | 3040 | 2625 | 2851 | 2834 |
| NM_153360 | APCDD1 L | -0.03 | 1.00 | 2412 | 2212 | 6576 | 13626 |
| NM_001130415 | APOLD1 | -0.22 | 0.00 | 245 | 167 | 135 | 157 |
| NM_015193 | ARC | -0.93 | 0.80 | 68 | 20 | 21 | 65 |
| NM_006015 | ARID 1A | -0.03 | 0.00 | 3424 | 3172 | 3396 | 3473 |
| NM_016374 | ARID4B | -0.04 | 0.28 | 367 | 297 | 401 | 567 |
| NM_006886 | ATP5E | -0.12 | 0.00 | 1135 | 946 | 1466 | 1436 |
| NM_001694 | ATP6V0C | -0.10 | 0.18 | 3092 | 2708 | 4147 | 4926 |
| NM_145230 | A TP6V0E 2 | -0.10 | 0.00 | 204 | 148 | 320 | 372 |

(continued)

| RefSeq ID | Gene Symbol | SR GFOLD | SEN GFOLD | SR IL-2 | SR IL-2 + | SR IL-2 - | SEN IL-2 + |
|---|---|---|---|---|---|---|---|
| NM_015994 | A TP6V1D | -0.05 | 0.18 | 1908 | 1710 | 2487 | 2996 |
| NM_178191 | ATPIF1 | -0.14 | 0.13 | 862 | 695 | 1832 | 2152 |
| NM_000489 | ATRX | -0.35 | 0.21 | 842 | 583 | 1543 | 1926 |
| NM_002973 | ATXN2 | -0.13 | 0.09 | 2071 | 1760 | 1882 | 2152 |
| NM_001098833 | ATXN7L3 | -0.11 | 0.00 | 1917 | 1644 | 1824 | 1786 |
| NM_001698 | AUH | -0.76 | 0.00 | 129 | 52 | 216 | 238 |
| NM_018842 | BAIAP2L 1 | -0.15 | 0.46 | 588 | 457 | 595 | 924 |
| NM_001291428 | BAX | -0.59 | 0.00 | 2775 | 1722 | 2647 | 2675 |
| NM_013450 | BAZ2B | -0.12 | 0.05 | 532 | 421 | 730 | 852 |
| NM_001204108 | BCL2L11 | -0.22 | 0.77 | 69 | 37 | 35 | 94 |
| NM_004183 | BEST1 | -0.03 | 0.47 | 165 | 123 | 424 | 680 |
| NM_001001786 | BLID | -0.43 | 0.00 | 94 | 46 | 73 | 91 |
| NM_181809 | BMP8A | -1.00 | 0.23 | 79 | 23 | 40 | 74 |
| NM_199186 | BPGM | -0.15 | 0.35 | 435 | 330 | 688 | 983 |
| NM_001281723 | BTD | -0.02 | 0.12 | 416 | 347 | 697 | 852 |
| NM_001009894 | C 12orf29 | -0.22 | 0.01 | 582 | 430 | 478 | 556 |
| NM_001194995 | C12orf65 | -0.01 | 0.00 | 380 | 316 | 264 | 309 |
| NM_001173978 | C14orf178 | -0.82 | 0.00 | 52 | 15 | 17 | 31 |
| NM_181656 | C17orf58 | -0.72 | 0.00 | 186 | 83 | 181 | 176 |
| NM_001288980 | C18orf54 | -0.06 | 0.00 | 197 | 147 | 202 | 170 |
| NM_018381 | C19orf66 | -0.05 | 0.00 | 648 | 545 | 878 | 932 |
| NM_024097 | C1orf50 | -0.31 | 0.07 | 118 | 67 | 123 | 170 |
| NM_001256609 | C1orf85 | -0.09 | 0.00 | 1489 | 1278 | 4226 | 4092 |
| NM_001286476 | C21orf58 | -0.29 | 0.00 | 195 | 122 | 156 | 140 |
| NM_182520 | C22orf15 | -0.10 | 0.00 | 85 | 53 | 106 | 140 |
| NM_001142964 | C22orf46 | -0.33 | 0.00 | 221 | 137 | 241 | 277 |
| NM_001170330 | C4orf3 | -0.17 | 0.78 | 1159 | 932 | 1479 | 2737 |
| NM_178569 | C5orf38 | -0.44 | 0.00 | 48 | 19 | 17 | 24 |
| NM_001204173 | C8orf44-SGK3 | -0.12 | 0.00 | 404 | 313 | 372 | 362 |
| **RefSeq ID** | **Gene Symbol** | **SR GFOLD** | **SEN GFOLD** | **SR IL-2** | **SR IL-2 +** | **SEN IL-2 -** | **SEN IL-2 +** |
| NM_001013842 | C8orf58 | -0.10 | 0.00 | 356 | 275 | 347 | 346 |
| NM_001099670 | C8orf59 | -0.15 | 0.07 | 261 | 189 | 195 | 255 |
| NM_001287391 | C9orf131 | -0.48 | 0.05 | 62 | 26 | 60 | 91 |
| NM_183241 | C9orf142 | -0.03 | 0.00 | 267 | 211 | 312 | 275 |
| NM_153045 | C9orf91 | -0.05 | 0.10 | 244 | 188 | 362 | 457 |
| NM_001242369 | CACFD1 | -0.15 | 0.00 | 120 | 77 | 206 | 207 |
| NM_001184902 | CARD8 | -0.06 | 0.00 | 231 | 175 | 378 | 364 |
| NM_020649 | CBX8 | -0.19 | 0.00 | 79 | 45 | 46 | 41 |

(continued)

| RefSeq ID | Gene Symbol | SR GFOLD | SEN GFOLD | SR IL-2 | SR IL-2 + | SEN IL-2 - | SEN IL-2 + |
|---|---|---|---|---|---|---|---|
| NM_001040440 | CCDC112 | -0.29 | 0.00 | 105 | 59 | 154 | 176 |
| NM_014157 | CCDC113 | -0.04 | 0.37 | 82 | 53 | 175 | 283 |
| NM_138771 | CCDC126 | -0.28 | 0.00 | 171 | 106 | 204 | 198 |
| NM_032779 | CCDC142 | -0.05 | 0.36 | 279 | 219 | 316 | 479 |
| NM_001080503 | CCDC159 | -0.09 | 0.16 | 73 | 44 | 181 | 253 |
| NM_001012506 | CCDC66 | -0.13 | 0.00 | 208 | 148 | 252 | 266 |
| NM_018318 | CCDC91 | -0.29 | 0.04 | 255 | 165 | 445 | 532 |
| NM_148672 | CCL28 | -0.20 | 1.39 | 68 | 37 | 44 | 170 |
| NM_030937 | CCNL2 | -0.03 | 0.18 | 1650 | 1482 | 2202 | 2669 |
| NM_014880 | CD302 | -0.28 | 0.09 | 175 | 109 | 173 | 233 |
| NM_016579 | CD320 | -0.29 | 0.32 | 1068 | 782 | 990 | 1360 |
| NM_001267698 | CD63 | -0.15 | 0.23 | 12326 | 10792 | 27628 | 33094 |
| NM_001251 | CD68 | -0.02 | 0.26 | 3830 | 3590 | 9144 | 11331 |
| NM_001782 | CD72 | -0.06 | 0.25 | 61 | 36 | 73 | 122 |
| NM_001785 | CDA | -0.23 | 0.89 | 220 | 146 | 293 | 640 |
| NM_145057 | CDC42EP 5 | -0.28 | 0.00 | 94 | 52 | 52 | 48 |
| NM_024011 | CDK11A | -0.02 | 0.26 | 210 | 163 | 177 | 266 |
| NM_001787 | CDK11B | -0.04 | 0.27 | 267 | 209 | 258 | 375 |
| NM_001800 | CDKN2D | -0.10 | 0.20 | 404 | 317 | 354 | 479 |
| NM_001127893 | CEACAM 19 | -0.11 | 0.00 | 124 | 83 | 127 | 146 |
| NM_001285878 | CEBPB | -0.05 | 0.55 | 2040 | 1825 | 2240 | 3503 |
| NM_001408 | CELSR2 | -0.18 | 0.51 | 88 | 52 | 27 | 65 |
| NM_001194998 | CEP152 | -0.10 | 0.00 | 482 | 385 | 245 | 231 |
| NM_025114 | CEP290 | -0.14 | 0.00 | 368 | 278 | 688 | 743 |
| NM_004365 | CETN3 | -0.26 | 0.00 | 177 | 112 | 125 | 126 |
| NM_006324 | CFDP1 | -0.01 | 0.00 | 982 | 874 | 1042 | 1046 |
| NM_001005271 | CHD3 | -0.07 | 0.12 | 2412 | 2149 | 3502 | 4015 |
| NM_017780 | CHD7 | -0.26 | 0.00 | 296 | 200 | 223 | 187 |
| NM_020244 | CHPT1 | -0.12 | 0.27 | 702 | 568 | 1175 | 1553 |
| NM_001171087 | CLCN2 | -0.43 | 0.00 | 242 | 141 | 233 | 264 |
| NM_001146079 | CLDN14 | -0.07 | 1.45 | 868 | 734 | 164 | 551 |
| NM_001040182 | CLDND1 | -0.03 | 0.41 | 1181 | 1047 | 942 | 1383 |
| NM_003993 | CLK2 | -0.08 | 0.04 | 404 | 322 | 368 | 445 |
| NM_015041 | CLUAP1 | -0.15 | 0.00 | 305 | 224 | 514 | 527 |
| NM_014255 | CNPY2 | -0.15 | 0.10 | 867 | 696 | 1680 | 1950 |
| NM_000614 | CNTF | -0.75 | 0.00 | 78 | 28 | 35 | 46 |
| NM_017738 | CNTLN | -0.02 | 0.00 | 228 | 178 | 277 | 335 |
| NM_001843 | CNTN1 | -0.13 | 0.00 | 61 | 34 | 4 | 0 |

(continued)

| RefSeq ID | Gene Symbol | SR GFOLD | SEN GFOLD | SR IL-2 | SR IL-2 + | SEN IL-2 - | SEN IL-2 + |
|---|---|---|---|---|---|---|---|
| NM_001856 | COL16A1 | -0.12 | 0.08 | 3354 | 2903 | 3032 | 3388 |
| NM_000093 | COL5A1 | -0.07 | 1.02 | 14317 | 13263 | 13133 | 27299 |
| NM_004369 | COL6A3 | -0.11 | 0.42 | 31491 | 28566 | 126573 | 171367 |
| NM_025147 | COQ10B | -0.11 | 0.52 | 1232 | 1037 | 1073 | 1684 |
| NM_017421 | COQ3 | -0.26 | 0.00 | 162 | 101 | 146 | 122 |
| NM_005694 | COX17 | -0.83 | 0.00 | 265 | 115 | 447 | 434 |
| NM_198076 | COX20 | -0.42 | 0.29 | 483 | 305 | 474 | 667 |
| NM_004374 | COX6C | -0.15 | 0.00 | 615 | 482 | 749 | 726 |
| NM_001304 | CPD | -0.08 | 0.38 | 1422 | 1232 | 2868 | 3946 |
| NM_001079533 | CPEB1 | -1.04 | 0.30 | 107 | 33 | 514 | 725 |
| NM_001286606 | CRACR2 B | -0.15 | 0.34 | 37 | 17 | 79 | 139 |
| NM_020825 | CRAMP1 L | -0.04 | 0.07 | 836 | 724 | 859 | 1002 |
| NM_014171 | CRIPT | -0.04 | 0.00 | 400 | 328 | 518 | 595 |
| NM_015989 | CSAD | -0.12 | 0.00 | 161 | 111 | 343 | 347 |
| NM_005211 | CSF1R | -0.27 | 0.98 | 80 | 43 | 189 | 455 |
| NM_152221 | CSNK1E | -0.04 | 0.35 | 3499 | 3209 | 3105 | 4176 |
| NM_000100 | CSTB | -0.15 | 0.50 | 3010 | 2546 | 7004 | 10268 |
| NM_172241 | CTAGE1 | -1.33 | 0.00 | 160 | 43 | 112 | 107 |
| NM_004388 | CTBS | -0.09 | 0.44 | 389 | 307 | 591 | 908 |
| NM_001336 | CTSZ | -0.02 | 0.70 | 6070 | 5747 | 10047 | 16785 |
| NM_005869 | CWC27 | -0.61 | 0.00 | 79 | 32 | 83 | 104 |
| NM_152434 | CWF19L2 | -0.22 | 0.00 | 282 | 195 | 306 | 338 |
| NM_001037333 | CYFIP2 | -0.11 | 0.00 | 109 | 71 | 79 | 79 |
| NM_000782 | CYP24A1 | -0.03 | 0.00 | 56 | 33 | 25 | 13 |
| NM_001136264 | DAZAP2 | -0.04 | 0.03 | 3305 | 3031 | 3841 | 4124 |
| NM_001290205 | DCP1A | -0.07 | 0.23 | 1188 | 1028 | 855 | 1111 |
| NM_001184781 | DGCR2 | -0.08 | 0.25 | 2247 | 1979 | 2161 | 2749 |
| NM_001012957 | DISC1 | -0.37 | 0.00 | 44 | 18 | 62 | 79 |
| NM_000108 | DLD | -0.05 | 0.10 | 943 | 817 | 1370 | 1604 |
| RefSeq ID | Gene Symbol | SR GFOLD | SEN GFOLD | SR IL-2 | SR IL-2 + | SEN IL-2- | SEN IL-2 + |
| NM_020730 | DLG3 | -0.11 | 0.00 | 169 | 119 | 410 | 433 |
| NM_013391 | DMGDH | -0.43 | 0.00 | 67 | 30 | 121 | 120 |
| NM_001081563 | DMPK | -0.05 | 0.20 | 877 | 754 | 1666 | 2059 |
| NM_012328 | DNAJB9 | -0.09 | 1.02 | 487 | 390 | 327 | 774 |
| NM_194283 | DNAJC21 | -0.04 | 0.33 | 792 | 683 | 971 | 1344 |
| NM_025219 | DNAJC5 | -0.12 | 0.37 | 3547 | 3082 | 3833 | 5196 |
| NM_012100 | DNPEP | -0.13 | 0.02 | 1509 | 1265 | 1886 | 2063 |
| NM_052951 | DNTTIP1 | -0.13 | 0.02 | 519 | 406 | 607 | 701 |

(continued)

| RefSeq ID | Gene Symbol | SR GFOLD | SEN GFOLD | SR IL-2 | SR IL-2 + | SEN IL-2- | SEN IL-2 + |
|---|---|---|---|---|---|---|---|
| NM_001280543 | DPF3 | -0.25 | 0.00 | 49 | 23 | 29 | 28 |
| NM_001935 | DPP4 | -0.05 | 0.13 | 356 | 285 | 1689 | 1991 |
| NM_001242901 | DPP9-AS1 | -0.17 | 0.00 | 425 | 319 | 451 | 390 |
| NM_024094 | DSCC1 | -0.20 | 0.00 | 118 | 73 | 108 | 81 |
| NM_001144769 | DST | -0.22 | 0.36 | 9290 | 7703 | 22150 | 29075 |
| NM_001256303 | DTNB | -0.27 | 0.00 | 145 | 88 | 98 | 129 |
| NM_001130987 | DYSF | -0.20 | 0.99 | 695 | 530 | 2392 | 5033 |
| NM_004215 | EBAG9 | -0.05 | 0.07 | 216 | 164 | 223 | 288 |
| NM_001290360 | EBF1 | -0.12 | 0.00 | 896 | 734 | 821 | 887 |
| NM_001199687 | EDA2R | -0.09 | 0.00 | 264 | 199 | 663 | 688 |
| NM_003566 | EEA1 | -0.11 | 0.25 | 1335 | 1129 | 2159 | 2754 |
| NM_005864 | EFS | -0.13 | 0.00 | 1084 | 892 | 1102 | 1135 |
| NM_006709 | EHMT2 | -0.05 | 0.02 | 1521 | 1347 | 2021 | 2201 |
| NM_001412 | EIF1AX | -0.05 | 0.33 | 1150 | 1003 | 1400 | 1911 |
| NM_003750 | EIF3A | -0.60 | 0.00 | 2520 | 1547 | 4265 | 4044 |
| NM_015904 | EIF5B | -0.37 | 0.00 | 601 | 399 | 869 | 937 |
| NM_175063 | EMC10 | -0.04 | 0.15 | 1293 | 1143 | 1508 | 1811 |
| NM_006331 | EMG1 | -0.02 | 0.00 | 1080 | 964 | 1004 | 996 |
| NM_020354 | ENTPD7 | -0.07 | 0.51 | 1171 | 1008 | 1362 | 2107 |
| NM_015409 | EP400 | -0.10 | 0.20 | 2486 | 2167 | 2891 | 3514 |
| NM_022140 | EPB41L4 A | -0.02 | 0.00 | 53 | 31 | 23 | 33 |
| NM_018341 | ERMARD | -0.17 | 0.14 | 273 | 195 | 327 | 427 |
| NM_207582 | ERVFRD-1 | -1.15 | 0.00 | 67 | 16 | 21 | 24 |
| NM_007036 | ESM1 | -0.34 | 1.94 | 2562 | 1887 | 1863 | 7612 |
| NM_001003927 | EVI2A | -0.14 | 0.00 | 47 | 24 | 112 | 85 |
| NM_001441 | FAAH | -0.49 | 0.12 | 44 | 16 | 33 | 59 |
| NM_199336 | FAHD2B | -0.15 | 0.30 | 143 | 95 | 135 | 214 |
| NM_173822 | FAM126B | -0.14 | 0.42 | 271 | 198 | 324 | 512 |
| NM_001291832 | FAM132B | -0.07 | 0.39 | 91 | 59 | 258 | 408 |
| NM_032130 | FAM186B | -0.84 | 0.00 | 62 | 19 | 35 | 20 |
| **RefSeq ID** | **Gene Symbol** | **SR GFOLD** | **SEN GFOLD** | **SR IL-2 -** | **SR IL-2 +** | **SEN IL-2 -** | **SEN IL-2 +** |
| NM_001039762 | FAM196A | -0.16 | 0.00 | 49 | 25 | 202 | 222 |
| NM_001082967 | FAM19A5 | -0.37 | 1.19 | 407 | 262 | 106 | 314 |
| NM_001040020 | FAM3C | -0.02 | 0.33 | 1297 | 1162 | 1518 | 2059 |
| NM_014722 | FAM65B | -0.25 | 0.00 | 76 | 41 | 146 | 144 |
| NM_174911 | FAM84B | -0.21 | 0.00 | 46 | 22 | 27 | 28 |
| NM_174905 | FAM98C | -0.24 | 0.00 | 146 | 91 | 189 | 201 |
| NM_001018113 | FANCB | -0.30 | 0.00 | 221 | 140 | 52 | 57 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_012166 | FBXO10 | -0.13 | 0.00 | 675 | 539 | 788 | 769 |
| NM_033393 | FHDC1 | -0.03 | 0.30 | 44 | 24 | 19 | 44 |
| NM_000801 | FKBP1A | -0.03 | 0.41 | 4437 | 4137 | 9144 | 12506 |
| NM_001458 | FLNC | -0.07 | 0.25 | 135215 | 127692 | 109037 | 130883 |
| NM_014053 | FLVCR1 | -0.22 | 0.00 | 191 | 126 | 110 | 142 |
| NM_002027 | FNTA | -0.05 | 0.13 | 542 | 451 | 693 | 854 |
| NM_005938 | FOXO4 | -0.01 | 0.38 | 308 | 251 | 343 | 525 |
| NM_005860 | FSTL3 | -0.05 | 0.25 | 3553 | 3252 | 1549 | 1998 |
| NM_017647 | FTSJ3 | -0.10 | 0.00 | 1235 | 1042 | 1250 | 1248 |
| NM_000152 | GAA | -0.40 | 0.77 | 591 | 384 | 1387 | 2556 |
| NM_001924 | GADD45 A | -0.32 | 0.38 | 1772 | 1300 | 1375 | 1937 |
| NM_138801 | GALM | -0.29 | 0.00 | 212 | 134 | 597 | 627 |
| NM_001145661 | GATA2 | -0.10 | 0.00 | 441 | 348 | 327 | 384 |
| NM_021167 | GATAD1 | -0.26 | 0.30 | 694 | 505 | 705 | 974 |
| NM_001190259 | GCSAM | -0.36 | 0.00 | 77 | 38 | 67 | 76 |
| NM_001001557 | GDF6 | -0.16 | 0.00 | 438 | 330 | 62 | 44 |
| NM_001165038 | GFRA2 | -0.36 | 0.00 | 124 | 68 | 46 | 55 |
| NM_001195087 | GGACT | -0.04 | 0.01 | 151 | 110 | 108 | 146 |
| NM_024835 | GGNBP2 | -0.04 | 0.24 | 1386 | 1232 | 1714 | 2179 |
| NM_057169 | GIT2 | -0.04 | 0.19 | 1688 | 1511 | 2406 | 2924 |
| NM_001261444 | GNAL | -0.21 | 0.00 | 174 | 114 | 351 | 386 |
| NM_001017998 | GNG10 | -0.04 | 0.00 | 323 | 258 | 655 | 695 |
| NM_001172713 | GOLGA4 | -0.42 | 0.23 | 4504 | 3197 | 3872 | 4784 |
| NM_014498 | GOLIM4 | -0.08 | 0.50 | 1160 | 994 | 1595 | 2431 |
| NM_152281 | GORAB | -0.16 | 0.26 | 208 | 145 | 183 | 274 |
| NM_001099652 | GPR137C | -0.10 | 0.00 | 50 | 27 | 67 | 85 |
| NM_020752 | GPR158 | -0.83 | 0.00 | 46 | 11 | 4 | 11 |
| NM_005282 | GPR4 | -0.56 | 0.00 | 151 | 74 | 146 | 166 |
| NM_001142966 | GREB1L | -0.08 | 0.00 | 51 | 28 | 62 | 65 |
| NM_007325 | GRIA3 | -0.24 | 0.99 | 429 | 306 | 1040 | 2257 |
| NM_001166247 | GRIK2 | -0.06 | 0.00 | 78 | 49 | 193 | 166 |
| NM_001195541 | GS1- | -0.37 | 1.22 | 66 | 31 | 35 | 126 |
| RefSeq ID | Gene Symbol | SR GFOLD | SEN GFOLD | SR IL-2 - | SR IL-2 + | SEN IL-2 - | SEN IL-2 + |
| | 259H13.2 | | | | | | |
| NM_004492 | GTF2A2 | -0.10 | 0.00 | 535 | 429 | 676 | 747 |
| NM_207118 | GTF2H5 | -0.13 | 0.00 | 547 | 429 | 799 | 841 |
| NM_004286 | GTPBP1 | -0.03 | 0.00 | 1725 | 1560 | 1963 | 1896 |
| NM_033107 | GTPBP10 | -0.03 | 0.04 | 270 | 214 | 412 | 494 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_012227 | GTPBP6 | -0.32 | 0.00 | 2625 | 1963 | 2554 | 2525 |
| NM_014170 | GTPBP8 | -0.33 | 0.00 | 194 | 118 | 229 | 240 |
| NM_006118 | HAX1 | -0.04 | 0.00 | 878 | 762 | 1504 | 1408 |
| NM_005335 | HCLS1 | -0.29 | 0.00 | 47 | 21 | 31 | 26 |
| NM_006037 | HDAC4 | -0.07 | 0.18 | 502 | 410 | 499 | 651 |
| NM_001177479 | HDX | -0.03 | 0.00 | 112 | 78 | 116 | 98 |
| NM_004667 | HERC2 | -0.11 | 0.18 | 4379 | 3858 | 6971 | 8220 |
| NM_001142853 | HES6 | -0.21 | 0.31 | 64 | 34 | 10 | 33 |
| NM_138571 | HINT3 | -0.15 | 0.29 | 171 | 117 | 356 | 510 |
| NM_005319 | HIST1H1 C | -0.11 | 0.11 | 15716 | 14224 | 8543 | 9530 |
| NM_005320 | HIST1H1 D | -0.01 | 0.00 | 2306 | 2134 | 162 | 133 |
| NM_003532 | HIST1H3 E | -0.65 | 0.75 | 794 | 440 | 52 | 128 |
| NM_003535 | HIST1H3J | -0.10 | 0.00 | 914 | 762 | 225 | 179 |
| NM_001136180 | HSBP1L1 | -0.05 | 0.00 | 126 | 88 | 223 | 257 |
| NM_025015 | HSPA12A | -0.03 | 0.17 | 1144 | 1012 | 2161 | 2599 |
| NM_021979 | HSPA2 | -0.06 | 0.00 | 615 | 514 | 372 | 388 |
| NM_001291860 | HSPG2 | -0.13 | 0.46 | 26212 | 23496 | 31269 | 43654 |
| NM_000203 | IDUA | -0.38 | 0.50 | 329 | 206 | 559 | 898 |
| NM_003897 | IER3 | -0.14 | 0.79 | 6943 | 6033 | 3138 | 5700 |
| NM_001136265 | IFFO2 | -0.05 | 0.41 | 1768 | 1576 | 1618 | 2322 |
| NM_000629 | IFNAR1 | -0.07 | 0.44 | 1096 | 942 | 1778 | 2593 |
| NM_020153 | IFT46 | -0.05 | 0.00 | 378 | 305 | 674 | 743 |
| NM_024660 | IGFLR1 | -0.12 | 0.00 | 45 | 23 | 21 | 24 |
| NM_001007189 | IGIP | -0.02 | 0.25 | 314 | 255 | 589 | 795 |
| NM_001172128 | IL16 | -0.35 | 0.00 | 129 | 72 | 150 | 120 |
| NM_001167986 | INCA1 | -0.20 | 0.00 | 99 | 59 | 100 | 124 |
| NM_001101426 | ISPD | -0.03 | 0.34 | 75 | 48 | 79 | 139 |
| NM_002207 | ITGA9 | -0.51 | 0.00 | 50 | 19 | 15 | 7 |
| NM_003724 | JRK | -0.09 | 0.00 | 845 | 706 | 940 | 887 |
| NM_002245 | KCNK1 | -0.34 | 1.06 | 158 | 92 | 60 | 177 |
| NM_004823 | KCNK6 | -0.10 | 0.00 | 700 | 575 | 528 | 558 |
| NM_199464 | KCNRG | -0.34 | 0.13 | 79 | 40 | 94 | 140 |
| NM_024076 | KCTD15 | -0.07 | 0.22 | 999 | 854 | 676 | 887 |
| NM_014663 | KDM4A | -0.07 | 0.06 | 553 | 454 | 774 | 904 |
| NM_015015 | KDM4B | -0.12 | 0.76 | 1017 | 839 | 1138 | 2096 |
| NM_001040715 | KIAA089 5L | -1.15 | 0.03 | 1032 | 407 | 547 | 640 |
| NM_007054 | KIF3A | -0.04 | 0.00 | 133 | 95 | 264 | 299 |
| NM_004984 | KIF5A | -0.43 | 0.60 | 36 | 11 | 46 | 105 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_004795 | KL | -1.21 | 0.00 | 60 | 12 | 31 | 24 |
| NM_172193 | KLHDC1 | -0.43 | 0.00 | 67 | 30 | 60 | 85 |
| NM_003482 | KMT2D | -0.27 | 0.04 | 4235 | 3338 | 4357 | 4695 |
| NM_182931 | KMT2E | -0.07 | 0.22 | 956 | 814 | 1237 | 1571 |
| NM_001012991 | KNOP1 | -0.21 | 0.00 | 429 | 312 | 393 | 423 |
| NM_002275 | KRT15 | -0.21 | 0.96 | 83 | 47 | 181 | 431 |
| NM_005560 | LAMA5 | -0.23 | 0.65 | 347 | 243 | 129 | 259 |
| NM_014017 | LAMTOR 2 | -0.14 | 0.00 | 988 | 805 | 1468 | 1440 |
| NM_006762 | LAPTM5 | -0.23 | 0.00 | 75 | 41 | 37 | 48 |
| NM_001013693 | LDLRAD 2 | -0.17 | 0.25 | 2048 | 1684 | 2498 | 3157 |
| NM_032287 | LDOC1L | -0.23 | 0.10 | 2009 | 1583 | 2078 | 2396 |
| NM_052925 | LENG8 | -0.01 | 0.00 | 1367 | 1235 | 2785 | 2898 |
| NM_022165 | LIN7B | -0.05 | 0.08 | 141 | 101 | 225 | 292 |
| NM_001292023 | LOC1001 29940 | -0.56 | 0.00 | 278 | 149 | 632 | 684 |
| NM_001278795 | LOC1009 96758 | -0.24 | 0.00 | 106 | 62 | 12 | 6 |
| NM_001291459 | LOC1010 60376 | -0.01 | 0.31 | 144 | 106 | 143 | 227 |
| NM_001162371 | LOC7283 92 | -0.07 | 0.12 | 590 | 486 | 493 | 617 |
| NM_024830 | LPCAT1 | -0.20 | 0.28 | 2873 | 2349 | 2354 | 3046 |
| NM_001008701 | LPHN1 | -0.34 | 0.00 | 282 | 178 | 227 | 227 |
| NM_001167671 | LPP | -0.10 | 0.00 | 3484 | 3072 | 6619 | 6874 |
| NM_014839 | LPPR4 | -0.21 | 0.00 | 191 | 127 | 223 | 194 |
| NM_006726 | LRBA | -0.12 | 0.17 | 912 | 747 | 1747 | 2118 |
| NM_002332 | LRP1 | -0.08 | 0.54 | 60577 | 56575 | 116461 | 171215 |
| NM_022143 | LRRC4 | -0.05 | 0.00 | 105 | 71 | 150 | 183 |
| NM_001142928 | LRRC61 | -0.56 | 0.00 | 23 | 4 | 81 | 96 |
| NM_144702 | LRRC71 | -0.38 | 0.00 | 303 | 188 | 119 | 157 |
| NM_001268284 | LRRC8E | -0.02 | 0.31 | 743 | 645 | 982 | 1342 |
| NM_001137552 | LRRFIP1 | -0.39 | 0.30 | 1023 | 697 | 1206 | 1619 |
| NM_001013615 | LURAP1 | -0.22 | 0.00 | 60 | 31 | 129 | 124 |
| NM_001198759 | LY75- CD302 | -0.23 | 0.05 | 175 | 113 | 175 | 229 |
| NM_002350 | LYN | -0.03 | 0.48 | 223 | 172 | 312 | 514 |
| NM_177476 | LYNX1 | -0.12 | 0.10 | 178 | 125 | 616 | 747 |
| NM_014628 | MAD2L1 BP | -0.19 | 0.00 | 569 | 429 | 705 | 774 |
| NM_001113482 | MANEAL | -0.40 | 0.48 | 40 | 15 | 62 | 124 |
| NM_005923 | MAP3K5 | -0.24 | 0.00 | 425 | 303 | 324 | 314 |
| NM_012326 | MAPRE3 | -0.10 | 0.20 | 186 | 133 | 657 | 852 |
| NM_002379 | MATN1 | -0.79 | 0.35 | 377 | 177 | 125 | 207 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_002380 | MATN2 | -0.33 | 0.05 | 528 | 359 | 643 | 756 |
| NM_004831 | MED26 | -0.10 | 0.02 | 387 | 303 | 412 | 488 |
| NM_017592 | MED29 | -0.02 | 0.00 | 1045 | 930 | 1770 | 1760 |
| NM_001123364 | METTL24 | -0.29 | 0.00 | 75 | 39 | 85 | 105 |
| NM_032718 | MFSD9 | -0.21 | 0.10 | 127 | 79 | 133 | 185 |
| NM_014938 | MLXIP | -0.05 | 0.40 | 2329 | 2103 | 2959 | 4140 |
| NM_001932 | MPP3 | -0.09 | 0.02 | 313 | 241 | 225 | 281 |
| NM_018000 | MREG | -0.53 | 0.99 | 109 | 51 | 106 | 274 |
| NM_014078 | MRPL13 | -0.01 | 0.00 | 537 | 459 | 734 | 675 |
| NM_023937 | MRPL34 | -0.02 | 0.00 | 481 | 407 | 626 | 680 |
| NM_016640 | MRPS30 | -0.03 | 0.00 | 575 | 488 | 674 | 682 |
| NM_001282755 | MTA3 | -0.06 | 0.16 | 387 | 312 | 717 | 898 |
| NM_021090 | MTMR3 | -0.14 | 0.40 | 1277 | 1055 | 1705 | 2421 |
| NM_015458 | MTMR9 | -0.08 | 0.00 | 879 | 740 | 1071 | 1161 |
| NM_005962 | MXI1 | -0.08 | 1.17 | 598 | 491 | 778 | 1932 |
| NM_138768 | MYEOV | -0.34 | 0.00 | 177 | 105 | 8 | 15 |
| NM_001256267 | MYPN | -0.03 | 0.52 | 603 | 514 | 630 | 1018 |
| NM_001085365 | MZT2A | -0.06 | 0.00 | 655 | 549 | 967 | 1070 |
| NM_018177 | N4BP2 | -0.49 | 0.00 | 131 | 66 | 129 | 139 |
| NM_001039703 | NBPF10 | -0.04 | 0.49 | 669 | 570 | 1042 | 1603 |
| NM_015383 | NBPF14 | -0.09 | 0.15 | 476 | 382 | 834 | 1031 |
| NM_001278267 | NBPF20 | -0.23 | 0.49 | 834 | 631 | 1190 | 1817 |
| NM_001037501 | NBPF8 | -0.17 | 0.24 | 982 | 778 | 996 | 1296 |
| NM_001277444 | NBPF9 | -0.02 | 0.14 | 905 | 799 | 1335 | 1599 |
| NM_207363 | NCKAP5 | -0.10 | 0.00 | 192 | 138 | 116 | 144 |
| NM_006312 | NCOR2 | -0.07 | 0.22 | 4358 | 3934 | 4960 | 6055 |
| NM_004541 | NDUFA1 | -0.23 | 0.04 | 667 | 495 | 888 | 1017 |
| NM_018838 | NDUFA1 2 | -0.07 | 0.00 | 715 | 599 | 990 | 980 |
| NM_019056 | NDUFB11 | -0.07 | 0.00 | 576 | 476 | 647 | 658 |
| NM_001278645 | NDUFB9 | -0.19 | 0.00 | 961 | 753 | 1928 | 1810 |
| NM_001278535 | NEURL2 | -0.74 | 0.40 | 71 | 25 | 44 | 89 |
| NM_138714 | NFAT5 | -0.08 | 0.27 | 3955 | 3550 | 3901 | 4957 |
| NM_006166 | NFYB | -0.01 | 0.20 | 195 | 150 | 106 | 163 |
| NM_002506 | NGF | -0.21 | 0.00 | 2337 | 1879 | 1135 | 1214 |
| NM_020345 | NKIRAS1 | -0.15 | 0.13 | 434 | 331 | 412 | 527 |
| NM_198175 | NME1 | -0.03 | 0.00 | 2547 | 2333 | 3716 | 3896 |
| NM_018055 | NODAL | -0.99 | 0.00 | 51 | 11 | 17 | 9 |
| NM_016167 | NOL7 | -0.10 | 0.00 | 1028 | 864 | 1285 | 1364 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_153240 | NPHP3 | -0.21 | 0.39 | 593 | 442 | 832 | 1209 |
| NM_130464 | NPIPB3 | -0.02 | 0.11 | 544 | 463 | 526 | 652 |
| NM_001278794 | NPY4R | -0.20 | 0.00 | 109 | 66 | 12 | 7 |
| NM_173474 | NTAN1 | -0.16 | 0.07 | 590 | 457 | 1092 | 1262 |
| NM_004822 | NTN1 | -0.96 | 0.08 | 67 | 19 | 6 | 22 |
| NM_024815 | NUDT18 | -0.10 | 0.00 | 302 | 230 | 289 | 303 |
| NM_032344 | NUDT22 | -0.14 | 0.00 | 476 | 367 | 626 | 566 |
| NM_019094 | NUDT4 | -0.12 | 0.13 | 1386 | 1162 | 1418 | 1691 |
| NM_001042483 | NUPR1 | -0.04 | 0.41 | 1550 | 1379 | 2785 | 3930 |
| NM_007224 | NXPH4 | -0.30 | 0.75 | 147 | 87 | 73 | 170 |
| NM_024578 | OCEL1 | -0.02 | 0.00 | 168 | 126 | 252 | 266 |
| NM_020729 | ODF2L | -0.10 | 0.42 | 309 | 235 | 345 | 542 |
| NM_017570 | OPLAH | -0.05 | 0.00 | 192 | 144 | 166 | 198 |
| NM_153451 | ORAOV1 | -0.33 | 0.00 | 486 | 328 | 522 | 527 |
| NM_022353 | OSGEPL1 | -0.21 | 0.01 | 160 | 103 | 146 | 190 |
| NM_004337 | OSGIN2 | -0.32 | 0.08 | 827 | 586 | 1048 | 1224 |
| NM_015430 | PAMR1 | -0.05 | 0.24 | 1386 | 1224 | 5906 | 7260 |
| NM_001004318 | PAPL | -1.30 | 0.00 | 74 | 16 | 10 | 2 |
| NM_004562 | PARK2 | -0.19 | 0.36 | 21 | 5 | 73 | 131 |
| NM_001127366 | PAX3 | -0.06 | 0.00 | 88 | 57 | 8 | 7 |
| NM_006196 | PCBP1 | -0.09 | 0.00 | 5487 | 4937 | 6511 | 6797 |
| NM_002600 | PDE4B | -0.14 | 0.31 | 252 | 182 | 364 | 530 |
| NM_001114107 | PDLIM3 | -0.05 | 0.00 | 219 | 167 | 329 | 375 |
| NM_015032 | PDS5B | -0.09 | 0.00 | 868 | 724 | 1031 | 930 |
| NM_003681 | PDXK | -0.02 | 0.05 | 6372 | 6019 | 6653 | 7177 |
| NM_001195263 | PDZD7 | -0.42 | 0.45 | 223 | 129 | 231 | 383 |
| NM_002567 | PEBP1 | -0.06 | 0.02 | 2519 | 2266 | 5501 | 5830 |
| NM_032177 | PHAX | -0.15 | 0.00 | 963 | 775 | 1531 | 1540 |
| NM_006224 | PITPNA | -0.10 | 0.30 | 1858 | 1604 | 2724 | 3554 |
| NM_000299 | PKP1 | -0.33 | 1.61 | 196 | 119 | 8 | 68 |
| NM_173542 | PLBD2 | -0.03 | 0.36 | 883 | 773 | 2909 | 3957 |
| NM_001130081 | PLD1 | -0.81 | 0.00 | 112 | 42 | 73 | 98 |
| NM_152666 | PLD5 | -0.36 | 0.98 | 49 | 21 | 54 | 153 |
| NM_201380 | PLEC | -0.10 | 0.60 | 12686 | 11469 | 21412 | 33088 |
| NM_015549 | PLEKHG 3 | -0.05 | 0.00 | 237 | 182 | 133 | 126 |
| NM_002668 | PLP2 | -0.01 | 0.42 | 3124 | 2912 | 4382 | 6124 |
| NM_025179 | PLXNA2 | -0.01 | 0.41 | 3653 | 3428 | 5203 | 7209 |
| NM_017514 | PLXNA3 | -0.03 | 0.73 | 2184 | 1984 | 4999 | 8625 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_002674 | PMCH | -0.40 | 0.00 | 59 | 26 | 44 | 46 |
| NM_000534 | PMS1 | -0.08 | 0.16 | 339 | 266 | 289 | 388 |
| NM_006029 | PNMA1 | -0.02 | 0.34 | 1372 | 1238 | 1868 | 2543 |
| NM_002687 | PNN | -0.06 | 0.00 | 451 | 367 | 420 | 449 |
| NM_203290 | POLR1C | -0.11 | 0.00 | 602 | 483 | 613 | 686 |
| NM_001281471 | PPIP5K2 | -0.19 | 0.00 | 908 | 709 | 1156 | 1205 |
| NM_001291310 | PPP2R2D | -0.21 | 0.00 | 1014 | 787 | 898 | 991 |
| NM_001204103 | PPT2 | -0.02 | 0.00 | 469 | 396 | 356 | 392 |
| NM_001127708 | PRG4 | -0.04 | 0.00 | 68 | 42 | 58 | 78 |
| NM_002730 | PRKACA | -0.03 | 0.09 | 1849 | 1675 | 2647 | 2989 |
| NM_175922 | PRR18 | -0.41 | 0.37 | 54 | 23 | 37 | 76 |
| NM_020200 | PRTFDC1 | -0.11 | 0.00 | 124 | 83 | 293 | 250 |
| NM_012455 | PSD4 | -0.03 | 0.18 | 520 | 437 | 499 | 649 |
| NM_021190 | PTBP2 | -0.13 | 0.00 | 164 | 113 | 216 | 196 |
| NM_004778 | PTGDR2 | -0.24 | 0.00 | 265 | 179 | 166 | 129 |
| NM_020440 | PTGFRN | -0.08 | 0.21 | 1827 | 1591 | 4571 | 5541 |
| NM_000320 | QDPR | -0.04 | 0.00 | 744 | 640 | 1630 | 1497 |
| NM_001076786 | QSER1 | -0.06 | 0.05 | 821 | 700 | 873 | 1009 |
| NM_001256619 | R3HCC1L | -0.14 | 0.42 | 414 | 315 | 306 | 484 |
| NM_022456 | RAB3IP | -0.47 | 0.25 | 152 | 80 | 79 | 131 |
| NM_006325 | RAN | -0.03 | 0.00 | 4674 | 4373 | 6364 | 6340 |
| NM_006267 | RANBP2 | -0.26 | 0.07 | 1632 | 1249 | 3059 | 3394 |
| NM_182663 | RASSF5 | -0.21 | 0.00 | 104 | 62 | 64 | 79 |
| NM_016024 | RBMX2 | -0.40 | 0.14 | 326 | 200 | 231 | 312 |
| NM_033544 | RCCD1 | -0.05 | 0.00 | 284 | 223 | 324 | 336 |
| NM_001285439 | RICTOR | -0.03 | 0.06 | 878 | 765 | 1354 | 1542 |
| NM_001000 | RPL39 | -0.05 | 0.00 | 1113 | 974 | 1485 | 1425 |
| NM_001128850 | RRAD | -0.66 | 2.39 | 47 | 15 | 54 | 396 |
| NM_016578 | RSF1 | -0.08 | 0.00 | 666 | 553 | 1086 | 1187 |
| NM_001271838 | RSRC1 | -0.15 | 0.00 | 314 | 232 | 385 | 438 |
| NM_001134888 | RTL1 | -0.37 | 0.00 | 256 | 157 | 266 | 264 |
| NM_001015051 | RUNX2 | -0.02 | 0.00 | 166 | 124 | 505 | 508 |
| NM_001031680 | RUNX3 | -0.20 | 0.67 | 188 | 125 | 69 | 153 |
| NM_001039517 | RUSC1-AS1 | -0.44 | 0.00 | 362 | 218 | 534 | 514 |
| NM_022753 | S100PBP | -0.07 | 0.00 | 566 | 465 | 566 | 604 |
| NM_001201338 | SAFB | -0.03 | 0.10 | 565 | 479 | 605 | 736 |
| NM_178448 | SAPCD2 | -0.06 | 0.00 | 171 | 125 | 100 | 76 |
| NM_001134707 | SARDH | -0.72 | 0.44 | 199 | 90 | 19 | 48 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_005505 | SCARB1 | -0.13 | 0.00 | 264 | 193 | 458 | 425 |
| NM_001037582 | SCD5 | -0.04 | 0.49 | 284 | 224 | 378 | 617 |
| NM_080489 | SDCBP2 | -0.08 | 0.42 | 103 | 68 | 100 | 179 |
| NM_001271918 | SEC11A | -0.03 | 0.03 | 985 | 866 | 1104 | 1240 |
| NM_001033578 | SGK3 | -0.10 | 0.00 | 389 | 304 | 370 | 357 |
| NM_019072 | SGTB | -0.04 | 0.41 | 1257 | 1111 | 1493 | 2152 |
| NM_015503 | SH2B1 | -0.24 | 0.09 | 1035 | 783 | 975 | 1146 |
| NM_025164 | SIK3 | -0.02 | 0.36 | 749 | 653 | 1739 | 2408 |
| NM_000617 | SLC11A2 | -0.15 | 0.17 | 705 | 556 | 1077 | 1335 |
| NM_001206951 | SLC16A3 | -0.06 | 0.75 | 9943 | 9200 | 11315 | 19558 |
| NM_020309 | SLC17A7 | -0.08 | 1.06 | 44 | 23 | 50 | 152 |
| NM_005116 | SLC23A2 | -0.05 | 0.02 | 693 | 589 | 1691 | 1848 |
| NM_052901 | SLC25A2 5 | -0.02 | 0.00 | 880 | 772 | 693 | 608 |
| NM_001151 | SLC25A4 | -0.22 | 0.67 | 2057 | 1631 | 1237 | 2137 |
| NM_006516 | SLC2A1 | -0.01 | 2.05 | 6356 | 6044 | 1329 | 5907 |
| NM_152313 | SLC36A4 | -0.06 | 0.36 | 368 | 295 | 424 | 630 |
| NM_144564 | SLC39A3 | -0.16 | 0.00 | 499 | 381 | 643 | 667 |
| NM_000341 | SLC3A1 | -0.09 | 0.07 | 149 | 104 | 335 | 416 |
| NM_005629 | SLC6A8 | -0.01 | 0.77 | 1157 | 1039 | 1462 | 2682 |
| NM_014331 | SLC7A11 | -0.07 | 1.04 | 854 | 722 | 1227 | 2728 |
| NM_004594 | SLC9A5 | -0.33 | 0.00 | 350 | 229 | 507 | 540 |
| NM_013272 | SLCO3A1 | -0.25 | 0.37 | 672 | 493 | 961 | 1372 |
| NM_014720 | SLK | -0.03 | 0.22 | 1020 | 901 | 1497 | 1896 |
| NM_001042493 | SMIM8 | -0.18 | 0.00 | 60 | 32 | 50 | 67 |
| NM_001270691 | SMOX | -0.10 | 0.73 | 664 | 540 | 549 | 1028 |
| NM_003955 | SOCS3 | -0.06 | 0.00 | 1425 | 1245 | 458 | 431 |
| NM_023016 | SOWAHC | -0.04 | 0.79 | 445 | 368 | 428 | 852 |
| NM_003112 | SP4 | -0.31 | 0.35 | 243 | 154 | 116 | 194 |
| NM_023071 | SPATS2 | -0.04 | 0.56 | 1173 | 1035 | 1352 | 2157 |
| NM_015001 | SPEN | -0.09 | 0.18 | 2365 | 2078 | 2479 | 2996 |
| NM_014230 | SRP68 | -0.11 | 0.39 | 3007 | 2624 | 2801 | 3889 |
| NM_001170760 | SRPK3 | -0.04 | 0.24 | 72 | 45 | 83 | 135 |
| NM_003769 | SRSF9 | -0.07 | 0.07 | 1723 | 1509 | 2157 | 2418 |
| RefSeq ID | Gene Symbol | SR GFOLD | SEN GFOLD | SR IL-2 - | SR IL-2 + | SEN IL-2 - | SEN IL-2 + |
| NM_198935 | SS18L1 | -0.12 | 0.58 | 456 | 355 | 289 | 512 |
| NM_006396 | SSSCA1 | -0.21 | 0.00 | 683 | 515 | 832 | 832 |
| NM_014188 | SSU72 | -0.05 | 0.00 | 2165 | 1946 | 3267 | 3318 |
| NM_006011 | ST8SIA2 | -0.05 | 0.00 | 80 | 51 | 0 | 2 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_020759 | STARD9 | -0.08 | 0.00 | 317 | 246 | 447 | 484 |
| NM_001271977 | STK25 | -0.01 | 0.10 | 3098 | 2887 | 5840 | 6529 |
| NM_003764 | STX11 | -0.16 | 0.00 | 76 | 44 | 23 | 18 |
| NM_145251 | STYX | -0.03 | 0.01 | 488 | 409 | 726 | 821 |
| NM_003169 | SUPT5H | -0.01 | 0.11 | 2766 | 2574 | 4255 | 4815 |
| NM_003170 | SUPT6H | -0.02 | 0.29 | 2072 | 1894 | 4132 | 5298 |
| NM_017673 | SWT1 | -0.15 | 0.00 | 88 | 53 | 131 | 116 |
| NM_182715 | SYPL1 | -0.16 | 0.16 | 1654 | 1362 | 2568 | 3050 |
| NM_001162951 | SYTL2 | -0.25 | 0.00 | 41 | 18 | 131 | 124 |
| NM_003487 | TAF15 | -0.19 | 0.00 | 619 | 471 | 828 | 830 |
| NM_138572 | TAF8 | -0.01 | 0.00 | 448 | 378 | 566 | 579 |
| NM_001123391 | TBC1D3 | -0.01 | 0.31 | 144 | 106 | 143 | 227 |
| NM_001001418 | TBC1D3C | -0.04 | 0.29 | 146 | 106 | 148 | 231 |
| NM_013254 | TBK1 | -0.13 | 0.00 | 801 | 647 | 975 | 1007 |
| NM_005149 | TBX19 | -0.63 | 0.64 | 74 | 29 | 56 | 126 |
| NM_003196 | TCEA3 | -0.27 | 0.09 | 267 | 177 | 724 | 867 |
| NM_001013632 | TCTEX1 D4 | -0.21 | 0.00 | 61 | 32 | 75 | 72 |
| NM_003211 | TDG | -0.03 | 0.00 | 310 | 250 | 227 | 214 |
| NM_015395 | TECPR1 | -0.04 | 0.00 | 729 | 626 | 1289 | 1390 |
| NM_006528 | TFPI2 | -0.12 | 0.03 | 18837 | 16887 | 45731 | 47463 |
| NM_013342 | TFPT | -0.15 | 0.00 | 309 | 227 | 453 | 497 |
| NM_006327 | TIMM23 | -0.32 | 0.00 | 1600 | 1171 | 1628 | 1542 |
| NM_001164407 | TLCD2 | -0.26 | 0.02 | 380 | 264 | 349 | 418 |
| NM_032027 | TM2D1 | -0.13 | 0.18 | 478 | 371 | 844 | 1067 |
| NM_001144903 | TM6SF1 | -0.44 | 0.58 | 96 | 47 | 60 | 129 |
| NM_024847 | TMC7 | -0.74 | 0.00 | 58 | 19 | 48 | 50 |
| NM_017510 | TMED9 | -0.17 | 0.61 | 10691 | 9190 | 11866 | 18591 |
| NM_178518 | TMEM10 2 | -0.24 | 0.69 | 211 | 139 | 85 | 185 |
| NM_015421 | TMEM18 6 | -0.17 | 0.00 | 220 | 153 | 210 | 200 |
| NM_014187 | TMEM20 8 | -0.10 | 0.00 | 828 | 684 | 1252 | 1229 |
| NM_001080501 | TMEM22 3 | -0.23 | 0.00 | 427 | 305 | 453 | 503 |
| NM_182614 | TMEM25 5B | -0.01 | 0.00 | 34 | 17 | 141 | 172 |
| RefSeq ID | Gene Symbol | SR GFOLD | SEN GFOLD | SR IL-2 - | SR IL-2 + | SEN IL-2 - | SEN IL-2 + |
| NM_018126 | TMEM33 | -0.20 | 0.00 | 1870 | 1501 | 1936 | 2009 |
| NM_001039770 | TMPPE | -0.30 | 0.00 | 310 | 205 | 266 | 268 |
| NM_021103 | TMSB10 | -0.20 | 0.16 | 41209 | 35245 | 46245 | 52478 |
| NM_001162501 | TNRC6B | -0.01 | 0.17 | 1842 | 1691 | 3768 | 4453 |
| NM_003291 | TPP2 | -0.05 | 0.00 | 904 | 777 | 1501 | 1582 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_016058 | TPRKB | -0.33 | 0.00 | 222 | 137 | 198 | 163 |
| NM_033502 | TRERF1 | -0.13 | 0.00 | 619 | 492 | 697 | 762 |
| NM_032765 | TRIM52 | -0.03 | 0.00 | 216 | 167 | 200 | 176 |
| NM_004239 | TRIP11 | -0.06 | 0.15 | 986 | 851 | 1190 | 1447 |
| NM_019083 | TRMT13 | -0.27 | 0.00 | 180 | 113 | 198 | 194 |
| NM_030934 | TRMT1L | -0.08 | 0.00 | 300 | 232 | 310 | 362 |
| NM_001258205 | TRPV3 | -0.24 | 0.58 | 99 | 57 | 54 | 118 |
| NM_001195131 | TSTD3 | -0.11 | 0.00 | 67 | 39 | 129 | 111 |
| NM_001135993 | TTC39C | -0.12 | 0.06 | 261 | 193 | 441 | 534 |
| NM_016078 | TVP23B | -0.12 | 0.18 | 486 | 381 | 499 | 649 |
| NM_001008224 | UACA | -0.01 | 0.19 | 2500 | 2317 | 6185 | 7357 |
| NM_030930 | UNC93B 1 | -0.21 | 0.17 | 399 | 288 | 453 | 590 |
| NM_001085372 | UQCC3 | -0.30 | 0.00 | 212 | 133 | 272 | 238 |
| NM_001089591 | UQCRHL | -0.08 | 0.00 | 1684 | 1466 | 2111 | 2203 |
| NM_000375 | UROS | -0.27 | 0.00 | 579 | 412 | 1158 | 1200 |
| NM_032747 | USMG5 | -0.04 | 0.00 | 335 | 270 | 634 | 588 |
| NM_001286767 | USP46 | -0.12 | 0.23 | 394 | 304 | 541 | 725 |
| NM_005800 | USPL1 | -0.06 | 0.00 | 465 | 381 | 422 | 458 |
| NM_016037 | UTP11L | -0.06 | 0.00 | 976 | 837 | 998 | 1035 |
| NM_007124 | UTRN | -0.03 | 0.24 | 3589 | 3322 | 7699 | 9442 |
| NM_198152 | UTS2B | -1.13 | 0.00 | 50 | 8 | 15 | 13 |
| NM_003372 | VBP1 | -0.25 | 0.00 | 695 | 512 | 907 | 878 |
| NM_001017980 | VMA21 | -0.14 | 0.00 | 1093 | 895 | 1163 | 1216 |
| NM_001163922 | VSIG10L | -0.30 | 0.43 | 119 | 68 | 112 | 198 |
| NM_016628 | WAC | -0.07 | 0.25 | 3135 | 2804 | 2914 | 3675 |
| NM_001276376 | WDR38 | -0.37 | 0.00 | 626 | 418 | 915 | 863 |
| NM_030581 | WDR59 | -0.19 | 0.17 | 656 | 500 | 824 | 1030 |
| NM_145294 | WDR90 | -0.03 | 0.31 | 267 | 211 | 208 | 318 |
| NM_194293 | XIRP1 | -0.11 | 0.00 | 501 | 397 | 358 | 401 |
| NM_024029 | YIPF2 | -0.02 | 0.02 | 1419 | 1278 | 1720 | 1885 |
| NM_032312 | YIPF4 | -0.13 | 0.35 | 412 | 317 | 482 | 704 |
| NM_001276320 | YOD1 | -0.16 | 0.00 | 523 | 401 | 485 | 525 |
| NM_145008 | YPEL4 | -0.95 | 0.00 | 89 | 28 | 29 | 48 |
| NM_180990 | ZACN | -0.17 | 0.00 | 585 | 448 | 1142 | 1168 |
| NM_001164343 | ZBTB20 | -0.10 | 0.00 | 715 | 587 | 1612 | 1538 |
| **Table 5C: Differentially Downregulated Genes in SR + IL-2** | | | | | | | |
| RefSeq ID | Gene Symbol | SR GFOLD | SEN GFOLD | SR IL-2 - | SR IL-2 + | SEN IL-2 - | SEN IL-2 + |
| NM_006977 | ZBTB25 | -0.02 | 0.26 | 110 | 77 | 154 | 235 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_015070 | ZC3H13 | -0.25 | 0.00 | 928 | 696 | 1474 | 1494 |
| NM_016353 | ZDHHC2 | -0.03 | 0.39 | 1262 | 1122 | 1312 | 1874 |
| NM_182491 | ZFAND2 A | -0.01 | 0.43 | 505 | 430 | 553 | 848 |
| NM_006885 | ZFHX3 | -0.11 | 0.30 | 2389 | 2061 | 2092 | 2760 |
| NM_020917 | ZFP14 | -0.55 | 0.00 | 65 | 26 | 83 | 87 |
| NM_031449 | ZMIZ2 | -0.21 | 0.60 | 1482 | 1171 | 1581 | 2575 |
| NM_001195156 | ZMYM6N B | -0.27 | 0.06 | 587 | 419 | 975 | 1122 |
| NM_138462 | ZMYND1 9 | -0.17 | 0.00 | 1017 | 810 | 670 | 693 |
| NM_015394 | ZNF10 | -0.74 | 0.00 | 71 | 25 | 92 | 126 |
| NM_015852 | ZNF117 | -0.24 | 0.10 | 106 | 62 | 191 | 255 |
| NM_003441 | ZNF141 | -0.48 | 0.00 | 104 | 50 | 81 | 100 |
| NM_006991 | ZNF197 | -0.06 | 0.00 | 178 | 131 | 239 | 233 |
| NM_182976 | ZNF326 | -0.27 | 0.00 | 222 | 144 | 320 | 316 |
| NM_006955 | ZNF33B | -0.09 | 0.00 | 115 | 77 | 141 | 159 |
| NM_001256648 | ZNF43 | -0.35 | 0.00 | 138 | 78 | 94 | 85 |
| NM_001143769 | ZNF438 | -0.08 | 0.03 | 189 | 138 | 225 | 283 |
| NM_198458 | ZNF497 | -0.10 | 0.26 | 202 | 146 | 393 | 549 |
| NM_032772 | ZNF503 | -0.22 | 0.19 | 273 | 188 | 867 | 1096 |
| NM_053042 | ZNF518B | -0.05 | 0.40 | 940 | 815 | 1044 | 1514 |
| NM_001277090 | ZNF550 | -0.04 | 0.29 | 160 | 117 | 160 | 248 |
| NM_001204817 | ZNF587 | -0.05 | 0.39 | 523 | 434 | 607 | 896 |
| NM_001286054 | ZNF595 | -0.06 | 0.00 | 159 | 115 | 164 | 198 |
| NM_178549 | ZNF678 | -0.62 | 0.00 | 97 | 41 | 83 | 113 |
| NM_021916 | ZNF70 | -0.16 | 0.00 | 308 | 225 | 285 | 344 |
| NM_182515 | ZNF714 | -0.53 | 0.03 | 249 | 135 | 125 | 168 |
| NM_007131 | ZNF75D | -0.26 | 0.00 | 233 | 153 | 308 | 292 |
| NM_001004309 | ZNF774 | -0.07 | 0.00 | 60 | 35 | 67 | 68 |
| NM_001004301 | ZNF813 | -0.10 | 0.00 | 115 | 76 | 148 | 128 |
| NM_001201552 | ZNF821 | -0.23 | 0.00 | 133 | 82 | 154 | 172 |
| NM_031218 | ZNF93 | -0.02 | 0.00 | 109 | 76 | 79 | 87 |
| NM_001206998 | ZNRF3 | -0.32 | 0.00 | 163 | 97 | 243 | 268 |
| NM_001112734 | ZSCAN30 | -0.25 | 0.00 | 182 | 116 | 354 | 342 |

**Table 5D: Differentially Downregulated Genes in SEN + IL-2**

| Table 5D: Differentially Downregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_153698 | AAED1 | 0.01 | -0.20 | 185 | 234 | 487 | 360 |
| NM_020745 | AARS2 | 0.06 | -0.07 | 476 | 575 | 736 | 616 |
| NM_001261434 | AARSD1 | 0.16 | -0.03 | 274 | 369 | 518 | 436 |
| NM_001271696 | ABCB7 | 0.12 | -0.02 | 153 | 212 | 406 | 338 |
| NM_019625 | ABCB9 | 0.00 | -0.32 | 161 | 167 | 279 | 179 |
| NM_020297 | ABCC9 | 0.23 | -0.22 | 721 | 949 | 2161 | 1723 |
| NM_001040876 | ABCE1 | 0.32 | -0.05 | 852 | 1178 | 1312 | 1152 |
| NM_001025780 | ABHD17B | 0.00 | -0.15 | 333 | 340 | 659 | 518 |
| NM_013375 | ABT1 | 0.00 | -0.02 | 750 | 674 | 682 | 591 |
| NM_000017 | ACADS | 0.00 | -0.22 | 162 | 205 | 487 | 355 |
| NM_016557 | ACKR4 | 0.00 | -1.33 | 35 | 38 | 235 | 68 |
| NM_020186 | ACN9 | 0.00 | -0.12 | 140 | 108 | 177 | 124 |
| NM_001037161 | ACOT1 | 0.00 | -0.02 | 153 | 162 | 289 | 233 |
| NM_001037171 | ACOT9 | 0.08 | -0.09 | 949 | 1114 | 1897 | 1645 |
| NM_001111034 | ACP5 | 0.00 | -0.44 | 0 | 9 | 146 | 78 |
| NM_001100 | ACTA1 | 0.31 | -0.15 | 91 | 153 | 108 | 68 |
| NM_001141945 | ACTA2 | 0.28 | -0.30 | 3308 | 4240 | 4529 | 3486 |
| NM_005159 | ACTC1 | 0.52 | -0.29 | 1327 | 2069 | 807 | 582 |
| NM_001199954 | ACTG1 | 0.15 | -0.20 | 124973 | 139507 | 229718 | 199115 |
| NM_005736 | ACTR1A | 0.38 | -0.10 | 2851 | 3921 | 8462 | 7590 |
| NM_000666 | ACY1 | 0.25 | -0.06 | 258 | 371 | 1171 | 1017 |

(continued)

| Table 5D: Differentially Downregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_203488 | ACYP1 | 0.16 | -0.15 | 55 | 91 | 133 | 87 |
| NM_001282447 | ADAM33 | 0.00 | -0.50 | 201 | 193 | 520 | 311 |
| NM_014244 | ADAMTS2 | 0.09 | -0.06 | 10108 | 11082 | 9512 | 8822 |
| NM_014243 | ADAMTS3 | 0.23 | -0.10 | 40 | 74 | 31 | 13 |
| NM_004036 | ADCY3 | 0.00 | -0.07 | 1641 | 1537 | 1999 | 1771 |
| NM_000671 | ADH5 | 0.52 | -0.23 | 1369 | 2133 | 3629 | 2924 |
| NM_000026 | ADSL | 0.07 | -0.14 | 873 | 1019 | 1545 | 1288 |
| NM_022767 | AEN | 0.13 | -0.17 | 1828 | 2156 | 2358 | 1946 |
| NM_006796 | AFG3L2 | 0.60 | -0.12 | 965 | 1606 | 2423 | 2081 |
| NM_018238 | AGK | 0.28 | -0.03 | 238 | 351 | 551 | 466 |
| NM_012199 | AGO1 | 0.00 | -0.05 | 1025 | 1124 | 2009 | 1795 |
| NM_000687 | AHCY | 0.03 | -0.15 | 1941 | 2136 | 3261 | 2765 |
| NM_020731 | AHRR | 0.12 | -0.27 | 687 | 842 | 1572 | 1192 |
| NM_018836 | AJAP1 | 0.00 | -0.90 | 754 | 729 | 873 | 408 |
| NM_000476 | AK1 | 0.00 | -0.11 | 922 | 1003 | 2805 | 2442 |
| NM_174858 | AK5 | 0.35 | -0.61 | 837 | 1181 | 4461 | 2767 |
| NM_005100 | AKAP12 | 0.08 | -0.03 | 2308 | 2601 | 5209 | 4882 |
| NM_001198656 | AKAP2 | 0.24 | -0.16 | 8623 | 10504 | 23286 | 20445 |
| NM_001353 | AKR1C1 | 0.43 | -0.40 | 11991 | 16663 | 17729 | 13053 |
| NM_001354 | AKR1C2 | 0.27 | -0.18 | 6380 | 7987 | 7310 | 6198 |
| NM_000031 | ALAD | 0.17 | -0.02 | 404 | 530 | 1622 | 1473 |

(continued)

| Table 5D: Differentially Downregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_153329 | ALDH16A1 | 0.00 | -0.02 | 213 | 182 | 297 | 240 |
| NM_000693 | ALDH1A3 | 0.16 | -0.69 | 502 | 645 | 4477 | 2617 |
| NM_000692 | ALDH1B1 | 0.44 | -0.42 | 1656 | 2418 | 2957 | 2067 |
| NM_000694 | ALDH3B1 | 0.21 | -0.18 | 365 | 494 | 1870 | 1523 |
| NM_001007027 | ALG8 | 0.26 | -0.02 | 313 | 444 | 886 | 780 |
| NM_052947 | ALPK2 | 0.16 | -0.16 | 3052 | 3605 | 4188 | 3558 |
| NM_000478 | ALPL | 0.00 | -0.28 | 53 | 64 | 37 | 15 |
| NM_005782 | ALYREF | 0.15 | -0.12 | 1078 | 1312 | 1524 | 1285 |
| NM_001267782 | AMBRA1 | 0.32 | -0.08 | 686 | 959 | 1574 | 1370 |
| NM_001634 | AMD1 | 0.27 | -0.15 | 1591 | 2068 | 2543 | 2144 |
| NM_016201 | AMOTL2 | 0.26 | -0.51 | 7839 | 9743 | 6636 | 4447 |
| NM_022662 | ANAPC1 | 0.15 | -0.04 | 985 | 1211 | 1460 | 1298 |
| NM_001256708 | ANAPC10 | 0.00 | -0.23 | 130 | 129 | 277 | 190 |
| NM_001242374 | ANAPC13 | 0.55 | -0.08 | 611 | 1010 | 1291 | 1107 |
| NM_013366 | ANAPC2 | 0.11 | -0.06 | 740 | 899 | 1674 | 1481 |
| NM_016238 | ANAPC7 | 0.31 | -0.23 | 812 | 1118 | 1999 | 1577 |
| NM_000037 | ANK1 | 0.00 | -0.10 | 49 | 53 | 137 | 94 |
| NM_030816 | ANKRD13C | 0.28 | -0.11 | 565 | 779 | 1029 | 854 |
| NM_173505 | ANKRD29 | 0.00 | -0.54 | 0 | 4 | 416 | 237 |
| NM_001164440 | ANKRD33B | 0.00 | -0.07 | 318 | 358 | 591 | 490 |
| NM_001039888 | ANKRD34A | 0.00 | -0.31 | 97 | 110 | 366 | 244 |

(continued)

| Table 5D: Differentially Downregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_016466 | ANKRD39 | 0.00 | -0.10 | 230 | 271 | 474 | 377 |
| NM_020337 | ANKRD50 | 0.13 | -0.05 | 1241 | 1488 | 2344 | 2116 |
| NM_173595 | ANKRD52 | 0.05 | -0.03 | 5569 | 6021 | 6208 | 5828 |
| NM_018685 | ANLN | 0.15 | -1.06 | 3260 | 3819 | 3309 | 1477 |
| NM_001286615 | ANO4 | 0.09 | -0.55 | 112 | 159 | 179 | 91 |
| NM_006401 | ANP32B | 0.18 | -0.05 | 759 | 961 | 1002 | 869 |
| NM_007193 | ANXA10 | 0.33 | -0.10 | 3 | 17 | 158 | 111 |
| NM_145869 | ANXA11 | 0.02 | -0.02 | 4548 | 4854 | 8306 | 7896 |
| NM_001159 | AOX1 | 0.00 | -0.11 | 1056 | 1029 | 2053 | 1765 |
| NM_004069 | AP2S1 | 0.46 | -0.17 | 1784 | 2636 | 5133 | 4338 |
| NM_004722 | AP4M1 | 0.32 | -0.02 | 195 | 302 | 732 | 638 |
| NM_001640 | APEH | 0.00 | -0.10 | 1762 | 1878 | 3897 | 3438 |
| NM_001244249 | APEX1 | 0.00 | -0.25 | 2375 | 2378 | 4756 | 3815 |
| NM_014481 | APEX2 | 0.26 | -0.07 | 498 | 682 | 705 | 591 |
| NM_144772 | APOA1BP | 0.00 | -0.03 | 593 | 621 | 1398 | 1250 |
| NM_004900 | APOBEC3B | 0.00 | -0.54 | 249 | 236 | 297 | 163 |
| NM_014508 | APOBEC3C | 0.44 | -0.29 | 317 | 509 | 1408 | 1050 |
| NM_152426 | APOBEC3D | 0.00 | -0.25 | 66 | 76 | 108 | 63 |
| NM_001136540 | APOL1 | 0.33 | -0.35 | 246 | 374 | 713 | 488 |
| NM_030882 | APOL2 | 0.01 | -0.03 | 998 | 1114 | 1728 | 1560 |
| NM_001195249 | APTX | 0.00 | -0.04 | 371 | 416 | 759 | 652 |

(continued)

| | | | | | | | |
|---|---|---|---|---|---|---|---|
| **Table 5D: Differentially Downregulated Genes in SEN + IL-2** | | | | | | | |
| RefSeq ID | Gene Symbol | SR GFOLD | SEN GFOLD | SR IL-2 - | SR IL-2 + | SEN IL-2 - | SEN IL-2 + |
| NM_018011 | ARGLU1 | 0.36 | -0.06 | 648 | 938 | 1169 | 1017 |
| NM_004308 | ARHGAP1 | 0.18 | -0.06 | 4080 | 4872 | 9533 | 8859 |
| NM_014783 | ARHGAP11 A | 0.00 | -0.53 | 1030 | 1081 | 1142 | 710 |
| NM_001199417 | ARHGAP23 | 0.00 | -0.07 | 3425 | 3226 | 3086 | 2758 |
| NM_020754 | ARHGAP31 | 0.33 | -0.29 | 2208 | 2953 | 4527 | 3510 |
| NM_004309 | ARHGDIA | 0.21 | -0.09 | 7926 | 9529 | 12881 | 11758 |
| NM_005224 | ARID3A | 0.00 | -0.34 | 238 | 287 | 327 | 211 |
| NM_005737 | ARL4C | 0.00 | -0.13 | 588 | 542 | 1547 | 1298 |
| NM_015161 | ARL6IP1 | 0.23 | -0.14 | 1327 | 1696 | 2084 | 1760 |
| NM_001290020 | ARMC4 | 0.00 | -0.55 | 13 | 26 | 225 | 118 |
| NM_001199196 | ARMC6 | 0.60 | -0.33 | 506 | 873 | 1404 | 1018 |
| NM_001271466 | ARMC9 | 0.09 | -0.45 | 627 | 757 | 3232 | 2220 |
| NM_016608 | ARMCX1 | 0.18 | -0.02 | 570 | 735 | 1160 | 1037 |
| NM_014862 | ARNT2 | 0.00 | -0.56 | 594 | 583 | 482 | 274 |
| NM_005718 | ARPC4 | 0.17 | -0.24 | 2515 | 3019 | 6199 | 5029 |
| NM_030978 | ARPC5L | 0.17 | -0.04 | 871 | 1087 | 1753 | 1569 |
| NM_182616 | ARPIN | 0.29 | -0.09 | 529 | 737 | 1248 | 1067 |
| NM_001247996 | ASAP1 | 0.21 | -0.05 | 3120 | 3808 | 6549 | 6061 |
| NM_017873 | ASB6 | 0.12 | -0.07 | 1758 | 2067 | 3271 | 2928 |
| NM_018154 | ASF1B | 0.00 | -0.28 | 642 | 675 | 362 | 246 |
| NM_001095 | ASIC1 | 0.65 | -0.20 | 35 | 87 | 227 | 155 |

(continued)

| Table 5D: Differentially Downregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_004317 | ASNA1 | 0.15 | -0.17 | 1310 | 1586 | 3030 | 2521 |
| NM_018136 | ASPM | 0.00 | -0.98 | 1399 | 1311 | 1318 | 597 |
| NM_018164 | ASUN | 0.17 | -0.04 | 432 | 562 | 925 | 802 |
| NM_014109 | ATAD2 | 0.00 | -0.15 | 2002 | 1911 | 1146 | 935 |
| NM_024857 | ATAD5 | 0.02 | -0.20 | 230 | 287 | 125 | 78 |
| NM_025092 | ATHL1 | 0.00 | -0.16 | 112 | 103 | 283 | 205 |
| NM_001940 | ATN1 | 0.00 | -0.07 | 3451 | 3524 | 3402 | 3068 |
| NM_001257335 | ATP5A1 | 0.33 | -0.34 | 3012 | 4000 | 7262 | 5506 |
| NM_001686 | ATP5B | 0.21 | -0.06 | 10702 | 12780 | 23492 | 21985 |
| NM_001001973 | ATP5C1 | 0.00 | -0.19 | 1506 | 1636 | 2851 | 2335 |
| NM_001688 | ATP5F1 | 0.00 | -0.01 | 865 | 851 | 1953 | 1793 |
| NM_005175 | ATP5G1 | 0.00 | -0.80 | 303 | 337 | 796 | 396 |
| NM_001190329 | ATP5G3 | 0.56 | -0.32 | 1599 | 2539 | 4750 | 3614 |
| NM_006356 | ATP5H | 0.32 | -0.14 | 422 | 611 | 1458 | 1205 |
| NM_007100 | ATP5I | 0.30 | -0.13 | 656 | 910 | 1635 | 1372 |
| NM_018035 | ATP5SL | 0.41 | -0.09 | 561 | 846 | 1364 | 1172 |
| NM_001198909 | ATP6V1F | 0.04 | -0.28 | 1068 | 1207 | 2660 | 2041 |
| NM_005603 | ATP8B1 | 0.14 | -0.11 | 3192 | 3725 | 4369 | 3841 |
| NM_198433 | AURKA | 0.00 | -0.27 | 563 | 596 | 670 | 482 |
| NM_001127229 | AURKAIP1 | 0.00 | -0.31 | 1228 | 1333 | 2367 | 1774 |
| NM_001284526 | AURKB | 0.00 | -0.66 | 776 | 825 | 501 | 266 |

(continued)

| Table 5D: Differentially Downregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_021913 | AXL | 0.26 | -0.30 | 24844 | 30431 | 25272 | 20136 |
| NM_003783 | B3GALT2 | 0.00 | -0.10 | 67 | 74 | 31 | 13 |
| NM_001199873 | B4GALT3 | 0.00 | -0.25 | 1091 | 1169 | 1266 | 963 |
| NM_004282 | BAG2 | 0.03 | -0.30 | 802 | 916 | 1375 | 1013 |
| NM_025045 | BAIAP2L2 | 0.00 | -0.13 | 54 | 65 | 337 | 253 |
| NM_001188 | BAK1 | 0.30 | -0.15 | 395 | 566 | 747 | 591 |
| NM_003860 | BANF1 | 0.17 | -0.27 | 1226 | 1505 | 2250 | 1737 |
| NM_006317 | BASP1 | 0.46 | -0.03 | 3439 | 4992 | 5336 | 4993 |
| NM_001127240 | BBC3 | 0.00 | -0.24 | 32 | 48 | 96 | 55 |
| NM_003567 | BCAR3 | 0.15 | -0.06 | 3909 | 4555 | 6958 | 6407 |
| NM_005872 | BCAS2 | 0.00 | -0.05 | 238 | 290 | 495 | 408 |
| NM_000055 | BCHE | 0.00 | -0.13 | 41 | 64 | 378 | 288 |
| NM_004765 | BCL7C | 0.29 | -0.04 | 232 | 345 | 668 | 567 |
| NM_004328 | BCS1L | 0.00 | -0.14 | 362 | 407 | 693 | 551 |
| NM_020139 | BDH2 | 1.24 | -0.13 | 19 | 80 | 200 | 142 |
| NM_170733 | BDNF | 0.00 | -0.30 | 3030 | 2961 | 3072 | 2346 |
| NM_001003800 | BICD2 | 0.33 | -0.22 | 2183 | 2932 | 5180 | 4253 |
| NM_001012271 | BIRC5 | 0.00 | -1.15 | 1350 | 1475 | 1637 | 664 |
| NM_001253823 | BLVRA | 0.16 | -0.11 | 454 | 586 | 1341 | 1129 |
| NM_000713 | BLVRB | 0.00 | -0.47 | 1026 | 1069 | 1628 | 1074 |
| NM_017637 | BNC2 | 0.00 | -0.02 | 898 | 895 | 1445 | 1303 |

(continued)

| Table 5D: Differentially Downregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_016074 | BOLA1 | 0.00 | -0.30 | 118 | 115 | 175 | 107 |
| NM_001031827 | BOLA2 | 0.00 | -0.06 | 909 | 927 | 1472 | 1290 |
| NM_001039182 | BOLA2B | 0.00 | -0.06 | 909 | 927 | 1472 | 1290 |
| NM_001286746 | BORA | 0.06 | -0.24 | 94 | 134 | 146 | 91 |
| NM_006085 | BPNT1 | 0.85 | -0.05 | 291 | 617 | 728 | 621 |
| NM_152743 | BRAT1 | 0.47 | -0.16 | 387 | 622 | 1329 | 1081 |
| NM_000059 | BRCA2 | 0.00 | -0.72 | 438 | 377 | 316 | 153 |
| NM_001173984 | BRD7 | 0.00 | -0.11 | 279 | 312 | 470 | 370 |
| NM_032043 | BRIP1 | 0.04 | -0.24 | 483 | 575 | 275 | 187 |
| NM_001197247 | BTN3A2 | 0.00 | -0.19 | 373 | 397 | 765 | 591 |
| NM_004336 | BUB1 | 0.07 | -1.05 | 926 | 1081 | 911 | 383 |
| NM_001211 | BUB1B | 0.03 | -0.98 | 602 | 698 | 514 | 216 |
| NM_004725 | BUB3 | 0.25 | -0.13 | 3362 | 4208 | 4245 | 3676 |
| NM_032725 | BUD13 | 0.00 | -0.08 | 410 | 418 | 426 | 340 |
| NM_001159767 | BZW2 | 0.67 | -0.11 | 583 | 1049 | 1348 | 1137 |
| NM_001163812 | C10orf2 | -0.01 | -0.07 | 442 | 373 | 393 | 314 |
| NM_170746 | C11orf31 | 0.00 | -0.40 | 790 | 782 | 2248 | 1577 |
| NM_024099 | C11orf48 | 0.00 | -0.15 | 508 | 564 | 809 | 643 |
| NM_001135635 | C11orf68 | 0.05 | -0.06 | 1741 | 1941 | 2379 | 2124 |
| NM_152318 | C12orf45 | 0.31 | -0.38 | 79 | 136 | 256 | 155 |
| NM_001145199 | C12orf75 | 0.00 | -0.22 | 1155 | 1156 | 2034 | 1621 |

(continued)

| Table 5D: Differentially Downregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_001102366 | C14orf159 | 0.22 | -0.09 | 325 | 447 | 1275 | 1089 |
| NM_001127393 | C14orf2 | 0.00 | -0.02 | 573 | 599 | 1056 | 939 |
| NM_025108 | C16orf59 | 0.00 | -0.30 | 127 | 127 | 83 | 44 |
| NM_001171251 | C17orf53 | 0.00 | -0.73 | 276 | 253 | 302 | 144 |
| NM_017941 | C17orf80 | 0.01 | -0.06 | 241 | 297 | 418 | 338 |
| NM_001086521 | C17orf89 | 0.00 | -0.27 | 265 | 262 | 697 | 503 |
| NM_001290149 | C19orf48 | 0.21 | -0.16 | 687 | 895 | 510 | 390 |
| NM_014047 | C19orf53 | 0.00 | -0.03 | 1591 | 1701 | 3367 | 3105 |
| NM_205767 | C19orf70 | 0.38 | -0.34 | 248 | 390 | 923 | 647 |
| NM_018186 | C1orf112 | 0.00 | -0.09 | 163 | 198 | 202 | 148 |
| NM_152485 | C1orf74 | 0.00 | -0.01 | 71 | 79 | 139 | 102 |
| NM_001212 | C1QBP | 0.02 | -0.16 | 1402 | 1552 | 1888 | 1558 |
| NM_015645 | C1QTNF5 | 0.00 | -0.35 | 469 | 461 | 1221 | 867 |
| NM_001733 | C1R | 0.00 | -0.50 | 739 | 693 | 613 | 373 |
| NM_016546 | C1RL | 0.00 | -0.17 | 174 | 138 | 618 | 477 |
| NM_001258430 | C20orf27 | 0.00 | -0.25 | 634 | 646 | 1543 | 1187 |
| NM_001143960 | C2orf74 | 0.00 | -0.08 | 111 | 145 | 310 | 240 |
| NM_024616 | C3orf52 | 0.00 | -0.08 | 224 | 242 | 312 | 242 |
| NM_017867 | C4orf27 | 0.00 | -0.29 | 159 | 196 | 297 | 196 |
| NM_001735 | C5 | 0.00 | -0.03 | 44 | 33 | 112 | 78 |
| NM_198566 | C5orf34 | 0.00 | -0.22 | 95 | 117 | 79 | 44 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| Table 5D: Differentially Downregulated Genes in SEN + IL-2 | | | | | | | |
| NM_ 138464 | C5orf55 | 0.00 | -0.43 | 68 | 58 | 94 | 46 |
| NM_ 178508 | C6orf1 | 0.20 | -0.35 | 529 | 693 | 1647 | 1185 |
| NM_ 001029863 | C6orf120 | 0.00 | -0.05 | 594 | 612 | 1094 | 950 |
| NM_ 177965 | C8orf37 | 0.04 | -0.45 | 26 | 47 | 92 | 44 |
| NM_ 001190972 | C8orf88 | 0.00 | -0.29 | 147 | 173 | 127 | 74 |
| NM_ 016390 | C9orf114 | 0.07 | -0.01 | 1047 | 1211 | 1720 | 1571 |
| NM_ 001048265 | C9orf116 | 0.00 | -0.18 | 12 | 19 | 106 | 65 |
| NM_ 017998 | C9orf40 | 0.17 | -0.55 | 195 | 272 | 314 | 172 |
| NM_ 032307 | C9orf64 | 0.32 | -0.06 | 335 | 494 | 603 | 501 |
| NM_ 182505 | C9orf85 | 0.00 | -0.01 | 100 | 98 | 158 | 118 |
| NM_ 001100619 | CABLES 1 | 0.00 | -0.13 | 1496 | 1589 | 1759 | 1484 |
| NM_ 182527 | CABP7 | 0.00 | -0.07 | 79 | 89 | 220 | 165 |
| NM_ 000723 | CACNB1 | 0.00 | -0.10 | 179 | 154 | 368 | 285 |
| NM_ 001080543 | CACTIN | 0.19 | -0.11 | 397 | 528 | 595 | 479 |
| NM_ 004341 | CAD | 0.07 | -0.07 | 2925 | 3251 | 3519 | 3159 |
| NM_ 183394 | CADPS | 0.00 | -0.26 | 168 | 211 | 1955 | 1508 |
| NM_ 033138 | CALD1 | 0.29 | -0.03 | 11161 | 14028 | 19721 | 18841 |
| NM_ 005184 | CALM3 | 0.11 | -0.15 | 6502 | 7321 | 12973 | 11353 |
| NM_ 020439 | CAMK1G | 0.00 | -0.63 | 11 | 15 | 148 | 68 |
| NM_ 024766 | CAMKMT | 0.00 | -0.13 | 28 | 33 | 121 | 79 |
| NM_ 014550 | CARD10 | 0.01 | -0.39 | 206 | 257 | 162 | 91 |

(continued)

| Table 5D: Differentially Downregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_024537 | CARS2 | 0.13 | -0.25 | 1424 | 1696 | 3508 | 2782 |
| NM_144508 | CASC5 | 0.15 | -0.86 | 529 | 674 | 670 | 314 |
| NM_001080125 | CASP8 | 0.47 | -0.04 | 86 | 162 | 272 | 214 |
| NM_014292 | CBX6 | 0.18 | -0.06 | 1274 | 1571 | 2851 | 2566 |
| NM_001002880 | CBY1 | 0.00 | -0.04 | 320 | 368 | 564 | 475 |
| NM_021174 | CCAR2 | 0.11 | -0.13 | 2194 | 2538 | 3438 | 2972 |
| NM_133459 | CCBE1 | 0.25 | -0.34 | 2421 | 3064 | 4280 | 3203 |
| NM_001287390 | CCBL1 | 0.00 | -0.16 | 229 | 267 | 586 | 453 |
| NM_025004 | CCDC15 | 0.03 | -0.42 | 114 | 155 | 146 | 79 |
| NM_138493 | CCDC167 | 0.00 | -0.04 | 136 | 151 | 401 | 327 |
| NM_152499 | CCDC24 | 0.00 | -0.19 | 36 | 36 | 225 | 155 |
| NM_024296 | CCDC28B | 0.23 | -0.33 | 79 | 129 | 160 | 94 |
| NM_175884 | CCDC71L | 0.06 | -0.15 | 508 | 607 | 1117 | 909 |
| NM_001080433 | CCDC85A | 0.50 | -0.58 | 335 | 558 | 603 | 346 |
| NM_006848 | CCDC85B | 0.42 | -0.18 | 669 | 1004 | 965 | 762 |
| NM_024098 | CCDC86 | 0.08 | -0.21 | 1184 | 1371 | 1310 | 1031 |
| NM_001135597 | CCDC88A | 0.03 | -0.06 | 1271 | 1424 | 2379 | 2122 |
| NM_018074 | CCDC94 | 0.29 | -0.02 | 239 | 354 | 435 | 364 |
| NM_001105564 | CCHCR1 | 0.38 | -0.08 | 500 | 743 | 988 | 837 |
| NM_002982 | CCL2 | 0.15 | -0.23 | 98 | 147 | 160 | 102 |
| NM_001237 | CCNA2 | 0.28 | -0.80 | 898 | 1208 | 778 | 388 |

(continued)

| Table 5D: Differentially Downregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_031966 | CCNB1 | 0.24 | -0.93 | 1156 | 1499 | 1849 | 880 |
| NM_004701 | CCNB2 | 0.20 | -0.57 | 355 | 481 | 489 | 277 |
| NM_001287427 | CCND3 | 0.00 | -0.41 | 1606 | 1704 | 2564 | 1797 |
| NM_001134375 | CCNJ | 0.00 | -0.09 | 175 | 138 | 237 | 177 |
| NM_005125 | CCS | 0.00 | -0.08 | 383 | 349 | 863 | 725 |
| NM_006431 | CCT2 | 0.15 | -0.02 | 2352 | 2781 | 3571 | 3327 |
| RefSeq ID | Gene Symbol | SR GFOLD | SEN GFOLD | SR IL-2 - | SR IL-2 + | SEN IL-2- | SEN IL-2 + |
| NM_006430 | CCT4 | 0.21 | -0.03 | 3016 | 3695 | 5648 | 5292 |
| NM_012073 | CCT5 | 0.36 | -0.26 | 4545 | 6112 | 6971 | 5599 |
| NM_001762 | CCT6A | 0.32 | -0.05 | 1760 | 2369 | 3232 | 2937 |
| NM_001166285 | CCT7 | 0.19 | -0.08 | 3521 | 4249 | 7420 | 6747 |
| NM_133493 | CD109 | 0.03 | -0.05 | 1845 | 2033 | 8131 | 7582 |
| NM_020404 | CD248 | 0.02 | -0.68 | 5111 | 5422 | 9229 | 5532 |
| NM_006110 | CD2BP2 | 0.10 | -0.08 | 1254 | 1468 | 2123 | 1871 |
| NM_033331 | CDC14B | 0.00 | -0.17 | 420 | 462 | 936 | 741 |
| NM_001255 | CDC20 | 0.11 | -1.31 | 886 | 1061 | 786 | 270 |
| NM_021873 | CDC25B | 0.00 | -0.40 | 1510 | 1513 | 1597 | 1109 |
| NM_001287582 | CDC25C | 0.00 | -0.56 | 54 | 69 | 139 | 67 |
| NM_001039802 | CDC42 | 0.22 | -0.02 | 2680 | 3308 | 5632 | 5301 |
| NM_152243 | CDC42EP1 | 0.36 | -0.17 | 1763 | 2434 | 2699 | 2238 |
| NM_006449 | CDC42EP3 | 0.32 | -0.14 | 1544 | 2083 | 3261 | 2780 |

(continued)

| RefSeq ID | Gene Symbol | SR GFOLD | SEN GFOLD | SR IL-2 - | SR IL-2 + | SEN IL-2- | SEN IL-2 + |
|---|---|---|---|---|---|---|---|
| NM_001178010 | CDC45 | 0.00 | -0.10 | 427 | 497 | 191 | 137 |
| NM_152562 | CDCA2 | 0.36 | -0.21 | 336 | 508 | 626 | 471 |
| NM_031299 | CDCA3 | 0.19 | -0.91 | 344 | 463 | 445 | 194 |
| NM_080668 | CDCA5 | 0.10 | -0.72 | 803 | 965 | 753 | 397 |
| NM_031942 | CDCA7 | 0.00 | -0.29 | 144 | 182 | 25 | 6 |
| NM_001127370 | CDCA7L | 0.23 | -0.10 | 697 | 921 | 370 | 287 |
| NM_001256875 | CDCA8 | 0.00 | -0.89 | 849 | 826 | 645 | 296 |
| NM_022478 | CDH24 | 0.00 | -0.07 | 228 | 237 | 391 | 312 |
| NM_001786 | CDK1 | 0.08 | -0.42 | 349 | 437 | 314 | 189 |
| NM_001798 | CDK2 | 0.30 | -0.34 | 1220 | 1639 | 1040 | 734 |
| NM_000075 | CDK4 | 0.28 | -0.29 | 1626 | 2130 | 3438 | 2643 |
| NM_001278167 | CDK5RAP1 | 0.46 | -0.03 | 264 | 434 | 751 | 651 |
| NM_001220778 | CDKN1A | 0.13 | -0.03 | 13883 | 15626 | 32012 | 30770 |
| NM_080656 | CDKN2AIPN L | 0.03 | -0.03 | 189 | 242 | 343 | 279 |
| NM_001262 | CDKN2C | 0.22 | -0.33 | 430 | 579 | 339 | 220 |
| NM_005192 | CDKN3 | 0.03 | -0.99 | 275 | 339 | 331 | 131 |
| NM_207327 | CDPF1 | 0.00 | -0.72 | 98 | 123 | 279 | 133 |
| NM_030928 | CDT1 | 0.00 | -0.47 | 740 | 794 | 297 | 172 |
| NM_005195 | CEBPD | 0.42 | -0.23 | 62 | 119 | 125 | 76 |
| NM_018689 | CEMIP | 0.17 | -0.38 | 9699 | 11231 | 3955 | 2869 |
| NM_001809 | CENPA | 0.00 | -0.46 | 91 | 121 | 96 | 46 |
| NM_001813 | CENPE | 0.00 | -0.68 | 558 | 593 | 665 | 357 |

(continued)

| RefSeq ID | Gene Symbol | SR GFOLD | SEN GFOLD | SR IL-2 - | SR IL-2 + | SEN IL-2- | SEN IL-2 + |
|---|---|---|---|---|---|---|---|
| NM_016343 | CENPF | 0.00 | -0.97 | 1916 | 1987 | 2882 | 1362 |
| NM_022909 | CENPH | 0.00 | -0.78 | 104 | 140 | 189 | 81 |
| NM_006733 | CENPI | 0.00 | -0.45 | 231 | 261 | 293 | 172 |
| RefSeq ID | Gene Symbol | SR GFOLD | SEN GFOLD | SR IL-2 - | SR IL-2 + | SEN IL-2 - | SEN IL-2 + |
| NM_024053 | CENPM | 0.55 | -0.91 | 230 | 407 | 329 | 139 |
| NM_001100624 | CENPN | 0.17 | -0.24 | 1035 | 1282 | 1179 | 900 |
| NM_001199803 | CENPO | 0.00 | -0.22 | 1199 | 1251 | 1458 | 1139 |
| NM_025082 | CENPT | 0.53 | -0.03 | 741 | 1193 | 1984 | 1806 |
| NM_001012507 | CENPW | 0.38 | -0.96 | 141 | 235 | 237 | 92 |
| NM_152446 | CEP128 | 0.00 | -0.55 | 225 | 198 | 256 | 137 |
| NM_018718 | CEP41 | 0.38 | -0.04 | 141 | 235 | 618 | 525 |
| NM_018131 | CEP55 | 0.00 | -1.08 | 953 | 966 | 734 | 296 |
| NM_001098802 | CEP78 | 0.30 | -0.02 | 203 | 308 | 514 | 436 |
| NM_032816 | CEP89 | 0.19 | -0.08 | 334 | 450 | 990 | 841 |
| NM_005507 | CFL1 | 0.17 | -0.09 | 15387 | 17812 | 29260 | 26863 |
| NM_001243645 | CFL2 | 0.32 | -0.13 | 1962 | 2633 | 4663 | 4042 |
| NM_005483 | CHAF1A | 0.09 | -0.13 | 1046 | 1225 | 707 | 566 |
| NM_005441 | CHAF1B | 0.19 | -0.34 | 332 | 447 | 272 | 172 |
| NM_017812 | CHCHD3 | 0.07 | -0.03 | 540 | 649 | 1171 | 1035 |
| NM_001114122 | CHEK1 | 0.00 | -0.26 | 637 | 623 | 462 | 325 |
| NM_024591 | CHMP6 | 0.00 | -0.31 | 553 | 556 | 925 | 662 |
| NM_001206602 | CHN1 | 0.00 | -0.23 | 416 | 409 | 586 | 431 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_032630 | CINP | 0.17 | -0.08 | 411 | 539 | 1017 | 865 |
| NM_001136498 | CISD3 | 0.12 | -0.22 | 716 | 875 | 1647 | 1299 |
| NM_013324 | CISH | 0.93 | -0.42 | 31 | 95 | 56 | 24 |
| NM_001206999 | CIT | 0.26 | -0.99 | 1141 | 1497 | 1739 | 791 |
| NM_006079 | CITED2 | 0.00 | -0.41 | 4428 | 4394 | 7932 | 5734 |
| NM_001098525 | CKAP2 | 0.02 | -0.03 | 967 | 1090 | 1115 | 985 |
| NM_152515 | CKAP2L | 0.05 | -0.42 | 621 | 729 | 493 | 311 |
| NM_001827 | CKS2 | 0.00 | -0.37 | 561 | 593 | 586 | 390 |
| NM_005602 | CLDN11 | 0.07 | -1.05 | 4182 | 4626 | 2909 | 1299 |
| NM_002975 | CLEC11A | 0.25 | -0.02 | 1071 | 1403 | 1601 | 1449 |
| NM_175060 | CLEC14A | 0.02 | -1.46 | 125 | 167 | 370 | 104 |
| NM_001287594 | CLIC1 | 0.31 | -0.40 | 6592 | 8471 | 17949 | 13255 |
| NM_015526 | CLIP3 | 0.09 | -0.21 | 487 | 596 | 2493 | 2015 |
| NM_000086 | CLN3 | 0.15 | -0.09 | 666 | 836 | 1454 | 1250 |
| NM_017882 | CLN6 | 0.00 | -0.15 | 716 | 670 | 923 | 743 |
| NM_001258394 | CLPB | 0.19 | -0.13 | 743 | 953 | 1414 | 1181 |
| NM_022111 | CLSPN | 0.14 | -0.26 | 892 | 1088 | 507 | 362 |
| NM_182523 | CMC1 | 0.43 | -0.27 | 40 | 84 | 141 | 85 |
| NM_020188 | CMC2 | 0.00 | -0.30 | 794 | 860 | 796 | 571 |
| NM_018235 | CNDP2 | 0.01 | -0.17 | 1479 | 1621 | 2903 | 2418 |
| NM_173515 | CNKSR3 | 0.07 | -0.07 | 435 | 530 | 320 | 250 |
| NM_001299 | CNN1 | 0.00 | -0.40 | 1730 | 1748 | 1308 | 895 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_004368 | CNN2 | 0.11 | -0.61 | 2892 | 3309 | 5484 | 3425 |
| NM_017546 | CNOT11 | 0.00 | -0.07 | 632 | 629 | 747 | 627 |
| NM_007018 | CNTRL | 0.04 | -0.33 | 273 | 340 | 443 | 296 |
| NM_016565 | COA4 | 0.25 | -0.07 | 630 | 845 | 1198 | 1033 |
| NM_023077 | COA7 | 0.45 | -0.13 | 292 | 474 | 611 | 484 |
| NM_015386 | COG4 | 0.31 | -0.02 | 924 | 1270 | 2489 | 2292 |
| NM_030582 | COL18A1 | 0.00 | -0.01 | 189 | 231 | 811 | 713 |
| NM_130386 | COLEC12 | 0.00 | -1.76 | 38 | 28 | 164 | 31 |
| NM_152516 | COMMD1 | 0.19 | -0.18 | 270 | 371 | 803 | 627 |
| NM_006837 | COPS5 | 0.00 | -0.08 | 874 | 864 | 1321 | 1140 |
| NM_006833 | COPS6 | 0.03 | -0.16 | 1970 | 2160 | 4889 | 4159 |
| NM_016035 | COQ4 | 0.04 | -0.29 | 203 | 260 | 1023 | 750 |
| NM_052820 | CORO2A | 0.04 | -0.12 | 201 | 257 | 383 | 294 |
| NM_016468 | COX16 | 0.00 | -0.10 | 341 | 350 | 707 | 580 |
| NM_001863 | COX6B1 | 0.40 | -0.12 | 1002 | 1459 | 3288 | 2848 |
| NM_001867 | COX7C | 0.05 | -0.03 | 662 | 776 | 1123 | 996 |
| NM_004074 | COX8A | 0.58 | -0.40 | 1081 | 1764 | 2993 | 2116 |
| NM_001177382 | CPEB2 | 0.00 | -0.02 | 1026 | 967 | 1029 | 915 |
| NM_152727 | CPNE2 | 0.11 | -0.07 | 599 | 734 | 851 | 719 |
| NM_014427 | CPNE7 | 0.00 | -0.06 | 664 | 703 | 2317 | 2079 |
| NM_153634 | CPNE8 | 0.00 | -0.23 | 282 | 275 | 593 | 438 |
| NM_001122633 | CPS1 | 0.28 | -0.13 | 102 | 166 | 320 | 240 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_ 001256456 | CPSF3L | 0.57 | -0.09 | 995 | 1625 | 2406 | 2107 |
| NM_ 001876 | CPT1A | 0.04 | -0.06 | 2237 | 2467 | 4074 | 3697 |
| NM_ 198148 | CPXM2 | 0.07 | -1.27 | 73 | 109 | 237 | 72 |
| NM_ 001144958 | CRACR2A | 0.23 | -0.01 | 174 | 256 | 514 | 438 |
| NM_ 014675 | CROCC | 0.03 | -0.02 | 136 | 181 | 387 | 320 |
| NM_ 004077 | CS | 0.13 | -0.15 | 1943 | 2282 | 3980 | 3408 |
| NM_ 001316 | CSE1L | 0.14 | -0.14 | 2065 | 2432 | 3818 | 3283 |
| NM_ 022048 | CSNK1G1 | 0.15 | -0.03 | 755 | 936 | 1425 | 1277 |
| NM_ 001325 | CSTF2 | 0.21 | -0.06 | 333 | 455 | 709 | 599 |
| NM_ 015343 | CTDNEP1 | 0.36 | -0.22 | 854 | 1217 | 1593 | 1250 |
| NM_ 001142544 | CTF1 | 0.00 | -0.43 | 70 | 72 | 191 | 107 |
| NM_ 175859 | CTPS2 | 0.33 | -0.04 | 240 | 365 | 620 | 525 |
| NM_ 001814 | CTSC | 0.19 | -0.35 | 921 | 1163 | 607 | 412 |
| NM_ 001912 | CTSL | 0.00 | -0.07 | 3362 | 3189 | 9600 | 8854 |
| NM_ 005231 | CTTN | 0.23 | -0.04 | 8090 | 9832 | 20056 | 19079 |
| NM_ 024040 | CUEDC2 | 0.26 | -0.37 | 596 | 811 | 2075 | 1486 |
| NM_ 001079872 | CUL4B | 0.23 | -0.06 | 1410 | 1799 | 3558 | 3222 |
| NM_ 015921 | CUTA | 0.00 | -0.16 | 1895 | 1814 | 3097 | 2606 |
| NM_ 018294 | CWF19L1 | 0.00 | -0.33 | 280 | 332 | 428 | 285 |
| NM_ 001914 | CYB5A | 0.63 | -0.16 | 109 | 221 | 576 | 445 |
| NM_ 030579 | CYB5B | 0.47 | -0.06 | 890 | 1366 | 2360 | 2113 |
| NM_ 016230 | CYB5R4 | 0.00 | -0.14 | 185 | 184 | 349 | 262 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_ 001916 | CYC1 | 0.00 | -0.11 | 3330 | 3349 | 5347 | 4743 |
| NM_ 018947 | CYCS | 0.00 | -0.11 | 1221 | 1262 | 1601 | 1360 |
| NM_ 019885 | CYP26B1 | 0.00 | -0.79 | 115 | 138 | 501 | 242 |
| NM_ 001554 | CYR61 | 0.00 | -0.05 | 31391 | 30861 | 12856 | 12074 |
| NM_ 032412 | CYSTM1 | 0.29 | -0.15 | 473 | 665 | 3088 | 2612 |
| NM_ 004227 | CYTH3 | 0.00 | -0.12 | 2292 | 2424 | 3592 | 3120 |
| NM_ 032552 | DAB2IP | 0.00 | -0.03 | 546 | 588 | 1633 | 1475 |
| NM_ 001009996 | DALRD3 | 0.00 | -0.03 | 848 | 845 | 1755 | 1588 |
| NM_ 001348 | DAPK3 | 0.29 | -0.04 | 1889 | 2474 | 3371 | 3100 |
| NM_ 018122 | DARS2 | 0.00 | -0.14 | 401 | 392 | 801 | 645 |
| NM_ 018959 | DAZAP1 | 0.10 | -0.26 | 1886 | 2179 | 1612 | 1231 |
| NM_ 006716 | DBF4 | 0.00 | -0.15 | 169 | 177 | 166 | 113 |
| NM_ 017741 | DCAF16 | 0.26 | -0.04 | 492 | 678 | 747 | 643 |
| NM_ 022836 | DCLRE1B | 0.00 | -0.07 | 552 | 531 | 468 | 381 |
| NM_ 001261412 | DCTN2 | 0.43 | -0.06 | 2286 | 3274 | 6249 | 5754 |
| NM_ 006571 | DCTN6 | 0.00 | -0.03 | 453 | 454 | 909 | 795 |
| NM_ 024096 | DCTPP1 | 0.57 | -0.17 | 422 | 723 | 809 | 636 |
| NM_ 005804 | DDX39A | 0.23 | -0.58 | 1537 | 1946 | 2019 | 1238 |
| NM_ 004640 | DDX39B | 0.07 | -0.06 | 1352 | 1552 | 2489 | 2229 |
| NM_ 019070 | DDX49 | 0.00 | -0.25 | 692 | 772 | 1485 | 1140 |
| NM_ 152678 | DENND6A | 0.09 | -0.17 | 227 | 297 | 347 | 255 |
| NM_ 001114120 | DEPDC1 | 0.00 | -0.77 | 200 | 233 | 279 | 128 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_022783 | DEPTOR | 0.09 | -0.24 | 42 | 70 | 96 | 55 |
| NM_001927 | DES | 0.43 | -0.07 | 362 | 569 | 135 | 94 |
| NM_005675 | DGCR6 | 0.00 | -0.17 | 118 | 124 | 299 | 216 |
| NM_001361 | DHODH | 0.00 | -0.14 | 252 | 219 | 250 | 181 |
| NM_024308 | DHRS11 | 0.74 | -0.17 | 35 | 92 | 154 | 102 |
| NM_021004 | DHRS4 | 0.00 | -0.25 | 192 | 229 | 337 | 233 |
| NM_001193636 | DHRS4L2 | 0.17 | -0.42 | 103 | 156 | 308 | 185 |
| NM_003587 | DHX16 | 0.40 | -0.03 | 1402 | 2005 | 2196 | 2006 |
| NM_004941 | DHX8 | 0.13 | -0.16 | 1247 | 1494 | 2219 | 1852 |
| NM_001042517 | DIAPH3 | 0.03 | -0.63 | 1363 | 1521 | 1050 | 603 |
| NM_014473 | DIMT1 | 0.08 | -0.02 | 680 | 809 | 1034 | 917 |
| NM_004675 | DIRAS3 | 0.00 | -0.23 | 7 | 11 | 175 | 113 |
| NM_012242 | DKK1 | 0.01 | -0.12 | 16497 | 17002 | 18658 | 16700 |
| NM_001146015 | DLGAP5 | 0.00 | -0.98 | 520 | 543 | 603 | 257 |
| NM_178120 | DLX1 | 0.00 | -0.32 | 58 | 65 | 119 | 67 |
| NM_018198 | DNAJC11 | 0.09 | -0.06 | 1429 | 1652 | 2298 | 2055 |
| NM_018163 | DNAJC17 | 0.15 | -0.01 | 146 | 208 | 266 | 213 |
| NM_032317 | DNAJC30 | 0.00 | -0.07 | 122 | 159 | 480 | 390 |
| NM_001278464 | DNM1L | 0.13 | -0.02 | 1825 | 2155 | 3107 | 2880 |
| NM_001130823 | DNMT1 | 0.04 | -0.19 | 3614 | 3923 | 2566 | 2102 |
| NM_001282062 | DOC2A | 0.51 | -0.08 | 100 | 189 | 225 | 168 |
| NM_005128 | DOPEY2 | 0.03 | -0.13 | 917 | 1037 | 2926 | 2512 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_ 003863 | DPM2 | 0.42 | -0.18 | 544 | 826 | 1377 | 1103 |
| NM_ 153741 | DPM3 | 0.54 | -0.20 | 176 | 319 | 745 | 569 |
| NM_ 032574 | DPY30 | 0.00 | -0.06 | 780 | 792 | 1113 | 963 |
| NM_ 004147 | DRG1 | 0.21 | -0.12 | 963 | 1227 | 1689 | 1427 |
| NM_ 001388 | DRG2 | 0.10 | -0.18 | 761 | 914 | 1564 | 1261 |
| NM_ 001939 | DRP2 | 0.00 | -0.13 | 76 | 90 | 1015 | 834 |
| NM_ 032160 | DSEL | 0.02 | -0.19 | 2349 | 2550 | 4151 | 3460 |
| NM_ 080664 | DTD2 | 0.19 | -0.06 | 155 | 225 | 387 | 311 |
| NM_ 016448 | DTL | 0.28 | -0.37 | 839 | 1135 | 518 | 342 |
| NM_ 012145 | DTYMK | 0.19 | -0.05 | 705 | 901 | 1177 | 1028 |
| NM_ 017803 | DUS2 | 0.00 | -0.44 | 384 | 452 | 701 | 449 |
| NM_ 020175 | DUS3L | 0.17 | -0.08 | 342 | 455 | 408 | 325 |
| NM_ 007026 | DUSP14 | 0.12 | -0.24 | 3052 | 3523 | 6002 | 4867 |
| NM_ 006519 | DYNLT1 | 0.23 | -0.13 | 1405 | 1787 | 2627 | 2240 |
| NM_ 001950 | E2F4 | 0.36 | -0.04 | 1548 | 2145 | 2394 | 2179 |
| NM_ 001258316 | ECT2 | 0.35 | -0.01 | 599 | 862 | 940 | 835 |
| NM_ 003797 | EED | 0.00 | -0.21 | 165 | 169 | 193 | 128 |
| NM_ 001959 | EEF1B2 | 0.46 | -0.03 | 684 | 1058 | 2015 | 1835 |
| NM_ 032378 | EEF1D | 0.19 | -0.14 | 1974 | 2423 | 3321 | 2834 |
| NM_ 001404 | EEF1G | 0.44 | -0.04 | 6878 | 9651 | 16581 | 15728 |
| NM_ 021937 | EEFSEC | 0.00 | -0.16 | 506 | 508 | 944 | 752 |
| NM_ 001258354 | EFTUD2 | 0.26 | -0.02 | 2650 | 3368 | 5207 | 4906 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_005228 | EGFR | 0.06 | -0.09 | 4980 | 5431 | 5971 | 5368 |
| NM_080732 | EGLN2 | 0.04 | -0.10 | 1313 | 1473 | 2365 | 2059 |
| NM_001166415 | EHHADH | 0.00 | -0.03 | 73 | 61 | 252 | 198 |
| NM_024757 | EHMT1 | 0.00 | -0.18 | 1654 | 1733 | 2616 | 2155 |
| NM_153232 | EID2 | 0.00 | -0.06 | 249 | 260 | 333 | 264 |
| NM_006893 | EIF2D | 0.33 | -0.17 | 682 | 966 | 1481 | 1201 |
| NM_001568 | EIF3E | 0.00 | -0.19 | 657 | 725 | 1458 | 1170 |
| NM_003754 | EIF3F | 0.22 | -0.28 | 748 | 977 | 1239 | 924 |
| NM_003755 | EIF3G | 0.26 | -0.05 | 2266 | 2896 | 4078 | 3747 |
| NM_003757 | EIF3I | 0.18 | -0.09 | 3964 | 4708 | 7709 | 6965 |
| NM_013234 | EIF3K | 0.52 | -0.10 | 661 | 1064 | 2071 | 1793 |
| NM_016091 | EIF3L | 0.38 | -0.03 | 1911 | 2668 | 4835 | 4508 |
| NM_001416 | EIF4A1 | 0.43 | -0.29 | 5254 | 7364 | 12022 | 9547 |
| NM_001970 | EIF5A | 0.28 | -0.18 | 3606 | 4616 | 7495 | 6346 |
| NM_001099692 | EIF5AL1 | 0.08 | -0.01 | 4612 | 5115 | 8013 | 7662 |
| NM_002212 | EIF6 | 0.13 | -0.01 | 3082 | 3571 | 5636 | 5338 |
| NM_001128636 | ELFN1 | 0.00 | -0.50 | 178 | 186 | 651 | 397 |
| NM_052906 | ELFN2 | 0.00 | -0.46 | 4 | 9 | 87 | 41 |
| NM_001278939 | ELN | 0.50 | -0.41 | 2316 | 3480 | 641 | 416 |
| NM_001256399 | ELOVL1 | 0.00 | -0.11 | 4142 | 4262 | 7144 | 6360 |
| NM_032048 | EMILIN2 | 0.00 | -0.77 | 940 | 947 | 1813 | 969 |
| NM_019063 | EML4 | 0.00 | -0.06 | 1138 | 1220 | 1244 | 1083 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001424 | EMP2 | 0.12 | -0.08 | 1343 | 1591 | 1533 | 1329 |
| NM_001426 | EN1 | 0.49 | -0.09 | 285 | 475 | 930 | 778 |
| NM_000118 | ENG | 0.13 | -0.05 | 13055 | 14733 | 15252 | 14379 |
| NM_017512 | ENOSF1 | 0.00 | -0.07 | 1286 | 1269 | 1004 | 861 |
| NM_182314 | ENOX2 | 0.00 | -0.01 | 208 | 195 | 428 | 359 |
| NM_006209 | ENPP2 | 0.05 | -0.31 | 491 | 587 | 1547 | 1137 |
| NM_004444 | EPHB4 | 0.15 | -0.15 | 402 | 521 | 468 | 359 |
| NM_004447 | EPS8 | 0.22 | -0.12 | 2317 | 2882 | 5800 | 5102 |
| NM_005702 | ERAL1 | 0.24 | -0.03 | 824 | 1084 | 1524 | 1372 |
| NM_000400 | ERCC2 | 0.21 | -0.18 | 2075 | 2576 | 4122 | 3460 |
| NM_017669 | ERCC6L | 0.00 | -0.53 | 335 | 284 | 179 | 92 |
| NM_001017420 | ESCO2 | 0.00 | -0.16 | 151 | 190 | 102 | 63 |
| NM_001984 | ESD | 0.00 | -0.01 | 1959 | 2043 | 3779 | 3545 |
| NM_012291 | ESPL1 | 0.00 | -0.59 | 690 | 640 | 424 | 233 |
| NM_001282451 | ESRRA | 0.00 | -0.16 | 603 | 662 | 832 | 660 |
| NM_005238 | ETS1 | 0.19 | -0.06 | 4177 | 5002 | 4465 | 4065 |
| NM_001135032 | EVA1A | 0.32 | -0.09 | 2051 | 2744 | 3371 | 2991 |
| NM_018166 | EVA1B | 0.04 | -0.32 | 593 | 696 | 1160 | 839 |
| NM_013986 | EWSR1 | 0.40 | -0.34 | 1983 | 2806 | 3094 | 2296 |
| NM_017820 | EXD3 | 0.11 | -0.10 | 60 | 95 | 220 | 161 |
| NM_130398 | EXO1 | 0.29 | -0.43 | 322 | 467 | 171 | 94 |
| NM_014285 | EXOSC2 | 0.28 | -0.23 | 524 | 726 | 1002 | 762 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_020158 | EXOSC5 | 0.00 | -0.16 | 215 | 176 | 354 | 262 |
| NM_058219 | EXOSC6 | 0.47 | -0.16 | 332 | 540 | 626 | 488 |
| NM_015004 | EXOSC7 | 0.22 | -0.07 | 373 | 510 | 780 | 656 |
| NM_001440 | EXTL3 | 0.40 | -0.11 | 1829 | 2599 | 5143 | 4530 |
| NM_000137 | FAH | 0.36 | -0.30 | 302 | 460 | 2019 | 1519 |
| NM_016044 | FAHD2A | 0.02 | -0.25 | 249 | 307 | 620 | 451 |
| NM_001002034 | FAM109B | 0.01 | -0.09 | 677 | 770 | 1061 | 898 |
| NM_052966 | FAM129A | 0.23 | -0.19 | 1922 | 2428 | 5659 | 4730 |
| NM_001166698 | FAM13C | 0.00 | -0.57 | 10 | 11 | 79 | 33 |
| NM_001001710 | FAM166A | 0.00 | -0.19 | 622 | 617 | 684 | 523 |
| NM_207368 | FAM195B | 0.50 | -0.10 | 991 | 1540 | 3354 | 2952 |
| NM_016613 | FAM198B | 0.00 | -0.16 | 228 | 251 | 1736 | 1425 |
| NM_001291328 | FAM228B | 0.67 | -0.20 | 100 | 211 | 618 | 466 |
| NM_153711 | FAM26E | 0.46 | -0.16 | 270 | 443 | 942 | 752 |
| NM_052943 | FAM46B | 0.73 | -0.36 | 175 | 360 | 127 | 70 |
| NM_030797 | FAM49A | 0.00 | -0.46 | 11 | 23 | 195 | 107 |
| NM_001013622 | FAM53A | 0.97 | -0.17 | 17 | 62 | 42 | 20 |
| NM_019013 | FAM64A | 0.16 | -1.32 | 173 | 244 | 245 | 72 |
| NM_001193523 | FAM65A | 0.43 | -0.02 | 2191 | 3147 | 4211 | 3944 |
| NM_032809 | FAM73B | 0.53 | -0.10 | 321 | 544 | 836 | 693 |
| NM_030919 | FAM83D | 0.12 | -0.70 | 450 | 566 | 545 | 283 |
| NM_000136 | FANCC | 0.00 | -0.11 | 227 | 271 | 264 | 196 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_ 001018115 | FANCD2 | 0.16 | -0.38 | 317 | 421 | 327 | 205 |
| NM_ 001271783 | FAR2 | 0.00 | -0.07 | 191 | 223 | 349 | 275 |
| NM_ 005687 | FARSB | 0.23 | -0.21 | 627 | 832 | 1518 | 1201 |
| NM_ 001136193 | FASTKD2 | 0.26 | -0.12 | 649 | 879 | 1266 | 1054 |
| NM_ 001008781 | FAT3 | 0.00 | -0.16 | 267 | 306 | 1148 | 924 |
| NM_ 001291285 | FAT4 | 0.10 | -0.50 | 1098 | 1297 | 1512 | 974 |
| NM_ 001436 | FBL | 0.34 | -0.01 | 1064 | 1480 | 1221 | 1098 |
| NM_ 017556 | FBLIM1 | 0.40 | -0.02 | 968 | 1412 | 3271 | 3044 |
| NM_ 006486 | FBLN1 | 0.00 | -0.85 | 376 | 396 | 524 | 242 |
| NM_ 001999 | FBN2 | 0.00 | -1.22 | 2156 | 2154 | 2350 | 922 |
| NM_ 017703 | FBXL12 | 0.38 | -0.01 | 232 | 367 | 686 | 597 |
| NM_ 024963 | FBXL18 | 0.00 | -0.02 | 1035 | 989 | 1770 | 1606 |
| NM_ 018998 | FBXW5 | 0.30 | -0.15 | 2712 | 3547 | 5736 | 4928 |
| NM_ 001031734 | FDX1L | 0.14 | -0.13 | 258 | 345 | 838 | 677 |
| NM_ 001258013 | FDXR | 0.10 | -0.47 | 119 | 168 | 430 | 259 |
| NM_ 004111 | FEN1 | 0.19 | -0.46 | 1865 | 2292 | 1339 | 880 |
| NM_ 002005 | FES | 0.00 | -0.58 | 370 | 399 | 636 | 364 |
| NM_ 002006 | FGF2 | -0.01 | -0.05 | 5573 | 5301 | 8776 | 8185 |
| NM_ 004464 | FGF5 | 0.34 | -0.12 | 4322 | 5738 | 3329 | 2895 |
| NM_ 002009 | FGF7 | 0.00 | -0.11 | 756 | 766 | 1131 | 946 |
| NM_ 001287492 | FIGNL1 | 0.00 | -0.03 | 325 | 353 | 225 | 174 |
| NM_ 001042459 | FILIP1L | 0.00 | -0.25 | 573 | 558 | 1695 | 1307 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_ 015258 | FKBP15 | 0.25 | -0.05 | 1391 | 1797 | 3111 | 2835 |
| NM_ 001079802 | FKTN | 0.00 | -0.09 | 419 | 485 | 1312 | 1120 |
| NM_ 025207 | FLAD1 | 0.10 | -0.08 | 426 | 532 | 1034 | 878 |
| NM_ 005803 | FLOT1 | 0.39 | -0.15 | 1488 | 2112 | 4993 | 4283 |
| NM_ 002019 | FLT1 | 0.00 | -0.26 | 92 | 113 | 148 | 91 |
| NM_ 001142500 | FLYWCH2 | 0.00 | -0.35 | 200 | 245 | 497 | 331 |
| NM_ 005438 | FOSL1 | 0.33 | -0.16 | 4479 | 5899 | 3947 | 3338 |
| NM_ 001451 | FOXF1 | 0.00 | -0.24 | 91 | 81 | 177 | 113 |
| NM_ 202002 | FOXM1 | 0.26 | -0.86 | 2104 | 2690 | 2175 | 1098 |
| NM_ 001102371 | FOXRED2 | 0.00 | -0.37 | 767 | 775 | 1813 | 1292 |
| NM_ 001018078 | FPGS | 0.23 | -0.23 | 1094 | 1406 | 2521 | 2006 |
| NM_ 174938 | FRMD3 | 0.15 | -0.29 | 654 | 819 | 851 | 614 |
| NM_ 001267046 | FRMD6 | 0.24 | -0.05 | 10248 | 12522 | 23265 | 21989 |
| NM_ 023037 | FRY | 0.10 | -0.22 | 263 | 342 | 674 | 503 |
| NM_ 003088 | FSCN1 | 0.12 | -0.22 | 5767 | 6530 | 6033 | 4967 |
| NM_ 003902 | FUBP1 | 0.06 | -0.16 | 2579 | 2854 | 3157 | 2651 |
| NM_ 001170634 | FUS | 0.08 | -0.03 | 4400 | 4886 | 5644 | 5299 |
| NM_ 001466 | FZD2 | 0.00 | -0.05 | 1403 | 1392 | 1381 | 1216 |
| NM_ 005754 | G3BP1 | 0.55 | -0.06 | 1586 | 2498 | 2968 | 2675 |
| NM_ 007278 | GABARAP | 0.23 | -0.03 | 1187 | 1521 | 3402 | 3155 |
| NM_ 000403 | GALE | 0.11 | -0.23 | 637 | 779 | 938 | 713 |
| NM_ 000154 | GALK1 | 0.00 | -0.14 | 484 | 531 | 1031 | 839 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_054110 | GALNT15 | 0.36 | -0.65 | 79 | 140 | 749 | 414 |
| NM_014568 | GALNT5 | 0.00 | -0.13 | 1964 | 2030 | 7636 | 6706 |
| NM_007210 | GALNT6 | 0.00 | -0.27 | 368 | 396 | 711 | 516 |
| NM_000155 | GALT | 0.04 | -0.12 | 248 | 311 | 830 | 677 |
| NM_000156 | GAMT | 0.07 | -0.22 | 105 | 148 | 630 | 468 |
| NM_014364 | GAPDHS | 0.24 | -0.27 | 37 | 70 | 131 | 78 |
| NM_006478 | GAS2L1 | 0.09 | -0.01 | 612 | 738 | 1154 | 1035 |
| NM_174942 | GAS2L3 | 0.38 | -0.70 | 136 | 228 | 191 | 87 |
| NM_004564 | GATB | 0.26 | -0.29 | 303 | 433 | 853 | 619 |
| NM_001282629 | GBGT1 | 0.66 | -0.03 | 60 | 136 | 141 | 102 |
| NM_002053 | GBP1 | 0.00 | -0.15 | 339 | 365 | 526 | 408 |
| NM_004120 | GBP2 | 0.00 | -0.29 | 65 | 70 | 243 | 157 |
| NM_000159 | GCDH | 0.00 | -0.06 | 204 | 238 | 458 | 373 |
| NM_000557 | GDF5 | 0.00 | -1.00 | 8 | 13 | 177 | 63 |
| NM_001494 | GDI2 | 0.22 | -0.02 | 4008 | 4895 | 6391 | 6041 |
| NM_001252156 | GEMIN5 | 0.11 | -0.09 | 910 | 1090 | 1439 | 1233 |
| NM_018988 | GFOD1 | 0.00 | -0.06 | 104 | 133 | 152 | 109 |
| NM_001099781 | GGT5 | 0.15 | -0.90 | 21 | 43 | 81 | 26 |
| NM_021067 | GINS1 | 0.00 | -0.11 | 311 | 330 | 148 | 102 |
| NM_016095 | GINS2 | 0.00 | -0.21 | 380 | 411 | 260 | 179 |
| NM_032336 | GINS4 | 0.00 | -0.07 | 388 | 398 | 378 | 301 |
| NM_001080383 | GJC1 | 0.11 | -0.08 | 1437 | 1688 | 1720 | 1497 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_000168 | GLI3 | 0.00 | -0.07 | 759 | 722 | 1100 | 943 |
| NM_006708 | GLO1 | 0.37 | -0.09 | 892 | 1280 | 2770 | 2440 |
| NM_015710 | GLTSCR2 | 0.79 | -0.03 | 368 | 736 | 990 | 871 |
| NM_002065 | GLUL | 0.31 | -0.41 | 2045 | 2708 | 3789 | 2686 |
| NM_001146686 | GMNC | 0.00 | -0.76 | 3 | 11 | 173 | 74 |
| NM_015895 | GMNN | 0.00 | -0.49 | 411 | 471 | 254 | 142 |
| NM_006098 | GNB2L1 | 0.14 | -0.01 | 16273 | 18409 | 33137 | 32212 |
| NM_002075 | GNB3 | 0.00 | -0.34 | 93 | 96 | 131 | 74 |
| NM_004126 | GNG11 | 0.02 | -0.03 | 1274 | 1410 | 4480 | 4166 |
| NM_005471 | GNPDA1 | 0.33 | -0.23 | 963 | 1332 | 2510 | 2000 |
| NM_002080 | GOT2 | 0.00 | -0.03 | 2328 | 2494 | 3658 | 3399 |
| NM_005708 | GPC6 | 0.13 | -0.30 | 505 | 636 | 1206 | 885 |
| NM_032777 | GPR124 | 0.05 | -0.16 | 1568 | 1763 | 2529 | 2115 |
| NM_198827 | GPR133 | 0.00 | -0.18 | 129 | 157 | 98 | 59 |
| NM_001276501 | GPSM3 | 0.23 | -0.02 | 80 | 130 | 243 | 192 |
| NM_201397 | GPX1 | 0.03 | -0.18 | 1172 | 1315 | 3188 | 2656 |
| NM_015696 | GPX7 | 0.47 | -0.07 | 677 | 1050 | 1373 | 1192 |
| NM_013372 | GREM1 | 0.14 | -0.22 | 183659 | 203725 | 239474 | 204292 |
| NM_012203 | GRHPR | 0.25 | -0.13 | 1219 | 1581 | 3575 | 3078 |
| NM_005308 | GRK5 | 0.25 | -0.07 | 394 | 546 | 499 | 407 |
| NM_001004106 | GRK6 | 0.00 | -0.24 | 788 | 872 | 1264 | 970 |
| NM_031485 | GRWD1 | 0.00 | -0.05 | 1241 | 1294 | 1398 | 1235 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001166237 | GSDMD | 0.00 | -0.97 | 343 | 350 | 412 | 170 |
| NM_000178 | GSS | 0.00 | -0.12 | 1070 | 1144 | 3612 | 3135 |
| NM_001512 | GSTA4 | 0.00 | -0.34 | 92 | 90 | 368 | 240 |
| NM_001031720 | GSTCD | 0.00 | -0.02 | 252 | 253 | 399 | 331 |
| NM_001143679 | GSTK1 | 0.81 | -0.19 | 767 | 1488 | 3488 | 2889 |
| NM_004832 | GSTO1 | 0.06 | -0.03 | 1983 | 2217 | 5045 | 4699 |
| NM_001516 | GTF2H3 | 0.00 | -0.08 | 482 | 431 | 690 | 573 |
| NM_001122823 | GTF3C5 | 0.31 | -0.09 | 1065 | 1449 | 2051 | 1786 |
| NM_021927 | GUF1 | 0.00 | -0.03 | 289 | 337 | 522 | 440 |
| NM_177925 | H2AFJ | 0.18 | -0.45 | 1315 | 1622 | 2263 | 1532 |
| NM_138635 | H2AFV | 0.34 | -0.05 | 873 | 1228 | 1414 | 1244 |
| NM_002105 | H2AFX | 0.42 | -0.26 | 946 | 1397 | 980 | 728 |
| NM_002106 | H2AFZ | 0.28 | -0.53 | 1961 | 2558 | 2263 | 1445 |
| NM_005324 | H3F3B | 0.00 | -0.14 | 2975 | 3001 | 3427 | 2928 |
| NM_021973 | HAND2 | 0.00 | -0.16 | 172 | 190 | 79 | 46 |
| NM_012208 | HARS2 | 0.11 | -0.01 | 309 | 398 | 824 | 726 |
| NM_005328 | HAS2 | 0.30 | -0.13 | 4581 | 5898 | 11051 | 9784 |
| NM_138443 | HAUS1 | 0.43 | -0.03 | 133 | 231 | 227 | 176 |
| NM_001166269 | HAUS4 | 0.19 | -0.03 | 177 | 254 | 468 | 390 |
| NM_001011699 | HAUS8 | 0.00 | -0.05 | 317 | 321 | 187 | 139 |
| NM_178423 | HDAC9 | 0.51 | -0.08 | 165 | 295 | 620 | 510 |
| NM_024602 | HECTD3 | 0.02 | -0.04 | 1182 | 1315 | 2425 | 2198 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_020760 | HECW2 | 0.05 | -0.09 | 672 | 784 | 1331 | 1137 |
| NM_001289067 | HELLS | 0.07 | -0.07 | 335 | 416 | 195 | 144 |
| NM_022079 | HERC4 | 0.13 | -0.04 | 3367 | 3880 | 6079 | 5663 |
| NM_001165967 | HES7 | 2.24 | -0.39 | 35 | 249 | 225 | 133 |
| NM_138820 | HIGD2A | 0.06 | -0.02 | 556 | 662 | 1333 | 1198 |
| NM_032593 | HINT2 | 0.00 | -0.05 | 204 | 176 | 347 | 279 |
| NM_005322 | HIST1H1B | 0.07 | -0.68 | 7478 | 8154 | 3354 | 1956 |
| NM_003513 | HIST1H2AB | 0.08 | -0.13 | 513 | 622 | 297 | 220 |
| NM_003512 | HIST1H2AC | 0.36 | -0.06 | 2031 | 2792 | 3369 | 3055 |
| NM_021065 | HIST1H2AD | 0.00 | -0.20 | 1097 | 1046 | 620 | 468 |
| NM_021052 | HIST1H2AE | 0.00 | -0.42 | 3005 | 3127 | 1171 | 786 |
| NM_021064 | HIST1H2AG | 0.52 | -0.25 | 1398 | 2168 | 665 | 486 |
| NM_080596 | HIST1H2AH | 0.12 | -0.06 | 1096 | 1309 | 476 | 390 |
| NM_003509 | HIST1H2AI | 0.00 | -0.72 | 1571 | 1475 | 828 | 438 |
| NM_021066 | HIST1H2AJ | 0.00 | -0.45 | 1154 | 1177 | 501 | 309 |
| NM_003511 | HIST1H2AL | 0.00 | -0.56 | 2557 | 2601 | 1098 | 664 |
| NM_003514 | HIST1H2AM | 0.00 | -0.32 | 1488 | 1521 | 634 | 440 |
| NM_021062 | HIST1H2BB | 0.25 | -0.52 | 874 | 1156 | 545 | 323 |
| NM_003526 | HIST1H2BC | 0.10 | -0.09 | 4518 | 5091 | 4074 | 3634 |
| NM_003518 | HIST1H2BG | 0.40 | -0.09 | 1290 | 1848 | 703 | 580 |
| NM_003525 | HIST1H2BI | 0.36 | -0.47 | 1494 | 2076 | 784 | 494 |
| NM_003521 | HIST1H2BM | 0.16 | -0.22 | 1063 | 1306 | 410 | 294 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_ 003520 | HIST1H2BN | 0.22 | -0.37 | 1498 | 1886 | 1468 | 1031 |
| NM_ 003527 | HIST1H2BO | 0.00 | -0.33 | 1336 | 1320 | 711 | 495 |
| NM_ 003537 | HIST1H3B | 0.11 | -0.54 | 6097 | 6875 | 2897 | 1859 |
| NM_ 003531 | HIST1H3C | 0.08 | -0.52 | 4759 | 5271 | 2009 | 1286 |
| NM_ 003530 | HIST1H3D | 0.00 | -0.33 | 2430 | 2402 | 1441 | 1044 |
| NM_ 003536 | HIST1H3H | 0.16 | -0.51 | 1688 | 2035 | 915 | 566 |
| NM_ 003533 | HIST1H3I | 0.00 | -0.02 | 768 | 823 | 295 | 237 |
| NM_ 003544 | HIST1H4B | 0.00 | -0.08 | 2461 | 2621 | 1462 | 1268 |
| NM_ 003542 | HIST1H4C | 0.29 | -0.15 | 1357 | 1801 | 1050 | 848 |
| NM_ 003495 | HIST1H4I | 0.00 | -0.09 | 383 | 341 | 146 | 102 |
| NM_ 175065 | HIST2H2AB | 0.22 | -0.27 | 458 | 617 | 406 | 281 |
| NM_ 003517 | HIST2H2AC | 0.00 | -0.21 | 1375 | 1382 | 1545 | 1222 |
| NM_ 003528 | HIST2H2BE | 0.00 | -0.20 | 2823 | 2960 | 2065 | 1667 |
| NM_ 001005464 | HIST2H3A | 0.80 | -0.61 | 3293 | 6017 | 2466 | 1501 |
| NM_ 021059 | HIST2H3C | 0.80 | -0.61 | 3293 | 6017 | 2466 | 1501 |
| NM_ 001123375 | HIST2H3D | 0.28 | -0.54 | 659 | 902 | 266 | 144 |
| NM_ 003548 | HIST2H4A | 0.00 | -0.34 | 8406 | 8202 | 2920 | 2155 |
| NM_ 001034077 | HIST2H4B | 0.00 | -0.34 | 8406 | 8202 | 2920 | 2155 |
| NM_ 018410 | HJURP | 0.22 | -0.76 | 757 | 987 | 562 | 281 |
| NM_ 000190 | HMBS | 0.21 | -0.17 | 545 | 719 | 1092 | 876 |
| NM_ 006339 | HMG20B | 0.00 | -0.16 | 1459 | 1420 | 2812 | 2364 |
| NM_ 145901 | HMGA1 | 0.31 | -0.31 | 11513 | 14723 | 23733 | 18724 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_ 002128 | HMGB1 | 0.00 | -0.19 | 1500 | 1384 | 1949 | 1579 |
| NM_ 001130688 | HMGB2 | 0.00 | -0.39 | 1214 | 1218 | 657 | 433 |
| NM_ 006353 | HMGN4 | 0.00 | -0.11 | 1098 | 1096 | 1828 | 1558 |
| NM_ 001142556 | HMMR | 0.00 | -0.49 | 253 | 288 | 312 | 179 |
| NM_ 001127204 | HMOX2 | 0.00 | -0.33 | 1467 | 1465 | 2356 | 1745 |
| NM_ 001002032 | HN1 | 0.00 | -0.24 | 1313 | 1374 | 1776 | 1386 |
| NM_ 031157 | HNRNPA1 | 0.18 | -0.20 | 1272 | 1569 | 2144 | 1726 |
| NM_ 001011724 | HNRNPA1L2 | 0.00 | -0.10 | 794 | 873 | 1102 | 926 |
| NM_ 031243 | HNRNPA2B 1 | 0.17 | -0.08 | 6298 | 7396 | 7674 | 6968 |
| NM_ 031370 | HNRNPD | 0.03 | -0.08 | 933 | 1056 | 695 | 575 |
| NM_ 001098206 | HNRNPF | 0.38 | -0.06 | 4775 | 6489 | 5786 | 5309 |
| NM_ 002140 | HNRNPK | 0.06 | -0.03 | 2537 | 2822 | 4536 | 4220 |
| NM_ 001533 | HNRNPL | 0.26 | -0.10 | 1383 | 1794 | 2633 | 2303 |
| NM_ 005968 | HNRNPM | 0.03 | -0.23 | 1954 | 2151 | 3130 | 2505 |
| NM_ 001102398 | HNRNPR | 0.00 | -0.21 | 2847 | 2697 | 2552 | 2065 |
| NM_ 014212 | HOXC11 | 0.43 | -0.29 | 37 | 79 | 347 | 233 |
| NM_ 022658 | HOXC8 | 0.27 | -0.07 | 545 | 751 | 1227 | 1057 |
| NM_ 000195 | HPS1 | 0.06 | -0.10 | 1026 | 1182 | 3644 | 3209 |
| NM_ 022460 | HS1BP3 | 0.28 | -0.22 | 452 | 632 | 1986 | 1580 |
| NM_ 001037811 | HSD17B10 | 0.06 | -0.40 | 517 | 617 | 1054 | 712 |
| NM_ 016246 | HSD17B14 | 0.00 | -0.10 | 31 | 35 | 329 | 253 |
| NM_ 001271969 | HSP90AB1 | 0.47 | -0.08 | 10026 | 14303 | 20597 | 19000 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001541 | HSPB2 | 0.36 | -0.28 | 427 | 635 | 753 | 543 |
| NM_001130106 | HSPBP1 | 0.06 | -0.32 | 391 | 479 | 838 | 593 |
| NM_002156 | HSPD1 | 0.15 | -0.13 | 2386 | 2826 | 3288 | 2824 |
| NM_033158 | HYAL2 | 0.15 | -0.46 | 910 | 1119 | 2830 | 1919 |
| NM_001243526 | HYI | 0.13 | -0.25 | 1583 | 1880 | 1148 | 871 |
| NM_001134793 | HYLS1 | 0.61 | -0.15 | 167 | 318 | 310 | 229 |
| NM_016545 | IER5 | 0.20 | -0.03 | 1035 | 1306 | 1343 | 1196 |
| NM_032036 | IFI27L2 | 0.10 | -0.01 | 420 | 525 | 1169 | 1048 |
| NM_005533 | IFI35 | 0.17 | -0.42 | 231 | 317 | 774 | 506 |
| NM_002038 | IFI6 | 0.00 | -0.14 | 195 | 177 | 168 | 115 |
| NM_003641 | IFITM1 | 0.18 | -0.47 | 294 | 399 | 277 | 159 |
| NM_006435 | IFITM2 | 0.58 | -0.53 | 1733 | 2790 | 2548 | 1640 |
| NM_021034 | IFITM3 | 0.36 | -0.52 | 4256 | 5736 | 5401 | 3591 |
| NM_014055 | IFT81 | 0.00 | -0.25 | 66 | 82 | 262 | 176 |
| NM_003640 | IKBKAP | 0.02 | -0.11 | 957 | 1075 | 2258 | 1941 |
| NM_014002 | IKBKE | 0.22 | -0.09 | 287 | 401 | 584 | 475 |
| NM_001243539 | IL15RA | 0.00 | -0.73 | 164 | 177 | 96 | 37 |
| NM_000877 | IL1R1 | 0.00 | -0.81 | 1086 | 1147 | 2976 | 1582 |
| NM_002185 | IL7R | 0.04 | -0.02 | 831 | 954 | 634 | 545 |
| NM_001267809 | ILF2 | 0.43 | -0.13 | 1344 | 1961 | 1878 | 1580 |
| NM_012218 | ILF3 | 0.00 | -0.04 | 5977 | 5913 | 6830 | 6368 |
| NM_001014795 | ILK | 0.11 | -0.03 | 5437 | 6108 | 8647 | 8164 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_006844 | ILVBL | 0.19 | -0.04 | 902 | 1144 | 1811 | 1630 |
| NM_033416 | IMP4 | 0.28 | -0.07 | 1156 | 1539 | 1788 | 1573 |
| NM_000884 | IMPDH2 | 0.18 | -0.23 | 3398 | 4069 | 5087 | 4142 |
| NM_019892 | INPP5E | 0.17 | -0.16 | 332 | 442 | 730 | 575 |
| NM_020395 | INTS12 | 0.13 | -0.03 | 378 | 485 | 842 | 734 |
| NM_016338 | IPO11 | 0.00 | -0.03 | 446 | 512 | 722 | 621 |
| NM_178229 | IQGAP3 | 0.13 | -0.96 | 1625 | 1927 | 1994 | 933 |
| NM_007199 | IRAK3 | 0.43 | -0.13 | 181 | 304 | 613 | 488 |
| NM_005101 | ISG15 | 0.00 | -0.56 | 166 | 189 | 474 | 270 |
| NM_024710 | ISOC2 | 0.66 | -0.03 | 527 | 944 | 1331 | 1190 |
| NM_018463 | ITFG2 | 0.18 | -0.01 | 252 | 347 | 551 | 473 |
| NM_181501 | ITGA1 | 0.00 | -0.20 | 231 | 253 | 1165 | 913 |
| NM_000885 | ITGA4 | 0.00 | -0.33 | 964 | 1052 | 2258 | 1667 |
| NM_033453 | ITPA | 0.17 | -0.30 | 340 | 453 | 994 | 721 |
| NM_020433 | JPH2 | 0.00 | -0.34 | 508 | 585 | 871 | 610 |
| NM_001130089 | KARS | 0.16 | -0.12 | 1917 | 2303 | 3065 | 2656 |
| NM_001017999 | KAZN | 0.00 | -0.25 | 563 | 571 | 578 | 418 |
| NM_012281 | KCND2 | 0.00 | -0.38 | 265 | 256 | 711 | 475 |
| NM_001017424 | KCNK2 | 0.07 | -0.34 | 2934 | 3257 | 3970 | 2965 |
| NM_173562 | KCTD20 | 0.08 | -0.12 | 5345 | 5895 | 9446 | 8412 |
| NM_153705 | KDELC2 | 0.46 | -0.17 | 892 | 1360 | 1329 | 1074 |
| NM_001251874 | KHDC1 | 0.00 | -0.14 | 116 | 138 | 216 | 153 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_ 003685 | KHSRP | 0.08 | -0.19 | 1516 | 1738 | 1874 | 1519 |
| NM_ 014736 | KIAA0101 | 0.00 | -0.53 | 311 | 261 | 264 | 144 |
| NM_ 014846 | KIAA0196 | 0.00 | -0.04 | 1089 | 1147 | 3045 | 2793 |
| NM_ 001100425 | KIAA0895 | 0.19 | -0.42 | 118 | 177 | 135 | 72 |
| NM_ 020890 | KIAA1524 | 0.15 | -0.61 | 297 | 394 | 403 | 218 |
| NM_ 001029864 | KIAA1755 | 0.00 | -0.11 | 37 | 51 | 216 | 157 |
| NM_ 004523 | KIF11 | 0.00 | -0.35 | 794 | 762 | 485 | 322 |
| NM_ 014875 | KIF14 | 0.00 | -0.33 | 329 | 331 | 381 | 251 |
| NM_ 020242 | KIF15 | 0.43 | -0.60 | 131 | 228 | 179 | 87 |
| NM_ 001122819 | KIF17 | 0.00 | -0.49 | 264 | 249 | 233 | 129 |
| NM_ 031217 | KIF18A | 0.00 | -0.34 | 340 | 281 | 433 | 287 |
| NM_ 001264573 | KIF18B | 0.14 | -1.16 | 719 | 891 | 520 | 192 |
| NM_ 005733 | KIF20A | 0.10 | -1.28 | 1309 | 1528 | 1258 | 458 |
| NM_ 001256269 | KIF22 | 0.15 | -0.48 | 963 | 1183 | 986 | 630 |
| NM_ 138555 | KIF23 | 0.05 | -0.54 | 1094 | 1251 | 1013 | 619 |
| NM_ 006845 | KIF2C | 0.22 | -0.59 | 734 | 959 | 913 | 536 |
| NM_ 012310 | KIF4A | 0.00 | -0.52 | 277 | 322 | 266 | 146 |
| NM_ 001099293 | KIF4B | 0.00 | -0.60 | 343 | 362 | 354 | 190 |
| NM_ 002263 | KIFC1 | 0.10 | -0.36 | 1217 | 1429 | 643 | 434 |
| NM_ 001130100 | KIFC3 | 0.12 | -0.16 | 2078 | 2414 | 3321 | 2800 |
| NM_ 000222 | KIT | 0.30 | -1.55 | 34 | 68 | 77 | 13 |
| NM_ 000899 | KITLG | 0.24 | -0.45 | 1768 | 2247 | 3831 | 2645 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_031918 | KLF16 | 0.00 | -0.20 | 652 | 611 | 630 | 475 |
| NM_003709 | KLF7 | 0.00 | -0.04 | 1855 | 1978 | 5022 | 4665 |
| NM_173546 | KLHDC8B | 0.23 | -0.18 | 529 | 711 | 1464 | 1176 |
| NM_014458 | KLHL20 | 0.00 | -0.07 | 308 | 298 | 559 | 460 |
| NM_032775 | KLHL22 | 0.00 | -0.13 | 722 | 751 | 1477 | 1237 |
| NM_001142761 | KNSTRN | 0.00 | -0.21 | 931 | 911 | 853 | 654 |
| NM_014708 | KNTC1 | 0.02 | -0.38 | 633 | 729 | 786 | 530 |
| NM_002266 | KPNA2 | 0.31 | -0.53 | 1865 | 2491 | 2932 | 1891 |
| NM_002277 | KRT31 | 0.81 | -0.45 | 46 | 120 | 403 | 244 |
| NM_021013 | KRT34 | 0.47 | -0.05 | 1517 | 2268 | 7254 | 6743 |
| NM_031957 | KRTAP1-5 | 0.14 | -0.26 | 328 | 430 | 2514 | 1956 |
| NM_001165252 | KRTAP2-3 | 0.00 | -0.22 | 371 | 327 | 1529 | 1196 |
| NM_016027 | LACTB2 | 0.00 | -0.22 | 154 | 145 | 354 | 251 |
| NM_198129 | LAMA3 | 0.00 | -0.75 | 46 | 51 | 890 | 462 |
| NM_001105207 | LAMA4 | 0.13 | -0.90 | 1519 | 1805 | 7836 | 4002 |
| NM_002291 | LAMB1 | 0.02 | -0.20 | 5680 | 6023 | 17211 | 14556 |
| NM_017907 | LAMTOR1 | 0.33 | -0.23 | 1354 | 1855 | 3768 | 3030 |
| NM_015907 | LAP3 | 0.08 | -0.25 | 422 | 518 | 1215 | 921 |
| NM_015340 | LARS2 | 0.00 | -0.08 | 562 | 643 | 1215 | 1039 |
| NM_031206 | LAS1L | 0.33 | -0.01 | 441 | 638 | 1034 | 924 |
| NM_001258390 | LAYN | 0.18 | -0.66 | 629 | 807 | 2171 | 1268 |
| NM_001170765 | LCOR | 0.21 | -0.04 | 536 | 706 | 776 | 669 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_ 153686 | LCORL | 0.21 | -0.11 | 175 | 255 | 152 | 105 |
| NM_ 001130834 | LDB2 | 0.00 | -0.33 | 213 | 180 | 426 | 283 |
| NM_ 001174097 | LDHB | 0.20 | -0.18 | 2524 | 3087 | 5261 | 4423 |
| NM_ 005567 | LGALS3BP | 0.34 | -0.18 | 4250 | 5634 | 5890 | 4970 |
| NM_ 001136472 | LITAF | 0.00 | -0.03 | 1020 | 1130 | 1395 | 1250 |
| NM_ 170707 | LMNA | 0.24 | -0.22 | 22240 | 26812 | 49065 | 41411 |
| NM_ 005573 | LMNB1 | 0.02 | -0.55 | 816 | 927 | 283 | 153 |
| NM_ 032737 | LMNB2 | 0.00 | -0.45 | 3588 | 3782 | 3070 | 2103 |
| NM_ 012134 | LMOD1 | 0.40 | -0.40 | 198 | 322 | 1522 | 1052 |
| NM_ 001290056 | LOC101927572 | 0.00 | -0.10 | 58 | 61 | 183 | 131 |
| NM_ 001199053 | LOC81691 | 0.79 | -0.07 | 63 | 154 | 160 | 115 |
| NM_ 024036 | LRFN4 | 0.00 | -0.30 | 754 | 825 | 776 | 553 |
| NM_ 153377 | LRIG3 | 0.22 | -0.23 | 120 | 183 | 225 | 150 |
| NM_ 024512 | LRRC2 | 0.19 | -0.47 | 288 | 393 | 208 | 115 |
| NM_ 001278211 | LRRC20 | 0.78 | -0.09 | 198 | 414 | 354 | 277 |
| NM_ 018509 | LRRC59 | 0.13 | -0.02 | 11496 | 12994 | 10752 | 10279 |
| NM_ 001127244 | LRRC8A | 0.12 | -0.02 | 3710 | 4256 | 6709 | 6370 |
| NM_ 024652 | LRRK1 | 0.00 | -0.37 | 600 | 646 | 1279 | 895 |
| NM_ 021177 | LSM2 | 0.26 | -0.53 | 225 | 330 | 435 | 250 |
| NM_ 012321 | LSM4 | 0.00 | -0.34 | 784 | 878 | 2179 | 1590 |
| NM_ 016199 | LSM7 | 0.89 | -0.17 | 224 | 500 | 678 | 525 |
| NM_ 002342 | LTBR | 0.12 | -0.01 | 2724 | 3155 | 5584 | 5288 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001160354 | LY6K | 0.00 | -0.76 | 755 | 802 | 539 | 268 |
| NM_007260 | LYPLA2 | 0.00 | -0.21 | 345 | 344 | 799 | 608 |
| NM_001164841 | LYRM4 | 0.00 | -0.04 | 234 | 277 | 589 | 499 |
| NM_021020 | LZTS1 | 0.00 | -0.25 | 326 | 335 | 304 | 207 |
| NM_002358 | MAD2L1 | 0.35 | -0.43 | 166 | 267 | 208 | 118 |
| NM_016249 | MAGEC2 | 0.00 | -1.31 | 2 | 6 | 87 | 20 |
| NM_001272061 | MAGED4 | 0.34 | -0.08 | 635 | 904 | 730 | 606 |
| NM_001242362 | MAGED4B | 0.34 | -0.08 | 635 | 904 | 730 | 606 |
| NM_002370 | MAGOH | 0.00 | -0.08 | 131 | 110 | 181 | 131 |
| NM_182574 | MAMSTR | 0.00 | -0.61 | 82 | 112 | 129 | 59 |
| NM_005921 | MAP3K1 | 0.00 | -0.06 | 410 | 411 | 333 | 264 |
| NM_003954 | MAP3K14 | 0.55 | -0.08 | 275 | 480 | 607 | 499 |
| NM_002969 | MAPK12 | 0.00 | -0.11 | 469 | 457 | 1133 | 945 |
| NM_001006617 | MAPKAP1 | 0.17 | -0.17 | 2344 | 2822 | 6879 | 5863 |
| NM_004759 | MAPKAPK2 | 0.07 | -0.04 | 3669 | 4067 | 4309 | 3976 |
| NM_001243925 | MAPKAPK3 | 0.31 | -0.02 | 455 | 648 | 1705 | 1555 |
| NM_001128608 | MAPKBP1 | 0.00 | -0.08 | 973 | 1075 | 2292 | 2017 |
| NM_014268 | MAPRE2 | 0.36 | -0.02 | 526 | 772 | 1520 | 1375 |
| NM_001042539 | MAZ | 0.37 | -0.24 | 3022 | 4128 | 3492 | 2784 |
| NM_178496 | MB21D2 | 0.03 | -0.66 | 506 | 596 | 632 | 342 |
| NM_203406 | MBLAC2 | 0.40 | -0.10 | 156 | 261 | 206 | 150 |
| NM_002386 | MC1R | 0.00 | -0.02 | 112 | 140 | 351 | 288 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_022132 | MCCC2 | 0.20 | -0.02 | 347 | 469 | 857 | 754 |
| NM_001112732 | MCF2L | 0.00 | -0.43 | 279 | 284 | 231 | 133 |
| NM_018518 | MCM10 | 0.51 | -0.75 | 474 | 772 | 193 | 85 |
| NM_004526 | MCM2 | 0.11 | -0.20 | 1864 | 2170 | 1389 | 1098 |
| NM_002388 | MCM3 | 0.30 | -0.33 | 2355 | 3087 | 1622 | 1177 |
| NM_005914 | MCM4 | 0.53 | -0.22 | 2312 | 3545 | 2685 | 2157 |
| NM_006739 | MCM5 | 0.08 | -0.16 | 2710 | 3050 | 1868 | 1538 |
| NM_001278595 | MCM7 | 0.02 | -0.31 | 3999 | 4282 | 2398 | 1795 |
| NM_001281520 | MCM8 | 0.36 | -0.01 | 279 | 427 | 362 | 299 |
| NM_014060 | MCTS1 | 0.00 | -0.11 | 240 | 254 | 595 | 479 |
| NM_005918 | MDH2 | 0.22 | -0.16 | 3961 | 4841 | 8106 | 6983 |
| NM_001270550 | MDK | 0.00 | -0.37 | 181 | 182 | 599 | 399 |
| NM_014611 | MDN1 | 0.00 | -0.05 | 1482 | 1458 | 3067 | 2778 |
| NM_001001683 | MED11 | 0.00 | -0.28 | 181 | 227 | 403 | 277 |
| NM_015335 | MED13L | 0.05 | -0.03 | 3484 | 3802 | 5991 | 5623 |
| NM_005481 | MED16 | 0.26 | -0.19 | 994 | 1311 | 2308 | 1885 |
| NM_181491 | MED22 | 0.00 | -0.18 | 498 | 547 | 1341 | 1079 |
| NM_080651 | MED30 | 0.00 | -0.04 | 111 | 88 | 110 | 76 |
| NM_004270 | MED7 | 0.00 | -0.24 | 123 | 105 | 277 | 189 |
| NM_018019 | MED9 | 0.00 | -0.03 | 213 | 264 | 283 | 226 |
| NM_001145784 | MEF2BNB | 0.00 | -0.26 | 176 | 209 | 458 | 322 |
| NM_001193350 | MEF2C | 0.00 | -0.24 | 261 | 251 | 643 | 473 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_002398 | MEIS1 | 0.07 | -0.17 | 252 | 322 | 944 | 749 |
| NM_014791 | MELK | 0.10 | -0.20 | 647 | 783 | 593 | 445 |
| NM_130802 | MEN1 | 0.00 | -0.03 | 952 | 966 | 1059 | 932 |
| NM_022566 | MESDC1 | 0.00 | -0.09 | 368 | 366 | 364 | 285 |
| NM_002402 | MEST | 0.81 | -0.27 | 182 | 393 | 195 | 124 |
| NM_024042 | METRN | 0.13 | -0.01 | 271 | 358 | 900 | 797 |
| NM_005371 | METTL1 | 0.00 | -0.19 | 309 | 267 | 422 | 311 |
| NM_001043229 | METTL12 | 0.00 | -0.17 | 34 | 56 | 100 | 61 |
| NM_015935 | METTL13 | 0.06 | -0.02 | 916 | 1064 | 1524 | 1383 |
| NM_022734 | METTL17 | 0.00 | -0.17 | 469 | 513 | 653 | 506 |
| NM_033418 | METTL18 | 0.00 | -0.44 | 59 | 44 | 83 | 39 |
| NM_019852 | METTL3 | 0.25 | -0.10 | 1047 | 1369 | 1655 | 1420 |
| NM_022840 | METTL4 | 0.00 | -0.29 | 151 | 169 | 335 | 224 |
| NM_001174118 | MEX3D | 0.06 | -0.18 | 529 | 634 | 855 | 667 |
| NM_031433 | MFRP | 0.00 | -0.35 | 469 | 461 | 1221 | 867 |
| NM_004668 | MGAM | 0.00 | -0.18 | 55 | 72 | 54 | 28 |
| NM_002412 | MGMT | 0.00 | -0.02 | 208 | 197 | 349 | 285 |
| NM_032339 | MIEN1 | 0.17 | -0.09 | 733 | 923 | 1522 | 1312 |
| NM_002417 | MKI67 | 0.00 | -0.99 | 5342 | 5358 | 3858 | 1821 |
| NM_170607 | MLX | 0.24 | -0.12 | 1716 | 2180 | 3198 | 2763 |
| NM_002421 | MMP1 | 0.50 | -0.04 | 11316 | 16457 | 6268 | 5830 |
| NM_002426 | MMP12 | 0.05 | -0.23 | 14 | 31 | 98 | 57 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_016155 | MMP17 | 0.00 | -0.29 | 139 | 179 | 591 | 418 |
| NM_002422 | MMP3 | 0.15 | -0.04 | 4384 | 5116 | 17523 | 16658 |
| NM_001286072 | MOV10 | 0.07 | -0.09 | 809 | 947 | 2161 | 1889 |
| NM_004870 | MPDU1 | 0.00 | -0.01 | 1266 | 1280 | 1757 | 1606 |
| NM_002434 | MPG | 0.54 | -0.04 | 617 | 1013 | 1460 | 1301 |
| NM_005590 | MRE11A | 0.37 | -0.24 | 162 | 264 | 389 | 274 |
| NM_001272054 | MRFAP1 | 0.02 | -0.12 | 5828 | 6145 | 9279 | 8268 |
| NM_001098515 | MRGPRF | 0.46 | -0.20 | 249 | 412 | 638 | 482 |
| NM_016050 | MRPL11 | 0.00 | -0.33 | 375 | 421 | 534 | 364 |
| NM_002949 | MRPL12 | 0.19 | -0.30 | 858 | 1089 | 1410 | 1042 |
| NM_032111 | MRPL14 | 0.00 | -0.05 | 1490 | 1541 | 2254 | 2024 |
| NM_017840 | MRPL16 | 0.05 | -0.43 | 420 | 507 | 1044 | 693 |
| NM_017971 | MRPL20 | 0.45 | -0.03 | 451 | 710 | 1204 | 1067 |
| NM_181514 | MRPL21 | 0.03 | -0.11 | 587 | 684 | 932 | 773 |
| NM_021134 | MRPL23 | 0.54 | -0.15 | 490 | 815 | 1129 | 919 |
| NM_145729 | MRPL24 | 0.00 | -0.04 | 383 | 444 | 971 | 848 |
| NM_016504 | MRPL27 | 0.00 | -0.04 | 1023 | 1056 | 1383 | 1225 |
| NM_145212 | MRPL30 | 0.00 | -0.06 | 395 | 429 | 732 | 617 |
| NM_004891 | MRPL33 | 0.05 | -0.27 | 475 | 570 | 747 | 542 |
| NM_032479 | MRPL36 | 0.12 | -0.04 | 647 | 793 | 1140 | 1002 |
| NM_032478 | MRPL38 | 0.01 | -0.12 | 713 | 808 | 1248 | 1041 |
| NM_146388 | MRPL4 | 0.00 | -0.04 | 1023 | 1123 | 1341 | 1189 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_003776 | MRPL40 | 0.31 | -0.31 | 214 | 326 | 786 | 556 |
| NM_032477 | MRPL41 | 0.13 | -0.17 | 463 | 585 | 1381 | 1118 |
| NM_019051 | MRPL50 | 0.00 | -0.40 | 175 | 209 | 412 | 259 |
| NM_016497 | MRPL51 | 0.14 | -0.05 | 974 | 1189 | 1770 | 1579 |
| NM_181304 | MRPL52 | 0.15 | -0.14 | 650 | 816 | 1616 | 1342 |
| NM_053050 | MRPL53 | 0.00 | -0.09 | 150 | 163 | 351 | 274 |
| NM_181462 | MRPL55 | 0.53 | -0.14 | 245 | 427 | 848 | 682 |
| NM_024026 | MRPL57 | 0.44 | -0.31 | 302 | 485 | 890 | 636 |
| NM_031420 | MRPL9 | 0.00 | -0.07 | 794 | 839 | 1231 | 1065 |
| NM_022839 | MRPS11 | 0.29 | -0.28 | 512 | 715 | 1239 | 924 |
| NM_033363 | MRPS12 | 0.38 | -0.15 | 483 | 719 | 932 | 749 |
| NM_031280 | MRPS15 | 0.08 | -0.04 | 976 | 1140 | 2225 | 2015 |
| NM_018135 | MRPS18A | 0.34 | -0.14 | 387 | 572 | 838 | 673 |
| NM_014046 | MRPS18B | 0.00 | -0.02 | 844 | 871 | 1491 | 1346 |
| NM_001286748 | MRPS27 | 0.00 | -0.20 | 1119 | 1112 | 1944 | 1567 |
| NM_023936 | MRPS34 | 0.08 | -0.22 | 964 | 1131 | 1934 | 1534 |
| NM_138777 | MRRF | 0.00 | -0.29 | 213 | 203 | 505 | 351 |
| NM_016183 | MRTO4 | 0.03 | -0.20 | 718 | 824 | 1185 | 932 |
| NM_130385 | MRVI1 | 0.05 | -0.99 | 657 | 768 | 3103 | 1455 |
| NM_001256532 | MSTO1 | 0.65 | -0.15 | 255 | 480 | 903 | 723 |
| NM_005946 | MT1A | 0.00 | -0.18 | 13 | 28 | 121 | 76 |
| NM_001099625 | MTFR1L | 0.35 | -0.03 | 833 | 1177 | 2535 | 2320 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_005956 | MTHFD1 | 0.16 | -0.29 | 1020 | 1258 | 1957 | 1475 |
| NM_001190487 | MTRNR2L6 | 2.14 | -0.03 | 56 | 344 | 1701 | 1536 |
| NM_025128 | MUS81 | 0.64 | -0.04 | 571 | 1003 | 1215 | 1074 |
| NM_002461 | MVD | 0.46 | -0.16 | 276 | 453 | 824 | 654 |
| NM_002466 | MYBL2 | 0.48 | -1.15 | 958 | 1471 | 327 | 115 |
| NM_001163424 | MYEOV2 | 0.00 | -0.10 | 454 | 458 | 749 | 614 |
| NM_021019 | MYL6 | 0.44 | -0.08 | 10866 | 15214 | 22801 | 21125 |
| NM_001199629 | MYL6B | 0.00 | -0.18 | 306 | 282 | 730 | 567 |
| NM_006097 | MYL9 | 0.83 | -0.21 | 10584 | 19391 | 38407 | 32665 |
| NM_001163735 | MYO19 | 0.18 | -0.25 | 1563 | 1921 | 1988 | 1543 |
| NM_004998 | MYO1E | 0.18 | -0.13 | 2962 | 3547 | 8920 | 7887 |
| NM_012335 | MYO1F | 0.00 | -0.13 | 26 | 32 | 83 | 50 |
| NM_013451 | MYOF | 0.11 | -0.13 | 16695 | 18456 | 32033 | 28645 |
| NM_032356 | NAA38 | 0.00 | -0.15 | 635 | 634 | 1402 | 1148 |
| NM_199290 | NACA2 | 0.04 | -0.20 | 1980 | 2190 | 3529 | 2900 |
| NM_144653 | NACC2 | 0.05 | -0.06 | 1557 | 1744 | 2776 | 2493 |
| NM_001018159 | NAE1 | 0.00 | -0.19 | 589 | 632 | 1225 | 974 |
| NM_018946 | NANS | 0.20 | -0.33 | 2987 | 3623 | 3030 | 2264 |
| NM_005969 | NAP1L4 | 0.20 | -0.08 | 1768 | 2187 | 3635 | 3248 |
| NM_003827 | NAPA | 0.49 | -0.07 | 1716 | 2597 | 4852 | 4392 |
| NM_001286829 | NAPRT | 0.00 | -0.10 | 147 | 149 | 208 | 152 |
| NM_001256281 | NARR | 0.34 | -0.05 | 290 | 437 | 757 | 647 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_024678 | NARS2 | 0.27 | -0.35 | 250 | 365 | 403 | 264 |
| NM_002482 | NASP | 0.35 | -0.29 | 578 | 835 | 564 | 396 |
| NM_024662 | NAT10 | 0.24 | -0.03 | 1273 | 1642 | 1716 | 1545 |
| NM_020378 | NAT14 | 0.00 | -0.44 | 94 | 118 | 462 | 287 |
| NM_001244963 | NAV2 | 0.00 | -0.02 | 6546 | 6417 | 7732 | 7325 |
| NM_014865 | NCAPD2 | 0.33 | -0.50 | 1673 | 2269 | 2612 | 1717 |
| NM_015261 | NCAPD3 | 0.05 | -0.17 | 1334 | 1506 | 1429 | 1161 |
| NM_022346 | NCAPG | 0.13 | -0.86 | 696 | 855 | 846 | 405 |
| NM_001281932 | NCAPG2 | 0.04 | -0.72 | 1148 | 1294 | 1323 | 721 |
| NM_001281710 | NCAPH | 0.00 | -0.56 | 679 | 692 | 599 | 347 |
| NM_002486 | NCBP1 | 0.10 | -0.23 | 681 | 826 | 1680 | 1312 |
| NM_014286 | NCS1 | 0.08 | -0.09 | 4139 | 4613 | 10953 | 9972 |
| NM_018087 | NDC1 | 0.00 | -0.08 | 582 | 643 | 724 | 603 |
| NM_006101 | NDC80 | 0.00 | -0.82 | 412 | 393 | 320 | 144 |
| NM_001242835 | NDRG4 | 1.38 | -0.11 | 24 | 105 | 162 | 113 |
| NM_004544 | NDUFA10 | 0.05 | -0.02 | 1363 | 1542 | 2968 | 2749 |
| NM_004542 | NDUFA3 | 0.00 | -0.32 | 261 | 282 | 645 | 447 |
| NM_005001 | NDUFA7 | 0.00 | -0.12 | 401 | 356 | 855 | 697 |
| NM_005003 | NDUFAB1 | 0.03 | -0.25 | 564 | 658 | 1017 | 765 |
| NM_002493 | NDUFB6 | 0.00 | -0.02 | 541 | 597 | 1235 | 1105 |
| NM_004146 | NDUFB7 | 0.23 | -0.11 | 824 | 1075 | 2019 | 1732 |
| NM_002496 | NDUFS8 | 0.20 | -0.33 | 1361 | 1705 | 3783 | 2848 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_ 001166102 | NDUFV1 | 0.00 | -0.11 | 1802 | 1705 | 3433 | 3002 |
| NM_ 021074 | NDUFV2 | 0.00 | -0.10 | 255 | 281 | 433 | 342 |
| NM_ 001144966 | NEDD4L | 0.41 | -0.14 | 517 | 782 | 455 | 349 |
| NM_ 006156 | NEDD8 | 0.18 | -0.17 | 977 | 1224 | 2460 | 2039 |
| NM_ 006403 | NEDD9 | 0.15 | -0.01 | 2617 | 3093 | 2123 | 1956 |
| NM_ 006158 | NEFL | 0.00 | -0.82 | 106 | 99 | 247 | 107 |
| NM_ 018248 | NEIL3 | 0.00 | -0.56 | 151 | 169 | 102 | 46 |
| NM_ 002497 | NEK2 | 0.00 | -1.11 | 170 | 185 | 195 | 65 |
| NM_ 001166171 | NEK6 | 0.23 | -0.38 | 1956 | 2469 | 6370 | 4682 |
| NM_ 001142651 | NEURL1B | 0.02 | -0.96 | 115 | 155 | 116 | 39 |
| NM_ 181832 | NF2 | 0.01 | -0.15 | 6567 | 6905 | 7713 | 6671 |
| NM_ 001134673 | NFIA | 0.00 | -0.47 | 806 | 762 | 753 | 475 |
| NM_ 206915 | NGFRAP1 | 0.06 | -0.04 | 1257 | 1438 | 4527 | 4177 |
| NM_ 017838 | NHP2 | 0.00 | -0.24 | 480 | 495 | 1131 | 861 |
| NM_ 032316 | NICN1 | 0.00 | -0.09 | 100 | 85 | 289 | 220 |
| NM_ 016101 | NIP7 | 0.00 | -0.02 | 716 | 729 | 936 | 828 |
| NM_ 003634 | NIPSNAP1 | 0.09 | -0.11 | 517 | 631 | 1435 | 1214 |
| NM_ 001014445 | NLE1 | 0.58 | -0.17 | 275 | 489 | 611 | 471 |
| NM_ 021077 | NMB | 0.23 | -0.14 | 79 | 129 | 3752 | 3216 |
| NM_ 002512 | NME2 | 0.38 | -0.21 | 2409 | 3329 | 5715 | 4713 |
| NM_ 001286438 | NME4 | 0.16 | -0.21 | 1706 | 2056 | 4852 | 3985 |
| NM_ 012343 | NNT | 0.00 | -0.12 | 1640 | 1771 | 4207 | 3664 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_015658 | NOC2L | 0.47 | -0.16 | 1143 | 1730 | 2408 | 2006 |
| NM_015462 | NOL11 | 0.53 | -0.20 | 205 | 363 | 416 | 305 |
| NM_022917 | NOL6 | 0.19 | -0.40 | 1820 | 2227 | 2803 | 1985 |
| NM_001145408 | NONO | 0.44 | -0.12 | 2593 | 3736 | 5299 | 4662 |
| NM_016391 | NOP16 | 0.04 | -0.29 | 713 | 826 | 1179 | 869 |
| NM_015934 | NOP58 | 0.00 | -0.05 | 967 | 953 | 944 | 817 |
| NM_001270960 | NOSIP | 0.00 | -0.06 | 347 | 300 | 586 | 488 |
| NM_002514 | NOV | 0.43 | -0.19 | 457 | 710 | 216 | 148 |
| NM_001291926 | NOX4 | 0.57 | -0.12 | 22 | 58 | 166 | 115 |
| NM_000272 | NPHP1 | 0.00 | -0.21 | 13 | 6 | 127 | 79 |
| NM_002520 | NPM1 | 0.00 | -0.24 | 1772 | 1914 | 2387 | 1878 |
| NM_006993 | NPM3 | 0.26 | -0.47 | 270 | 388 | 730 | 458 |
| NM_000906 | NPR1 | 0.00 | -0.48 | 47 | 68 | 62 | 26 |
| NM_001243247 | NPRL3 | 0.00 | -0.28 | 778 | 835 | 1106 | 821 |
| NM_002522 | NPTX1 | 0.00 | -0.70 | 210 | 203 | 512 | 264 |
| NM_000903 | NQO1 | 0.63 | -0.08 | 5148 | 8328 | 15747 | 14464 |
| NM_021005 | NR2F2 | 0.13 | -0.14 | 2093 | 2448 | 3047 | 2590 |
| NM_000901 | NR3C2 | 0.50 | -0.15 | 612 | 974 | 1996 | 1664 |
| NM_001142481 | NREP | 0.00 | -0.11 | 579 | 628 | 2186 | 1885 |
| NM_198465 | NRK | 0.27 | -0.46 | 98 | 159 | 385 | 231 |
| NM_001270707 | NRM | 0.00 | -0.55 | 264 | 309 | 470 | 268 |
| NM_001286798 | NTMT1 | 0.18 | -0.05 | 1671 | 2042 | 2113 | 1893 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001113226 | NTNG1 | 0.00 | -0.21 | 174 | 191 | 510 | 377 |
| NM_030952 | NUAK2 | 0.14 | -0.90 | 477 | 607 | 443 | 194 |
| NM_025152 | NUBPL | 0.43 | -0.25 | 42 | 87 | 239 | 159 |
| NM_001193452 | NUDT16L1 | 0.00 | -0.28 | 306 | 273 | 643 | 458 |
| NM_145697 | NUF2 | 0.19 | -0.84 | 215 | 302 | 254 | 109 |
| NM_018230 | NUP133 | 0.65 | -0.07 | 508 | 904 | 1275 | 1105 |
| NM_004298 | NUP155 | 0.16 | -0.18 | 879 | 1091 | 1433 | 1152 |
| NM_015354 | NUP188 | 0.29 | -0.27 | 3261 | 4208 | 5817 | 4597 |
| NM_005085 | NUP214 | 0.00 | -0.09 | 1834 | 1883 | 3180 | 2813 |
| NM_024844 | NUP85 | 0.63 | -0.12 | 635 | 1103 | 1144 | 950 |
| NM_014669 | NUP93 | 0.37 | -0.06 | 1342 | 1885 | 2206 | 1965 |
| NM_001243142 | NUSAP1 | 0.00 | -0.76 | 794 | 883 | 661 | 335 |
| NM_022463 | NXN | 0.03 | -0.02 | 1270 | 1418 | 1400 | 1266 |
| NM_178507 | OAF | 0.10 | -0.13 | 2203 | 2531 | 4962 | 4310 |
| NM_006187 | OAS3 | 0.01 | -0.27 | 144 | 188 | 162 | 100 |
| NM_000274 | OAT | 0.02 | -0.20 | 2509 | 2712 | 4908 | 4072 |
| NM_002540 | ODF2 | 0.36 | -0.03 | 1086 | 1529 | 2003 | 1815 |
| NM_015441 | OLFML2B | 0.05 | -0.73 | 299 | 372 | 772 | 405 |
| NM_001286352 | OLFML3 | 0.06 | -0.13 | 1102 | 1261 | 3444 | 2969 |
| NM_012381 | ORC3 | 0.06 | -0.26 | 167 | 221 | 501 | 355 |
| NM_014321 | ORC6 | 0.00 | -0.31 | 450 | 419 | 354 | 235 |
| NM_017807 | OSGEP | 0.26 | -0.36 | 263 | 380 | 732 | 497 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_145260 | OSR1 | 0.00 | -0.19 | 478 | 533 | 890 | 695 |
| NM_012383 | OSTF1 | 0.00 | -0.11 | 1081 | 1123 | 2200 | 1895 |
| NM_000436 | OXCT1 | 0.16 | -0.17 | 473 | 609 | 1169 | 939 |
| NM_000916 | OXTR | 0.07 | -1.20 | 13965 | 15094 | 10394 | 4346 |
| NM_006191 | PA2G4 | 0.35 | -0.50 | 1583 | 2177 | 2375 | 1555 |
| NM_001114734 | PABPC4L | 0.00 | -0.62 | 117 | 101 | 235 | 118 |
| NM_019088 | PAF1 | 0.21 | -0.05 | 756 | 977 | 1368 | 1209 |
| NM_002573 | PAFAH1B3 | 0.07 | -0.08 | 314 | 392 | 830 | 697 |
| NM_024516 | PAGR1 | 0.00 | -0.01 | 633 | 691 | 884 | 782 |
| NM_007203 | PALM2-AKAP2 | 0.22 | -0.15 | 8867 | 10707 | 24263 | 21369 |
| NM_001618 | PARP1 | 0.24 | -0.36 | 1308 | 1681 | 1682 | 1201 |
| NM_032789 | PARP10 | 0.00 | -0.27 | 274 | 306 | 559 | 397 |
| NM_001003931 | PARP3 | 0.00 | -0.02 | 594 | 656 | 1412 | 1272 |
| NM_031458 | PARP9 | 0.46 | -0.14 | 146 | 256 | 520 | 405 |
| NM_017915 | PARPBP | 0.00 | -0.23 | 126 | 128 | 200 | 131 |
| NM_018492 | PBK | 0.00 | -0.48 | 516 | 519 | 401 | 237 |
| NM_019035 | PCDH18 | 0.49 | -0.39 | 1973 | 2962 | 6738 | 4915 |
| NM_007144 | PCGF2 | 0.00 | -0.17 | 1277 | 1386 | 1942 | 1599 |
| NM_002592 | PCNA | 0.40 | -0.21 | 1467 | 2090 | 1543 | 1222 |
| NM_002593 | PCOLCE | 0.10 | -0.23 | 1538 | 1782 | 3084 | 2462 |
| NM_004716 | PCSK7 | 0.40 | -0.21 | 2203 | 3109 | 4952 | 4072 |
| NM_001184917 | PCYT2 | 0.05 | -0.05 | 448 | 538 | 1678 | 1492 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_ 014976 | PDCD11 | 0.00 | -0.13 | 1820 | 1941 | 2381 | 2026 |
| NM_ 004708 | PDCD5 | 0.00 | -0.05 | 188 | 236 | 441 | 362 |
| NM_ 024065 | PDCL3 | 0.00 | -0.28 | 139 | 139 | 289 | 192 |
| NM_ 018945 | PDE7B | 0.00 | -0.10 | 509 | 471 | 643 | 523 |
| NM_ 022341 | PDF | 0.00 | -0.01 | 198 | 247 | 451 | 381 |
| NM_ 002607 | PDGFA | 0.32 | -0.04 | 612 | 866 | 790 | 682 |
| NM_ 006206 | PDGFRA | 0.15 | -0.63 | 3877 | 4533 | 5085 | 3109 |
| NM_ 001173454 | PDHA1 | 0.18 | -0.03 | 1069 | 1328 | 1770 | 1604 |
| NM_ 001173468 | PDHB | 0.00 | -0.02 | 1090 | 1144 | 2069 | 1900 |
| NM_ 021630 | PDLIM2 | 0.00 | -0.03 | 1881 | 2005 | 4074 | 3773 |
| NM_ 001080471 | PEAR1 | 0.21 | -1.16 | 1926 | 2387 | 510 | 187 |
| NM_ 001040152 | PEG10 | 0.14 | -0.32 | 1116 | 1351 | 616 | 425 |
| NM_ 001135690 | PENK | 0.60 | -1.15 | 4042 | 6409 | 3625 | 1525 |
| NM_ 000285 | PEPD | 0.42 | -0.23 | 1234 | 1795 | 2797 | 2238 |
| NM_ 004813 | PEX16 | 0.00 | -0.22 | 326 | 378 | 801 | 608 |
| NM_ 012393 | PFAS | 0.36 | -0.47 | 716 | 1026 | 1198 | 778 |
| NM_ 002622 | PFDN1 | 0.68 | -0.15 | 484 | 884 | 1651 | 1366 |
| NM_ 000289 | PFKM | 0.00 | -0.16 | 1855 | 1786 | 4190 | 3564 |
| NM_ 033419 | PGAP3 | 0.10 | -0.08 | 118 | 167 | 528 | 431 |
| NM_ 001100164 | PHACTR2 | 0.00 | -0.12 | 4592 | 4615 | 5384 | 4739 |
| NM_ 001048183 | PHACTR4 | 0.08 | -0.06 | 620 | 743 | 859 | 732 |
| NM_ 001281496 | PHB | 0.05 | -0.21 | 1356 | 1529 | 2793 | 2257 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001144831 | PHB2 | 0.13 | -0.25 | 2884 | 3352 | 5103 | 4100 |
| NM_015651 | PHF19 | 0.33 | -0.03 | 1010 | 1397 | 1291 | 1152 |
| NM_005392 | PHF2 | 0.18 | -0.02 | 1074 | 1334 | 1778 | 1614 |
| NM_032758 | PHF5A | 0.00 | -0.20 | 391 | 409 | 528 | 394 |
| NM_001134438 | PHLDB2 | 0.31 | -0.13 | 783 | 1079 | 2263 | 1928 |
| NM_001100876 | PHYHD1 | 0.48 | -0.50 | 39 | 85 | 239 | 131 |
| NM_058004 | PI4KA | 0.17 | -0.08 | 2111 | 2551 | 6416 | 5828 |
| NM_001286496 | PIF1 | 0.00 | -0.13 | 167 | 127 | 258 | 189 |
| NM_002642 | PIGC | 0.00 | -0.08 | 163 | 160 | 262 | 200 |
| NM_004204 | PIGQ | 0.00 | -0.03 | 434 | 410 | 886 | 773 |
| NM_080476 | PIGU | 0.39 | -0.13 | 231 | 367 | 580 | 460 |
| NM_001193621 | PINLYP | 0.07 | -0.24 | 38 | 64 | 89 | 50 |
| NM_001135638 | PIP5K1A | 0.31 | -0.13 | 1294 | 1751 | 1531 | 1277 |
| NM_014338 | PISD | 0.10 | -0.13 | 728 | 875 | 1321 | 1100 |
| NM_181805 | PKIG | 0.25 | -0.17 | 599 | 809 | 1275 | 1028 |
| NM_000929 | PLA2G5 | 0.00 | -0.14 | 91 | 108 | 98 | 61 |
| NM_001288989 | PLCE1 | 0.00 | -0.04 | 185 | 178 | 464 | 384 |
| NM_020904 | PLEKHA4 | 0.00 | -0.11 | 173 | 216 | 482 | 381 |
| NM_022835 | PLEKHG2 | 0.46 | -0.51 | 431 | 683 | 1470 | 939 |
| NM_015432 | PLEKHG4 | 0.22 | -0.41 | 528 | 704 | 1414 | 963 |
| NM_172069 | PLEKHH2 | 0.51 | -0.08 | 48 | 102 | 364 | 287 |
| NM_001164189 | PLIN3 | 0.14 | -0.04 | 7305 | 8341 | 16793 | 15924 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_005030 | PLK1 | 0.39 | -1.00 | 957 | 1382 | 1177 | 521 |
| NM_014264 | PLK4 | 0.00 | -0.34 | 427 | 469 | 291 | 185 |
| NM_021127 | PMAIP1 | 0.43 | -0.26 | 200 | 332 | 204 | 131 |
| NM_001199653 | PMF1 | 0.01 | -0.24 | 412 | 486 | 934 | 702 |
| NM_001199662 | PMF1-BGLAP | 0.00 | -0.16 | 371 | 378 | 676 | 529 |
| NM_002676 | PMM1 | 0.43 | -0.06 | 732 | 1104 | 2978 | 2688 |
| NM_001282946 | PMPCA | 0.03 | -0.08 | 1069 | 1207 | 2352 | 2079 |
| NM_006556 | PMVK | 0.03 | -0.08 | 370 | 444 | 973 | 824 |
| NM_001077399 | PNKD | 0.65 | -0.37 | 83 | 177 | 487 | 318 |
| NM_004650 | PNPLA4 | 0.00 | -0.53 | 120 | 148 | 258 | 140 |
| NM_033109 | PNPT1 | 0.00 | -0.07 | 222 | 192 | 237 | 179 |
| NM_001161581 | POC1A | 0.00 | -0.42 | 389 | 419 | 464 | 292 |
| NM_002689 | POLA2 | 0.62 | -0.14 | 547 | 950 | 728 | 579 |
| NM_001278255 | POLE3 | 0.53 | -0.02 | 782 | 1257 | 1229 | 1100 |
| NM_019896 | POLE4 | 0.00 | -0.04 | 459 | 496 | 1036 | 908 |
| NM_006502 | POLH | 0.13 | -0.08 | 1379 | 1641 | 2244 | 1978 |
| NM_199420 | POLQ | 0.00 | -0.73 | 358 | 388 | 337 | 163 |
| NM_015425 | POLR1A | 0.18 | -0.12 | 1411 | 1739 | 3067 | 2660 |
| NM_001282777 | POLR1B | 0.00 | -0.33 | 478 | 498 | 693 | 481 |
| NM_002695 | POLR2E | 0.01 | -0.09 | 2075 | 2237 | 3937 | 3508 |
| NM_021128 | POLR2L | 0.40 | -0.45 | 2046 | 2889 | 5698 | 3989 |
| NM_176869 | PPA2 | 0.71 | -0.22 | 182 | 368 | 1079 | 834 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_ 003713 | PPAP2B | 0.48 | -0.45 | 1703 | 2546 | 2327 | 1579 |
| NM_ 203453 | PPAPDC2 | 0.64 | -0.17 | 58 | 130 | 260 | 185 |
| NM_ 032728 | PPAPDC3 | 0.00 | -0.28 | 172 | 212 | 728 | 523 |
| NM_ 138712 | PPARG | 0.00 | -0.78 | 277 | 283 | 185 | 79 |
| NM_ 006347 | PPIH | 0.39 | -0.19 | 84 | 151 | 252 | 176 |
| NM_ 014634 | PPM1F | 0.23 | -0.11 | 1853 | 2335 | 3575 | 3139 |
| NM_ 021959 | PPP1R11 | 0.30 | -0.01 | 676 | 936 | 2075 | 1913 |
| NM_ 138689 | PPP1R14B | 0.15 | -0.22 | 782 | 971 | 1585 | 1250 |
| NM_ 145030 | PPP1R35 | 0.03 | -0.09 | 207 | 262 | 302 | 231 |
| NM_ 178001 | PPP2R4 | 0.48 | -0.15 | 1780 | 2666 | 5669 | 4867 |
| NM_ 000310 | PPT1 | 0.18 | -0.01 | 1067 | 1330 | 2017 | 1854 |
| NM_ 003981 | PRC1 | 0.03 | -0.98 | 2695 | 2934 | 2502 | 1170 |
| NM_ 013237 | PRELID1 | 0.49 | -0.06 | 1259 | 1927 | 3151 | 2850 |
| NM_ 020820 | PREX1 | 0.00 | -0.10 | 114 | 111 | 193 | 139 |
| NM_ 001291314 | PRH1 | 0.00 | -0.15 | 182 | 153 | 362 | 270 |
| NM_ 000947 | PRIM2 | 0.37 | -0.14 | 362 | 549 | 460 | 355 |
| NM_ 206907 | PRKAA1 | 0.07 | -0.02 | 994 | 1156 | 2136 | 1957 |
| NM_ 006253 | PRKAB1 | 0.00 | -0.03 | 382 | 410 | 678 | 584 |
| NM_ 145040 | PRKCDBP | 0.05 | -0.27 | 975 | 1117 | 2261 | 1743 |
| NM_ 006904 | PRKDC | 0.05 | -0.04 | 6452 | 6943 | 13025 | 12318 |
| NM_ 005044 | PRKX | 0.00 | -0.17 | 134 | 128 | 206 | 142 |
| NM_ 014502 | PRPF19 | 0.02 | -0.04 | 2027 | 2207 | 2051 | 1858 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_002764 | PRPS1 | 0.38 | -0.37 | 2762 | 3797 | 4731 | 3486 |
| NM_001039091 | PRPS2 | 0.00 | -0.03 | 618 | 690 | 584 | 497 |
| NM_018304 | PRR11 | 0.00 | -0.78 | 1439 | 1460 | 1633 | 860 |
| NM_018457 | PRR13 | 0.33 | -0.09 | 395 | 577 | 1553 | 1338 |
| NM_016644 | PRR16 | 0.18 | -0.21 | 413 | 545 | 424 | 309 |
| NM_004638 | PRRC2A | 0.18 | -0.12 | 8554 | 10007 | 12164 | 10817 |
| NM_016307 | PRRX2 | 0.10 | -0.27 | 482 | 597 | 776 | 566 |
| NM_003619 | PRSS12 | 0.00 | -0.06 | 1843 | 1817 | 1583 | 1392 |
| NM_001184825 | PSG1 | 0.36 | -0.04 | 110 | 187 | 204 | 155 |
| NM_021016 | PSG3 | 0.00 | -0.05 | 218 | 267 | 343 | 274 |
| NM_002780 | PSG4 | 0.34 | -0.07 | 1589 | 2168 | 3350 | 3018 |
| NM_002790 | PSMA5 | 0.23 | -0.19 | 1051 | 1360 | 1915 | 1547 |
| NM_001282232 | PSMA6 | 0.34 | -0.07 | 730 | 1034 | 1458 | 1268 |
| NM_002792 | PSMA7 | 0.10 | -0.11 | 3420 | 3876 | 6549 | 5802 |
| NM_002795 | PSMB3 | 0.19 | -0.10 | 1288 | 1607 | 3259 | 2872 |
| NM_001144932 | PSMB5 | 0.37 | -0.06 | 3103 | 4225 | 6906 | 6342 |
| NM_001270481 | PSMB6 | 0.13 | -0.05 | 2176 | 2554 | 4586 | 4218 |
| NM_002799 | PSMB7 | 0.00 | -0.07 | 3400 | 3560 | 7530 | 6909 |
| NM_004159 | PSMB8 | 0.17 | -0.19 | 420 | 549 | 861 | 669 |
| NM_002800 | PSMB9 | 0.00 | -1.12 | 91 | 124 | 171 | 55 |
| NM_002803 | PSMC2 | 0.10 | -0.12 | 880 | 1046 | 2063 | 1760 |
| NM_002804 | PSMC3 | 0.22 | -0.21 | 1655 | 2085 | 4143 | 3397 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_002807 | PSMD1 | 0.16 | -0.01 | 2277 | 2720 | 5162 | 4882 |
| NM_002817 | PSMD13 | 0.41 | -0.05 | 2208 | 3125 | 4723 | 4344 |
| NM_005047 | PSMD5 | 0.06 | -0.06 | 911 | 1056 | 2073 | 1845 |
| NM_002813 | PSMD9 | 0.06 | -0.09 | 1517 | 1712 | 2795 | 2460 |
| NM_002818 | PSME2 | 0.46 | -0.17 | 131 | 232 | 430 | 322 |
| NM_001267045 | PSME3 | 0.30 | -0.05 | 2495 | 3266 | 4166 | 3811 |
| NM_032302 | PSMG3 | 0.30 | -0.37 | 315 | 460 | 510 | 336 |
| NM_001128592 | PSMG4 | 0.17 | -0.01 | 129 | 189 | 212 | 165 |
| NM_001032291 | PSRC1 | 0.78 | -0.66 | 138 | 300 | 304 | 153 |
| NM_024430 | PSTPIP2 | 0.41 | -0.09 | 237 | 382 | 428 | 340 |
| NM_002819 | PTBP1 | 0.12 | -0.04 | 4837 | 5496 | 5499 | 5122 |
| NM_001284405 | PTCD2 | 0.00 | -0.12 | 156 | 127 | 299 | 224 |
| NM_001166292 | PTCH2 | 1.39 | -0.50 | 1767 | 4937 | 3298 | 2185 |
| NM_000956 | PTGER2 | 0.00 | -0.34 | 61 | 44 | 376 | 246 |
| NM_001136042 | PTGES3L-AARSD1 | 0.33 | -0.04 | 259 | 391 | 532 | 445 |
| NM_002824 | PTMS | 1.00 | -0.05 | 2432 | 5150 | 3406 | 3107 |
| NM_001010915 | PTPLAD2 | 0.00 | -0.03 | 43 | 44 | 125 | 89 |
| NM_015967 | PTPN22 | 0.00 | -0.40 | 7 | 12 | 206 | 120 |
| NM_016077 | PTRH2 | 0.00 | -0.04 | 416 | 473 | 796 | 684 |
| NM_001282382 | PTTG1 | 0.27 | -1.35 | 389 | 548 | 595 | 192 |
| NM_002852 | PTX3 | 0.51 | -0.41 | 8141 | 11999 | 2774 | 1952 |
| NM_013328 | PYCR2 | 0.00 | -0.05 | 1366 | 1453 | 1824 | 1627 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_023078 | PYCRL | 0.00 | -0.46 | 192 | 232 | 451 | 275 |
| NM_005051 | OARS | 0.00 | -0.04 | 2528 | 2638 | 4257 | 3935 |
| NM_014298 | QPRT | 0.00 | -0.27 | 11 | 26 | 651 | 468 |
| NM_018292 | QRSL1 | 0.03 | -0.22 | 174 | 224 | 393 | 281 |
| NM_006834 | RAB32 | 0.85 | -0.08 | 1109 | 2178 | 2263 | 1987 |
| NM_004283 | RAB3D | 0.00 | -0.10 | 270 | 238 | 572 | 462 |
| NM_013401 | RAB3IL1 | 0.15 | -0.05 | 256 | 343 | 1073 | 932 |
| NM_006822 | RAB40B | 0.00 | -0.09 | 122 | 141 | 424 | 335 |
| NM_016154 | RAB4B | 0.00 | -0.09 | 204 | 243 | 657 | 538 |
| NM_004251 | RAB9A | -0.01 | -0.02 | 585 | 505 | 838 | 736 |
| NM_005833 | RABEPK | 0.00 | -0.11 | 545 | 606 | 1225 | 1030 |
| NM_001173988 | RABL6 | 0.00 | -0.02 | 2179 | 2117 | 3683 | 3449 |
| NM_005052 | RAC3 | 0.00 | -0.10 | 82 | 94 | 177 | 126 |
| NM_001126104 | RACGAP1 | 0.00 | -0.55 | 1187 | 1254 | 1237 | 758 |
| NM_020165 | RAD18 | 0.00 | -0.12 | 681 | 643 | 609 | 488 |
| NM_001270362 | RAD23A | 0.06 | -0.18 | 3990 | 4387 | 6726 | 5700 |
| NM_002875 | RAD51 | 0.07 | -0.44 | 380 | 469 | 297 | 176 |
| NM_001130862 | RAD51AP1 | 0.00 | -0.28 | 195 | 205 | 110 | 63 |
| NM_001142571 | RAD51D | 0.00 | -0.06 | 240 | 247 | 283 | 220 |
| NM_012415 | RAD54B | 0.00 | -0.48 | 116 | 115 | 119 | 59 |
| NM_152663 | RALGPS2 | 0.00 | -0.53 | 707 | 794 | 1385 | 865 |
| NM_005855 | RAMP1 | 0.00 | -0.34 | 36 | 42 | 156 | 91 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001278639 | RANBP1 | 0.34 | -0.21 | 347 | 517 | 624 | 468 |
| NM_001278651 | RANGAP1 | 0.00 | -0.07 | 6719 | 6809 | 7876 | 7238 |
| NM_017805 | RASIP1 | 0.29 | -0.90 | 334 | 482 | 863 | 401 |
| NM_178169 | RASSF3 | 0.20 | -0.43 | 903 | 1148 | 2117 | 1449 |
| NM_001204467 | RBM10 | 0.01 | -0.12 | 1571 | 1713 | 2061 | 1756 |
| NM_006047 | RBM12 | 0.06 | -0.20 | 2660 | 2953 | 2425 | 1974 |
| NM_006328 | RBM14 | 0.04 | -0.06 | 1616 | 1800 | 1778 | 1571 |
| NM_001198845 | RBM14-RBM4 | 0.00 | -0.03 | 1303 | 1253 | 2200 | 2009 |
| NM_152945 | RBM45 | 0.00 | -0.30 | 150 | 133 | 268 | 174 |
| NM_001098634 | RBM47 | 0.00 | -0.25 | 57 | 45 | 383 | 268 |
| NM_032120 | RBM48 | 0.00 | -0.04 | 94 | 111 | 139 | 100 |
| NM_001003793 | RBMS3 | 0.00 | -0.28 | 1828 | 1872 | 2119 | 1614 |
| NM_006867 | RBPMS | 0.00 | -0.10 | 868 | 949 | 1081 | 911 |
| NM_001032279 | RCE1 | 0.00 | -0.12 | 285 | 327 | 397 | 307 |
| NM_025232 | REEP4 | 0.04 | -0.21 | 478 | 569 | 616 | 462 |
| NM_005045 | RELN | 0.17 | -0.29 | 2490 | 2989 | 925 | 671 |
| NM_020385 | REXO4 | 0.00 | -0.18 | 640 | 642 | 1102 | 878 |
| NM_001278791 | RFC2 | 0.21 | -0.03 | 457 | 611 | 493 | 414 |
| NM_002915 | RFC3 | 0.00 | -0.49 | 403 | 440 | 308 | 176 |
| NM_002916 | RFC4 | 0.07 | -0.17 | 422 | 516 | 318 | 231 |
| NM_018339 | RFK | 0.23 | -0.02 | 374 | 513 | 788 | 688 |
| NM_018124 | RFWD3 | 0.11 | -0.18 | 1265 | 1495 | 1346 | 1081 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_003721 | RFXANK | 0.32 | -0.26 | 606 | 853 | 1395 | 1057 |
| NM_001012761 | RGMB | 0.44 | -0.12 | 6096 | 8610 | 9602 | 8534 |
| NM_001282848 | RGN | 0.39 | -0.49 | 31 | 67 | 195 | 105 |
| NM_003702 | RGS20 | 0.00 | -0.31 | 48 | 41 | 173 | 105 |
| NM_001282871 | RHD | 0.00 | -0.35 | 44 | 54 | 106 | 57 |
| NM_014578 | RHOD | 0.41 | -0.21 | 193 | 317 | 670 | 505 |
| NM_031430 | RILP | 0.00 | -0.05 | 82 | 86 | 283 | 222 |
| NM_032848 | RITA1 | 0.00 | -0.04 | 305 | 313 | 638 | 542 |
| NM_006397 | RNASEH2A | 0.00 | -0.40 | 807 | 755 | 815 | 543 |
| NM_032015 | RNF26 | 0.00 | -0.39 | 1620 | 1637 | 2196 | 1553 |
| NM_203387 | RNH1 | 0.25 | -0.01 | 5557 | 6898 | 14959 | 14456 |
| NM_001286076 | RPA2 | 0.23 | -0.06 | 400 | 547 | 532 | 440 |
| NM_032194 | RPF2 | 0.00 | -0.04 | 269 | 294 | 395 | 323 |
| NM_006013 | RPL10 | 0.15 | -0.08 | 4357 | 5056 | 9288 | 8468 |
| NM_007104 | RPL10A | 0.27 | -0.39 | 1480 | 1938 | 3232 | 2307 |
| NM_000975 | RPL11 | 0.07 | -0.12 | 4723 | 5204 | 8961 | 7965 |
| NM_000976 | RPL12 | 1.00 | -0.11 | 317 | 741 | 1013 | 843 |
| NM_033251 | RPL13 | 0.41 | -0.38 | 4442 | 6194 | 10648 | 7885 |
| NM_012423 | RPL13A | 0.44 | -0.22 | 2671 | 3849 | 5987 | 4915 |
| NM_001253382 | RPL15 | 0.26 | -0.24 | 6011 | 7518 | 14955 | 12327 |
| NM_000979 | RPL18 | 0.11 | -0.29 | 4194 | 4757 | 7699 | 6039 |
| NM_000980 | RPL18A | 0.49 | -0.41 | 1354 | 2065 | 3525 | 2503 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_000981 | RPL19 | 0.64 | -0.23 | 1380 | 2332 | 3506 | 2817 |
| NM_000983 | RPL22 | 0.19 | -0.01 | 1208 | 1507 | 2658 | 2466 |
| NM_001099645 | RPL22L1 | 0.00 | -1.05 | 699 | 675 | 759 | 314 |
| NM_000984 | RPL23A | 0.24 | -0.12 | 1690 | 2158 | 3078 | 2671 |
| NM_000986 | RPL24 | 1.00 | -0.16 | 345 | 801 | 980 | 784 |
| NM_000987 | RPL26 | 0.31 | -0.09 | 1272 | 1720 | 3084 | 2725 |
| NM_016093 | RPL26L1 | 0.00 | -0.05 | 234 | 285 | 553 | 462 |
| NM_000990 | RPL27 A | 0.12 | -0.03 | 2132 | 2480 | 3388 | 3122 |
| NM_001136134 | RPL28 | 0.69 | -0.02 | 4317 | 7259 | 9013 | 8553 |
| NM_000967 | RPL3 | 0.45 | -0.28 | 6338 | 8994 | 11900 | 9482 |
| NM_001099693 | RPL31 | 0.43 | -0.13 | 380 | 597 | 1027 | 843 |
| NM_001007074 | RPL32 | 0.97 | -0.46 | 3916 | 8037 | 68665 | 49312 |
| NM_033625 | RPL34 | 0.33 | -0.20 | 374 | 548 | 772 | 593 |
| NM_007209 | RPL35 | 0.07 | -0.22 | 2140 | 2415 | 5538 | 4532 |
| NM_033643 | RPL36 | 0.80 | -0.35 | 822 | 1579 | 2196 | 1593 |
| NM_021029 | RPL36A | 0.13 | -0.52 | 69 | 108 | 166 | 85 |
| NM_052969 | RPL39L | 1.09 | -0.17 | 82 | 234 | 302 | 218 |
| NM_000968 | RPL4 | 0.31 | -0.31 | 4844 | 6298 | 10964 | 8527 |
| NM_000969 | RPL5 | 0.22 | -0.12 | 2280 | 2836 | 5160 | 4514 |
| NM_001024662 | RPL6 | 0.00 | -0.02 | 2195 | 2240 | 3596 | 3364 |
| NM_000971 | RPL7 | 0.39 | -0.16 | 506 | 757 | 1111 | 893 |
| NM_000972 | RPL7A | 0.36 | -0.45 | 2918 | 3971 | 7740 | 5431 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001024921 | RPL9 | 1.18 | -0.12 | 389 | 1013 | 1096 | 906 |
| NM_053275 | RPLP0 | 0.31 | -0.17 | 10422 | 13300 | 18261 | 15881 |
| NM_001003 | RPLP1 | 0.70 | -0.34 | 1964 | 3408 | 3157 | 2338 |
| NM_001004 | RPLP2 | 1.11 | -0.03 | 1117 | 2624 | 2797 | 2577 |
| NM_001017 | RPS13 | 0.04 | -0.17 | 752 | 870 | 1281 | 1035 |
| NM_001025070 | RPS14 | 0.87 | -0.48 | 1170 | 2319 | 4280 | 2911 |
| NM_001018 | RPS15 | 0.38 | -0.55 | 1636 | 2301 | 4648 | 3017 |
| NM_001020 | RPS16 | 0.46 | -0.24 | 2732 | 3995 | 5147 | 4159 |
| NM_022551 | RPS18 | 0.90 | -0.26 | 517 | 1090 | 1845 | 1416 |
| NM_002952 | RPS2 | 0.18 | -0.35 | 4327 | 5142 | 7042 | 5303 |
| NM_001025 | RPS23 | 0.60 | -0.38 | 1525 | 2500 | 3660 | 2652 |
| NM_001029 | RPS26 | 0.00 | -0.08 | 258 | 308 | 576 | 471 |
| NM_015920 | RPS27L | 0.16 | -0.13 | 1071 | 1313 | 3228 | 2773 |
| NM_001260506 | RPS3 | 0.23 | -0.35 | 5032 | 6168 | 8541 | 6445 |
| NM_001007 | RPS4X | 0.20 | -0.17 | 3478 | 4204 | 6929 | 5928 |
| NM_001009 | RPS5 | 0.54 | -0.29 | 1682 | 2634 | 3764 | 2915 |
| NM_001010 | RPS6 | 0.22 | -0.25 | 9254 | 11144 | 18727 | 15381 |
| NM_021135 | RPS6KA2 | 0.19 | -0.20 | 4011 | 4813 | 7445 | 6236 |
| NM_001006944 | RPS6KA4 | 0.26 | -0.32 | 994 | 1311 | 1805 | 1327 |
| NM_001011 | RPS7 | 0.53 | -0.62 | 972 | 1548 | 2452 | 1477 |
| NM_001012 | RPS8 | 0.24 | -0.28 | 5557 | 6843 | 12336 | 9848 |
| NM_001013 | RPS9 | 0.00 | -0.46 | 1923 | 1876 | 2824 | 1920 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_ 002295 | RPSA | 0.00 | -0.14 | 3931 | 3743 | 4311 | 3708 |
| NM_ 173659 | RPUSD3 | 0.00 | -0.25 | 493 | 453 | 815 | 606 |
| NM_ 032795 | RPUSD4 | 0.15 | -0.22 | 615 | 775 | 977 | 749 |
| NM_ 001271634 | RQCD1 | 0.16 | -0.26 | 1830 | 2205 | 2227 | 1728 |
| NM_ 001033 | RRM1 | 0.28 | -0.11 | 1840 | 2399 | 2573 | 2224 |
| NM_ 001034 | RRM2 | 0.00 | -0.69 | 999 | 967 | 460 | 237 |
| NM_ 016052 | RRP15 | 0.06 | -0.03 | 356 | 439 | 451 | 375 |
| NM_ 033112 | RRP36 | 0.68 | -0.15 | 522 | 950 | 1720 | 1427 |
| NM_ 015703 | RRP7A | 0.25 | -0.08 | 892 | 1180 | 1539 | 1338 |
| NM_ 004704 | RRP9 | 0.35 | -0.12 | 505 | 734 | 690 | 558 |
| NM_ 018346 | RSAD1 | 0.00 | -0.15 | 833 | 922 | 1450 | 1192 |
| NM_ 032730 | RTN4IP1 | 0.33 | -0.10 | 35 | 71 | 135 | 92 |
| NM_ 001105203 | RUSC1 | 0.10 | -0.05 | 970 | 1148 | 1809 | 1614 |
| NM_ 003707 | RUVBL1 | 0.00 | -0.35 | 842 | 901 | 1512 | 1083 |
| NM_ 006666 | RUVBL2 | 0.37 | -0.23 | 1628 | 2263 | 2928 | 2342 |
| NM_ 016104 | RWDD1 | 0.25 | -0.06 | 443 | 610 | 973 | 839 |
| NM_ 002960 | S100A3 | 0.28 | -0.11 | 50 | 91 | 162 | 113 |
| NM_ 001400 | S1PR1 | 0.05 | -0.14 | 1060 | 1212 | 799 | 641 |
| NM_ 005226 | S1PR3 | 0.00 | -0.16 | 650 | 619 | 663 | 518 |
| NM_ 005500 | SAE1 | 0.38 | -0.20 | 1853 | 2596 | 3022 | 2466 |
| NM_ 001101676 | SAMD12 | 0.00 | -0.24 | 390 | 415 | 287 | 196 |
| NM_ 001258275 | SAMD3 | 0.00 | -0.42 | 22 | 24 | 121 | 63 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_017827 | SARS2 | 0.68 | -0.24 | 168 | 334 | 553 | 403 |
| NM_133491 | SAT2 | 0.30 | -0.28 | 639 | 890 | 1325 | 989 |
| NM_005698 | SCAMP3 | 0.22 | -0.14 | 1598 | 2012 | 3818 | 3286 |
| NM_152540 | SCFD2 | 0.18 | -0.56 | 1264 | 1559 | 3440 | 2189 |
| NM_007281 | SCRG1 | 0.52 | -0.14 | 77 | 153 | 44 | 22 |
| NM_002999 | SDC4 | 0.29 | -0.08 | 2244 | 2921 | 3165 | 2823 |
| NM_138432 | SDSL | 0.00 | -0.40 | 24 | 37 | 295 | 179 |
| NM_006808 | SEC61B | 0.91 | -0.21 | 318 | 701 | 1125 | 876 |
| NM_001258289 | SELENBP1 | 0.19 | -0.77 | 36 | 66 | 204 | 89 |
| NM_153617 | SEMA6D | 0.16 | -0.29 | 234 | 320 | 58 | 28 |
| NM_016955 | SEPSECS | 0.00 | -0.02 | 109 | 106 | 193 | 148 |
| NM_001018067 | SERBP1 | 0.00 | -0.19 | 2706 | 2729 | 3267 | 2697 |
| NM_004155 | SERPINB9 | 0.24 | -0.74 | 217 | 315 | 258 | 120 |
| NM_002615 | SERPINF1 | 0.34 | -0.56 | 229 | 353 | 1210 | 734 |
| NM_001207014 | SERPINH1 | 0.28 | -0.06 | 8513 | 10678 | 10523 | 9763 |
| NM_012426 | SF3B3 | 0.31 | -0.12 | 3833 | 4988 | 6451 | 5689 |
| NM_005850 | SF3B4 | 0.10 | -0.07 | 1271 | 1488 | 1522 | 1325 |
| NM_031287 | SF3B5 | 0.21 | -0.02 | 1359 | 1706 | 2859 | 2654 |
| NM_005066 | SFPQ | 0.33 | -0.05 | 2459 | 3294 | 2512 | 2274 |
| NM_003012 | SFRP1 | 0.00 | -0.15 | 1351 | 1346 | 4190 | 3575 |
| NM_001261411 | SFSWAP | 0.28 | -0.17 | 602 | 829 | 952 | 754 |
| NM_005627 | SGK1 | 0.05 | -0.06 | 8304 | 8922 | 3589 | 3244 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001080826 | SGK223 | 0.01 | -0.07 | 561 | 646 | 1487 | 1298 |
| NM_001160033 | SGOL2 | 0.00 | -0.13 | 374 | 341 | 385 | 294 |
| NM_001103161 | SH2D5 | 0.08 | -0.50 | 729 | 869 | 738 | 453 |
| NM_014521 | SH3BP4 | 0.28 | -0.06 | 4207 | 5375 | 6443 | 5944 |
| NM_203349 | SHC4 | 0.00 | -0.61 | 1 | 7 | 81 | 33 |
| NM_024745 | SHCBP1 | 0.05 | -0.64 | 929 | 1066 | 782 | 436 |
| NM_138356 | SHF | 1.28 | -2.03 | 243 | 699 | 524 | 100 |
| NM_001272067 | SHISA5 | 0.12 | -0.29 | 2286 | 2655 | 4887 | 3789 |
| NM_020859 | SHROOM3 | 0.25 | -0.21 | 1335 | 1723 | 2408 | 1939 |
| NM_001135054 | SIGIRR | 0.00 | -0.32 | 243 | 194 | 559 | 383 |
| NM_012237 | SIRT2 | 0.11 | -0.06 | 862 | 1036 | 2057 | 1837 |
| NM_012241 | SIRT5 | 0.04 | -0.32 | 183 | 237 | 489 | 331 |
| NM_016539 | SIRT6 | 0.43 | -0.11 | 207 | 341 | 532 | 423 |
| NM_006427 | SIVA1 | 0.31 | -0.20 | 410 | 591 | 674 | 512 |
| NM_001039535 | SKA1 | 0.00 | -0.49 | 319 | 316 | 225 | 124 |
| NM_182620 | SKA2 | 0.17 | -0.02 | 338 | 448 | 485 | 410 |
| NM_145061 | SKA3 | 0.00 | -0.32 | 364 | 405 | 277 | 177 |
| NM_005983 | SKP2 | 0.07 | -0.45 | 390 | 480 | 539 | 336 |
| NM_001270622 | SLC16A7 | 0.00 | -0.52 | 787 | 798 | 742 | 451 |
| NM_194255 | SLC19A1 | 0.00 | -0.38 | 245 | 206 | 237 | 142 |
| NM_001270888 | SLC25A10 | 0.00 | -0.25 | 265 | 285 | 485 | 346 |
| NM_024698 | SLC25A22 | 0.29 | -0.07 | 665 | 918 | 898 | 763 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_013386 | SLC25A24 | 0.14 | -0.01 | 984 | 1196 | 1714 | 1569 |
| NM_213611 | SLC25A3 | 0.24 | -0.22 | 5866 | 7201 | 10822 | 8978 |
| NM_018843 | SLC25A40 | 0.22 | -0.03 | 173 | 254 | 287 | 229 |
| NM_001152 | SLC25A5 | 0.25 | -0.46 | 2798 | 3536 | 4390 | 3018 |
| NM_005094 | SLC27A4 | 0.08 | -0.19 | 934 | 1093 | 2874 | 2357 |
| NM_012254 | SLC27A5 | 0.05 | -0.22 | 34 | 58 | 79 | 44 |
| NM_080670 | SLC35A4 | 0.07 | -0.22 | 1472 | 1678 | 2512 | 2018 |
| NM_198277 | SLC37A2 | 0.00 | -0.01 | 116 | 119 | 530 | 453 |
| NM_001271957 | SLC39A1 | 0.44 | -0.22 | 1202 | 1783 | 2593 | 2079 |
| NM_001278206 | SLC43A3 | 0.00 | -0.50 | 1549 | 1671 | 630 | 384 |
| NM_001287587 | SLC50A1 | 0.00 | -0.20 | 523 | 583 | 1190 | 939 |
| NM_021095 | SLC5A6 | 0.00 | -0.11 | 608 | 676 | 830 | 684 |
| NM_020949 | SLC7A14 | 0.00 | -0.68 | 21 | 18 | 198 | 92 |
| NM_004787 | SLIT2 | 0.22 | -0.20 | 2629 | 3250 | 3479 | 2859 |
| NM_005902 | SMAD3 | 0.42 | -0.27 | 984 | 1446 | 2394 | 1852 |
| NM_022733 | SMAP2 | 0.05 | -0.44 | 1083 | 1234 | 2159 | 1475 |
| NM_003069 | SMARCA1 | 0.04 | -0.31 | 765 | 885 | 1770 | 1312 |
| NM_003073 | SMARCB1 | 0.00 | -0.03 | 1651 | 1755 | 2028 | 1848 |
| NM_058182 | SMIM11 | 0.00 | -0.03 | 149 | 180 | 241 | 189 |
| NM_001124767 | SMIM4 | 0.12 | -0.27 | 132 | 187 | 597 | 427 |
| NM_003068 | SNAI2 | 0.72 | -0.20 | 777 | 1415 | 3885 | 3205 |
| NM_007241 | SNF8 | 0.16 | -0.03 | 516 | 659 | 1348 | 1203 |

(continued)

| RefSeq ID RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_ 004596 | SNRPA | 0.00 | -0.03 | 743 | 753 | 965 | 848 |
| NM_ 003091 | SNRPB | 0.01 | -0.19 | 5364 | 5663 | 5415 | 4543 |
| NM_ 003092 | SNRPB2 | 0.00 | -0.18 | 410 | 475 | 468 | 351 |
| NM_ 003093 | SNRPC | 0.19 | -0.03 | 448 | 593 | 851 | 741 |
| NM_ 006938 | SNRPD1 | 0.00 | -0.12 | 314 | 372 | 387 | 298 |
| NM_ 177542 | SNRPD2 | 0.10 | -0.02 | 2155 | 2468 | 4706 | 4427 |
| NM_ 003095 | SNRPF | 0.00 | -0.62 | 159 | 129 | 277 | 142 |
| NM_ 003102 | SOD3 | 0.00 | -0.62 | 149 | 179 | 297 | 153 |
| NM_ 080627 | SOGA1 | 0.00 | -0.11 | 2506 | 2469 | 3238 | 2817 |
| NM_ 006461 | SPAG5 | 0.19 | -0.74 | 1406 | 1748 | 1310 | 706 |
| NM_ 004890 | SPAG7 | 0.00 | -0.10 | 728 | 707 | 1524 | 1299 |
| NM_ 001142854 | SPATC1L | 0.00 | -0.23 | 153 | 172 | 308 | 214 |
| NM_ 182513 | SPC24 | 0.62 | -0.83 | 97 | 199 | 235 | 100 |
| NM_ 020675 | SPC25 | 0.49 | -0.65 | 87 | 166 | 133 | 59 |
| NM_ 015330 | SPECC1L | 0.00 | -0.02 | 2530 | 2652 | 4076 | 3810 |
| NM_ 182965 | SPHK1 | 0.01 | -0.47 | 5455 | 5745 | 7699 | 5351 |
| NM_ 001080394 | SPIDR | 0.07 | -0.14 | 461 | 559 | 986 | 799 |
| NM_ 144569 | SPOCD1 | 0.10 | -0.54 | 830 | 995 | 3261 | 2103 |
| NM_ 032641 | SPSB2 | 0.00 | -0.30 | 97 | 104 | 412 | 279 |
| NM_ 003131 | SRF | 0.00 | -0.02 | 1689 | 1742 | 2132 | 1957 |
| NM_ 003130 | SRI | 0.00 | -0.12 | 578 | 567 | 1117 | 926 |
| NM_ 005839 | SRRM1 | 0.20 | -0.07 | 1724 | 2133 | 2466 | 2201 |

(continued)

| RefSeq ID RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_003142 | SSB | 0.00 | -0.11 | 681 | 706 | 903 | 745 |
| NM_018984 | SSH1 | 0.25 | -0.09 | 5186 | 6444 | 7975 | 7198 |
| NM_017857 | SSH3 | 0.29 | -0.10 | 153 | 238 | 780 | 641 |
| NM_003731 | SSNA1 | 0.57 | -0.07 | 722 | 1197 | 1926 | 1693 |
| NM_003145 | SSR2 | 0.29 | -0.08 | 3942 | 5062 | 6441 | 5854 |
| NM_003146 | SSRP1 | 0.19 | -0.05 | 2681 | 3257 | 3741 | 3414 |
| NM_001049 | SSTR1 | 0.00 | -1.02 | 1568 | 1598 | 2042 | 921 |
| NM_213618 | ST5 | 0.17 | -0.01 | 440 | 572 | 984 | 876 |
| NM_175039 | ST6GALNA C4 | 0.78 | -0.20 | 396 | 782 | 1572 | 1251 |
| NM_015136 | STAB1 | 0.69 | -0.20 | 210 | 412 | 724 | 551 |
| NM_001013841 | STAP2 | 0.23 | -0.24 | 57 | 98 | 133 | 81 |
| NM_032016 | STARD3NL | 0.66 | -0.08 | 347 | 641 | 1040 | 882 |
| NM_007315 | STAT1 | 0.42 | -0.20 | 2776 | 3945 | 6961 | 5798 |
| NM_012449 | STEAP1 | 0.00 | -0.10 | 501 | 502 | 1549 | 1322 |
| NM_207342 | STEAP1B | 0.21 | -0.04 | 259 | 363 | 327 | 262 |
| NM_182915 | STEAP3 | 0.26 | -0.23 | 849 | 1128 | 3350 | 2682 |
| NM_001282936 | STIL | 0.02 | -0.20 | 517 | 604 | 485 | 359 |
| RefSeq ID | Gene Symbol | SR GFOLD | SEN GFOLD | SR IL-2 - | SR IL-2 + | SEN IL-2 - | SEN IL-2 + |
| NM_001243313 | STK36 | 0.00 | -0.21 | 249 | 263 | 761 | 580 |
| NM_005563 | STMN1 | 0.10 | -0.54 | 2797 | 3175 | 3413 | 2203 |
| NM_001199214 | STMN2 | 0.54 | -1.62 | 5 | 26 | 946 | 261 |
| NM_015894 | STMN3 | 0.00 | -0.77 | 121 | 89 | 218 | 96 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_004099 | STOM | 0.02 | -0.50 | 4540 | 4816 | 6430 | 4331 |
| NM_013442 | STOML2 | 0.14 | -0.37 | 1807 | 2150 | 4018 | 2932 |
| NM_001271006 | STRA13 | 0.23 | -0.31 | 621 | 826 | 955 | 686 |
| NM_001171137 | STRBP | 0.00 | -0.14 | 76 | 84 | 160 | 109 |
| NM_001039877 | STRN4 | 0.00 | -0.10 | 2120 | 2221 | 2980 | 2615 |
| NM_016930 | STX18 | 0.29 | -0.09 | 201 | 304 | 624 | 510 |
| NM_003848 | SUCLG2 | 0.15 | -0.18 | 509 | 648 | 1065 | 847 |
| NM_001017392 | SUGP2 | 0.21 | -0.10 | 1536 | 1918 | 3361 | 2950 |
| NM_001128205 | SULF1 | 0.13 | -0.12 | 6706 | 7605 | 21270 | 19087 |
| NM_000456 | SUOX | 0.49 | -0.36 | 211 | 362 | 641 | 433 |
| NM_003599 | SUPT3H | 0.00 | -0.32 | 127 | 98 | 281 | 181 |
| NM_001278942 | SURF6 | 0.10 | -0.08 | 463 | 574 | 693 | 577 |
| NM_019601 | SUSD2 | 0.00 | -0.37 | 31 | 30 | 79 | 39 |
| NM_001287005 | SUSD3 | 0.00 | -0.45 | 106 | 139 | 102 | 50 |
| NM_003173 | SUV39H1 | 0.11 | -0.32 | 353 | 448 | 391 | 261 |
| NM_014849 | SV2A | 0.00 | -0.32 | 607 | 629 | 857 | 608 |
| NM_033025 | SYDE1 | 0.31 | -0.16 | 2110 | 2793 | 2691 | 2253 |
| NM_030786 | SYNC | 0.11 | -0.10 | 2376 | 2729 | 6052 | 5397 |
| NM_001159677 | SYNCRIP | 0.19 | -0.11 | 1592 | 1967 | 2518 | 2183 |
| NM_015180 | SYNE2 | 0.00 | -0.63 | 329 | 317 | 447 | 240 |
| NM_018373 | SYNJ2BP | 0.00 | -0.03 | 575 | 657 | 1483 | 1329 |
| NM_001040709 | SYPL2 | 0.00 | -0.46 | 20 | 19 | 218 | 122 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001114600 | SZRD1 | 0.37 | -0.05 | 4090 | 5541 | 6802 | 6320 |
| NM_006342 | TACC3 | 0.25 | -0.66 | 815 | 1078 | 821 | 455 |
| NM_016360 | TACO1 | 0.00 | -0.33 | 502 | 506 | 703 | 486 |
| NM_006354 | TADA3 | 0.34 | -0.05 | 1229 | 1697 | 3296 | 3005 |
| NM_014409 | TAF5L | 0.54 | -0.10 | 370 | 627 | 653 | 532 |
| NM_001190415 | TAF6 | 0.47 | -0.13 | 763 | 1179 | 1568 | 1311 |
| NM_001001522 | TAGLN | 0.57 | -0.44 | 34290 | 51712 | 62715 | 45611 |
| NM_003564 | TAGLN2 | -0.01 | -0.02 | 11144 | 10731 | 15732 | 15146 |
| NM_006755 | TALDO1 | 0.09 | -0.09 | 6037 | 6707 | 11030 | 10065 |
| NM_001145909 | TANC1 | 0.24 | -0.04 | 1163 | 1505 | 3687 | 3403 |
| NM_024562 | TANGO6 | 0.57 | -0.10 | 648 | 1079 | 1491 | 1275 |
| NM_000593 | TAP1 | 0.00 | -0.11 | 1499 | 1493 | 2439 | 2116 |
| NM_000544 | TAP2 | 0.00 | -0.05 | 931 | 1008 | 1585 | 1401 |
| NM_145647 | TBC1D31 | 0.00 | -0.09 | 118 | 150 | 189 | 137 |

**Table 5D: Differentially Downregulated Genns in SEN + IL-2**

| RefSeq ID | Gene Symbol | SR GFOLD | SEN GFOLD | SR IL- 2- | SR IL-2 + | SEN IL-2 - | SEN IL-2 + |
|---|---|---|---|---|---|---|---|
| NM_001139466 | TBL1X | 0.11 | -0.04 | 715 | 870 | 1666 | 1494 |
| NM_006453 | TBL3 | -0.01 | -0.17 | 1041 | 932 | 1452 | 1181 |
| NM_001006935 | TCEAL4 | 0.00 | -0.23 | 1056 | 1093 | 1947 | 1532 |
| NM_152278 | TCEAL7 | 0.00 | -0.06 | 93 | 69 | 356 | 283 |
| NM_153333 | TCEAL8 | 0.07 | -0.10 | 576 | 691 | 1304 | 1103 |
| NM_007109 | TCF19 | 0.09 | -0.37 | 1654 | 1905 | 1316 | 921 |

(continued)

| Table 5D: Differentially Downregulated Genns in SEN + IL-2 | | | | | | | |
|---|---|---|---|---|---|---|---|
| RefSeq ID | Gene Symbol | SR GFOLD | SEN GFOLD | SR IL- 2- | SR IL-2 + | SEN IL-2 - | SEN IL-2 + |
| NM_198392 | TCF21 | 1.22 | -1.91 | 17 | 73 | 69 | 6 |
| NM_001134851 | TCF7 | 0.00 | -0.13 | 229 | 208 | 1770 | 1495 |
| NM_031283 | TCF7L1 | 0.00 | -0.03 | 492 | 492 | 416 | 344 |
| NM_018319 | TDP1 | 0.00 | -0.04 | 649 | 620 | 678 | 580 |
| NM_001256661 | TEAD2 | 0.16 | -0.10 | 545 | 696 | 1065 | 895 |
| NM_003213 | TEAD4 | 0.50 | -0.39 | 384 | 632 | 233 | 139 |
| NM_016111 | TELO2 | 0.00 | -0.03 | 619 | 685 | 1123 | 993 |
| NM_170754 | TENC1 | 0.13 | -0.08 | 597 | 744 | 1603 | 1388 |
| NM_017746 | TEX10 | 0.06 | -0.16 | 532 | 637 | 1298 | 1054 |
| NM_007111 | TFDP1 | 0.15 | -0.01 | 1415 | 1708 | 1857 | 1702 |
| NM_015927 | TGFB1I1 | 0.25 | -0.16 | 1804 | 2303 | 3088 | 2590 |
| NM_138350 | THAP3 | 0.17 | -0.20 | 485 | 629 | 844 | 651 |
| NM_000361 | THBD | 0.30 | -0.06 | 381 | 547 | 2575 | 2307 |
| NM_003247 | THBS2 | 0.26 | -0.03 | 13115 | 16124 | 32251 | 31038 |
| NM_003249 | THOP1 | 0.08 | -0.29 | 1244 | 1441 | 1965 | 1482 |
| NM_018676 | THSD1 | 0.00 | -0.05 | 129 | 147 | 362 | 292 |
| NM_024817 | THSD4 | 0.02 | -0.53 | 790 | 897 | 1707 | 1081 |
| NM_012454 | TIAM2 | 0.20 | -0.31 | 456 | 607 | 1483 | 1091 |
| NM_182919 | TICAM1 | 0.63 | -0.38 | 443 | 786 | 961 | 658 |
| NM_012458 | TIMM13 | 0.00 | -0.33 | 1275 | 1270 | 2196 | 1614 |
| NM_001167947 | TIMM17B | 0.32 | -0.37 | 239 | 363 | 595 | 397 |

(continued)

| Table 5D: Differentially Downregulated Genns in SEN + IL-2 | | | | | | | |
|---|---|---|---|---|---|---|---|
| RefSeq ID | Gene Symbol | SR GFOLD | SEN GFOLD | SR IL- 2- | SR IL-2 + | SEN IL-2 - | SEN IL-2 + |
| NM_003258 | TK1 | 0.31 | -0.43 | 1595 | 2129 | 1042 | 688 |
| NM_001135055 | TKT | 0.06 | -0.15 | 13833 | 14856 | 17057 | 14972 |
| NM_001282748 | TLE4 | 0.01 | -0.10 | 1903 | 2067 | 1104 | 930 |
| NM_015059 | TLN2 | 0.09 | -0.10 | 2058 | 2352 | 4839 | 4309 |
| NM_018352 | TMA16 | 0.00 | -0.46 | 383 | 363 | 324 | 190 |
| NM_015933 | TMA7 | 0.00 | -0.16 | 128 | 152 | 331 | 244 |
| NM_181719 | TMCO4 | 0.63 | -0.34 | 323 | 586 | 537 | 362 |
| NM_032354 | TMEM107 | 1.49 | -0.03 | 1693 | 5070 | 5099 | 4754 |
| NM_181724 | TMEM119 | 0.00 | -1.22 | 65 | 56 | 435 | 150 |
| NM_032928 | TMEM141 | 0.47 | -0.12 | 314 | 515 | 1210 | 1009 |
| NM_017854 | TMEM160 | 0.00 | -0.23 | 51 | 51 | 98 | 57 |
| NM_001161342 | TMEM171 | 0.00 | -0.33 | 222 | 255 | 293 | 187 |
| NM_198282 | TMEM173 | 0.00 | -0.60 | 939 | 947 | 1069 | 627 |
| Table 5D: Differentially Downregulated Genes in SEN + IL-2 | | | | | | | |
| RefSeq ID | Gene Symbol | SR GFOLD | SEN GFOLD | SR IL-2 - | SR IL-2 + | SEN IL-2 - | SEN IL-2 + |
| NM_001105198 | TMEM177 | 0.00 | -0.33 | 89 | 123 | 179 | 107 |
| NM_001142645 | TMEM194B | 0.00 | -0.40 | 57 | 73 | 87 | 43 |
| NM_053045 | TMEM203 | 0.17 | -0.01 | 639 | 814 | 1414 | 1281 |
| NM_001004313 | TMEM220 | 0.00 | -0.38 | 111 | 92 | 177 | 102 |
| NM_032933 | TMEM241 | 0.00 | -0.03 | 218 | 250 | 476 | 399 |
| NM_152766 | TMEM256 | 0.00 | -0.10 | 171 | 140 | 478 | 379 |

(continued)

| Table 5D: Differentially Downregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_033428 | TMEM261 | 0.00 | -0.18 | 356 | 343 | 632 | 486 |
| NM_018056 | TMEM39B | 0.36 | -0.05 | 354 | 534 | 711 | 603 |
| NM_014573 | TMEM97 | 0.40 | -0.08 | 219 | 353 | 482 | 388 |
| NM_001032283 | TMPO | 0.22 | -0.50 | 619 | 815 | 539 | 323 |
| NM_021109 | TMSB4X | 0.45 | -0.08 | 10426 | 14703 | 30300 | 28081 |
| NM_032813 | TMTC4 | 0.71 | -0.03 | 171 | 348 | 349 | 283 |
| NM_002160 | TNC | 0.26 | -1.03 | 3327 | 4199 | 13501 | 6370 |
| NM_021137 | TNFAIP1 | 0.24 | -0.18 | 2185 | 2763 | 3569 | 2981 |
| NM_152362 | TNFAIP8L1 | 0.81 | -0.10 | 54 | 137 | 185 | 133 |
| NM_002546 | TNFRSF11B | 0.01 | -0.49 | 5506 | 5798 | 1795 | 1168 |
| NM_016639 | TNFRSF12A | 0.05 | -0.10 | 6207 | 6707 | 6863 | 6142 |
| NM_003820 | TNFRSF14 | 0.00 | -0.02 | 320 | 331 | 959 | 847 |
| NM_001066 | TNFRSF1B | 0.00 | -0.48 | 237 | 265 | 351 | 205 |
| NM_020243 | TOMM22 | 0.34 | -0.34 | 731 | 1034 | 1884 | 1375 |
| NM_006809 | TOMM34 | 0.00 | -0.10 | 1396 | 1469 | 3332 | 2926 |
| NM_001134484 | TOMM5 | 0.45 | -0.04 | 479 | 749 | 911 | 791 |
| NM_001134493 | TOMM6 | 0.03 | -0.08 | 1043 | 1174 | 2389 | 2107 |
| NM_001067 | TOP2A | 0.00 | -0.76 | 2346 | 2513 | 2562 | 1401 |
| NM_004881 | TP53I3 | 0.50 | -0.40 | 387 | 634 | 2269 | 1599 |
| NM_022445 | TPK1 | 0.00 | -0.24 | 39 | 45 | 89 | 50 |
| NM_213674 | TPM2 | 0.18 | -0.13 | 16559 | 19170 | 30082 | 26996 |

(continued)

| Table 5D: Differentially Downregulated Genes in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_001278189 | TPM3 | 0.29 | -0.04 | 4631 | 5922 | 9286 | 8747 |
| NM_001145160 | TPM4 | 0.19 | -0.27 | 26570 | 30835 | 63316 | 51679 |
| NM_012112 | TPX2 | 0.41 | -1.02 | 1714 | 2454 | 2354 | 1068 |
| NM_004593 | TRA2B | 0.00 | -0.04 | 2176 | 2086 | 1824 | 1634 |
| NM_021138 | TRAF2 | 0.09 | -0.15 | 380 | 476 | 422 | 320 |
| NM_005879 | TRAIP | 0.54 | -0.24 | 91 | 178 | 189 | 122 |
| NM_001042646 | TRAK1 | 0.25 | -0.02 | 2483 | 3133 | 2190 | 2007 |
| NM_016381 | TREX1 | 0.00 | -0.24 | 416 | 487 | 580 | 423 |
| NM_013381 | TRHDE | 0.00 | -1.11 | 321 | 324 | 152 | 48 |
| NM_014788 | TRIM14 | 0.00 | -0.13 | 270 | 284 | 807 | 654 |
| NM_016102 | TRIM17 | 0.00 | -0.05 | 59 | 45 | 71 | 44 |
| NM_003141 | TRIM21 | 0.31 | -0.23 | 353 | 513 | 684 | 510 |
| NM_015431 | TRIM58 | 0.00 | -0.45 | 462 | 453 | 865 | 558 |
| Table 5D: Differentially Downregulated Genns in SEN + IL-2 | | | | | | | |
| RefSeq ID | Gene Symbol | SR GFOLD | SEN GFOLD | SR IL-2 - | SR IL-2 + | SEN IL-2 - | SEN IL-2 + |
| NM_173084 | TRIM59 | 0.00 | -0.14 | 461 | 442 | 451 | 346 |
| NM_001003819 | TRIM6-TRIM34 | 0.08 | -0.03 | 276 | 352 | 545 | 462 |
| NM_173547 | TRIM65 | 0.24 | -0.02 | 410 | 563 | 497 | 421 |
| NM_018073 | TRIM68 | 0.06 | -0.14 | 259 | 328 | 433 | 331 |
| NM_004237 | TRIP13 | 0.00 | -0.62 | 820 | 884 | 950 | 545 |
| NM_003302 | TRIP6 | 0.18 | -0.42 | 1387 | 1710 | 2899 | 2020 |

(continued)

| Table 5D: Differentially Downregulated Genns in SEN + IL-2 | | | | | | | |
|---|---|---|---|---|---|---|---|
| RefSeq ID | Gene Symbol | SR GFOLD | SEN GFOLD | SR IL-2 - | SR IL-2 + | SEN IL-2 - | SEN IL-2 + |
| NM_ 001286082 | TRMT112 | 0.38 | -0.30 | 753 | 1097 | 1699 | 1270 |
| NM_ 001257994 | TRMT2A | 0.43 | -0.04 | 621 | 944 | 1300 | 1150 |
| NM_ 024917 | TRMT2B | 0.00 | -0.35 | 355 | 346 | 645 | 438 |
| NM_ 017910 | TRMT61B | 0.25 | -0.23 | 149 | 226 | 308 | 214 |
| NM_ 005480 | TROAP | 0.00 | -1.46 | 408 | 454 | 291 | 79 |
| NM_ 003306 | TRPC4 | 0.30 | -1.13 | 948 | 1285 | 1004 | 399 |
| NM_ 016113 | TRPV2 | 0.37 | -0.33 | 2156 | 2966 | 9808 | 7508 |
| NM_ 015679 | TRUB2 | 0.00 | -0.05 | 645 | 731 | 1620 | 1440 |
| NM_ 000368 | TSC1 | 0.13 | -0.08 | 1217 | 1458 | 3363 | 3009 |
| NM_ 207346 | TSEN54 | 0.05 | -0.05 | 229 | 292 | 220 | 168 |
| NM_ 001290212 | TSPAN10 | 0.00 | -0.48 | 7 | 9 | 162 | 85 |
| NM_ 001256530 | TSPO | 0.00 | -0.05 | 1794 | 1699 | 3467 | 3153 |
| NM_ 052841 | TSSK3 | 0.44 | -0.08 | 24 | 57 | 48 | 26 |
| NM_ 003313 | TSTA3 | 0.03 | -0.06 | 918 | 1041 | 1624 | 1434 |
| NM_ 001282500 | TTC1 | 0.27 | -0.07 | 710 | 960 | 1583 | 1381 |
| NM_ 022492 | TTC31 | 0.00 | -0.04 | 374 | 440 | 832 | 719 |
| NM_ 017931 | TTC38 | 0.00 | -0.02 | 435 | 482 | 832 | 728 |
| NM_ 003594 | TTF2 | 0.08 | -0.20 | 631 | 754 | 728 | 555 |
| NM_ 001166691 | TTK | 0.15 | -0.92 | 255 | 343 | 335 | 140 |
| NM_ 025250 | TTYH3 | 0.19 | -0.32 | 2562 | 3105 | 3136 | 2364 |
| NM_ 001270399 | TUBA1A | 0.67 | -0.43 | 5700 | 9409 | 17311 | 12504 |

(continued)

| Table 5D: Differentially Downregulated Genns in SEN + IL-2 | | | | | | | |
|---|---|---|---|---|---|---|---|
| **RefSeq ID** | **Gene Symbol** | **SR GFOLD** | **SEN GFOLD** | **SR IL-2 -** | **SR IL-2 +** | **SEN IL-2 -** | **SEN IL-2 +** |
| NM_006082 | TUBA1B | 0.49 | -0.64 | 24029 | 34465 | 32484 | 20447 |
| NM_032704 | TUBA1C | 0.39 | -0.64 | 15529 | 20794 | 21822 | 13643 |
| NM_178014 | TUBB | 0.48 | -0.35 | 30537 | 43320 | 56651 | 43657 |
| NM_001069 | TUBB2A | 0.27 | -0.19 | 2069 | 2675 | 4157 | 3462 |
| NM_178012 | TUBB2B | 0.56 | -0.43 | 541 | 903 | 1657 | 1126 |
| NM_006086 | TUBB3 | 0.19 | -0.39 | 1065 | 1341 | 8198 | 6004 |
| NM_001289130 | TUBB4A | 1.77 | -0.90 | 13 | 89 | 116 | 41 |
| NM_006088 | TUBB4B | 0.30 | -0.52 | 12969 | 16445 | 15350 | 10364 |
| NM_032525 | TUBB6 | 0.42 | -0.36 | 17083 | 23402 | 33459 | 25606 |
| NM_001070 | TUBG1 | 0.75 | -0.17 | 964 | 1778 | 2236 | 1850 |
| NM_000474 | TWIST1 | 0.00 | -0.33 | 312 | 362 | 337 | 220 |
| NM_001002926 | TWISTNB | 0.10 | -0.01 | 310 | 396 | 568 | 488 |
| **RefSeq ID** | **Gene Symbol** | **SR GFOLD** | **SEN GFOLD** | **+SR IL-2 -** | **SR IL-2 +** | **SEN IL-2 -** | **SEN IL-2 +** |
| NM_012473 | TXN2 | 0.23 | -0.06 | 642 | 853 | 1587 | 1401 |
| NM_006440 | TXNRD2 | 0.00 | -0.06 | 335 | 398 | 695 | 584 |
| NM_001071 | TYMS | 0.00 | -0.28 | 1940 | 2079 | 873 | 638 |
| NM_001025204 | U2AF1 | 0.00 | -0.18 | 698 | 642 | 684 | 527 |
| NM_003968 | UBA3 | 0.00 | -0.02 | 801 | 856 | 1389 | 1253 |
| NM_003335 | UBA7 | 0.00 | -0.10 | 684 | 725 | 1304 | 1107 |
| NM_016172 | UBAC1 | 0.30 | -0.05 | 715 | 985 | 1994 | 1786 |
| NM_018449 | UBAP2 | 0.20 | -0.04 | 1613 | 2004 | 2304 | 2092 |

(continued)

| RefSeq ID | Gene Symbol | SR GFOLD | SEN GFOLD | +SR IL-2 - | SR IL-2 + | SEN IL-2 - | SEN IL-2 + |
|---|---|---|---|---|---|---|---|
| NM_ 014847 | UBAP2L | 0.08 | -0.03 | 4869 | 5372 | 7433 | 6989 |
| NM_ 181801 | UBE2C | 0.00 | -0.60 | 500 | 428 | 439 | 240 |
| NM_ 015983 | UBE2D4 | 0.11 | -0.05 | 186 | 252 | 622 | 523 |
| NM_ 004223 | UBE2L6 | 0.00 | -0.11 | 696 | 728 | 1718 | 1469 |
| NM_ 014501 | UBE2S | 0.37 | -0.50 | 597 | 871 | 761 | 468 |
| NM_ 014176 | UBE2T | 0.18 | -0.41 | 255 | 350 | 297 | 179 |
| NM_ 024292 | UBL5 | 0.00 | -0.14 | 388 | 386 | 799 | 640 |
| NM_ 020131 | UBQLN4 | 0.00 | -0.03 | 844 | 891 | 1223 | 1085 |
| NM_ 001261450 | UCK1 | 0.10 | -0.06 | 461 | 572 | 977 | 843 |
| NM_ 003358 | UGCG | 0.00 | -0.08 | 783 | 782 | 921 | 778 |
| NM_ 001048201 | UHRF1 | 0.15 | -0.71 | 1218 | 1481 | 547 | 283 |
| NM_ 001080419 | UNK | 0.66 | -0.21 | 225 | 430 | 672 | 506 |
| NM_ 001003684 | UQCR10 | 0.10 | -0.29 | 792 | 952 | 1955 | 1471 |
| NM_ 003366 | UQCRC2 | 0.22 | -0.01 | 1675 | 2101 | 3250 | 3039 |
| NM_ 014825 | URB1 | 0.00 | -0.14 | 2551 | 2630 | 2999 | 2554 |
| NM_ 001265582 | URM1 | 0.33 | -0.36 | 925 | 1282 | 2562 | 1863 |
| NM_ 003368 | USP1 | 0.00 | -0.04 | 979 | 1001 | 586 | 494 |
| NM_ 001272075 | USP10 | 0.03 | -0.01 | 1256 | 1401 | 2250 | 2081 |
| NM_ 003940 | USP13 | 0.00 | -0.18 | 1038 | 1125 | 1628 | 1322 |
| NM_ 018218 | USP40 | 0.05 | -0.10 | 483 | 577 | 1510 | 1294 |
| NM_ 006649 | UTP14A | 0.34 | -0.25 | 273 | 415 | 526 | 379 |
| NM_ 032175 | UTP15 | 0.11 | -0.15 | 320 | 410 | 534 | 412 |

(continued)

| RefSeq ID | Gene Symbol | SR GFOLD | SEN GFOLD | +SR IL-2 - | SR IL-2 + | SEN IL-2 - | SEN IL-2 + |
|---|---|---|---|---|---|---|---|
| NM_016001 | UTP18 | 0.35 | -0.16 | 171 | 274 | 416 | 314 |
| NM_014503 | UTP20 | 0.03 | -0.16 | 862 | 978 | 1356 | 1107 |
| NM_006634 | VAMP5 | 0.00 | -0.07 | 568 | 541 | 992 | 847 |
| NM_003370 | VASP | 0.47 | -0.20 | 1454 | 2178 | 3876 | 3200 |
| NM_020927 | VAT1L | 0.00 | -0.21 | 69 | 92 | 595 | 444 |
| NM_015873 | VILL | 0.00 | -0.15 | 92 | 110 | 156 | 105 |
| NM_032353 | VPS25 | 0.00 | -0.12 | 909 | 978 | 1657 | 1397 |
| NM_001271087 | VPS72 | 0.07 | -0.28 | 892 | 1042 | 1352 | 1011 |
| NM_004913 | VPS9D1 | 0.00 | -0.17 | 342 | 324 | 717 | 560 |
| NM_016440 | VRK3 | 0.39 | -0.14 | 439 | 664 | 1029 | 839 |
| RefSeq ID | Gene Symbol | SR GFOLD | SEN GFOLD | SR IL-2 - | SR IL-2 + | SEN IL-2 - | SEN IL-2 + |
| NM_006370 | VTI1B | 0.00 | -0.05 | 657 | 743 | 1343 | 1185 |
| NM_152559 | WBSCR27 | 0.15 | -0.47 | 62 | 100 | 164 | 87 |
| NM_020830 | WDFY1 | 0.16 | -0.16 | 2012 | 2405 | 3664 | 3098 |
| NM_017883 | WDR13 | 0.00 | -0.06 | 665 | 751 | 1497 | 1314 |
| NM_024100 | WDR18 | 0.18 | -0.40 | 293 | 397 | 626 | 410 |
| NM_052844 | WDR34 | 0.48 | -0.43 | 276 | 459 | 969 | 641 |
| NM_001260474 | WDR4 | 0.22 | -0.14 | 445 | 598 | 510 | 396 |
| NM_017588 | WDR5 | 0.14 | -0.20 | 616 | 769 | 994 | 776 |
| NM_017706 | WDR55 | 0.00 | -0.03 | 892 | 928 | 1298 | 1157 |
| NM_018031 | WDR6 | 0.00 | -0.05 | 3465 | 3598 | 4964 | 4586 |
| NM_001083961 | WDR62 | 0.49 | -0.16 | 439 | 710 | 462 | 351 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_024102 | WDR77 | 0.33 | -0.06 | 746 | 1049 | 1911 | 1702 |
| NM_133330 | WHSC1 | 0.10 | -0.26 | 4227 | 4755 | 3920 | 3096 |
| NM_032387 | WNK4 | 0.12 | -0.18 | 3386 | 3883 | 2441 | 2007 |
| NM_001080436 | WTIP | 0.45 | -0.04 | 684 | 1050 | 821 | 710 |
| NM_001100119 | XRCC3 | 0.00 | -0.23 | 538 | 579 | 493 | 359 |
| NM_021141 | XRCC5 | 0.06 | -0.13 | 5681 | 6182 | 9774 | 8660 |
| NM_022167 | XYLT2 | 0.24 | -0.03 | 1661 | 2111 | 2818 | 2588 |
| NM_006761 | YWHAE | 0.34 | -0.06 | 6442 | 8456 | 13305 | 12358 |
| NM_139121 | YY1AP1 | 0.00 | -0.35 | 1724 | 1821 | 3379 | 2495 |
| NM_133646 | ZAK | 0.00 | -0.04 | 2997 | 3039 | 5026 | 4664 |
| NM_032799 | ZDHHC12 | 0.00 | -0.11 | 528 | 549 | 934 | 773 |
| NM_024721 | ZFHX4 | 0.00 | -0.13 | 4031 | 3981 | 3978 | 3455 |
| NM_003407 | ZFP36 | 0.01 | -0.41 | 1190 | 1319 | 1102 | 745 |
| NM_032527 | ZGPAT | 0.00 | -0.30 | 420 | 478 | 767 | 547 |
| NM_003412 | ZIC1 | 0.29 | -0.28 | 188 | 285 | 437 | 303 |
| NM_019103 | ZMAT5 | 0.00 | -0.07 | 219 | 268 | 651 | 542 |
| NM_003434 | ZNF133 | 0.25 | -0.23 | 244 | 351 | 510 | 372 |
| NM_006963 | ZNF22 | 0.00 | -0.18 | 338 | 397 | 418 | 309 |
| NM_001099282 | ZNF239 | 0.37 | -0.02 | 52 | 100 | 214 | 166 |
| NM_005773 | ZNF256 | 0.00 | -0.30 | 82 | 111 | 162 | 98 |
| NM_014487 | ZNF330 | 0.26 | -0.04 | 747 | 1003 | 990 | 863 |
| NM_001282933 | ZNF341 | 0.33 | -0.03 | 147 | 236 | 160 | 118 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_153695 | ZNF367 | 0.00 | -0.24 | 594 | 663 | 241 | 161 |
| NM_144689 | ZNF420 | 0.00 | -0.05 | 81 | 63 | 143 | 102 |
| NM_152355 | ZNF441 | 0.00 | -0.03 | 105 | 130 | 254 | 200 |
| NM_005815 | ZNF443 | 0.00 | -0.06 | 60 | 47 | 98 | 65 |
| NM_021224 | ZNF462 | 0.19 | -0.19 | 513 | 671 | 1410 | 1128 |
| NM_001006656 | ZNF473 | 0.19 | -0.03 | 210 | 295 | 318 | 257 |
| NM_032752 | ZNF496 | 0.00 | -0.09 | 445 | 462 | 553 | 449 |
| NM_001271318 | ZNF512 | 0.00 | -0.03 | 291 | 317 | 562 | 477 |
| NM_133444 | ZNF526 | 0.13 | -0.05 | 758 | 930 | 1183 | 1037 |
| NM_001042474 | ZNF565 | 0.02 | -0.49 | 75 | 108 | 173 | 91 |
| NM_022752 | ZNF574 | 0.41 | -0.15 | 508 | 772 | 886 | 708 |
| NM_001145347 | ZNF576 | 0.00 | -0.08 | 216 | 257 | 449 | 360 |
| NM_016089 | ZNF589 | 0.00 | -0.03 | 121 | 118 | 185 | 140 |
| NM_133374 | ZNF618 | 0.05 | -0.03 | 1493 | 1678 | 2710 | 2484 |
| NM_001080417 | ZNF629 | 0.16 | -0.07 | 833 | 1036 | 1788 | 1571 |
| NM_024804 | ZNF669 | 0.00 | -0.34 | 112 | 100 | 158 | 92 |
| NM_145271 | ZNF688 | 0.00 | -0.11 | 79 | 86 | 187 | 133 |
| NM_001282795 | ZNF7 | 0.00 | -0.05 | 206 | 246 | 376 | 303 |
| NM_025069 | ZNF703 | 0.21 | -0.16 | 285 | 396 | 433 | 327 |
| NM_001010880 | ZNF780A | 0.00 | -0.06 | 70 | 96 | 220 | 166 |
| NM_001195605 | ZNF865 | 0.22 | -0.02 | 185 | 269 | 283 | 227 |
| NM_014205 | ZNHIT2 | 0.96 | -0.14 | 48 | 137 | 220 | 157 |

(continued)

| RefSeq ID | Gene Symbol | SR GFOLD | SEN GFOLD | SR IL-2 - | SR IL-2 + | SEN IL-2 - | SEN IL-2 + |
|---|---|---|---|---|---|---|---|
| NM_001278786 | ZNRD1 | 0.00 | -0.10 | 374 | 341 | 437 | 344 |
| NM_001042697 | ZSWIM7 | 0.00 | -0.10 | 122 | 152 | 339 | 261 |
| NM_001287821 | ZWILCH | 0.26 | -0.21 | 310 | 440 | 497 | 366 |
| NM_032997 | ZWINT | 0.47 | -0.53 | 607 | 947 | 819 | 497 |

**Table 6: Proteins Expression With and Without IL-2 treatment in SR and SEN hADSCs**

v data protein microarray ( normalized values against negative )

| Protein Name | 10% PRP | SR 24h | 48h | 72h | SR + IL2 24h | 48h | 72h | SEN 24h | 48h | 72h | SEN + IL2 24h | 48h | 72h |
|---|---|---|---|---|---|---|---|---|---|---|---|---|---|
| Acrp30 | 1.9906 | 1.7267 | 0.0000 | 1.9846 | 1.3016 | 2.3799 | 1.3797 | 1.4034 | 1.6601 | 1.2449 | 1.1842 | 2.1786 | 2.7266 |
| Activin A | 5.5127 | 3.8425 | 7.0180 | 3.5865 | 2.6392 | 7.2831 | 5.8653 | 4.8911 | 8.4566 | 2.2450 | 3.2213 | 6.7031 | 2.7259 |
| AgRP | 2.1403 | 2.0670 | 1.1545 | 2.3200 | 1.4000 | 2.5571 | 1.4548 | 1.4184 | 1.3673 | 0.0000 | 1.2969 | 1.9093 | 2.8466 |
| ALCAM | 5.2309 | 4.1953 | 7.6635 | 3.5971 | 2.3671 | 7.2204 | 5.4144 | 5.1089 | 7.8102 | 2.4079 | 3.0986 | 5.3750 | 2.7221 |
| ANG | 0.0000 | 0.0000 | 0.0000 | 1.5127 | 0.0000 | 0.0000 | 1.1264 | 1.1170 | 0.0000 | 0.0000 | 1.1488 | 0.0000 | 0.0000 |
| ANGPT2 | 2.1696 | 1.9598 | 0.0000 | 2.1267 | 1.6403 | 2.2390 | 1.3215 | 1.2514 | 1.4214 | 1.2900 | 1.1470 | 1.8578 | 2.8152 |
| AREG | 1.8281 | 1.6344 | 0.0000 | 1.9837 | 1.2505 | 1.9108 | 1.1054 | 0.0000 | 0.0000 | 0.0000 | 1.1028 | 1.2819 | 1.8805 |
| Axl | 1.7897 | 1.4567 | 0.0000 | 1.7697 | 0.0000 | 1.6622 | 0.0000 | 0.0000 | 0.0000 | 1.1594 | 1.0313 | 1.1653 | 1.5707 |
| b-NGF | 1.4432 | 1.2653 | 0.0000 | 1.4735 | 0.0000 | 1.4234 | 0.0000 | 1.1838 | 0.0000 | 1.3909 | 1.3112 | 1.1327 | 1.7046 |
| BDNF | 0.0000 | 1.3009 | 1.5763 | 2.2460 | 1.1868 | 1.4820 | 1.5919 | 1.7004 | 1.3962 | 0.0000 | 1.7874 | 2.1395 | 1.7741 |
| bFGF | 2.0525 | 1.6365 | 1.1390 | 1.8419 | 1.3603 | 1.7292 | 0.0000 | 1.1812 | 1.2779 | 1.5579 | 1.4446 | 1.6634 | 2.5339 |
| BLC (CXCL13) | 0.0000 | 0.0000 | 1.1843 | 1.5858 | 0.0000 | 0.0000 | 0.0000 | 1.2457 | 0.0000 | 0.0000 | 1.4209 | 1.3900 | 0.0000 |
| BMP 5 | 4.8192 | 5.1370 | 8.6495 | 3.7048 | 1.4304 | 7.1869 | 6.1152 | 5.9347 | 11.7418 | 3.2899 | 3.6011 | 7.1250 | 3.4143 |
| BMP 7 | 4.1543 | 4.9228 | 7.1538 | 3.5586 | 0.0000 | 6.1955 | 5.2938 | 5.5754 | 8.4778 | 2.9665 | 3.1606 | 4.5625 | 3.2136 |
| BMP4 | 0.0000 | 0.0000 | 1.3010 | 1.6810 | 0.0000 | 0.0000 | 1.1395 | 1.3177 | 1.4824 | 0.0000 | 1.8827 | 2.1601 | 1.2792 |
| BMP6 | 0.0000 | 0.0000 | 0.0000 | 1.3908 | 0.0000 | 0.0000 | 0.0000 | 1.1172 | 1.1257 | 0.0000 | 1.9253 | 1.8054 | 0.0000 |
| BTC | 1.2327 | 1.2265 | 1.2349 | 1.4869 | 0.0000 | 1.3380 | 0.0000 | 1.1707 | 1.3156 | 1.5758 | 1.5331 | 1.1887 | 2.0910 |
| CCL23 | 0.0000 | 0.0000 | 1.4156 | 1.7443 | 0.0000 | 1.1108 | 1.3719 | 1.6100 | 1.6595 | 0.0000 | 3.0483 | 2.5000 | 1.2505 |
| CCL28 | 0.0000 | 0.0000 | 1.1480 | 1.6428 | 0.0000 | 0.0000 | 1.6927 | 0.0000 | 1.1613 | 1.1350 | 0.0000 | 1.2443 | 2.5985 |
| CD14 | 3.1727 | 4.6425 | 7.4476 | 4.3188 | 0.0000 | 5.7709 | 4.8656 | 6.7465 | 8.4355 | 3.8385 | 4.4048 | 5.5625 | 3.4105 |
| CD80 | 4.2398 | 3.3165 | 7.9128 | 3.3382 | 1.5886 | 6.5427 | 5.1120 | 5.2037 | 8.0751 | 2.6430 | 2.9403 | 5.0625 | 2.6128 |
| CNTF | 0.0000 | 0.0000 | 1.3880 | 1.5508 | 0.0000 | 1.1316 | 1.2175 | 1.6206 | 1.6156 | 0.0000 | 3.3783 | 2.8787 | 1.5144 |
| CT-1 | 3.6318 | 4.8724 | 7.7881 | 4.4073 | 0.0000 | 6.5615 | 5.5979 | 6.7356 | 9.8767 | 3.6226 | 4.1365 | 7.0938 | 3.7065 |
| CTACK (CCL27) | 5.9157 | 8.2865 | 1.6690 | 4.5118 | 11.2803 | 6.6482 | 4.0186 | 3.7664 | 10.6590 | 2.8290 | 3.9831 | 5.8344 | 7.8050 |
| CXCL16 | 1.4312 | 2.6425 | 5.4889 | 3.4248 | 0.0000 | 3.4910 | 2.8627 | 5.1182 | 3.8786 | 2.9027 | 3.9219 | 2.3125 | 2.5887 |
| DR6 (TNFRSF21) | 1.1811 | 3.2283 | 7.4088 | 4.6902 | 0.0000 | 4.4782 | 24.8942 | 6.0855 | 10.5125 | 4.3387 | 5.4060 | 7.5000 | 4.1333 |

298

**Table 6: Proteins Expression With and Without IL-2 treatment in SR and SEN hADSCs**

v data  protein microarray ( normalized values against negative )

| Protein Name | 10% PRP | SR | | | SR + IL2 | | | SEN | | | SEN + IL2 | | |
|---|---|---|---|---|---|---|---|---|---|---|---|---|---|
| | | 24h | 48h | 72h | 24h | 48h | 72h | 24h | 48h | 72h | 24h | 48h | 72h |
| Dtk (TYRO) | 2.5474 | 2.5898 | 0.0000 | 1.8760 | 2.4431 | 3.2288 | 2.1533 | 2.1400 | 2.4860 | 0.0000 | 2.3962 | 2.7945 | 1.9706 |
| E selectin | 3.9643 | 4.6929 | 8.5605 | 4.0955 | 2.7215 | 7.5927 | 5.8635 | 4.7247 | 7.9374 | 2.2535 | 3.1821 | 10.5625 | 2.9482 |
| EGF | 0.0000 | 0.0000 | 1.2835 | 1.4322 | 0.0000 | 0.0000 | 1.2638 | 1.4882 | 1.8373 | 0.0000 | 3.5073 | 2.6390 | 1.3703 |
| EGFR | 2.2096 | 2.0563 | 0.0000 | 1.6972 | 1.5212 | 2.7657 | 1.8194 | 2.0575 | 1.9257 | 0.0000 | 1.9208 | 2.1894 | 1.5882 |
| ENA-78 (CXCL5) | 2.3239 | 2.1126 | 0.0000 | 2.0400 | 1.4142 | 3.0456 | 1.9233 | 2.2308 | 1.8874 | 0.0000 | 2.0352 | 2.0060 | 2.1837 |
| Endoglin | 8.0299 | 9.2661 | 13.1501 | 4.0926 | 7.0000 | 12.8678 | 9.3310 | 6.7481 | 23.0173 | 3.6180 | 5.1092 | 24.7188 | 4.2247 |
| Eotaxin 1 (CCL11) | 0.0000 | 0.0000 | 1.1909 | 1.5127 | 0.0000 | 0.0000 | 2.4429 | 1.2626 | 1.4283 | 0.0000 | 3.3451 | 2.0151 | 1.9433 |
| Eotaxin 2 (CCL24) | 1.3070 | 1.1844 | 1.2730 | 2.2540 | 1.4237 | 1.2616 | 1.8056 | 1.5405 | 2.5752 | 1.2580 | 1.4635 | 2.3263 | 1.6947 |
| Eotaxin 3 CCL26) | 1.2536 | 1.2052 | 1.4199 | 2.2715 | 1.3567 | 1.3012 | 1.7425 | 1.5603 | 1.8755 | 1.1319 | 1.6620 | 1.7742 | 1.5389 |
| ErbB3 | 6.2568 | 7.8142 | 12.1552 | 4.5574 | 5.6392 | 11.7007 | 8.1198 | 5.6967 | 15.8112 | 3.1470 | 4.3441 | 22.2031 | 3.8487 |
| Fas | 2.4938 | 2.4572 | 0.0000 | 2.1110 | 1.3889 | 3.1952 | 1.7997 | 2.3597 | 1.9900 | 0.0000 | 2.3167 | 2.2383 | 1.8808 |
| Fas ligand | 5.2372 | 3.9402 | 8.6851 | 4.3178 | 2.2215 | 7.7056 | 5.3060 | 5.4386 | 10.0568 | 2.3741 | 3.3074 | 5.6563 | 2.5277 |
| FGF-4 | 2.2505 | 2.0995 | 0.0000 | 2.0520 | 1.1243 | 2.9649 | 1.5879 | 2.0905 | 1.6439 | 0.0000 | 1.8951 | 1.8751 | 1.7765 |
| FGF-6 | 1.2219 | 1.2272 | 1.4400 | 2.2757 | 1.3695 | 1.2044 | 1.5075 | 1.6746 | 1.7251 | 1.1717 | 1.8035 | 2.3610 | 1.8691 |
| FGF-7 | 0.0000 | 0.0000 | 1.1132 | 1.8267 | 0.0000 | 0.0000 | 1.2607 | 1.2692 | 1.3712 | 0.0000 | 1.2963 | 1.7545 | 1.5419 |
| FGF-9 | 2.8139 | 2.6479 | 1.3408 | 2.6706 | 1.7241 | 3.5488 | 1.8125 | 2.2538 | 2.2583 | 1.4065 | 2.1909 | 2.2993 | 2.9721 |
| Flt-3 Ligand | 0.0000 | 0.0000 | 1.1708 | 1.8777 | 0.0000 | 1.1343 | 1.1448 | 1.3154 | 1.1395 | 0.0000 | 1.3677 | 1.3139 | 1.2909 |
| Fractalkine (CX3CL1) | 0.0000 | 0.0000 | 0.0000 | 1.7596 | 0.0000 | 1.1105 | 1.1444 | 1.2833 | 0.0000 | 0.0000 | 1.3101 | 1.2827 | 1.2385 |
| G-CFS (CSF3) | 3.2969 | 3.6056 | 0.0000 | 1.9394 | 2.2853 | 2.6573 | 1.3552 | 2.2872 | 1.9620 | 0.0000 | 2.1202 | 1.6272 | 1.2719 |
| GCP-2 (CXCL6) | 0.0000 | 0.0000 | 1.2054 | 1.7991 | 0.0000 | 1.1592 | 1.2924 | 1.4153 | 0.0000 | 0.0000 | 1.4873 | 1.4507 | 1.2751 |
| GDNF | 1.1058 | 1.2071 | 1.3212 | 1.8110 | 1.1124 | 1.1926 | 1.3473 | 1.3364 | 1.6682 | 1.1385 | 1.6584 | 2.0279 | 1.5048 |
| GITR | 2.2719 | 2.1740 | 1.2024 | 2.0871 | 1.6620 | 2.4562 | 1.3362 | 1.7997 | 1.3507 | 1.1715 | 1.8460 | 1.4030 | 1.7477 |
| GITR ligand | 2.2175 | 2.3456 | 1.1608 | 1.6428 | 1.6049 | 2.6509 | 1.6927 | 1.9125 | 1.3618 | 1.1350 | 1.9824 | 1.6455 | 2.5985 |

**Table 6: Proteins Expression With and Without IL-2 treatment in SR and SEN hADSCs**

v data protein microarray ( normalized values against negative )

| Protein Name | 10% PRP | SR | | | SR + IL2 | | | SEN | | | SEN + IL2 | | |
|---|---|---|---|---|---|---|---|---|---|---|---|---|---|
| | | 24h | 48h | 72h | 24h | 48h | 72h | 24h | 48h | 72h | 24h | 48h | 72h |
| GM CSF (CSF2) | 0.0000 | 0.0000 | 0.0000 | 1.5127 | 0.0000 | 0.0000 | 2.4429 | 0.0000 | 0.0000 | 0.0000 | 1.4081 | 1.1090 | 1.9433 |
| GRO | 2.7029 | 2.9214 | 1.4072 | 2.4796 | 2.1248 | 2.5889 | 1.3419 | 2.3881 | 2.0397 | 1.4234 | 2.7892 | 1.7303 | 1.9509 |
| GRO alpha (CXCL1) | 2.8341 | 2.2512 | 1.2201 | 1.7872 | 2.3741 | 1.9669 | 0.0000 | 2.1295 | 2.0200 | 1.6963 | 3.0249 | 1.9727 | 2.3982 |
| HCC-4 (CCL16) | 2.1082 | 1.6714 | 1.2824 | 1.7032 | 1.8196 | 1.8947 | 0.0000 | 1.4507 | 1.5859 | 1.4496 | 2.6994 | 1.4501 | 2.0235 |
| HGF | 0.0000 | 0.0000 | 0.0000 | 0.0000 | 0.0000 | 0.0000 | 0.0000 | 0.0000 | 0.0000 | 1.3151 | 1.2064 | 1.1948 | 1.3155 |
| I-309 (CCL1) | 0.0000 | 1.1716 | 0.0000 | 1.3749 | 0.0000 | 0.0000 | 0.0000 | 1.2270 | 0.0000 | 0.0000 | 1.8541 | 1.1163 | 0.0000 |
| I-TAC (CXCL11) | 1.7773 | 2.4167 | 0.0000 | 1.1429 | 1.9403 | 2.4790 | 1.6060 | 2.0923 | 4.4408 | 1.1135 | 1.9897 | 2.8718 | 2.1238 |
| ICAM 2 | 7.7260 | 5.1717 | 10.6862 | 5.1954 | 2.9367 | 11.5585 | 7.0205 | 8.4510 | 21.9788 | 4.0921 | 5.0098 | 17.6250 | 4.3301 |
| ICAM-1 | 2.2744 | 2.7507 | 0.0000 | 1.3208 | 2.4726 | 2.2341 | 1.4577 | 2.3083 | 3.6382 | 1.1280 | 2.3291 | 3.0441 | 2.4095 |
| ICAM-3 | 2.6613 | 2.2837 | 0.0000 | 1.4283 | 2.0152 | 3.2082 | 1.9390 | 1.8424 | 2.4951 | 0.0000 | 2.1678 | 2.6968 | 1.8250 |
| IFN gamma | 0.0000 | 1.3137 | 0.0000 | 1.3248 | 0.0000 | 0.0000 | 1.1216 | 1.3209 | 1.1836 | 0.0000 | 2.0549 | 2.2996 | 1.2292 |
| IGF 1 | 1.2639 | 1.1234 | 0.0000 | 1.7804 | 1.5034 | 1.6790 | 1.7504 | 1.5738 | 2.4959 | 0.0000 | 1.3921 | 2.0765 | 1.7998 |
| IGF-1 sR | 2.3210 | 2.1679 | 0.0000 | 1.8193 | 1.1657 | 3.8612 | 1.8814 | 2.1052 | 2.0054 | 1.1465 | 2.0784 | 2.2369 | 1.7936 |
| IGF-2 | 7.8210 | 5.3984 | 13.3460 | 5.3629 | 3.4114 | 13.2883 | 7.4609 | 8.4417 | 18.6936 | 3.7455 | 5.2313 | 13.0938 | 3.9503 |
| IGFBP 1 | 0.0000 | 1.4184 | 1.2150 | 1.3997 | 0.0000 | 0.0000 | 1.3760 | 1.5956 | 1.7953 | 1.2422 | 2.5289 | 2.1571 | 1.1460 |
| IGFBP 2 | 0.0000 | 1.3743 | 1.3597 | 1.5149 | 0.0000 | 1.1398 | 1.4701 | 1.6614 | 2.1925 | 1.3702 | 2.9630 | 3.7437 | 1.5504 |
| IGFBP 3 | 2.8931 | 2.1995 | 0.0000 | 1.5877 | 1.8361 | 3.2348 | 1.9069 | 1.9777 | 2.8991 | 1.2437 | 2.2331 | 3.0895 | 1.9370 |
| IGFBP 4 | 0.0000 | 0.0000 | 1.1015 | 1.4862 | 0.0000 | 0.0000 | 0.0000 | 1.1592 | 1.5931 | 1.2422 | 2.7576 | 2.5841 | 0.0000 |
| IGFBP 6 | 2.6328 | 2.0586 | 0.0000 | 1.6428 | 1.5540 | 3.6886 | 1.6927 | 1.9589 | 2.4686 | 1.1350 | 2.1048 | 2.6761 | 2.5985 |
| IL 1 beta | 1.2030 | 1.1404 | 1.1401 | 1.6718 | 1.2215 | 1.2587 | 1.4024 | 1.3315 | 1.1020 | 0.0000 | 1.4070 | 1.8535 | 1.5430 |
| IL 1ra | 1.4787 | 1.4602 | 1.5494 | 2.0854 | 1.6168 | 1.5486 | 1.7840 | 1.6918 | 1.6712 | 1.1120 | 1.7498 | 2.6571 | 1.9368 |
| IL-1 alpha | 0.0000 | 0.0000 | 0.0000 | 1.4987 | 1.2027 | 1.1183 | 1.1942 | 1.1610 | 0.0000 | 0.0000 | 1.1988 | 1.4154 | 1.3277 |
| IL-1 R1 | 1.8982 | 1.7219 | 0.0000 | 1.7535 | 0.0000 | 3.4568 | 1.5748 | 1.6351 | 1.5707 | 0.0000 | 1.7159 | 1.6807 | 1.5087 |
| IL-1 R4 | 3.5852 | 4.2726 | 1.7262 | 3.0683 | 2.2333 | 5.9276 | 2.2413 | 3.3577 | 3.4899 | 1.7249 | 4.1825 | 3.5853 | 2.9641 |
| IL-10 | 0.0000 | 0.0000 | 0.0000 | 1.4609 | 1.2651 | 1.2192 | 1.2172 | 1.2012 | 1.9078 | 0.0000 | 1.1018 | 1.7283 | 1.7007 |

300

**Table 6: Proteins Expression With and Without IL-2 treatment in SR and SEN hADSCs**

v data protein microarray ( normalized values against negative )

| Protein Name | 10% PRP | SR 24h | 48h | 72h | SR + IL2 24h | 48h | 72h | SEN 24h | 48h | 72h | SEN + IL2 24h | 48h | 72h |
|---|---|---|---|---|---|---|---|---|---|---|---|---|---|
| IL-10 R beta | 4.2176 | 2.0724 | 6.9379 | 2.8859 | 1.2468 | 6.4507 | 4.4566 | 4.9829 | 8.2023 | 2.4840 | 2.9986 | 6.1719 | 2.6166 |
| IL-11 | 1.7938 | 1.7272 | 0.0000 | 1.6827 | 1.2060 | 2.9873 | 1.4332 | 1.7263 | 1.4346 | 0.0000 | 1.7404 | 1.4698 | 1.3806 |
| IL-12 p40 (IL12b) | 2.2191 | 2.2428 | 1.3027 | 2.2102 | 1.7044 | 3.5189 | 1.7550 | 2.0841 | 1.7512 | 1.3740 | 2.5586 | 1.6757 | 2.0308 |
| IL-12 p70 (IL12a) | 2.2645 | 2.4740 | 1.2300 | 1.9482 | 1.9702 | 2.8214 | 1.6628 | 1.8080 | 1.7086 | 1.3278 | 2.3665 | 1.6441 | 1.8878 |
| IL-13 | 0.0000 | 0.0000 | 0.0000 | 1.4879 | 1.1238 | 1.1229 | 0.0000 | 1.1386 | 1.2390 | 0.0000 | 0.0000 | 1.1181 | 1.3577 |
| IL-13 R alpha 2 | 4.5248 | 2.3811 | 7.9506 | 13.7069 | 1.3987 | 7.6889 | 24.8942 | 5.9393 | 10.0674 | 10.9439 | 3.4112 | 7.7500 | 18.0928 |
| IL-15 | 0.0000 | 0.0000 | 1.1631 | 1.5127 | 1.3185 | 1.3656 | 2.4429 | 1.4099 | 1.3914 | 0.0000 | 1.3143 | 1.7417 | 1.9433 |
| IL-16 | 0.0000 | 0.0000 | 1.1357 | 1.7959 | 1.3020 | 1.2950 | 1.3526 | 1.2798 | 1.3054 | 0.0000 | 1.2625 | 2.0206 | 1.8628 |
| IL-17 | 2.2418 | 2.3619 | 0.0000 | 1.5032 | 2.1217 | 1.9841 | 1.3163 | 1.7896 | 2.0121 | 1.2222 | 2.0095 | 1.4174 | 1.5038 |
| IL-18 BP alpha | 3.0904 | 1.6630 | 6.0565 | 3.1044 | 0.0000 | 5.0158 | 3.7960 | 5.2131 | 6.5491 | 2.4993 | 2.9264 | 5.2500 | 2.3956 |
| IL-18 R beta | 4.4583 | 2.5134 | 8.1398 | 4.7816 | 1.1519 | 7.1012 | 5.5612 | 7.3950 | 14.0732 | 3.7855 | 4.5409 | 9.6563 | 3.0637 |
| IL-1R 2 | 4.7686 | 3.2220 | 7.3541 | 3.0255 | 1.5728 | 7.1200 | 4.3168 | 5.5334 | 9.2091 | 2.5308 | 3.3827 | 7.3438 | 2.8186 |
| IL-2 | 0.0000 | 1.1072 | 0.0000 | 1.2525 | 1.1035 | 0.0000 | 1.1067 | 0.0000 | 1.0095 | 0.0000 | 1.2570 | 1.9272 | 1.3422 |
| IL-2 beta | 2.0328 | 2.0031 | 5.7894 | 3.8280 | 0.0000 | 4.6163 | 3.0567 | 5.0653 | 6.6551 | 3.0717 | 4.1858 | 6.5313 | 2.4540 |
| IL-21 R | 8.9735 | 11.7008 | 13.9870 | 4.8471 | 4.4430 | 15.6644 | 9.3886 | 7.5070 | 21.6821 | 4.1674 | 5.6212 | 22.4688 | 3.7522 |
| IL-2R alpha | 2.9958 | 2.7251 | 1.1476 | 1.5468 | 2.8307 | 1.8495 | 1.0695 | 2.6661 | 2.2709 | 1.4120 | 3.4867 | 1.7784 | 1.8648 |
| IL-2R gamma | 1.3045 | 1.8961 | 6.9868 | 4.1725 | 0.0000 | 4.6728 | 2.6250 | 5.1058 | 9.2408 | 2.8873 | 4.8788 | 8.3125 | 2.8821 |
| IL-3 | 1.1011 | 1.2907 | 0.0000 | 1.3258 | 1.1775 | 1.1401 | 1.2611 | 1.3045 | 1.6659 | 0.0000 | 1.5734 | 3.1692 | 1.9542 |
| IL-4 | 0.0000 | 1.3943 | 0.0000 | 1.2039 | 0.0000 | 0.0000 | 1.1511 | 1.3160 | 1.2914 | 0.0000 | 1.6728 | 1.5556 | 0.0000 |
| IL-5 | 1.1291 | 1.4900 | 1.1305 | 1.1746 | 0.0000 | 0.0000 | 1.1690 | 1.2913 | 1.7102 | 1.1760 | 1.8514 | 2.2417 | 1.2944 |
| IL-5 R alpha | 4.2683 | 7.0835 | 7.8727 | 3.8540 | 1.6203 | 8.2097 | 4.6996 | 4.0544 | 7.8844 | 2.3564 | 3.0745 | 9.8594 | 2.6166 |
| IL-6 | 1.1682 | 1.5695 | 1.2615 | 1.5127 | 1.1780 | 1.1444 | 2.4429 | 1.2190 | 1.6850 | 0.0000 | 1.6620 | 2.3157 | 1.9433 |
| IL-6R | 2.3560 | 2.0135 | 1.1477 | 1.6428 | 2.5712 | 1.6563 | 1.6927 | 1.6988 | 1.7804 | 1.1350 | 2.8486 | 1.5573 | 2.5985 |
| IL-7 | 0.0000 | 1.1716 | 0.0000 | 1.3876 | 0.0000 | 0.0000 | 0.0000 | 0.0000 | 1.5363 | 1.1676 | 0.0000 | 2.4300 | 0.0000 |
| IL-8 | 0.0000 | 1.1393 | 0.0000 | 1.9613 | 1.4520 | 1.3270 | 0.0000 | 0.0000 | 1.8381 | 2.1195 | 1.8577 | 1.5563 | 2.4861 |
| IL-9 | 4.0340 | 4.8063 | 6.7442 | 3.8011 | 1.0190 | 6.9234 | 4.5056 | 3.9440 | 8.9653 | 2.3180 | 2.9429 | 8.4063 | 2.6065 |

**Table 6: Proteins Expression With and Without IL-2 treatment in SR and SEN hADSCs**

v data protein microarray ( normalized values against negative )

| Protein Name | 10% PRP | SR 24h | 48h | 72h | SR + IL2 24h | 48h | 72h | SEN 24h | 48h | 72h | SEN + IL2 24h | 48h | 72h |
|---|---|---|---|---|---|---|---|---|---|---|---|---|---|
| IP-10 (CXCL10) | 5.0377 | 4.9197 | 8.5583 | 13.7069 | 1.4114 | 9.4689 | 24.8942 | 4.9673 | 13.4692 | 10.9439 | 3.4745 | 10.9688 | 18.0928 |
| L Selectin | 5.8958 | 2.4315 | 7.8482 | 3.6086 | 1.6804 | 9.4815 | 6.0278 | 6.0855 | 16.1079 | 3.7632 | 4.4776 | 13.1563 | 3.5312 |
| LAP (TGF b1) | 5.2942 | 3.9591 | 7.4721 | 3.9233 | 1.2595 | 9.5400 | 4.9198 | 5.0311 | 11.8266 | 3.1224 | 3.4517 | 10.0313 | 3.1641 |
| Leptin | 1.1812 | 1.1291 | 1.1443 | 1.7150 | 1.5761 | 1.8687 | 1.5569 | 1.3935 | 2.5268 | 0.0000 | 1.3439 | 1.7122 | 1.9316 |
| Leptin R | 7.8368 | 5.3795 | 10.9243 | 4.5776 | 2.6203 | 12.8511 | 6.7601 | 7.9487 | 21.3642 | 4.0821 | 4.9288 | 14.9375 | 3.7928 |
| LIF | 4.5659 | 2.2646 | 6.5506 | 2.9504 | 1.2342 | 7.3563 | 4.4042 | 4.8414 | 9.4422 | 2.6891 | 3.3099 | 6.5938 | 2.6192 |
| LIGHT (TNFSF14) | 1.3757 | 1.3005 | 1.4893 | 1.9354 | 1.7565 | 2.0072 | 1.5816 | 1.5204 | 2.2014 | 0.0000 | 1.4663 | 2.3087 | 2.5589 |
| M-CSF (CSF1) | 1.4403 | 1.2881 | 1.4465 | 1.7755 | 1.4197 | 1.5868 | 1.5603 | 1.7082 | 1.6379 | 0.0000 | 1.6473 | 2.5690 | 1.9209 |
| M-CSF R (CSF1R) | 7.6278 | 3.5591 | 12.3711 | 5.0751 | 3.2342 | 13.9597 | 9.1562 | 9.5568 | 27.8179 | 5.3521 | 6.1984 | 18.9219 | 4.1117 |
| MCP-1 (CCL2) | 1.2320 | 1.4263 | 1.6123 | 3.3764 | 1.5828 | 2.2230 | 1.5260 | 1.4099 | 1.6576 | 0.0000 | 1.3597 | 2.2346 | 2.4159 |
| MCP-2 (CCL8) | 1.1270 | 1.1287 | 1.1030 | 1.6005 | 1.3047 | 1.5253 | 1.2381 | 1.3177 | 1.5346 | 0.0000 | 1.2708 | 1.9635 | 1.9084 |
| MCP-3 (CCL7) | 1.1107 | 0.0000 | 0.0000 | 1.3730 | 1.1270 | 1.3022 | 0.0000 | 1.1747 | 1.1412 | 0.0000 | 1.1329 | 1.3170 | 1.3534 |
| MCP-4 (CCL13) | 1.1581 | 0.0000 | 0.0000 | 1.4008 | 1.1299 | 1.2833 | 1.1590 | 1.2178 | 0.0000 | 0.0000 | 1.1745 | 1.4451 | 1.3498 |
| MDC (CCL22) | 1.2655 | 1.1861 | 1.3001 | 1.5127 | 1.3103 | 1.3770 | 2.4429 | 1.4027 | 1.3767 | 0.0000 | 1.3528 | 2.0833 | 1.9433 |
| MIF | 2.1597 | 2.1795 | 0.0000 | 1.8483 | 1.6636 | 4.6365 | 2.5650 | 2.1712 | 2.3463 | 1.3275 | 2.1213 | 2.6968 | 2.4719 |
| MIG (CXCL9) | 1.1859 | 1.1170 | 0.0000 | 1.2173 | 1.1761 | 1.2199 | 1.2101 | 1.1147 | 0.0000 | 0.0000 | 0.0000 | 1.5816 | 0.0000 |
| MIP-1 alpha (CCL3) | 2.4060 | 2.1377 | 0.0000 | 1.9708 | 1.6713 | 4.3292 | 2.3397 | 2.2547 | 2.3589 | 1.4853 | 2.2950 | 2.8582 | 2.6981 |
| MIP-1 beta (CCL4) | 2.2925 | 2.1884 | 1.1355 | 1.9657 | 1.4562 | 4.2223 | 2.2441 | 2.2207 | 2.5274 | 1.6386 | 2.2041 | 2.9260 | 3.0404 |

**Table 6: Proteins Expression With and Without IL-2 treatment in SR and SEN hADSCs**

data protein microarray ( normalized values against negative )

| Protein Name | 10% PRP | SR 24h | 48h | 72h | SR + IL2 24h | 48h | 72h | SEN 24h | 48h | 72h | SEN + IL2 24h | 48h | 72h |
|---|---|---|---|---|---|---|---|---|---|---|---|---|---|
| MIP-1 delta (CCL15) | 1.1882 | 1.1553 | 0.0000 | 1.1814 | 1.1814 | 1.2850 | 1.2868 | 1.2058 | 1.2023 | 0.0000 | 1.1628 | 1.6067 | 0.0000 |
| MIP-3 alpha (CCL20) | 1.2230 | 1.2933 | 1.1993 | 1.2213 | 1.2494 | 1.2286 | 1.3463 | 1.2933 | 1.2335 | 1.1105 | 1.2473 | 1.5692 | 1.1384 |
| MIP-3 beta (CCL19) | 1.6491 | 1.8158 | 0.0000 | 1.5196 | 0.0000 | 3.4060 | 1.7735 | 1.8004 | 1.7130 | 1.1557 | 1.7639 | 1.9080 | 1.5432 |
| MMP 1 | 3.2550 | 0.0000 | 7.0447 | 3.2391 | 0.0000 | 6.7937 | 4.8499 | 4.8212 | 8.8911 | 3.1985 | 3.3213 | 8.0000 | 2.4960 |
| MMP 13 | 4.0308 | 1.3165 | 7.1004 | 3.4623 | 0.0000 | 7.1618 | 4.8866 | 5.4572 | 9.7071 | 3.7133 | 3.7485 | 8.2813 | 2.4667 |
| MMP 3 | 4.3474 | 3.5780 | 9.0012 | 5.6564 | 0.0000 | 7.8855 | 5.4633 | 7.0731 | 14.0308 | 4.3687 | 4.9143 | 15.0156 | 3.7077 |
| MMP 9 | 3.1980 | 1.1780 | 6.3658 | 3.3045 | 0.0000 | 6.0030 | 3.5391 | 5.1462 | 10.7987 | 3.4759 | 3.9074 | 7.3438 | 2.1936 |
| MPIF-1 | 2.8972 | 1.2945 | 7.4209 | 13.7069 | 0.0000 | 7.0510 | 24.8942 | 5.3281 | 13.3844 | 10.9439 | 5.1244 | 12.1250 | 18.0928 |
| MSP alpha (HGF like) | 1.5158 | 1.5849 | 0.0000 | 1.4188 | 0.0000 | 3.1234 | 1.6842 | 1.5789 | 1.4513 | 0.0000 | 1.5278 | 1.3833 | 1.3102 |
| NAP-2 | 1.3355 | 1.6746 | 1.5684 | 1.4006 | 1.4223 | 1.3801 | 1.6883 | 1.5784 | 2.6414 | 1.8530 | 1.5222 | 3.8348 | 2.1190 |
| NGFR | 0.0000 | 0.0000 | 3.9620 | 2.0131 | 0.0000 | 2.5434 | 1.1675 | 2.8227 | 3.8786 | 1.5059 | 3.3074 | 2.8438 | 1.5420 |
| NT-3 | 1.4553 | 1.8727 | 1.7590 | 1.4913 | 1.6149 | 1.3835 | 1.6577 | 1.7653 | 2.9707 | 2.0114 | 1.7024 | 6.5136 | 2.7886 |
| NT-4 | 1.7015 | 1.7823 | 0.0000 | 1.6428 | 1.1224 | 3.1103 | 1.6927 | 1.7011 | 1.5498 | 1.1350 | 1.7016 | 1.2623 | 2.5985 |
| OPG | 1.9588 | 2.0288 | 1.2671 | 1.9805 | 1.3503 | 3.1937 | 2.0473 | 1.8625 | 1.6066 | 1.3444 | 2.1143 | 1.5444 | 1.9144 |
| OSM | 2.9682 | 3.0088 | 1.5496 | 2.1498 | 2.5752 | 3.1750 | 2.0925 | 2.2717 | 2.2816 | 1.8480 | 2.8324 | 2.2654 | 3.5391 |
| PARC (CCL18) | 1.4146 | 1.8942 | 1.8088 | 1.5028 | 1.6723 | 1.5219 | 1.4773 | 1.9464 | 2.2464 | 1.4573 | 1.8771 | 3.9748 | 2.1256 |
| PDGF AA | 4.3728 | 7.2819 | 5.8806 | 2.9061 | 0.0000 | 9.9312 | 4.9390 | 4.1586 | 6.0934 | 2.0829 | 2.9321 | 8.0313 | 2.1721 |
| PDGF AB | 4.3474 | 7.3953 | 6.6908 | 3.5759 | 0.0000 | 9.7576 | 5.3602 | 4.3733 | 8.7746 | 2.8182 | 3.7947 | 8.4375 | 2.6395 |
| PDGF BB | 1.1755 | 1.1456 | 1.2772 | 2.1257 | 1.8991 | 1.9970 | 1.9589 | 1.9444 | 3.4659 | 0.0000 | 1.8752 | 2.4537 | 3.1652 |
| PDGF R alpha | 2.6218 | 4.0220 | 4.1601 | 2.6213 | 0.0000 | 5.3358 | 3.4010 | 2.2862 | 3.3276 | 1.9000 | 2.3809 | 3.8125 | 1.9993 |
| PDGF R beta | 2.7991 | 1.9717 | 3.3721 | 2.3615 | 0.0000 | 4.5682 | 2.9152 | 2.2333 | 2.9884 | 1.8701 | 2.1214 | 2.2500 | 1.5865 |
| PECAM 1 | 3.5242 | 2.0472 | 5.2218 | 2.6925 | 0.0000 | 6.5657 | 3.8869 | 3.6330 | 7.3545 | 2.6653 | 2.6562 | 4.1563 | 2.0831 |
| PLGF | 1.9995 | 2.1042 | 1.2736 | 1.4493 | 1.9781 | 2.0050 | 1.5313 | 2.0033 | 1.8854 | 1.2553 | 2.0916 | 1.5373 | 2.1870 |
| PRL | 4.8002 | 2.3811 | 7.5433 | 3.0255 | 0.0000 | 9.0485 | 4.7852 | 4.8072 | 9.9615 | 3.1424 | 3.3080 | 8.3281 | 2.5328 |

**Table 6: Proteins Expression With and Without IL-2 treatment in SR and SEN hADSCs**

v data protein microarray ( normalized values against negative )

| Protein Name | 10% PRP | SR | | | SR + IL2 | | | SEN | | | SEN + IL2 | | |
|---|---|---|---|---|---|---|---|---|---|---|---|---|---|
| | | 24h | 48h | 72h | 24h | 48h | 72h | 24h | 48h | 72h | 24h | 48h | 72h |
| RANTES (CCL5) | 1.3096 | 1.2135 | 1.6016 | 2.1810 | 1.7488 | 2.1340 | 1.7569 | 1.8236 | 3.6534 | 1.2143 | 1.7586 | 3.3439 | 3.7305 |
| SCF | 1.2204 | 1.1268 | 1.3764 | 1.5127 | 1.3769 | 1.8305 | 2.4429 | 1.5836 | 1.8959 | 0.0000 | 1.5272 | 1.9418 | 1.9433 |
| SCF R | 4.9364 | 1.8457 | 7.9796 | 3.3844 | 1.1329 | 10.1508 | 5.4878 | 5.3686 | 12.3565 | 3.5988 | 3.7536 | 8.7031 | 2.8834 |
| SDF-1 alpha | 1.3057 | 1.1283 | 1.3137 | 1.6077 | 1.3562 | 1.8259 | 1.2496 | 1.7122 | 1.6701 | 0.0000 | 1.6512 | 1.9230 | 1.9613 |
| SDF-1 beta | 4.2619 | 0.0000 | 6.3080 | 13.7069 | 0.0000 | 7.6826 | 24.8942 | 4.7605 | 9.9827 | 10.9439 | 3.4973 | 8.0313 | 18.0928 |
| sgp130 (IL6ST) | 2.2686 | 2.3484 | 1.4306 | 1.4705 | 2.5113 | 1.7665 | 1.3003 | 2.3061 | 1.7749 | 1.3635 | 2.6473 | 1.6322 | 2.2869 |
| Siglec 5 | 2.5299 | 0.0000 | 4.7655 | 2.4182 | 0.0000 | 5.4111 | 3.5740 | 3.4557 | 6.3796 | 2.9173 | 2.8220 | 4.0938 | 2.1276 |
| sTNFRI (TNFRSF1A) | 0.0000 | 0.0000 | 1.1578 | 0.0000 | 1.4643 | 0.0000 | 0.0000 | 0.0000 | 1.2100 | 1.6022 | 1.7870 | 1.8964 | 2.3271 |
| sTNFRII (TNFRSF1B) | 1.9044 | 1.9186 | 1.4867 | 1.4075 | 2.6485 | 1.5852 | 1.0310 | 1.7671 | 1.7607 | 1.5579 | 2.6495 | 1.7601 | 2.3271 |
| TARC (CCL17) | 1.3270 | 1.1513 | 1.2954 | 1.6107 | 1.2686 | 1.6083 | 1.1221 | 1.6134 | 1.7633 | 1.1083 | 1.5560 | 2.3620 | 1.8573 |
| TECK (CCL25) | 1.1900 | 2.0126 | 0.0000 | 1.3492 | 1.2024 | 2.7107 | 2.0992 | 1.5479 | 3.3719 | 0.0000 | 1.7041 | 1.7530 | 1.8681 |
| TGF alpha | 2.0360 | 0.0000 | 4.4116 | 2.2402 | 0.0000 | 4.1582 | 3.0532 | 3.0140 | 4.3025 | 2.6146 | 2.4005 | 1.9688 | 1.5979 |
| TGF beta 1 | 1.2030 | 0.0000 | 1.1813 | 1.5739 | 0.0000 | 1.4450 | 0.0000 | 1.4940 | 1.5380 | 0.0000 | 1.4408 | 1.6989 | 1.4131 |
| TGF beta 2 | 3.9168 | 0.0000 | 6.9535 | 3.1688 | 0.0000 | 6.1578 | 3.6003 | 4.6998 | 10.9470 | 3.4282 | 3.7264 | 6.3438 | 2.2800 |
| TGF beta 3 | 1.3716 | 1.1878 | 1.3123 | 1.6893 | 1.1931 | 1.5166 | 1.2988 | 1.5534 | 1.4329 | 0.0000 | 1.4981 | 1.7555 | 1.3785 |
| THPO | 1.6967 | 1.2428 | 0.0000 | 1.4502 | 1.2371 | 2.8090 | 1.9955 | 1.3720 | 1.9699 | 1.2798 | 1.3259 | 1.8384 | 1.6889 |
| TIE-1 | 3.4260 | 0.0000 | 6.7108 | 3.1987 | 0.0000 | 6.3795 | 3.4971 | 4.6781 | 12.2505 | 3.5942 | 3.7327 | 5.9375 | 2.2889 |
| TIE-2 | 3.3437 | 0.0000 | 8.8410 | 3.4287 | 0.0000 | 8.2160 | 4.4549 | 6.0062 | 16.0655 | 4.4463 | 5.2440 | 11.1250 | 2.8415 |
| TIMP-1 | 1.6136 | 9.7600 | 7.0577 | 14.7773 | 4.6085 | 24.4847 | 15.5688 | 2.9073 | 7.2915 | 2.6604 | 3.3255 | 4.3043 | 5.6560 |
| TIMP-2 | 1.1656 | 1.5467 | 1.1132 | 1.6428 | 1.2063 | 4.0519 | 1.6927 | 1.2993 | 1.6808 | 1.1350 | 1.2203 | 1.5861 | 2.5985 |
| TIMP-4 | 1.4439 | 0.0000 | 7.1471 | 2.0362 | 0.0000 | 5.3609 | 3.0130 | 3.7558 | 11.1696 | 3.4805 | 4.3137 | 8.5625 | 2.6852 |
| TNF alpha | 1.2282 | 0.0000 | 1.1371 | 1.3016 | 0.0000 | 1.2323 | 0.0000 | 0.0000 | 1.1535 | 0.0000 | 0.0000 | 1.4096 | 1.1837 |
| TNF beta | 1.3485 | 1.1618 | 1.3546 | 1.4087 | 1.2383 | 1.3726 | 1.1619 | 1.2614 | 1.5829 | 0.0000 | 1.2165 | 2.1747 | 1.5305 |

**Table 6: Proteins Expression With and Without IL-2 treatment in SR and SEN hADSCs**

v data  protein microarray ( normalized values against negative )

| Protein Name | 10% PRP | SR 24h | 48h | 72h | SR + IL2 24h | 48h | 72h | SEN 24h | 48h | 72h | SEN + IL2 24h | 48h | 72h |
|---|---|---|---|---|---|---|---|---|---|---|---|---|---|
| TRAIL R3 (TNFRSD10C) | 1.4030 | 1.4235 | 0.0000 | 1.5132 | 0.0000 | 2.7369 | 2.1068 | 1.3606 | 1.3436 | 0.0000 | 1.2645 | 1.3823 | 1.3020 |
| TRAIL R4 (TNFRSF10D) | 1.2073 | 1.4240 | 0.0000 | 1.4843 | 0.0000 | 2.4524 | 2.0421 | 1.4239 | 1.2442 | 0.0000 | 1.2353 | 1.1666 | 0.0000 |
| uPAR | 1.2991 | 1.5921 | 0.0000 | 1.7088 | 0.0000 | 2.5193 | 2.1857 | 1.4108 | 1.2572 | 0.0000 | 1.1521 | 1.1368 | 0.0000 |
| VE Cadherin | 2.9669 | 4.3591 | 2.8824 | 1.7976 | 0.0000 | 7.7307 | 4.3098 | 1.7449 | 2.7341 | 1.5712 | 1.9955 | 3.2969 | 1.8583 |
| VEGF | 1.6355 | 1.6877 | 0.0000 | 1.6013 | 1.3020 | 2.1407 | 1.8824 | 1.4757 | 1.9375 | 1.3938 | 1.1928 | 1.6292 | 1.3449 |
| VEGF R2 | 4.0593 | 5.0457 | 3.9375 | 2.1459 | 0.0000 | 9.0108 | 4.9163 | 2.4588 | 5.5530 | 2.1928 | 2.6074 | 6.1406 | 2.2254 |
| VEGF R3 | 8.6854 | 9.4142 | 9.9049 | 13.7069 | 0.0000 | 15.7355 | 24.8942 | 6.2240 | 18.9056 | 10.9439 | 4.7200 | 18.5156 | 18.0928 |
| VEGF-D | 1.7188 | 1.8505 | 1.2501 | 1.7738 | 1.2923 | 2.2446 | 2.0839 | 1.6580 | 1.6986 | 1.3904 | 1.4664 | 1.4556 | 1.6349 |
| XCL1 | 2.3969 | 2.7051 | 0.0000 | 1.7291 | 0.0000 | 4.6858 | 2.3568 | 2.3074 | 3.3162 | 1.3284 | 2.4838 | 3.8532 | 2.6120 |
| | | | | | | | | | | | | | |
| Array 6 | | | | | | | | | | | | | |
| Mean positive | 3.1982 | 3.5238 | 4.1985 | 4.4906 | 2.8622 | 3.0507 | 3.7518 | 3.6569 | 3.5047 | 4.4157 | 3.9684 | 4.3600 | 4.0705 |
| Mean negative | 0.9894 | 0.9930 | 0.9932 | 0.9928 | 0.9825 | 0.9880 | 0.9972 | 0.9898 | 0.9956 | 1.0073 | 0.9894 | 0.9919 | 0.9947 |
| | | | | | | | | | | | | | |
| Array 7 | | | | | | | | | | | | | |
| Mean positive | 2.4540 | 3.5311 | 3.2874 | 3.6983 | 2.9950 | 3.2025 | 2.4273 | 4.5576 | 3.0852 | 2.3917 | 5.6725 | 2.9576 | 4.4572 |
| Mean negative | 0.9675 | 0.9729 | 0.9906 | 0.9920 | 0.9707 | 0.9879 | 0.9650 | 0.9788 | 0.9930 | 0.9979 | 0.9787 | 0.9786 | 0.9736 |
| | | | | | | | | | | | | | |
| Array 8 | | | | | | | | | | | | | |
| Mean positive | 5.6484 | 3.8376 | 6.8396 | 7.6892 | 3.9872 | 5.6337 | 4.8771 | 4.9257 | 4.5014 | 6.6287 | 5.2075 | 4.0405 | 3.7488 |
| Mean negative | 0.9030 | 0.7308 | 0.8919 | 0.9719 | 1.0128 | 0.8939 | 0.9503 | 0.9263 | 0.8167 | 0.9445 | 0.9649 | 0.7984 | 0.9817 |

EP 3 303 636 B1

**Claims**

1. A method of determining whether an individual eligible to receive an IL-2-based therapy may experience an adverse event associated with the IL-2-based therapy, the method comprising:

    (a) measuring the expression levels of at least two biomarkers selected from a panel of biomarkers in a blood, plasma or serum sample obtained from the individual, wherein the sample is combined with IL-2 *in vitro* before IL-2-based therapy, and wherein the panel of biomarkers consists of TGFβ1, PLEKHA6, CTSB, FERMT1, CRMP1, VEGFB, VEGFA, and PLEKHA1; and
    (b) comparing the levels of the biomarkers to reference levels, wherein an increase in the levels above the reference levels indicates that the individual may experience an adverse event associated with the IL-2-based therapy, and wherein a decrease or no change in the levels compared to the references levels indicates that the individual may not experience an adverse event associated with the IL-2-based therapy.

2. An IL-2-based therapeutic for use in a method of treating an individual for cancer, the method comprising administering an effective amount of the IL-2-based therapeutic to the individual when the expression levels of at least two biomarkers selected from a panel of biomarkers is decreased, or exhibits no change, in comparison to reference levels in a blood, plasma or serum sample from the individual wherein the sample is combined with IL-2 *in vitro* before IL-2-based therapy, and wherein the panel of biomarkers consists of TGFβ1, PLEKHA6, CTSB, FERMT1, CRMP1, VEGFB, VEGFA, and PLEKHA1.

3. The method of claim 1, or the IL-2-based therapeutic for use according to claim 2, wherein the sample is combined with IL-2 for 24 hours; or the expression levels are measured 24, 48, or 72 hours following the removal of IL-2 from the sample *in vitro.*

4. An *in vitro* method of determining whether a population of mesenchymal stem cells (MSCs) is suitable for administration into an individual for a MSC-based therapy, comprising:

    (a) incubating IL-2 with the population of MSCs;
    (b) measuring the expression levels in the MSCs of at least two biomarkers selected from a panel of biomarkers, wherein the panel of biomarkers consists of TGFβ1, PLEKHA6, CTSB, FERMT1, CRMP1, VEGFB, VEGFA, and PLEKHA1; and
    (c) comparing the levels of the biomarkers to reference levels, wherein an increase in the levels above the reference levels indicates that the MSCs are not suitable for administration into an individual and no change or a decrease in the levels below the reference levels indicates that the MSCs are suitable for administration into an individual.

5. The method of claims 1 or 4, or the IL-2-based therapeutic for use according to claim 2, wherein the method comprises measuring the expression levels of at least three biomarkers, at least four biomarkers, or at least five biomarkers from the panel of biomarkers.

6. The method of claims 1 or 4, or the IL-2-based therapeutic for use according to claim 2, wherein the method comprises:

    (i) measuring protein levels of the biomarkers; optionally wherein the measuring is with an ELISA assay, an antibody proteomic array, immunohistochemistry, or mass spectrometry; or
    (ii) measuring RNA levels of the biomarkers; optionally wherein the measuring is with a Q-PCR assay or RNA-seq.

7. The method of claim 4, wherein the cells are incubated with the IL-2 for 24 hours; or wherein the measuring is carried out 24, 48, or 72 hours following removal of the IL-2.

8. A population of mesenchymal stem cells (MSCs) for use in a method of treatment, the method comprising the method of claim 4, and further comprising administering the population of cells to the individual.

**Patentansprüche**

1. Verfahren zum Bestimmen, ob ein Individuum, das für das Empfangen einer IL-2-basierten Therapie berechtigt ist, ein mit der IL-2-basierten Therapie verbundenes unerwünschtes Ereignis erleiden könnte, wobei das Verfahren Folgendes umfasst:

(a) Messen der Expressionsspiegel von zumindest zwei Biomarkern, ausgewählt aus einem Panel von Biomarkern in einer Blut-, Plasma- oder Serumprobe, die von dem Individuum erhalten wurde, wobei die Probe vor der IL-2-basierten Therapie *in vitro* mit IL-2 kombiniert wird, und wobei das Panel von Biomarkern aus TGFβ1, PLEKHA6, CTSB, FERMT1, CRMP1, VEGFB, VEGFA und PLEKHA1 besteht; und
(b) Vergleichen der Spiegel der Biomarker mit Referenzspiegeln, wobei ein Anstieg in den Spiegeln über die Referenzspiegel darauf hindeutet, dass das Individuum ein mit der IL-2-basierten Therapie verbundenes unerwünschtes Ereignis erleiden könnte, und wobei eine Abnahme oder keine Veränderung in den Spiegeln verglichen mit den Referenzspiegeln darauf hindeutet, dass das Individuum kein mit der IL-2-basierten Therapie verbundenes unerwünschtes Ereignis erleiden könnte.

2. IL-2-basiertes Therapeutikum zur Verwendung in einem Verfahren zur Behandlung eines Individuums bei Krebs, wobei das Verfahren das Verabreichen einer wirksamen Menge des IL-2-basierten Therapeutikums an das Individuum umfasst, wenn die Expressionsspiegel von zumindest zwei Biomarkern, ausgewählt aus einem Panel von Biomarkern abgenommen haben oder keine Veränderung im Vergleich zu den Referenzspiegeln in einer Blut-, Plasma- oder Serumprobe des Individuums aufzeigt, wobei die Probe vor der IL-2-basierten Therapie mit IL-2 *in vitro* kombiniert wird, und wobei das Panel von Biomarkern aus TGFβ1, PLEKHA6, CTSB, FERMT1, CRMP1, VEGFB, VEGFA und PLEKHA1 besteht.

3. Verfahren nach Anspruch 1 oder IL-2-basiertes Therapeutikum zur Verwendung nach Anspruch 2, wobei die Probe über 24 Stunden mit IL-2 kombiniert wird oder die Expressionsspiegel 24, 48 oder 72 Stunden nach dem Entfernen von IL-2 aus der Probe *in vitro* gemessen werden.

4. *In-vitro*-Verfahren zum Bestimmen, ob eine Population von mesenchymalen Stammzellen (MSCs) zur Verabreichung an ein Individuum für eine MSC-basierten Therapie geeignet ist, umfassend:

(a) Inkubieren von IL-2 mit der Population von MSCs;
(b) Messen der Expressionsspiegel in den MSCs von zumindest zwei Biomarkern, ausgewählt aus einem Panel von Biomarkern, wobei das Panel von Biomarkern aus TGFβ1, PLEKHA6, CTSB, FERMT1, CRMP1, VEGFB, VEGFA und PLEKHA1 besteht; und
(c) Vergleichen der Spiegel der Biomarker mit Referenzspiegeln, wobei ein Anstieg in den Spiegeln über die Referenzspiegel darauf hindeutet, dass die MSCs nicht für die Verabreichung an ein Individuum geeignet sind und keine Veränderung oder eine Abnahme in den Spiegeln unter die Referenzspiegel darauf hindeutet, dass die MSCs für die Verabreichung an eine Individuum geeignet sind.

5. Verfahren nach Anspruch 1 oder 4 oder IL-2-basiertes Therapeutikum zur Verwendung nach Anspruch 2, wobei das Verfahren das Messen der Expressionsspiegel von zumindest zwei Biomarkern, zumindest vier Biomarkern oder zumindest fünf Biomarkern aus dem Panel von Biomarkern umfasst.

6. Verfahren nach Anspruch 1 oder 4 oder IL-2-basiertes Therapeutikum zur Verwendung nach Anspruch 2, wobei das Verfahren Folgendes umfasst:

(i) Messen der Proteinspiegel der Biomarker; wobei gegebenenfalls das Messen durch ein ELISA-Assay, Antikörper-Proteomarray, Immunhistochemie oder Massenspektrometrie erfolgt; oder
(ii) Messen der RNA-Spiegel der Biomarker; wobei gegebenenfalls das Messen durch ein Q-PCR-Assay oder RNA-Seq. erfolgt.

7. Verfahren nach Anspruch 4, wobei die Zellen über 24 Stunden mit dem IL-2 inkubiert werden; oder wobei das Messen 24, 48 oder 72 Stunden nach dem Entfernen des IL-2 durchgeführt wird.

8. Population von mesenchymalen Stammzellen (MSCs) zur Verwendung in einem Verfahren zur Behandlung, wobei das Verfahren das Verfahren nach Anspruch 4 umfasst und ferner das Verabreichen der Population von Zellen an das Individuum umfasst.

**Revendications**

1. Procédé pour déterminer si un individu éligible pour recevoir une thérapie à base d'IL-2 peut subir un effet indésirable associé à la thérapie à base d'IL-2, le procédé comprenant :

   (a) la mesure des taux d'expression d'au moins deux biomarqueurs choisis parmi un panel de biomarqueurs dans un échantillon de sang, de plasma ou de sérum obtenu à partir de l'individu, dans lequel l'échantillon est combiné avec IL-2 *in vitro* avant une thérapie à base d'IL-2, et dans lequel le panel de biomarqueurs est constitué de TGFβ1, PLEKHA6, CTSB, FERMT1, CRMP1, VEGFB, VEGFA et PLEKHA1 ; et
   (b) la comparaison des taux des biomarqueurs à des taux de référence, dans lequel une augmentation des taux au-dessus des taux de référence indique que l'individu peut subir un effet indésirable associé à la thérapie à base d'IL-2, et dans lequel une diminution ou aucun changement des taux par rapport aux taux de référence indique que l'individu ne peut pas subir un effet indésirable associé à la thérapie à base d'IL-2.

2. Agent thérapeutique à base d'IL-2 pour utilisation dans un procédé de traitement d'un individu pour un cancer, le procédé comprenant l'administration d'une quantité efficace de l'agent thérapeutique à base d'IL-2 à l'individu lorsque les taux d'expression d'au moins deux biomarqueurs choisis parmi un panel de biomarqueurs sont diminués, ou ne présentent aucun changement, par rapport à des taux de référence dans un échantillon de sang, de plasma ou de sérum provenant de l'individu où l'échantillon est combiné avec IL-2 *in vitro* avant une thérapie à base d'IL-2, et où le panel de biomarqueurs est constitué de TGFβ1, PLEKHA6, CTSB, FERMT1, CRMP1, VEGFB, VEGFA et PLEKHA1.

3. Procédé selon la revendication 1, ou agent thérapeutique à base d'IL-2 pour utilisation selon la revendication 2, où l'échantillon est combiné avec IL-2 pendant 24 heures ; ou les taux d'expression sont mesurés 24, 48 ou 72 heures après le retrait d'IL-2 de l'échantillon *in vitro.*

4. Procédé *in vitro* pour déterminer si des cellules souches mésenchymateuses (CSM) sont adaptées pour administration à un individu pour une thérapie à base de CSM, comprenant :

   (a) l'incubation d'IL-2 avec la population de CSM ;
   (b) la mesure des taux d'expression dans les CSM d'au moins deux biomarqueurs choisis parmi un panel de biomarqueurs, dans lequel le panel de biomarqueurs est constitué de TGFβ1, PLEKHA6, CTSB, FERMT1, CRMP1, VEGFB, VEGFA et PLEKHA1 ; et
   (c) la comparaison des taux des biomarqueurs à des taux de référence, dans lequel une augmentation des taux au-dessus des taux de référence indique que les CSM ne sont pas adaptées pour administration à un individu et aucun changement ou une diminution des taux au-dessous des taux de référence indique que les CSM sont adaptées pour administration à un individu.

5. Procédé selon les revendications 1 ou 4, ou agent thérapeutique à base d'IL-2 pour utilisation selon la revendication 2, où le procédé comprend la mesure des taux d'expression d'au moins trois biomarqueurs, au moins quatre biomarqueurs, ou au moins cinq biomarqueurs parmi le panel de biomarqueurs.

6. Procédé selon les revendications 1 ou 4, ou agent thérapeutique à base d'IL-2 pour utilisation selon la revendication 2, où le procédé comprend :

   (i) la mesure des taux de protéine des biomarqueurs ; facultativement dans lequel la mesure est effectuée avec un dosage ELISA, un réseau protéomique à anticorps, l'immunohistochimie ou la spectrométrie de masse ; ou
   (ii) la mesure des taux d'ARN des biomarqueurs ; facultativement dans lequel la mesure est effectuée avec un dosage Q-PCR ou ARN-seq.

7. Procédé selon la revendication 4, dans lequel les cellules sont incubées avec l'IL-2 pendant 24 heures ; ou dans lequel la mesure est conduite 24, 48 ou 72 heures après le retrait de l'IL-2.

8. Population de cellules souches mésenchymateuses (CSM) pour utilisation dans un procédé de traitement, le procédé comprenant le procédé selon la revendication 4, et comprenant en outre l'administration de la population de cellules à l'individu.

# FIG. 1A

FIG. 1B

```
┌─────────────────────────────────┐
│     Treat patient with Il-2      │
│               101                │
└─────────────────────────────────┘
                 │
                 ▼
┌─────────────────────────────────┐
│  Take a blood sample (e.g., within │
│     24 hrs of treatment)         │
│               103                │
└─────────────────────────────────┘
                 │
                 ▼
┌─────────────────────────────────┐
│   Test sample for levels of anti- │
│    apoptotic and metastasis      │
│        promoting proteins        │
│               105                │
└─────────────────────────────────┘
                 │
                 ▼
┌─────────────────────────────────┐
│  Compare protein expression      │
│  level to threshold: matches     │
│     senescent phenotype?         │
│               107                │
└─────────────────────────────────┘
                 │
                 ▼
┌─────────────────────────────────┐
│ If yes, then do not treat with Il-2 │
│               109                │
└─────────────────────────────────┘
```

FIGS. 2A-2B

201

VEGFA

205

PLEKHA1

VEGFB

CRMP1

FERMT1

CTSB

PLEKHA6

GNB2L1

SIVA1

TGFB1

FIG. 2A

VEGFA

PLEKHA1

VEGFB

CRMP1

FERMT1

CTSB

PLEKHA6

GNB2L1

SIVA1

TGFB1

FIG. 2B

FIGS. 3A-D

A — Growth Curve for hADSCs

B — Senescence-associated β-galactosidase

C — Migration of ADSCs

D — Migration in presence of various cytokines

# FIGS. 4A-C

**A**

**B qPCR**

IL2Rα   IL2Rβ   IL2Rγ

**C ELISA**   IL2Rα   IL2Rβ

# FIG. 5

STAT5A

VEGFA

STAT5B

## FIGS. 6A-D

## FIGS. 7A-D

FIGS. 7A-D

FIGS. 8A-8D

FIG. 6B

## FIG. 6C

Up in SR    Up in Both    Up in SEN

SR SEN    SR SEN    SR SEN

IL-2  - + - +    - + - +    - + - +

-1.5   2.0  -1.5   2.0  -1.5   1.5

Gene expression

## FIG. 6D

Down in SR    Down in Both   Down in SEN

SR SEN    SR SEN    SR SEN

IL-2  - + - +    - + - +    - + - +

-2.0   2.0  -1.5   2.0  -1.5   1.5

Gene expression

## FIG. 7A
### Trophic factors

TGFBRAP1
SDF2
IL17D
TGFA
CSF1
TGFBR2
LIF
FGF1
EGFR
SRF
FGF7
FGF2
SFRP1
PTGES2
PDGFRA
PDGFA
FGF5
TGFB2
TGFB1
IL11
IL1B
FGF14
TNFSF13B
FGF11

SR          SEN
IL-2    -    +    -    +

## FIG. 7B
### Anti-inflammatory and immunomodulatory

CSF1R
TNFSF13
HIVEP2
ITGAV
ITFGI
IL32
PTGER1
HIVEP1
CERCAM
CD99
CD68
IL6
CD83
CD320
CD72
CHST3
TNFRSF25
CMIP
ESM1
CCL28
CLEC11A
LRRCBA
IL33
IRAK1
TNFRSF21
IL12A
IL12A
NOS3
MYL9
IRAK3
ILF3
IL16
PEAR1
IL7R
ILF2
CCL2
KIF14

SR          SEN
IL-2    -    +    -    +

## FIG. 7C
### Anti-appoptotic and metastasis promoting

VEGFA
PLEKHA1
VEGFB
CRMP1
FERMT1
CTSB
PLEKHA6
GNB2L1
SIVA1
TGFB1

SR          SEN
IL-2    -    +    -    +

-1    0    1
Gene expression

## FIG. 7D

Migration and angiogenesis
promoting

## FIG. 8A

# FIG. 8B

# FIG. 8C

# FIG. 8D

FIG. 9

ERCC Quality Control

## FIG. 10A  BIOLOGICAL PATHWAYS UPREGULATED UPON IL-2 TREATMENT

| PATHWAY | SELF-REPLICATING | FDR q-value | PATHWAY | SELF-REPLICATING | FDR q-value |
|---|---|---|---|---|---|
| ATM | CHEK2, CDKN1A, RAD51, MRE11A, NFKBIA | 2.62 e-2 | PROTEASOME | PSMB5, PSMB6, PSMD11, PSMD6, PSMA6, PSMA1, PSMA2, PSMA5, PSMA7, PSMB1, PSMB2, PSMB3, PSMB4, PSMC2, PSMC3, PSMC4 | 1 e-10 |
| PDGF | ELK1, PDGFA, RAF1, PDGFRA, STAT1 | 1.07 e-1 | | | |
| AKT | CHUK, BAD, HSP90AA1, NFKBIA | 1.06 e-1 | CELL CYCLE | CDK4, CCNH, CDKN1A, CDKN2C, CDK1, CDC25A, CDK2, CDKN1B,TFDP1, CCNB1 | 1.54 e-5 |
| VEGF | NOSS, EIF2S3, HIF1A, EIF2S1, EIF2B2, EIF2B3 | 2.94 e-2 | TNFR1 | CASP8, CASP2, PAK2, PAK1, LMNA, PARP1, CRADD, TNFRSF1A, TRAF2, DFFA, PRKDC | 1.75 e-5 |
| INTEGRIN | ZYX, ACTA1, BCAR1, RAF1, CAPN1 | 1.64 e-1 | | | |
| RAS | CDC42, ELK1, CHUK, RAF1, BAD | 4.27 e-2 | FAS | CASP8, PAK2, DAXX, PAK1, LMNA, PARP1, FAS, CASP7, DFFA, PRKDC | 1.36 e-4 |
| IL1R | IL1RAP, MYD88, CHUK, IRAK1, MAP3K14, IRAK3, NFKBIA | 1.57 e-2 | MAPK | RPS6KA4, ELK1, PAK1, DAXX, MAPKAPK3, CHUK, MAP3K11, MYC, PAK2, RAF1, MAP3K14, MAP3K6, MAPKAPK2, STAT1, NFKBIA, RPS6KA2, TRAF2 | 2.42 e-4 |
| RHO | PIP5K1A, ARPC1A, ARPC5, ARHGAP1, DIAPH1, ARHGEF5, CFL1, ARPC4, ACTR3, ARHGEF1 | 2.34 e-4 | G2 | CHEK2, PLK1, ATR, CCNB1, CDC25A, CDKN1A, CDK1, PRKDC | 5.94 e-4 |
| WNT | CTBP1, MYC, HDAC1, TLE1 | 1.51 e-1 | NFKB | TNFRSF1A, MYD88, CHUK, IRAK1, TLR4, MAP3K14, NFKBIA | 2.63 e-3 |
| | | | STRESS | TNFRSF1A, CHUK,CRADD, MAP3K14, NFKBIA, TRAF2, CASP2 | 4.3 e-3 |
| | | | CERAMIDE | TNFRSF1A, CASP8, AIFM1, RAF1, BAD,TRAF2 | 9.96 e-3 |
| | | | P53 | CDK4, CDKN1A, CDK2, PCNA, APAF1 | 1.18 e-2 |
| | | | ERK | ELK1, MYC,NGFR, RAF1, EGFR, PDGFRA | 2.62 e-2 |
| | | | TOLL | MAP3K14, ELK1, MYD88, IRAK1, CHUK, NFKBIA, TLR4 | 2.63 e-2 |
| | | | FMLP | FPR1, RAF1, PAK1, NFATC1, ELK1, CALM3, NFKBIA | 3.41 e-2 |
| | | | SODD | CASP8, TNFRSF1A, TRAF2 | 6.41 e-2 |
| | | | ETS | E2F4, ETS2, DDX20, ETS1 | 6.57 e-2 |
| | | | TNFR2 | MAP3K14, CHUK, TRAF2, NFKBIA | 6.57 e-2 |
| | | | RAC1 | CDK5, PAK1, CFL1, PDGFRA | 1.17 e-1 |
| | | | CXCR4 | RAF1, GNAI1, BCAR1, CXCL12 | 1.3 e-1 |
| | | | CD40 | MAP3K14, CHUK, NFKBIA | 1.34 e-1 |

SELF-REPLICATING

PROTEASOME, CD40, AKT, IL1R, STRESS, ETS, VEGF, TNFR2, NFKB, TOLL, ATM, RAS, MAPK, SODD, CERAMIDE, CELL CYCLE, FMLP, INTEGRIN, WNT, ERK, RHO, TNFR1, PDGF, CXCR4, RAC1, P53, G2, FAS

## FIG. 10A (Continued)

| PATHWAY | SENESCENT | FDR q-value |
|---|---|---|
| ATM | JUN, GADD45A, MDM2, NBN, MAPK8, ATM, RBBP8, ABL1, RAD50 | 8.15 e-5 |
| PDGF | RASA1, JUN, FOS, PRKCA, PIK3CA, MAPK8, STAT5A, HRAS, STAT4 | 6.86 e-4 |
| AKT | FOXO4, CASP9, PIK3CA, AKT1, PDPK1, FOXO1, FOXO3 | 1.6 e-3 |
| VEGF | VEGFA,EIF2S2, EIF1AX, PRKCA, PIK3CA, EIF2B4, HRAS | 5.24 e-3 |
| INTEGRIN | JUN, RAP1A, MAPK8, ROCK1, CSK, HRAS, SRC, PPP1R12B | 5.55 e-3 |
| RAS | RALA, FOXO4, CASP9, PIK3CA, AKT1, HRAS | 6.57 e-3 |
| IL1R | JUN, IL1RN, IRAK2, TRAF6, MAPK8, IL6 | 2.51 e-2 |
| RHO | ARHGAP5, BAIAP2, ROCK1, SRC, PPP1R12B, OPHN1 | 2.34 e-2 |
| WNT | GSK3B, BTRC, SMAD4, APC, AXIN1 | 3.37 e-2 |
| EGF | RASA1, JUN, FOS, EGF, PRKCA, PIK3CA, MAPK8, STAT5A, HRAS, STAT4 | 2.33 e-4 |
| LONGEVITY | SOD2, IGF1R, PIK3CA, AKT1, SOD1, FOXO3, HRAS | 2.53 e-4 |
| IL2 | JUN, FOS, JAK3, MAPK8, STAT5A, HRAS | 5.55 e-3 |
| IL6 | JUN, CEBPB, FOS, JAK3, IL6, HRAS | 5.55 e-3 |
| IL7 | PTK2B, JAK3, EP300, PIK3CA, STAT5A | 8.47 e-3 |
| TGFB | TGFBR1, SKIL, EP300, SMAD4, APC | 1.25 e-2 |
| IL2RB | FOS, JAK3, PIK3CA, STAT5A, AKT1, CBL, HRAS | 1.51 e-2 |
| B-CELL SURVIVAL | FOS, PIK3CA, XIAP, AKT1 | 2.76 e-2 |
| LEPTIN | LEP, PRKAB2, PRKAA2 | 5.08 e-2 |
| IL12 | JUN, ETV5, MAPK8, STAT4 | 7.39 e-2 |
| NOS1 | PRKACB, GRIN2D, PRKCA, DLG4 | 8.32 e-2 |

SENESCENT

Node size — #Genes: 3 4 5 6 7 8 9 10 11 12

EP 3 303 636 B1

## FIG. 10B  BIOLOGICAL PATHWAYS DOWNREGULATED UPON IL-2 TREATMENT

| PATHWAY | SELF-REPLICATING | FDR q-value |
|---------|------------------|-------------|
| G2 | GADD45A, CDKN2D | 5.35 e-1 |
| P53 | GADD45A, BAX | 5.35 e-1 |
| MTOR | EIF3A, FKBP1A | 5.35 e-1 |
| ETS | NCOR2, CSF1R | 5.35 e-1 |

FIG. 10B
(Continued)

SENESCENT

## FIG. 10B
## (Continued)

| PATHWAY | SENESCENT | FDR q-value |
|---|---|---|
| G2 | PRKDC, CDK1, CDKN1A, CCNB1, CHEK1, CDC25C, CDC25B, PLK1 | 3.06 e-5 |
| P53 | CDK2, CDKN1A, CDK4, PCNA | 1.86 e-2 |
| MTOR | EIF4A1, PRS6, TSC1 | 1.59 e-1 |
| MCM | CDK2, KITLG, ORC6, ORC3, MCM2, MCM3, MCM4, MCM5, MCM7, CDT1 | 1.62 e-8 |
| PROTEA-SOME | PSMA7, PSMA5, PSMA6, PSMB3, PSMB5, PSMB6, PSMB7, PSMC2, PSMC3 | 2.17 e-5 |
| HIV-I NEF | PRKDC, CASP8, LMNA, PARP1, LMNB1, LMNB2, MAP3K1, TRAF2, CYCS, MAP3K14, TNFRSF1B, ACTG1 | 2.52 e-5 |
| CELL CYCLE | CDK2, CDK1, CDKN1A, CCNB1, CDK4, TFDP1, CDKN2C, CCND3 | 2.52 e-5 |
| RB | CDK2, CDK1, CDK4, CHEK1, CDC25C, CDC25B | 6.97 e-5 |
| TNFR1 | PRKDC, CASP8, LMNA, PARP1, LMNB1, LMNB2, MAP3K1, TRAF2 | 1.02 e-4 |
| FAS | PRKDC, CASP8, LMNA, PARP1, LMNB1, LMNB2, MAP3K1, TRAF2 | 1.1 e-3 |
| TNFR2 | MAP3K1, TRAF2, MAP3K14, TNFRSF1B, IKBKAP | 4.02 e-3 |
| NFAT | ACTA1, MEF2C, CALM3, CAMK1G, CTF1, FGF2, HAND2 | 2.9 e-2 |
| SODD | CASP8, TRAF2, TNFRSF1B | 3.24 e-2 |
| ATM | CDKN1A, CHEK1, MRE11A, RAD51 | 3.28 e-2 |

| PATHWAY | SENESCENT | FDR q-value |
|---|---|---|
| PDGF | MAP3K1, STAT1, SRF, PDGFRA, PDGFA | 3.28 e-2 |
| CERAMIDE | CASP8, MAP3K1, TRAF2, CYCS | 3.92 e-2 |
| NFKB | MAP3K1, MAP3K14, TNFRSF1B, IL1R1 | 4.25 e-2 |
| RAC1 | MAP3K1, PDGFRA, CFL1, CHN1 | 4.25 e-2 |
| CD40 | IKBKAP, MAP3K1, MAP3K14 | 6.79 e-2 |
| B-CELL SURVIVAL | BIRC5, CCT4, ITGA1 | 7.61 e-2 |
| CDC42RAC | ARPC4, CDC42, PDGFRA | 7.61 e-2 |
| IL-10 | BLVRA, BLVRB, STAT1 | 8.64 e-2 |
| EGF | EGFR, MAP3K1, SRF, STAT1 | 9.19 e-2 |
| RHO | ARHGAP1, ARPC4, CFL1, PIP5K1A | 1 e-1 |
| IL-1R | IL1R1, IRAK3, MAP3K1, MAP3K14 | 1.09 e-1 |
| mCALPAIN | ACTA1, EGFR, ITGA1 | 1.79 e-1 |
| STRESS | MAP3K1, MAP3K14, TRAF2 | 1.79 e-1 |
| TALL1 | MAP3K14, TRAF2 | 2.81 e-1 |
| NO1 | ACTA1, CALM3, FLT1 | 2.97 e-1 |
| 4-1BB | MAP3K1, TRAF2 | 3.21 e-1 |
| LAIR | C5, ITGA4 | 3.21 e-1 |
| ETS | E2F4, ETS1 | 3.4 e-1 |
| FMLP | CALM3, CAMK1G, MAP3K1 | 3.8 e-1 |
| CCR5 | CALM3, CCL2 | 3.8 e-1 |
| ERK | EGFR, PDGFRA | 5.82 e-1 |
| TOLL | MAP3K1, MAP3K14 | 8.06 e-1 |
| INTEGRIN | ACTA1, ITGA1 | 8.25 e-1 |

FIG. 11

SR-MSCs
No IL-2 Stimulation

FIG. 12

SR-MSCs
No IL-2 Stimulation

FIG. 13

SR-MSCs
No IL-2 Stimulation

FIG. 14

SR-MSCs
No IL-2 Stimulation

# FIG. 15

SR-MSCs
No IL-2 Stimulation

FIG. 16

SR-MSCs
No IL-2 Stimulation

FIG. 17

SR-MSCs
No IL-2 Stimulation

## FIG. 18

SR-MSCs
No IL-2 Stimulation

## FIG. 19

SR-MSCs
No IL-2 Stimulation

FIG. 20

SR-MSCs
No IL-2 Stimulation

FIG. 21

SR-MSCs
No IL-2 Stimulation

## FIG. 22

SR-MSCs
No IL-2 Stimulation

# FIG. 23

SR-MSCs
No IL-2 Stimulation

FIG. 24

SR-MSCs
No IL-2 Stimulation

FIG. 25

SR-MSCs
No IL-2 Stimulation

FIG. 26

SR-MSCs
No IL-2 Stimulation

FIG. 27

SR-MSCs
No IL-2 Stimulation

FIG. 28

SR-MSCs
No IL-2 Stimulation

FIG. 29

SR-MSCs
No IL-2 Stimulation

FIG. 30

SR-MSCs
No IL-2 Stimulation

FIG. 31

SR-MSCs
No IL-2 Stimulation

FIG. 32

SR-MSCs
No IL-2 Stimulation

FIG. 33

SR-MSCs
No IL-2 Stimulation

FIG. 34

SR-MSCs
No IL-2 Stimulation

FIG. 35

SR-MSCs
No IL-2 Stimulation

## FIG. 36

SR-MSCs
No IL-2 Stimulation

## FIG. 37

SR-MSCs
No IL-2 Stimulation

FIG. 38

SR-MSCs
No IL-2 Stimulation

FIG. 39

SR-MSCs
No IL-2 Stimulation

FIG. 40

SR-MSCs
No IL-2 Stimulation

FIG. 41

SR-MSCs
No IL-2 Stimulation

FIG. 42

SR-MSCs
No IL-2 Stimulation

FIG. 43

SR-MSCs
+ IL-2 Stimulation

FIG. 44

SR-MSCs
+ IL-2 Stimulation

FIG. 45

SR-MSCs
+ IL-2 Stimulation

## FIG. 46

SR-MSCs
+ IL-2 Stimulation

## FIG. 47

SR-MSCs
+ IL-2 Stimulation

FIG. 48

SR-MSCs
+ IL-2 Stimulation

FIG. 49

SR-MSCs
+ IL-2 Stimulation

FIG. 50

SR-MSCs
+ IL-2 Stimulation

FIG. 51

SR-MSCs
+ IL-2 Stimulation

FIG. 52

SR-MSCs
+ IL-2 Stimulation

FIG. 53

SR-MSCs
+ IL-2 Stimulation

FIG. 54

■24h □48h ▨72h

SR-MSCs
+ IL-2 Stimulation

■24h □48h

FIG. 55

SR-MSCs
+ IL-2 Stimulation

FIG. 56

SR-MSCs
+ IL-2 Stimulation

FIG. 57

SR-MSCs
+ IL-2 Stimulation

FIG. 58

SR-MSCs
+ IL-2 Stimulation

FIG. 59

SEN-MSCs
No IL-2 Stimulation

FIG. 60

SEN-MSCs
No IL-2 Stimulation

FIG. 61

SEN-MSCs
No IL-2 Stimulation

FIG. 62

SEN-MSCs
No IL-2 Stimulation

FIG. 63

SEN-MSCs
No IL-2 Stimulation

# FIG. 64

SEN-MSCs
No IL-2 Stimulation

FIG. 65

SEN-MSCs
No IL-2 Stimulation

EP 3 303 636 B1

## FIG. 66

SEN-MSCs
No IL-2 Stimulation

FIG. 67

SEN-MSCs
No IL-2 Stimulation

# FIG. 68

SEN-MSCs
No IL-2 Stimulation

## FIG. 69

SEN-MSCs
No IL-2 Stimulation

FIG. 70

SEN-MSCs
No IL-2 Stimulation

FIG. 71

SEN-MSCs
No IL-2 Stimulation

FIG. 72

SEN-MSCs
No IL-2 Stimulation

## FIG. 73

SEN-MSCs
No IL-2 Stimulation

## FIG. 74

SEN-MSCs
No IL-2 Stimulation

FIG. 75

FIG. 76

SEN-MSCs
+ IL-2 Stimulation

FIG. 77

SEN-MSCs
+ IL-2 Stimulation

FIG. 78

SEN-MSCs
+ IL-2 Stimulation

FIG. 79

SEN-MSCs
+ IL-2 Stimulation

# FIG. 80

SEN-MSCs
+ IL-2 Stimulation

FIG. 81

SEN-MSCs
+ IL-2 Stimulation

# FIG. 82

SEN-MSCs
+ IL-2 Stimulation

# FIG. 83

SEN-MSCs
+ IL-2 Stimulation

## FIG. 84

SEN-MSCs
+ IL-2 Stimulation

FIG. 85

SEN-MSCs
+ IL-2 Stimulation

FIG. 86

SEN-MSCs
+ IL-2 Stimulation

## FIG. 87

SEN-MSCs
+ IL-2 Stimulation

FIG. 88

SEN-MSCs
+ IL-2 Stimulation

FIG. 89

SEN-MSCs
+ IL-2 Stimulation

## FIG. 90

SEN-MSCs
+ IL-2 Stimulation

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2013252821 A1

### Non-patent literature cited in the description

- **KUCEROVA.** *Cancer Res,* 01 July 2007, vol. 67, 6304
- **PANELLI MONICA C et al.** Forecasting the cytokine storm following systemic interleukin (IL)-2 administration. *JOURNAL OF TRANSLATIONAL MEDICINE, BIOMED CENTRAL,* 02 June 2004, vol. 2, 17
- **FENG et al.** *Bioinformatics,* 2012
- **DEENICK EK ; GETT AV ; HODGKIN PD.** *J Immunol,* 2003, vol. 170, 4963-4972
- **WANG J ; GEESMAN GJ ; HOSTIKKA SL ; ATALLAH M ; BLACKWELL B et al.** Inhibition of activated pericentromeric SINE/Alu repeat transcription in senescent human adult stem cells reinstates self-renewal. *Cell cycle,* 2011, vol. 10, 3016-3030
- **PEREZ LM ; BERNAL A ; SAN MARTIN N ; GALVEZ BG.** *Arch Physiol Biochem,* 2013, vol. 119, 195-201
- **PEREZ et al.** *Arch Physiol Biochem,* 2013, vol. 119, 195-201
- **DEENICK EK ; GETT AV ; HODGKIN PD.** Stochastic model of T cell proliferation: a calculus revealing IL-2 regulation of precursor frequencies, cell cycle time, and survival. *J Immunol,* 2003, vol. 170, 4963-4972
- **FENG J ; MEYER CA ; WANG Q ; LIU JS ; SHIRLEY LIU X et al.** GFOLD: a generalized fold change for ranking differentially expressed genes from RNA-seq data. *Bioinformatics,* 2012, vol. 28, 2782-2788